(19) Europäisches Patentamt / European Patent Office / Office européen des brevets

(11) EP 4 509 500 A1

(12) **EUROPEAN PATENT APPLICATION**
published in accordance with Art. 153(4) EPC

(43) Date of publication:
**19.02.2025 Bulletin 2025/08**

(21) Application number: **23807064.3**

(22) Date of filing: **19.05.2023**

(51) International Patent Classification (IPC):
***C07D 401/04*** (2006.01) ***C07D 401/14*** (2006.01)
***C07D 471/08*** (2006.01) ***C07D 487/04*** (2006.01)
***C07D 409/06*** (2006.01) ***C07D 207/14*** (2006.01)
***C07D 211/58*** (2006.01) ***C07D 405/14*** (2006.01)
***C07D 413/04*** (2006.01) ***C07D 417/14*** (2006.01)
***C07D 413/14*** (2006.01) ***C07D 409/14*** (2006.01)
***C07D 471/10*** (2006.01) ***C07D 403/10*** (2006.01)
***A61K 31/4545*** (2006.01) ***A61K 31/4427*** (2006.01)
***A61K 31/506*** (2006.01) ***A61K 31/439*** (2006.01)
***A61P 35/00*** (2006.01) ***A61P 37/00*** (2006.01)

(52) Cooperative Patent Classification (CPC):
**A61K 31/439; A61K 31/4427; A61K 31/4545; A61K 31/506; A61P 35/00; A61P 37/00; C07D 207/14; C07D 211/58; C07D 401/04; C07D 401/14; C07D 403/10; C07D 405/14; C07D 409/06; C07D 409/14; C07D 413/04;** (Cont.)

(86) International application number:
**PCT/CN2023/095304**

(87) International publication number:
**WO 2023/222115 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.05.2022 CN 202210550737**
**03.11.2022 CN 202211367769**

(71) Applicants:
- **Sichuan Huiyu Pharmaceutical Co., Ltd.**
**Neijiang, Sichuan 641000 (CN)**
- **Sichuan Huiyu Seacross Pharmaceutical Technology Co., Ltd.**
**Chengdu, Sichuan 610200 (CN)**

(72) Inventors:
- **CHEN, Shoujun**
**Neijiang, Sichuan 641000 (CN)**
- **YAN, Jiaming**
**Neijiang, Sichuan 641000 (CN)**
- **DING, Zhao**
**Neijiang, Sichuan 641000 (CN)**
- **LI, Min**
**Neijiang, Sichuan 641000 (CN)**
- **WANG, Haibo**
**Neijiang, Sichuan 641000 (CN)**

(74) Representative: **Huang, Liwei**
**Cäcilienstraße 12**
**40597 Düsseldorf (DE)**

# (54) HYDROXYAMIDE DERIVATIVE AND USE THEREOF

(57) Provided in the present application is a hydroxylamide derivative represented by formula(I), and a tautomer, stereoisomer, solvate, metabolite, isotopically-labeled compound, pharmaceutically acceptable salt or co-crystal thereof. The compound provided in the present application has an inhibitory effect on both HDAC and LSD1, and can be used for treating diseases mediated by LSDI and/or HDAC.

EP 4 509 500 A1

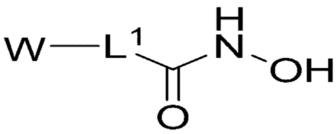

(I)

(52) Cooperative Patent Classification (CPC): (Cont.)
**C07D 413/14; C07D 417/14; C07D 471/08;
C07D 471/10; C07D 487/04**

## Description

## FIELD OF THE INVENTION

The present disclosure belongs to the field of medicinal chemistry, and specifically discloses a hydroxylamide derivative and use thereof. This class of the compound has obvious inhibition effects on LSD1 and HDAC protein activities, can be used as an inhibitor for LSD1 and/or HDAC protein, can be used in the manufacture of a medicament for treating cancer and other diseases mediated by LSD1, HDAC protein, and has a wide application prospect.

## BACKGROUND

LSD1 protein (Lysine Specific Demethylase 1, Histone Demethylase, also known as KDM1A) was first discovered and reported by Shi Yang team of Harvard University in 2004 (Shi, Y., Lan, F., Matson, C., Mulligan, P., Whetstine, J.R., Cole, P.A., Casero, R.A., and Shi, Y.. Histone demethylation mediated by the nuclear amine oxidase homolog LSD1. Cell 2004, 119, 941-953). As a histone demethylase involved in transcriptional regulation, LSD1 has a variety of biological functions, mainly including promoting tumor proliferation, inhibiting energy metabolism, promoting lipogenesis, inhibiting lipolysis and regulating cell differentiation, etc. Inhibition of LSD1 function can enhance the expression of endogenous retroviral elements (ERVs) and inhibit the function of RISC (RNA-induced silencing complex) complex, resulting in the overexpression of double-stranded RNA (dsRNA) and the activation of type I interferon (IFN) (Doll, S., Kriegmair, M. C., Santos, A., Wierer, M., Coscia, F., Neil, H. M., et al. Rapid proteomic analysis for solid tumors reveals LSD1 as a drug target in an end-stage cancer patient. Molecular Oncology, 2018, 12(8), 1296-1307.). At the same time, researchers found that LSD1 showed a trend of overexpression in some types of cancer with regard to public databases of human cancers. The survival time of patients with LSD1 overexpression was significantly shortened, suggesting that LSD1 overexpression is a poor prognostic factor. In addition, LSD1 was also found to be highly expressed in various cancer tissues, and more and more reports indicated that LSD1 was involved in various tumor processes and embryonic development as an epigenetic regulator. The TCGA cancer database also showed that LSD1 expression was negatively correlated with IFN antiviral effects and CD8 T cell infiltration, which is consistent with the tests in mouse models. Therefore, inhibition of LSD1 can enhance tumor immunogenicity and promote T cell infiltration, activate anti-tumor T cell immunity, and can be used as a target for tumor therapy in combination with anti-PD-1 immunotherapy. Relevant research results also indicated that inhibition of DNA methylation alone or in combination with HDAC inhibitors can lead to the activation of tumor interferon (IFN) pathway and enhance the efficacy of tumor immunotherapy. Meanwhile, blocking DNA methylation in T cells can also enhance T cell activity and tumor suppression mediated by PD-1/PD-L1 immunotherapy (Chiappinelli, K.B., Strissel, P.L., Desrichard, A., Li, H., Henke, C., Akman, B., Hein, A., Rote, N.S., Cope, L.M., Snyder, A.,et al. Inhibiting DNA methylation causes an interferon response in cancer via dsRNA including endogenous retroviruses. Cell 2015, 162, 974-986; Topper, M.J., Vaz, M., Chiappinelli, K.B., DeStefano Shields, C.E., Niknafs, N., Yen, R.C., Wenzel, A., Hicks, J., Ballew, M., Stone, M., et al. Epigenetic therapy ties MYC depletion to reversing immune evasion and treating lung cancer. Cell 2017, 171, 1284-130; Ghoneim, H.E., Fan, Y., Moustaki, A., Abdelsamed, H.A., Dash, P., Dogra, P., Carter, R., Awad, W., Neale, G., Thomas, P.G., et al. De novo epigenetic programs inhibit PD-1 blockade-mediated t cell rejuvenation. Cell 2017, 170, 142-157).

LSD1 is composed of 852 amino acids and has a molecular weight of 93 kDa. Analysis of 27 tissue samples from 95 individuals showed that LSD1 was widely expressed in the body, with less secretion in the liver, pancreas and salivary glands, and higher expression in testicular tissues, while the expression levels in other tissues were similar. Research found that the expression level of LSD1 was significantly increased in different tumor tissues, such as neuroblastoma, breast cancer (Wang, Y.; Zhang, H.; et al, Cell 2009, 138(4), 660-72.), prostate cancer (Zhao, L.-J.; Fan, Q.-Q.; et al., Pharmacol. Res. 2020, 159, 104991), pancreatic cancer (Sehrawat, A.; Gao,L.; et al., Proc. Nat. Acad. Sci. USA 2018, 115(18), E4179-E4188.), colon cancer and glioma and blood cancer (Hatzi , K.; Geng, H.; et al., Nature Immunology 2019, 20(1), 86-96.). Moreover, high expression of LSD1 is often associated with poorer tumor prognosis and recurrence after treatment (Lynch, J.; Harris, W.; et al., Expert Opinion on Therapeutic Targets 2012, 16(12), 1239-1249.).

Researches showed that LSD1 exerted the biological functions thereof not only by demethylating histones, but also by demethylating non-histone proteins p53 and Dnmt1. The biological roles of LSD1 are mainly manifested in the regulation of sex hormone receptor-mediated gene transcription, the regulation of tumor cell proliferation, apoptosis and metastasis, as well as the regulation of embryonic development (Ancelin, K.; Syx , L.; et al., eLife 2016, 5, e08851/1-e08851/24.), mitosis, etc. Additionally, LSD1 was reported to be associated with osteoporosis(Sun, J.; Ermann, J.; et al., Bone Res. 2018, 6(1), 1-12); in addition, LSD1 inhibition was found to be associated with macrophage phenotypic polarization (Tan, A.H.Y.; Tu, W.J.; et al., Front. Immunol. 2019, 10, 1351.) and CD8+ T-cell infiltration in the tumor microenvironment (Hatzi , K.; Geng, H.; et al., Nature Immunology 2019, 20(1), 86-96) in studies of LSD1 inhibition. Therefore, the development of the LSD1 inhibitor is one of the hotspots in the field of tumor research.

Histone deacetylase (HDAC) is involved in histone acetylation, binding to deacetylated proteins and interacting

with nonhistone proteins. HDAC has a wide range of biological functions, including neurodegeneration, inflammation, metabolic disorders, cancer, etc. HDAC1 is a possible prognostic marker for lung cancer and breast cancer and is overexpressed in prostate cancer, gastric cancer and colon cancer; HDAC2 is commonly overexpressed in colorectal cancer and gastric cancer; HDAC3 expression is elevated in lung cancer and most solid tumors; HDAC6 is mainly overexpressed in breast cancer; knockdown of HDAC8 can inhibit tumor cell growth and proliferation in various human tumor cells. In cancer cells, overexpression of HDACs leads to enhanced deacetylation and unfavorable expression of specific genes, including some tumor suppressor genes. So far, five HDAC inhibitors (HDACis) have been approved for marketing, namely Vorinostat (SAHA), Romidepsin (FK228), Belinostat (PXD-101), Panobinostat (LBH-589) and Chidamide, for treating various tumors such as malignant lymphoma, myeloma, hematologic cancers, and pancreatic cancer. Additionally, there are also several HDACs inhibitor candidates in clinical trials. The LSD1 and HDACs targets both play important roles in the occurrence and development of certain tumors, and in various cancers such as bladder, breast and lung cancers, reducing LSD1 expression or inhibiting LSD1 activity can significantly enhance the sensitivity of cancer cells to HDACs inhibitors. Duan, et al., reported that simultaneous inhibition of the activities of LSD1 and HDACs with small molecule inhibitors had synergistic antitumor effects (Duan, Y.C.; et al, Eur J Med Chem. 2021, 220, 113453. doi: 10.1016/j.ejmech.2021.113453.). Meanwhile, studies showed that dual-targeted drugs had more predictable complex metabolic pathways, better PK/PD properties, and better bioavailability than multidrug combinations (Giulia S.; et al, Current Opinion in Chemical Biology 2019, 50, 89-100). In addition, dual-targeted drugs can ensure that the dual pharmacodynamic moieties can synchronize their effects at the same time in the same cell, as opposed to a combination of drugs. (de Lera, A.R.; Ganesan, A., Clin Epigenetics 2016, 8:105.) Additional advantages of dual-targeted monotherapy include improved patient compliance and lower medication costs. (Fu, R.G., Sun, Y, Sheng, W.B., Liao, D.F., Eur. J. Med. Chem. 2017, 136, 195-211) Cole and colleagues recently reported a class of LSD1/HDAC1 dual-targeted inhibitors that showed good *in vivo* activity in a mouse model of melanoma (Kalin, J.H.; et al; Nat. Commun., 2018, 9, 53). Other academic institutions such as Xinxiang Medical College have recently disclosed several patents for dual LSD1/HDAC inhibitors (CN111592487; CN113444038; CN113527195).

In view of the fact that no dual-targeted drug has been marketed yet, there is still an urgent need for the development of new dual-targeted inhibitors that inhibit both HDAC and LSD1.

## SUMMARY OF THE INVENTION

The present disclosure provides a compound represented by formula (I) or a tautomer, stereoisomer, solvate, metabolite, isotopically-labeled compound, pharmaceutically acceptable salt or co-crystal thereof,

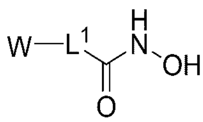

**(I)**

wherein,

$L^1$ is selected from a bond, $-C_{1-10}$ alkyl-, $-C_{2-6}$ alkenyl-, $-C_{1-10}$ alkyl-$C_{2-6}$ alkenyl-, $-C_{2-6}$ alkynyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-NR^a-$, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, $-C_{1-10}$ alkyl-NH-6-10-membered heteroaryl-, $-C_{1-10}$ alkyl-6-10-membered heteroaryl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$ cycloalkenyl-, $-C_{1-10}$ alkyl-$C_{6-10}$ aryl-$C_{3-6}$ cycloalkyl-, $-C_{1-10}$ alkyl-O-$C_{6-10}$ aryl-, $-C_{1-10}$ alkyl-6-10-membered heteroaryl-$C_{1-10}$ alkyl-, $-C_{1-10}$ alkyl-6-10-membered aryl-O-$C_{1-10}$ alkyl-, $-C_{1-10}$ alkyl-6-10-membered heteroaryl-O-$C_{1-10}$ alkyl-, $-C_{1-10}$ alkyl-6-10-membered aryl-S-$C_{1-10}$ alkyl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $-NR^aR^b$, COOH, $-C(=O)NR^aR^b$, the heteroaryl, heterocycloalkyl contain 1 to 4 heteroatoms optionally selected from N, O, or S; alternatively, one or more alkyl groups of the alkyl may optionally be replaced by one or more groups selected from -C(=O)-, $-S(=O)_2-$ or $-NR^a-$;

preferably, $L^1$ is selected from a bond, $-C_{1-10}$ alkyl-, $-C_{2-6}$ alkenyl-, $-C_{1-10}$ alkyl-$C_{2-6}$ alkenyl-, $-C_{2-6}$ alkynyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-NR^a-$, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, $-C_{1-10}$ alkyl-NH-6-10-membered heteroaryl-, $-C_{1-10}$ alkyl-6-10-membered heteroaryl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$

cycloalkenyl-, -$C_{1-10}$ alkyl-$C_{6-10}$ aryl-$C_{3-6}$ cycloalkyl-, -$C_{1-10}$ alkyl-O-$C_{6-10}$ aryl-, -$C_{1-10}$ alkyl-6-10-membered heteroaryl-O-$C_{1-10}$ alkyl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, -$NR^aR^b$, COOH, -C(=O)$NR^aR^b$, the heteroaryl, heterocycloalkyl contain 1 to 4 heteroatoms optionally selected from N, O, or S; alternatively one or more alkyl groups of the alkyl may optionally be replaced by one or more groups selected from -C(=O)-, -$S(=O)_2$- or -$NR^a$-;

preferably, the left end group of $L^1$ is connected to W, the right end group of $L^1$ is connected to

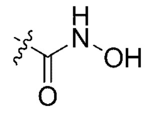

;

W is selected from:

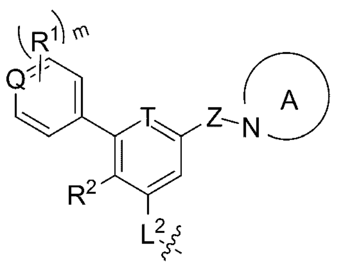

,

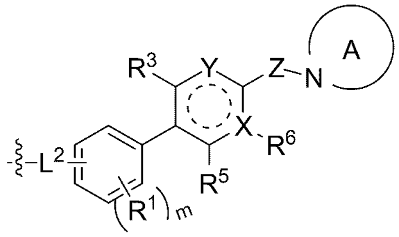

,

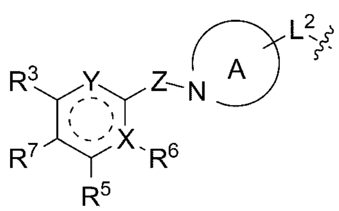

,

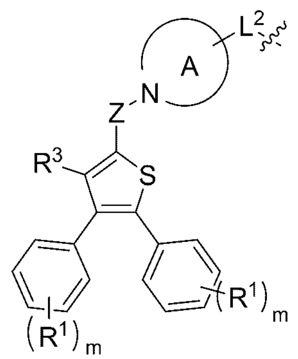

or

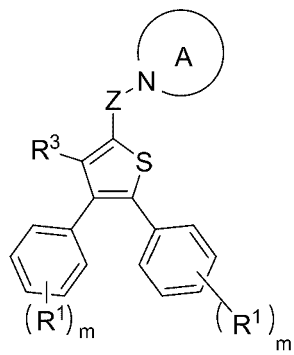

$L^2$ is selected from a bond, -O-, -C(=O)-, -$NR^a$-, -$CH_2$-$NR^a$-, -$NR^a$-C(O)-, -$NR^a$-$S(=O)_2$-, -S- or -$S(=O)_2$-;

preferably, $L^2$ is selected from a bond, -O-, -C(=O)-, -$NR^a$-, -$NR^a$-C(O)-, -$NR^a$-$S(=O)_2$-, -S- or - $S(=O)_2$-;

ring A is selected from nitrogen-containing $C_{3-10}$ heteroaryl, $C_{3-10}$ heterocycloalkyl or $C_{3-10}$ heterocycloalkenyl, wherein, the heteroaryl, heterocycloalkyl, heterocycloalkenyl are optionally substituted with one or more $R^4$, the heteroaryl, heterocycloalkyl contain 1 to 4 heteroatoms optionally selected from N, O, or S;

$R^4$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, COOH, -$NR^aR^b$, -C(=O)$NR^aR^b$; alternatively, when $R^4$ is selected from $C_{1-6}$ alkyl, any two $R^4$ and the atom to which they connect can collectively form a 5 to 10-membered heteroalicyclic;

$R^1$, $R^6$ are each independently selected at each occurrence from hydrogen, halogen, CN, hydroxyl, $C_{1-6}$ alkyl, $C_{1-6}$ alkyl-CN, $C_{1-6}$ alkoxy, hydroxyl-substituted $C_{1-6}$ alkyl, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl, COOH, -$NR^aR^b$, -C(=O)$NR^aR^b$, -$S(=O)_2R^a$, -$C_{2-6}$ alkenyl-C(=O)$NR^aR^b$;

preferably, $R^1$, $R^6$ are each independently selected at each occurrence from hydrogen, halogen, CN, hydroxyl, $C_{1-6}$ alkyl, $C_{1-6}$ alkoxy, hydroxyl-substituted $C_{1-6}$ alkyl, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl, COOH, -$NR^aR^b$, -C(=O)$NR^aR^b$, -$S(=O)_2R^a$, -$C_{2-6}$ alkenyl-C(=O)$NR^aR^b$;

$R^2$, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkoxy, halogen-substituted $C_{1-6}$ alkyl, hydroxyl-substituted $C_{1-6}$ alkyl, hydroxyl-substituted $C_{1-6}$ alkoxy, hydroxyl-substituted $C_{1-6}$ alkoxy-$C_{3-6}$ cycloalkyl, COOH, -$NR^aR^b$, -$S(=O)_2R^a$, -C(=O)$NR^aR^b$, -$C_{2-6}$ alkenyl-C(=O)$NR^aR^b$, $C_{3-6}$ cycloalkyl, 3 to 6-membered cycloheterocycloalkyl, heterocycloalkenyl, the heteroaryl, heterocycloalkyl contain 1 to 4 heteroatoms optionally selected from N, O, or S;

preferably, $R^2$, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkoxy, hydroxyl-substituted $C_{1-6}$ alkyl, hydroxyl-substituted $C_{1-6}$ alkoxy, COOH, $-NR^aR^b$, $-S(=O)_2R^a$, $-C(=O)NR^aR^b$, $-C_{2-6}$ alkenyl-$C(=O)NR^aR^b$, $C_{3-6}$ cycloalkyl, 3 to 6-membered cycloheterocycloalkyl, heterocycloalkenyl, the heteroaryl, heterocycloalkyl contain 1 to 4 heteroatoms optionally selected from N, O, or S;

m is selected from 0, 1, 2, 3, 4 or 5;

Q, T are each independently selected from N or C;

X, Y are each independently selected from C and N;

Z is selected from a bond, $-CH_2-$, -C(=O) or $-S(=O)_2-$;

preferably, $R^5$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, =O, COOH, $-NR^aR^b$, $-C(=O)NR^aR^b$, $C_{3-6}$ cycloalkyl, 3 to 6-membered cycloheterocycloalkyl, $C_{6-10}$ aryl or $C_{6-10}$ heteroaryl, wherein, the alkyl, alkoxy, alkenyl, alkynyl, cycloalkyl, heterocycloalkyl, aryl or heteroaryl is optionally substituted with one or more substituents selected from hydrogen, halogen, CN, $NO_2$, $CF_3$, $CHF_2$, hydroxyl, $C_{1-6}$ alkyl, $C_{1-6}$ alkoxy, $-C(=O)-C_{1-6}$ alkoxy, $C_{2-6}$ alkenyl, $C_{2-6}$ alkynyl, COOH, $-NR^aR^b$, $-C(=O)NR^aR^b$, the heteroaryl, heterocycloalkyl contain 1 to 4 heteroatoms optionally selected from N, O, or S;

$R^5$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, =O, COOH, $-NR^aR^b$, $-C(=O)NR^aR^b$, $C_{3-6}$ cycloalkyl, 3 to 6-membered cycloheterocycloalkyl, $C_{6-10}$ aryl or $C_{6-10}$ heteroaryl, wherein, the alkyl, alkoxy, alkenyl, alkynyl, cycloalkyl, heterocycloalkyl, aryl or heteroaryl is optionally substituted with one or more substituents selected from hydrogen, halogen, CN, $NO_2$, $CF_3$, hydroxyl, $C_{1-6}$ alkyl, $C_{1-6}$ alkoxy, $C_{2-6}$ alkenyl, $C_{2-6}$ alkynyl, COOH, $-NR^aR^b$, $-C(=O)NR^aR^b$, the heteroaryl, heterocycloalkyl contain 1 to 4 heteroatoms optionally selected from N, O, or S;

alternatively, $R^5$, $R^6$ and the atom to which both of them directly connect collectively form cycloalkyl, heteroalicyclic, aryl or heteroaryl, wherein, the cycloalkyl, heteroalicyclic, aryl, heteroaryl are optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl, $-NR^aR^b$, $-C(=O)NR^aR^b$;

$R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkyl, $C_{3-6}$ cycloalkyl, 3 to 6-membered cycloheterocycloalkyl, $C_{6-10}$ aryl or $C_{6-10}$ heteroaryl, the heteroaryl, heterocycloalkyl contain 1 to 4 heteroatoms optionally selected from N, O, or S;

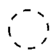

indicates a double bond may be present or not present at any position within the ring.

The present disclosure provides a compound represented by formula (I) or a tautomer, stereoisomer, solvate, metabolite, isotopically-labeled compound, pharmaceutically acceptable salt or co-crystal thereof, wherein,

$L^1$ is selected from a bond, $-C_{1-10}$alkyl-, $-C_{2-6}$ alkenyl-, $-C_{1-10}$ alkyl-$C_{2-6}$ alkenyl-, $-C_{2-6}$ alkynyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-NR^a-$, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $-NR^aR^b$, COOH, $-C(=O)NR^aR^b$, the heteroaryl, heterocycloalkyl contain 1 to 4 heteroatoms optionally selected from N, O, or S; alternatively, one or more alkyl groups of the alkyl may optionally be replaced by one or more groups selected from -C(=O)-, $-S(=O)_2-$ or $-NR^a-$;

W is selected from:

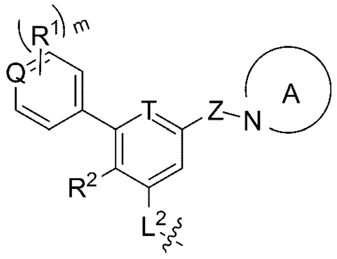,
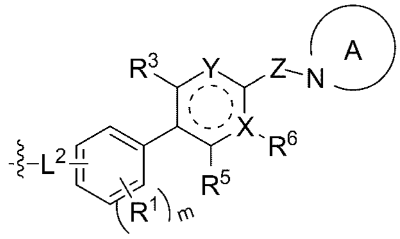,
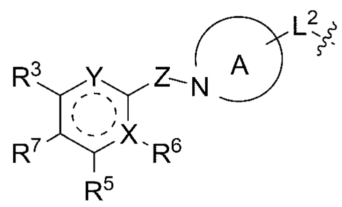,

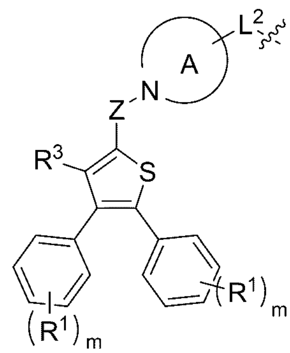
or
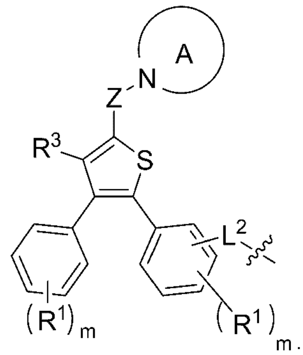;

$L^2$ is selected from a bond, -O-, -C(=O)-, -$NR^a$-, -S- or - $S(=O)_2$-;

ring A is selected from nitrogen-containing $C_{3-10}$ heteroaryl, $C_{3-10}$ heterocycloalkyl or $C_{3-10}$ heterocycloalkenyl, wherein, the heteroaryl, heterocycloalkyl, heterocycloalkenyl are optionally substituted with one or more $R^4$, the heteroaryl, heterocycloalkyl contain 1 to 4 heteroatoms optionally selected from N, O, or S;

$R^4$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, COOH, -$NR^aR^b$, -C(=O)$NR^aR^b$; alternatively, when $R^4$ is selected from $C_{1-6}$ alkyl, any two $R^4$ and the atom to which they connect can collectively form a 5 to 10-membered heteroalicyclic;

$R^1$, $R^6$ are each independently selected at each occurrence from hydrogen, halogen, CN, hydroxyl, $C_{1-6}$ alkyl, $C_{1-6}$ alkoxy, hydroxyl-substituted $C_{1-6}$ alkyl, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl, COOH, -$NR^aR^b$, -C(=O)$NR^aR^b$, - $S(=O)_2R^a$, -$C_{2-6}$ alkenyl-C(=O)$NR^aR^b$;

$R^2$, $R^3$, $R^7$ are each independently selected from hydrogen, $C_{1-6}$ alkyl, $C_{1-6}$ alkoxy, $C_{6-10}$ aryl or $C_{6-10}$ heteroaryl, $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, COOH, -$NR^aR^b$, - $S(=O)_2R^a$, -C(=O)$NR^aR^b$, -$C_{2-6}$ alkenyl-C(=O)$NR^aR^b$, $C_{3-6}$ cycloalkyl, 3 to 6-membered cycloheterocycloalkyl, heterocycloalkenyl, the heteroaryl, heterocycloalkyl contain 1 to 4 heteroatoms optionally selected from N, O, or S;

m is selected from 0, 1, 2, 3, 4 or 5;

Q, T are each independently selected from N or C;

X, Y are each independently selected from C, N, -$NR^c$- or -$CR^d$-;

Z is selected from a bond, -$CH_2$-, -C(=O) or -$S(=O)_2$-;

$R^5$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, =O, COOH, -$NR^aR^b$, -C(=O)$NR^aR^b$, $C_{3-6}$ cycloalkyl, 3 to 6-membered cycloheterocycloalkyl, $C_{6-10}$ aryl or $C_{6-10}$ heteroaryl, wherein, the alkyl, alkoxy, alkenyl, alkynyl, cycloalkyl, heterocycloalkyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, COOH, -$NR^aR^b$, -C(=O)$NR^aR^b$, the heteroaryl, heterocycloalkyl contain 1 to 4 heteroatoms optionally selected from N, O, or S;

alternatively, $R^5$, $R^6$ and the atom to which both of them directly connect collectively form cycloalkyl, heteroalicyclic, aryl or heteroaryl, wherein, the cycloalkyl, heteroalicyclic, aryl, heteroaryl are optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl, -$NR^aR^b$, -C(=O)$NR^aR^b$;

$R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkyl, $C_{3-6}$ cycloalkyl, 3 to 6-membered cycloheterocycloalkyl, $C_{6-10}$ aryl or $C_{6-10}$ heteroaryl, the heteroaryl, heterocycloalkyl contain 1 to 4 heteroatoms optionally selected from N, O, or S;

$R^c$, $R^d$ are each independently selected at each occurrence from a bond, H, $C_{1-6}$ alkyl, $C_{1-6}$ alkoxy, $C_{2-6}$ alkenyl, $C_{2-6}$

alkynyl or $C_{3-6}$ cycloalkyl,

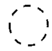 indicates a double bond may be present or not present at any position within the ring.

The present disclosure discloses the compound represented by formula (I) or automer, stereoisomer, solvate, metabolite, isotopically-labeled compound, pharmaceutically acceptable salt or co-crystal thereof, wherein, the compound is represented by formula (II), (III), (IV), (V) or (VI):

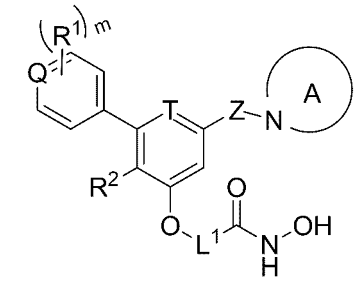

**(II)**

,

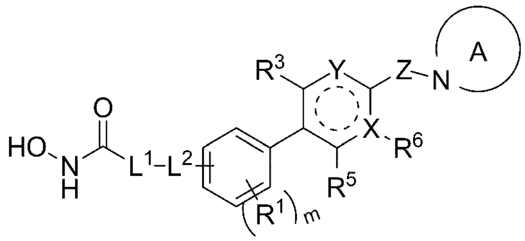

**(III)**

,

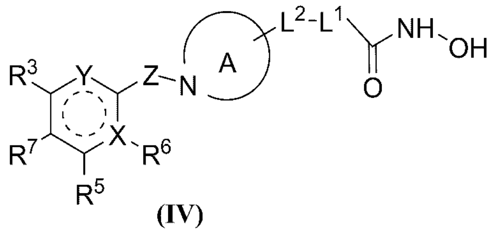

**(IV)**

,

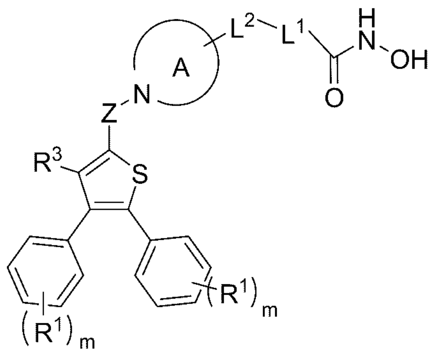

**(V)**

or

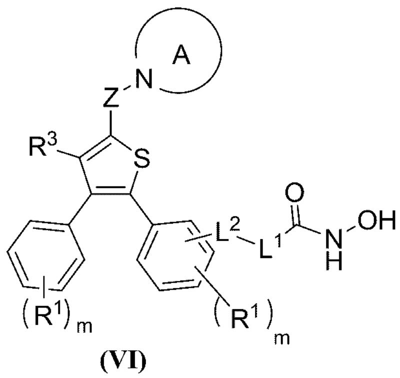

**(VI)**

, wherein the definition of each substituent in formula (II), (III), (IV), (V), or (VI) is consistent with the definition above.

The present disclosure provides the compound represented by formula (II-1):

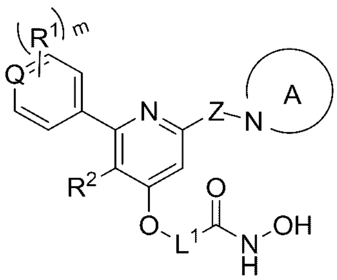

**(II-1)**

wherein,

$L^1$ is selected from -$C_{1-10}$ alkyl-, -$C_{2-6}$ alkenyl-, -$C_{1-10}$ alkyl-$C_{2-6}$ alkenyl-, -$C_{2-6}$ alkynyl-, -$C_{1-10}$ alkyl-($C_{6-10}$ aryl)-$C_{2-6}$ alkenyl-, the alkyl, alkenyl are optionally substituted with one or more substituents selected from hydrogen, halogen, CN, $CF_3$, hydroxyl, $C_{1-6}$ alkyl, $C_{1-6}$ alkoxy.

Preferably, $L^1$ is selected from -$C_{1-10}$ alkyl-, -$C_{1-10}$ alkyl-$C_{2-6}$ alkenyl-, -$C_{1-10}$ alkyl-($C_{6-10}$ aryl)-$C_{2-6}$ alkenyl-.

Preferably, $L^1$ is selected from -$C_{1-10}$ alkyl-, -$C_{1-10}$ alkyl-($C_{6-10}$ aryl)-$C_{2-6}$ alkenyl-.

In some embodiments, $L^1$ is selected from -$C_{1-6}$ alkyl-, -$C_{1-6}$ alkyl-phenylene-$C_{2-6}$ alkenyl-.

In one aspect of the present disclosure, wherein,

ring A is selected from nitrogen-containing $C_{3-10}$ heteroaryl or $C_{3-10}$ heterocycloalkyl, wherein, the heteroaryl, heterocycloalkyl are optionally substituted with one or more $R^4$.

Preferably, ring A is selected from nitrogen-containing $C_{3-10}$ heterocycloalkyl, wherein, the heterocycloalkyl is optionally substituted with one or more $R^4$.

Preferably, ring A is selected from:

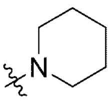 , 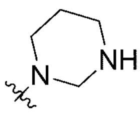 , 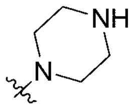 , 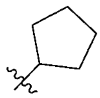 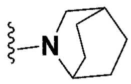 ,

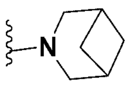 , 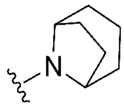 , 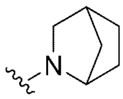

, wherein the

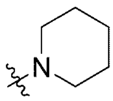 , 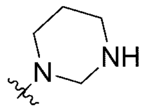 , 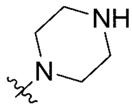 , 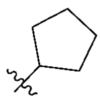 ,

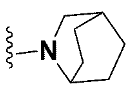 , 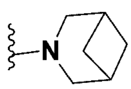 , 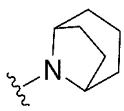

are optionally substituted with $R^4$;

preferably, ring A is selected from:

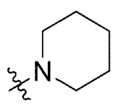 , 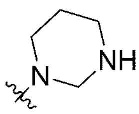 , 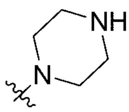 , 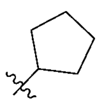 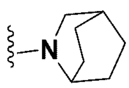 , 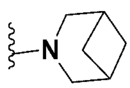 ,

wherein the

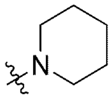 , 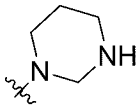 , 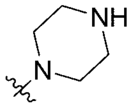 , 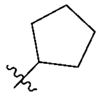 , 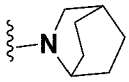 , 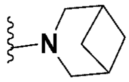

are optionally substituted with $R^4$.

Preferably, ring A is selected from:

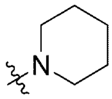 , 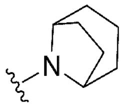 and 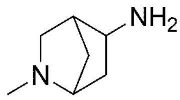

, the

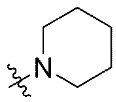 ,

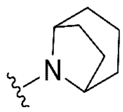 and 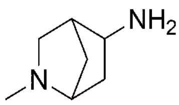

are optionally substituted with $R^4$.

Preferably, ring A is selected from:

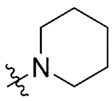 , 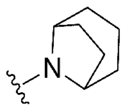 and 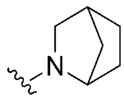

, the

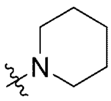 , 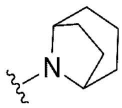

and

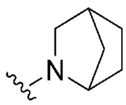

are optionally substituted with $R^4$.

Preferably, ring A is selected from:

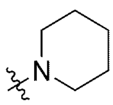 , 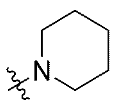

is optionally substituted with $R^4$.

In one aspect of the present disclosure, wherein,

$R^4$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, $-C(=O)NR^aR^b$.

Preferably, $R^4$ is each independently selected at each occurrence from hydrogen, halogen, CN, hydroxyl, $C_{1-6}$

alkyl, $C_{1-6}$ alkoxy, $-NR^aR^b$.

Preferably, $R^4$ is selected from hydrogen, $-NR^aR^b$, $C_{1-6}$ alkyl.

Preferably, $R^4$ is selected from hydrogen, $-NR^aR^b$.

In one aspect of the present disclosure, wherein,

$R^1$ is selected from hydrogen, halogen, CN, hydroxyl, $C_{1-6}$ alkyl, $C_{1-6}$ alkoxy, hydroxyl-substituted $C_{1-6}$ alkyl, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl.

Preferably, $R^1$ is selected from hydrogen, halogen, CN, $C_{1-6}$ alkyl.

Preferably, $R^1$ is selected from hydrogen, halogen, CN.

$R^2$ is selected from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, - $S(=O)_2R^a$, $-O-C_{1-6}$ alkyl-OH, the heteroaryl, heterocycloalkyl contain 1 to 4 heteroatoms optionally selected from N, O, or S;

$R^2$ is selected from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, - $S(=O)_2R^a$, the heteroaryl, heterocycloalkyl contain 1 to 4 heteroatoms optionally selected from N, O, or S.

In some embodiments, $R^2$ is selected from hydrogen, $C_{6-10}$ aryl or $C_{6-10}$ heteroaryl, wherein, the aryl or heteroaryl is optionally substituted with one or more substituents selected from hydrogen, halogen, hydroxyl, $C_{1-6}$ alkyl, $C_{1-6}$ alkoxy, $-NR^aR^b$, $-S(=O)_2R^a$, $-O-C_{1-6}$ alkyl-OH, the heteroaryl, heterocycloalkyl contain 1 to 4 heteroatoms optionally selected from N, O, or S;

preferably, $R^2$ is selected from hydrogen, $C_{6-10}$ aryl or $C_{6-10}$ heteroaryl, wherein, the aryl or heteroaryl is optionally substituted with one or more substituents selected from hydrogen, halogen, hydroxyl, $C_{1-6}$ alkyl, $C_{1-6}$ alkoxy, $-NR^aR^b$, $-S(=O)_2R^a$, the heteroaryl, heterocycloalkyl contain 1 to 4 heteroatoms optionally selected from N, O, or S.

In some embodiments, $R^2$ is selected from hydrogen,

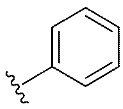 , 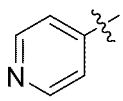 , 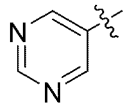 ,

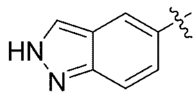 ,

wherein, the

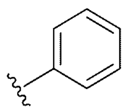 , 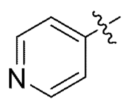 , 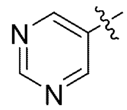 , 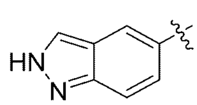

are optionally substituted with one or more substituents selected from hydrogen, halogen, hydroxyl, $C_{1-6}$ alkyl, $C_{1-6}$ alkoxy, $-NR^aR^b$, $-S(=O)_2-R^a$, $-O-C_{1-6}$ alkyl-OH;

preferably, $R^2$ is selected from hydrogen,

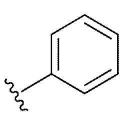 , 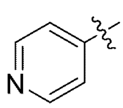 , 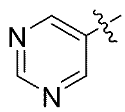

, wherein, the

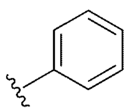 ,

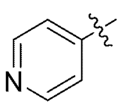 , 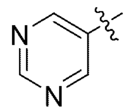

are optionally substituted with one or more substituents selected from hydrogen, halogen, hydroxyl, $C_{1-6}$ alkyl, $C_{1-6}$ alkoxy, -$NR^aR^b$, - $S(=O)_2$-$R^a$.

Preferably, $R^2$ is selected from

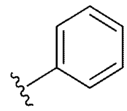

, wherein, the

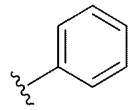

is optionally substituted with one or more substituents selected from hydrogen, hydroxyl, $C_{1-6}$ alkoxy.

In one aspect of the present disclosure, wherein,

m is selected from 0, 1, 2 or 3; preferably, m is selected from 1 and 2; more preferably, m is 2.

Q is each independently selected from N or C; preferably, Q is selected from C.

In one aspect of the present disclosure, wherein,

$R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkyl, $C_{3-6}$ cycloalkyl.

Preferably, $R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl.

Preferably, $R^a$, $R^b$ are each independently selected at each occurrence from hydrogen and methyl.

Preferably, $R^a$, $R^b$ are each independently selected from hydrogen.

The present disclosure provides the compound represented by formula (III-1):

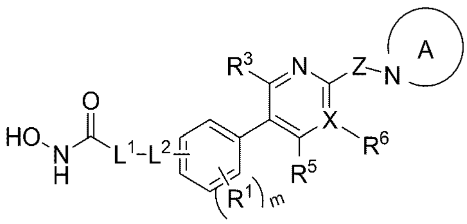

**(III-1)**

wherein,

X is selected from C and N;

$L^1$ is selected from a bond, -$C_{1-10}$ alkyl-, -$C_{2-6}$ alkenyl-, -$C_{1-10}$ alkyl-$C_{2-6}$ alkenyl-, -$C_{2-6}$ alkynyl-, -$C_{1-10}$ alkyl-($C_{6-10}$ aryl or heteroaryl)-$C_{2-6}$ alkenyl-, -$C_{1-10}$ alkyl-($C_{6-10}$ aryl)-, -$NR^a$-, the alkyl, alkoxy, alkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, -$NR^aR^b$, COOH;

alternatively, one or more carbon atoms in the alkyl can optionally be replaced by one or more groups selected from -NH-.

Preferably, $L^1$ is selected from a bond, -$C_{1-10}$ alkyl-, -$C_{2-6}$ alkenyl-, -$C_{1-10}$ alkyl-$C_{2-6}$ alkenyl-, -$C_{1-10}$ alkyl-($C_{6-10}$ aryl or heteroaryl)-$C_{2-6}$ alkenyl-, -$C_{1-10}$ alkyl-($C_{6-10}$ aryl)-, the alkyl, alkoxy, alkenyl, aryl or heteroaryl is optionally substituted with one or more substituents selected from hydrogen, halogen, CN, $CF_3$, hydroxyl, $C_{1-6}$ alkoxy, $C_{2-6}$ alkenyl, -$NR^aR^b$, -$NR^aR^b$;

alternatively, one or more carbon atoms in the alkyl can optionally be replaced by one or more groups selected from -NH-.

Preferably, $L^1$ is selected from $-C_{1-10}$ alkyl-, $-C_{1-10}$ alkyl-($C_{6-10}$ aryl or heteroaryl)-$C_{2-6}$ alkenyl-, $-C_{1-10}$ alkyl-($C_{6-10}$ aryl)-, the alkyl, aryl or heteroaryl is optionally substituted with one or more substituents selected from hydrogen, halogen, CN, $CF_3$, hydroxyl, $C_{1-6}$ alkyl, $C_{1-6}$ alkoxy;
preferably, $L^1$ is $-C_{1-6}$ alkyl-.

In one aspect of the present disclosure, wherein,

$L^2$ is selected from a bond, -O-, -C(=O)-, $-NR^a-$, $-NR^a-C(O)-$ or - $S(=O)_2-$;
in some embodiments, $L^2$ is selected from a bond, -O-, -C(=O)-, $-NR^a-$, or - $S(=O)_2-$.

Preferably, $L^2$ is selected from a bond, -O-, $-NR^a-$.
Preferably, $L^2$ is selected from -O-, $-NR^a-$.
In one aspect of the present disclosure, wherein,
ring A is selected from nitrogen-containing $C_{3-10}$ heteroaryl or $C_{3-10}$ heterocycloalkyl, wherein, the heteroaryl, heterocycloalkyl are optionally substituted with one or more $R^4$.
Preferably, ring A is selected from nitrogen-containing $C_{3-10}$ heterocycloalkyl, $C_{3-10}$ heterocycloalkyl is optionally substituted with one or more $R^4$.
In some embodiments, ring A is selected from:

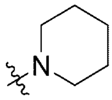, 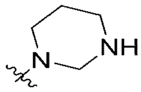, 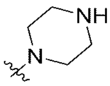, 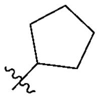 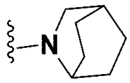,

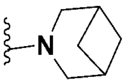, 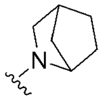,

wherein the

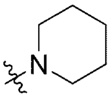, 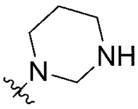, 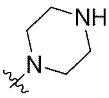, 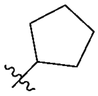, 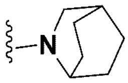, 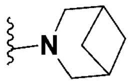,

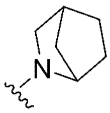

are optionally substituted with $R^4$.
Preferably, ring A is selected from

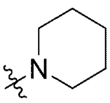, 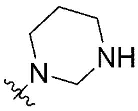, 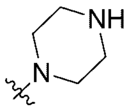, 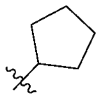 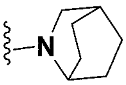, 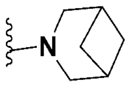,

wherein the

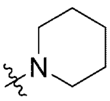, 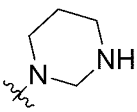, 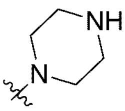, 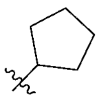, 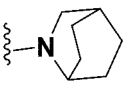, 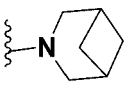

are optionally substituted with $R^4$.
In some embodiments, ring A is selected from:

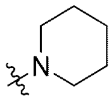 and 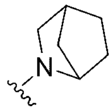

, the

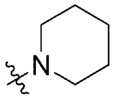 and 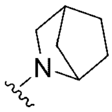

are optionally substituted with $R^4$.

Preferably, ring A is selected from:

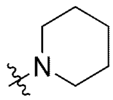 , 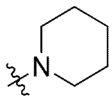

is optionally substituted with $R^4$.

In one aspect of the present disclosure, wherein,

$R^4$ is each independently selected at each occurrence from hydrogen, halogen, CN, hydroxyl, $C_{1-6}$ alkyl, $C_{1-6}$ alkoxy, $C_{2-6}$ alkenyl, $-NR^aR^b$.

Preferably, $R^4$ is selected from hydrogen, $C_{1-6}$ alkyl, $-NR^aR^b$.

Preferably, $R^4$ is selected from hydrogen, $-NR^aR^b$.

In one aspect of the present disclosure, wherein,

$R^1$, $R^6$ are each independently selected at each occurrence from hydrogen, halogen, CN, hydroxyl, $C_{1-6}$ alkyl, $C_{1-6}$ alkoxy, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl, COOH, $-NR^aR^b$, $-C(=O)NR^aR^b$.

Preferably, $R^1$, $R^6$ are each independently selected at each occurrence from hydrogen, CN, hydroxyl, $C_{1-6}$ alkoxy.

Preferably, $R^1$, $R^6$ are each independently selected at each occurrence from hydrogen, CN, hydroxyl.

Preferably, $R^1$ is each independently selected at each occurrence from hydrogen, $C_{1-6}$ alkoxy and hydroxyl; $R^6$ is each independently selected at each occurrence from hydrogen and CN.

$R^3$ is selected from hydrogen, $C_{1-6}$ alkyl, $C_{1-6}$ alkoxy, $C_{6-10}$ aryl or $C_{6-10}$ heteroaryl, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

Preferably, $R^3$ is selected from hydrogen, $C_{1-6}$ alkyl, $C_{6-10}$ aryl or $C_{6-10}$ heteroaryl, wherein, the aryl or heteroaryl is optionally substituted with one or more substituents selected from hydrogen, halogen, CN, the heteroaryl, heterocycloalkyl contain 1 to 4 heteroatoms optionally selected from N, O, or S.

Preferably, $R^3$ is selected from hydrogen, $C_{6-10}$ aryl or $C_{6-10}$ heteroaryl, wherein, the aryl or heteroaryl is optionally substituted with one or more substituents selected from hydrogen, halogen, CN, the heteroaryl, heterocycloalkyl contain 1 to 4 heteroatoms optionally selected from N, O, or S.

Preferably, $R^3$ is selected from hydrogen, methyl,

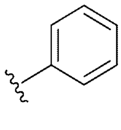 , 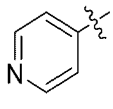 , 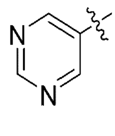

, the

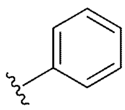 ,

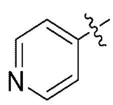, 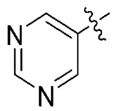

are optionally substituted with one or more substituents selected from hydrogen, halogen, CN.

Preferably, $R^3$ is selected from hydrogen,

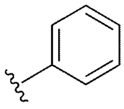, 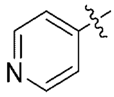, 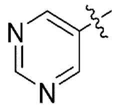,

the

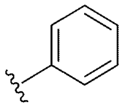, 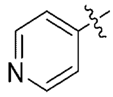,

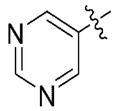

are optionally substituted with one or more substituents selected from hydrogen, halogen, CN.

Preferably, $R^3$ is selected from hydrogen,

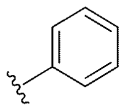,

the

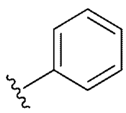

is optionally substituted with one or more substituents selected from hydrogen, halogen, CN.

In one aspect of the present disclosure, wherein,

$R^5$ is each independently selected at each occurrence from hydrogen, halogen, CN, hydroxyl, $C_{1-6}$ alkyl, $C_{1-6}$ alkoxy, $C_{2-6}$ alkenyl, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

Alternatively, $R^5$, $R^6$ and the atom to which both of them directly connect collectively form cycloalkyl, heteroalicyclic, aryl or heteroaryl, wherein, the cycloalkyl, heteroalicyclic, aryl, heteroaryl are optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl.

Preferably, $R^5$ is each independently selected from hydroxyl, $C_{1-6}$ alkoxy,

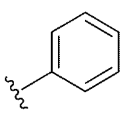, 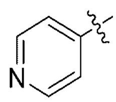

, wherein, the alkyl, alkoxy,

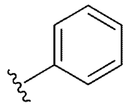 , 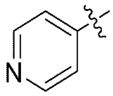

are optionally substituted with one or more substituents selected from hydrogen, halogen, CN, hydroxyl, $C_{1-6}$ alkyl, $C_{1-6}$ alkoxy.

Alternatively, $R^5$, $R^6$ and the atom to which both of them directly connect collectively form

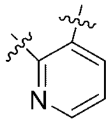 ,

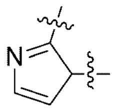 , 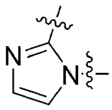

, wherein, the

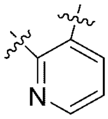 , 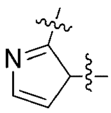 , 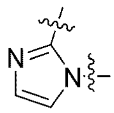

are optionally substituted with one or more of the following groups: hydrogen, halogen, CN, hydroxyl, $C_{1-6}$ alkyl, $C_{1-6}$ alkoxy.

Preferably, $R^5$ is selected from

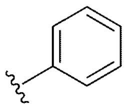 ,

wherein, the

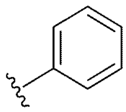

is optionally substituted with one or more substituents selected from hydrogen, halogen, CN.

Alternatively, $R^5$, $R^6$ and the atom to which both of them directly connect collectively form

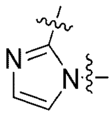 .

In one aspect of the present disclosure, wherein,

m is selected from 0, 1, 2 or 3; preferably, m is selected from 1 and 2.

Z is selected from a bond, $-CH_2-$ or -C(=O); preferably, Z is selected from a bond.

In one aspect of the present disclosure, wherein,

$R^a$, $R^b$ are each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, COOH, wherein, the alkyl, alkoxy, alkenyl, alkynyl are optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, COOH.

Preferably, $R^a$, $R^b$ are each independently selected at each occurrence from hydrogen, $C_{1-6}$ alkyl, preferably, $R^a$, $R^b$ are each independently selected at each occurrence from hydrogen and methyl.

In some embodiments, the present disclosure provides the compound represented by formula (III-2):

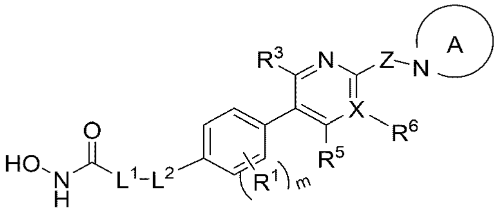

(III-2)

wherein, the definition of each substituent is consistent with the definition of formula (III-1).

The present disclosure provides the compound represented by formula (IV-1a):

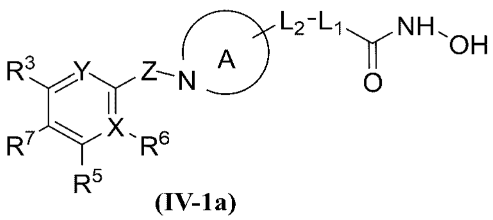

(IV-1a)

wherein,

$L^1$ is selected from a bond, $-C_{1-10}$ alkyl-, $-C_{2-6}$ alkenyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-NR^a$-, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, $-C_{1-10}$ alkyl-NH-6-10-membered heteroaryl-, $-C_{1-10}$ alkyl-6-10-membered heteroaryl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$ cycloalkenyl-, $-C_{1-10}$ alkyl-$C_{6-10}$ aryl-$C_{3-6}$ cycloalkyl-, $-C_{1-10}$ alkyl-O-$C_{6-10}$ aryl-, $-C_{1-10}$ alkyl-6-10-membered heteroaryl-$C_{1-10}$ alkyl-, $-C_{1-10}$ alkyl-6-10-membered aryl-O-$C_{1-10}$ alkyl-, $-C_{1-10}$ alkyl-6-10-membered heteroaryl-O-$C_{1-10}$ alkyl-, $-C_{1-10}$ alkyl-6-10-membered aryl-S-$C_{1-10}$ alkyl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S;

preferably, $L^1$ is selected from a bond, $-C_{1-10}$ alkyl-, $-C_{2-6}$ alkenyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-NR^a$-, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, $-C_{1-10}$ alkyl-NH-6-10-membered heteroaryl-, $-C_{1-10}$ alkyl-6-10-membered heteroaryl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$ cycloalkenyl-, $-C_{1-10}$ alkyl-$C_{6-10}$ aryl-$C_{3-6}$ cycloalkyl-, $-C_{1-10}$ alkyl-O-$C_{6-10}$ aryl-, $-C_{1-10}$ alkyl-6-10-membered heteroaryl-O-$C_{1-10}$ alkyl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S;

alternatively, one or more alkyl groups of the alkyl may optionally be replaced by one or more groups selected from -C(=O)-, $-S(=O)_2$- or $-NR^a$-.

In some embodiments, in the formula (IV-1a) provided in the present application, $L^1$ is selected from a bond, $-C_{1-10}$ alkyl-, $-C_{2-6}$ alkenyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-NR^a$-, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S;

alternatively, one or more alkyl groups of the alkyl may optionally be replaced by one or more groups selected from -C(=O)-, $-S(=O)_2$- or $-NR^a$-.

In some embodiments, in the formula (IV-1a) provided in the present application, $L^1$ is selected from $-C_{1-10}$ alkyl-,

$-C_{2-6}$ alkenyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, $-C_{1-10}$ alkyl-NH-6-10-membered heteroaryl-, $-C_{1-10}$ alkyl-6-10-membered heteroaryl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$ cycloalkenyl-, $-C_{1-10}$ alkyl-$C_{6-10}$ aryl-$C_{3-6}$ cycloalkyl-, $-C_{1-10}$ alkyl-O-$C_{6-10}$ aryl-, $-C_{1-10}$ alkyl-6-10-membered heteroaryl-$C_{1-10}$ alkyl-, $-C_{1-10}$ alkyl-6-10-membered aryl-O-$C_{1-10}$ alkyl-, $-C_{1-10}$ alkyl-6-10-membered heteroaryl-O-$C_{1-10}$ alkyl-, $-C_{1-10}$ alkyl-6-10-membered aryl-S-$C_{1-10}$ alkyl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

In some embodiments, in the formula (IV-1a) provided in the present application, $L^1$ is selected from $-C_{1-10}$ alkyl-, $-C_{2-6}$ alkenyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, $-C_{6-10}$ aryl-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

Preferably, $L^1$ is selected from $-C_{1-10}$ alkyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-C_{1-10}$ alkyl-($C_{6-10}$ heterocycloalkyl)-($C_{6-10}$ aryl)-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

In some embodiments, in the formula (IV-1a) provided in the present application, $L^1$ is selected from $-C_{1-10}$ alkyl-, $-C_{6-10}$ heteroaryl-, $-C_{1-10}$ alkyl-(heteroaryl or heterocycloalkyl or heterocycloalkenyl)-$C_{2-6}$ alkenyl- $-C_{1-10}$ alkyl-($C_{6-10}$ aryl)-vinyl-, -(6-10-membered heteroaryl)-$C_{2-6}$ alkenyl-, $-C_{1-10}$ alkyl-($C_{6-10}$ aryl)-$C_{1-10}$ alkyl-, $-C_{1-10}$ alkyl-($C_{6-10}$ aryl)-$C_{2-6}$ alkynyl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$ cycloalkyl-, $-C_{1-10}$ alkyl-O-$C_{6-10}$ aryl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

Preferably, $L^1$ is selected from $-C_{1-10}$ alkyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-C_{1-10}$ alkyl-($C_{6-10}$ heterocycloalkyl)-($C_{6-10}$ aryl)-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

Preferably, $L^1$ is selected from $-C_{1-10}$ alkyl-($C_{6-10}$ aryl or heteroaryl)-$C_{2-6}$ alkenyl-, the alkyl, aryl or heteroaryl is optionally substituted with one or more substituents selected from hydrogen, halogen, CN, $CF_3$, hydroxyl, $C_{1-6}$ alkyl, $C_{1-6}$ alkoxy.

Preferably, $L^1$ is selected from $-CH_2-$, $-CH_2-(C{=}C)-$, $-(CH_2)_4-$, $-(CH_2)_6-$, -(C=O)-phenyl-(C=C)-, $-CH_2$-phenyl-, $-(CH_2)_3$-phenyl-, $-CH_2$-phenyl-$(CH_2)_2-$, $-(CH_2)_2$-phenyl-$CH_2-$, $-CH_2$-phenyl-(C=C)-, $-(CH_2)_2$-phenyl-(C=C)-, $-CH_2$-phenyl-(C≡C)-, $-CH_2$-phenyl-(C=C)-$CH_2-$, -phenyl-(C=C)-, pyrimidinyl,

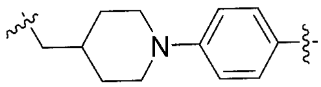

,

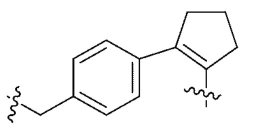

,

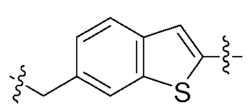

,

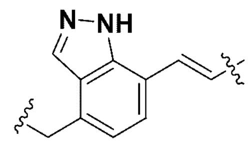

,

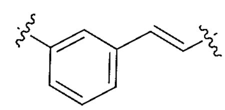

,

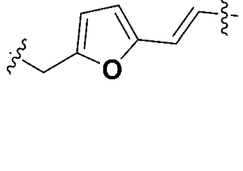

,

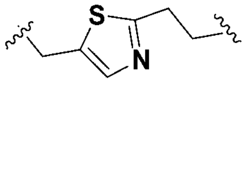

,

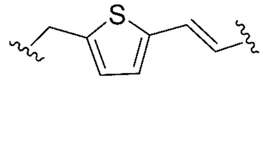

,

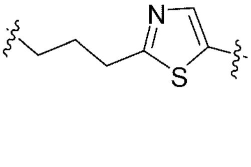

,

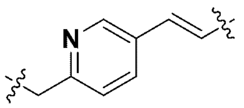 , 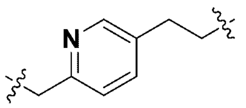 , 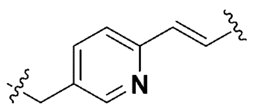 , 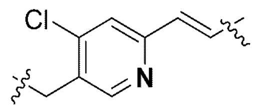 ,

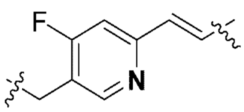 , 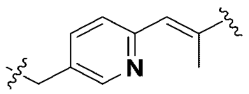 , 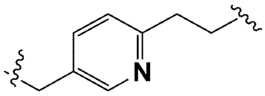 , 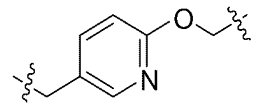 ,

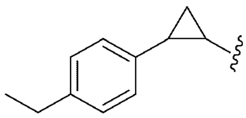 , 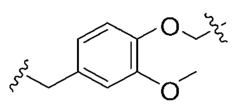 , 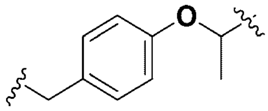 , 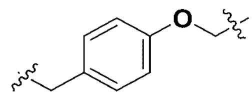 ,

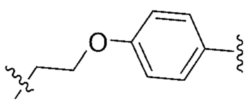 , 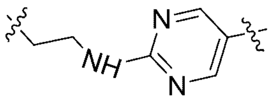 , 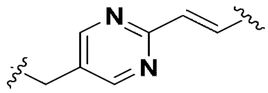 , 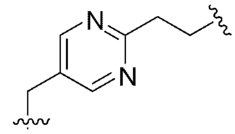 ,

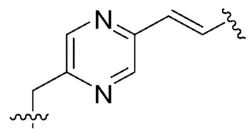 , 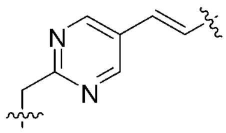 , 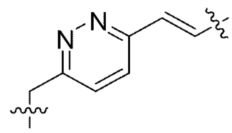 , 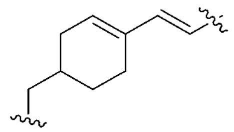 ,

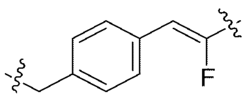 , 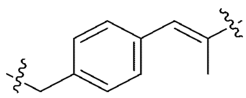 , 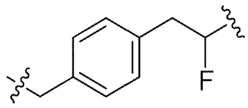 , 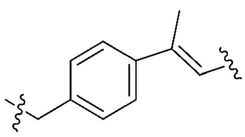 ,

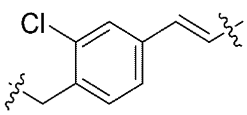 , 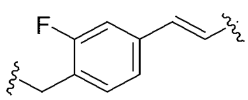 , 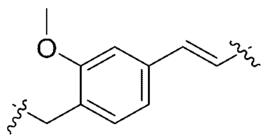 , 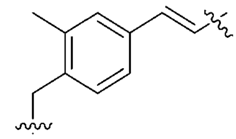 ,

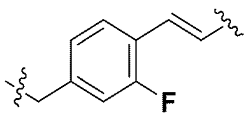 , 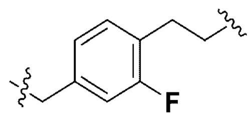 , 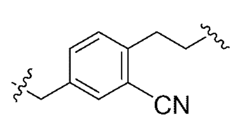 , 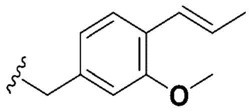 ,

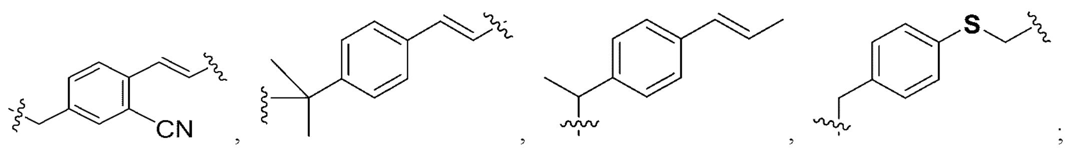 , , , ;

preferably, $L^1$ is selected from

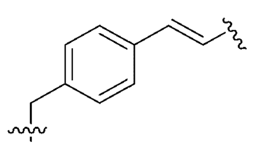 , 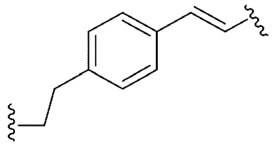 , 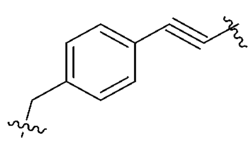 ,

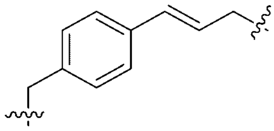, 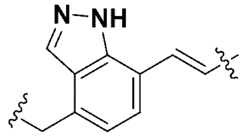, 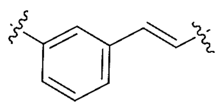, 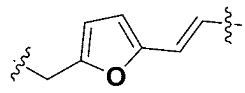,

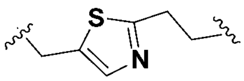, 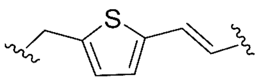, 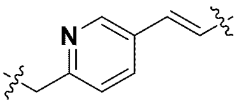, 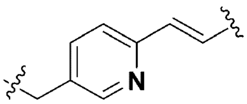,

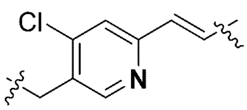, 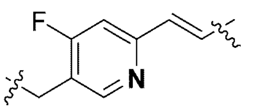, 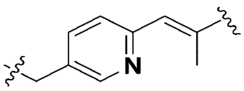, 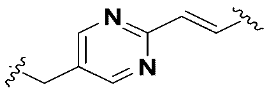,

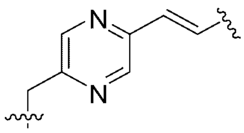, 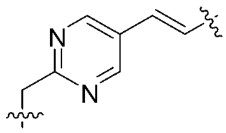, 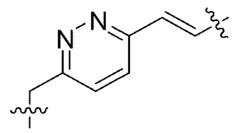, 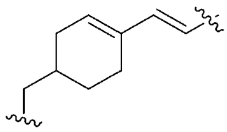,

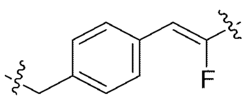, 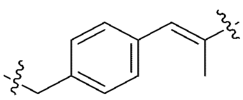, 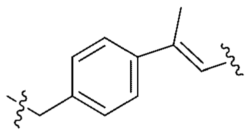, 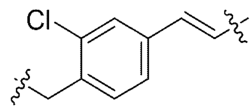,

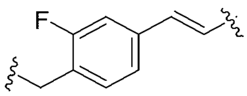, 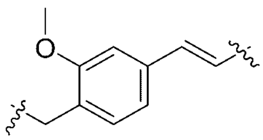, 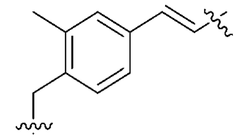, 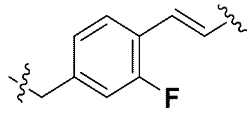,

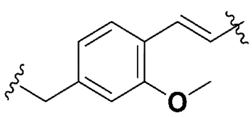, 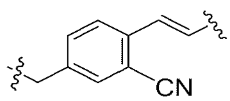, 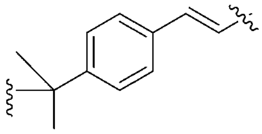, 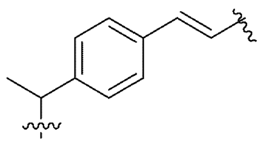

In one aspect of the present disclosure, wherein,

$L^2$ is selected from a bond, -O-, -C(=O)-, -S-, -NR$^a$-, -$CH_2$-NR$^a$-, -NR$^a$-C(=O) and -NR$^a$-S$(=O)_2$-, in some embodiments, $L^2$ is selected from a bond, -O-, -C(=O)-, -S-, -NR$^a$-, -NR$^a$-C(=O) and -NR$^a$-S$(=O)_2$-.

In some embodiments, $L^2$ is selected from a bond, -O-, -C(=O)-, -S- or -NR$^a$-.

In some embodiments, $L^2$ is selected from a bond, -NR$^a$-, -$CH_2$-NR$^a$-, -NR$^a$-C(=O) and -NR$^a$-S$(=O)_2$-.

In some embodiments, $L^2$ is selected from a bond, -NR$^a$-, -NR$^a$-C(=O) and -NR$^a$-S$(=O)_2$-.

Preferably, $L^2$ is selected from a bond, -C(=O)- or -NR$^a$-.

Preferably, $L^2$ is selected from -NR$^a$-.

In one aspect of the present disclosure, wherein,
ring A is selected from nitrogen-containing $C_{3-10}$ heteroaryl or $C_{3-10}$ heterocycloalkyl, wherein, the heteroaryl, heterocycloalkyl are optionally substituted with one or more $R^4$; the heteroaryl, heterocycloalkyl contain 1 to 4 heteroatoms optionally selected from N, O, or S.

Preferably, ring A is selected from $C_{3-10}$ heterocycloalkyl, wherein, the heterocycloalkyl is optionally substituted with one or more $R^4$.

Preferably, ring A is selected from:

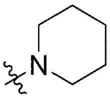, 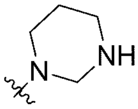, 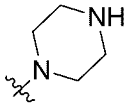, 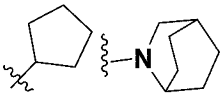, 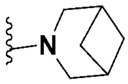,

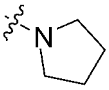, 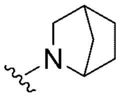, 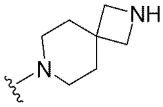, 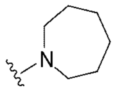,

wherein the

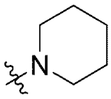, 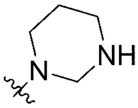, 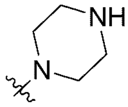,

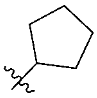, 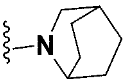, 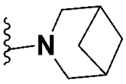, 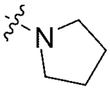, 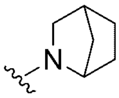, 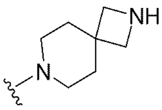, 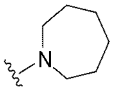

are optionally substituted with $R^4$.

In some embodiments, ring A is selected from:

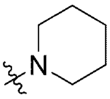, 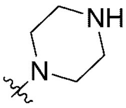, 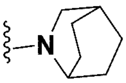, 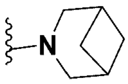, 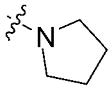,

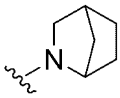, 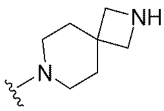, 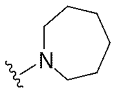

, wherein the

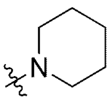, 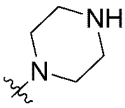, 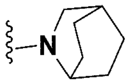, 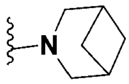,

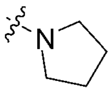, 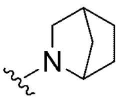, 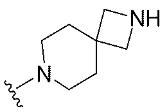, 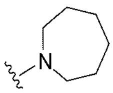

are optionally substituted with $R^4$.

In some embodiments, ring A is selected from

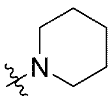, 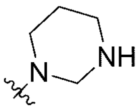, 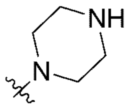, 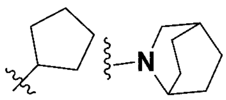,

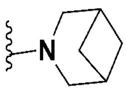

, wherein the

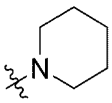, 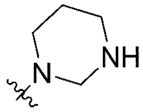, 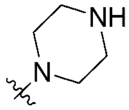, 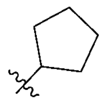, 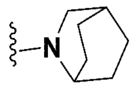, 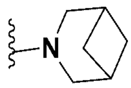

are optionally substituted with $R^4$.

In some embodiments, ring A is selected from:

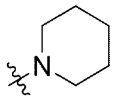, 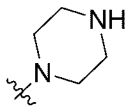, 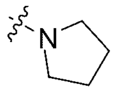, 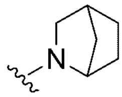,

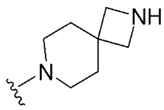, 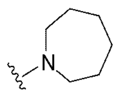

, wherein the

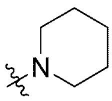, 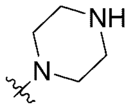, 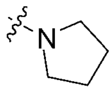, 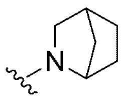, 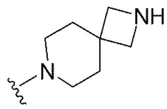,

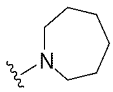

are optionally substituted with $R^4$.

Preferably, ring A is selected from:

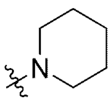, 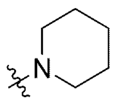

is optionally substituted with $R^4$.

In one aspect of the present disclosure, wherein,

$R^4$ is each independently selected at each occurrence from hydrogen, halogen, CN, hydroxyl, $C_{1-6}$ alkyl, $C_{1-6}$ alkoxy, $C_{2-6}$alkenyl, $-NR^aR^b$.

Preferably, $R^4$ is selected from hydrogen, $C_{1-6}$ alkyl, $-NR^aR^b$.

Preferably, $R^4$ is selected from hydrogen, $C_{1-6}$ alkyl.

In one aspect of the present disclosure, wherein,

$R^6$ is each independently selected at each occurrence from hydrogen, halogen, CN, hydroxyl, $C_{1-6}$ alkyl, $C_{1-6}$ alkyl-CN, halogen-substituted $C_{1-6}$ alkyl, $C_{1-6}$ alkoxy, $C_{3-6}$ cycloalkyl, $C_{2-6}$ alkenyl, $C_{2-6}$ alkynyl, COOH, $-NR^aR^b$, $-C(=O)NR^aR^b$.

Preferably, $R^6$ is each independently selected at each occurrence from hydrogen, halogen, CN, hydroxyl, $C_{1-6}$ alkyl, $C_{1-6}$ alkoxy, $C_{1-6}$ cycloalkyl, $C_{2-6}$alkenyl, $C_{2-6}$ alkynyl, COOH, $-NR^aR^b$, $-C(=O)NR^aR^b$.

Preferably, $R^6$ is each independently selected at each occurrence from hydrogen, halogen, $CH_2$-CN, CN, $C_{1-6}$ alkyl, $C_{1-6}$ alkoxy, halogen-substituted $C_{1-6}$ alkyl.

Preferably, $R^6$ is each independently selected at each occurrence from hydrogen, halogen, CN, $C_{1-6}$ alkyl.

Preferably, $R^6$ is each independently selected at each occurrence from hydrogen, CN.

$R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkoxy, halogen-substituted $C_{1-6}$ alkyl, hydroxyl-substituted $C_{1-6}$ alkyl, hydroxyl-substituted $C_{1-6}$

alkoxy, hydroxyl-substituted $C_{1-6}$ alkoxy-$C_{3-6}$ cycloalkyl, COOH, $-NR^aR^b$, $-S(=O)_2R^a$, $-C(=O)NR^aR^b$, 3 to 6-membered heterocycloalkyl, hydroxyl-substituted $C_{1-6}$ alkoxy-$C_{3-6}$ cycloalkyl, heterocycloalkenyl, the heteroaryl, heterocycloalkyl contain 1 to 4 heteroatoms optionally selected from N, O, or S.

In some embodiments, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkoxy, hydroxyl-substituted $C_{1-6}$ alkyl, hydroxyl-substituted $C_{1-6}$ alkoxy, COOH, $-NR^aR^b$, $-S(=O)_2R^a$, $-C(=O)NR^aR^b$, heterocycloalkenyl, the heteroaryl, heterocycloalkyl contain 1 to 4 heteroatoms optionally selected from N, O, or S.

In some embodiments, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, halogen-substituted $C_{1-6}$ alkoxy, halogen-substituted $C_{1-6}$ alkyl, hydroxyl-substituted $C_{1-6}$ alkoxy, hydroxyl-substituted $C_{1-6}$ alkoxy-$C_{3-6}$ cycloalkyl, COOH, $-NR^aR^b$, $-S(=O)_2R^a$, $-C(=O)NR^aR^b$, 3 to 6-membered heterocycloalkyl, heterocycloalkenyl, the heteroaryl, heterocycloalkyl contain 1 to 4 heteroatoms optionally selected from N, O, or S.

Preferably, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, hydroxyl, $C_{1-6}$ alkyl, $C_{1-6}$ alkoxy, heterocycloalkenyl, the heteroaryl, heterocycloalkyl contain 1 to 4 heteroatoms optionally selected from N, O, or S.

Preferably, $R^3$, $R^7$ are selected from hydrogen,

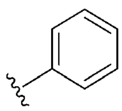 , 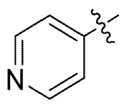 , 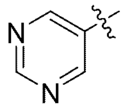 , 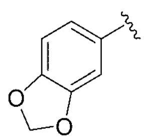 ,

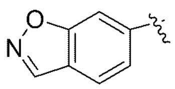 , 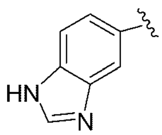 , 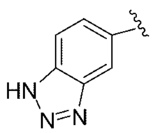 , 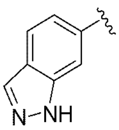 , the 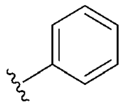 , 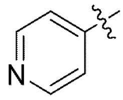 , 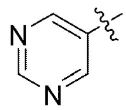 ,

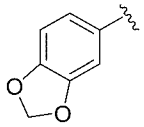 , 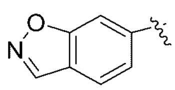 , 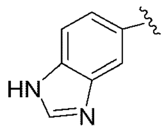 , 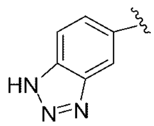 , 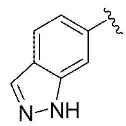

are optionally substituted with one or more substituents selected from hydrogen, halogen, CN, heterocycloalkenyl, hydroxyl, $CF_3$, $C_{1-6}$ alkyl, $C_{1-6}$ alkoxy, $C_{3-6}$ cycloalkyl, halogen-substituted $C_{1-6}$ alkoxy, 3 to 6-membered heterocycloalkyl, hydroxyl-substituted $C_{1-6}$ alkoxy-$C_{3-6}$ cycloalkyl, hydroxyl-substituted $C_{1-6}$ alkyl, hydroxyl-substituted $C_{1-6}$ alkoxy, $-NH_2$, $-N(C_{1-6}\ alkyl)_2$, $-NH(C_{1-6}\ alkyl)$.

Preferably, $R^3$, $R^7$ are selected from hydrogen,

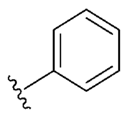 , 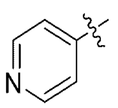 , 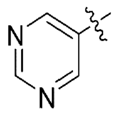 , 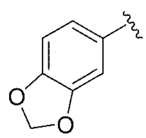 ,

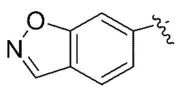 , 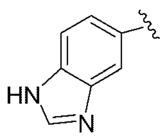 , 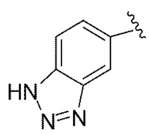 , 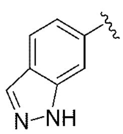

, the

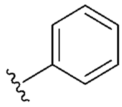, 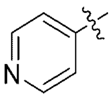, 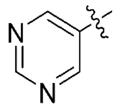,

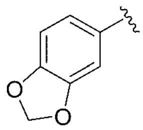, 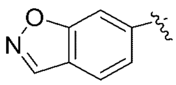, 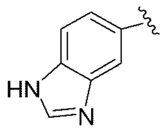, 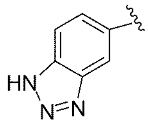, 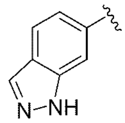

are optionally substituted with one or more substituents selected from hydrogen, halogen, CN, heterocycloalkenyl, hydroxyl, $CF_3$, $C_{1-6}$ alkyl, $C_{1-6}$ alkoxy, $C_{3-6}$ cycloalkyl, halogen-substituted $C_{1-6}$ alkoxy, hydroxyl-substituted $C_{1-6}$ alkyl, hydroxyl-substituted $C_{1-6}$ alkoxy.

In some embodiments, $R^3$, $R^7$ are selected from hydrogen,

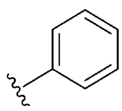, 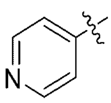, 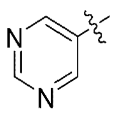,

the

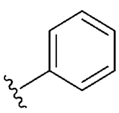, 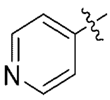, 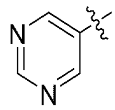

are optionally substituted with one or more substituents selected from hydrogen, halogen, heterocycloalkenyl, hydroxyl.

In some embodiments, $R^3$, $R^7$ are selected from hydrogen,

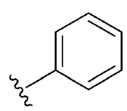, 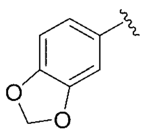, 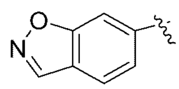,

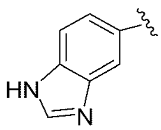, 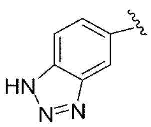, 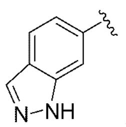, the 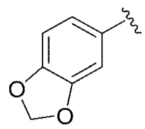, 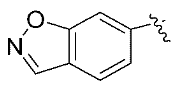, 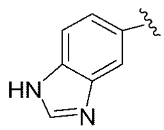, 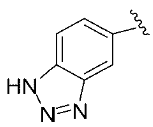,

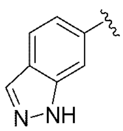

are optionally substituted with one or more substituents selected from hydrogen, halogen, CN, hydroxyl, $CF_3$, $C_{1-6}$ alkyl, $C_{1-6}$ alkoxy, $C_{3-6}$ cycloalkyl, halogen-substituted $C_{1-6}$ alkoxy, 3 to 6-membered heterocycloalkyl, hydroxyl-substituted $C_{1-6}$ alkoxy-$C_{3-6}$ cycloalkyl, hydroxyl-substituted $C_{1-6}$ alkyl, hydroxyl-substituted $C_{1-6}$ alkoxy, $-NH_2$, $-N(C_{1-6}\ alkyl)_2$, $-NH(C_{1-6}\ alkyl)$.

Preferably, $R^3$, $R^7$ are selected from hydrogen,

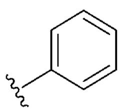,

the

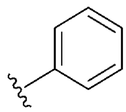

is optionally substituted with one or more substituents selected from hydrogen, halogen, hydroxyl, CN, $CF_3$, $C_{1-6}$ alkyl, $C_{1-6}$ alkoxy, $C_{3-6}$ cycloalkyl, halogen-substituted $C_{1-6}$ alkoxy, 3 to 6-membered heterocycloalkyl, hydroxyl-substituted $C_{1-6}$ alkoxy-$C_{3-6}$ cycloalkyl, hydroxyl-substituted $C_{1-6}$ alkyl, hydroxyl-substituted $C_{1-6}$ alkoxy, $-NH_2$, $-N(C_{1-6}$ alkyl$)_2$, $-NH(C_{1-6}$ alkyl). Substituted by a substituent of.

Preferably, $R^3$, $R^7$ are selected from hydrogen,

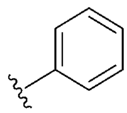
,

the

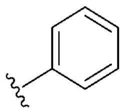

is optionally substituted with one or more substituents selected from hydrogen, halogen, heterocycloalkenyl, hydroxyl.

In one aspect of the present disclosure, wherein,

$R^5$ is each independently selected at each occurrence from hydrogen, halogen, CN, hydroxyl, $C_{1-6}$ alkyl, $C_{1-6}$ alkoxy, =O, $C_{2-6}$ alkenyl, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, $NO_2$, $CF_3$, $CHF_2$, hydroxyl, $C_{1-6}$ alkyl, $C_{1-6}$ alkoxy, -C(=O)-$C_{1-6}$ alkoxy, $C_{2-6}$ alkenyl, -C(=O)-$NH_2$, the heteroaryl, heterocycloalkyl contain 1 to 4 heteroatoms optionally selected from N, O, or S.

In some embodiments, $R^5$ is each independently selected at each occurrence from hydrogen, halogen, CN, hydroxyl, $C_{1-6}$ alkyl, $C_{1-6}$ alkoxy, =O, $C_{2-6}$ alkenyl, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, $NO_2$, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O, or S.

In some embodiments, $R^5$ is each independently selected at each occurrence from hydrogen, halogen, CN, hydroxyl, $C_{1-6}$ alkyl, $C_{1-6}$ alkoxy, =O, $C_{2-6}$ alkenyl, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, $NO_2$, $CF_3$, hydroxyl, $C_{1-6}$ alkyl, $C_{1-6}$ alkoxy, -C(=O)-$C_{1-6}$ alkoxy, $C_{2-6}$ alkenyl, the heteroaryl, heterocycloalkyl contain 1 to 4 heteroatoms optionally selected from N, O, or S.

In some embodiments, $R^5$ is each independently selected at each occurrence from hydrogen, CN, $C_{1-6}$ alkoxy, =O, $C_{6-10}$ aryl or $C_{6-10}$ heteroaryl, wherein, the alkoxy, aryl or heteroaryl is optionally substituted with one or more substituents selected from hydrogen, halogen, CN, $NO_2$, $CF_3$, $CHF_2$, hydroxyl, $C_{1-6}$ alkyl, -C(=O)-$C_{1-6}$ alkoxy, -C(=O)-$NH_2$, the heteroaryl, heterocycloalkyl contain 1 to 4 heteroatoms optionally selected from N, O, or S;

alternatively, $R^5$, $R^6$ and the atom to which both of them directly connect collectively form cycloalkyl, heteroalicyclic, aryl or heteroaryl, wherein, the cycloalkyl, heteroalicyclic, aryl, heteroaryl are optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl.

Preferably, $R^5$ is each independently selected from CN, $C_{1-6}$ alkoxy, =O,

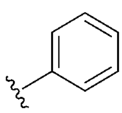
,
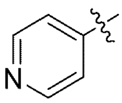
,
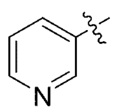
,

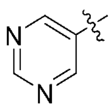 , 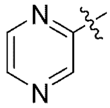 ,

wherein, the alkoxy,

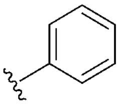 , 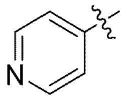 , 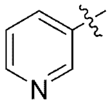 , 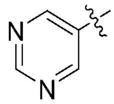 , 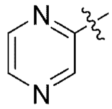

are optionally substituted with one or more substituents selected from hydrogen, halogen, CN, $NO_2$, $CF_3$, $CHF_2$, hydroxyl, $C_{1-6}$alkyl, $-C(O)OCH_3$, $-C(=O)-NH_2$.

Preferably, $R^5$ is each independently selected from

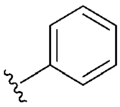 , 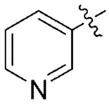 , 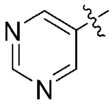 , 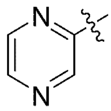

, wherein,

the 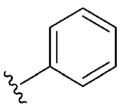 , 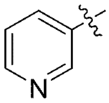 , 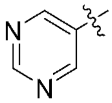 , 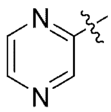

are optionally substituted with one or more substituents selected from hydrogen, halogen, CN, $NO_2$, $CF_3$, $CHF_2$, hydroxyl, $C_{1-6}$ alkyl, $-C(O)OCH_3$, $-C(=O)-NH_2$

Preferably, $R^5$ is each independently selected from CN, $C_{1-6}$ alkoxy, =O,

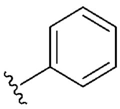 , 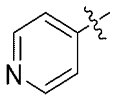 , 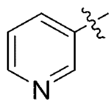 ,

wherein, the alkoxy,

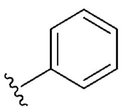 , 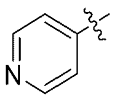 , 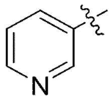

are optionally substituted with one or more substituents selected from hydrogen, halogen, CN, $NO_2$, hydroxyl.

In some embodiments, $R^5$ is each independently selected from $C_{1-6}$ alkoxy, =O,

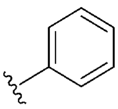 , 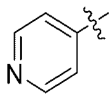 ,

wherein, the alkoxy,

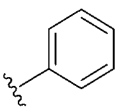 , 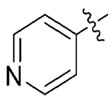

are optionally substituted with one or more substituents selected from hydrogen, halogen, CN, hydroxyl.

Alternatively, $R^5$, $R^6$ and the atom to which both of them directly connect collectively form

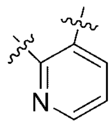

,

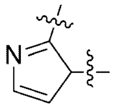

,

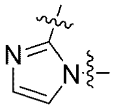

,

wherein, the

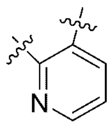

,

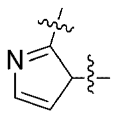

,

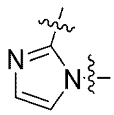

are optionally substituted with one or more of the following groups: hydrogen, halogen, CN, hydroxyl, $C_{1-6}$ alkyl, $C_{1-6}$ alkoxy.

Preferably, $R^5$ is selected from $C_{1-6}$ alkoxy,

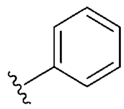

;

wherein, the

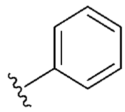

;

is optionally substituted with one or more substituents selected from hydrogen, halogen, CN, $NO_2$.

In some embodiments, $R^5$ is selected from $C_{1-6}$ alkoxy,

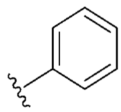

, wherein, the

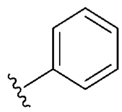

is optionally substituted with one or more substituents selected from hydrogen, halogen, CN.

Alternatively, $R^5$, $R^6$ and the atom to which both of them directly connect collectively form

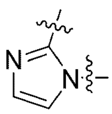

.

In one aspect of the present disclosure, wherein,
X, Y are each independently selected from C, N.

Z is selected from a bond, $-CH_2-$, -C(=O) or $-S(=O)_2-$.

Preferably, Z is selected from a bond, $-CH_2-$ or -C(=O).

Preferably, Z is selected from a bond.

In one aspect of the present disclosure, wherein,
$R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkyl, the heteroaryl, heterocycloalkyl contain 1 to 4 heteroatoms optionally selected from N, O, or S.

Preferably, $R^a$, $R^b$ are each independently selected at each occurrence from hydrogen, $C_{1-6}$ alkyl.

Preferably, $R^a$, $R^b$ are each independently selected at each occurrence from hydrogen, methyl, ethyl, n-propyl, isopropyl.

Preferably, $R^a$, $R^b$ are each independently selected at each occurrence from hydrogen.

In some embodiments, the present disclosure provides the compound represented by formula (IV-2a), formula (IV-3a), formula (IV-4a):

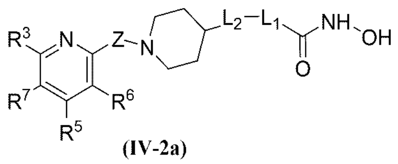

(IV-2a)

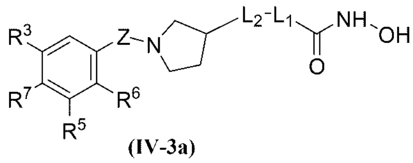

(IV-3a)

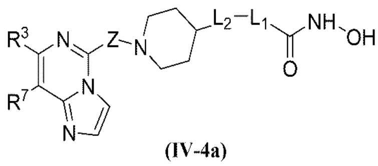

(IV-4a)

wherein, the definition of each substituent is consistent with the definition in formula (IV-1a).

The present disclosure provides the compound represented by formula (V):

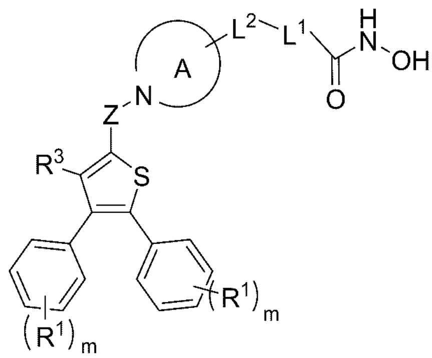

(V)

wherein,
$L^1$ is selected from a bond, $-C_{1-10}$ alkyl-, $-C_{2-6}$ alkenyl-, $-C_{1-10}$ alkyl-($C_{6-10}$ aryl or heteroaryl)-$C_{2-6}$ alkenyl-, $-NR^a-$, the alkyl, alkoxy, alkenyl, alkynyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl.

Preferably, $L^1$ is selected from $-C_{1-10}$ alkyl-($C_{6-10}$ aryl or heteroaryl)-$C_{2-6}$ alkenyl-, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl.

Preferably, $L^1$ is selected from $-C_{1-10}$ alkyl-($C_{6-10}$ aryl)-$C_{2-6}$ alkenyl-, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl.

In one aspect of the present disclosure, wherein,
$L^2$ is selected from a bond or $-NR^a-$.

Preferably, $L^2$ is selected from $-NR^a-$.

In one aspect of the present disclosure, wherein,

ring A is selected from nitrogen-containing $C_{3-10}$ heteroaryl or $C_{3-10}$ heterocycloalkyl, wherein, the heteroaryl, heterocycloalky are optionally substituted with one or more $R^4$.

Preferably, ring A is selected from $C_{3-10}$ heterocycloalkyl, wherein, the heterocycloalkyl is optionally substituted with one or more $R^4$.

Preferably, ring A is selected from:

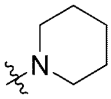, 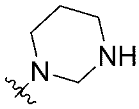, 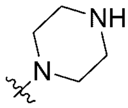, 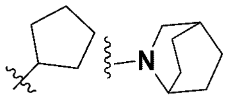, 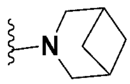,

wherein the

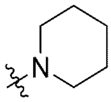, 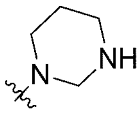, 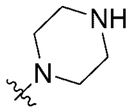, 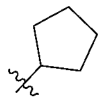, 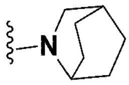, 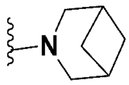

are optionally substituted with one or more $R^4$.

Preferably, ring A is selected from

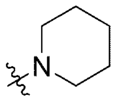, 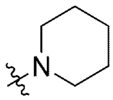

are optionally substituted with one or more $R^4$.

In one aspect of the present disclosure, wherein,

$R^4$ is each independently selected at each occurrence from hydrogen, halogen, CN, hydroxyl, $C_{1-6}$ alkyl, wherein, the alkyl, alkoxy are optionally substituted with one or more substituents selected from hydrogen, halogen, CN, $CF_3$, hydroxyl, $C_{1-6}$ alkyl, $C_{1-6}$ alkoxy.

Preferably, $R^4$ is selected from hydrogen.

In one aspect of the present disclosure, wherein,

$R^1$ is each independently selected at each occurrence from hydrogen, halogen, CN, hydroxyl, $C_{1-6}$ alkyl, $C_{1-6}$ alkoxy, $C_{2-6}$alkenyl, $-NR^aR^b$, $C_{6-10}$ aryl or $C_{6-10}$ heteroaryl.

Preferably, $R^1$ is each independently selected at each occurrence from hydrogen, halogen, CN, hydroxyl, $C_{1-6}$ alkoxy.

$R^3$ is each independently selected at each occurrence from hydrogen, halogen, CN, hydroxyl, $C_{1-6}$ alkyl, $C_{1-6}$ alkoxy, $C_{2-6}$ alkenyl.

Preferably, $R^3$ is each independently selected at each occurrence from hydrogen.

In one aspect of the present disclosure, wherein,

Z is selected from a bond, $-CH_2-$ or $-C(=O)$.

Preferably, Z is selected from $-C(=O)$.

In one aspect of the present disclosure, wherein,

$R^a$, $R^b$ are each independently selected at each occurrence from hydrogen, halogen, CN, hydroxyl, $C_{1-6}$ alkyl, $C_{1-6}$ alkoxy, wherein, the alkyl, alkoxy are optionally substituted with one or more substituents selected from hydrogen, halogen, CN, $CF_3$, hydroxyl.

Preferably, $R^a$, $R^b$ are each independently selected at each occurrence from hydrogen.

The present disclosure provides the compound represented by formula (VI):

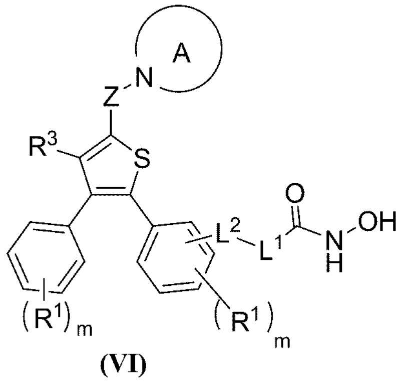

(VI)

wherein,
$L^1$ is selected from a bond, -$C_{1-10}$ alkyl-, -$C_{2-6}$ alkenyl-, -$NR^a$-, the alkyl, alkoxy, alkenyl, alkynyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl.

Preferably, $L^1$ is selected from -$C_{1-10}$ alkyl-.

In one aspect of the present disclosure, wherein,
$L^2$ is selected from a bond, -O- or -$NR^a$-.

Preferably, $L^2$ is selected from -O-.

In one aspect of the present disclosure, wherein,
ring A is selected from nitrogen-containing $C_{3-10}$ heteroaryl or $C_{3-10}$ heterocycloalkyl, wherein, the heteroaryl, heterocycloalkyl are optionally substituted with one or more $R^4$.

Preferably, ring A is selected from $C_{3-10}$ heterocycloalkyl, wherein, the heterocycloalkyl is optionally substituted with one or more $R^4$.

Preferably, ring A is selected from:

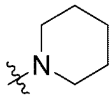, 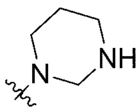, 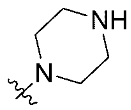, 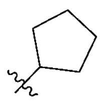 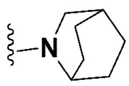,

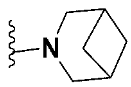, 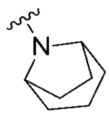

, wherein the

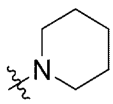, 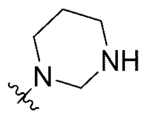, 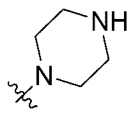, 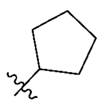, 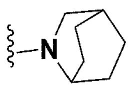,

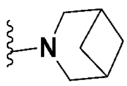, 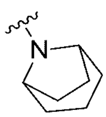

are optionally substituted with $R^4$.

In some embodiments, ring A is selected from:

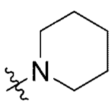, 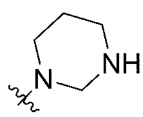, 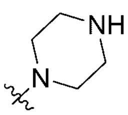, 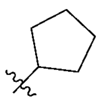 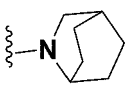,

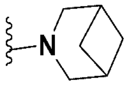,

wherein the

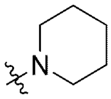, 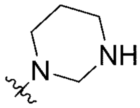, 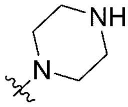, 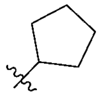, 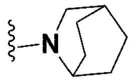, 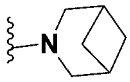

are optionally substituted with $R^4$.

Preferably, ring A is selected from:

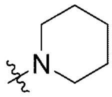 and 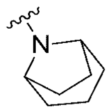,

the

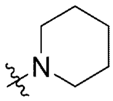 and 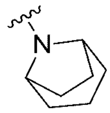

are optionally substituted with $R^4$.

Preferably, ring A is selected from:

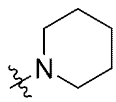, 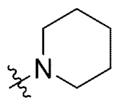

are optionally substituted with $R^4$.

In one aspect of the present disclosure, wherein,

$R^4$ is each independently selected at each occurrence from hydrogen, halogen, CN, hydroxyl, $C_{1-6}$ alkyl, $-NR^aR^b$, wherein, the alkyl, alkoxy are optionally substituted with one or more substituents selected from hydrogen, halogen, CN, $CF_3$, hydroxyl, $C_{1-6}$ alkyl, $C_{1-6}$ alkoxy.

Preferably, $R^4$ is selected from hydrogen, $C_{1-6}$ alkyl, $-NR^aR^b$.

In one aspect of the present disclosure, wherein,

$R^1$ is each independently selected at each occurrence from hydrogen, halogen, CN, hydroxyl, $C_{1-6}$ alkyl, $C_{1-6}$ alkoxy, $C_{2-6}$ alkenyl, $-NR^aR^b$.

Preferably, $R^1$ is each independently selected at each occurrence from hydrogen, halogen, CN, hydroxyl.

$R^3$ is each independently selected at each occurrence from hydrogen, halogen, CN, hydroxyl, $C_{1-6}$ alkyl, $C_{1-6}$ alkoxy, $C_{2-6}$ alkenyl, $-NR^aR^b$.

Preferably, $R^3$ is each independently selected at each occurrence from hydrogen.

In one aspect of the present disclosure, wherein,

Z is selected from a bond, $-CH_2-$ or -C(=O).

Preferably, Z is selected from -C(=O).

In one aspect of the present disclosure, wherein,

$R^a$, $R^b$ are each independently selected at each occurrence from hydrogen, halogen, CN, hydroxyl, $C_{1-6}$ alkyl, $C_{1-6}$ alkoxy, wherein, the alkyl, alkoxy are optionally substituted with one or more substituents selected from hydrogen, halogen, CN, $CF_3$, hydroxyl.

Preferably, $R^a$, $R^b$ are each independently selected at each occurrence from hydrogen.

The present disclosure provides the following compounds:

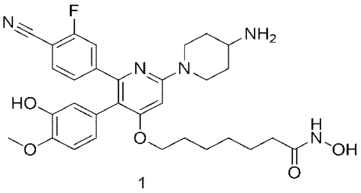
1

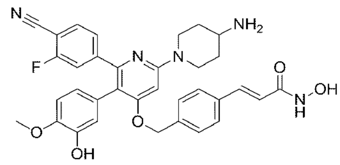
2

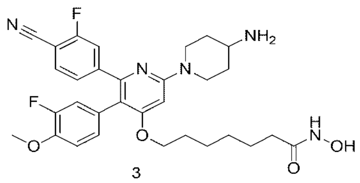
3

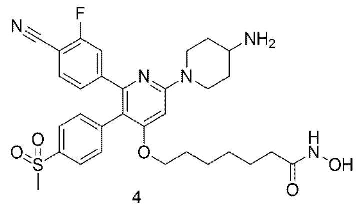
4

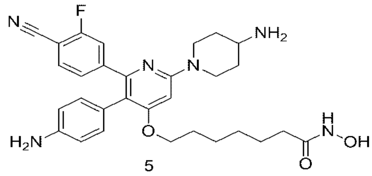
5

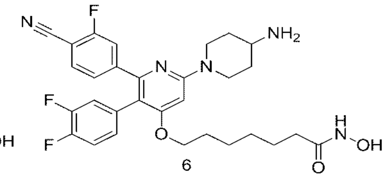
6

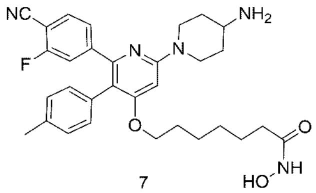
7

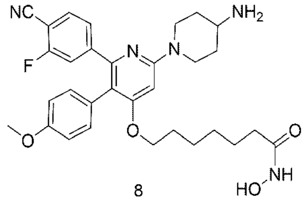
8

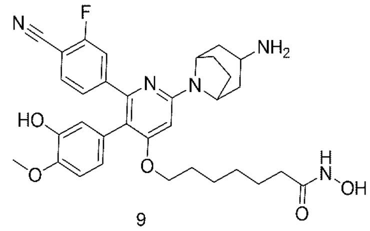
9

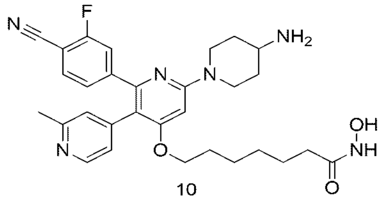
10

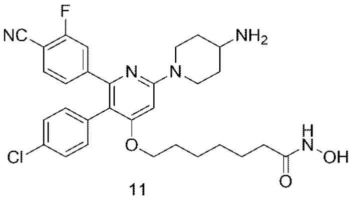
11

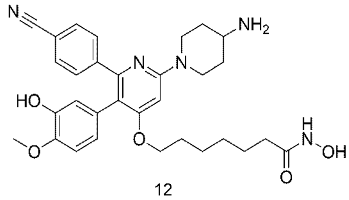
12

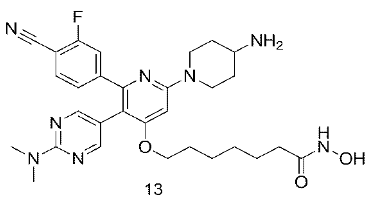
13

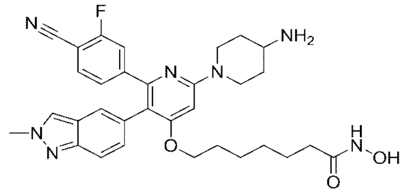
14

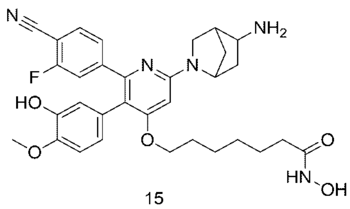
15

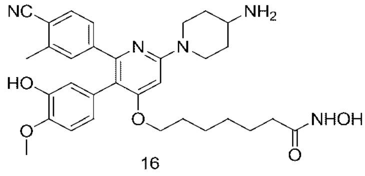
16

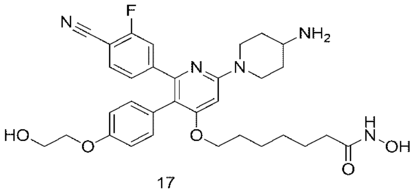
17

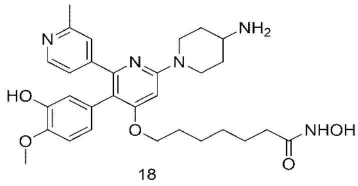
18

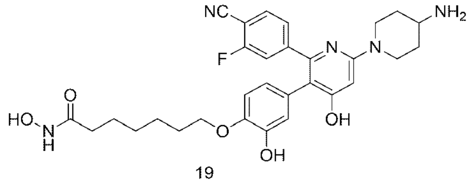
19

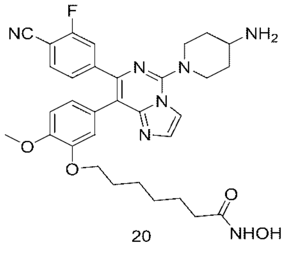
20

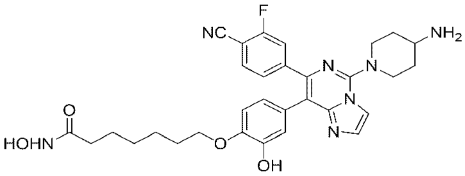

21

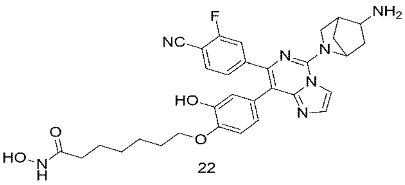

22

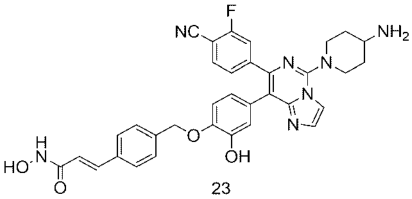

23

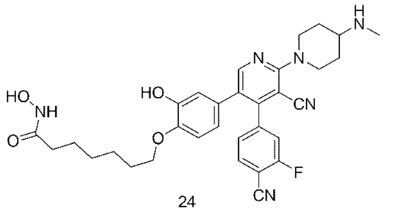

24

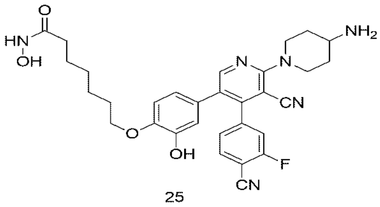

25

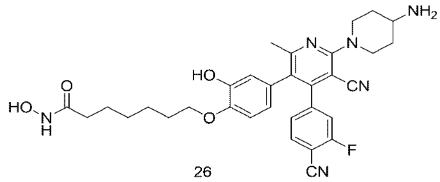

26

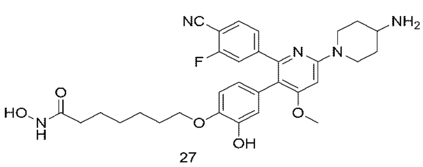

27

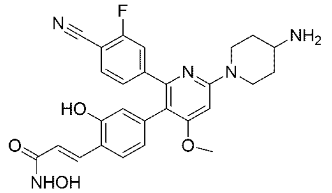

28

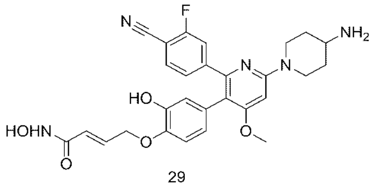

29

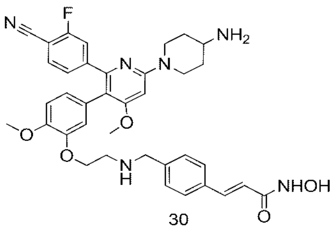

30

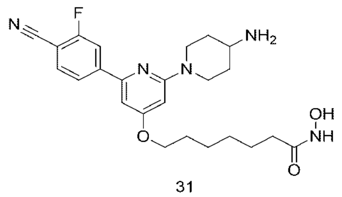

31

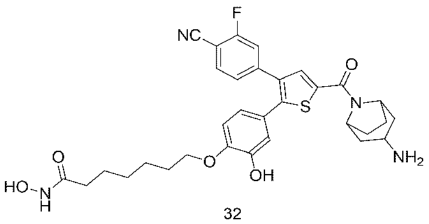

32

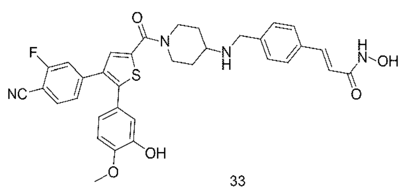

33

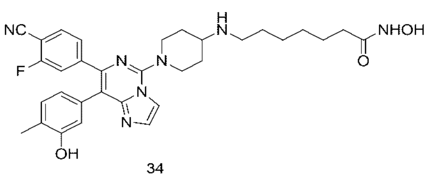

34

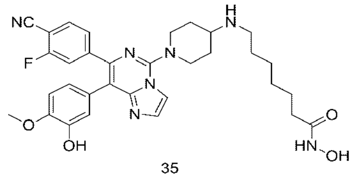

35

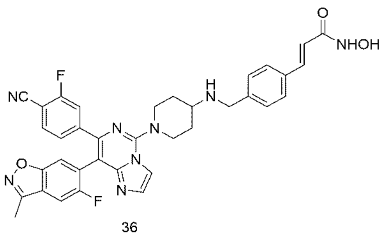

36

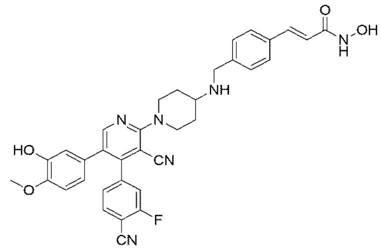

37

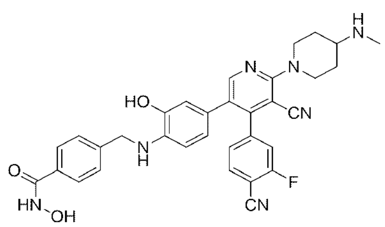

39

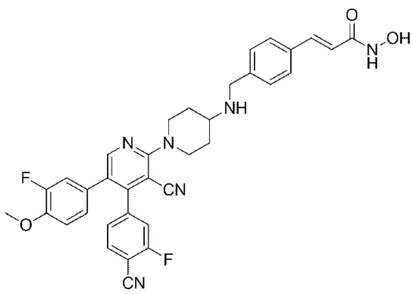

40

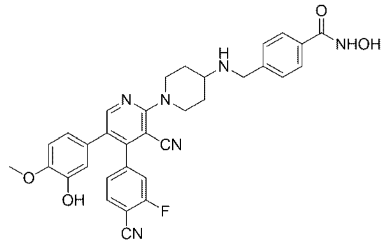

41

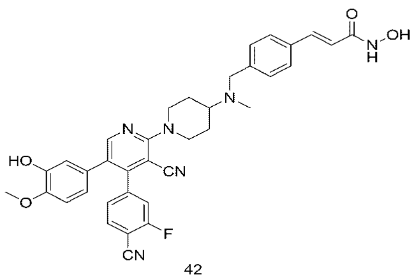

42

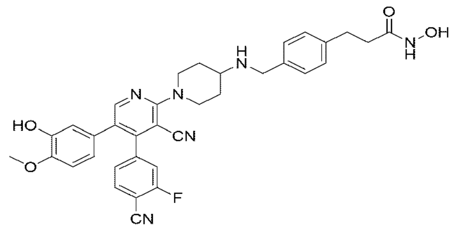

43

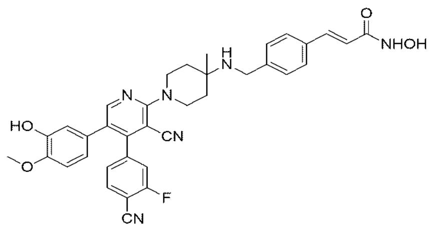

44

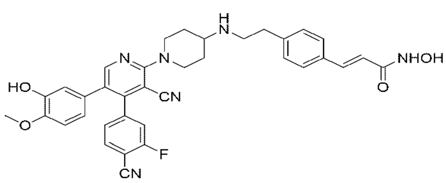

45

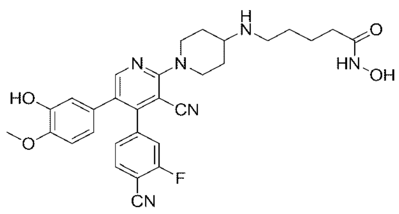

46

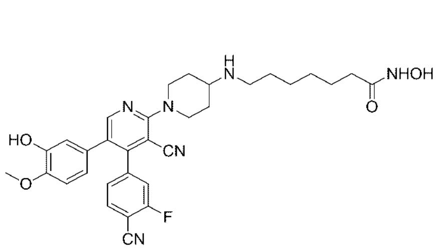

47

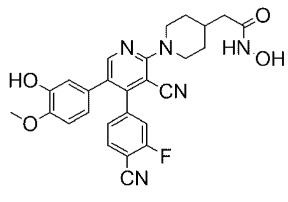

48

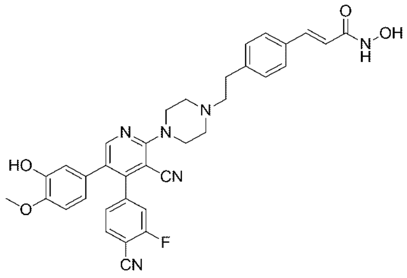

49

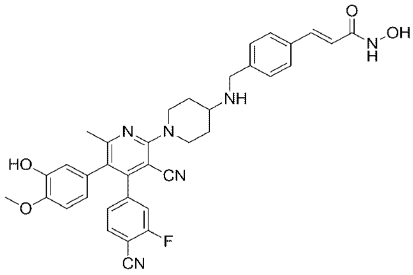

50

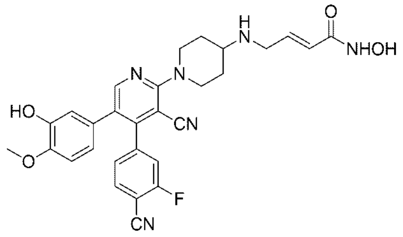

51

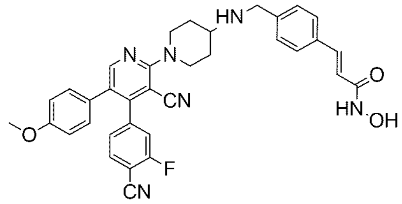

52

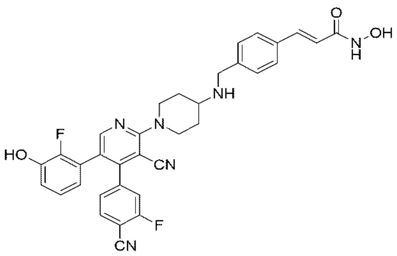

53

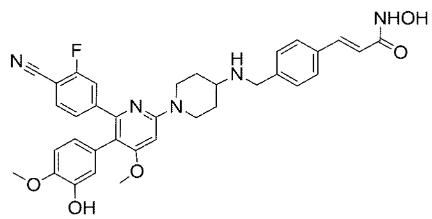

54

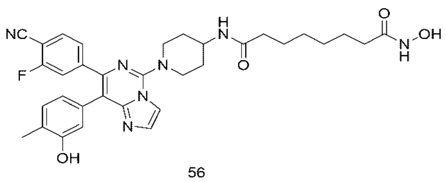

56

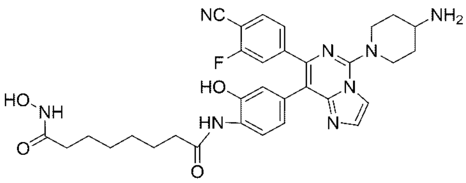

57

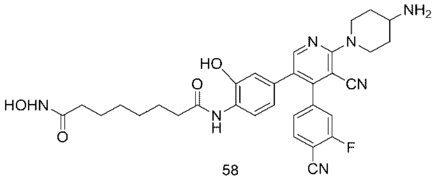

58

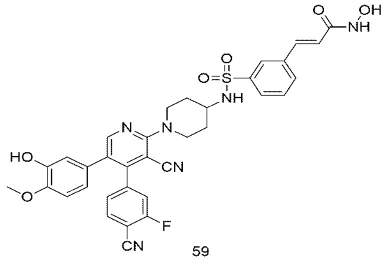

59

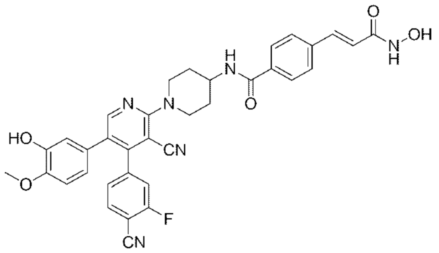

60

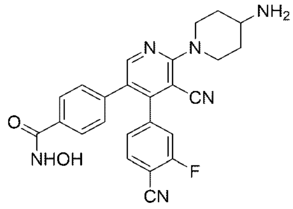

61

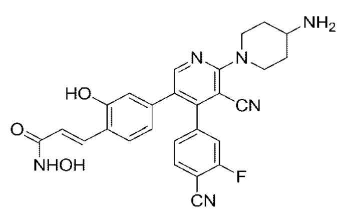

62

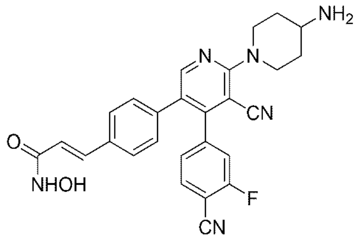

63

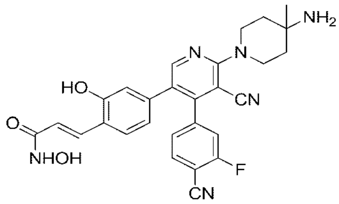

64

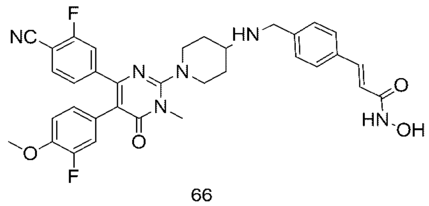

66

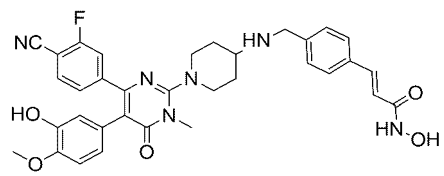

67

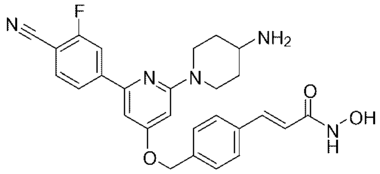

69

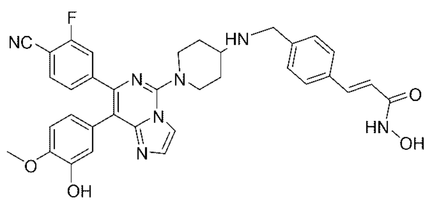

70

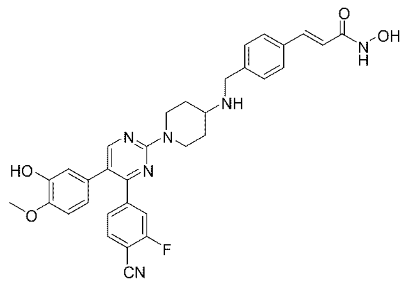

71

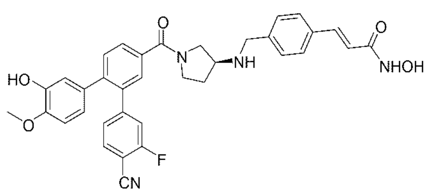

72

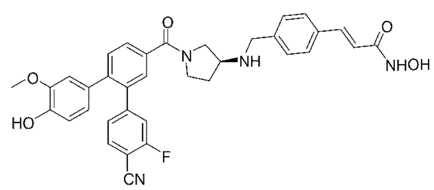

73

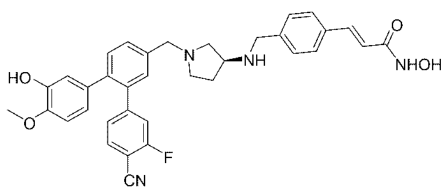

74

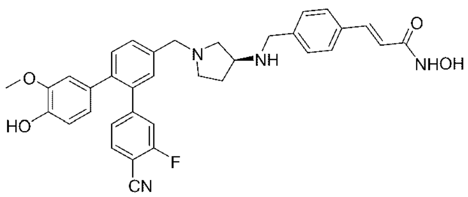

75

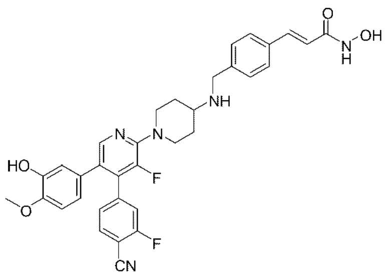

76

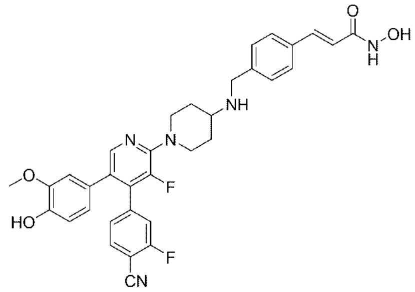

77

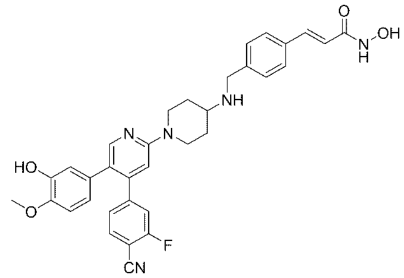

80

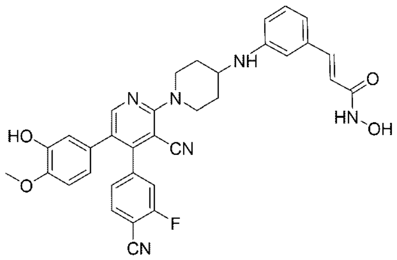

81

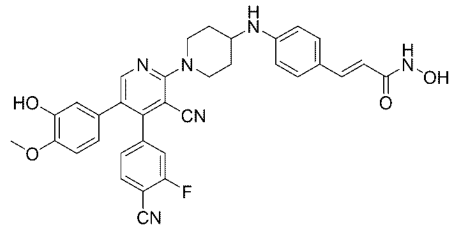

82

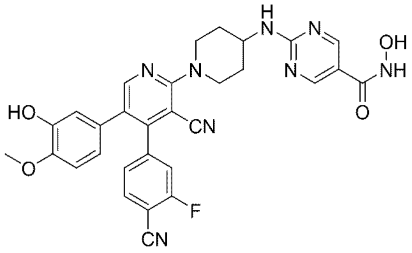

83

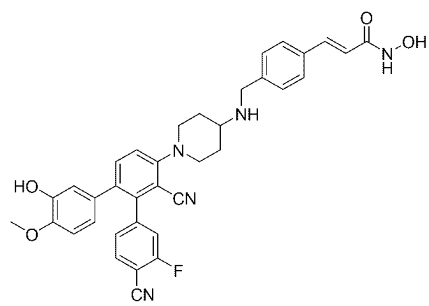

84

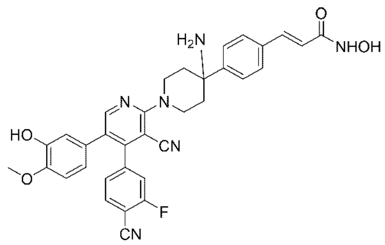

85

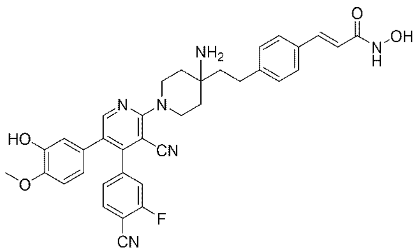

86

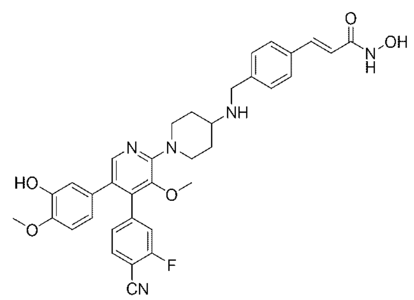

88

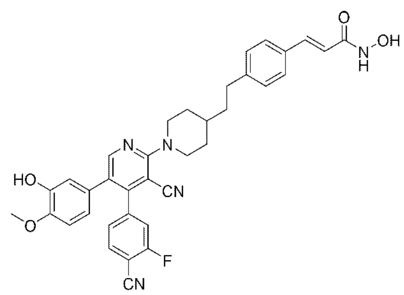

89

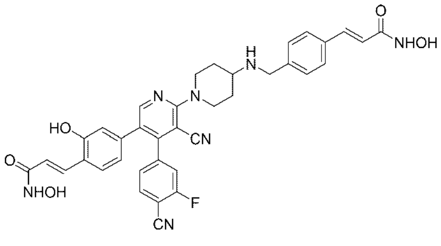

90

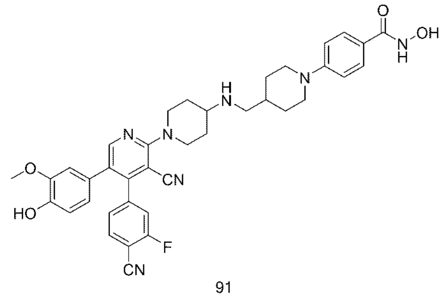

91

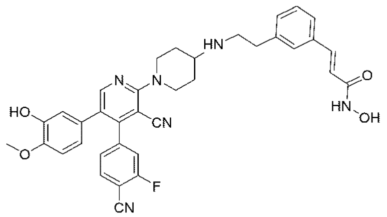

92

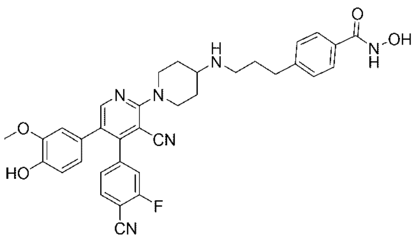

93

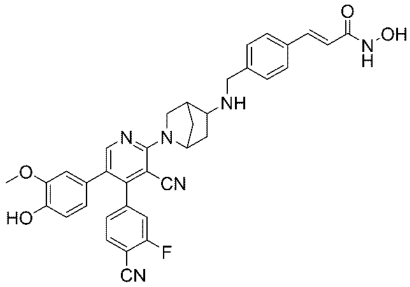

94

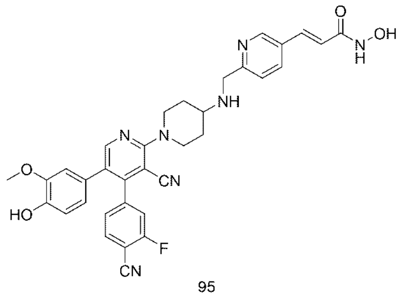

95

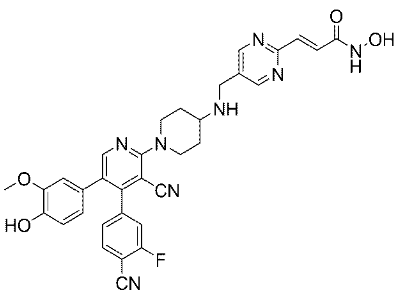

96

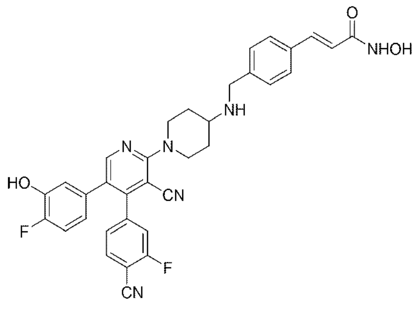

97

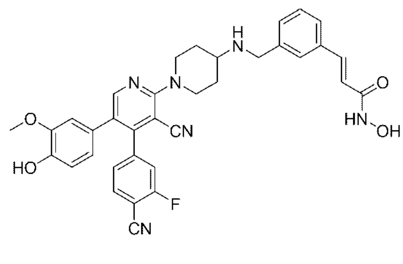

98

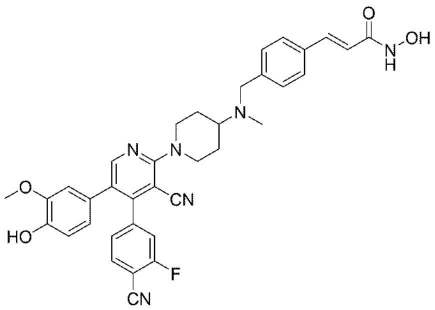

99

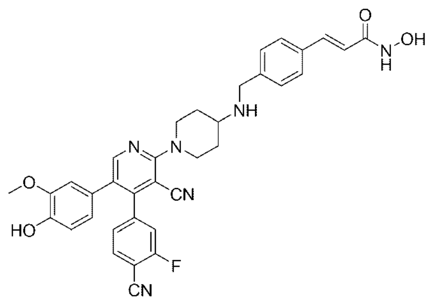

100

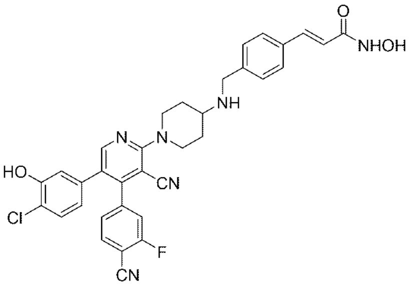

101

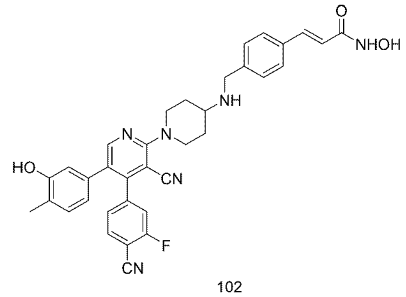

102

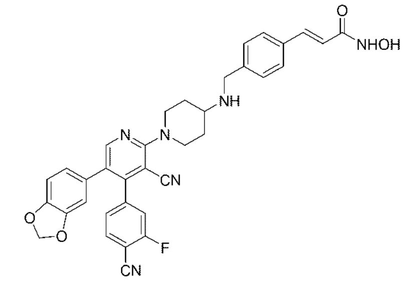

103

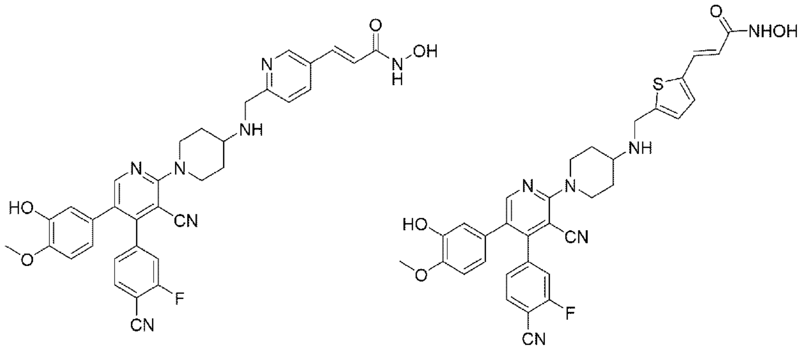

104

105

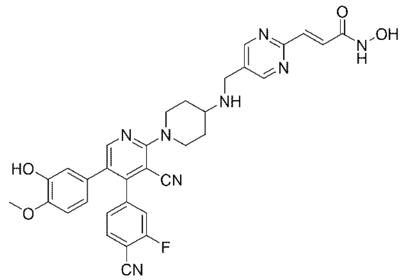

106

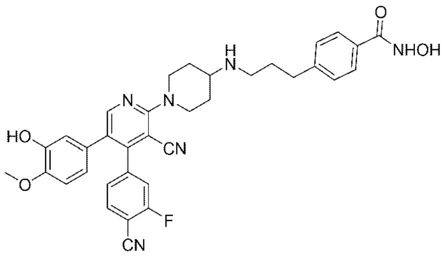

107

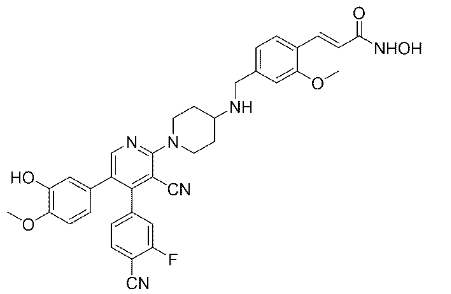

108

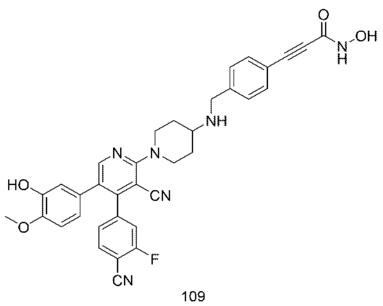

109

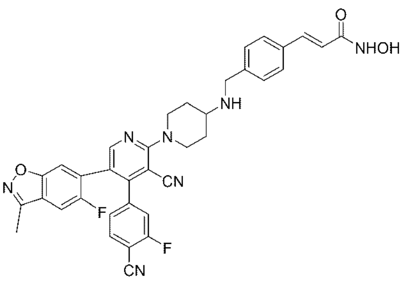

110

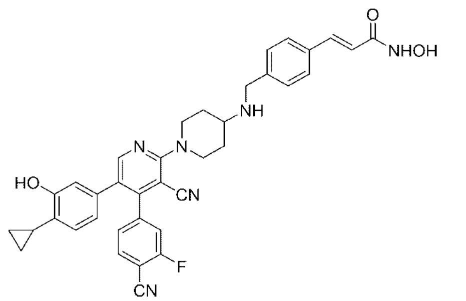

111

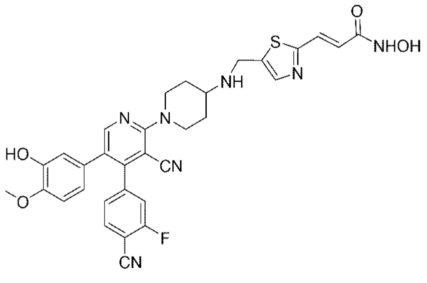

112

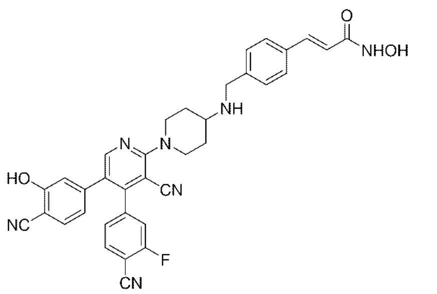

113

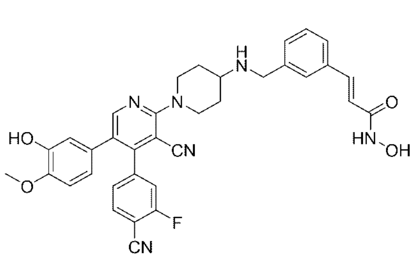

114

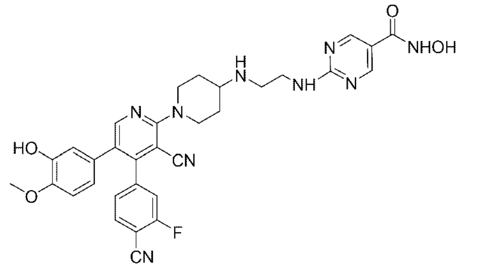

115

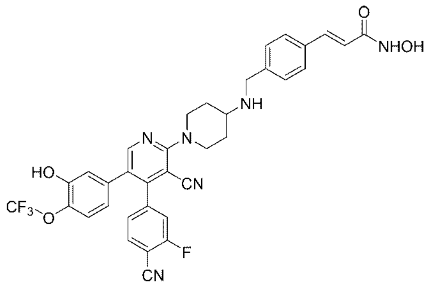

116

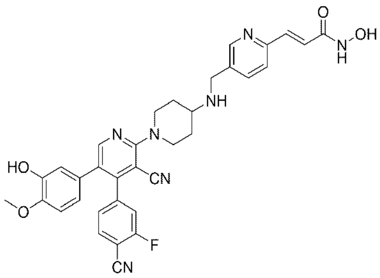

117

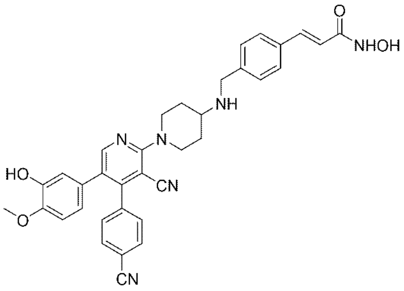

118

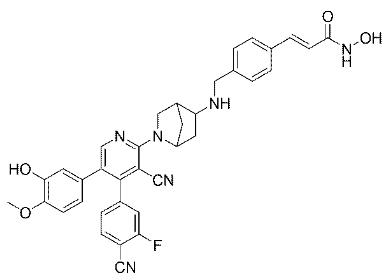

119

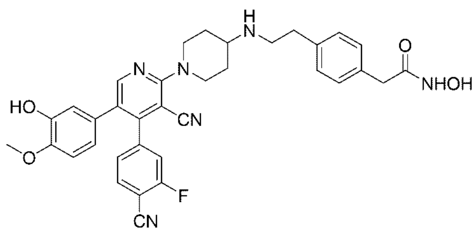

120

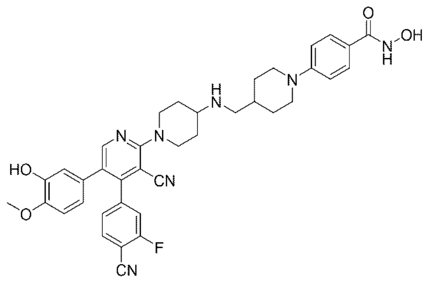

121

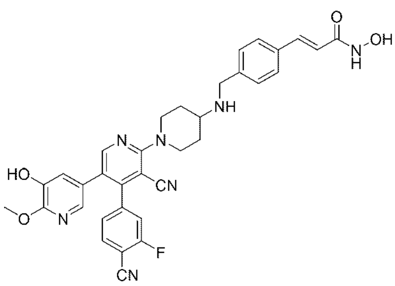

123

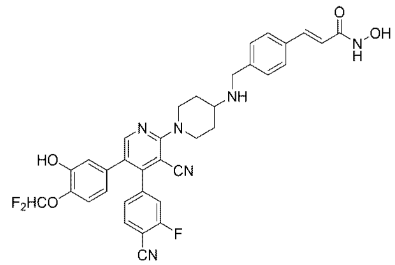

124

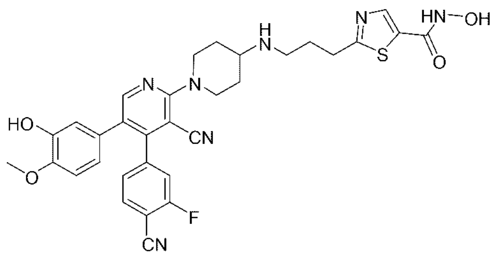

125

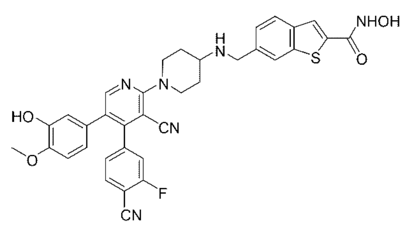

126

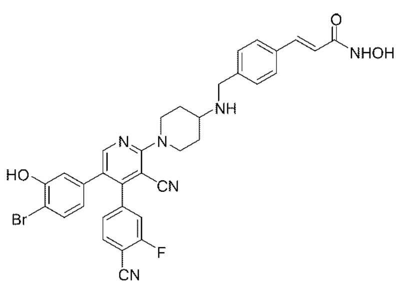

127

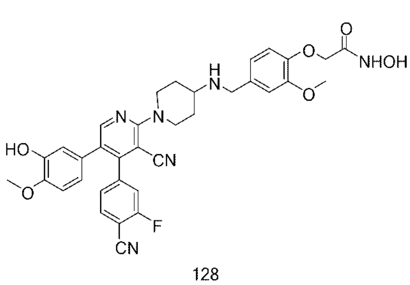

128

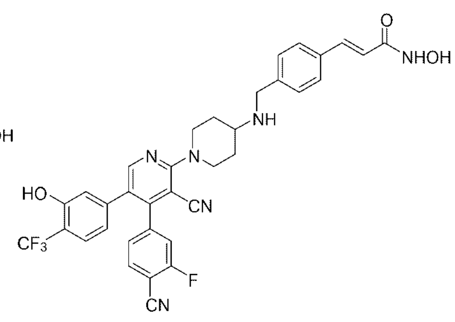

129

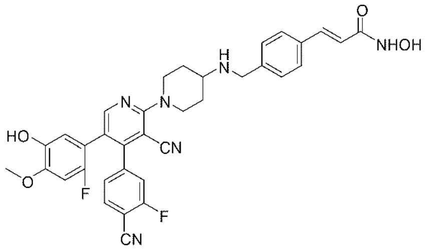

130

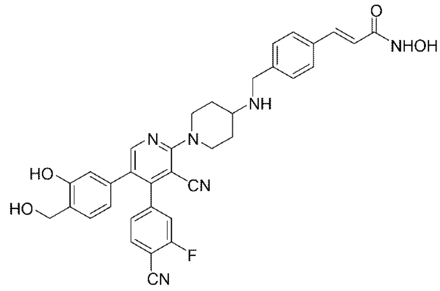

131

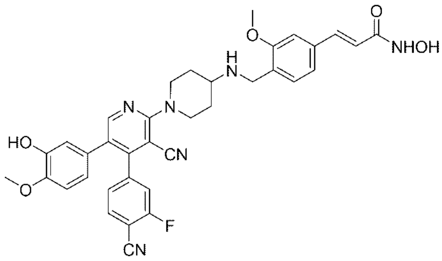

132

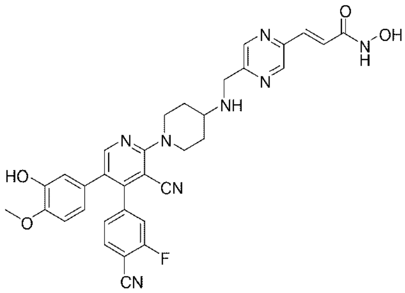

133

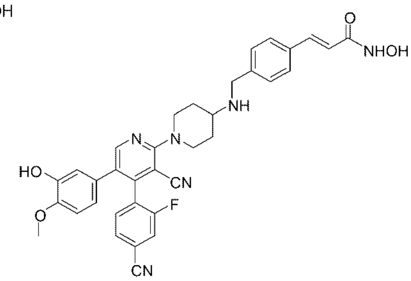

134

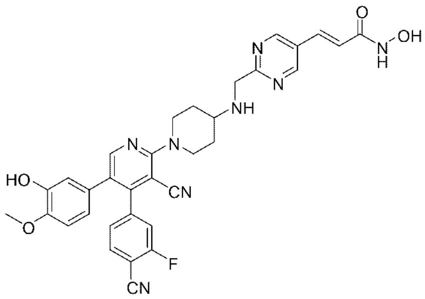

135

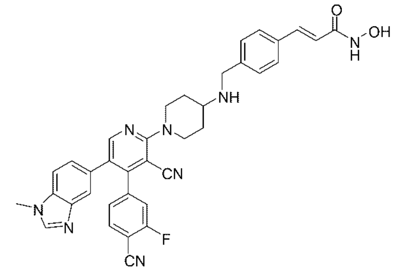

136

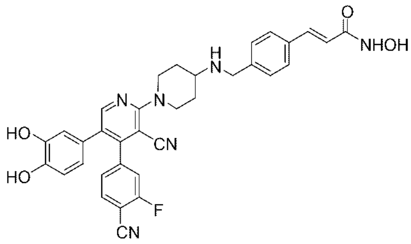

137

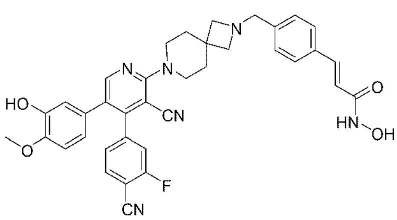

138

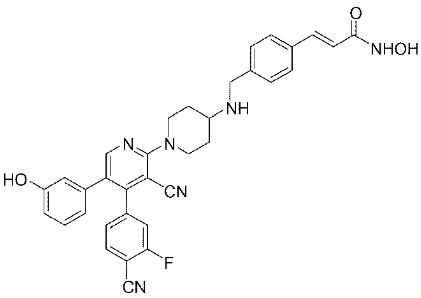

139

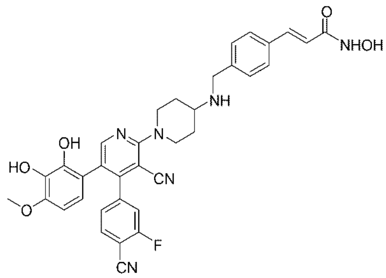

140

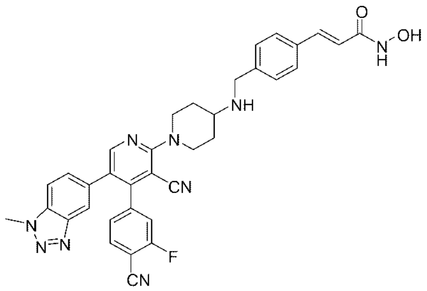

141

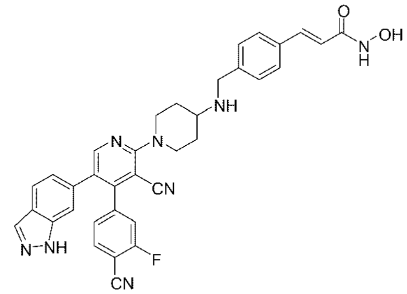

142

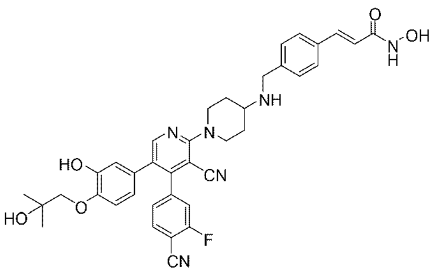

143

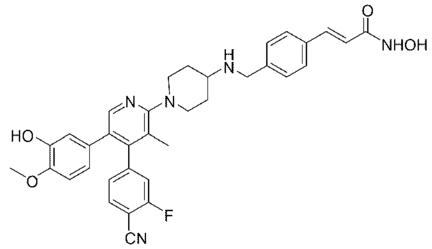

144

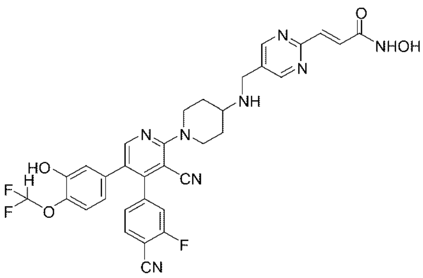

145

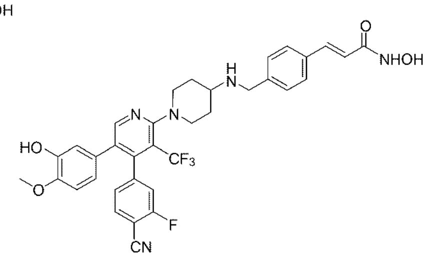

147

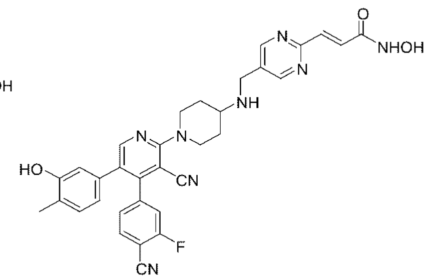

148

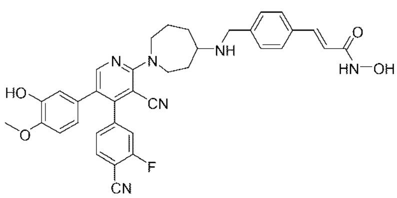

149

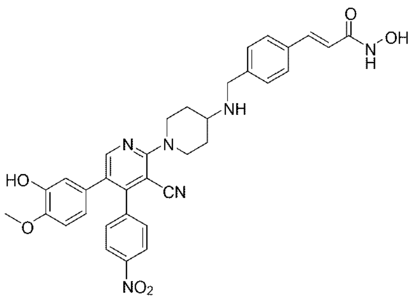

150

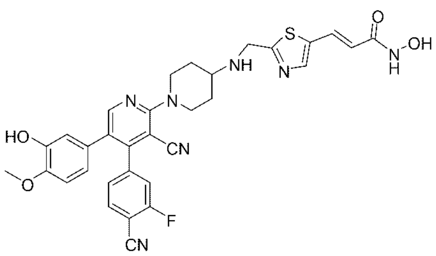

151

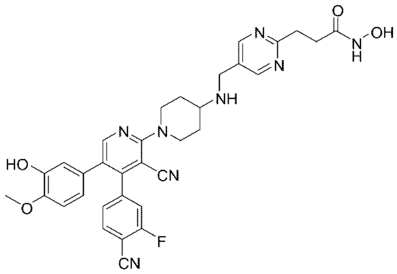

152

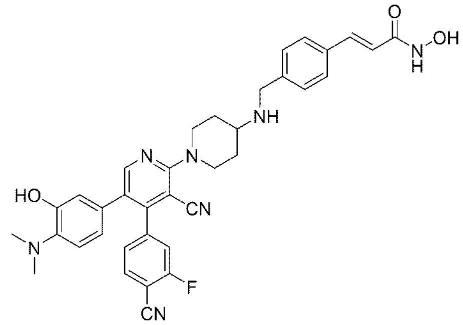

153

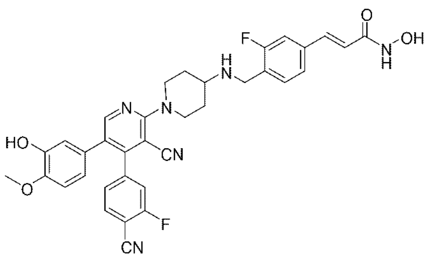

154

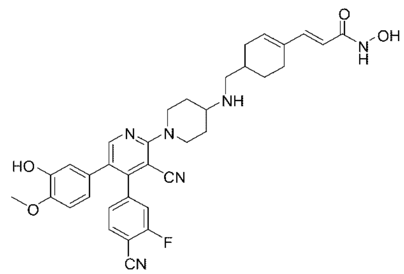

155

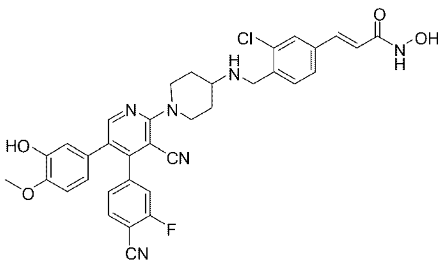

156

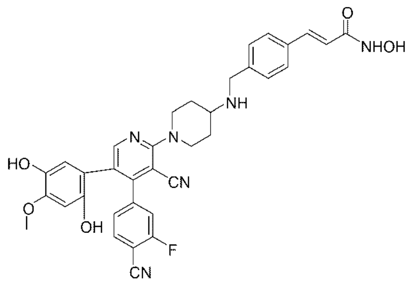

158

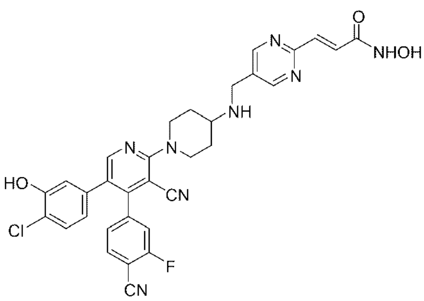

159

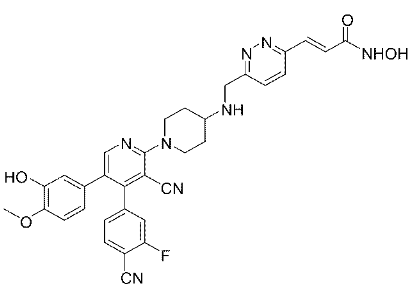

160

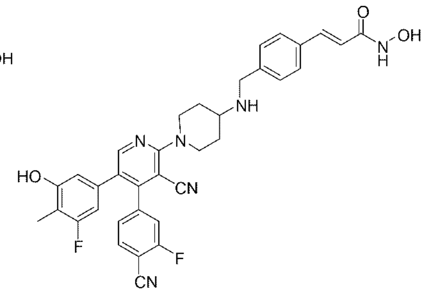

161

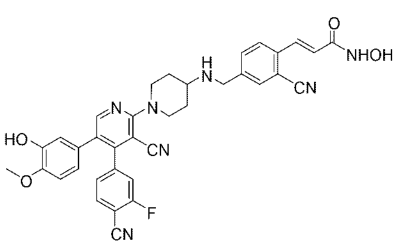

162

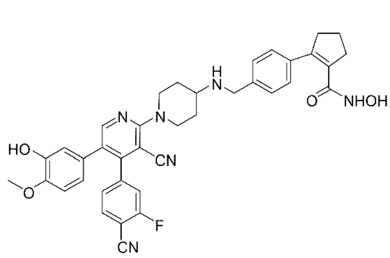

163

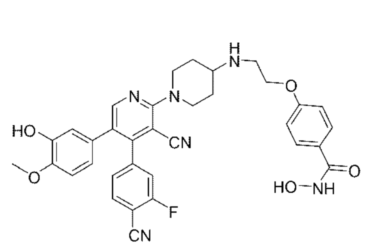

164

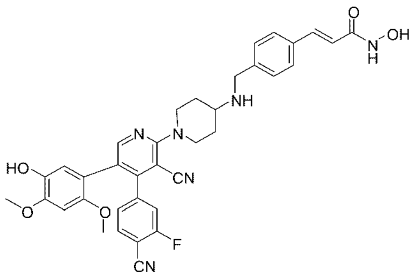
165

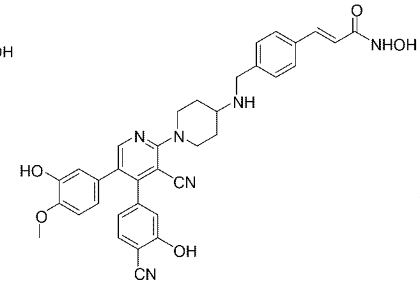
166

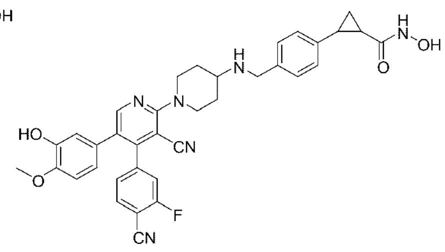
167

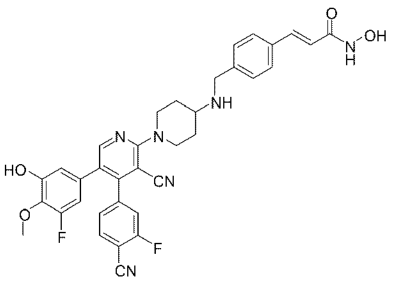
168

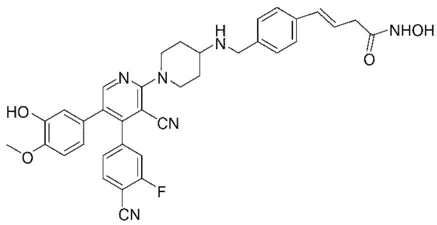
169

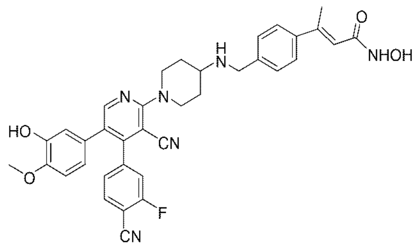
170

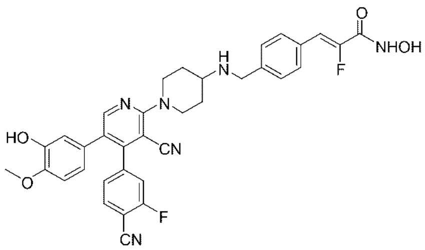
171

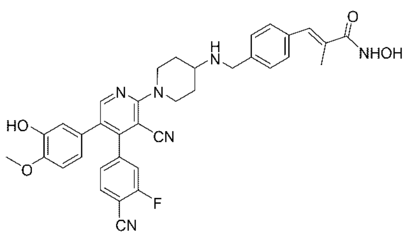
172

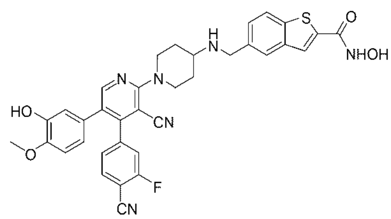
173

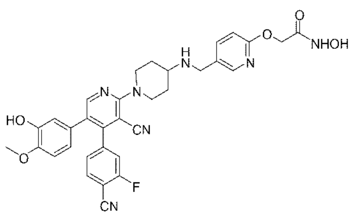
174

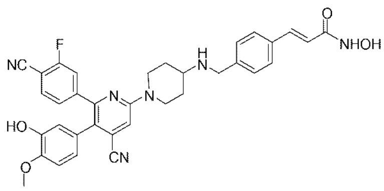
176

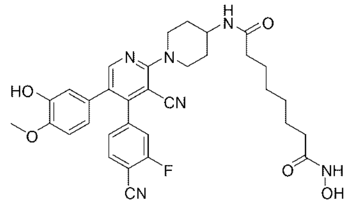
177

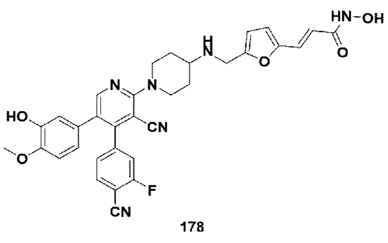
178

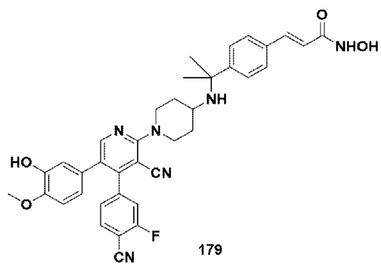
179

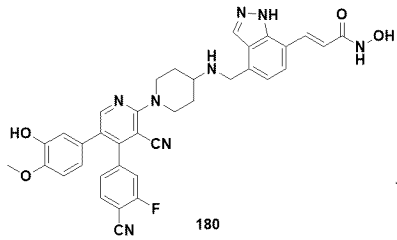
180

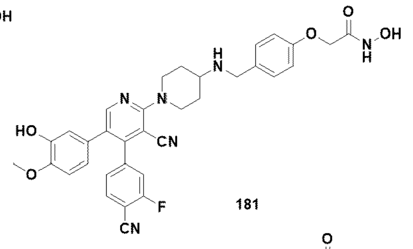
181

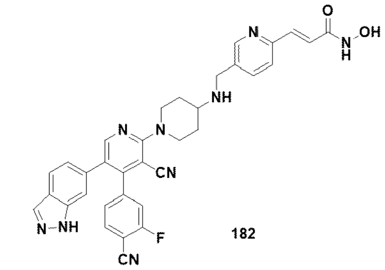
182

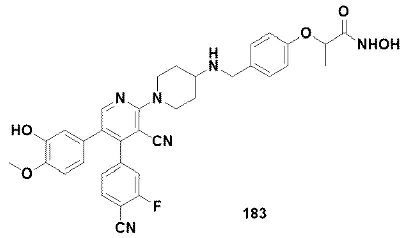
183

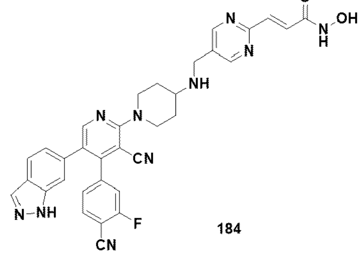
184

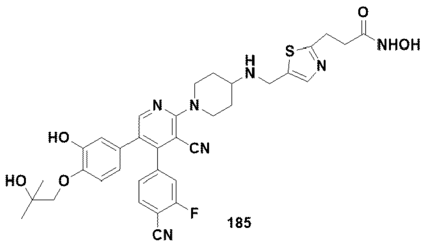
185

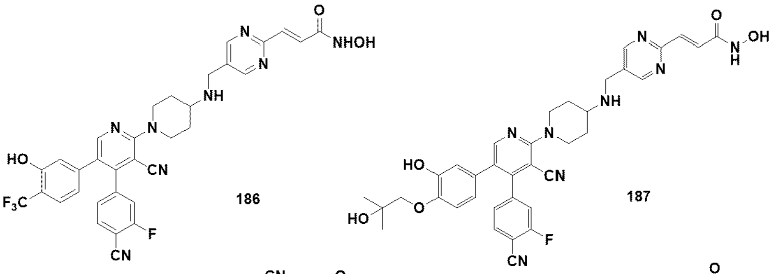
186

187

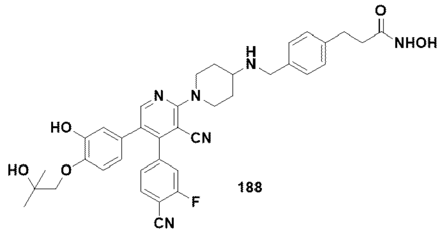
188

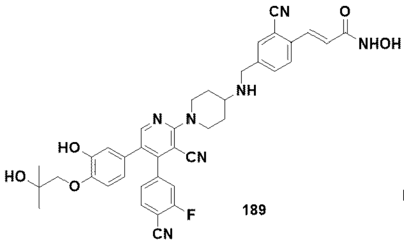
189

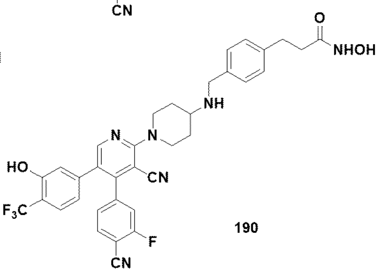
190

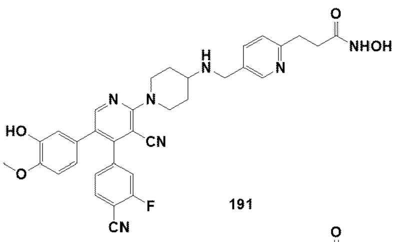
191

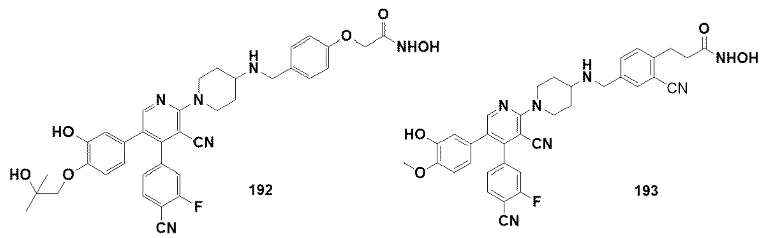
192

193

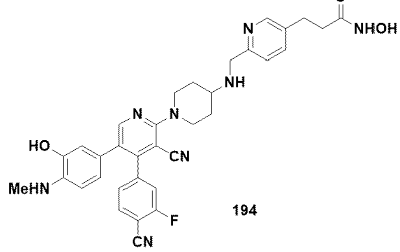
194

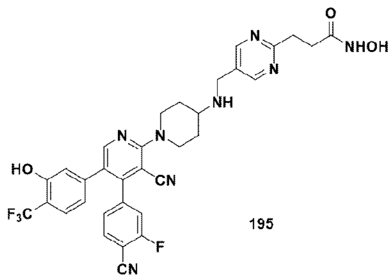
195

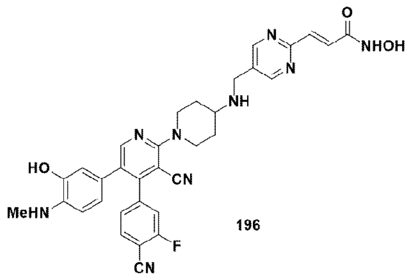
196

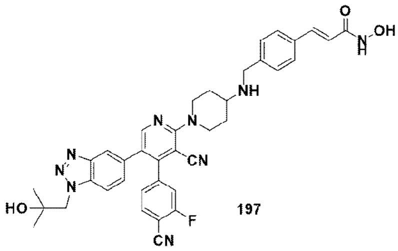
197

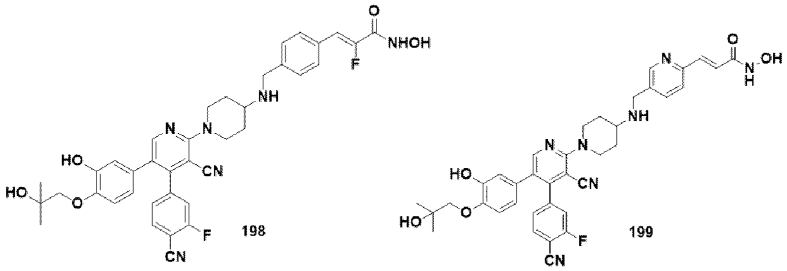

198

199

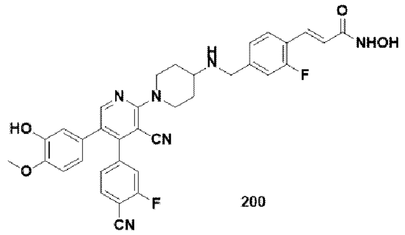

200

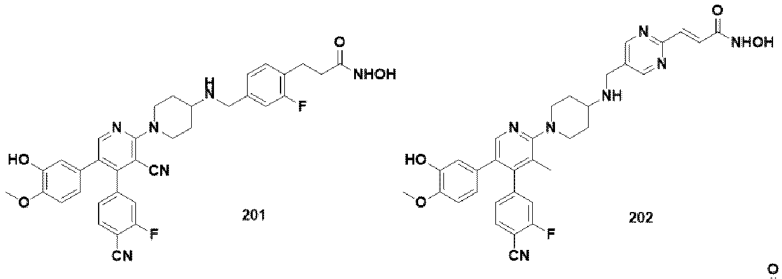

201

202

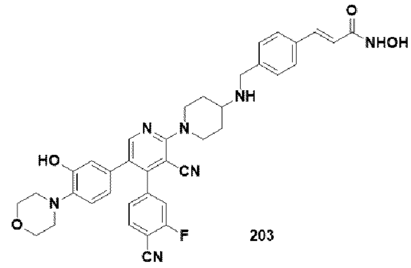

203

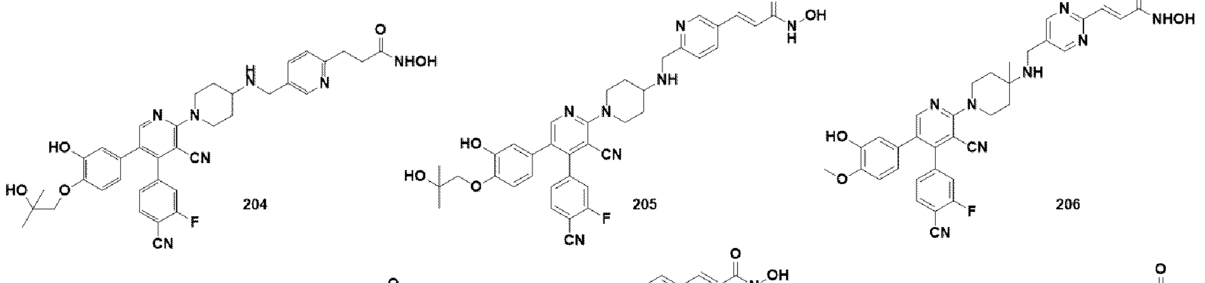

204

205

206

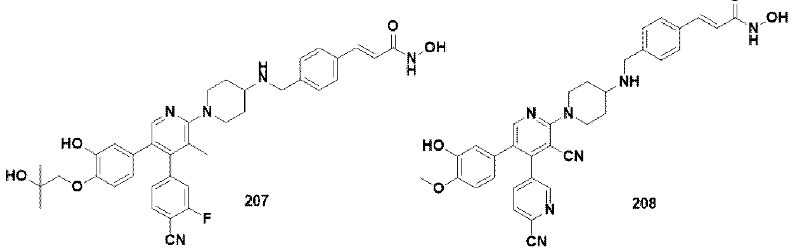

207

208

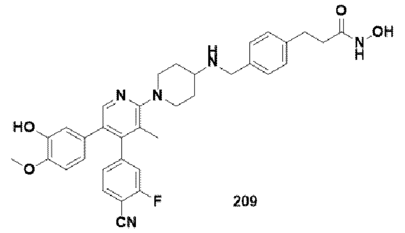

209

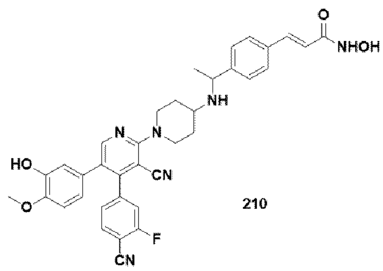

210

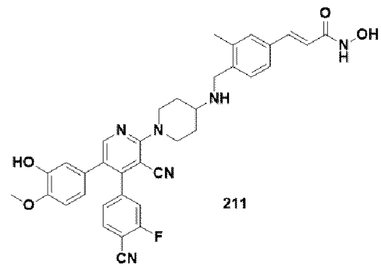

211

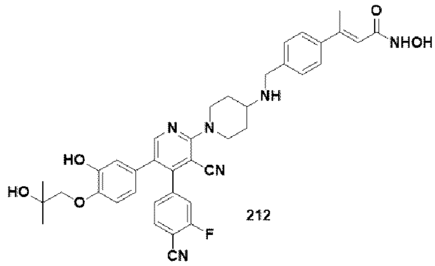

212

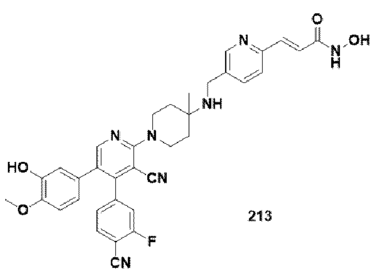

213

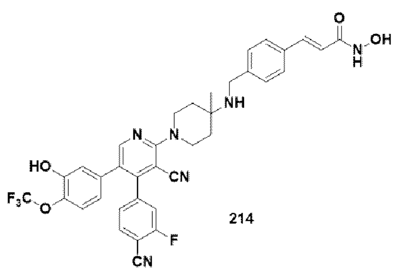

214

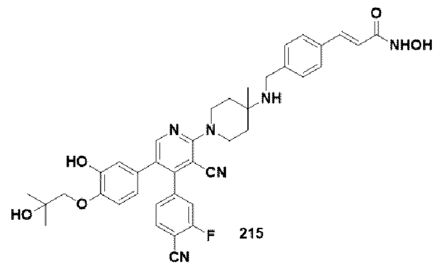

215

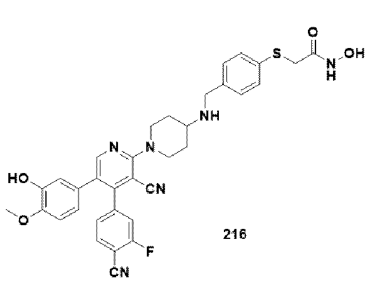
216

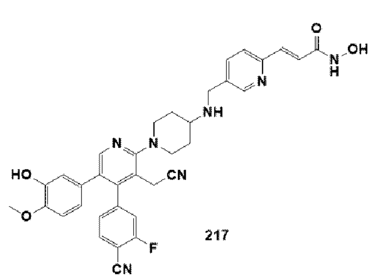
217

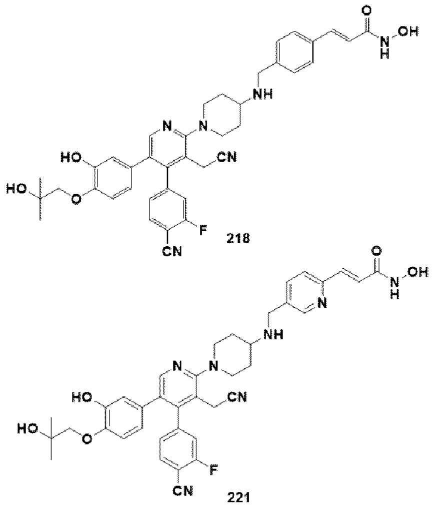
218

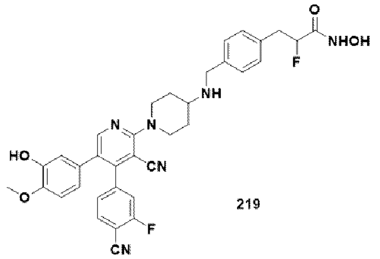
219

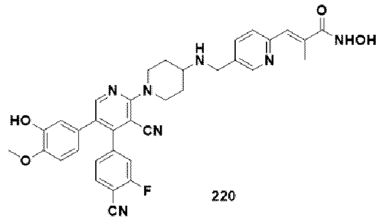
220

221

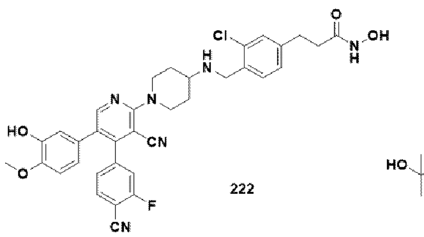
222

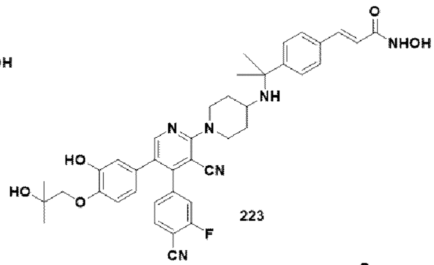
223

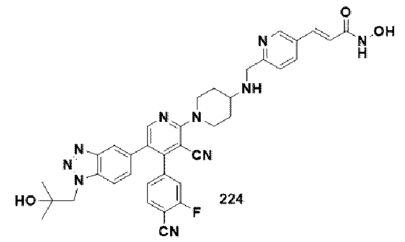
224

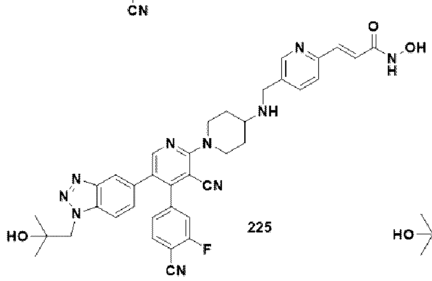
225

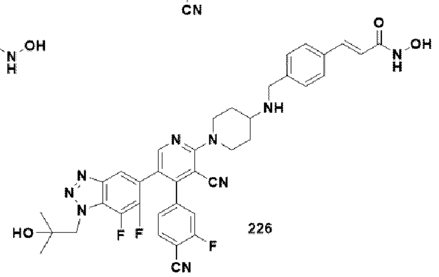
226

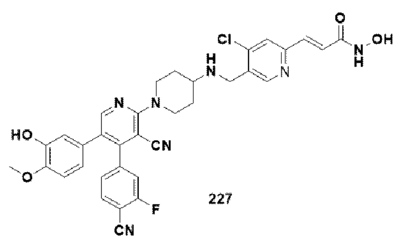
227

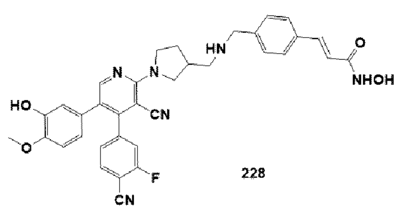
228

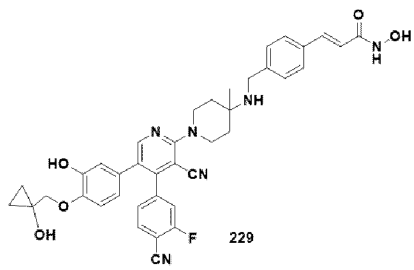
229

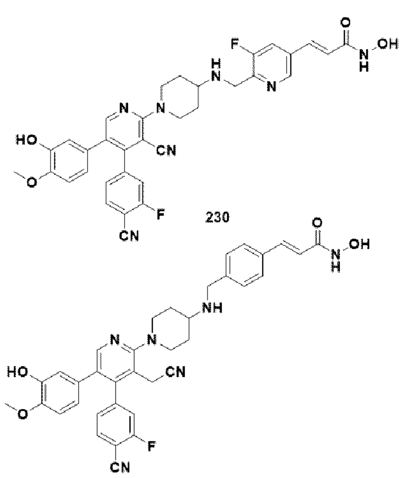
230

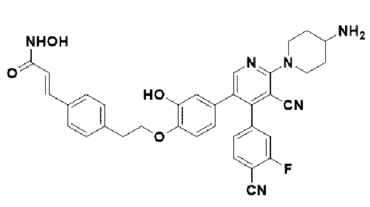
231

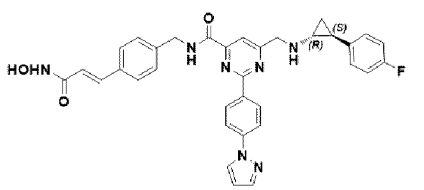
232

233

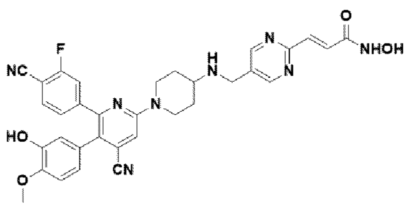
234

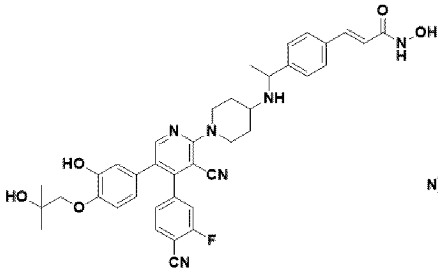
235

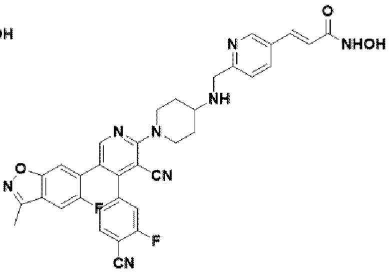
236

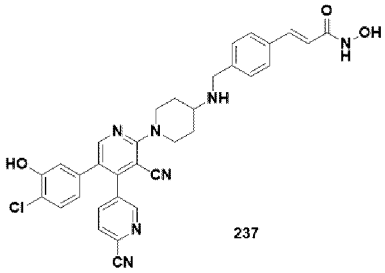
237

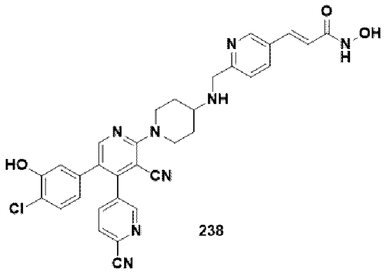
238

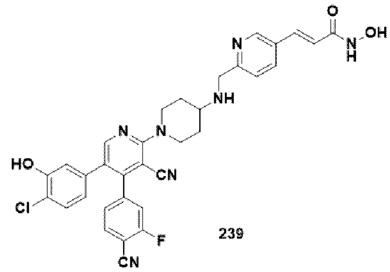
239

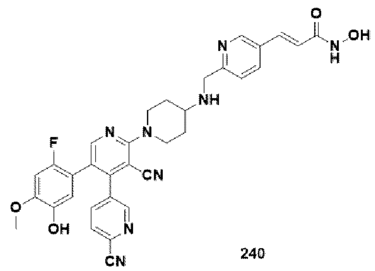
240

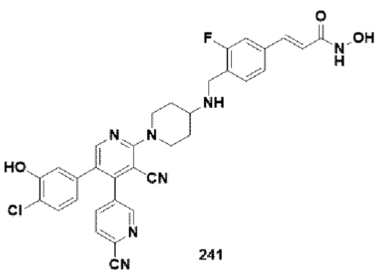
241

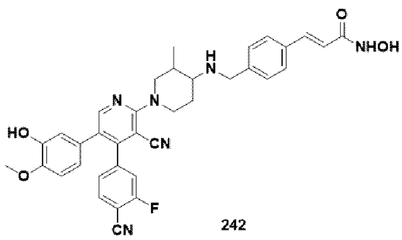
242

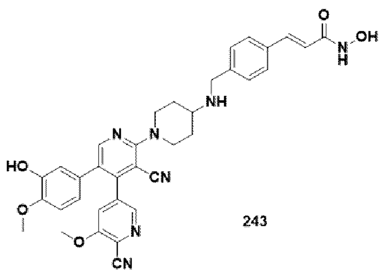
243

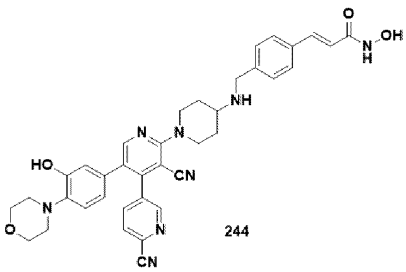
244

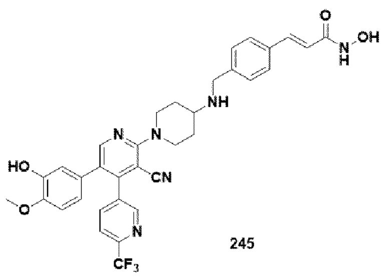
245

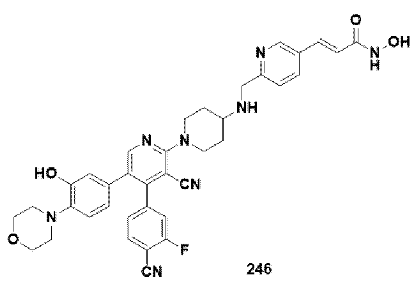
246

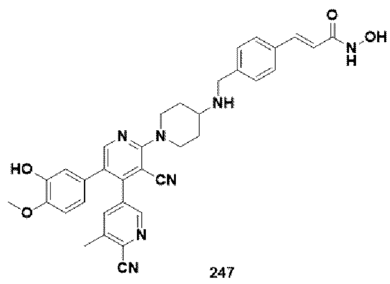
247

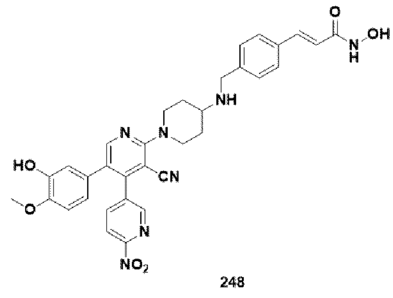
248

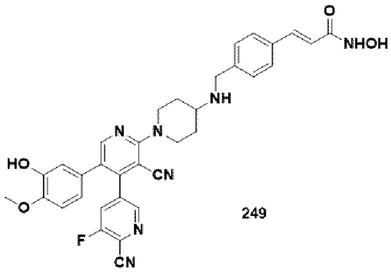
249

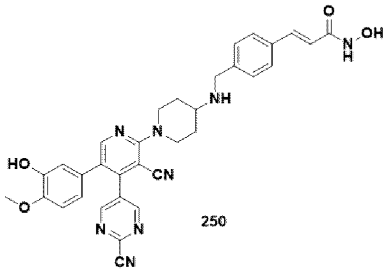
250

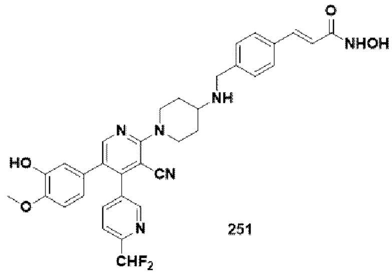
251

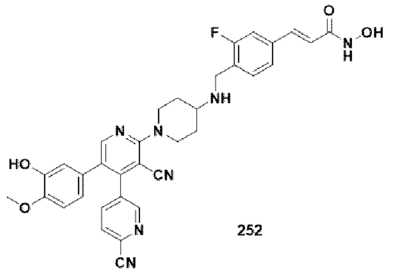
252

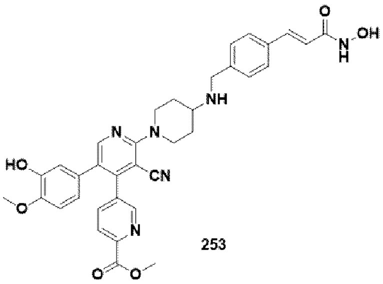
253

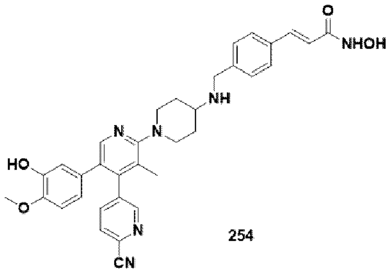
254

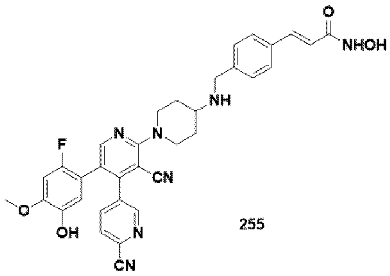
255

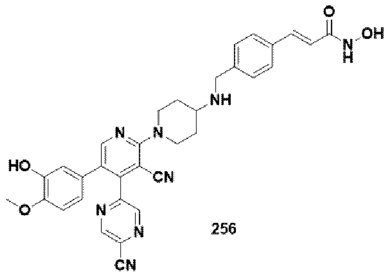
256

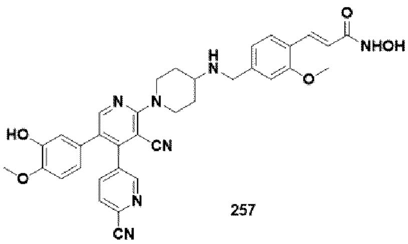
257

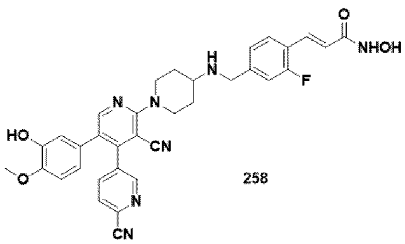
258

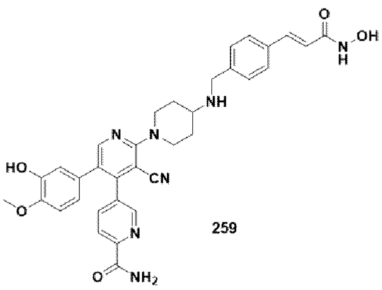
259

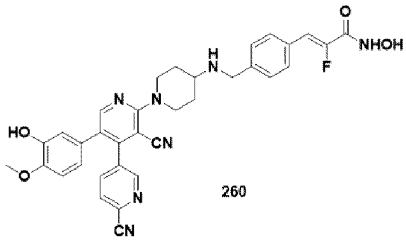
260

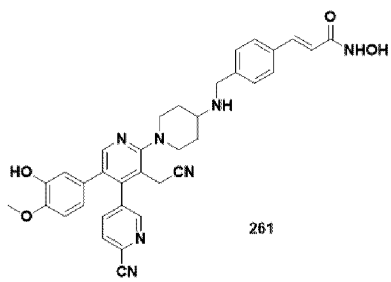
261

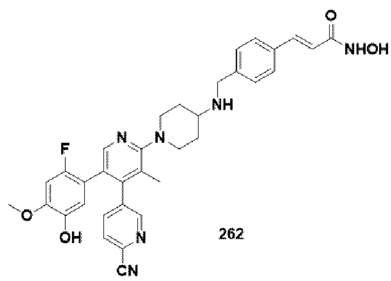
262

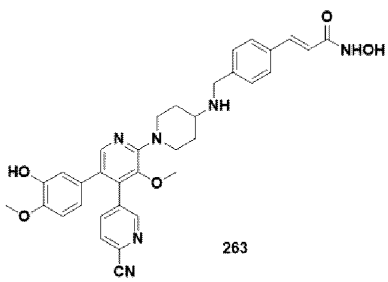
263

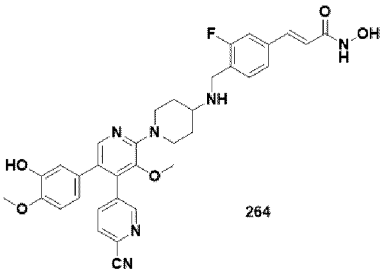
264

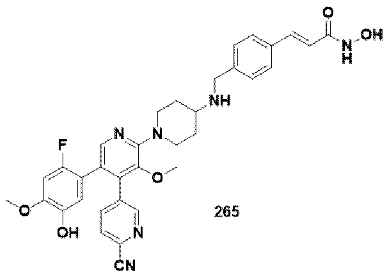
265

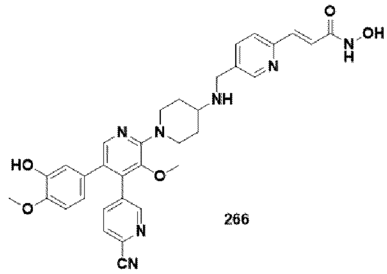
266

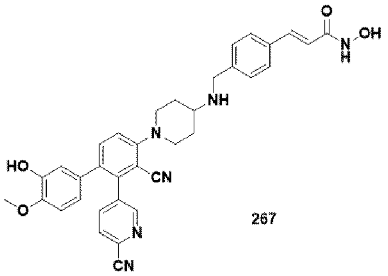
267

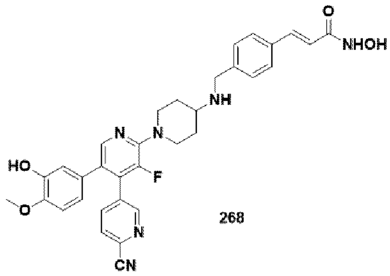
268

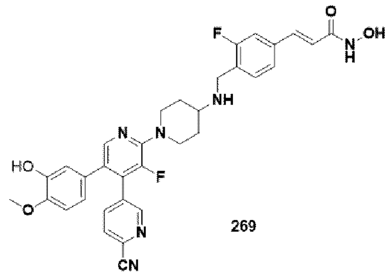
269

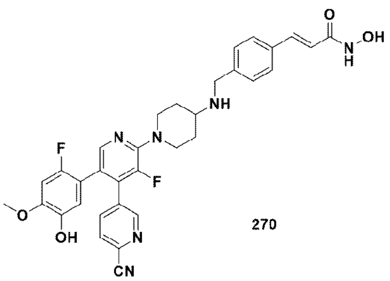
270

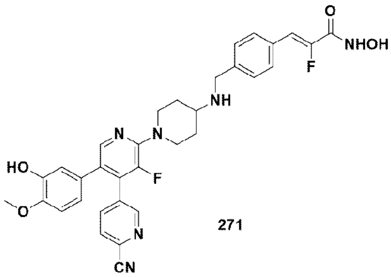
271

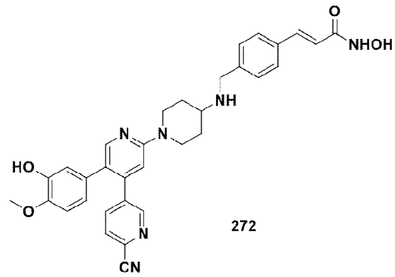
272

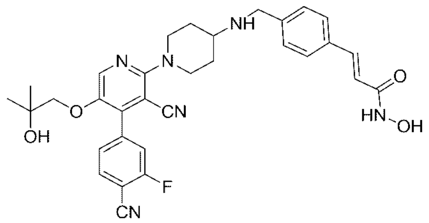

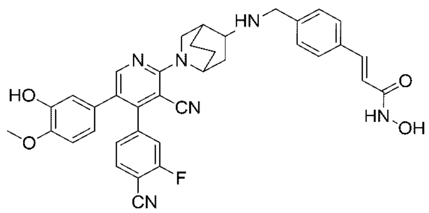

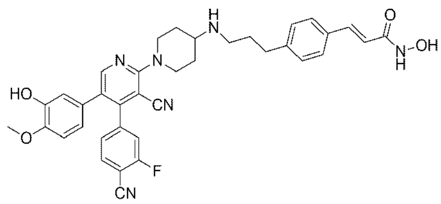

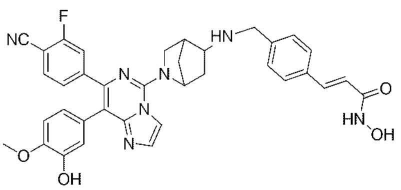

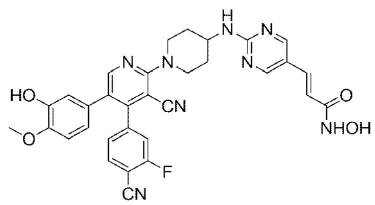

The present disclosure provides a pharmaceutical composition characterized in that the active ingredient of the pharmaceutical composition is selected from one or a combination of two or more of the compound or tautomer, stereoisomer, solvate, metabolite, isotopically-labeled compound, pharmaceutically acceptable salt or co-crystal thereof mentioned above.

The present disclosure provides a use or method of the compound or tautomer, stereoisomer, solvate, metabolite, isotopically-labeled compound, pharmaceutically acceptable salt or co-crystal thereof mentioned above for preventing and treating the related disease mediated respectively or synergistically by LSD1 and/or HDAC.

The use or method comprising the step of administering to a patient in need thereof a therapeutically effective amount of the compound or tautomer, stereoisomer, solvate, metabolite, isotopically-labeled compound, pharmaceutically acceptable salt or co-crystal thereof mentioned above.

In one aspect of the present disclosure, the HDAC enzyme comprises, but is not limited to isoforms of HDAC1,

HDAC2, HDAC3, HDAC4, HDAC5, HDAC6, HDAC7, HDAC8 etc., preferably HDAC1, HDAC8 isoforms, further preferably HDAC1 isoform.

The present disclosure a use of the compound or tautomer, stereoisomer, solvate, metabolite, isotopically-labeled compound, pharmaceutically acceptable salt or co-crystal thereof mentioned above in the manufacture of a medicament for treating the disease mediated by LSD1 and/or HDAC.

In one aspect of the present disclosure, it is the use in the manufacture of a medicament for treating the disease mediated by one or more of LSD1, HDAC.

In one aspect of the present disclosure, the disease is cancer or autoimmune disease.

In one aspect of the present disclosure, the cancer is selected from: non-small cell lung cancer, small cell lung cancer, pancreatic cancer, ovarian cancer, bladder cancer, prostate cancer, chronic myeloid leukemia, colorectal cancer, brain cancer, liver cancer, kidney cancer, gastric cancer, breast cancer, triple negative breast cancer, skin cancer, melanoma, head and neck cancer, bone cancer, cervical cancer, pelvic cancer, vaginal cancer, oral cancer, lymphoma, blood cancer, esophageal cancer, urethral cancer, nasal cavity cancer.

The present disclosure provides a use of the compound or tautomer, stereoisomer, solvate, metabolite, isotopically-labeled compound, pharmaceutically acceptable salt or co-crystal thereof mentioned above, for preventing and treating the related disease mediated respectively or synergistically by LSD1 protein and/or HDAC1 protein, LSD1 protein and/or HDAC8 protein.

In one aspect of the present disclosure, the disease is a disease mediated by the abnormal activity of the protein mentioned above.

## DETAILED DESCRIPTION OF THE INVENTION

Unless otherwise defined hereinafter, all technical and scientific terms used herein are intended to have the same meaning as commonly understood by those skilled in the art. References to the art as used herein are intended to refer to the art as commonly understood in the art, including those variations or substitutions of equivalent art that are obvious to those skilled in the art. While the following terms are believed to be well understood by those skilled in the art, the following definitions are set forth to better explain the present disclosure.

### I. Definiton

The terms "including", "comprising" or "containing" and other variations thereof herein are inclusive or open-ended and do not exclude other elements or method steps not listed. It should be understood by those skilled in the art that terms such as "comprising" cover the meaning of "consisting of".

The term "one or more" or the similar expression "at least one" may denote, for example, 1, 2, 3, 4, 5, 6, 7, 8, 9, 10 or more.

The term "aryl" refers to an all-carbon monocyclic or densely cyclic polycyclic aromatic group having a conjugated $\pi$-electron system. As used herein, the term "C6-10 aryl" refers to an aromatic group containing 6 to 10 carbon atoms, such as phenyl or naphthyl. The aryl may optionally be substituted with one or more suitable substituents, e.g., with cyano (CN), halogen (F, Cl, Br). When the aryl is substituted with more than one substituent, two adjacent substituents may form a 5-6-membered cycloalkyl or a 5-6-membered heterocycloalkyl together with the carbon atoms to which they attach, examples include, but are not limited to,

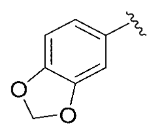

, et al.

The term "heteroaryl" refers to a monocyclic, bicyclic or tricyclic aromatic ring system comprising at least one heteroatom which may be the same or different (the heteroatom is, e.g., oxygen, nitrogen or sulphur) and which may additionally, in each case, be benzo-fused. As used herein, the term "C6-10 heteroaryl" refers to a monocyclic, bicyclic or tricyclic aromatic ring system having 6-10 ring atoms and containing at least one heteroatom that may be the same or different (the heteroatom is, e.g., oxygen, nitrogen or sulfur). The heteroaryl may optionally be substituted with one or more suitable substituents, e.g., substituted with cyano (CN), halogen (F, Cl, Br). Examples include, but are not limited to,

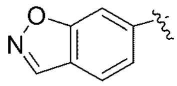, 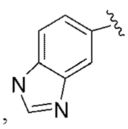, 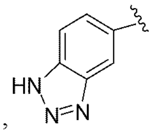, 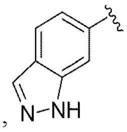,

et al.

The term "cycloalkyl" refers to a saturated monocyclic or polycyclic (e.g., bicyclic) hydrocarbon ring (e.g., monocyclic ring such as cyclopropyl, cyclobutyl, cyclopentyl, cyclohexyl, or bicyclic ring including spirocyclic, fused, or bridged systems (e.g., bicyclo[2.2.1]heptyl, et al). As used herein, the term "C3-6 cycloalkyl" refers to a saturated monocyclic or polycyclic (e.g., bicyclic) hydrocarbon ring (e.g., cyclopropyl, cyclobutyl, cyclopentyl, cyclohexyl) having 3 to 6 ring-forming carbon atoms. The cycloalkyl may optionally be substituted with one or more suitable substituents.

The term "heterocycloalkyl" refers to a saturated monocyclic or polycyclic (e.g., bicyclic) group having 2, 3, 4, 5, 6, 7, 8, 9, or 10 carbon atoms and one or more heteroatoms in the ring; the heterocycloalkyl group may be connected to the rest of the molecule by any one of the carbon atoms or by a heteroatom. As used herein, the term C3-10 heterocycloalkyl is a saturated monocyclic or polycyclic (e.g., bicyclic) group having 3-10 ring-forming carbon atoms in the ring and containing at least one heteroatom which may be the same or different (the heteroatom is, e.g., oxygen, nitrogen or sulfur). The heterocycloalkyl may optionally be substituted with one or more suitable substituents.

The term "heterocycloalkenyl" refers to a class of cycloalkenyl groups as defined above in which at least one of the ring-forming carbon atoms is replaced by a heteroatom, e.g., nitrogen, oxygen, or sulfur. Examples of C3-10 heterocycloalkenyl include, but are not limited to, tetrahydropyridine, dihydropyran, dihydrofuran, pyrrolizidine, et al, and can be a monocyclic or multicyclic (e.g., bicyclic) group. The heterocyclicalkenyl may optionally be substituted with one or more suitable substituents.

The term "halogen-substituted" or "halogen" group is defined to include F, Cl, Br or I.

The term "hydroxyl" refers to -OH.

The term "alkyl" is defined as a straight or branched saturated aliphatic hydrocarbon. As used herein, the term "$C_{1-6}$ alkyl" refers to a straight or branched saturated aliphatic hydrocarbon group having 1, 2, 3, 4, 5 or 6 carbon atoms, e.g., methyl, ethyl, n-propyl, isopropyl, n-butyl, sec-butyl, tert-butyl, n-amyl, n-hexyl, et al.

The term "halogen-substituted alkyl", when used herein alone or in combination with other groups, refers to an alkyl, as defined above, in which one or more hydrogen atoms are substituted with a halogen. It should be understood by those skilled in the art that when there is more than one halogen substituent, the halogens may be the same or different and may be located on the same or different C atoms. As used herein, the term "halogen-substituted $C_{1-6}$ alkyl" refers to a $C_{1-6}$ alkyl group in which one or more hydrogen atoms have been replaced by a halogen, e.g, trifluoromethyl.

The term "hydroxyl-substituted alkyl" means an alkyl as defined above in which one or more hydrogen atoms have been replaced by hydroxyl. As used herein, the term "hydroxyl-substituted $C_{1-6}$ alkyl" means one or more hydrogen atoms of the $C_{1-6}$ alkyl have been replaced by hydroxyl, e.g,

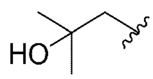

The term "alkoxy" refers to an oxygen atom attached to an "alkyl" as defined above, i.e., an "alkoxy" group can be defined as -OR, where R is an alkyl as defined above. As used herein, examples of the term "$C_{1-6}$ alkoxy" include, but are not limited to, methoxy, ethoxy, n-propoxy, isopropoxy, n-butoxy, sec-butoxy, tert-butoxy, n-pentyloxy, n-hexyloxy, et al.

The term "halogen-substituted alkoxy" refers to the alkoxy as defined above in which one or more hydrogen atoms are substituted with a halogen. It should be understood by those skilled in the art that when there is more than one halogen substituent, the halogens can be the same or different and can be located on the same or different C atoms. As used herein, the term "halogen-substituted $C_{1-6}$ alkoxy" refers to one or more hydrogen atoms of the $C_{1-6}$ alkoxy have been replaced by a halogen, e.g, difluoromethoxy, trifluoromethoxy, et al.

The term "alkenyl" refers to a straight or branched aliphatic hydrocarbon group containing at least one carbon-carbon double bond. The double bond may be present as an E or Z isomer. The double bond may be located at any possible position in the hydrocarbon chain. As used herein, the term "C2-6 alkenyl" refers to an alkenyl containing from 2 to 6 carbon atoms, e.g, vinyl, propenyl, butenyl, butadienyl, pentenyl, pentadienyl, hexenyl, hexadienyl, et al. The alkenyl may optionally be substituted with one or more suitable substituents.

The term "alkynyl" refers to a straight or branched aliphatic hydrocarbon group containing at least one C≡C triple bond. The triple bond may be located at any possible position in the hydrocarbon chain. As used herein, the term "$C_{2-6}$ alkynyl" refers to an alkynyl containing from 2 to 6 carbon atoms, e.g, ethynyl, propynyl, butynyl, pentynyl, hexynyl, et al. The alkynyl may optionally be substituted with one or more suitable substituents.

The term "-(aryl or heteroaryl)-alkenyl-" refers to the aryl or heteroaryl attached to the alkenyl as defined above.

As used herein, the term "-($C_{6-10}$ aryl or heteroaryl)-$C_{2-6}$ alkenyl-" refers to the aryl or C6-10 heteroaryl containing 6 to 10 carbon atoms attached to the alkenyl containing 2 to 6 carbon atoms, e.g,

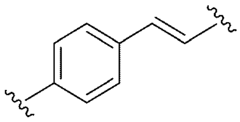 or 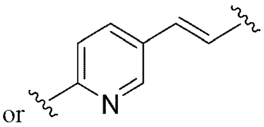

"Alternatively, one or more alkyl groups of the alkyl may be optionally replaced with one or more groups selected from -C(=O)-, -$S(=O)_2$- or -NRa-", refers to one or more alkyl or alkylene fragments of an alkyl group, e.g., $C_{1-10}$ alkyl, is optionally replaced by one or more groups selected from -C(=O)-, -$S(=O)_2$-, or -NRa-, e.g., when $L^1$ is -$C_{10}$ alkyl-$C_{6-10}$ aryl-$C_{2-6}$ alkenyl-, a segment of the $C_{10}$ alkyl issubstituted with -C(=O)- to afford -$C_3$ alkyl-C(=O)-$C_6$ alkyl--$C_{6-10}$ aryl-$C_{2-6}$ alkenyl- or -$C_9$ alkyl-C(=O)-$C_{6-10}$ aryl-$C_{2-6}$ alkenyl, non-limitingly comprising a methylene group in

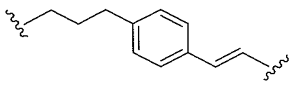

substituted with -NH- to afford

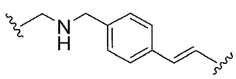, 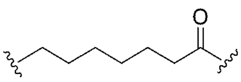,

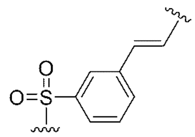, 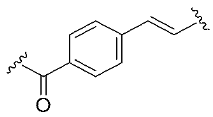

et al.

The term "substituted" refers to one or more (e.g., one, two, three or four) hydrogens on the designated atom are replaced by a selection from the indicated groups, provided that the normal atomic valence of the designated atom in the present case is not exceeded and the substitution results in a stable compound. Combinations of substituents and/or variables are permissible only if such combinations form a stable compound.

The term "hydroxyl-substituted $C_{1-6}$ alkoxy-$C_{3-6}$ cycloalkyl" non-limitingly includes

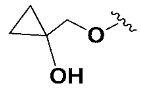

The term "optionally substituted" refers to optionally substituted with a specific group, atom group or portion.

When a group is described as "optionally substituted with one or more substituents", the group may be (1) unsubstituted or (2) substituted. If the carbon on a given moiety is described as being optionally substituted with one or more substituents, the one or more hydrogens on the carbon (to the extent of any hydrogens present) may be substituted or unsubstituted individually and/or collectively with independently selected substituents. If the nitrogen on a group is described as optionally substituted with one or more substituents, the one or more hydrogens on the nitrogen (to the extent of any hydrogen present) may each be substituted or unsubstituted with independently selected substituents.

When the bond of the substituent is shown to pass through a bond connecting two atoms in the ring, such a substituent may be bonded to any of the ring-forming atoms in the substitutable ring.

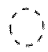

indicates a double bond may be present or not present at any position within the ring, which means that it includes various scenarios such as saturated cyclic systems, unsaturated non-aromatic cyclic systems with double bonds, and aromatic cyclic systems

The compounds of the present disclosure may also comprise one or more (e.g., one, two, three, or four) isotopic

replacement.

The term "stereoisomer" refers to an isomer formed due to at least one asymmetric center. In a compound having one or more (e.g., one, two, three, or four) asymmetric centers, racemates, racemic mixtures, mono enantiomers, diastereoisomeric mixtures, and individual diastereoisomers may result. Specific individual molecules may also exist in geometric isomers (cis/trans). Similarly, the compound of the present disclosure may exist in mixtures of two or more structurally different forms in rapid equilibrium (commonly referred to as tautomer). Representative examples of tautomers include keto-enol tautomers, phenol-keto tautomers, nitroso-oxime tautomers, imine-enamine tautomers, et al. The scope of the present application covers all such isomers or mixtures thereof in any ratio (e.g., 60%, 65%, 70%, 75%, 80%, 85%, 90%, 95%, 96%, 97%, 98%, 99%).

Pharmaceutically acceptable salts of the compound of the present disclosure may include acid addition salts and alkali salts of the compound. Suitable acid addition salts are formed from acids that form non-toxic salts. For a review of suitable salts, see Stahl and Wermuth's "Handbook of Pharmaceutical Salts: properties, selection, and use" (Wiley-VCH, Weinheim. Germany, 2002).

In some embodiments, pharmaceutically acceptable salts of the compound of the present disclosure, are selected from hydrochloride, formate, trifluoroacetate, et al.

The term "co-crystal" refers to a crystal formed by the combination of an active pharmaceutical ingredient and a co-crystal-forming agent (e.g., a coformer) in the presence of hydrogen bonds or other non-covalent bonds, and more specifically, a co-crystal formed from the compound of formula (I) and a pharmaceutically acceptable coformer.

The term "therapeutically effective amount" refers to a sufficient amount of a drug or agent that is non-toxic but achieves the desired effect. In embodiments of the present disclosure, when treating a patient in accordance with the present disclosure, the amount of a given drug depends on a number of factors, such as the specific dosing regimen, the type of disease or condition and the severity thereof, and the uniqueness (e.g., body weight) of the one to be treated or host to be treated. However, the dose to be administered may be routinely determined by methods known in the art based on the particular surrounding circumstances, including, for example, the specific drug that has been employed, the route of administration, the condition to be treated, and the one to be treated or host to be treated. Typically, for therapeutic use in adults, the administered dose is typically in the range of 0.02-5000 mg/day, such as about 1-1500 mg/day. This desired dose may conveniently be expressed as a single dose, or as divided doses administered concurrently (or over a short period of time) or at appropriate intervals, such as two, three, four or more divided doses per day. It will be appreciated by one of skill in the art that, although the above dosage ranges are given, the specific effective amount may be appropriately adjusted according to the patient's condition and in conjunction with the physician's diagnosis.

## II. Examples

The following detailed description of the implementation process and the beneficial effects produced by the present disclosure by means of specific examples is intended to help the reader better understand the essence and characteristics of the present disclosure, and is not intended to be a limitation on the implementable scope of the present case.

The structure of the compound of the present disclosure was determined by nuclear magnetic resonance (NMR) or/and mass spectrometry (MS). The NMR chemical shifts ($\delta$) are given in parts per million (ppm).The NMR determinations were made using an AVANCE NEO 400 MHz Bruker instrument, the solvents for the determinations were deuterated dimethyl sulfoxide (DMSO-$d_6$), deuterated chloroform ($CDCl_3$), deuterated methanol ($CD_3OD$) and the internal standard was tetramethylsilane (TMS). MS was determined using an ISQ-EC Thermo Fisher LC-MS instrument. The instrument used for preparative chromatography was a GX-281 Gilson chromatograph. The following separation methods were: separation method 1: Sun Fire Prep C18 OBDTM 5$\mu$m, 30 $\times$ 150 mm Column, 0.04% HCl aqueous solution/acetonitrile; separation method 2: Sun Fire Prep C18 OBDTM 5 $\mu$m, 30 $\times$ 150 mm Column, 0.02% TFA aqueous solution/acetonitrile; separation method 3: Sun Fire Prep C18 OBDTM 5$\mu$m, 30 $\times$ 150 mm Column, 0.06% TFA aqueous solution/acetonitrile; separation method 4: Xbridge Prep C18 OBDTM 5$\mu$m, 30 $\times$ 150 mm Column, 10 mM $NH_4HCO_3$ aqueous solution/acetonitrile; separation method 5: Xbridge Prep C18 OBDTM 5 $\mu$m, 30 $\times$ 150 mm Column, 0.6% $NH_3.H_2O$ aqueous solution/acetonitrile.

The solvents used in the present disclosure are commercially available.

Unless otherwise specified in the Examples, solutions are aqueous solutions.

Unless otherwise specified in the Examples, the temperature for the reaction is room temperature, i.e., 20°C to 30°C.

HATU refers to O-(7-azabenzotriazol-1-yl)-N,N,N',N'-tetramethyluronium hexafluorophosphate;
DIPEA refers to diisopropylethylamine
DMSO refers to dimethyl sulfoxide;
EA refers to ethyl acetate;

$Pd_2(dba)_3$ refers to tris(dibenzylideneacetone)dipalladium;
DCE refers to dichloroethane;
DMF refers to N,N-dimethylformamide;
NMP refers to N-methylpyrrolidone;
TFA refers to trifluoroacetic acid;
NBS refers to N-bromosuccinimide;
TfOH refers to trifluoromethanesulfonic acid;
DPPA refers to diphenyl phosphate azide.

Positive references are **CC-90011** and **SAHA** (Vorinostat), respectively.

**Example 1**

Preparation of 7-((6-(4-aminopiperidin-1-yl)-2-(4-cyano-3-fluorophenyl)-3-(3-hydroxy-4-methoxyphenyl)pyridin-4-yl) oxy)-N-hyd roxyheptanamide hydrochloride

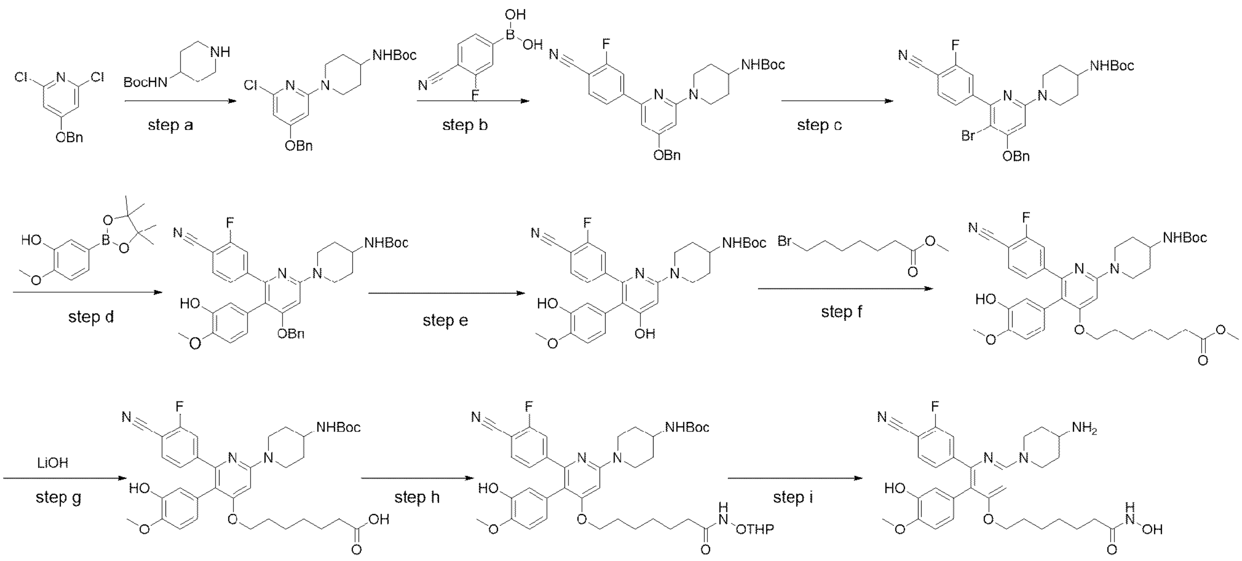


Step a): preparation of tert-butyl (1-(4-(benzyloxy)-6-chloropyridin-2-yl)piperidin-4-yl)carbamate

4-(Benzyloxy)-2,6-dichloropyridine (1.8 g, 7.1 mmol), tert-butylpiperidine-4-carbamate (1.4 g, 7.1 mmol), and DIPEA (415 mg, 14.2 mg) were dissolved in NMP (20 mL), and the reaction was heated up to 130°C and reacted for 2 hours. When the reaction was completed as monitored by LC-MS, the reaction liquid was cooled to room temperature, then added with water (50 mL), and extracted with ethyl acetate (30 mL × 3). The organic phases were combined, washed with brine (20 mL), dried over anhydrous sodium sulfate, filtered, concentrated in vacuum, and the residue was purified by silica gel chromatography (eluent: petroleum ether/ethyl acetate = 2:1) to afford tert-butyl (1-(4-(benzyloxy)-6-chloropyridin-2-yl)piperidin-4-yl) carbamate with a yield of 77.4 %.
ESI-MS m/z = 418.2 $[M+H]^+$.

Step b): preparation of tert-butyl (1-(4-(benzyloxy)-6-(4-cyano-3-fluorophenyl)pyridin-2-yl)piperidin-4-yl)carbamate

Tert-butyl (1-(4-(benzyloxy)-6-chloropyridin-2-yl)piperidin-4-yl)carbamate (2.3 g, 5.5 mmol), (4-cyano-3-fluorophenyl)boronic acid (909 mg, 5.5 mmol), $Cs_2CO_3$ (3.5 g, 11.0 mmol), and $Pd(dppf)Cl_2$ (77.2 mg, 0.11 mmol) were dissolved in 1,4-dioxane (40 mL) and water (4 mL) was added, the reaction mixture was purged with nitrogen three times, then heated to 100°C and reacted for 2 hours, and the reaction was completed as indicated by LC-MS. After cooling to room temperature, water (50 mL) was added, and the mixture was extracted with ethyl acetate (30 mL × 3). The organic phases were combined, washed with brine (20 mL), dried over anhydrous sodium sulfate, filtered, and concentrated under vacuum. The residue was purified by silica gel chromatography (eluent: petroleum ether/ethyl acetate = 1:1) to afford tert-butyl (1-(4-(benzyloxy)-6-(4-cyano-3-fluorophenyl)pyridin-2-yl)piperidin-4-yl)carbamate with a yield of 76.0%.

ESI-MS m/z = 503.2 $[M+H]^+$.

Step c): preparation of tert-butyl (1-(4-(benzyloxy)-5-bromo-6-(4-cyano-3-fluorophenyl)pyridin-2-yl)piperidin-4-yl)carbamate

Tert-butyl (1-(4-(benzyloxy)-6-(4-cyano-3-fluorophenyl)pyridin-2-yl)piperidin-4-yl)carbamate (1.8 g, 3.58 mmol) and NBS (687 mg, 2.41 mmol) were dissolved in DMF (30 mL) and the reaction was conducted at room temperature for 2 hours, and the reaction was completed as indicated by LC-MS. Water (80 mL) was added, and the mixture was extracted with ethyl acetate (40 mL×3). The organic layers were combined, washed with brine (20 mL), dried over anhydrous sodium sulfate, filtered, and concentrated under vacuum. The residue was then purified by silica gel chromatography (eluent: petroleum ether/ethyl acetate = 1:2) to afford tert-butyl (1-(4-(benzyloxy)-5-bromo-6-(4-cyano-3-fluorophenyl)pyridin-2-yl)piperidin-4-yl)carbamate with a yield of 81.0%.

ESI-MS m/z = 581.2 $[M+H]^+$.

Step d): preparation of tert-butyl (1-(4-(benzyloxy)-6-(4-cyano-3-fluorophenyl)-5-(3-hydroxy-4-methoxyphenyl)pyridin-2-yl)piperidin-4-yl)carbamat e

Tert-butyl (1-(4-(benzyloxy)-5-bromo-6-(4-cyano-3-fluorophenyl)pyridin-2-yl)piperidin-4-yl)carbamate (1.7 g, 2.9 mmol), 2-methoxy-5-(4,4,5,5-tetramethyl-1,3,2-dioxobenzaldehyde-2-yl)phenol (498 mg, 2.9 mmol), $Cs_2CO_3$ (1.9 g, 5.8 mmol), and $Pd(dppf)Cl_2$ (214 mg, 0.29 mmol) were dissolved in 1,4-dioxane (30 mL) and water (6 mL) was added, the reaction mixture was purged with nitrogen three times, then heated to 100°C and reacted for 2 hours, and the starting materials were completely consumed as indicated by LC-MS. After cooling the reaction liquid to room temperature, water (40 mL) was added, and the mixture was extracted with ethyl acetate (40 mL×3). The organic layers were combined, washed with saturated brine (20 mL), dried over anhydrous sodium sulfate, filtered, and concentrated under vacuum. The residue was then purified by silica gel chromatography (eluent: petroleum ether/ethyl acetate = 1:1) to afford (1-(4-(benzyloxy)-6-(4-cyano-3-fluorophenyl)-5-(3-hydroxy-4-methoxyphenyl)pyridin-2-yl)piperidin-4-yl)carbamat e with a yield of 43.8%.

ESI-MS m/z = 625.3 $[M+H]^+$.

Step e): preparation of tert-butyl (1-(6-(4-cyano-3-fluorophenyl)-4-hydroxy-5-(3-hydroxy-4-methoxyphenyl)pyridin-2-yl) piperidin-4-yl)carbamate

Tert-butyl (1-(4-(benzyloxy)-6-(4-cyano-3-fluorophenyl)-5-(3-hydroxy-4-methoxyphenyl)pyridin-2-yl)piperidin-4-yl)carbamat e (200 mg, 0.32 mmol) was dissolved in methanol (20 mL), and 100 mg of 10% $Pd(OH)_2$ was added. The mixture was purged with hydrogen three times, then reacted at room temperature for 4 hours. The reaction liquid was ultrasonicated for 15 minutes and then filtered, and the filtrate was concentrated under vacuum to afford tert-butyl (1-(6-(4-cyano-3-fluorophenyl)-4-hydroxy-5-(3-hydroxy-4-methoxyphenyl)pyridin-2-yl)piperidin-4-yl)carbamate with a yield of 96.8%.

ESI-MS m/z = 535.2 $[M+H]^+$.

Step f): preparation of methyl 7-((6-(4-((tert-butoxycarbonyl)amino)piperidin-1-yl)-2-(4-cyano-3-fluorophenyl)-3-(3-hydroxy-4-methoxyphenyl)p yridin-4-yl)oxy)heptanoate

Tert-butyl (1-(6-(4-cyano-3-fluorophenyl)-4-hydroxy-5-(3-hydroxy-4-methoxyphenyl)pyridin-2-yl)piperidin-4-yl) carbamate (150 mg, 0.22 mmol) was dissolved in acetonitrile (3 mL), followed by adding diisopropylethylamine (86 mg, 0.66 mmol) and methyl 7-bromoheptanoate (74 mg, 0.33 mmol). The reaction was conducted at 60°C overnight. The mixture was concentrated under vacuum, and the residue was purified by silica gel column (eluent: petroleum ether: ethyl acetate = 1:1) to afford methyl 7-((6-(4-((tert-butoxycarbonyl)amino)piperidin-1-yl)-2-(4-cyano-3-fluorophenyl)-3-(3-hydroxy-4-methoxyphenyl)p yridin-4-yl)oxy)heptanoate with a yield of 87%.

ESI-MS m/z = 677.3 $[M+H]^+$.

Step g): preparation of 7-((6-(4-((tert-butoxycarbonyl)amino)piperidin-1-yl)-2-(4-cyano-3-fluorophenyl)-3-(3-hydroxy-4-methoxyphenyl)p yridin-4-yl)oxy)heptanoic acid

Methyl 7-((6-(4-((tert-butoxycarbonyl)amino)piperidin-1-yl)-2-(4-cyano-3-fluorophenyl)-3-(3-hydroxy-4-methoxyphenyl)p yridin-4-yl)oxy)heptanoate (135 mg, 0.20 mmol) was dissolved in a mixture of $THF/H_2O$ = 5:1 (3 mL) and the reaction was conducted at room temperature overnight, the pH of the system was adjusted to 2~3 with 2 N HCl, followed by extraction with ethyl acetate, and the organic phase was washed once with brine and then dried and

concentrated, and the residue was used directly for the subsequent step.
ESI-MS m/z = 663.3 [M+H]+.

Step h): preparation of tert-butyl (1-(6-(4-cyano-3-fluorophenyl)-5-(3-hydroxy-4-methoxyphenyl)-4-((7-oxo-7-((tetrahydro-2H-pyran-2-yl)oxy)amin o)heptyl)oxy)pyridin-2-yl)piperidin-4-yl)carbamate

7-((6-(4-((Tert-butoxycarbonyl)amino)piperidin-1-yl)-2-(4-cyano-3-fluorophenyl)-3-(3-hydroxy-4-methoxyphe nyl)pyridin-4-yl)oxy)heptanoic acid (132 mg, 0.20 mmol) was dissolved in DMF (3 mL), and HATU (114.1 mg, 0.3 mmol) and O-(tetrahydro-2H-pyran-2-yl)hydroxylamine (47 mg, 0.4 mmol) were added, the reaction was conducted at room temperature for 1 hour, then quenched with water, and extracted with ethyl acetate, and the organic phase was washed twice with water, dried, and concentrated under vacuum. The residue was purified by silica gel column (eluent: petroleum ether: ethyl acetate = 1:1) to afford tert-butyl (1-(6-(4-cyano-3-fluorophenyl)-5-(3-hydroxy-4-methoxyphenyl)-4-((7-oxo-7-((tetrahydro-2H-pyran-2-yl)oxy)amin o)heptyl)oxy)pyridin-2-yl)piperidin-4-yl)carbamate with a yield of 85%.
ESI-MS m/z = 762.4 [M+H]+.

Step i): preparation of 7-((6-(4-aminopiperidin-1-yl)-2-(4-cyano-3-fluorophenyl)-3-(3-hydroxy-4-methoxyphenyl)pyridin-4-yl)oxy)-N-hyd roxyheptanamide hydrochloride

Tert-butyl (1-(6-(4-cyano-3-fluorophenyl)-5-(3-hydroxy-4-methoxyphenyl)-4-((7-oxo-7-((tetrahydro-2H-pyran-2-yl)oxy)amin o)heptyl)oxy)pyridin-2-yl)piperidin-4-yl)carbamate (130 mg, 0.17 mmol) was dissolved in a solution of HCl/EA (3 mL, 4M) and the reaction was conducted at room temperature for 1 hour. Solid was formed in the reaction liquid, and LC-MS analysis indicated that the reaction was completed. The reaction liquid was filtered, and the solid was obtained, then prepared and purified by Prep-HPLC (separation method 1) to afford 7-((6-(4-aminopiperidin-1-yl)-2-(4-cyano-3-fluorophenyl)-3-(3-hydroxy-4-methoxyphenyl)pyridin-4-yl)oxy)-N-hyd roxyheptanamide hydrochloride with a yield of 65.2%.
$^1$H NMR (400 MHz, DMSO-$d_6$) δ 10.41 (d, *J* = 27.2 Hz, 1H), 8.39 (s, 3H), 7.86 (t, *J* = 7.4 Hz, 1H), 7.50 (d, *J* = 10.6 Hz, 1H), 7.29 (dd, *J* = 8.2, 1.4 Hz, 1H), 6.84 (d, *J* = 8.2 Hz, 1H), 6.70 (s, 1H), 6.59 (d, *J* = 2.0 Hz, 1H), 6.45 (dd, *J* = 8.2, 2.0 Hz, 1H), 4.47 (d, *J* = 13.3 Hz, 2H), 4.17 (t, *J* = 6.3 Hz, 2H), 3.79 (s, 3H), 3.39 (dq, *J* = 11.5, 5.5 Hz, 1H), 3.13 (t, *J* = 12.9 Hz, 2H), 2.13 - 2.04 (m, 2H), 1.98 (t, *J* = 7.4 Hz, 2H), 1.68 (tq, *J* = 13.6, 6.8, 5.2 Hz, 4H), 1.51 (p, *J* = 7.4 Hz, 2H), 1.30 (dq, *J* = 19.9, 6.6, 5.3 Hz, 4H).
ESI-MS m/z = 578.3 [M+H]+.
**The compounds of Examples 2-18** were prepared according to the synthetic method of Example **1** (the separation method for the compound: hydrochloride, trifluoroacetate, formate, and free base were prepared by separation methods 1, 2, 3, and 4, respectively), and the structure and characterization data are as follows:

| Example | Chemical name | Structure | $^1$H NMR | MS(M+H)+ |
|---|---|---|---|---|
| 2 | (E)-3-(4-(((6-(4-Aminopi peridin-1-yl)-2-(4-cyano-3-fluorophenyl)-3-(3-hyd roxy-4-methoxyphenyl)p yridin-4-yl)oxy)methyl)p henyl)-N-hydroxyacryla mide hydrochloride | 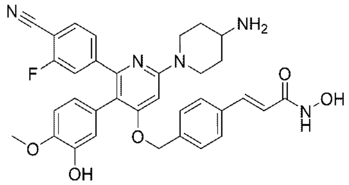 | $^1$H NMR (400 MHz, DMSO-$d_6$) δ ppm : 10.82 (s, 1H), 8.17 (s, 3H), 7.77 (t, *J* = 7.6 Hz, 1H), 7.54 (d, *J* = 8.0 Hz, 2H), 7.43 (d, *J* = 15.8 Hz, 1H), 7.36 (d, *J* = 8.4 Hz, 3H), 7.21 (dt, *J* = 8.2, 1.4 Hz, 1H), 6.80 (d, *J* = 8.4 Hz, 1H), 6.69 (s, 1H), 6.62 (d, *J* = 2.0 Hz, 1H), 6.53 - 6.39 (m, 2H), 5.27 (s, 2H), 4.42 (d, *J* = 13.4 Hz, 2H), 3.74 (s, 3H), 3.38 - 3.23 (m, 1H), 2.98 (t, *J* = 12.8 Hz, 2H), 1.98 (d, *J* = 12.0 Hz, 2H), 1.65 - 1.44 (m, 2H). | 610.3 |

(continued)

| Example | Chemical name | Structure | $^1$H NMR | MS(M+H)$^+$ |
|---|---|---|---|---|
| 3 | 7-(6-(4-Aminopiperidin-1-yl)-2-(4-cyano-3-fluor ophenyl)-3-(3-fluoro-4-methoxyphenyl)pyridin-4-yl)- N-hydroxyheptana mide hydrochloride | 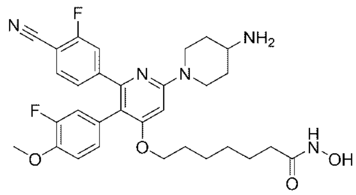 | $^1$H NMR (400 MHz, DMSO-$d_6$) δ ppm: 10.34 (s, 1H), 9.02 (s, 1H), 8.12 (s, 3H), 7.77 (t, *J* = 7.6 Hz, 1H), 7.37 (d, *J* = 10.6 Hz, 1H), 7.16 (d, *J* = 8.2 Hz, 1H), 7.05 - 6.94 (m, 2H), 6.71 (d, *J* = 8.4 Hz, 1H), 6.58 (s, 1H), 4.45 (d, *J* = 13.5 Hz, 2H), 4.06 (t, *J* = 6.3 Hz, 2H), 3.81 (s, 3H), 3.31 (m, 1H), 2.97 (t, *J* = 12.6 Hz, 2H), 1.98 (d, *J* = 12.4 Hz, 2H), 1.91 (t, *J* = 7.4 Hz, 2H), 1.56 (dq, *J* = 12.4, 6.0, 5.2 Hz, 4H), 1.43 (q, *J* = 7.2 Hz, 2H), 1.25 (dp, *J* = 21.0, 6.8 Hz, 4H). | 580.3 |
| 4 | 7-(6-(4-Aminopiperidin-1-yl)-2-(4-cyano-3-fluor ophenyl)-3-(4-(methylsul fonyl) phenyl)pyridin-4-y 1)oxy)-N-hydroxyheptan amide hydrochloride | 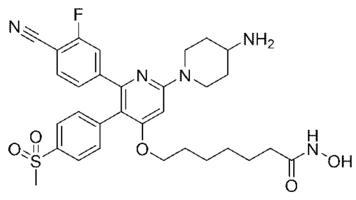 | $^1$H NMR (400 MHz, DMSO-$d_6$) δ ppm: 10.37 (s, 1H), 8.27 (s, 3H), 7.76 (dd, *J* = 16.6, 7.8 Hz, 3H), 7.35 (m, 3H), 7.10 (d, *J* = 8.2 Hz, 1H), 6.63 (s, 1H), 4.48 (d, *J* = 13.4 Hz, 2H), 4.09 (t, *J* = 6.4 Hz, 2H), 3.32 (s, 1H), 3.21 (s, 3H), 3.00 (t, *J* = 12.6 Hz, 2H), 2.01 (d, *J* = 12.2 Hz, 2H), 1.92 (q, *J* = 9.4, 7.4 Hz, 2H), 1.57 (h, *J* = 9.2, 8.4 Hz, 4H), 1.42 (p, *J* = 7.4 Hz, 2H), 1.24 (d, *J* = 15.2 Hz, 4H). | 610.3 |
| 5 | 7-(3-(4-Aminophenyl)-6-(4-aminopiperidin-1-yl)-2-(4-cyano-3-fluorophen yl)pyridin-4-yl)oxy)-N-h ydroxyheptanamide hydrochloride | 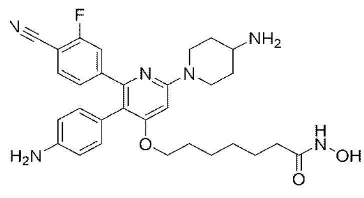 | $^1$H NMR (400 MHz, DMSO-$d_6$) δ ppm: 10.23 (s, 2H), 8.28 - 8.05 (m, 2H), 7.75 (t, *J* = 7.6 Hz, 1H), 7.26 (m, 3H), 7.16 (dd, *J* = 10.8, 8.2 Hz, 3H), 6.59 (s, 1H), 4.46 (d, *J* = 13.4 Hz, 2H), 4.06 (t, *J* = 6.4 Hz, 2H), 3.30 (d, *J* = 15.4 Hz, 1H), 2.97 (t, *J* = 12.8 Hz, 2H), 1.98 (dd, *J* = 13.0, 4.0 Hz, 2H), 1.92 (t, *J* = 7.4 Hz, 2H), 1.55 (q, *J* = 6.0, 5.0 Hz, 4H), 1.43 (p, *J* = 7.4 Hz, 2H), 1.22 (dt, *J* = 18.4, 8.8 Hz, 4H). | 547.3 |

(continued)

| Example | Chemical name | Structure | $^1$H NMR | MS(M+H)$^+$ |
|---|---|---|---|---|
| 6 | 7-(6-(4-Aminopiperidin-1-yl)-2-(4-cyano-3-fluor ophenyl)-3-(3,4-difluoro phenyl) pyridin-4-yl)oxy) -N-hydroxyheptanamide hydrochloride | 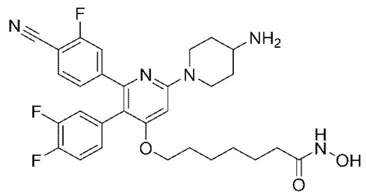 | $^1$H NMR (400 MHz, DMSO-$d_6$) δ ppm: 10.35 (s, 1H), 8.57 - 8.29 (m, 3H), 7.81 (t, *J* = 7.4 Hz, 1H), 7.47 (d, *J* = *10.4* Hz, 1H), 7.31 (dd, *J* = 10.8, 8.4 Hz, 1H), 7.28 - 7.22 (m, 1H), 7.19 (t, *J* = 6.4 Hz, 2H), 6.91 - 6.76 (m, 1H), 6.66 (s, 1H), 4.45 (d, *J* = 13.4 Hz, 2H), 4.12 (t, *J* = 6.2 Hz, 2H), 3.32 (tt, *J* = 10.6, 5.4 Hz, 1H), 3.06 (t, *J* = 12.6 Hz, 2H), 2.11 - 1.98 (m, 2H), 1.92 (t, *J* = 7.4 Hz, 2H), 1.61 (dp, *J* = 19.6, 6.4, 5.2 Hz, 4H), 1.43 (p, *J* = 7.4 Hz, 2H), 1.23 (t, *J* = 10.8 Hz, 4H). | 568.3 |
| 7 | 7-[(6-(4-Aminopiperidin-1-yl)-2-(4-cyano-3-fluor ophenyl)-3-(4-methylphe nyl)pyridin-4-yl)oxy]-N-hydroxyheptanamidehyd rochloride | 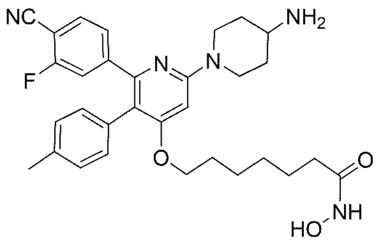 | $^1$H NMR (400 MHz, Methanol-$d_4$) δ ppm 7.72 (d, *J* = 7.6 Hz, 1H), 7.49 (d, *J* = 9.8 Hz, 1H), 7.34 (d, *J* = 8.2 Hz, 1H), 7.13 (d, *J* = 7.6 Hz, 2H), 7.00 (d, *J* = 7.8 Hz, 2H), 6.81 (s, 1H), 4.37 (d, *J* = 13.8 Hz, 2H), 4.30 (d, *J* = 5.8 Hz, 2H), 3.56 (s, 1H), 3.42 (t, *J* = 13.2 Hz, 2H), 2.33 (s, 3H), 2.25 (d, *J* = 13.4 Hz, 2H), 2.04 (d, *J* = 7.6 Hz, 2H), 1.88 (q, *J* = 12.6 Hz, 2H), 1.72 (d, *J* = 8.2 Hz, 2H), 1.56 (t, *J* = 7.6 Hz, 2H), 1.32 (t, *J* = 11.2 Hz, 4H). | 546.3 |

(continued)

| Example | Chemical name | Structure | $^1$H NMR | MS(M +H)$^+$ |
|---|---|---|---|---|
| 8 | 7-[(6-(4-Aminopiperidin-1-yl)-2-(4-cyano-3-fluor ophenyl)-3-(4-methoxyp henyl) pyridin-4-yl)oxy]-N-hydroxy-heptanamide trifluoroacetate | 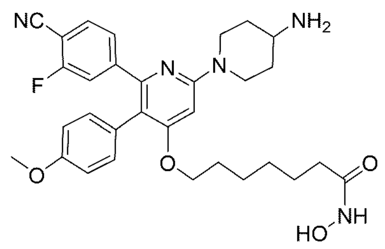 | $^1$H NMR (400 MHz, DMSO-$d_6$) δ ppm 7.57 (t, $J$ = 7.4 Hz, 1H), 7.31 (d, $J$ = 10.4 Hz, 1H), 7.23 (d, $J$ = 8.1 Hz, 1H), 6.98 (d, $J$ = 8.4 Hz, 2H), 6.84 (d, $J$ = 8.3 Hz, 2H), 6.58 (s, 1H), 4.49 (d, $J$ = 13.6 Hz, 2H), 4.11 (t, $J$ = 6.1 Hz, 2H), 3.78 (s, 3H), 3.45 (ddt, $J$ = 11.6, 7.9, 4.3 Hz, 1H), 3.14 (t, $J$ = 12.8 Hz, 2H), 2.13 (dd, $J$ = 12.8, 3.9 Hz, 2H), 2.04 (t, $J$ = 7.4 Hz, 2H), 1.71 (d, $J$ = 1 7.4, 6.4 Hz, 4H), 1.56 (p, $J$ = 7.4 Hz, 2H), 1.43 - 1.20 (m, 4H). | 562.3 |
| 9 | 7-((6-(3-Amino-8-azabic yclo [3.2.1]octan-8-yl)-2-(4-cyano-3-fluorophenyl) -3-(3-hydroxy-4-methox yphenyl) pyridin-4-yl)oxy )-N-hydroxy-heptanamide hydrochloride | 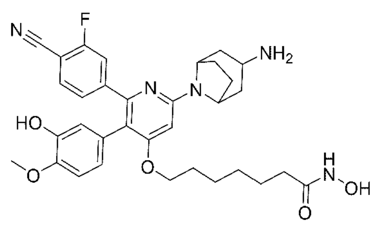 | 2.10 - 1.96 (m, 6H), 1.85 - 1.66 (m, 2H), 1.55 (t, $J$ = 7.4 Hz, 2H), 1.33 (dp, $J$ = 12.6, 7.2 Hz, 4H). | 604.3 |
| 10 | 7-((6-(4-Aminopiperidin-1-yl)-2-(4-cyano-3-fluor ophenyl)-2'-methyl-[3,4'-bipyridin]-4-yl)oxy)-N-h ydroxy-heptanamide hydrochloride | 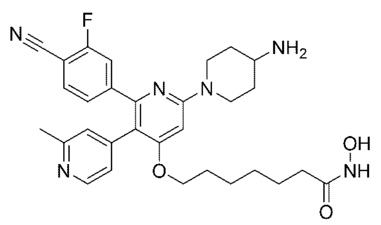 | $^1$H NMR (400 MHz, Methanol-$d_4$) δ 8.12 (dd, $J$ = 7.6, 6.5 Hz, 1H), 7.56 (m,3H), 7.2 (d, $J$ = 8.2 Hz, 2H), 6.9 (s, 1H), 4.65 (d, $J$ = 12.6 Hz, 2H), 4.28 (t, $J$ = 7.2 Hz, 2H), 3.56 (s, 1H), 3.41 (t, $J$ = 12.9 Hz, 2H), 2.69(s, 3H), 2.31 - 2.17 (m, 2H), 2.05 (t, $J$ = 7.3 Hz, 2H), 1.95 - 1.79 (m, 2H), 1.78 - 1.64 (m, 2H), 1.55 (d, $J$ = 7.2 Hz, 2H), 1.31 (dd, $J$ = 10.9, 6.6 Hz, 4H). | 547.3 |

(continued)

| Example | Chemical name | Structure | $^1$H NMR | MS(M+H)$^+$ |
|---|---|---|---|---|
| 11 | 7-((6-(4-Aminopiperidin-1-yl)-3-(4-chlorophenyl)-2-(4-cyano-3-fluorophen yl)pyridin-4-yl)oxy)-N-h ydroxyheptanamide hydrochloride | 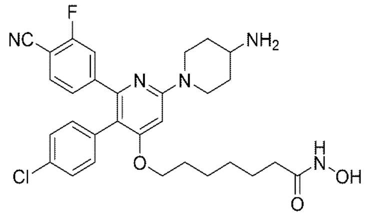 | $^1$H NMR (400 MHz, Methanol-$d_4$) δ 7.73 (dd, *J* = 7.9, 6.5 Hz, 1H), 7.53 (d, *J* = 9.6 Hz, 1H), 7.32 (m, 3H), 7.11 (d, *J* = 8.2 Hz, 2H), 6.80 (s, 1H), 4.37 (d, *J* = 13.7 Hz, 2H), 4.28 (t, *J* = 6.1 Hz, 2H), 3.56 (d, *J* = 11.7 Hz, 1H), 3.41 (t, *J* = 12.9 Hz, 2H), 2.31 - 2.17 (m, 2H), 2.05 (t, *J* = 7.3 Hz, 2H), 1.95 - 1.79 (m, 2H), 1.78 - 1.64 (m, 2H), 1.55 (p, *J* = 7.2 Hz, 2H), 1.31 (qd, *J* = 10.9, 6.6 Hz, 4H). | 566.2 |
| 12 | 7-((6-(4-Aminopiperidin-1-yl)-2-(4-cyanophenyl)-3-(3-hydroxy-4-methoxy phenyl) pyridin-4-yl)oxy) -N-hydroxy-heptanamide (free ) | 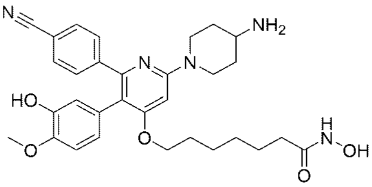 | $^1$H NMR (400 MHz, DMSO-$d_6$) δ 7.71 (d, *J* = 8.1 Hz, 2H), 7.45 (d, *J* = 8.1 Hz,2H), 6.80 (d, *J* = 8.3 Hz, 1H), 6.55 (s, 1H), 6.49 (s, 1H), 6.41 (d, *J* = 8.0 Hz, 1H), 4.34 (d, *J* = 12.4 Hz, 2H), 4.07 (t, *J* = 6.4 Hz, 2H), 3.78 (s, 3H), 2.96 (t, *J* = 12.3 Hz, 2H), 2.86 (s, 1H), 1.98 (t, *J* = 7.4 Hz, 2H), 1.83 (d, *J* = 12.2 Hz, 2H), 1.63 (p, *J* = 6.6 Hz, 2H), 1.51 (p, *J* = 7.4 Hz, 2H), 1.30 (dq, *J* = 14.2, 9.0, 8.1 Hz, 6H). | 560.3 |
| 13 | 7-((6-(4-Aminopiperidin-1-yl)-2-(4-cyano-3-fluor ophenyl)-3-(2-(dimethyla mino) pyrimidin-5-yl)pyri din-4-yl) oxy)-N-hydroxy heptanamide formate | 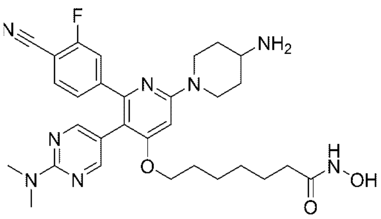 | $^1$H NMR (400 MHz, DMSO-$d_6$) δ ppm: 8.40 (s, 1H), 7.98 (s, 2H), 7.81 (t, *J* = 7.6 Hz, 1H), 7.43 (d,*J* = 10.6 Hz, 1H), 7.21 (d, *J* = 8.2 Hz, 1H), 6.54 (s, 1H), 4.39 (d, *J* = 13.2 Hz, 2H), 4.05 (t, *J* = 6.4 Hz, 2H), 3.12 (s, 2H), 3.08 (s, 6H), 2.93 (t, *J* = 12.6 Hz, 2H), 1.90 (q, *J* = 6.8 Hz, 3H), 1.61 (p, *J* = 6.6 Hz, 2H), 1.43 (tt, *J* = 12.2, 5.6 Hz, 4H), 1.25 (dd, *J* = 20.0, 7.2 Hz, 4H). | 577.3 |

(continued)

| Example | Chemical name | Structure | $^1$H NMR | MS(M+H)$^+$ |
|---|---|---|---|---|
| 14 | 7-((6-(4-Aminopiperidin-1-yl)-2-(4-cyano-3-fluor ophenyl)-3-(2-methyl-2H -indazol-5-yl)pyridin-4-y l)oxy)-N-hydroxyheptan amide formate | 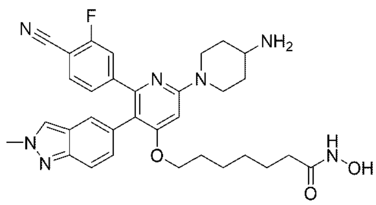 | $^1$H NMR (400 MHz, DMSO-$d_6$) δ ppm: 8.40 (d, *J* = 10.2 Hz, 1H), 8.17 (s, 1H), 7.70 - 7.61 (m, 1H), 7.47 (d, *J* = 8.8 Hz, 1H), 7.32 (dd, *J* = 10.8, 1.4 Hz, 1H), 7.25 (s, 1H), 7.15 (dd, *J = 8.2,* 1.4 Hz, 1H), 6.96 (dd, *J* = 8.8, 1.6 Hz, 1H), 6.54 (s, 1H), 4.39 (d, *J* = 13.4 Hz, 2H), 4.12 (s, 3H), 4.03 (t, *J* = 6.2 Hz, 2H), 3.57 (s, 3H), 2.93 (t, *J* = 12.0 Hz, 2H), 2.17 (t, *J* = 7.4 Hz, 2H), 1.89 (s, 2H), 1.53 (p, *J* = 6.4 Hz, 2H), 1.40 (p, *J* = 7.4 Hz, 4H), 1.20 (dq, *J* = 22.2, 8.2 Hz, 4H). | 586.3 |
| 15 | 7-((6-(5-Amino-2-azabic yclo [2.2.1]heptan-2-yl)-2 -(4-cyano-3-fluorophenyl )-3-(3-hydroxy-4-methox yphenyl) pyridin-4-yl)oxy )-N-hydroxyheptanamide hydrochloride | 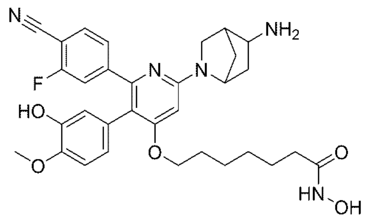 | $^1$H NMR (400 MHz, Methanol-$d_4$) δ 7.71 (ddd, *J* = 8.1, 6.6, 3.0 Hz, 1H), 7.63 - 7.41 (m, 1H), 7.41 - 7.18 (m, 1H), 6.79 (d, *J* = 8.2 Hz, 1H), 6.58 (s, 1H), 6.44 (s, 2H), 4.78 (s, 1H), 4.24 (dp, *J* = 13.8, 4.4 Hz, 2H), 3.90 (dq, *J* = 14.6, 5.2, 4.7 Hz, 2H), 3.81 (s, 4H), 3.13 (td, *J* = 3.6, 1.8 Hz, 1H), 2.53 - 2.31 (m, 1H), 2.15 (dd, *J* = 11.2, 11.0 Hz, 1H), 2.03 (t, *J* = 7.4 Hz, 3H), 1.94 - 1.79 (m, 2H), 1.72 (p, *J* = 6.4 Hz, 2H), 1.55 (p, *J* = 7.4 Hz, 2H), 1.31 (dq,*J* = 10.6, 6.8, 6.0 Hz, 4H). | 590.3 |

(continued)

| Example | Chemical name | Structure | $^1$H NMR | MS(M+H)$^+$ |
|---|---|---|---|---|
| 16 | 7-((6-(4-Aminopiperidin-1-yl)-2-(4-cyano-3-meth ylphenyl)-3-(3-hydroxy-4-methoxyphenyl)pyridi n-4-yl)oxy)-N-hydroxyh eptanamide hydrochloride | 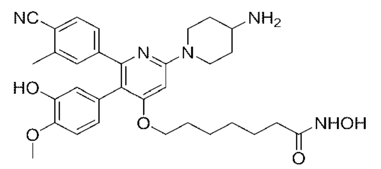 | $^1$H NMR (400 MHz, Methanol-$d_4$) δ ppm 7.61 (d, *J* = 8.0 Hz, 1H), 7.48 (s, 1H), 7.28 (d, *J* = 8.2 Hz, 1H), 6.83 - 6.73 (m, 2H), 6.59 (d, *J* = 2.0 Hz, 1H), 6.45 (dd, *J* = 8.2, 2.2 Hz, 1H), 4.37 - 4.23 (m, 4H), 3.82 (s, 3H), 3.54 (dt, *J* = 11.6, 7.0 Hz, 1H), 3.39 (t, *J* = 12.8 Hz, 2H), 2.49 (s, 3H), 2.27 - 2.19 (m, 2H), 2.05 (t, *J* = 7.4 Hz, 2H), 1.93 - 1.79 (m, 2H), 1.74 (t, *J* = 6.6 Hz, 2H), 1.56 (p, *J* = 7.4 Hz, 2H), 1.34 (dt, *J* = 22.6, 7.6 Hz, 4H). | 574.3 |
| 17 | 7-((6-(4-Aminopiperidin-1-yl)-2-(4-cyano-3-fluor ophenyl)-3-(4-(2-hydrox yethoxy) phenyl)pyridin-4-yl)oxy)-N-hydroxyhept anamide formate | 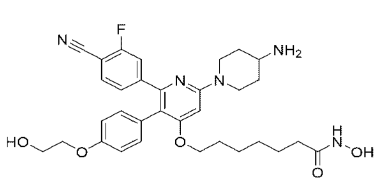 | $^1$H NMR (400 MHz, Methanol-$d_4$) δ 8.55 (s, 1H), 7.49 (t, *J* = 7.4 Hz, 1H), 7.29 - 7.16 (m, 2H), 6.96 (d, *J* = 8.6 Hz, 2H), 6.86 (d, *J* = 8.4 Hz, 2H), 6.46 (s, 1H), 4.52 (d, *J* = 13.4 Hz, 2H), 4.12 - 3.97 (m, 4H), 3.87 (t, *J* = 4.7 Hz, 2H), 3.00 (t, *J* = 12.2 Hz, 2H), 2.03 (q, *J* = 8.2, 7.7 Hz, 4H), 1.77 - 1.49 (m, 6H), 1.31 (dp, *J*= 20.6, 7.5 Hz, 5H). | 592.3 |
| 18 | 7-((6-(4-Aminopiperidin-1-yl)-3-(3-hydroxy-4-me thoxyphenyl)-2'-methyl-[ 2,4'-bipyridin]-4-yl)oxy)-N-hydroxyheptanamide diformate | 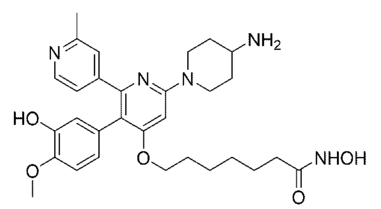 | $^1$H NMR (400 MHz, Methanol-$d_4$) δ ppm 8.55 (s, 2H), 8.16 (d, *J* = 5.4 Hz, 1H), 7.20 (s, 1H), 7.07 (dd, *J* = 5.4, 1.8 Hz, 1H), 6.79 (d, *J* = 8.2 Hz, 1H), 6.58 (d, *J* = 2.2 Hz, 1H), 6.49 - 6.37 (m, 2H), 4.53 (d, *J* = 13.6 Hz, 2H), 4.03 (t, *J* = 6.2 Hz, 2H), 3.83 (s, 3H), 3.00 (t, *J* = 12.6 Hz, 2H), 2.39 (s, 3H), 2.04 (q, *J* = 7.8 Hz, 4H), 1.61 (m, 6H), 1.32 (dq, *J*= 23.6, 7.4 Hz, 4H) . | 550.3 |

**Example** 19

Preparation of 7-(5-(5-(4-aminopiperidin-1-yl)-7-(4-cyano-3-fluorophenyl)imidazo[1,2-c]pyrimidin-8-yl)-2-methoxyphenoxy)-N-h ydroxyheptanamide hydrochloride

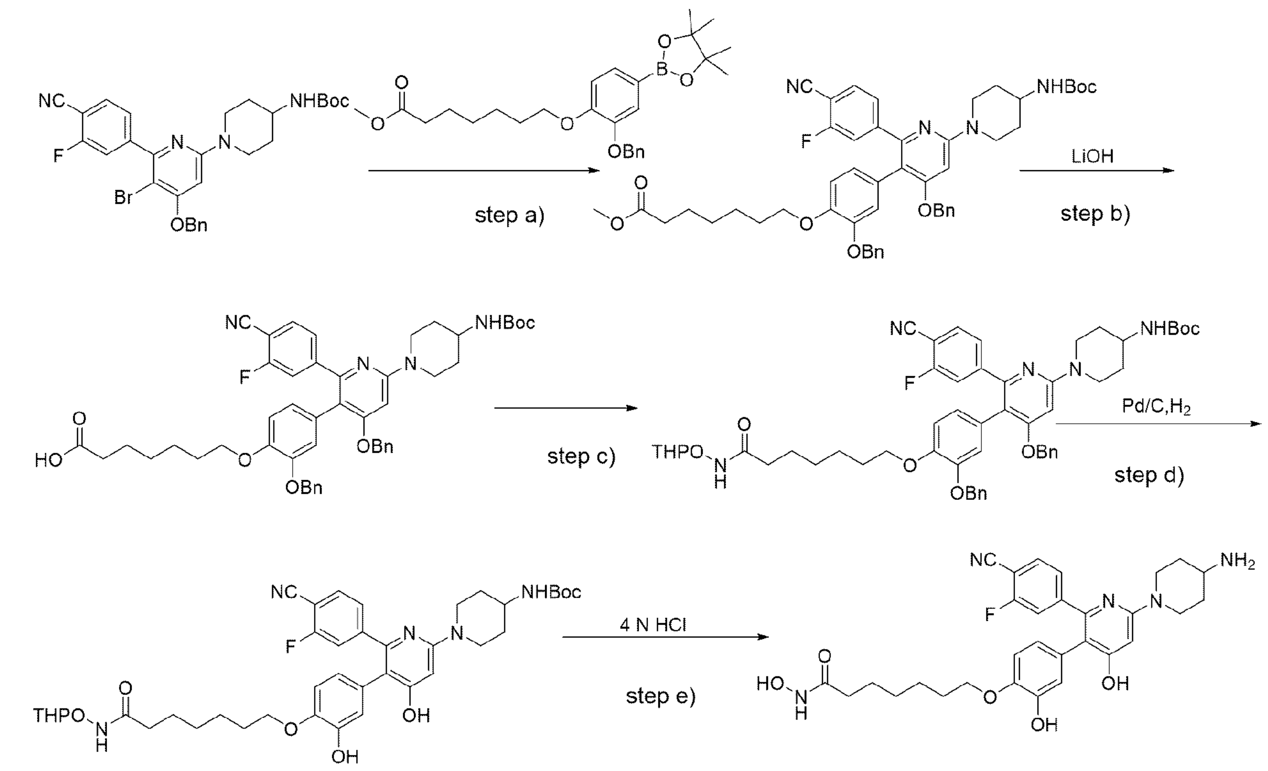


Step a): preparation of methyl 7-(2-(benzyloxy)-4-(4-(benzyloxy)-6-(4-(tert-butoxycarbonyl)amino)piperidin-1-yl)-2-(4-cyano-3-fluorophenyl)pyri din-3-yl)phenoxy)heptanoate

The product of Example 1 Step c): tert-butyl (1-(4-(benzyloxy)-5-bromo-6-(4-cyano-3-fluorophenyl)pyridin-2-yl) piperidin-4-yl)carbamate (200 mg, 345 μmol), methyl 7-(2-(benzyloxy)-4-(4,4,5,5-tetramethyl-1,3,2-dioxobenzaldehyde-2-yl)phenoxy)heptanoate (241 mg, 517 μmol), $Cs_2CO_3$ (225 mg, 690 μmol), Pd(dppf)$Cl_2$ (26 mg, 35 μmol), 1,4-dioxane (10 mL), and $H_2O$ (2.5 mL) were added in a reaction flask, and the reaction was stirred at 120°C for 1 hour. The mixture was concentrated to dryness under reduced pressure, and the residue was then purified by silica gel chromatography (eluent: petroleum ether/ethyl acetate = 1/2) to afford methyl 7-(2-(benzyloxy)-4-(4-(benzyloxy)-6-(4-(tert-butoxycarbonyl)amino)piperidin-1-yl)-2-(4-cyano-3-fluorophenyl)pyri din-3-yl)phenoxy)heptanoate with a yield of 58.0%.
ESI-MS(m/z) =843.4 $[M+H]^+$.

Step b): preparation of 2-chloro-6-methylpyrimidine-4-carboxylic acid

Methyl 7-(2-(benzyloxy)-4-(4-(benzyloxy)-6-(4-(tert-butoxycarbonyl)amino)piperidin-1-yl)-2-(4-cyano-3-fluorophenyl)pyri din-3-yl)phenoxy)heptanoate (169 mg, 200 μmol), lithium hydroxide monohydrate (42 mg, 1.0 mmol), tetrahydrofuran (2 mL), isopropanol (2 mL), and water (1 mL) were added to a reaction flask and stirred for reaction at room temperature for 12 hours. Under stirring in an ice bath, 1N concentrated hydrochloric acid was slowly added dropwise to adjust the pH to 3~4, and water (10 mL) was added, followed by extraction with ethyl acetate (10 mL×3), and the organic phases were combined, washed with saturated brine (10 mL×2), concentrated to dryness under reduced pressure to afford 7-(2-(benzyloxy)-4-(4-(benzyloxy)-6-(4-(tert-butoxycarbonyl)amino)piperidin-1-yl)-2-(4-cyano-3-fluorophenyl)pyri din-3-yl)phenoxy)heptanoic acid which was used directly for the next step.
ESI-MS(m/z) = 829.4$[M+H]^+$.

Step c): preparation of tert-butyl (1-(4-(benzyloxy)-5-(3-(benzyloxy)-4-((7-oxo-7-((tetrahydro-2H-pyran-2-yl)oxy)amino) heptyl)oxy)phenyl)-6-(4-cy ano-3-fluorophenyl)pyridin-2-yl)piperidin-4-yl)carbamate

7-(2-(Benzyloxy)-4-(4-(benzyloxy)-6-(4-(tert-butoxycarbonyl)amino)piperidin-1-yl)-2-(4-cyano-3-fluoropheny l) pyridin-3-yl)phenoxy)heptanoic acid (99 mg, 200 μmol), O-(tetrahydro-2H-pyran-2-yl)hydroxylamine (47 mg, 400 μmol), DIEA (52 mg, 400 μmol), and DMF (2 mL) were added to a reaction flask, and HATU (114 mg, 300 μmol) was added under stirring at room temperature, and the reaction was maintained for 1 hour at room temperature. After the reaction was completed, water (10 mL) was added to quench the reaction, followed by extraction with ethyl acetate (20 mL × 2), and the

organic phases were combined, washed sequentially with saturated sodium bicarbonate aqueous solution (20 mL ×1) and saturated brine (10 mL × 2), dried over anhydrous sodium sulfate, filtered, and the filtrate was concentrated under reduced pressure, and the residue was purified by silica gel chromatography (eluent: petroleum ether/ethyl acetate = 1/1) to afford tert-butyl (1-(4-(benzyloxy)-5-(3-(benzyloxy)-4-((7-oxo-7-((tetrahydro-2H-pyran-2-yl)oxy)amino)heptyl)oxy) phenyl)-6-(4-cy ano-3-fluorophenyl)pyridin-2-yl)piperidin-4-yl)carbamate with a yield of 62.7%.

ESI-MS(m/z) =928.5 $[M+H]^+$.

Step d): preparation of tert-butyl (1-(6-(4-cyano-3-fluorophenyl)-4-hydroxy-5-(3-hydroxy-4-((7-oxo-7-((tetrahydro-2H-pyran-2-yl)oxy)amino)heptyl) oxy)phenyl)pyridin-2-yl)piperidin-4-yl)carbamate

Tert-butyl (1-(4-(benzyloxy)-5-(3-(benzyloxy)-4-((7-oxo-7-((tetrahydro-2H-pyran-2-yl)oxy)amino)heptyl)oxy) phenyl)-6-(4-cy ano-3-fluorophenyl)pyridin-2-yl)piperidin-4-yl)carbamate (117 mg, 126 μmol), palladium on carbon (25 mg, 5%), and ethanol (5 mL) were sequentially added to a reaction flask, stirred until dissolved, and the mixture was purged with hydrogen three times, and stirred at room temperature for 2 hours under hydrogen atmosphere. After the reaction was completed, it was filtered, and the filtrate was concentrated under reduced pressure to afford tert-butyl (1-(6-(4-cyano-3-fluorophenyl)-4-hydroxy-5-(3-hydroxy-4-((7-oxo-7-((tetrahydro-2H-pyran-2-yl)oxy)amino)heptyl) oxy) phenyl)pyridin-2-yl)piperidin-4-yl)carbamate with a yield of 85.0%.

ESI-MS(m/z) =748.4$[M+H]^+$.

Step e): preparation of 7-(4-(6-(4-aminopiperidin-1-yl)-2-(4-cyano-3-fluorophenyl)-4-hydroxypyridin-3-yl)-2-hydroxyphenoxy)-N-hydroxy heptanamide hydrochloride

Tert-butyl (1-(6-(4-cyano-3-fluorophenyl)-4-hydroxy-5-(3-hydroxy-4-((7-oxo-7-((tetrahydro-2H-pyran-2-yl)oxy) amino)heptyl) oxy)phenyl)pyridin-2-yl)piperidin-4-yl)carbamate (80 mg, 107 μmol)was added to a reaction flask, followed by adding hydrogen chloride solution in ethyl acetate (4M, 2.5 mL), and the mixture was stirred at room temperature for 1 hour, resulting in the precipitation of a significant amount of solid. After concentrating under reduced pressure, the crude product was purified by Prep-HPLC (separation method 1) to afford 7-(4-(6-(4-aminopiperidin-1-yl)-2-(4-cyano-3-fluorophenyl)-4-hydroxypyridin-3-yl)-2-hydroxyphenoxy)-N-hydroxy heptanamide hydrochloride with a yield of 35.8%.

$^1H$ NMR (400 MHz, Methanol-$d_4$) δ 7.63 (t, *J* = 7.2 Hz, 1H), 7.39 (d, *J* = 9.6 Hz, 1H), 7.23 (d, *J* = 7.8 Hz, 1H), 6.70 (d, *J* = 8.2 Hz, 1H), 6.53 (d, *J* = 6.2 Hz, 2H), 6.39 (d, *J* = 8.0 Hz, 1H), 4.08 (d, *J* = 13.2 Hz, 2H), 3.89 (t, *J = 6.4* Hz, 2H), 3.43 (td, *J* = 11.6, 11.2, 5.4 Hz, 1H), 3.27 (d, *J* = 12.6 Hz, 2H), 2.21 - 2.08 (m, 2H), 2.03 (t, *J* = 7.4 Hz, 2H), 1.72 (dp, *J* = 21.6, 7.6, 6.8 Hz, 4H), 1.62 - 1.48 (m, 2H), 1.38 - 1.28 (m, 4H).

ESI-MS(m/z) =564.3 $[M+H]^+$.

**Example** 20

Preparation of 7-(5-(5-(4-aminopiperidin-1-yl)-7-(4-cyano-3-fluorophenyl)imidazo[1,2-c]pyrimidin-8-yl)-2-methoxyphenoxy)-N-h ydroxyheptanamide hydrochloride

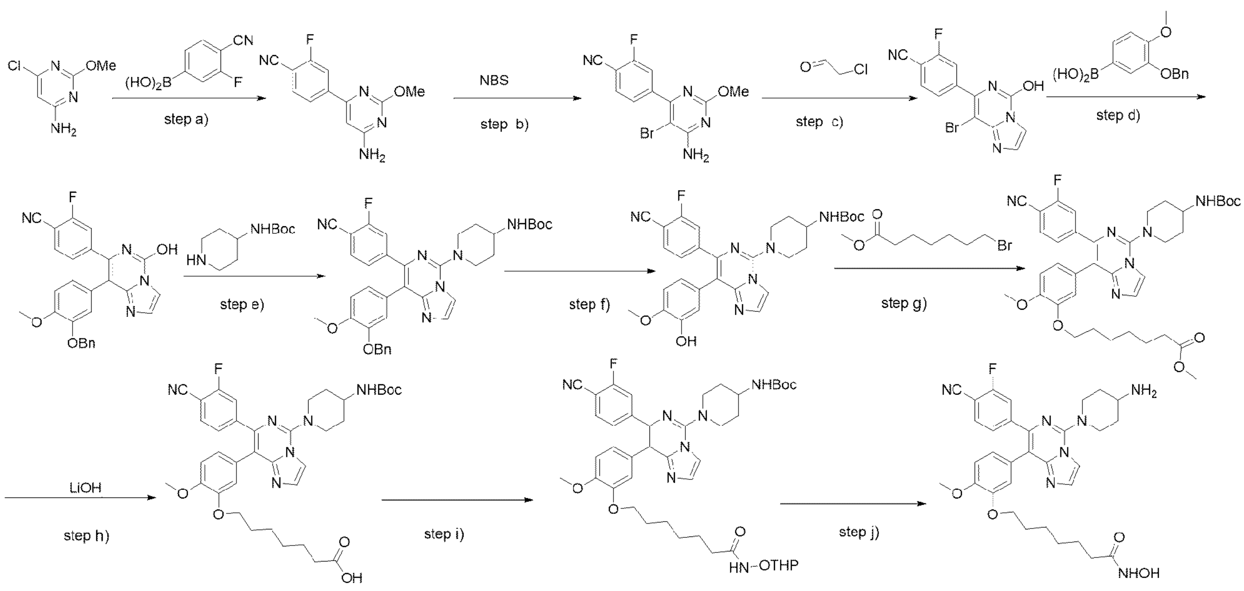


Step a): preparation of 4-(6-amino-2-methoxypyrimidin-4-yl)-2-fluorobenzonitrile

6-Chloro-2-methoxypyrimidin-4-amine (3.0 g, 0.03 mol), $Na_2CO_3$ (9.9 g, 0.09 mol), (4-cyano-3-fluorophenyl) boronic acid (7.8 g, 0.05 mol), Pd(aphos)$_2$Cl$_2$ (4.3 g, 6 mmol) were dissolved in a mixture of 1,4-dioxane:water = 5:1 (150 mL), and nitrogen was bubbled through the mixture and the mixture was protected under nitrogen atmosphere. The reaction was heated in an oil bath to 95°C. After the reaction was completed as indicated by LCMS, the reaction liquid was concentrated under vacuum, then dissolved in ethyl acetate (20 mL), washed with water (20 mL), and the aqueous phase was extracted with ethyl acetate (20 mL × 3), and the organic phases were washed with saturated brine, dried over anhydrous sodium sulfate, filtered, and concentrated, and the residue was purified by silica gel chromatography (eluent: petroleum ether/ethyl acetate = 2/1) to afford 4-(6-amino-2-methoxypyrimidin-4-yl)-2-fluorobenzonitrile with a yield of 88.6%.

ESI-MS m/z: 245.1 $[M+H]^+$.

Step b): preparation of 4-(6-amino-5-bromo-2-methoxypyrimidin-4-yl)-2-fluorobenzonitrile

4-(6-Amino-2-methoxypyrimidin-4-yl)-2-fluorobenzonitrile (2.0 g, 8 mmol) was dissolved in dry DMSO (10 mL) and anhydrous acetonitrile (50 mL). Under nitrogen protection and cooling in an ice bath, NBS (1.45 g, 8 mmol) was added, and the reaction was continued for 2 hours. After the reaction was completed as indicated by LCMS, water (40 mL) was added to quench the reaction, followed by extraction with ethyl acetate (50 mL × 3), the organic phases were washed with saturated brine, dried with anhydrous sodium sulfate, filtered, and concentrated. The residue was purified by silica gel chromatography (eluent: petroleum ether/ethyl acetate = 3/1) to afford 4-(6-amino-5-bromo-2-methoxypyrimidin-4-yl)-2-fluorobenzonitrile with a yield of 78.5%.

ESI-MS m/z: 323.0$[M+H]^+$.

Step c ): preparation of 4-(8-bromo-5-hydroxyimidazopyrimidin-7-yl)-2-fluorobenzonitrile

4-(6-Amino-5-bromo-2-methoxypyrimidin-4-yl)-2-fluorobenzonitrile (500 mg, 1.55 mmol) was dissolved in isopropanol (13 mL), followed by adding chloroacetaldehyde (6.09 g, 31.06 mmol), the reaction liquid was purged with nitrogen and then heated at 110°C overnight, with the reaction progress monitored by TLC and LCMS. After the reaction was completed as indicated by LCMS, the reaction liquid was concentrated under vacuum, and the residue was dissolved in dichloromethane (5 mL). The residue was purified by silica gel chromatography (eluent: methanol/dichloromethane = 1/20) to afford 4-(8-bromo-5-hydroxyimidazopyrimidin-7-yl)-2-fluorobenzonitrile with a yield of 85.7%.

$^1$H NMR (400 MHz, DMSO-$d_6$) δ ppm 12.28 (s, 1H), 8.14 (dt, *J* = 9.0, 4.6 Hz, 1H), 7.97 (d, *J* = 2.6 Hz, 1H), 7.84 (dd, *J* = 10.2, 2.6 Hz, 1H), 7.65 (dd, *J* = 7.8, 2.4 Hz, 1H), 7.50 (d, *J* = 2.4 Hz, 1H).

ESI-MS m/z: 333.0$[M+H]^+$.

Step d ): preparation of 4-(8-(3-(benzyloxy)-4-methoxyphenyl)-5-hydroxyimidazo[1,2-c]pyrimidin-7-yl)-2-fluorobenzonitrile

4-(8-Bromo-5-hydroxyimidazopyrimidin-7-yl)-2-fluorobenzonitrile (380 mg, 1.14 mmol), (3-(benzyloxy)-4-methoxyphenyl)boronic acid (292 mg, 1.72 mmol), Pd(dppf)$Cl_2$ (167 mg, 0.21 mmol), and $Na_2CO_3$ (243 mg, 2.29 mmol) were dissolved in 1,4-dioxane (15 mL) and water (3 mL) was added. The mixture was bubbled with $N_2$ and protected with $N_2$, and reacted in a microwave reactor at 105°C for 30 minutes. After the reaction was completed as indicated by LCMS, the mixture was extracted with ethyl acetate (10 mL × 3), washed with 10 mL saturated brine, dried with anhydrous sodium sulfate, filtered, and concentrated. The residue was purified by silica gel chromatography (eluent: methanol/dichloromethane = 1/20) to afford 4-(8-(3-(benzyloxy)-4-methoxyphenyl)-5-hydroxyimidazo[1,2-c]pyrimidin-7-yl)-2-fluorobenzonitrile with a yield of 89.7%.
ESI-MS m/z: 467. 1[M+H] +.

Step e ): preparation of tert-butyl (1-(8-(3-(benzyloxy)-4-methoxyphenyl)-7-(4-cyano-3-fluorophenyl)imidazo[1,2-c]pyrimidin-5-yl)piperidin-4-yl)car bamate

4-(8-(3-(Benzyloxy)-4-methoxyphenyl)-5-hydroxyimidazo[1,2-c]pyrimidin-7-yl)-2-fluorobenzonitrile (200 mg, 0.530 mmol), tert-butylpiperidine-4-carbamate (318 mg, 1.57 mmol), and BOP Reagent (352 mg, 0.795 mmol) were dissolved in 16 mL anhydrous acetonitrile, then DIPEA (206 mg, 1.59 mmol) was added, the reaction liquid was bubbled with nitrogen and heated to 60°C and reacted overnight under nitrogen protection. The progress of the reaction was monitored by TLC and LCMS. After the reaction was completed, the the reaction liquid was cooled and concentrated under vacuum, and the concentrate was directly purified by thin-layer chromatography on a preparative plate (eluent: methanol/dichloromethane = 1/10) to afford tert-butyl (1-(8-(3-(benzyloxy)-4-methoxyphenyl)-7-(4-cyano-3-fluorophenyl)imidazo[1,2-c]pyrimidin-5-yl)piperidin-4-yl)car bamate with a yield of 58.4%.
ESI-MS m/z: 649.3 [M+H] +.

Step f): preparation of tert-butyl (1-(7-(4-cyano-3-fluorophenyl)-8-(3-hydroxy-4-methoxyphenyl)imidazo[1,2-c]pyrimidin-5-yl)piperidin-4-yl)carba mate

Tert-butyl (1-(8-(3-(benzyloxy)-4-methoxyphenyl)-7-(4-cyano-3-fluorophenyl)imidazo[1,2-c]pyrimidin-5-yl)piperidin-4-yl)car bamate (200 mg, 309 μmol), palladium on carbon (50 mg, 5%), and ethanol (10 mL) were sequentially added to a reaction flask, the mixture was purged with hydrogen three times and stirred at room temperature for 2 hours under nitrogen protection. After the reaction was completed, the mixture was filtered, and the filtrate was concentrated under reduced pressure to afford tert-butyl (1-(7-(4-cyano-3-fluorophenyl)-8-(3-hydroxy-4-methoxyphenyl)imidazo[1,2-c]pyrimidin-5-yl)piperidin-4-yl)carba mate with a yield of 83.0%.
ESI-MS(m/z) = 559.2[M+H] +.

Step g): preparation of tert-butyl (1-(5-bromo-6-(4-cyano-3-fluorophenyl)-4-methyl-3-nitropyridin-2-yl)piperidin-4-yl) carbamate

Tert-butyl (1-(7-(4-cyano-3-fluorophenyl)-8-(3-hydroxy-4-methoxyphenyl)imidazo[1,2-c]pyrimidin-5-yl)piperidin-4-yl)carba mate (143 mg, 256 μmol), methyl 7-bromoheptanoate (114 mg, 512 μmol), $K_2CO_3$ (72 mg, 512 μmol), and acetonitrile (5 mL) were added to a reaction flask. The mixture was stirred for reaction at 75°C for 12 hours and concentrated under reduced pressure, and the residue was then purified by silica gel chromatography (eluent: petroleum ether/ethyl acetate = 8/3) to afford methyl 7-(5-(5-(4-(tert-butoxycarbonyl)amino)piperidin-1-yl)-7-(4-cyano-3-fluorophenyl)imidazo[1,2-c]pyrimidin-8-yl)-2-methoxyphenoxy)heptanoate with a yield of 57.0%.
ESI-MS(m/z) =701.3 [M+H]+.

Step h): preparation of tert-butyl (1-(5-bromo-6-(4-cyano-3-fluorophenyl)-4-methyl-3-nitropyridin-2-yl)piperidin-4-yl) carbamate

Methyl 7-(5-(5-(4-(tert-butoxycarbonyl)amino)piperidin-1-yl)-7-(4-cyano-3-fluorophenyl)imidazo[1,2-c]pyrimidin-8-yl)-2-methoxyphenoxy)heptanoate (102 mg, 146 μmol), lithium hydroxide monohydrate (31 mg, 730 μmol), THF (2 mL), isopropanol (2 mL), and water (2 mL) were added to a reaction flask. The mixture was stirred for reaction at room temperature for 8 hours. After the reaction was completed, 0.5N HCl aqueous solution (10 mL) was added, and the mixture was extracted with ethyl acetate (10 mL × 3), and the organic phases were combined and washed with saturated brine (10 mL × 2), concentrated under reduced pressure. The residue was purified by silica gel chromatography (eluent: petroleum ether/ethyl acetate = 8/3) to afford 7-(5-(5-(4-((tert-butoxycarbonyl)amino)piperidin-1-yl)-7-(4-cyano-3-fluorophenyl)imidazo[1,2-c]pyrimidin-8-yl)-2-methoxyphenoxy)heptanoic acid with a yield of 89.0%.

ESI-MS(m/z) =687.3 [M+H] +.

Step i): preparation of tert-butyl (1-(3-amino-5-bromo-6-(4-cyano-3-fluorophenyl)-4-methylpyridin-2-yl)piperidin-4-yl) carbamate

7-(5-(5-(4-((Tert-butoxycarbonyl)amino)piperidin-1-y1)-7-(4-cyano-3-fluorophenyl)imidazo[1,2-c]pyrimidin-8-yl)-2-methoxyphenoxy)heptanoic acid (89 mg, 139 μmol), O-(tetrahydro-2H-pyran-2-yl)hydroxylamine (33 mg, 278 μmol), DIEA (36 mg, 278 μmol), and DMF (2 mL) were added to a reaction flask, and HATU (79 mg, 208 μmol) was added under stirring at room temperature. The reaction was maintained at room temperature for 1 hour. After the reaction was completed, water (10 mL) was added to quench the reaction, followed by extraction with ethyl acetate (20 mL × 2), and the organic phases were combined and then washed sequentially with saturated sodium bicarbonate aqueous solution (20 mL × 2) and saturated brine (10 mL × 2). The organic phases were dried with anhydrous sodium sulfate, filtered and concentrated under reduced pressure, and the residue was purified by silica gel chromatography (eluent: petroleum ether/ethyl acetate = 1/1) to afford tert-butyl (1-(7-(4-cyano-3-fluorophenyl)-8-(4-methoxy-3-((7-oxo-7-((tetrahydro-2H-pyran-2-yl)oxy)amino)heptyl)oxy)pheny l)imidazo[1,2-c]pyrimidin-5-yl)piperidin-4-yl)carbamate with a yield of 72.7%.

ESI-MS(m/z) =786.4 [M+H]+.

Step j): preparation of 7-(5-(5-(4-aminopiperidin-1-yl)-7-(4-cyano-3-fluorophenyl)imidazo[1,2-c]pyrimidin-8-yl)-2-methoxyphenoxy)-N-h ydroxyheptanamide hydrochloride

Tert-butyl (1-(7-(4-cyano-3-fluorophenyl)-8-(4-methoxy-3-((7-oxo-7-((tetrahydro-2H-pyran-2-yl)oxy)amino) heptyl)oxy)pheny l)imidazo[1,2-c]pyrimidin-5-yl)piperidin-4-yl)carbamate (79 mg, 101 μmol)was added to a reaction flask, followed by adding hydrogen chloride solution in ethyl acetate (4M, 2.5 mL), and the mixture was stirred at room temperature for 1 hour, resulting in the precipitation of a significant amount of solid. After concentrating under reduced pressure, the crude product was purified by Prep-HPLC (separation method 1) to afford 7-(5-(5-(4-aminopiperidin-1-yl)-7-(4-cyano-3-fluorophenyl)imidazo[1,2-c]pyrimidin-8-yl)-2-methoxyphenoxy)-N-h ydroxyheptanamide hydrochloride with a yield of 22.5%.

$^1$H NMR (400 MHz, DMSO-$d_6$) δ ppm 10.35 (s, 1H), 8.24-8.19 (m, 3H), 7.99 (s, 1H), 7.87 (dd, J = 8.2, 6.8 Hz, 1H), 7.55 (dd, J = 10.6, 1.6 Hz, 1H), 7.33 (dd, J = 8.2, 1.6 Hz, 1H), 7.03 (d, J = 8.4 Hz, 1H), 6.97 - 6.75 (m, 2H), 4.08 (s, 2H),3.81 (m, 5H), 3.41 (s, 1H),3.23 (t, J = 12.4 Hz, 2H), 2.09 (d, J = 11.8 Hz, 2H), 200 - 1.79 (m, 4H), 1.70 - 1.39 (m, 4H), 1.41 - 1.17 (m, 4H).

ESI-MS(m/z) =602.3 [M+H]+.

**The compounds of Examples 21-23** were prepared according to the synthetic method of Example **20** (separation method 1), and the structure and characterization data are as follows:

| Example | Chemical name | Structure | $^1$H NMR | MS( M+H )+ |
|---|---|---|---|---|
| 21 | 7-(4-(5-(4-Aminopiperidin-1-yl)-7-(4-cyano-3-fluorophenyl)imidazo [ 1,2-c]pyrimidin-8-yl)-2 -hydroxyphenoxy)-N-hydroxyheptanamide hydrochloride | 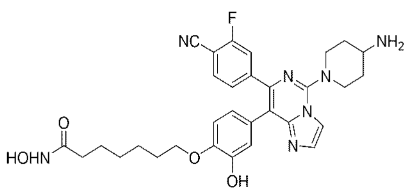 | $^1$H NMR (400 MHz, Methanol-$d_4$)δ ppm 8.11 (d, *J* = 2.4 Hz, 1H), 7.91 (d, *J* = 2.4 Hz, 1H), 7.59 (dd, *J* = 8.2, 6.8 Hz, 1H), 7.44 (dd, *J* = 10.6, 1.6 Hz, 1H), 7.35 (dd, *J* = 8.0, 1.6 Hz, 1H), 6.97 (d, *J* = 8.4 Hz, 1H), 6.80 - 6.58 (m, 2H), 4.16 (d, *J* = 10.6 Hz, 2H), 4.01 (t, *J* = 6.6 Hz, 2H), 3.45 (tt, *J* = 11.4, 4.2 Hz, 1H), 3.38 - 3.25 (m, 2H), 2.21 - 1.85 (m, 6H), 1.76 (p, *J* = 6.8 Hz, 2H), 1.57 (dt, *J* = 7.6, 4.0 Hz, 2H), 1.52 - 1.39 (m, 2H), 1.39 - 1.27 (m, 2H). | 588.3 |

(continued)

| Example | Chemical name | Structure | $^1$H NMR | MS( M+H )$^+$ |
|---|---|---|---|---|
| 22 | 7-(4-(5-(5-(5-Amino-2-azabicyclo[2.2.1]heptan-2-yl)-7-(4-cyano-3-fluorophenyl)imidazo[1,2-c]pyrimidin-8-yl)-2-hydroxyphenoxy)-N-hydroxyheptanamide hydrochloride | 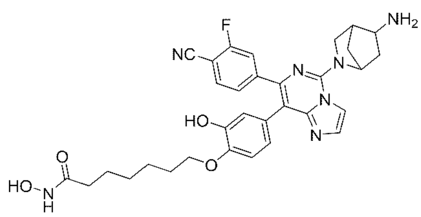 | $^1$H NMR (400 MHz, Methanol-$d_4$) δ ppm 8.28 (dd, *J* = 5.0, 2.6 Hz, 1H), 7.87 (t, *J* = 2.4 Hz, 1H), 7.66 (m, 1H), 7.56 - 7.48 (m, 1H), 7.41 (m, 1H), 7.04 (d, *J* = 8.2 Hz, 1H), 6.87 - 6.67 (m, 2H), 4.98 (d, *J* = 11.2 Hz, 1H), 4.41 - 4.28 (m, 1H), 4.10 (t, *J* = 6.6 Hz, 2H), 3.98 - 3.78 (m, 1H), 3.60 (dd, *J* = 7.6, 3.8 Hz, 1H), 3.20 - 2.93 (m, 1H), 2.77 (dt, *J* = 13.2, 7.4 Hz, 1H), 2.42 (m, 1H), 2.21 - 2.06 (m, 3H), 1.92 - 1.78 (m, 3H), 1.75 - 1.60 (m, 2H), 1.60 - 1.48 (m, 2H), 1.42 (q, *J* = 7.8 Hz, 2H). | 600.3 |
| 23 | (E)-3-(4-(4-(5-(4-Aminopiperidin-1-yl)-7-(4-cyano-3-fluorophenyl)imidazo[1,2-c]pyrimidin-8-yl)-2-hydroxyphenoxy)methyl)phenyl)-N-hydroxyacrylamide hydrochloride | 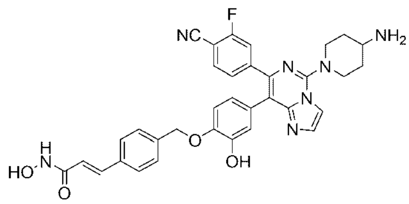 | $^1$H NMR (400 MHz, Methanol-$d_4$) δ 8.17 (d, *J* = 2.4 Hz, 1H), 7.98 (d, *J* = 2.4 Hz, 1H), 7.71 - 7.44 (m, 7H), 7.38 (dd, *J* = 8.2, 1.6 Hz, 1H), 7.08 (d, *J* = 8.4 Hz, 1H), 6.85 (d, *J* = 2.2 Hz, 1H), 6.73 (dd, *J* = 8.2, 2.2 Hz, 1H), 6.49 (d, *J* = 15.9 Hz, 1H), 5.29 (s, 2H), 4.24 (d, *J* = 13.6 Hz, 2H), 3.53 (s, 1H), 3.40 (d, *J* = 12.6 Hz, 2H), 2.21 (d, *J* = 11.8 Hz, 2H), 1.98 (tt, *J* = 12.8, 6.8 Hz, 2H). | 620.2 |

**Example 24**

Preparation of 7-(4-(5-cyano-4-(4-cyano-3-fluorophenyl)-6-(4-(methylamino)piperidin-1-yl)pyridin-3-yl)-2-hydroxyphenoxy)-N-h ydroxyheptanamide hydrochloride

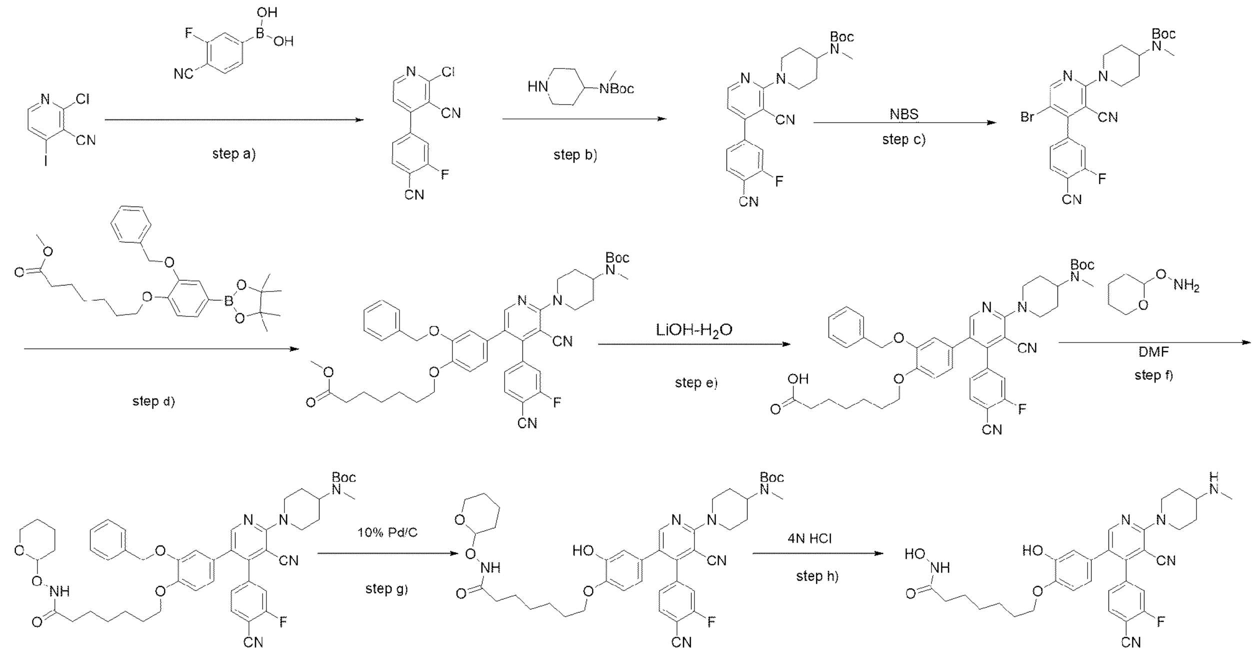


Step a): preparation of 2-chloro-4-(4-cyano-3-fluorophenyl)nicotinonitrile

2-Chloro-4-iodonicotinonitrile (1.0 g, 3.79 mmol), (4-cyano-3-fluorophenyl)boronic acid (688 mg, 4.17 mmol), $Cs_2CO_3$ (3.7 g, 11.37 mmol), Pd(dppf)$Cl_2$ (275 mg, 0.38 mmol), 1,4-dioxane (10 mL), and $H_2O$ (2.5 mL) were added to a reaction flask and stirred for reaction at 100°C for 1 hour. The reaction liquid was concentrated to dryness under reduced pressure, and the residue was purified by silica gel chromatography (eluent: petroleum ether/ethyl acetate = 3/1) to afford 2-chloro-4-(4-cyano-3-fluorophenyl)nicotinonitrile with a yield of 68.0%.
ESI-MS(m/z) = 258.1 [M+H] $^+$.

Step b): preparation of tert-butyl (1-(3-cyano-4-(4-cyano-3-fluorophenyl)pyridin-2-yl)piperidin-4-yl)(methyl)carbamate

2-Chloro-4-(4-cyano-3-fluorophenyl)nicotinonitrile (660 mg, 2.58 mmol), tert-butyl piperidin-4-yl-carbamate (552 mg, 2.58 mmol), DIPEA (332 mg, 2.58 mmol), and NMP (10 mL) were added to a reaction flask and stirred at 130°C for 1 hour. After the reaction was completed, water (20 mL) was added, followed by extraction with ethyl acetate (20 mL × 3), and the organic phases were combined, washed with saturated brine (20 mL × 2), and then concentrated to dryness under reduced pressure. The residue was purified by silica gel chromatography (eluent: petroleum ether/ethyl acetate = 3/1) to afford tert-butyl (1-(3-cyano-4-(4-cyano-3-fluorophenyl)pyridin-2-yl)piperidin-4-yl)(methyl)carbamate with a yield of 68.5%.
ESI-MS(m/z) = 436.2[M+H] $^+$.

Step c): preparation of tert-butyl (1-(5-bromo-3-cyano-4-(4-cyano-3-fluorophenyl)pyridin-2-yl)piperidin-4-yl)(methyl) carbamate

Tert-butyl (1-(3-cyano-4-(4-cyano-3-fluorophenyl)pyridin-2-yl)piperidin-4-yl)(methyl)carbamate (600 mg, 1.38 mmol) and DMF (10 mL) were added to a reaction flask, and NBS (270 mg, 1.5 mmol) was added in portions under stirring in an ice bath, and the mixture was stirred for reaction at room temperature for 30 minutes. After the reaction was completed, water (40 mL) was added, followed by extraction with ethyl acetate (40 mL × 3), and the organic phases were combined, washed with saturated brine (40 mL × 2), and then concentrated to dryness under reduced pressure. The residue was purified by silica gel chromatography (eluent: petroleum ether/ethyl acetate = 2/1) to afford tert-butyl (1-(5-bromo-3-cyano-4-(4-cyano-3-fluorophenyl)pyridin-2-yl)piperidin-4-yl)(methyl)carbamate with a yield of 77.0%.
ESI-MS(m/z) =514.1[M+H] $^+$.

Step d): preparation of methyl 7-(2-(benzyloxy)-4-(6-(4-(tert-butoxycarbonyl)(methyl)amino)piperidin-1-yl)-5-cyano-4-(4-cyano-3-fluorophenyl)p yridin-3-yl)phenoxy)heptanoate

Tert-butyl (1-(5-bromo-3-cyano-4-(4-cyano-3-fluorophenyl)pyridin-2-yl)piperidin-4-yl)(methyl)carbamate (500

mg, 0.97 mmol), methyl 7-(2-(benzyloxy)-4-(4,4,5,5-tetramethyl-1,3,2-dioxobenzaldehyde-2-yl)phenoxy)heptanoate (501 mg, 1.07 mmol), $Cs_2CO_3$ (950.8 mg, 2.92 mmol), Pd(dppf)$Cl_2$ (70.6 mg, 0.1 mmol), 1,4-dioxane (10 mL), and $H_2O$ (2.5 mL) were added to a reaction flask and stirred at 100°C for 1 hour. The reaction liquid was concentrated to dryness under reduced pressure, and the residue was purified by silica gel chromatography (eluent: petroleum ether/ethyl acetate = 3/1) to afford methyl 7-(2-(benzyloxy)-4-(6-(4-(tert-butoxycarbonyl)(methyl)amino)piperidin-1-yl)-5-cyano-4-(4-cyano-3-fluorophenyl)p yridin-3-yl)phenoxy)heptanoate with a yield of 68.0%.

ESI-MS(m/z) =776.4[M+H] $^+$.

Step e): preparation of 7-(2-(benzyloxy)-4-(6-(4-((tert-butoxycarbonyl)(methyl)amino)piperidin-1-yl)-5-cyano-4-(4-cyano-3-fluorophenyl) pyridin-3-yl)phenoxy)heptanoic acid

Methyl 7-(2-(benzyloxy)-4-(6-(4-(tert-butoxycarbonyl)(methyl)amino)piperidin-1-yl)-5-cyano-4-(4-cyano-3-fluorophenyl)p yridin-3-yl)phenoxy)heptanoate (500 mg, 0.66 mmol) was dissolved in a solution of tetrahydrofuran (6 mL) and water (1 mL), and lithium hydroxide (95.3 mg, 3.97 mmol) was added, and the mixture was reacted at room temperature for 3 hours, then the pH of the system was adjusted to 2~3 with 1N HCl, then the mixture was extracted with ethyl acetate, and the organic phases were dried and concentrated under vacuum. The residue was used directly for the subsequent step.

ESI-MS(m/z) =762.4 [M+H]$^+$.

Step f): preparation of tert-butyl (1-(5-(3-(benzyloxy)-4-((7-oxo-7-((tetrahydro-2H-pyran-2-yl)oxy)amino)heptyl)oxy) phenyl)-3-cyano-4-(4-cyano-3-fluorophenyl)pyridin-2-yl)piperidin-4-yl)(methyl)carbamate

7-(2-(Benzyloxy)-4-(6-(4-((tert-butoxycarbonyl)(methyl)amino)piperidin-1-yl)-5-cyano-4-(4-cyano-3-fluoroph enyl)pyridin-3-yl)phenoxy)heptanoic acid (186 mg, 0.24 mmol) and O-(tetrahydro-2H-pyran-2-yl)hydroxylamine (84.35 mg, 0.72 mmol) were dissolved in N,N-dimethylformamide (4 mL), followed by adding N,N-diisopropylethylamine (309.6 mg, 2.4 mmol) and HATU (118.63 mg, 0.31 mmol), and the mixture was reacted for 30 minutes, then water (60 mL) was added to quench the reaction, followed by extraction with ethyl acetate (60 mL × 2), The organic phases were combined, washed with saturated brine (45 mL × 2), dried over anhydrous sodium sulfate, filtered, and concentrated to obtain a crude product. The residue was then purified by silica gel chromatography (eluent: dichloromethane/ethyl acetate = 10/7) to afford tert-butyl (1-(5-(3-(benzyloxy)-4-((7-oxo-7-((tetrahydro-2H-pyran-2-yl)oxy)amino)heptyl)oxy)phenyl)-3-cyano-4-(4-cyano-3-fluorophenyl)pyridin-2-yl)piperidin-4-yl)(methyl)carbamate with a yield of 96%.

ESI-MS m/z=861.4 [M+H]$^+$

Step g): preparation of tert-butyl (1-(3-cyano-4-(4-cyano-3-fluorophenyl)-5-(3-hydroxy-4-((7-oxo-7-((tetrahydro-2H-pyran-2-yl)oxy)amino)heptyl)o xy)phenyl)pyridin-2-yl)piperidin-4-yl)(methyl)carbamate

Tert-butyl (1-(5-(3-(benzyloxy)-4-((7-oxo-7-((tetrahydro-2H-pyran-2-yl)oxy)amino)heptyl)oxy)phenyl)-3-cyano-4-(4-cyano-3-fluorophenyl)pyridin-2-yl)piperidin-4-yl)(methyl)carbamate (200 mg, 0.23 mmol) was dissolved in ethyl acetate (10 mL), and 10% palladium on carbon (50 mg) was added. The mixture was purged with hydrogen and reacted for 30 minutes, and the reaction liquid was then filtered to obtain a filtrate and the filtrate was concentrated to obtain a crude product, which was directly used in the next reaction step.

ESI-MS m/z=771.4 [M+H]$^+$

Step h): preparation of 7-(4-(5-cyano-4-(4-cyano-3-fluorophenyl)-6-(4-(methylamino)piperidin-1-yl)pyridin-3-yl)-2-hydroxyphenoxy)-N-h ydroxyheptanamide

Tert-butyl (1-(3-cyano-4-(4-cyano-3-fluorophenyl)-5-(3-hydroxy-4-((7-oxo-7-((tetrahydro-2H-pyran-2-yl)oxy) amino)heptyl)o xy)phenyl)pyridin-2-yl)piperidin-4-yl)(methyl)carbamate (200 mg, 0.26 mmol) was dissolved in 4N HCl/EA (5 mL) and the reaction was conducted for 30 minutes, and the mixture was concentrated to obtain a crude product, and purified by Prep-HPLC (separation method 1) to afford 7-(4-(5-cyano-4-(4-cyano-3-fluorophenyl)-6-(4-(methylamino) piperidin-1-yl)pyridin-3-yl)-2-hydroxyphenoxy)-N-h ydroxyheptanamide hydrochloride with a yield of 24%.

$^1$H NMR (400 MHz, Methanol-$d_4$) δ ppm 8.31 (s, 1H), 7.67 (t, *J* = 7.2 Hz, 1H), 7.31 (d, *J* = 9.6 Hz, 1H), 7.18 (d, *J* = 7.8 Hz, 1H), 6.72 (d, *J* = 8.2 Hz, 1H), 6.48 - 6.36 (m, 2H), 4.35 (d, *J* = 13.2 Hz, 2H), 3.88 (t, *J* = 6.6 Hz, 2H), 3.21 (m, 3H), 2.67 (s, 3H), 2.29 - 2.05 (m, 4H), 1.73 (ddt, *J* = 16.8, 13.2, 7.4 Hz, 4H), 1.55 (dp, *J* = 12.6, 7.2 Hz, 2H), 1.36 (dp, *J* = 12.2, 8.2, 7.2 Hz, 4H).

ESI-MS m/z=587.3 [M+H]$^+$

**Examples 25-26** were prepared according to the synthetic method of Example **24** (separation method 1), and the structure and characterization data are as follows:

| Example | Chemical name | Structure | $^1$H NMR | MS (M +H) + |
|---|---|---|---|---|
| 25 | 7-(4-(6-(4-Aminopiperidi n-1-yl)-5-cyano-4-(4-cya no-3-fluorophenyl)pyridi n-3-yl)-2-hydroxyphenox y)-N-hydroxyheptanamid e hydrochloride | 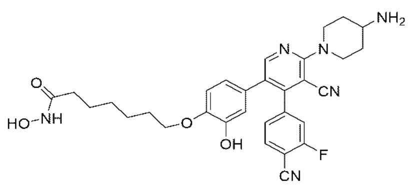 | $^1$H NMR (400 MHz, Methanol-$d_4$) δ 8.45 (s, 1H), 7.77 (dd, *J* = 7.8, 6.6 Hz, 1H), 7.40 (dd, *J* = 9.6, 1.4 Hz, 1H), 7.26 (dd, *J* = 8.0, 1.4 Hz, 1H), 6.83 (d, *J* = 8.2 Hz, 1H), 6.66 - 6.36 (m, 2H), 4.42 (d, *J* = 13.4 Hz, 2H), 4.00 (t, *J* = 6.4 Hz, 2H), 3.45 (ddt, *J* = 11.4, 8.6, 4.2 Hz, 1H), 3.25 (d, *J* = 11.8 Hz, 2H), 2.26 - 2.07 (m, 4H), 1.84 (ddd, *J* = 2.2, 12.2, 5.4 Hz, 4H), 1.66 (h, *J* = 7.2 Hz, 2H), 1.56 - 1.35 (m, 4H). | 573. 3 |
| 26 | 7-(4-(6-(4-Aminopiperidi n-1-yl)-5-cyano-4-(4-cya no-3-fluorophenyl)-2-me thylpyridin-3-yl)-2-hydro xyphenoxy)-N-hydroxyh eptanamide hydrochloride | 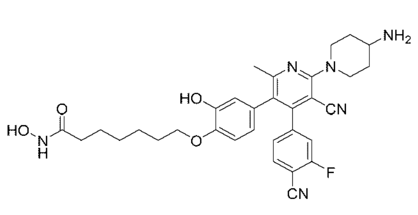 | $^1$H NMR (400 MHz, Methanol-$d_4$) δ ppm 7.66 (t, *J* = 7.4 Hz, 1H), 7.26 (d, *J* = 9.8 Hz, 1H), 7.17 (dd, *J* = 8.0, 1.4 Hz, 1H), 6.79 (d, *J* = 8.2 Hz, 1H), 6.49 (d, *J* = 2.0 Hz, 1H), 6.43 (dd, *J* = 8.2, 2.2 Hz, 1H), 4.40 (d, *J* = 13.4 Hz, 2H), 3.97 (t, *J* = 6.4 Hz, 2H), 3.41 (td, *J* = 11.2, 5.4 Hz, 1H), 3.25 - 3.06 (m, 2H), 2.33 (s, 3H), 2.13 (q, *J* = 8.0 Hz, 4H), 1.80 (qd, *J* = 15.6, 13.9, 5.4 Hz, 4H), 1.64 (p, *J* = 7.4 Hz, 2H), 1.56 - 1.34 (m, 4H). | 587. 3 |

**Example 27**

Preparation of 7-(4-(6-(4-aminopiperidin-1-yl)-2-(4-cyano-3-fluorophenyl)-4-methoxypyridin-3-yl)-2-hydroxyphenoxy)-N-hydrox yheptanamide hydrochloride

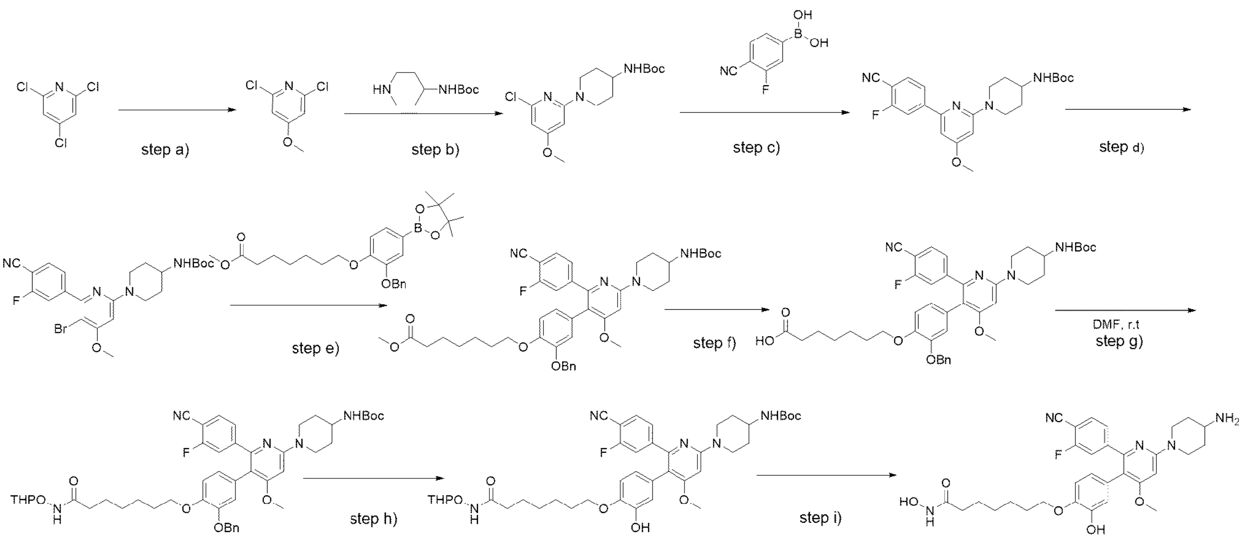

Step a): preparation of 2,6-dichloro-4-methoxypyridine

4-(Benzyloxy)-2,6-dichloropyridine (2 g, 11.049 mmol) and MeOH (20 mL) were added to a reaction flask and stirred for reaction at room temperature for 16 hours. After the reaction was completed, water (100 mL) was added, followed by extraction with ethyl acetate (100 mL × 3), and the organic phases were combined, washed with saturated brine (100 mL × 2), and then concentrated to dryness under reduced pressure. The residue was purified by silica gel chromatography (eluent: petroleum ether/ethyl acetate = 2/1) to afford 2,6-dichloro-4-methoxypyridine with a yield of 60.5%.

$^1$H NMR (400 MHz, Chloroform-*d*) δ ppm 6.79 (s, 2H), 3.87 (s, 3H).

ESI-MS(m/z) =178.0[M+H] $^+$.

Step b): preparation of tert-butyl (1-(6-chloro-4-methoxypyridin-2-yl)piperidin-4-yl)carbamate

2,6-Dichloro-4-methoxypyridine (1.2 g, 6.63 mmol), tert-butyl piperidin-4-yl-carbamate (2.7 g, 13.26 mmol), and NMP (15 mL) were added to a microwave reactor and stirred for reaction at 130°C for 2 hours. After the reaction was completed, water (100 mL) was added, followed by extraction with ethyl acetate (100 mL × 3), and the organic phases were combined, washed with saturated brine (100 mL × 2), and then concentrated to dryness under reduced pressure. The residue was purified by silica gel chromatography (eluent: petroleum ether/ethyl acetate = 1/1) to afford tert-butyl (1-(6-chloro-4-methoxypyridin-2-yl)piperidin-4-yl)carbamate with a yield of 52.0%.

ESI-MS(m/z) = 342.5[M+H] $^+$.

Step c): preparation of tert-butyl (1-(6-(4-cyano-3-fluorophenyl)-4-methoxypyridin-2-yl)piperidin-4-yl)carbamate

Tert-butyl (1-(6-chloro-4-methoxypyridin-2-yl)piperidin-4-yl)carbamate (1.2 g, 3.45 mmol), (4-cyano-3-fluorophenyl)boronic acid (853 mg, 5.17 mmol), $Cs_2CO_3$ (2.2 g, 6.9 mmol), Pd(dppf)$Cl_2$ (253 mg, 0.35 mmol), 1,4-dioxane (10 mL), and $H_2O$ (2.5 mL) were added to a reaction flask and stirred for reaction at 120°C for 1 hour. The reaction liquid was concentrated to dryness under reduced pressure. The residue was then purified by silica gel chromatography (eluent: petroleum ether/ethyl acetate = 2/3) to afford tert-butyl (1-(6-(4-cyano-3-fluorophenyl)-4-methoxypyridin-2-yl)piperidin-4-yl)carbamate with a yield of 73.0%.

$^1$H NMR (400 MHz, DMSO-*d*$_6$) δ 8.12 (dd, *J* = 15.4, 9.8 Hz, 2H), 7.02 (d, *J* = 1.8 Hz, 1H), 6.83 (d, *J* = 7.8 Hz, 1H), 6.40 (s, 1H), 4.33 (d, *J* = 13.2 Hz, 2H), 3.86 (m, 3H), 3.31 (s, 3H), 2.94 (t, *J* = 12.4 Hz, 2H), 1.80 (d, *J* = 12.0 Hz, 2H), 1.39 (s, 9H).

ESI-MS(m/z) =427.2 [M+H]$^+$.

Step d): preparation of tert-butyl (1-(5-bromo-6-(4-cyano-3-fluorophenyl)-4-methoxypyridin-2-yl)piperidin-4-yl)carbamate

Tert-butyl (1-(6-(4-cyano-3-fluorophenyl)-4-methoxypyridin-2-yl)piperidin-4-yl)carbamate (1.1 g, 2.52 mmol) and DMF (20 mL) were added to a reaction flask, and NBS (448 mg, 2.516 mmol) was added in portions under stirring in an ice bath, and the mixture was stirred for reaction at room temperature for 30 minutes. After the reaction was completed, water (40 mL) was added, followed by extraction with ethyl acetate (40 mL × 3), and the organic phases were combined, washed with saturated brine (40 mL × 2), and then concentrated to dryness under reduced pressure. The residue was purified by silica gel chromatography (eluent: petroleum ether/ethyl acetate = 2/3) to afford tert-butyl (1-(5-bromo-6-(4-cyano-3-fluorophenyl)-4-methoxypyridin-2-yl)piperidin-4-yl)carbamate with a yield of 72.0%.

ESI-MS(m/z) = 505.2[M+H] $^+$.

Step e): preparation of methyl 7-(2-(benzyloxy)-4-(6-(4-(tert-butoxycarbonyl)amino)piperidin-1-yl)-2-(4-cyano-3-fluorophenyl)-4-methoxypyridin -3-yl)phenoxy )heptanoate

Tert-butyl (1-(5-bromo-6-(4-cyano-3-fluorophenyl)-4-methoxypyridin-2-yl)piperidin-4-yl)carbamate (200 mg, 397 μmol), methyl 7-(2-(benzyloxy)-4-(4,4,5,5-tetramethyl-1,3,2-dioxobenzaldehyde-2-yl)phenoxy)heptanoate (279 mg, 596 μmol), $Cs_2CO_3$ (259 mg, 794 μmol), Pd(dppf)$Cl_2$ (29 mg, 39.7 μmol), 1,4-dioxane (10 mL), and $H_2O$ (2.5 mL) were added to a reaction flask and the mixture was stirred for reaction at 120°C for 1 hour. The reaction liquid was concentrated to dryness under reduced pressure. The residue was then purified by silica gel chromatography (eluent: petroleum ether/ethyl acetate = 1/2) to afford methyl 7-(2-(benzyloxy)-4-(6-(4-(tert-butoxycarbonyl)amino)piperidin-1-yl)-2-(4-cyano-3-fluorophenyl)-4-methoxypyridin -3-yl)phenoxy)heptanoate with a yield of 56.0%.

ESI-MS(m/z) =767.4 [M+H]$^+$.

Step f): preparation of 7-(2-(benzyloxy)-4-(6-(4-(tert-butoxycarbonyl)amino)piperidin-1-yl)-2-(4-cyano-3-fluorophenyl)-4-methoxypyridin -3-yl)phenoxy)heptanoic acid

Methyl 7-(2-(benzyloxy)-4-(6-(4-(tert-butoxycarbonyl)amino)piperidin-1-yl)-2-(4-cyano-3-fluorophenyl)-4-methoxypyridin -3-yl)phenoxy)heptanoate (171 mg, 222 μmol), lithium hydroxide monohydrate (43 mg, 1.1 mmol), tetrahydrofuran (2 mL), isopropanol (2 mL), and water (1 mL) were added to a reaction flask and stirred for reation at room temperature for 12 hours. Under stirring in an ice bath, 1N hydrochloric acid was slowly added dropwise to adjust the pH to 3~4, and water (10 mL) was added, followed by extraction with ethyl acetate (10 mL × 3). The organic phases were combined, washed with saturated brine (10 mL × 2), and then concentrated to dryness under reduced pressure to afford 7-(2-(benzyloxy)-4-(6-(4-(tert-butoxycarbonyl)amino)piperidin-1-yl)-2-(4-cyano-3-fluorophenyl)-4-methoxypyridin -3-yl) phenoxy)heptanoic acid, which was used directly for the subsequent step.
ESI-MS(m/z) =753.4[M+H]$^+$.

Step g): preparation of tert-butyl (1-(5-(3-(benzyloxy)-4-((7-oxo-7-((tetrahydro-2H-pyran-2-yl)oxy)amino)heptyl)oxy) phenyl)-6-(4-cyano-3-fluoroph enyl)-4-methoxypyridin-2-yl)piperidin-4-yl)carbamate

7-(2-(Benzyloxy)-4-(6-(4-(tert-butoxycarbonyl)amino)piperidin-1-yl)-2-(4-cyano-3-fluorophenyl)-4-methoxyp yridin-3-yl)phenoxy)heptanoic acid (167 mg, 222 μmol), O-(tetrahydro-2H-pyran-2-yl)hydroxylamine (52 mg, 444 μmol), DIEA (53 mg, 444 μmol), and DMF (2 mL) were added to a reaction flask, and the mixture was stirred for reaction at room temperature, and HATU (114 mg, 300 μmol) was added. The reaction was maintained at room temperature for 1 hour. After the reaction was completed, water (10 mL) was added to quench the reaction, followed by extraction with ethyl acetate (20 mL × 2). The organic phases were combined, sequentially washed with saturated sodium bicarbonate aqueous solution (20 mL × 1) and saturated brine (10 mL × 2), dried with anhydrous sodium sulfate, filtered, and the filtrate was concentrated under reduced pressure. The residue was purified by silica gel chromatography (eluent: petroleum ether/ethyl acetate = 1/1) to afford tert-butyl (1-(5-(3-(benzyloxy)-4-((7-oxo-7-((tetrahydro-2H-pyran-2-yl)oxy)amino) heptyl)oxy)phenyl)-6-(4-cyano-3-fluoroph enyl)-4-methoxypyridin-2-yl)piperidin-4-yl)carbamate with a yield of 64.7%.
ESI-MS(m/z) =852.4 [M+H]$^+$.

Step h): preparation of tert-butyl (1-(6-(4-cyano-3-fluorophenyl)-5-(3-hydroxy-4-((7-oxo-7-((tetrahydro-2H-pyran-2-yl) oxy)amino)heptyl)oxy)pheny l)-4-methoxypyridin-2-yl)piperidin-4-yl)carbamate

Tert-butyl (1-(5-(3-(benzyloxy)-4-((7-oxo-7-((tetrahydro-2H-pyran-2-yl)oxy)amino)heptyl)oxy)phenyl)-6-(4-cyano-3-fluoroph enyl)-4-methoxypyridin-2-yl)piperidin-4-yl)carbamate (122 mg, 143 μmol), palladium on carbon (25 mg, 5%), and ethanol (5 mL) were sequentially added to a reaction flask, and the mixture was purged with hydrogen three times and stirred at room temperature for 2 hours under hydrogen atmosphere. After the reaction was completed, it was filtered, and the filtrate was concentrated under reduced pressure to afford tert-butyl (1-(6-(4-cyano-3-fluorophenyl)-5-(3-hydroxy-4-((7-oxo-7-((tetrahydro-2H-pyran-2-yl)oxy)amino)heptyl)oxy)pheny l)-4-methoxypyridin-2-yl)piperidin-4-yl)carbamate with a yield of 87.0%.
ESI-MS(m/z) =762.4[M+H] $^+$.

Step i): preparation of 7-(4-(6-(4-aminopiperidin-1-yl)-2-(4-cyano-3-fluorophenyl)-4-methoxypyridin-3-yl)-2-hydroxyphenoxy)-N-hydrox yheptanamid hydrochloride

Tert-butyl (1-(6-(4-cyano-3-fluorophenyl)-5-(3-hydroxy-4-((7-oxo-7-((tetrahydro-2H-pyran-2-yl)oxy)amino) heptyl)oxy)pheny l)-4-methoxypyridin-2-yl)piperidin-4-yl)carbamate (95 mg, 124 μmol) was added to a reaction flask, followed by adding hydrogen chloride solution in ethyl acetate (4M, 2.5 mL), and the mixture was stirred at room temperature for 1 hour, resulting in the precipitation of a significant amount of solid, After concentrating under reduced pressure, the crude product was purified by Prep-HPLC (separation method 1) to afford 7-(4-(6-(4-aminopiperidin-1-yl)-2-(4-cyano-3-fluorophenyl)-4-methoxypyridin-3-yl)-2-hydroxyphenoxy)-N-hydrox yheptanamide hydrochloride with a yield of 34.0%.
$^1$H NMR (400 MHz, Methanol-$d_4$) δ 7.63 (t, *J* = 7.0 Hz, 1H), 7.39 (d, *J* = 9.4 Hz, 1H), 7.24 (d, *J* = 7.8 Hz, 1H), 6.79 - 6.57 (m, 2H), 6.49 (s, 1H), 6.36 (d, *J* = 7.8 Hz, 1H), 4.26 (d, *J* = 13.0 Hz, 2H), 3.95 (s, 3H), 3.88 (t, *J*= 6.4 Hz, 2H), 3.46 (s, 1H), 3.32 (t, *J* = 12.8 Hz, 2H), 2.14 (d, *J* = 12.2 Hz, 2H), 2.03 (t, *J* = 7.4 Hz, 2H), 1.84 - 1.62 (m, 4H), 1.57 - 1.50 (m, 2H), 1.44 - 1.26 (m, 4H).
ESI-MS(m/z) =578.3 [M+H]$^+$.
**Examples 28-31** were prepared according to the synthetic method of Example **27** (the separation method for the compound: hydrochloride and formate were prepared by separation method 1 and 3, respectively), and the structures and characterization data are as follows:

| Example | Chemical name | Structure | $^1$H NMR | MS(M+H)$^+$ |
|---|---|---|---|---|
| 28 | (E)-3-(4-(6-(4-Aminopiperid in-1-yl)-2-(4-cyano-3-fluoro phenyl)-4-methoxypyridin-3 -yl)-2-hydroxyphenyl)-N-hydroxyacrylamide formate | 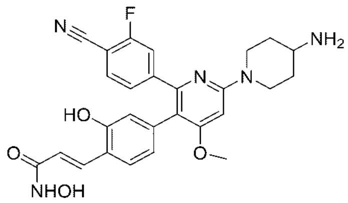 | $^1$H NMR (400 MHz, Methanol-$d_4$) δ 8.54 (s, 3H), 7.78 (d, *J* = 15.7 Hz, 1H), 7.51 (t, *J* = 7.4 Hz, 1H), 7.35 - 7.26 (m, 2H), 7.19 (d, *J* = 7.8 Hz, 1H), 6.67 - 6.45 (m, 3H), 4.57 (d, *J* = 11.8 Hz, 2H), 3.84 (s, 3H), 3.37 (s, 1H), 3.02 (t, *J* = 12.1 Hz, 2H), 2.08 (s, 2H), 1.65 (s, 2H). | 504.2 |
| 29 | (E)-4-(4-(6-(4-Aminopiperid in-1-yl)-2-(4-cyano-3-fluoro phenyl)-4-methoxypyridin-3 -yl)-2-hydroxyphenoxy)-N-hydroxy-2-enamide hydrochloride | 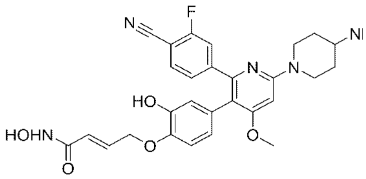 | $^1$H NMR (400 MHz, Methanol-$d_4$) δ 7.72 (dd, *J* = 8.0, 6.6 Hz, 1H), 7.50 (dd, *J* = 9.6, 1.4 Hz, 1H), 7.32 (dd, *J* = 8.0, 1.4 Hz, 1H), 6.94 (dt, *J* = 15.6, 4.2 Hz, 1H), 6.79 (d, *J* = 7.8 Hz, 2H), 6.61 (d, *J* = 2.0 Hz, 1H), 6.47 (dd, *J* = 8.2, 2.0 Hz, 1H), 6.14 (dt, *J* = 15.4, 2.0 Hz, 1H), 4.74 (dd, *J* = 4.6, 1.8 Hz, 2H), 4.36 (d, *J* = 13.8 Hz, 2H), 4.04 (s, 3H), 3.55 (tt, *J* = 10.2, 4.4 Hz, 1H), 3.47 - 3.36 (m, 2H), 2.33 - 2.13 (m, 2H), 1.87 (qd, *J* = 12.4, 3.8 Hz, 2H). | 534.2 |
| 30 | (E)-3-(4-((2-(5-(6-(4-Amino piperidin-1-yl)-2-(4-cyano-3 -fluorophenyl)-4-methoxypy ridin-3 -yl)-2-methoxypheno xy)ethyl)amino) methyl)phen yl)-N-hydroxyacrylamide hydrochloride | 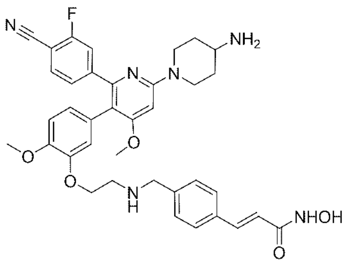 | $^1$H NMR (400 MHz, Methanol-$d_4$) δ ppm 7.70 - 7.63 (m, 3H), 7.62 - 7.55 (m, 3H), 7.42 (d, *J* = 10.0 Hz, 1H), 7.33 (dd, *J* = 8.2, 1.4 Hz, 1H), 6.94 (d, *J* = 8.4 Hz, 1H), 6.81 (d, *J* = 2.0 Hz, 1H), 6.76 - 6.67 (m, 2H), 6.54 (d, *J* = 15.8 Hz, 1H), 4.45 (d, *J* = 13.8 Hz, 2H), 4.37 (s, 2H), 4.18 (t, *J* = 4.6 Hz, 2H), 3.96 (s, 3H), 3.85 (s, 3H), 3.51 (d, *J* = 4.2 Hz, 1H), 3.45 (t, *J* = 4.8 Hz, 2H), 3.29 - 3.22 (m, 2H), 2.26 - 2.11 (m, 2H), 1.81 (qd, *J* = 12.0, 4.0 Hz, 2H) . | 667.3 |

**Example 31**

Preparation of 7-((2-(4-aminopiperidin-1-yl)-6-(4-cyano-3-fluorophenyl)pyridin-4-yl)oxy)-N-hydroxyheptanamide hydrochloride

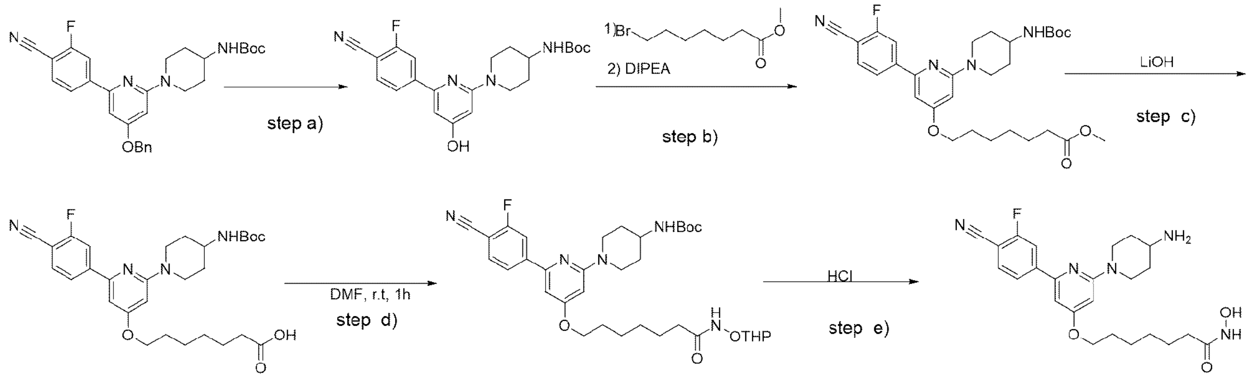


Step a): preparation of tert-butyl (1-(6-(4-cyano-3-fluorophenyl)-4-hydroxypyridin-2-yl)piperidin-4-yl)carbamate

The product of Step b) in Example 1: tert-butyl (1-(4-(benzyloxy)-6-(4-cyano-3-fluorophenyl)pyridin-2-yl)piperidin-4-yl)carbamate (600 mg, 1.2 mmol) was dissolved in dichloromethane (12 mL), the mixture was cooled to 0°C, then 1 N boron tribromide solution in dichloromethane (5 mL) was added dropwise, and the reaction was maintained at 0°C for 1 hour, then the reaction was quenched with water, and the pH was adjusted to an alkalic system with sodium bicarbonate. Additional 10 mL of tetrahydrofuran and $Boc_2O$ (523.38 mg, 2.4 mmol) were added, and the mixture was stirred for 30 minutes, then extracted with ethyl acetate (30 mL × 3), then the organic phases were washed once with saturated brine, and the filtrates were dried and concentrated under reduced pressure. The residue was purified by silica gel column (eluent: petroleum ether: ethyl acetate = 1:1) to afford tert-butyl (1-(6-(4-cyano-3-fluorophenyl)-4-hydroxypyridin-2-yl) piperidin-4-yl)carbamate with a yield of 80%.
ESI-MS m/z=413.2 $[M+H]^+$.

Step b): preparation of methyl 7-((2-(4-((tert-butoxycarbonyl)amino)piperidin-1-yl)-6-(4-cyano-3-fluorophenyl)pyridin-4-yl)oxy)heptanoate hydrochloride

Tert-butyl (1-(6-(4-cyano-3-fluorophenyl)-4-hydroxypyridin-2-yl)piperidin-4-yl)carbamate (400 mg, 0.97 mmol) was dissolved in acetonitrile (4 mL), methyl 7-bromoheptanoate (1.3 g, 5.82 mmol) and N,N-diisopropylethylamine (752.2 mg, 5.82 mmol) were weighed and added at room temperature. The mixture was purged with nitrogen, heated to 90°C and reacted overnight, and the reaction was monitored by LCMS, which indicated that the starting material was completely consumed. After the reaction liquid was cooled to room temperature, water (5 mL) was added to quench the reaction, followed by extraction with ethyl acetate (5 mL × 2), and the organic phases were combined, washed with saturated brine (5 mL × 2), dried with anhydrous sodium sulfate, filtered, and concentrated under reduced pressure. The residue was purified by silica gel chromatography (eluent: petroleum ether: ethyl acetate = 2:1) to afford methyl 7-((2-(4-((tert-butoxycarbonyl)amino)piperidin-1-yl)-6-(4-cyano-3-fluorophenyl)pyridin-4-yl)oxy)heptanoate with a yield of 77.0%.
ESI-MS m/z=555.3 $[M+H]^+$.

Step c): preparation of 7-((2-(4-((tert-butoxycarbonyl)amino)piperidin-1-yl)-6-(4-cyano-3-fluorophenyl)pyridin-4-yl)oxy) heptanoic acid

Lithium hydroxide (129.6 mg, 5.4 mmol) was dissolved in a mixture of tetrahydrofuran : water = 2:1 (11 mL), then, methyl 7-((2-(4-((tert-butoxycarbonyl)amino)piperidin-1-yl)-6-(4-cyano-3-fluorophenyl)pyridin-4-yl)oxy)heptanoate (300 mg, 0.54 mmol) was added to the lithium hydroxide solution. The mixture was purged with nitrogen and stirred at room temperature overnight, and the reaction was monitored by LCMS, which indicated that the starting material was completely consumed. Water (20 mL) was added, and the pH was adjusted to 3-4 with 1N hydrochloric acid solution, and the mixture was extracted with ethyl acetate (20 mL × 2), and the organic phases were combined, washed with saturated brine (15 mL × 2), then dried with anhydrous sodium sulfate, filtered, and concentrated to obtain the crude product 7-((2-(4-((tert-butoxycarbonyl)amino)piperidin-1-yl)-6-(4-cyano-3-fluorophenyl)pyridin-4-yl)oxy)heptanoic acid, which was used directly for the subsequent step.
ESI-MS m/z=541.3 $[M+H]^+$.

Step d): preparation of tert-butyl (1-(6-(4-cyano-3-fluorophenyl)-4-((7-oxo-7-(((tetrahydro-2H-pyran-2-yl)oxy)amino) heptyl)oxy)pyridin-2-yl)piperid in-4-yl)carbamate

7-((2-(4-((Tert-butoxycarbonyl)amino)piperidin-1-yl)-6-(4-cyano-3-fluorophenyl)pyridin-4-yl)oxy)heptanoic

acid (300 mg, 0.47 mmol) was dissolved in DMF (5 mL) and stirred at room temperature for ten minutes, then, diisopropylethylamine (179.8 mg, 1.41 mmol), O-(tetrahydro-2H-pyran-2-yl)hydroxylamine (65 mg, 0.56 mmol), and hexafluorophosphate (211.8 mg, 0.56 mmol) were added, the reaction liquid was purged with nitrogen and stirred at room temperature for one hour, and the reaction was monitored by LCMS, which indicated that the starting material was completely consumed. After the reaction liquid was cooled to room temperature, water (5 mL) was added to quench the reaction, followed by extraction with ethyl acetate (5 mL × 2). The organic phases were combined, washed with saturated brine (5 mL × 2), dried with anhydrous sodium sulfate, filtered, and concentrated under reduced pressure. The residue was purified by silica gel chromatography (eluent: petroleum ether: ethyl acetate = 2:1) to afford tert-butyl (1-(6-(4-cyano-3-fluorophenyl)-4-((7-oxo-7-((tetrahydro-2H-pyran-2-yl)oxy)amino)heptyl)oxy)pyridin-2-yl)piperidi n-4-yl)carbamate, which was used directly for the subsequent reaction.

ESI-MS m/z=640.3 $[M+H]^+$.

Step e): preparation of 7-((2-(4-aminopiperidin-1-yl)-6-(4-cyano-3-fluorophenyl)pyridin-4-yl)oxy)-N-hydroxyheptanamide

Ethyl acetate (10 mL) was added to tert-butyl (1-(6-(4-cyano-3-fluorophenyl)-4-((7-oxo-7-(((tetrahydro-2H-pyran-2-yl)oxy)amino)heptyl)oxy)pyridin-2-yl)piperid in-4-yl)carbamate (200 mg, 0.31 mmol), followed by adding hydrochloric acid (11.47 mg, 0.31 mmol), then the mixture was stirred at room temperature for one hour, and the reaction was monitored by LCMS, which indicated that the starting material was completely consumed. After the reaction liquid was cooled to room temperature, it was concentrated under reduced pressure. The residue was then purified by Prep-HPLC (separation method 1) to afford 7-((2-(4-aminopiperidin-1-yl)-6-(4-cyano-3-fluorophenyl)pyridin-4-yl)oxy)-N-hydroxyheptanamide hydrochloride with a yield of 31%.

$^1$H NMR (400 MHz, DMSO-$d_6$) δ 10.36 (s, 1H), 8.34 - 7.90 (m, 2H), 7.62(s, 1H), 7.08 (d, $J$ = 10.0 Hz, 1H), 6.45 (s, 2H), 4.45 (d, $J$ = 13.2 Hz, 2H), 4.11 (s, 2H), 3.29 (s, 1H), 2.92 (d, $J$ = 13.2 Hz, 2H), 1.96 (m, 4H), 1.72 (s, 2H), 1.59 - 1.26 (m, 8H).

ESI-MS m/z=456.2 $[M+H]^+$.

**Example 32**

Preparation of 7-(4-(5-(3-amino-8-azabicyclo[3.2.1]octane-8-carbonyl)-3-(4-cyano-3-fluorophenyl)thiophen-2-yl)-2-hydroxypheno xy)-N-hydroxyheptanamide hydrochloride

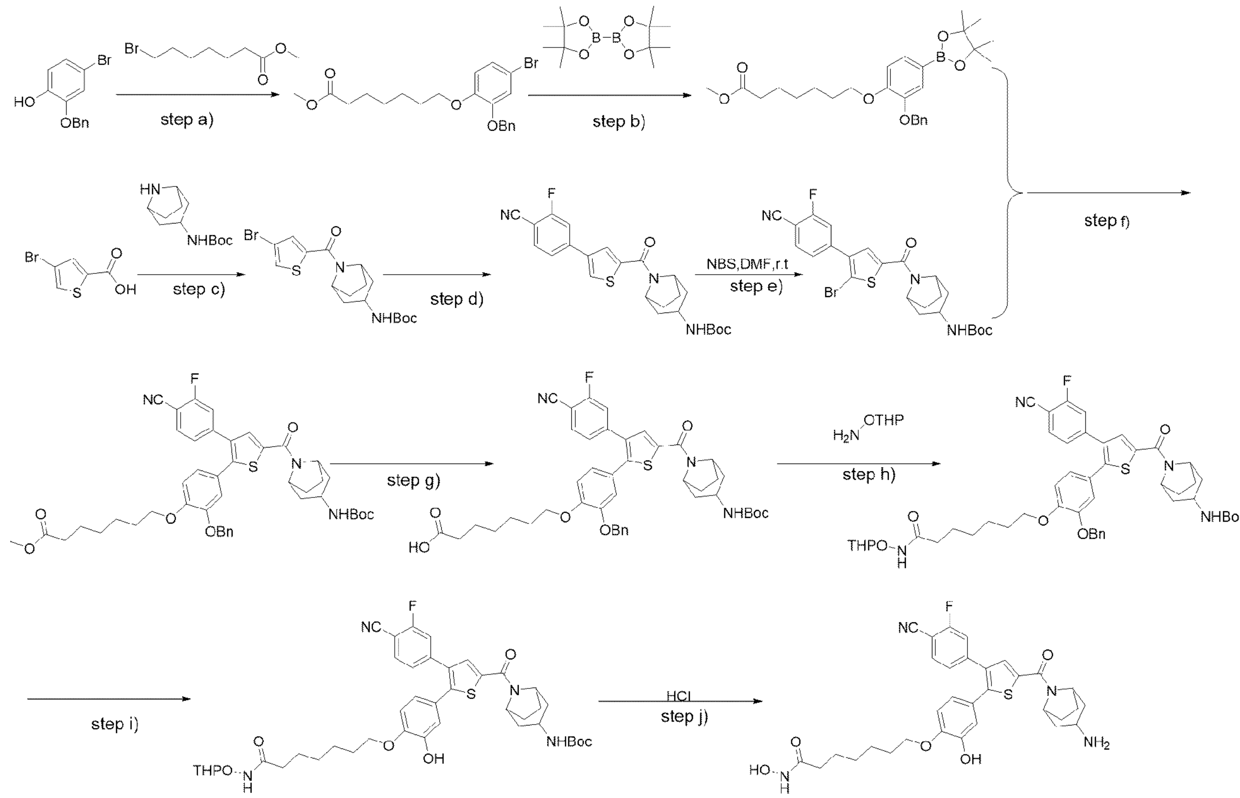


Step a): preparation of methyl 7-(2-(benzyloxy)-4-bromophenoxy)heptanoate

2-(Benzyloxy)-4-bromophenol (2 g, 7.16 mmol), methyl 7-bromoheptanoate (2.39 g, 10.74 mmol), and DIEA (3.7 g, 28.64 mmol) were added to a reaction flask containing acetonitrile (20 mL), and the mixture was stirred at 90°C for 16 hours. The reaction liquid was concentrated by adding silica gel and the residue was purified by silica gel chromatography (eluent: petroleum ether/ethyl acetate = 1/1) to afford methyl 7-(2-(benzyloxy)-4-bromophenoxy)heptanoate with a yield of 70%.

ESI-MS m/z=421.1 $[M+H]^+$.

Step b): preparation of methyl 7-(2-(benzyloxy)-4-(4,4,5,5-tetramethyl-1,3,2-dioxaborolan-2-yl)phenoxy)heptanoate

Methyl 7-(2-(benzyloxy)-4-bromophenoxy)heptanoate (2 g, 4.75 mmol), bis(pinacolato)diboron (2.41 g, 9.5 mmol), Pd(dppf)$Cl_2$ (0.35 g, 0.48 mmol), and potassium acetate (0.93 g, 9.5 mmol) were added to a reaction flask containing 1,4-dioxane (20 mL), and the mixture was stirred at 110°C for 3 hours. The reaction liquid was concentrated by adding silica gel and the residue was purified by silica gel chromatography (eluent: petroleum ether/ethyl acetate = 3/1) to afford methyl 7-(2-(benzyloxy)-4-(4,4,5,5-tetramethyl-1,3,2-dioxaborolan-2-yl)phenoxy)heptanoate with a yield of 72%.

ESI-MS m/z=469.3 $[M+H]^+$.

Step c): preparation of tert-butyl (8-(4-bromothiophene-2-carbonyl)-8-azabicyclo[3.2.1]octan-3-yl)carbamate

4-Bromothiophene-2-carboxylic acid (3 g, 14.47 mmol), HATU (8.25 g, 21.71 mmol), DIEA (5.61 g, 43.41 mmol), and tert-butyl (8-azabicyclo[3.2.1]octan-3-yl)carbamate (3.27 g, 14.47 mmol) were added to a reaction flask containing DMF (30 mL), and the mixture was stirred at room temperature for 2 hours. After the reaction was completed, water (20 mL) was added to quench the reaction, and the mixture was extracted with ethyl acetate (40 mL × 2). The organic phases were combined, washed with saturated brine (40 mL × 2), and then dried with anhydrous sodium sulfate. After filtering, the residue was purified by silica gel chromatography (eluent: petroleum ether/ethyl acetate = 1/1) to afford tert-butyl (8-(4-bromothiophene-2-carbonyl)-8-azabicyclo[3.2.1]octan-3-yl)carbamate with a yield of 90%.

ESI-MS m/z=415.1$[M+H]^+$.

Step d): preparation of tert-butyl (8-(4-(4-cyano-3-fluorophenyl)thiophene-2-carbonyl)-8-azabicyclo[3.2.1]octan-3-yl) carbamate

Tert-butyl (8-(4-bromothiophene-2-carbonyl)-8-azabicyclo[3.2.1]octan-3-yl)carbamate (5.5 g, 13.24 mmol), 4-cyano-3-fluorophenylboronic acid (3.28 g, 19.86 mmol), Pd(dppf)$Cl_2$ (0.97 g, 1.32 mmol), and cesium carbonate (8.63 g, 26.48 mmol) were added to a microwave tube containing 1,4-dioxane (40 mL) and water (8 mL), and the mixture was stirred at 70°C for 1 hour. The reaction liquid was concentrated by adding silica gel and the residue was purified by silica gel chromatography (eluent: petroleum ether/ethyl acetate = 1/1) to afford tert-butyl (8-(4-(4-cyano-3-fluorophenyl)thiophene-2-carbonyl)-8-azabicyclo[3.2.1]octan-3-yl)carbamate with a yield of 98%.
ESI-MS m/z=456.2 $[M+H]^+$.

Step e): preparation of tert-butyl (8-(5-bromo-4-(4-cyano-3-fluorophenyl)thiophene-2-carbonyl)-8-azabicyclo[3.2.1]octan-3-yl)carbamate

Tert-butyl (8-(4-(4-cyano-3-fluorophenyl)thiophene-2-carbonyl)-8-azabicyclo[3.2.1]octan-3-yl)carbamate (4 g, 8.78 mmol) and NBS (1.72 g, 9.66 mmol) were added to a reaction flask containing DMF (40 mL), and the mixture was stirred at 60°C for 2 hours. Water (20 mL) was added to quench the reaction, and the mixture was extracted with ethyl acetate (40 mL × 2). The organic phases were combined, washed with saturated brine (40 mL × 2), dried with anhydrous sodium sulfate. After filtering, the solution was concentrated, and the residue was purified by silica gel chromatography (eluent: petroleum ether/ethyl acetate = 1/1) to afford tert-butyl (8-(5-bromo-4-(4-cyano-3-fluorophenyl)thiophene-2-carbonyl)-8-azabicyclo[3.2.1]octan-3-yl)carbamate with a yield of 51%.
ESI-MS m/z=534.1 $[M+H]^+$.

Step f): preparation of methyl 7-(2-(benzyloxy)-4-(5-(3-((tert-butoxycarbonyl)amino)-8-azabicyclo[3.2.1]octane-8-carbonyl)-3-(4-cyano-3-fluorop henyl)thiophen-2-yl)phenoxy)heptanoate

Methyl 7-(2-(benzyloxy)-4-(4,4,5,5-tetramethyl-1,3,2-dioxaborolan-2-yl)phenoxy)heptanoate (200 mg, 0.43 mmol), tert-butyl (8-(5-bromo-4-(4-cyano-3-fluorophenyl)thiophene-2-carbonyl)-8-azabicyclo[3.2.1]octan-3-yl)carbamate (0.23 g, 0.43 mmol), Pd(dppf)$Cl_2$ (63 mg, 0.086 mmol), and cesium carbonate (0.28 g, 0.86 mmol) were added to a reaction liquid containing 1,4-dioxane (6 mL) and water (1.5 mL), and the mixture was stirred under microwave heating at 120°C for 1 hour. The reaction liquid was concentrated by adding silica gel and the residue was purified by silica gel chromatography (eluent: petroleum ether/ethyl acetate = 1/1) to afford methyl 7-(2-(benzyloxy)-4-(5-(3-((tert-butoxycarbonyl)amino)-8-azabicyclo[3.2.1]octane-8-carbonyl)-3-(4-cyano-3-fluorop henyl)thiophen-2-yl)phenoxy)heptanoate with a yield of 70%.
ESI-MS m/z=796.3 $[M+H]^+$.

Step g): preparation of 7-(2-(benzyloxy)-4-(5-(3-((tert-butoxycarbonyl)amino)-8-azabicyclo[3.2.1]octane-8-carbonyl)-3-(4-cyano-3-)fluoro phenyl)thiophen-2-yl)phenoxy)heptanoic acid

Methyl 7-(2-(benzyloxy)-4-(5-(3-((tert-butoxycarbonyl)amino)-8-azabicyclo[3.2.1]octane-8-carbonyl)-3-(4-cyano-3-fluorop henyl)thiophen-2-yl)phenoxy)heptanoate (300 mg, 0.38 mmol) and lithium hydroxide (0.16 g, 3.8 mmol) were added to a reaction liquid containing THF (10 mL), MeOH (6 mL), and water (2 mL), and the mixture was stirred at room temperature for 2 hours. Ice-water mixture (10 mL) was added to the reaction liquid, followed by the adjustment of pH to 3 with 2M HCl, and the mixture was then extracted with a mixture of DCM/MeOH (5:1) (20 mL × 2). The organic phases were combined, washed with saturated brine (15 mL × 2), dried with anhydrous sodium sulfate, filtered, and concentrated to afford 7-(2-(benzyloxy)-4-(5-(3-((tert-butoxycarbonyl)amino)-8-azabicyclo[3.2.1]octane-8-carbonyl)-3-(4-cyano-3-) fluoro phenyl)thiophen-2-yl)phenoxy)heptanoic acid with a yield of 62%.
ESI-MS m/z=782.3 $[M+H]^+$.

Step h): preparation of tert-butyl (8-(5-(3-(benzyloxy)-4-((7-oxo-7-((tetrahydro-2H-pyran-2-yl)oxy)amino)heptyl)oxy) phenyl)-4-(4-cyano-3-fluoroph enyl)thiophene-2-carbonyl)-8-azabicyclo[3.2.1]octan-3-yl)carbamate

7-(2-(Benzyloxy)-4-(5-(3-((tert-butoxycarbonyl)amino)-8-azabicyclo[3.2.1]octane-8-carbonyl)-3-(4-cyano-3-)f luorophenyl)thiophen-2-yl)phenoxy)heptanoic acid (130 mg, 0.17 mmol), HATU (4.5 mg, 65 μmol), O-(tetrahydro-2H-pyran-2-yl)hydroxylamine (97 mg, 0.26 mmol), and DIEA (66 mg, 0.51 mmol) were added to a reaction liquid containing DMF (10 mL), and the mixture was stirred at room temperature for 2 hours. Water (10 mL) was added to quench the reaction, followed by extraction with ethyl acetate (20 mL × 2), the organic phases were combined, washed with saturated brine (15 mL × 2), dried with anhydrous sodium sulfate, and concentrated under reduced pressure. The residue was

purified by silica gel chromatography (eluent: petroleum ether/ethyl acetate = 1/1) to afford tert-butyl (8-(5-(3-(benzyloxy)-4-((7-oxo-7-((tetrahydro-2H-pyran-2-yl)oxy)amino)heptyl)oxy)phenyl)-4-(4-cyano-3-fluoroph enyl)thiophene-2-carbonyl)-8-azabicyclo[3.2.1]octan-3-yl)carbamate with a yield of 67%.

ESI-MS m/z=881.4 $[M+H]^+$.

Step i): preparation of tert-butyl (8-(4-(4-cyano-3-fluorophenyl)-5-(3-hydroxy-4-((7-oxo-7-(((tetrahydro-2H-pyran-2-yl)oxy)))amino)heptyl)oxy)phe nyl)thiophene-2-carbonyl)-8-azabicyclo[3.2.1]octan-3-yl)carbamate

Tert-butyl (8-(5-(3-(benzyloxy)-4-((7-oxo-7-((tetrahydro-2H-pyran-2-yl)oxy)amino)heptyl)oxy)phenyl)-4-(4-cyano-3-fluoroph enyl)thiophene-2-carbonyl)-8-azabicyclo[3.2.1]octan-3-yl)carbamate (100 mg, 0.11 mmol) and palladium on carbon (21 mg, 0.11 mmol) were added to a reaction flask containing ethyl acetate (5 mL), and the mixture was stirred at 40°C for 36 hours under hydrogen atmosphere. The reaction liquid was filtered with diatomaceous earth and concentrated to afford tert-butyl (8-(4-(4-cyano-3-fluorophenyl)-5-(3-hydroxy-4-((7-oxo-7-(((tetrahydro-2H-pyran-2-yl)oxy)))amino)heptyl)oxy)phe nyl)thiophene-2-carbonyl)-8-azabicyclo[3.2.1]octan-3-yl)carbamate with a yield of 75%.

ESI-MS m/z=791.3 $[M+H]^+$.

Step j): preparation of 7-(4-(5-(3-amino-8-azabicyclo[3.2.1]octane-8-carbonyl)-3-(4-cyano-3-fluorophenyl)thiophen-2-yl)-2-hydroxypheno xy)-N-hydroxyheptanamide hydrochloride

4.0M Hydrochloric acid solution in EA (3 mL) was added to a reaction flask containing tert-butyl (8-(4-(4-cyano-3-fluorophenyl)-5-(3-hydroxy-4-((7-oxo-7-(((tetrahydro-2H-pyran-2-yl)oxy)))amino)heptyl)oxy)phe nyl)thiophene-2-carbonyl)-8-azabicyclo[3.2.1]octan-3-yl)carbamate (85 mg, 0.11 mmol), and the mixture was stirred at room temperature for 1 hour. The mixture was concentrated and then purified by Prep-HPLC (separation method 1) to afford 7-(4-(5-(3-amino-8-azabicyclo[3.2.1]octane-8-carbonyl)-3-(4-cyano-3-fluorophenyl)thiophen-2-yl)-2-hydroxypheno xy)-N-hydroxyheptanamide hydrochloride with a yield of 16%.

$^1H$ NMR (400 MHz, DMSO-$d_6$) δ ppm 10.32 (s, 1H), 9.16 (t, *J* = 8.0 Hz, 1H), 8.63 (s, 1H), 7.88 (t, *J* = 7.6 Hz, 2H), 7.65 (s, 1H), 7.57 (dd, *J* = 10.8, 1.4 Hz, 1H), 7.27 (dd, *J* = 8.0, 1.6 Hz, 1H), 6.92 (d, *J* = 8.6 Hz, 1H), 6.70 (d, *J* = 6.8 Hz, 2H), 4.77 (s, 2H), 3.95 (t, *J* = 6.6 Hz, 2H), 3.62 (s, 1H), 2.12 - 1.89 (m, 6H), 1.72 (dq, *J* = 21.8, 7.8, 6.8 Hz, 6H), 1.51 (p, *J* = 7.4 Hz, 2H), 1.41 (p, *J* = 7.4 Hz, 2H), 1.30 (q, *J* = 8.0 Hz, 2H).

ESI-MS m/z=607.2 $[M+H]^+$.

**Example 33**

Preparation of (E)-3-(4-(((1-(4-(4-cyano-3-fluorophenyl)-5-(3-hydroxy-4-methoxyphenyl)thiophene-2-carbonyl)piperidin-4-yl)ami no)methyl)phenyl)-N-hydroxyacrylamide hydrochloride

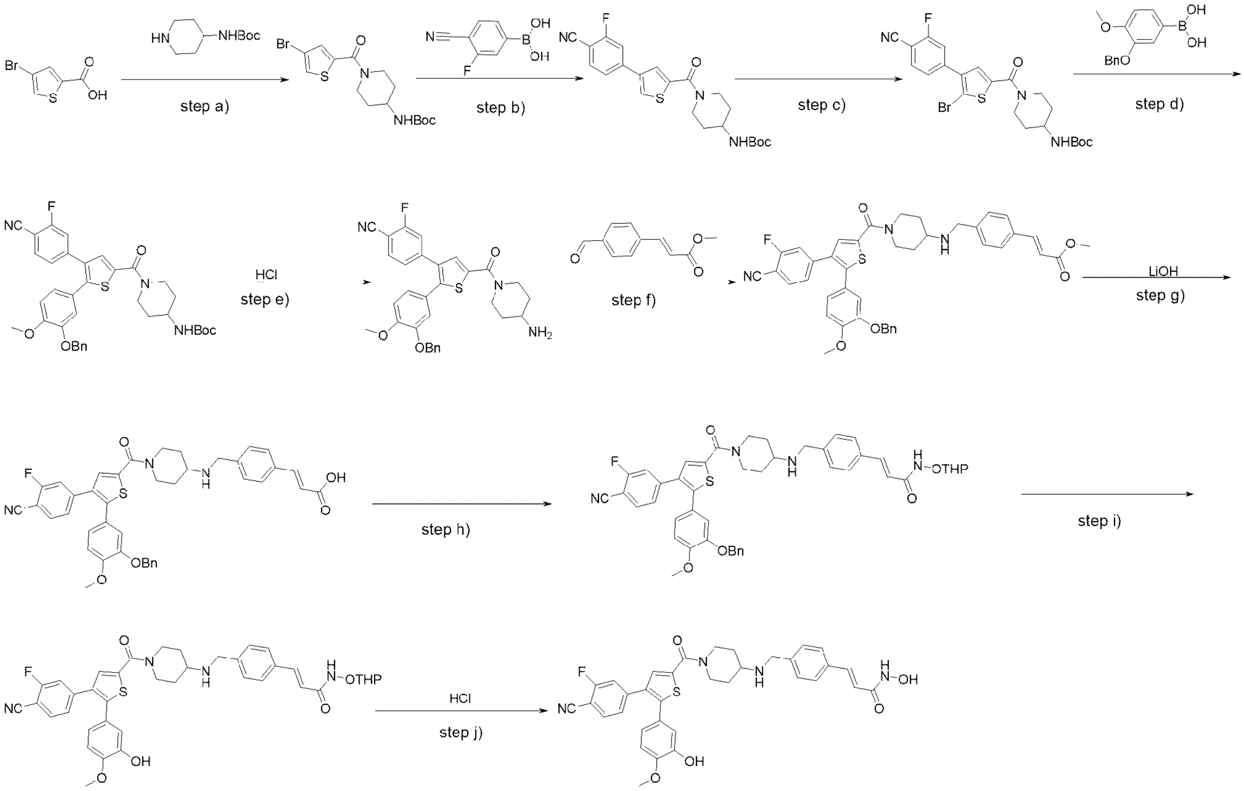


Step a): preparation of tert-butyl (1-(4-bromothiophene-2-carbonyl)piperidin-4-yl)carbamate

4-Bromothiophene-2-carboxylic acid (1 g, 4.82 mmol), tert-butyl piperidin-4-yl-carbamate (1.16 g, 5.78 mmol), HATU (2.75 g, 7.23 mmol), and DIEA (1.87 g, 14.46 mmol) were added to a reaction flask containing DMF (20 mL), and the mixture was stirred at room temperature for 2 hours. Water (20 mL) was added to quench the reaction, followed by extraction with ethyl acetate (20 mL × 2), and the organic phases were combined, washed with saturated brine (20 mL × 2), then dried with anhydrous sodium sulfate, filtered. The residue was purified by silica gel chromatography (eluent: petroleum ether/ethyl acetate = 1/1) to afford tert-butyl (1-(4-bromothiophene-2-carbonyl)piperidin-4-yl)carbamate with a yield of 91%.

ESI-MS m/z=389.1 $[M+H]^+$.

Step b): preparation of tert-butyl (1-(4-(4-cyano-3-fluorophenyl)thiophene-2-carbonyl)piperidin-4-yl)carbamate

Tert-butyl (1-(4-bromothiophene-2-carbonyl)piperidin-4-yl)carbamate (1.6 g, 4.11 mmol), (4-cyano-3-fluorophenyl)boronic acid (0.81 g, 4.93 mmol), Pd(dppf)$Cl_2$ (0.30 g, 0.41 mmol), and cesium carbonate (2.68 g, 8.22 mmol) were added to a microwave tube containing 1,4-dioxane (10 mL) and water (2 mL), and the mixture was stirred under microwave heating at 120°C for 45 minutes. The reaction liquid was concentrated by adding silica gel and the residue was purified by silica gel chromatography (eluent: petroleum ether/ethyl acetate = 1/1) to afford tert-butyl (1-(4-(4-cyano-3-fluorophenyl) thiophene-2-carbonyl)piperidin-4-yl)carbamate with a yield of 99%.

ESI-MS m/z=430.2 $[M+H]^+$.

Step c): preparation of tert-butyl (1-(5-bromo-4-(4-cyano-3-fluorophenyl)thiophene-2-carbonyl)piperidin-4-yl)carbamate

Tert-butyl (1-(4-(4-cyano-3-fluorophenyl)thiophene-2-carbonyl)piperidin-4-yl)carbamate (1.7 g, 3.96 mmol) and NBS (1.06 g, 5.94 mmol) were added to a reaction flask containing DMF (15 mL), and the mixture was stirred at 60°C for 2 hours. The reaction liquid was quenched by adding water (20 mL), then extracted with ethyl acetate (20 mL × 2), and the organic phases were combined, washed with saturated brine (15 mL × 2), dried over anhydrous sodium sulfate, and concentrated under vacuum. The residue was purified by silica gel chromatography (eluent: petroleum ether/ethyl acetate = 1/1) to afford tert-butyl (1-(5-bromo-4-(4-cyano-3-fluorophenyl)thiophene-2-carbonyl)piperidin-4-yl)carbamate with a yield of 98%.

ESI-MS m/z=508.1[M+H]+.

Step d): preparation of tert-butyl (1-(5-(3-(benzyloxy)-4-methoxyphenyl)-4-(4-cyano-3-fluorophenyl)thiophene-2-carbonyl)piperidin-4-yl)carbamate

Tert-butyl (1-(5-bromo-4-(4-cyano-3-fluorophenyl)thiophene-2-carbonyl)piperidin-4-yl)carbamate (1.7 g, 3.34 mmol), (3-(benzyloxy)-4-methoxyphenyl)boronic acid (1.29 g, 5.01 mmol), Pd(dppf)$Cl_2$ (0.24 g, 0.33 mmol), and cesium carbonate (2.18 g, 6.68 mmol) were added to a microwave tube containing 1,4-dioxane (14 mL) and water (2 mL), and the mixture was stirred at 120°C for 1 hour. The reaction liquid was concentrated by adding silica gel, and the residue was purified by silica gel chromatography (eluent: petroleum ether/ethyl acetate = 1/1) to afford tert-butyl (1-(5-(3-(benzyloxy)-4-methoxyphenyl)-4-(4-cyano-3-fluorophenyl)thiophene-2-carbonyl)piperidin-4-yl)carbamate with a yield of 93%.

ESI-MS m/z=642.2 [M+H]+.

Step e): preparation of 4-(5-(4-aminopiperidine-1-carbonyl)-2-(3-(benzyloxy)-4-methoxyphenyl)thiophen-3-yl)-2-fluorobenzonitrile

Tert-butyl (1-(5-(3-(benzyloxy)-4-methoxyphenyl)-4-(4-cyano-3-fluorophenyl)thiophene-2-carbonyl)piperidin-4-yl)carbamate (900 mg, 1.40 mmol) and 2M hydrochloric acid solution in ethyl acetate (10 mL) were added to a reaction flask and the mixture was stirred at room temperature for 1 hour. The reaction liquid was concentrated to afford 4-(5-(4-aminopiperidine-1-carbonyl)-2-(3-(benzyloxy)-4-methoxyphenyl)thiophen-3-yl)-2-fluorobenzonitrile with a yield of 92%.

ESI-MS m/z=542.2 [M+H]+.

Step f): preparation of methyl (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)acrylate

4-(5-(4-Aminopiperidine-1-carbonyl)-2-(3-(benzyloxy)-4-methoxyphenyl)thiophen-3-yl)-2-fluorobenzonitrile (680 mg, 1.26 mmol) and methyl (E)-3-(4-formylphenyl)acrylate (0.24 g, 1.26 mmol) were added to a reaction flask containing DCE (10 mL), and the mixture was stirred at room temperature for 2 hours. After the complete consumption of the starting materials monitored by LC-MS, sodium cyanoborohydride (0.32 g, 5.04 mmol) was added to the reaction liquid at an ice bath, and the mixture was further stirred at room temperature for 2 hours. The reaction liquid was quenched by adding ice-water mixture of saturated sodium bicarbonate, then extracted with ethyl acetate (20 mL × 2). The organic phases were combined, washed with saturated brine (15 mL × 2), dried over anhydrous sodium sulfate, filtered, and the residue was purified by silica gel chromatography (eluent: petroleum ether/ethyl acetate = 1/1) to afford methyl (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)acrylate with a yield of 72%.

ESI-MS m/z=716.3 [M+H]+.

Step g): preparation of (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)acrylic acid

Methyl (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)acrylate (630 mg, 0.88 mmol) and lithium hydroxide (0.21 g, 8.8 mmol) were added to a reaction flask containing THF (5 mL), MeOH (3 mL), and water (2 mL), and the mixture was stirred at room temperature for 2 hours. The reaction liquid was acidified to pH 4 by adding 2M hydrochloric acid, then extracted with a mixture of MeOH/DCM (1:5) (20 mL × 2). The organic phases were combined, washed with saturated brine (15 mL × 2), dried over anhydrous sodium sulfate, and concentrated to afford (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)acrylic acid with a yield of 97%.

ESI-MS m/z=702.2 [M+H]+.

Step h): preparation of (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)-N-((tetrahydro-2H-pyran-2-yl)oxy)acrylamide

(E)-3-(4-(((1-(5-(3-(Benzyloxy)-4-methoxyphenyl)-4-(4-cyano-3-fluorophenyl)thiophene-2-carbonyl)piperidin-4-yl)amino)methyl)phenyl)acrylic acid (600 mg, 0.85 mmol), O-(tetrahydro-2H-pyran-2-yl)hydroxylamine (0.20 g, 1.71 mmol), HATU (0.39 g, 1.02 mmol), and DIEA (0.33 g, 2.55 mmol) were added to a reaction flask containing DMF (10 mL), and the mixture was stirred at room temperature for 2 hours. The reaction liquid was quenched by adding water (10 mL), then extracted with ethyl acetate (20 mL × 2), and the organic phases were combined, washed with saturated brine

(15 mL × 2), dried over anhydrous sodium sulfate, and concentrated under reduced pressure. The residue was purified by silica gel chromatography (eluent: DCM/MeOH = 10/1) to afford (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)-N-((tetrahydro-2H-pyran-2-yl)oxy)acrylamide with a yield of 44%.

ESI-MS m/z=801.3 $[M+H]^+$.

Step i): preparation of (E)-3-(4-(((1-(4-(4-cyano-3-fluorophenyl)-5-(3-hydroxy-4-methoxyphenyl)thiophene-2-carbonyl) piperidin-4-yl)ami no)methyl)phenyl)-N-((tetrahydro-2H-pyran-2-yl)oxy)acrylamide

1M Solution of boron tribromide solution in DCM (0.78 g, 3.11 mmol) was added dropwise to a reaction flask containing (E)-3-(4-(((1-(5-(3-(benzyloxy)-4-methoxyphenyl)-4-(4-cyano-3-fluorophenyl)thiophene-2-carbonyl)piperidin-4-yl) amino)methyl)phenyl)-N-((tetrahydro-2H-pyran-2-yl)oxy)acrylamide (60 mg, 0.075 mmol) and DCM (5 mL) at -60°C, and the mixture was stirred at -60°C for 20 minutes. The reaction liquid was quenched by adding water (10 mL), then extracted with ethyl acetate (10 mL × 2), and the organic phases were combined, washed with saturated brine (10 mL × 2), dried over anhydrous sodium sulfate, and concentrated to afford (E)-3-(4-(((1-(4-(4-cyano-3-fluorophenyl)-5-(3-hydroxy-4-methoxyphenyl)thiophene-2-carbonyl)piperidin-4-yl)ami no)methyl)phenyl)-N-((tetrahydro-2H-pyran-2-yl)oxy)acrylamide with a yield of 75%.

ESI-MS m/z=711.3 $[M+H]^+$.

Step j): preparation of (E)-3-(4-(((1-(4-(4-cyano-3-fluorophenyl)-5-(3-hydroxy-4-methoxyphenyl)thiophene-2-carbonyl) piperidin-4-yl)ami no)methyl)phenyl)-N-hydroxyacrylamide hydrochloride

4.0M Hydrochloric acid solution in EA (4 mL) was added to a reaction flask containing (E)-3-(4-(((1-(4-(4-cyano-3-fluorophenyl)-5-(3-hydroxy-4-methoxyphenyl)thiophene-2-carbonyl)piperidin-4-yl)ami no)methyl)phenyl)-N-((tetrahydro-2H-pyran-2-yl)oxy)acrylamide (40 mg, 0.056 mmol), and the mixture was stirred at room temperature for 1 hour. After concentration of the reaction liquid, the residue was purified by Prep-HPLC (separation method 1) to afford (E)-3-(4-(((1-(4-(4-cyano-3-fluorophenyl)-5-(3-hydroxy-4-methoxyphenyl)thiophene-2-carbonyl)piperidin-4-yl)ami no) methyl)phenyl)-N-hydroxyacrylamide hydrochloride with a yield of 7.44%.

$^1$H NMR (400 MHz, DMSO-$d_6$) δ ppm 10.82 (s, 1H), 9.28 (d, *J* = 1.6 Hz, 1H), 9.04 (s, 1H), 7.88 (t, *J*= 7.6 Hz, 1H), 7.62 (s, 3H), 7.57 - 7.43 (m, 2H), 7.37 - 7.23 (m, 1H), 7.13 (d, *J* = 12.6 Hz, 2H), 6.94 (d, *J* = 8.4 Hz, 1H), 6.76 - 6.65 (m, 2H), 6.56 - 6.44 (m, 1H), 4.45 (d, *J* = 13.2 Hz, 2H), 4.19 (s, 2H), 3.78 (s, 3H), 2.74 (d, *J* = 2.4 Hz, 2H), 2.47 (s, 1H), 2.21 (d, *J* = 12.4 Hz, 2H), 1.67 (d, *J* = 12.8 Hz, 2H).

ESI-MS m/z=627.2 $[M+H]^+$.

## Example 34

Preparation of 7-(1-(7-(4-cyano-3-fluorophenyl)-8-(3-hydroxy-4-methylphenyl)imidazo[1,2-c]pyrimidin-5-yl)piperidin-4-yl)amino) -N-hydroxyheptanamide hydrochloride, and the starting material were prepared according to the synthetic method of Example 20.

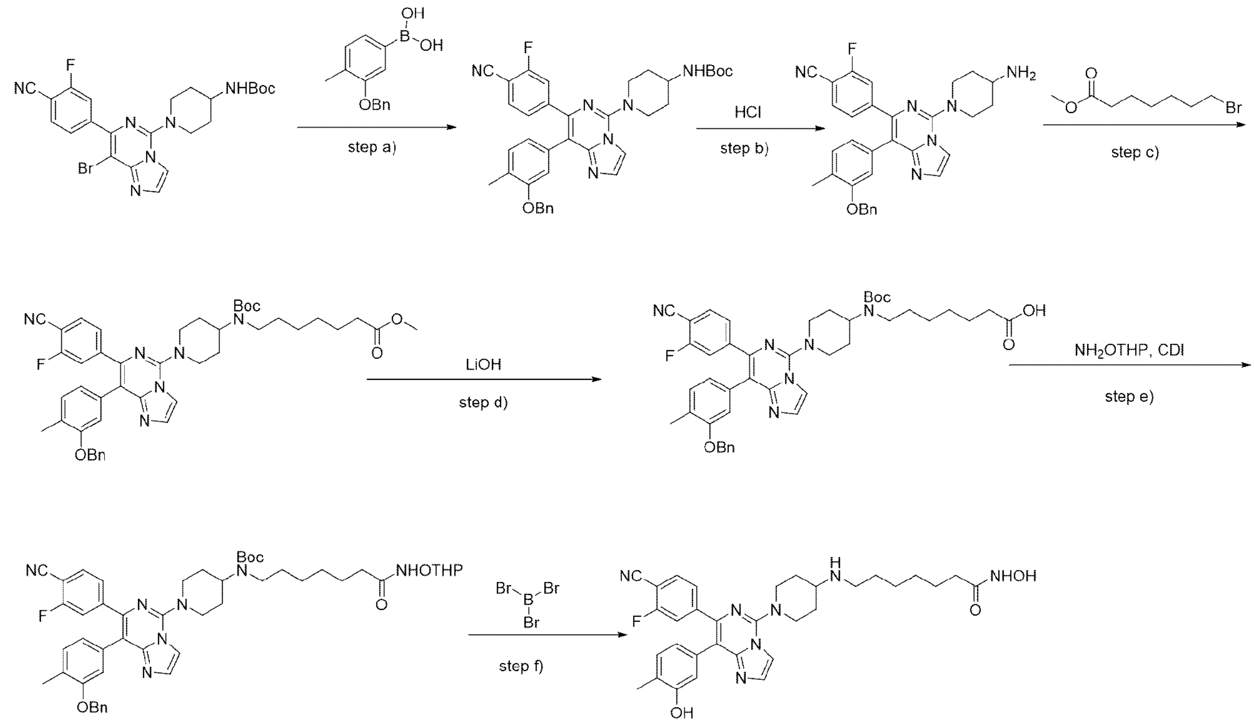


Step a): preparation of tert-butyl (1-(8-(3-(benzyloxy)-4-methylphenyl)-7-(4-cyano-3-fluorophenyl)imidazo[1,2-c]pyrimidin-5-yl)piperidin-4-yl)carb amate

1-(3-Amino-5-bromo-6-(4-cyano-3-fluorophenyl)-4-hydroxypyridin-2-yl)piperidin-4-yl)carbamic acid tert-butyl ester (350 mg, 0.68 mmol), cesium carbonate (664.6 mg, 2.04 mmol), (3-(benzyloxy)-4-methylphenyl)boronic acid (246.9 mg, 1.02 mmol), and [1,1'-bis(diphenylphosphino)ferrocene]palladium dichloride (6.0 mg, 0.01 mmol) were weighed and dissolved in a mixture of 1,4-dioxane (12.5 mL) and water (2.5 mL), and the reaction liquid was subjected to microwave heating at 110°C and reacted for 35 minutes under nitrogen protection. Water was added to dilute the mixture, which was then extracted with ethyl acetate (15 mL × 3), and the organic phases were combined and washed with saturated brine (10 mL × 2), dried over anhydrous sodium sulfate, filtered, and concentrated under reduced pressure. The residue was purified by silica gel chromatography (eluent: ethyl acetate/petroleum ether = 1:1) to afford tert-butyl (1-(8-(3-(benzyloxy)-4-methylphenyl)-7-(4-cyano-3-fluorophenyl)imidazo[1,2-c]pyrimidin-5-yl)piperidin-4-yl)carb amate with a yield of 84.9%.

ESI-MS m/z: 633.3[M+H] $^+$.

Step b): preparation of 4-(5-(4-aminopiperidin-1-yl)-8-(3-(benzyloxy)-4-methylphenyl)imidazo[1,2-c]pyrimidin-7-yl)-2-fluorobenzonitrile

Tert-butyl (1-(8-(3-(benzyloxy)-4-methylphenyl)-7-(4-cyano-3-fluorophenyl)imidazo[1,2-c]pyrimidin-5-yl)piperidin-4-yl)carb amate (230 mg, 0.36 mmol) was weighed and hydrochloric acid solution in ethyl acetate (4M, 2 mL) was added, and the mixture was stirred under nitrogen protection for 30 minutes. After the reaction was completed as indicated by LCMS, the reaction liquid was concentrated to dryness to afford 4-(5-(4-aminopiperidin-1-yl)-8-(3-(benzyloxy)-4-methylphenyl)imidazo[1,2-c]pyrimidin-7-yl)-2-fluorobenzonitrile with a yield of 95.1%.

ESI-MS m/z: 533.2[M+H] +.

Step c): preparation of methyl 7-(1-(8-(3-(benzyloxy)-4-methylphenyl)-7-(4-cyano-3-fluorophenyl)imidazo[1,2-c]pyrimidin-5-yl)piperidin-4-yl)(te rt-butoxycarbonyl)amino)heptanoate

4-(5-(4-Aminopiperidin-1-yl)-8-(3-(benzyloxy)-4-methylphenyl)imidazo[1,2-c]pyrimidin-7-yl)-2-fluorobenzon itrile (182.2 mg, 0.34 mmol), methyl 7-bromoheptanoate (169.5 mg, 0.76 mmol), and potassium carbonate (234.9 mg, 1.7 mmol) were dissolved in dry DMSO (2 mL), and the mixture was stirred under nitrogen protection at 50°C for 16 hours, then cooled to room temperature. Di-tert-butyl dicarbonate (83 mg, 0.38 mmol) was added, and the reaction was continued for an additional 30 minutes. The mixture was concentrated to dryness under reduced pressure, and the residue was purified by silica gel chromatography (eluent: ethyl acetate/petroleum ether = 2:1) to afford methyl 7-(1-(8-(3-(benzyloxy)-4-methylphenyl)-7-(4-cyano-3-fluorophenyl)imidazo[1,2-c]pyrimidin-5-yl)piperidin-4-yl)(te rt-butoxycarbonyl)amino)heptanoate with a yield of 17.8%.

ESI-MS m/z: 775.4[M+H]+.

Step d): preparation of 7-(1-(8-(3-(benzyloxy)-4-methylphenyl)-7-(4-cyano-3-fluorophenyl)imidazo[1,2-c]pyrimidin-5-yl)piperidin-4-yl)(te rt-butoxycarbonyl)amino)heptanoic acid

Methyl 7-(1-(8-(3-(benzyloxy)-4-methylphenyl)-7-(4-cyano-3-fluorophenyl)imidazo[1,2-c]pyrimidin-5-yl)piperidin-4-yl)(te rt-butoxycarbonyl)amino)heptanoate (80 mg, 0.10 mmol) was dissolved in a mixture of tetrahydrofuran : water = 1: 1 (2 mL), and lithium hydroxide (43.9 mg, 1.0 mmol) was added, and the mixture was stirred for reaction for 30 minutes, then extracted with ethyl acetate (10 mL × 3). The organic phases were combined and washed with saturated brine (10 mL × 2), dried with anhydrous sodium sulfate, filtered, and concentrated under reduced pressure to afford 7-(1-(8-(3-(benzyloxy)-4-methylphenyl)-7-(4-cyano-3-fluorophenyl)imidazo[1,2-c]pyrimidin-5-yl)piperidin-4-yl)(te rt-butoxycarbonyl)amino)heptanoic acid with a yield of 94.6%.

ESI-MS m/z: 761.4[M+H]+.

Step e): preparation of tert-butyl (1-(8-(3-(benzyloxy)-4-methylphenyl)-7-(4-cyano-3-fluorophenyl)imidazo[1,2-c]pyrimidin-5-yl)piperidin-4-yl)(7-o xo-7-(((tetrahydro-2-pyranyl)carbamoyl)amino)heptane

7-(1-(8-(3-(Benzyloxy)-4-methylphenyl)-7-(4-cyano-3-fluorophenyl)imidazo[1,2-c]pyrimidin-5-yl)piperidin-4-yl)(tert-butoxycarbonyl)amino)heptanoic acid (90 mg, 0.12 mmol) and N,N'-carbonyldiimidazole (97.3 mg, 0.60 mmol) were dissolved in dry THF (3 mL), and the mixture was heated to 40°C and stirred for 1 hour under nitrogen protection, then, triethylamine (121.4 mg, 1.2 mmol) was added, followed by adding 4-(aminooxy)tetrahydropyran (70.3 mg, 0.6 mmol), and the reaction was continued under nitrogen protection. After the reaction was completed as indicated by LCMS, the mixture was concentrated to dryness under reduced pressure, the residue was purified by silica gel chromatography (eluent: methanol/dichloromethane, 1:20) to afford tert-butyl (1-(8-(3-(benzyloxy)-4-methylphenyl)-7-(4-cyano-3-fluorophenyl)imidazo[1,2-c]pyrimidin-5-yl)piperidin-4-yl)(7-o xo-7-(((tetrahydro-2-pyranyl)carbamoyl)amino)heptane with a yield of 98.8%.

ESI-MS m/z: 860.4 [M+H] +.

Step f): preparation of 7-(1-(7-(4-cyano-3-fluorophenyl)-8-(3-hydroxy-4-methylphenyl)imidazo[1,2-c]pyrimidin-5-yl)piperidin-4-ylamino) -N-hydroxyheptanamide hydrochloride

tert-butyl (1-(8-(3-(benzyloxy)-4-methylphenyl)-7-(4-cyano-3-fluorophenyl)imidazo[1,2-c]pyrimidin-5-yl)piperidin-4-yl)(7-o xo-7-(((tetrahydro-2-pyranyl)carbamoyl)amino)heptane (97 mg, 0.11 mmol) was weighed and dissolved in dry dichloromethane (1 mL). Under nitrogen protection, the reaction liquid was cooled in an ice bath to 0°C, and 1M boron tribromide (1.5 mL) was added, and the mixture was stirred for reacton for 30 minutes, resulting in the precipitation of a solid. Water (3 mL) was added to quench the reaction, and the mixture was filtered and concentrated. The residue was purified by Pre-HPLC (separation method 1) to afford 7-(1-(7-(4-cyano-3-fluorophenyl)-8-(3-hydroxy-4-methylphenyl)imidazo[1,2-c]pyrimidin-5-yl)piperidin-4-ylamino) -N-hydroxyheptanamide hydrochloride with a yield of 22.9%.

$^1$H NMR (400 MHz, DMSO-$d_6$) δ ppm 10.39 (s, 1H), 9.71 (d, *J* = 16.2 Hz, 1H), 9.10 (s, 2H), 8.23 (s, 1H), 8.08 (s, 1H), 7.88 (dd, *J* = 8.2, 6.9 Hz, 1H), 7.59 (dd, *J* = 10.6, 1.5 Hz, 1H), 7.35 (dd, *J* = 8.2, 1.6 Hz, 1H), 7.17 (d, *J* = 7.6 Hz, 1H), 6.80 (d, *J* = 1.8 Hz, 1H), 6.64 (dd, *J* = 7.6, 1.8 Hz, 1H), 4.14 (s, 2H), 3.40 (s, 1H), 3.23 (t, *J* = 12.6 Hz, 2H), 2.92 (s, 2H), 2.18 (s, 5H), 1.94 (dt, *J* = 12.0, 5.9 Hz, 4H), 1.66 (p, *J* = 7.6 Hz, 2H), 1.50 (p, *J* = 7.4 Hz, 2H), 1.30 (dq, *J* = 12.6, 8.0, 7.0 Hz, 4H) .

ESI-MS m/z: 586.3 [M+H] +.

**Examples 35-36** were prepared according to the synthetic method of Example **34** (the separation method for the compounds: hydrochloride and formate were prepared according to separation method 1 and 3, respectively), and the structure and characterization data are as follows:

| Example | Chemical name | Structure | $^1H$ NMR | MS(M+H)$^+$ |
|---|---|---|---|---|
| 35 | 7-((1-(7-(4-Cyano-3-fluorophenyl)-8-(3-hydroxy-4-methoxyphenyl)imidazo[1,2-c]pyrimidin-5-yl)pi-peridin-4-yl)amin-o)-N-hydroxyheptanamide hydrochloride | 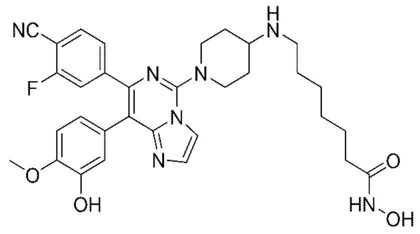 | $^1H$ NMR (400 MHz, Methanol-$d_4$) δ 8.22 (s, 1H), 8.01 (s, 1H), 7.68 (t, *J* = 7.2 Hz, 1H), 7.54 (d, *J* = 10.4 Hz, 1H), 7.45 (d, *J* = 8.0 Hz, 1H), 7.08 (d, *J* = 7.4 Hz, 1H), 6.83 (d, *J* = 11.0 Hz, 2H), 4.29 (d, *J* = 13.0 Hz, 2H), 3.93 (s, 3H), 3.55 (s, 1H), 3.39 (t, *J* = 12.0 Hz, 2H), 3.11 (s, 2H), 2.40-2.26 (m, 2H), 2.19 (d, *J* = 6.8 Hz, 2H), 2.17 - 1.99 (m, 2H), 1.84 - 1.59 (m, 4H), 1.46 (d, *J* = 13.8 Hz, 4H). | 602.3 |
| 36 | (E)-3-(4-(((1-(7-(4-Cyano-3-fluorophe-nyl)-8-(5-fluoro-3-methylbenzo[d]iso-xazol-6-yl)imidazo[1,2-c]pyrimidin-5-yl)piperidin-4-yl)amino)methyl)phenyl)-N-hydroxyacrylamide formate | 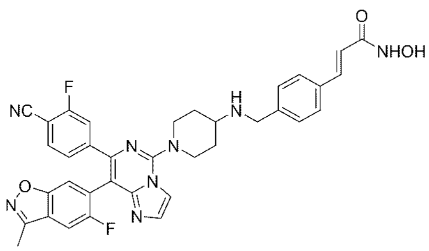 | $^1H$ NMR (400 MHz, DMSO-$d_6$) δ ppm: 10.76 (s, 1H), 8.38 (s, 1H), 7.93 (d, *J* = 1.6 Hz, 1H), 7.88 (d, *J* = 5.4 Hz, 1H), 7.79 (dd, *J* = 8.4, 6.4 Hz, 2H), 7.65 (d, *J* = 1.4 Hz, 1H), 7.58 (dd, *J* = 10.8, 1.6 Hz, 1H), 7.52 (d, *J* = 8.0 Hz, 2H), 7.45 - 7.39 (m, 3H), 7.28 (dd, *J* = 8.2, 1.6 Hz, 1H), 4.05 - 3.94 (m, 2H), 3.81 (s, 2H), 3.16 (t, *J* = 11.8 Hz, 2H), 2.75 (dt, *J* = 9.8, 5.4 Hz, 1H), 2.57 (s, 3H), 2.04 (d, *J* = 12.6 Hz, 2H), 1.61 (d, *J* = 12.0 Hz, 2H). | 661.2 |

**Example 37**

Preparation of (E)-3-(4-(((1-(3-cyano-4-(4-cyano-3-fluorophenyl)-5-(3-hydroxy-4-methoxyphenyl)pyridin-2-yl)piperidin-4-yl)amino)methyl)phenyl)-N-hydroxyacrylamide hydrochloride

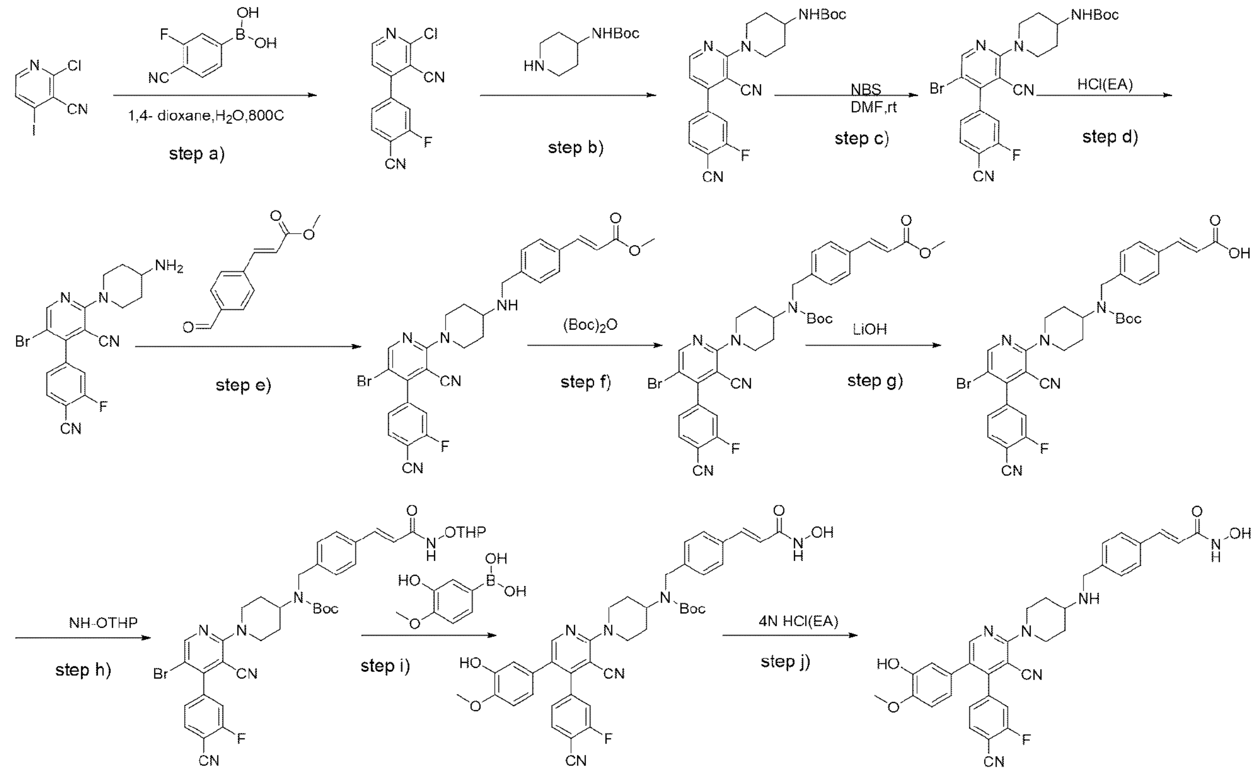


Step a): preparation of 2-chloro-4-(4-cyano-3-fluorophenyl)nicotinonitrile

2-Chloro-4-iodopyridine-3-carbonitrile (6.4 g, 24.20 mmol), (4-cyanophenyl)boronic acid (4.19 g, 25.41 mmol), [1,1'-bis(diphenylphosphino)ferrocene]dichloropalladium(II) (1.77 g, 2.42 mmol), and cesium carbonate (14.19 g, 43.56 mmol) were added to a mixture of 1,4-Dioxane:$H_2O$ = 5:1 (80 mL). After nitrogen purging, the reaction liquid was heated to 100°C and reacted for 1 hour, and concentrating was conducted to remove the solvent. The residue was mixed with silica gel and purified a normal-phase column to afford 2-chloro-4-(4-cyano-3-fluorophenyl)pyridine-3-carbonitrile with a yield of 93.02%.

ESI-MS m/z: 258.1 $[M+H]^+$.

Step b): preparation of tert-butyl N-(1-(3-cyano-4-(4-cyano-3-fluorophenyl)pyridin-2-yl)piperidin-4-yl)carbamate

2-Chloro-4-(4-cyano-3-fluorophenyl)pyridine-3-carbonitrile (2.0 g, 7.76 mmol), tert-butyl piperidin-4-ylcarbamate (1.6 g, 8.15 mmol), and ethyl bis(2-propyl)amine (1.2 g, 9.31 mmol) were added to NMP (100 mL), and the mixture was heated to 120°C and reacted overnight. The reaction was monitored by LCMS until the starting materials were completely consumed, the reaction liquid was then extracted by adding water and ethyl acetate. The organic phases were combined, washed with saturated brine, and concentrating was conducted to remove the solvent. The residue was mixed with silica gel and purified by a normal-phase column to afford tert-butyl N-(1-(3-cyano-4-(4-cyano-3-fluorophenyl) pyridin-2-yl)piperidin-4-yl)carbamate with a yield of 85.6%.

ESI-MS m/z: 422.2 $[M+H]^+$.

Step c): preparation of tert-butyl N-(1-(5-bromo-3-cyano-4-(4-cyano-3-fluorophenyl)pyridin-2-yl)piperidin-4-yl)carbamate

Tert-butyl (1-(3-cyano-4-(4-cyano-3-fluorophenyl)pyridin-2-yl)piperidin-4-yl)carbamate (2.0 g, 4.7 mmol) and N-bromosuccinimide (3.3 g, 18.5 mmol) were added to DMF (4 mL), and the mixture was stirred at room temperature for 1 hour, and the reaction was monitored by LCMS until the starting materials were completely consumed. Water was then added to the system, and the mixture was extracted with ethyl acetate. The organic phases were combined, and concentrating was conducted to remove the solvent. The residue was mixed with silica gel and purified by a normal-phase column to afford tert-butyl N-(1-(5-bromo-3-cyano-4-(4-cyano-3-fluorophenyl)pyridin-2-yl)piperidin-4-yl)carbamate with a yield of 93.5%.

ESI-MS m/z: 500.1 $[M+H]^+$.

Step d): preparation of 2-(4-Aminopiperidin-1-yl)-5-bromo-4-(4-cyano-3-fluorophenyl)pyridine-3-formonitrile

Tert-butyl N-(1-(5-bromo-3-cyano-4-(4-cyano-3-fluorophenyl)pyridin-2-yl)piperidin-4-yl)carbamate (1.0 g, 2.0 mmol) was added to 4N HCl/EA (10 mL) and the mixture was reacted at room temperature for 0.5 hours, and the reaction was monitored by LCMS until the starting materials were completely consumed. The reaction system was poured into a saturated sodium bicarbonate aqueous solution, then ethyl acetate was then added for extraction, and the organic phase was washed with saturated brine. After concentrating by removing the solvent, the crude product 2-(4-aminopiperidin-1-yl)-5-bromo-4-(4-cyano-3-fluorophenyl)pyridine-3-formonitrile was obtained with a yield of 91.2%.
ESI-MS m/z: 400.1 $[M+H]^+$.

Step e): preparation of methyl (E)-3-(4-((1-(5-bromo-3-cyano-4-(4-cyano-3-fluorophenyl)pyridin-2-yl)piperidin-4-yl) amino)methyl)phenyl)acrylat e

2-(4-Aminopiperidin-1-yl)-5-bromo-4-(4-cyano-3-fluorophenyl)pyridine-3-formonitrile (600 mg, 1.50 mmol) and methyl (E)-3-(4-formylphenyl)acrylate (381.55 mg, 1.50 mmol) were added to a mixture of DCE:MeOH:AcOH = 10:0.1:0.01 (20 mL), and the mixture was stirred at room temperature for 0.5 hours, then cooled in an ice bath, and sodium cyanoborohydride (285.26 mg, 4.5 mmol) was added. The reaction was maintained in the ice bath for 2 hours and monitored by LCMS, which showed approximately 5% of the starting material was left and the product was dominant. Then the reaction was stopped, and sodium bicarbonate solution was added into the system to quench the reaction. It was directly used for the next reaction step.
ESI-MS m/z: 574.1 $[M+H]^+$.

Step f): preparation of methyl (E)-3-(4-((1-(5-bromo-3-cyano-4-(4-cyano-3-fluorophenyl)pyridin-2-yl)piperidin-4-yl)(tert-butoxycarbonyl)amino) methyl)phenyl)acrylate

Methyl (E)-3-(4-{[(1-(5-bromo-3-cyano-4-(4-cyano-3-fluorophenyl)pyridin-2-yl)piperidin-4-yl)amino]methyl} phenyl)prop-2-enoate (200 mg, 0.35 mmol) was dissolved in a mixture of $THF/H_2O$ = 5:1 (5 mL), and di-tert-butyl dicarbonate (78 mg, 0.35 mmol) was added, and the reaction was conducted at room temperature for 1 hour. The reaction was monitored by LCMS until the starting materials were completely consumed. EA was added for extraction, and the organic phase was washed with saturated brine. After concentrating was conducted to remove the solvent, the residue was mixed with silica gel and purified by a normal-phase column to afford methyl (E)-3-(4-{[(1-(5-bromo-3-cyano-4-(4-cyano-3-fluorophenyl)pyridin-2-yl)piperidin-4-yl)(tert-butoxycarbonyl)amino) methyl)phenyl)prop-2-enoate with a yield of 84.71%.
ESI-MS m/z: 674.2 $[M+H]^+$.

Step g): preparation of (E)-3-(4-((1-(5-bromo-3-cyano-4-(4-cyano-3-fluorophenyl)pyridin-2-yl)piperidin-4-yl)(tert-butoxycarbonyl)amino) methyl)phenyl)acrylic acid

Methyl (E)-3-(4-{[(1-(5-bromo-3-cyano-4-(4-cyano-3-fluorophenyl)pyridin-2-yl)piperidin-4-yl)[(tert-butoxycarbonyl]amin o]methyl}phenyl)prop-2-enoate (100 mg, 0.15 mmol) and lithium hydroxide (17.96 mg, 0.75 mmol) were added to a mixture of $THF/H_2O$ =5:1 (1 mL), and the reaction was conducted at room temperature overnight. The reaction was monitored by LCMS until the starting materials were completely consumed and the product was dominant. After the reaction was completed, the pH was adjusted to weakly acidic with dilute hydrochloric acid, and EA was then added for extraction. The organic phase was washed with saturated brine, and concentrating was conducted to remove the solvent to obtain a solid product, which was directly used in the next reaction step.
ESI-MS m/z: 660.2 $[M+H]^+$.
Step h): preparation of tert-butyl (E)-(1-(5-bromo-3-cyano-4-(4-cyano-3-fluorophenyl)pyridin-2-yl)piperidin-4-yl) (4-(3-oxo-3-((tetrahydro-2H-pyran -2-yl)oxy)amino)prop-1-en-1-yl)benzyl)carbamate (E)-3-(4-{[(1-(5-bromo-3-cyano-4-(4-cyano-3-fluorophenyl)pyridin-2-yl)piperidin-4-yl)[(tert-butoxy)carbonyl]amin o]methyl}phenyl)prop-2-enoic acid (100 mg, 0.15 mmol), O-(tetrahydro-2H-pyran-2-yl)hydroxylamine (21.09 mg, 0.18 mmol), HATU (68.44 mg, 0.18 mmol), and ethyldiisopropylamine (58.16 mg, 0.45 mmol) were added to DMF (4 mL). The reaction was conducted at room temperature for 0.5 hours and monitored by LCMS until the starting materials were completely consumed and the product was dominant. The system was poured into water, and EA was added for extraction. The organic phase was concentrated by removing the solvent, the residue was mixed with silica gel and purified by a normal-phase column to afford the product tert-butyl (E)-(1-(5-bromo-3-cyano-4-(4-cyano-3-fluorophenyl)pyridin-2-yl)piperidin-4-yl)(4-(3-oxo-3-((tetrahydro-2H-pyran -2-yl)oxy)amino)prop-1-en-1-yl)benzyl)carbamate with a yield of 71.96%.
ESI-MS m/z: 759.2 $[M+H]^+$.

Step i): preparation of tert-butyl (E)-(1-(3-cyano-4-(4-cyano-3-fluorophenyl)-5-(3-hydroxy-4-methoxyphenyl)pyridin-2-yl)piperidin-4-yl)(4-(3-yloxy -3-((tetrahydro-2H-pyran-2-yl)oxy)amino)prop-1-en-1-yl)benzyl)carbamate

Tert-butyl (E)-(1-(5-bromo-3-cyano-4-(4-cyano-3-fluorophenyl)pyridin-2-yl)piperidin-4-yl)(4-(3-oxo-3-((tetrahydro-2H-pyran -2-yl)oxy)amino)prop-1-en-1-yl)benzyl)carbamate (100 mg, 0.13 mmol), 2-methoxy-5-(tetramethyl-1,3,2-dioxaborolan-2-yl)phenol (48.77 mg, 0.20 mmol), [1,1'-bis(diphenylphosphino)ferrocene]palladium(II) dichloride (9.51 mg, 0.013 mmol), and cesium carbonate (84.71 mg, 0.26 mmol) were added to a mixture of dioxane/$H_2O$ = 5:1(5 mL). After purging with nitrogen, the mixture was subjected to microwave heating at 110°C for 1 hour, and the reaction was monitored by LCMS until the starting materials were completely consumed andthe product was dominant. Concentrating was directly conducted by removing the solvent, and the residue was mixed with silica gel and purified by a normal-phase column to afford tert-butyl (E)-(1-(3-cyano-4-(4-cyano-3-fluorophenyl)-5-(3-hydroxy-4-methoxyphenyl)pyridin-2-yl)piperidin-4-yl)(4-(3-oxo-3-((tetrahydro-2H-pyran-2-yl)oxy)amino)prop-1-en-1-yl)benzyl)carbamate with a yield of 82.39%.
ESI-MS m/z: 719.3 $[M+H]^+$.

Step j): preparation of (E)-3-(4-(((1-(3-cyano-4-(4-cyano-3-fluorophenyl)-5-(3-hydroxy-4-methoxyphenyl)pyridin-2-yl)piperidin-4-yl)ami no)methyl)phenyl)-N-hydroxyacrylamide hydrochloride

Tert-butyl (E)-(1-(3-cyano-4-(4-cyano-3-fluorophenyl)-5-(3-hydroxy-4-methoxyphenyl)pyridin-2-yl)piperidin-4-yl)(4-(3-oxo-3-((tetrahydro-2H-pyran-2-yl)oxy)amino)prop-1-en-1-yl)benzyl)carbamate (60 mg, 75 μmol) was added to 4N HCl (EA) (4 mL), and the reaction was conducted at room temperature for 0.5 hours and monitored by LCMS until the starting materials were completely consumed. Concentrating was conducted to remove the solvent, and the product was purified by Prep-HPLC (separation method 1) to afford (E)-3-(4-(((1-(3-cyano-4-(4-cyano-3-fluorophenyl)-5-(3-hydroxy-4-methoxyphenyl)pyridin-2-yl)piperidin-4-yl)ami no)methyl)phenyl)-N-hydroxyacrylamide hydrochloride with a yield of 71.12%.
$^1$H NMR (400 MHz, DMSO-$d_6$) δ 10.41 (d, *J* = 18.6 Hz, 1H), 8.39 (s, 3H), 7.86 (t, *J*= 7.6 Hz, 1H), 7.50 (d, *J* = 10.4 Hz, 1H), 7.29 (dd, *J* = 8.0, 1.4 Hz, 1H), 6.84 (d, *J* = 8.0 Hz, 1H), 6.70 (s, 1H), 6.59 (d, *J* = 2.2 Hz, 1H), 6.45 (dd, *J* = 8.2, 2.0 Hz, 1H), 4.47 (d, *J* = 13.2 Hz, 2H), 4.17 (t, *J* = 6.2 Hz, 2H), 3.79 (s, 3H), 3.39 (dq, *J* = 11.4, 5.6 Hz, 1H), 3.13 (t, *J* = 12.8 Hz, 2H), 2.13 - 2.04 (m, 2H), 1.98 (t, *J* = 7.4 Hz, 2H), 1.68 (tq, *J* = 13.6, 6.8, 5.2 Hz, 4H), 1.51 (p, *J* = 7.4 Hz, 2H), 1.30 (dq, *J* = 16.8, 6.6, 5.4 Hz, 4H).
ESI-MS m/z: 619.2 $[M+H]^+$.
**Examples 39-53** were prepared according to the synthetic method of Example 37 (the separation method for the compounds: hydrochloride and formate were prepared by separation method 1 and 3, respectively), and the structure and characterization data are as follows:

| Example | Chemical name | Structure | $^1$H NMR | MS(M+H)$^+$ |
|---|---|---|---|---|
| 39 | 4-((4-(4-(5-Cyano-4-(4-cyano-3-fluorophenyl)-6-(4-( methylamino)piperidin-1-y l)pyridin-3-yl)-2-hydroxyp henyl)amino) methyl)-N-hy droxybenzamide hydrochloride | 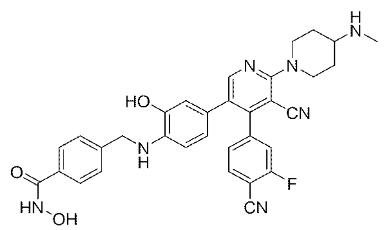 | (m, 2H), 1.80 (qd, *J* = 12.2, 3.9 Hz, 2H). | 592.3 |

(continued)

| Example | Chemical name | Structure | $^1$H NMR | MS(M+H)$^+$ |
|---|---|---|---|---|
| 40 | (E)-3-(4-(((1-(3-Cyano-4-( 4-cyano-3-fluorophenyl)-5 -(3-fluoro-4-methoxyphen yl)pyridin-2-yl)piperidin-4 -yl)amino) methyl)phenyl)-N-hydroxyacrylamide hydrochloride | 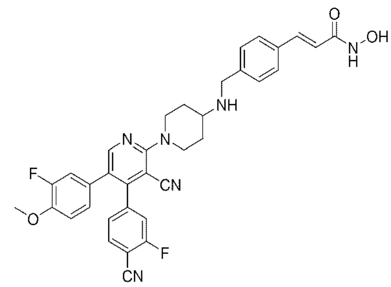 | $^1$H NMR (400 MHz, DMSO-$d_6$) δ 8.48 (s, 3H), 8.46 (s, 1H), 7.97 (dd, *J* = 8.0, 6.8 Hz, 1H), 7.67 (dd, *J* = 10.0, 1.6 Hz, 1H), 7.50 (d, *J* = 8.0 Hz,2H), 7.41 (t, *J* = 8.8 Hz, 2H), 7.37 - 7.27 (m, 2H), 7.10 - 6.94 (m,2H), 6.79 (dt, *J* = 8.4, 1.6 Hz, 1H), 6.45 (d, *J* = 15.8 Hz, 1H), 4.19 (d, *J* = 13.0 Hz, 2H), 3.79 (m, 5H), 3.19 (t, *J* = 11.8 Hz, 2H), 2.77 - 2.63 (m, 1H), 2.03 - 1.88 (m, 2H), 1.43 (q, *J* = 11.2, 10.8 Hz, 2H). | 621.2 |
| 41 | 4-(((1-(3-Cyano-4-(4-cyan o-3-fluorophenyl)-5-(3-hy droxy-4-methoxyphenyl)p yridin-2-yl)piperidin-4-yl) amino)methyl)-N-hydroxy benzamide hydrochloride | 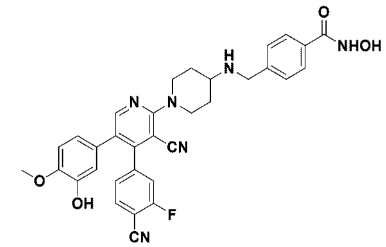 | $^1$H NMR (400 MHz, Methanol-$d_4$) δ 8.50 (s, 1H), 8.42 (s, 1H), 7.80 (d, *J* = 7.6 Hz, 2H), 7.74 (t, *J* = 7.2 Hz, 1H), 7.56 (d, *J* = 7.8 Hz, 2H), 7.36 (d, *J* = 9.6 Hz, 1H), 7.23 (d, *J* = 7.8 Hz, 1H), 6.81 (d, *J* = 8.6 Hz, 1H), 6.50 (d, *J* = 6.6 Hz, 2H), 4.41 (d, *J* = 13.2 Hz, 2H), 4.15 (s, 2H), 3.81 (s, 3H), 3.15 (t, *J* = 12.6 Hz, 3H), 2.22 (d, *J* = 12.2 Hz, 2H), 1.74 (d, *J* = 12.6 Hz, 2H). | 593.2 |
| 42 | (E)-3-(4-(((1-(3-Cyano-4-( 4-cyano-3-fluorophenyl)-5 -(3-hydroxy-4-methoxyphe nyl)pyridin-2-yl)piperidin-4-yl)(methyl) amino)methy l)phenyl)-N-hydroxyacryla mide formate | 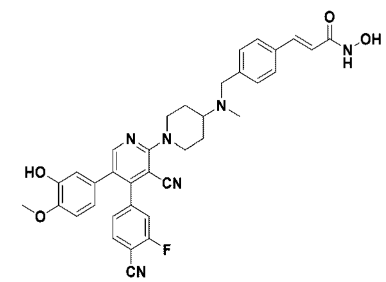 | $^1$H NMR (400 MHz, DMSO-$d_6$) δ 10.73 (s, 1H), 9.05 (s, 1H), 8.42 (s, 1H), 7.95 (dd, *J* = 8.0, 6.8 Hz, 1H), 7.66 (dd, *J* = 10.2, 1.6 Hz, 1H), 7.51 (d, *J* = 7.8 Hz, 2H), 7.44 (d, *J* = 15.8 Hz, 1H), 7.36 (d, *J* = 7.8 Hz, 2H), 7.28 (dd, *J* = 8.0, 1.6 Hz, 1H), 6.80 (d, *J* = 8.0 Hz, 1H), 6.52 - 6.39 (m, 3H), 4.34 (d, *J* = 12.8 Hz, 2H), 3.71 (s, 3H), 3.61 (s, 2H), 3.07 (t, *J* = 12.4 Hz, 2H), 2.80 - 2.62 (m, 1H), 2.14 (s, 3H), 1.93 (d, *J* = 12.2 Hz, 2H), 1.77 - 1.54 (m, 2H). | 633.3 |

(continued)

| Example | Chemical name | Structure | $^1$H NMR | MS(M+H)$^+$ |
|---|---|---|---|---|
| 43 | 3-(4-((1-(3-Cyano-4-(4-cyano-3-fluorophenyl)-5-(3-hydroxy-4-methoxyphenyl) pyridin-2-yl)piperidin-4-yl ) amino)methyl)phenyl)-N-hydroxypropanamide formate | 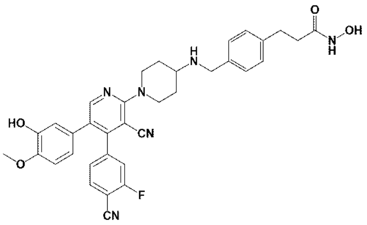 | $^1$H NMR (400 MHz, DMSO-$d_6$) δ 8.48 (s, 2H), 8.46 (s, 1H), 7.97 (dd, *J* = 8.0, 6.8 Hz, 1H), 7.67 (dd, *J* = 10.0, 1.6 Hz, 1H), 7.50 (d, *J* = 8.0 Hz,2H), 7.41 (t, *J* = 8.8 Hz, 2H), 7.37 - 7.27 (m, 2H), 7.10 - 6.94 (m,2H), 6.79 (dt, *J* = 8.4, 1.6 Hz, 1H), 6.45 (d, *J* = 15.8 Hz, 1H), 4.19 (d, *J* = 13.0 Hz, 2H), 3.79 (m, 5H), 3.19 (t, *J* = 11.8 Hz, 2H), 2.77 - 2.63 (m, 3H),2.28 (t, *J* = 12.0, 2H) 2.03-1.88 (m, 2H), 1.43 (q, *J* = 11.2, 10.8 Hz, 2H). | 621.3 |
| 44 | (E)-3-(4-(((1-(3-Cyano-4-( 4-cyano-3-fluorophenyl)-5 -(3-hydroxy-4-methoxyphe nyl)pyridin-2-yl)-4-methyl piperidin-4-yl) amino)meth yl)phenyl)-N-hydroxyacryl amide formate | 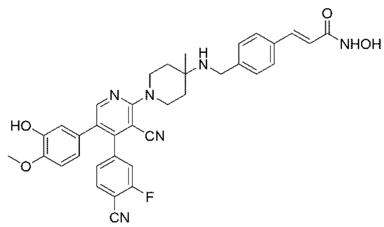 | $^1$H NMR (400 MHz, DMSO-$d_6$) δ ppm: 8.44 (s, 2H), 8.40 (s, 1H), 7.95 (dd, *J* = 8.0, 6.8 Hz, 1H), 7.66 (dd, *J* = 10.2, 1.6 Hz, 1H), 7.47 - 7.39 (m, 5H), 7.27 (dd, *J* = 8.0, 1.6 Hz, 1H), 6.83 - 6.77 (m, 1H), 6.50 - 6.44 (m, 2H), 3.79 (d, *J* = 13.6 Hz, 2H), 3.71 (m, 5H), 3.67 (d, *J* = 10.2 Hz, 2H), 1.81-1.71 (m, 2H), 1.64 - 1.52 (m, 2H), 1.18 (s, 3H). | 633.3 |
| 45 | (E)-3-(4-(2-((1-(3-Cyano-4 -(4-cyano-3-fluorophenyl)-5-(3-hydroxy-4-methoxyp henyl)pyridin-2-yl)piperidi n-4-yl)amino)ethyl) phenyl )-N-hydroxyacrylamide hydrochloride | 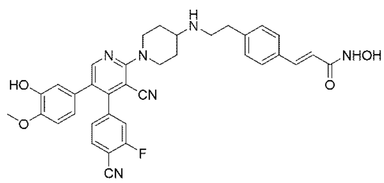 | $^1$H NMR (400 MHz, Methanol-$d_4$) δ 8.44 (s, 1H), 7.74 (dd, *J* = 8.0, 6.6 Hz, 1H), 7.63 - 7.48 (m, 3H), 7.37 (dd, *J* = 8.0, 1.8 Hz, 3H), 7.23 (dd, *J* = 8.0, 1.6 Hz, 1H), 6.90 - 6.75 (m, 1H), 6.59 - 6.36 (m, 3H), 4.43 (d, *J* = 13.6 Hz, 2H),3.5 (s, 1H), 3.81 (s, 3H), 3.37 (d, *J* = 8.2 Hz, 2H), 3.17 (t, *J* = 12.0 Hz, 2H), 3.10 - 2.98 (m, 2H), 2.27 (d, *J* = 10.8 Hz, 2H), 1.91 - 1.73 (m, 2H). | 633.3 |

(continued)

| Example | Chemical name | Structure | 1H NMR | MS(M+H)+ |
|---|---|---|---|---|
| 46 | 5-(1-(3-Cyano-4-(4-cyano-3-fluorophenyl)-5-(3-hydr oxy-4-methoxyphenyl) pyri din-2-yl)piperidin-4-yl) ami no)-N-hydroxypentanamid e formate | 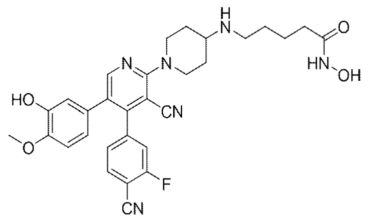 | $^1$H NMR (400 MHz, DMSO-$d_6$) δ ppm: 8.42 (s, 1H), 8.26 (s, 1H), 7.95 (dd, *J* = 8.0, 6.8 Hz, 1H), 7.66 (dd, *J* = 10.2, 1.4 Hz, 1H), 7.27 (dd, *J* = 8.0, 1.6 Hz, 1H), 6.84 - 6.76 (m, 1H), 6.47 (d, *J* = 7.2 Hz, 2H), 4.21 (d, *J* = 13.0 Hz, 2H), 3.71 (s, 3H), 3.22 - 3.08 (m, 3H), 2.70 (t, *J* = 7.2 Hz, 2H), 2.07 - 1.93 (m, 4H), 1.62 - 1.40 (m, 6H). | 559.3 |
| 47 | 1-(3-Cyano-4-(4-cyano-3-fluorophenyl)-5-(3-hydroxy-4-methoxyphenyl)pyridin-2-yl)piperidin-4-ylamino)-N-hydroxyheptanamide formate | 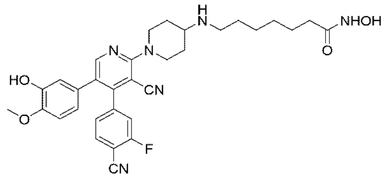 | $^1$H NMR (400 MHz, DMSO-$d_6$) δ ppm: 10.35 (s, 1H), 9.05 (s, 1H), 8.43 (s, 1H), 8.29 (s, 1H), 7.96 (dd, *J* = 8.0, 6.8 Hz, 1H), 7.67 (dd, *J* = 10.2, 1.6 Hz, 1H), 7.28 (dd, J = 8.0, 1.6 Hz, 1H), 6.84 - 6.75 (m, 1H), 6.52 - 6.42 (m, 2H), 4.22 (d, *J* = 13.2 Hz, 2H), 3.71 (s, 3H), 3.14 (t, *J* = 12.2 Hz, 2H), 2.96 (s, 1H), 2.70 (q, *J* = 8.8, 8.2 Hz, 2H), 2.04 (d, *J* = 12.2 Hz, 2H), 1.94 (t, *J* = 7.4 Hz, 2H), 1.49 (p, J = 7.6 Hz, 6H), 1.28 (tt, *J* = 12.4, 6.8 Hz, 4H). | 587.3 |
| 48 | 2-(1-(3-Cyano-4-(4-cyano-3-fluorophenyl)-5-(3-hydr oxy-4-methoxyphenyl) pyri din-2-yl)piperidin-4-yl)-N-hydroxyacetamide formate | 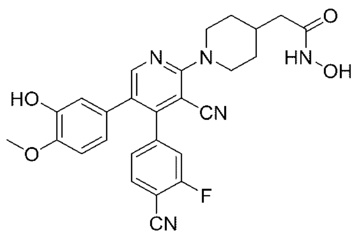 | $^1$H NMR (400 MHz, Methanol-$d_4$) δ ppm 8.55 (s, 1H), 8.38 (s, 1H), 7.73 (dd, *J* = 8.0, 6.6 Hz, 1H), 7.35 (dd, *J* = 9.8, 1.6 Hz, 1H), 7.22 (dd, *J* = 8.0, 1.6 Hz, 1H), 6.88 - 6.77 (m, 1H), 6.50 (d, *J* = 6.8 Hz, 2H), 4.33 (d, *J* = 13.0 Hz, 2H), 3.80 (s, 3H), 3.21 - 2.93 (m, 2H), 2.16 - 2.02 (m, 3H), 1.94-1.76 (m, 2H), 1.43 (q, *J* = 11.6, 11.2 Hz, 2H). | 502.2 |

(continued)

| Example | Chemical name | Structure | $^1$H NMR | MS(M+H)$^+$ |
|---|---|---|---|---|
| 49 | (E)-3-(4-(2-(4-(3-Cyano-4-(4-cyano-3-fluorophenyl)-5-(3-hydroxy-4-methoxyp henyl)pyridin-2-yl)piperazi n-1-yl)ethyl)phenyl)-N-hy droxyacrylamide hydrochloride | 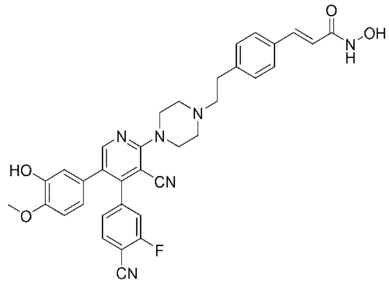 | $^1$H NMR (400 MHz, DMSO-$d_6$) δ ppm 11.04 (s, 1H), 10.77 (s, 1H), 9.07 (s, 1H), 8.53 (s, 1H), 7.99 (t, *J* = 7.2 Hz, 1H), 7.66 (d, *J* = 10.0 Hz, 1H), 7.55 (d, *J* = 8.0 Hz, 2H), 7.44 (d, *J* = 8.0 Hz, 1H), 7.34 (d, *J* = 8.0 Hz, 2H), 7.28 (d, *J* = 8.0 Hz, 1H), 6.83 (d, *J* = 8.0 Hz, 1H), 6.50-6.44 (m, 3H), 4.31 (d, *J* = 13.6 Hz, 2H), 3.78-3.72 (m, 5H), 3.62-3.56 (m, 4H), 3.27-3.22 (m, 2H), 3.15-3.11 (m, 2H). | 619.3 |
| 50 | (E)-3-(4-(((1-(3-Cyano-4-( 4-cyano-3-fluorophenyl)-5 -(3-hydroxy-4-methoxyphe nyl)-6-methylpyridin-2-yl) piperidin-4-yl) amino)meth yl)phenyl)-N-hydroxyacryl amideformate | 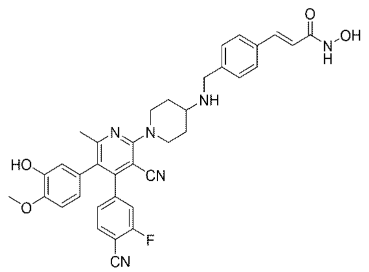 | $^1$H NMR (400 MHz, Methanol-$d_4$) δ 8.53 (m, 4H), 7.63 (dd, *J* = 16.8, 7.6 Hz, 3H), 7.52 (d, *J* = 15.6 Hz, 2H), 7.25 (d, *J* = 9.6 Hz, 1H), 7.16 (d, *J* = 7.8 Hz, 1H), 6.80 (d, *J* = 8.0 Hz, 1H), 6.50 - 6.41 (m, 2H), 4.42 (d, *J* = 13.0 Hz, 2H), 4.09 (s, 2H), 3.80 (s, 3H), 3.12 (dd, *J* = 15.8, 9.0 Hz, 3H), 2.31 (s, 3H), 2.19 (t, *J* = 7.8 Hz, 2H), 1.72 (d, *J* = 12.8 Hz, 2H). | 633.3 |
| 51 | (E)-4-((1-(3-Cyano-4-(4-cyano-3-fluorophenyl)-5-(3-hydroxy-4-methoxyphenyl ) pyridin-2-yl)piperidin-4-y l) amino)-N-hydroxy-2-ena mide hydrochloride | 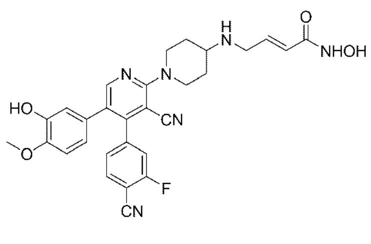 | $^1$H NMR (400 MHz, Methanol-$d_4$) δ 8.45 (s, 1H), 7.76 (dd, *J* = 8.0, 6.6 Hz, 1H), 7.38 (dd, *J* = 9.8, 1.4 Hz, 1H), 7.25 (dd, *J* = 8.0, 1.4 Hz, 1H), 6.92 - 6.70 (m, 2H), 6.52 (dd, *J* = 5.8, 2.2 Hz, 2H), 6.27 (d, *J* = 15.4 Hz, 1H), 4.45 (d, *J* = 13.6 Hz, 2H), 3.95 (d, *J* = 7.0 Hz, 2H), 3.82 (s, 3H), 3.50 (s, 1H), 3.20 (t, *J* = 12.6 Hz, 2H), 2.29 (d, *J* = 11.0 Hz, 2H), 1.85 (tt, *J* = 12.4, 6.2 Hz, 2H). | 543.2 |

(continued)

| Example | Chemical name | Structure | $^1H$ NMR | MS(M+H)$^+$ |
|---|---|---|---|---|
| 52 | (E)-3-(4-(((1-(3-Cyano-4-( 4-cyano-3-fluorophenyl)-5 -(4-methoxyphenyl)pyridin -2-yl)piperidin-4-yl)amino )methyl)phenyl)-N-hydrox yacrylamide-hydrochloride | 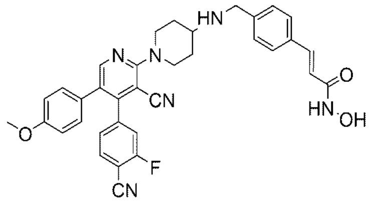 | $^1H$ NMR (400 MHz, DMSO-$d_6$) δ 10.80 (s, 1H), 9.20 (s, 2H), 8.50 (s, 1H), 8.01 - 7.94 (m, 1H), 7.71 - 7.58 (m, 5H), 7.48 (m, 1H), 7.30 (dd, *J* = 8.0, 1.4 Hz, 1H), 7.03 - 6.97 (m, 2H), 6.87 - 6.81 (m, 2H), 6.52 (d, *J* = 15.6 Hz, 1H), 4.40 - 4.18 (m, 5H), 3.71 (s, 3H), 3.40 (s, 2H), 3.13 (t, *J* = 12.6 Hz, 2H), 2.28 (d, *J* = 12.2 Hz, 2H), 1.79 (d, *J* = 11.8 Hz, 3H). | 603.3 |
| 53 | (E)-3-(4-(((1-(3-Cyano-4-( 4-cyano-3-fluorophenyl)-5 -(2-fluoro-3-hydroxypheny l)pyridin-2-yl) piperidin-4-yl)amino) methyl)phenyl)-N-hydroxyacrylamideform ate | 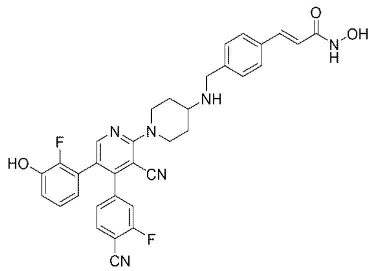 | $^1H$ NMR (400 MHz, DMSO-$d_6$) δ ppm: 8.41 (s, 1H), 8.38 (s, 2H), 7.93 (t, *J* = 7.4 Hz, 1H), 7.62 (dd, *J* = 10.2, 1.4 Hz, 1H), 7.50 (d, *J* = 7.8 Hz, 2H), 7.40 (d, *J* = 8.0 Hz, 3H), 7.27 (dd, *J* = 8.0, 1.4 Hz, 1H), 6.88 (dd, *J* = 6.2, 4.2 Hz, 2H), 6.58 (dq, *J* = 6.8, 3.4, 2.2 Hz, 1H), 6.46 (d, *J* = 15.8 Hz, 1H), 4.23 (d, *J* = 13.4 Hz, 2H), 3.79 (s, 2H), 3.22 (t, *J* = 12.0 Hz, 2H), 2.78 - 2.66 (m, 1H), 2.04-1.93 (m, 2H), 1.44 (q, *J* = 11.0, 10.2 Hz, 2H). | 607.2 |

**Example 54**

Preparation of (E)-3-(4-(((1-(6-(4-cyano-3-fluorophenyl)-5-(3-hydroxy-4-methoxyphenyl)-4-methoxypyridin-2-yl)piperidin-4-yl)a mino)methyl)phenyl)-N-hydroxyacrylamide formate

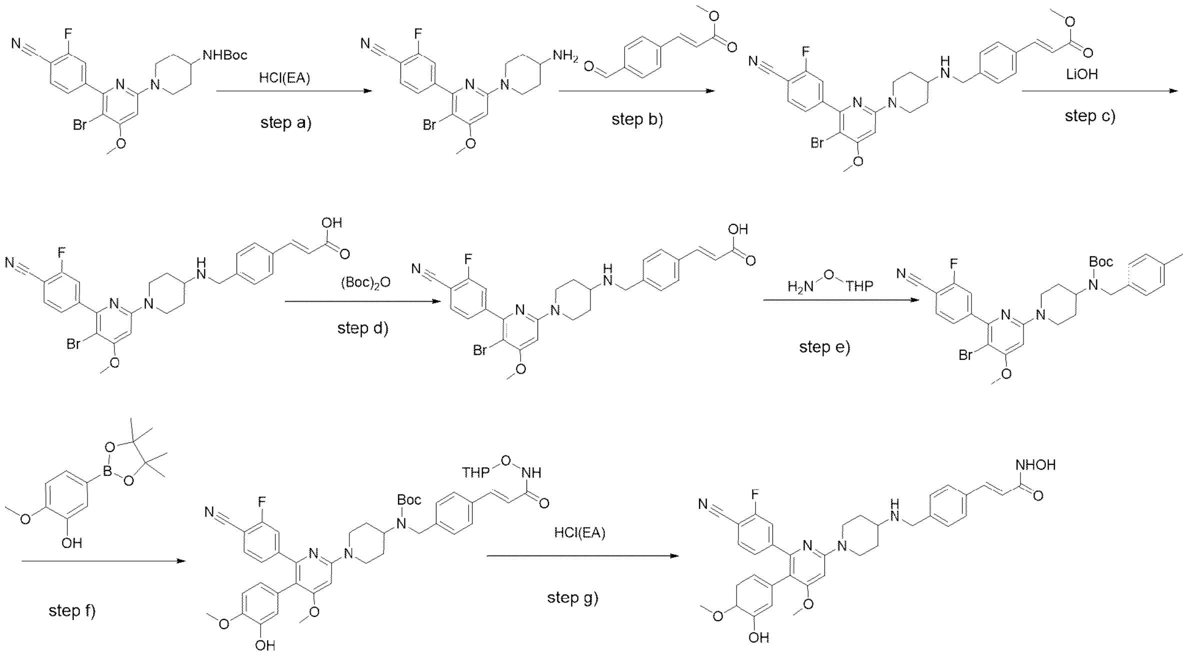


Step a): preparation of 4-(6-(4-aminopiperidin-1-yl)-3-bromo-4-methoxypyridin-2-yl)-2-fluorobenzonitrile

The product of Step d) in Example 27: tert-butyl (1-(5-bromo-6-(4-cyano-3-fluorophenyl)-4-methoxypyridin-2-yl) piperidin-4-yl)carbamate (600 mg, 1.20 mmol) was added to HCl(EA) (4 mL) and reacted at room temperature for 1 hour. After concentrating by removing the solvent, the crude product 4-(6-(4-aminopiperidin-1-yl)-3-bromo-4-methoxypyridin-2-yl)-2-fluorobenzonitrile was obtained with a yield of 41.81%.

ESI-MS m/z: 405.1 [M+H] $^+$.

Step b): preparation of methyl (E)-3-(4-{(1-(5-bromo-6-(4-cyano-3-fluorophenyl)-4-methoxypyridin-2-yl)piperidin-4-yl) amino]methyl}phenyl)pro p-2-enoate

4-(6-(4-Aminopiperidin-1-yl)-3-bromo-4-methoxypyridin-2-yl)-2-fluorobenzonitrile (420 mg, 1.04 mmol) and methyl 4-formylcinnamate (356.05 mg, 1.87 mmol) were added to a mixture of DCE/MeOH/$CH_3COOH$ = 20:1:0.1 (8 mL), and the mixture was stirred at room temperature for 0.5 hours, then cooled in an ice bath, and sodium cyanoborohydride (326.77 mg, 5.2 mmol) was added. The reaction was slowly warmed back to room temperature and reacted for 1 hour, and monitored by LCMS until the starting materials were completely consumed, and TLC(EA:PE = 1:2). The reaction system was poured into water and extracted with DCM. The organic phases were combined, and concentrating was conducted to remove the solvent. The residue was mixed with silica gel and purified by a normal-phase column to afford methyl (E)-3-(4-{(1-(5-bromo-6-(4-cyano-3-fluorophenyl)-4-methoxypyridin-2-yl)piperidin-4-yl)amino] methyl}phenyl) prop-2-enoate with a yield of 79.65%.

ESI-MS m/z: 479.1 [M+H] $^+$.

Step c): preparation of (E)-3-(4-{(1-(5-bromo-6-(4-cyano-3-fluorophenyl)-4-methoxypyridin-2-yl)piperidin-4-yl)amino] methyl}phenyl)pro p-2-enoic acid

Methyl (E)-3-(4-{(1-(5-bromo-6-(4-cyano-3-fluorophenyl)-4-methoxypyridin-2-yl)piperidin-4-yl)amino]methyl} phenyl)pro p-2-enoate (500 mg, 0.86 mmol) and lithium hydroxide (123.58 mg, 5.16 mmol) were added to a mixture of THF/$H_2O$ = 4:1(10 mL), and the mixture was reacted at room temperature overnight, and monitored by LCMS until the starting materials were completely consumed. The reaction liquid was acidified to pH = 3 with dilute hydrochloric acid, and then extracted with EA. The organic phases were combined, and concentrating was conducted to remove the solvent to afford the crude product(E)-3-(4-{(1-(5-bromo-6-(4-cyano-3-fluorophenyl)-4-methoxypyridin-2-yl)piperidin-4-yl)amino] methyl}phenyl)prop-2-enoi c acid, which was directly used in the next step.

ESI-MS m/z: 565.1 [M+H] $^+$.

Step d): preparation of E)-3-(4-{(1-(5-bromo-6-(4-cyano-3-fluorophenyl)-4-methoxypyridin-2-yl)piperidin-4-yl)[(tert-butoxy)carbonyl]ami no]methyl}phenyl)prop-2-enoic acid

(E)-3-(4-{(1-(5-Bromo-6-(4-cyano-3-fluorophenyl)-4-methoxypyridin-2-yl)piperidin-4-yl)amino]methyl}phen yl) prop-2-enoic acid (300 mg, 0.53 mmol), di-tert-butyl dicarbonate (347.02 mg, 1.59 mmol), and sodium bicarbonate (222.63 mg, 2.65 mmol) were added to a mixture of THF/$H_2O$ = 4:1 (4 mL), and the reaction was conducted at room temperature overnight and monitored by LCMS until the starting materials were completely consumed. Dilute hydrochloric acid was added to adjust the pH to 7, followed by extraction with EA, and the organic phases were combined, and concentrating was conducted to remove the solvent. The residue was mixed with silica gel and purified by a normal-phase column to afford (E)-3-(4-{(1-(5-bromo-6-(4-cyano-3-fluorophenyl)-4-methoxypyridin-2-yl)piperidin-4-yl)[(tert-butoxycarbonyl)ami no]methyl}phenyl) prop-2-enoic acid with a yield of 90.72%.
ESI-MS m/z: 664.2 $[M+H]^+$.

Step e): preparation of tert-butyl (E)-(1-(5-bromo-6-(4-cyano-3-fluorophenyl)-4-methoxypyridin-2-yl)piperidin-4-y1) (4-(3-oxo-3-((tetrahydro-2H-pyr an-2-yl)oxy)amino)prop-1-en-1-yl)benzyl)carbamate

(E)-3-(4-{(1-(5-bromo-6-(4-cyano-3-fluorophenyl)-4-methoxypyridin-2-yl)piperidin-4-yl)[(tert-butoxy)carbon yl) amino]methyl}phenyl)prop-2-enoic acid (50 mg, 75 μmol), O-(tetrahydro-2H-pyran-2-yl)hydroxylamine (10.54 mg, 0.09 mmol), HATU (34.22 mg, 90 μmol), and diisopropylethylamine (29.08 mg, 0.22 mmol) were added to DMF (8 mL), and the reaction was conducted at room temperature for 0.5 h and monitored by LCMS until the starting materials were completely consumed and the product was dominant. The reaction was terminate and the system was poured into water, followed by extraction with EA. The organic phase was washed with saturated brine, and concentrating was conducted to remove the solvent. The residue was mixed with silica gel and purified by a normal-phase column to afford tert-butyl (E)-(1-(5-bromo-6-(4-cyano-3-fluorophenyl)-4-methoxypyridin-2-yl)piperidin-4-yl)(4-(3-oxo-3-((tetrahydro-2H-pyr an-2-yl)oxy) amino)prop-1-en-1-yl)benzyl)carbamate with a yield of 73.23%.
ESI-MS m/z: 764.2 $[M+H]^+$.

Step f): preparation of tert-butyl (E)-(1-(6-(4-cyano-3-fluorophenyl)-5-(3-hydroxy-4-methoxyphenyl)-4-methoxypyridin-2-yl)piperidin-4-yl)(4-(3-ox o-3-((tetrahydro-2H-pyran-2-yl)oxy)amino)prop-1-en-1-yl)benzyl)carbamate

Tert-butyl (E)-(1-(5-bromo-6-(4-cyano-3-fluorophenyl)-4-methoxypyridin-2-yl)piperidin-4-yl)(4-(3-oxo-3-((tetrahydro-2H-pyr an-2-yl)oxy)amino)prop-1-en-1-yl)benzyl)carbamate (50 mg, 65 μmol), 2-methoxy-5-(tetramethyl-1,3,2-dioxaborolan-2-yl)phenol (19.51 mg, 78 μmol), [1,1'-bis(diphenylphosphino)ferrocene]dichloropalladium(II) (4.76 mg, 6.5 μmol), and cesium carbonate (38.12 mg, 0.12 mmol) were added to a mixture of 1,4-Dioxane/$H_2O$ = 5:1(4 mL). After purging with nitrogen, the reaction liquid was heated to 100°C and stirred for 1 hour, and LCMS indicated the disappearance of the starting materials and the presence of the main peak corresponding to the product. Concentrating was conducted to remove the solvent, and the residue was mixed with silica gel and purified by a normal-phase column to afford tert-butyl (E)-(1-(6-(4-cyano-3-fluorophenyl)-5-(3-hydroxy-4-methoxyphenyl)-4-methoxypyridin-2-yl)piperidin-4-yl)(4-(3-ox o-3-((tetrahydro-2H-pyran-2-yl)oxy)amino)prop-1-en-1-yl)benzyl)carbamate with a yield of 66.65%.
ESI-MS m/z: 808.4 $[M+H]^+$.

Step g): preparation of (E)-3-(4-(((1-(6-(4-cyano-3-fluorophenyl)-5-(3-hydroxy-4-methoxyphenyl)-4-methoxypyridin-2-yl)piperidin-4-yl)a mino)methyl)phenyl)-N-hydroxyacrylamide formate

Tert-butyl (E)-(1-(6-(4-cyano-3-fluorophenyl)-5-(3-hydroxy-4-methoxyphenyl)-4-methoxypyridin-2-yl)piperidin-4-yl)(4-(3-ox o-3-((tetrahydro-2H-pyran-2-yl)oxy)amino)prop-1-en-1-yl)benzyl)carbamate (150 mg, 0.19 mmol) was added to HCl(EA) (4M, 5 mL), and the mixture was reacted at room temperature for 0.5 hours, and monitored by LCMS until the starting materials were completely consumed. Concentrating was conducted to remove the solvent, and the residue was purified by Prep-HPLC (separation method 3) to afford (E)-3-(4-(((1-(6-(4-cyano-3-fluorophenyl)-5-(3-hydroxy-4-methoxyphenyl)-4-methoxypyridin-2-yl)piperidin-4-yl)a mino)methyl)phenyl)-N-hydroxyacrylamide formate with a yield of 90%.
$^1H$ NMR (400 MHz, DMSO-$d_6$) δ 10.74 (s, 1H), 8.28 (s, 1H), 7.73 (dd, *J* = 8.0, 7.0 Hz, 1H), 7.51 (d, *J* = 7.8 Hz, 2H), 7.48 - 7.36 (m, 3H), 7.27 (dd, *J* = 11.0, 1.6 Hz, 1H), 7.19 (dd, *J* = 8.0, 1.6 Hz, 1H), 6.78 (d, *J* = 8.4 Hz, 1H), 6.54 - 6.44 (m, 2H), 6.42 (s, 1H), 6.37 (dd, *J* = 8.2, 2.0 Hz, 1H), 4.29 (m, 2H), 3.81 (s, 2H), 3.77 (s, 3H), 3.73 (s, 3H), 3.04 - 2.83 (m, 2H), 2.77 - 2.63 (m, 1H), 2.02 - 1.86 (m, 2H), 1.33 (q, *J* = 9.6 Hz, 2H).
ESI-MS m/z: 624.3 $[M+H]^+$.

**Example 56**

Preparation of N1-(1-(7-(4-cyano-3-fluorophenyl)-8-(3-hydroay-4-methylphenyl)imidazo[1,2-c]pyrimidin-5-yl)piperidin-4-yl)-N8-hydroxyoctanediamide hydrochloride

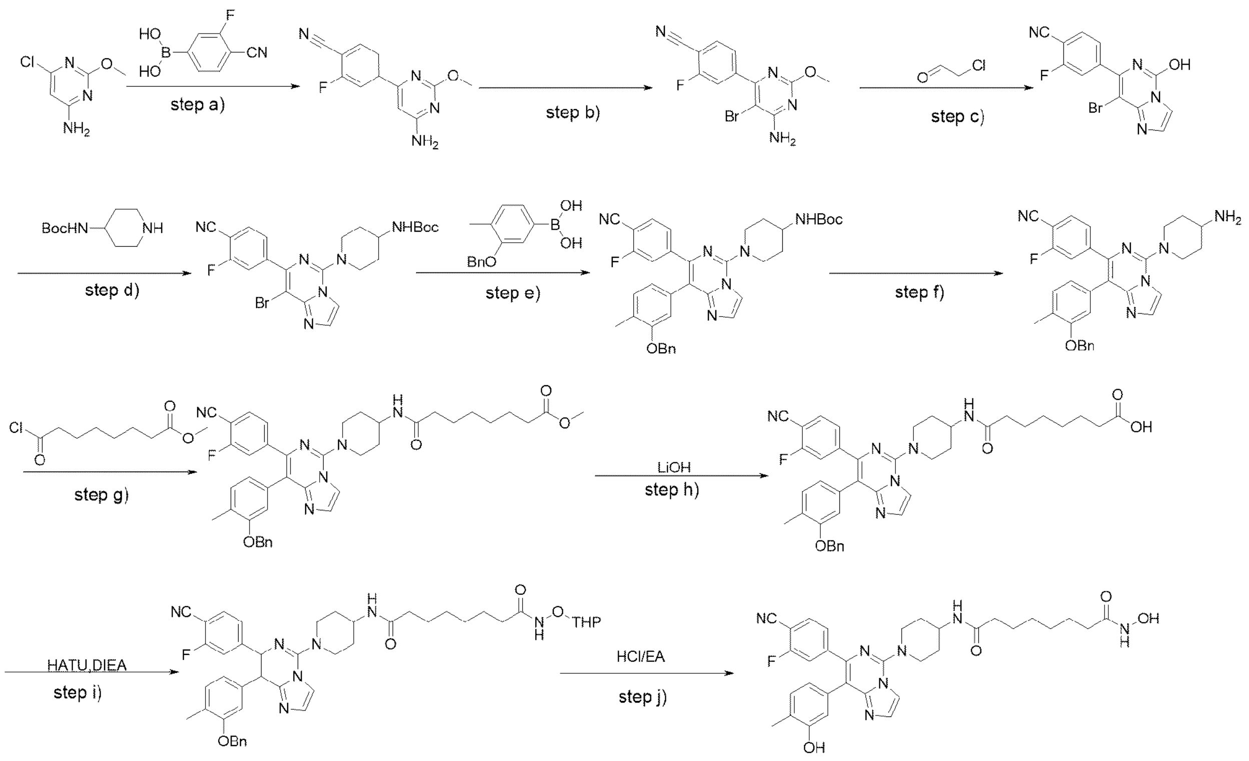


Step a): preparation of 4-(6-amino-2-methoxypyrimidin-4-yl)-2-fluorobenzonitrile

6-Chloro-2-methoxypyrimidin-4-amine (24 g, 150.40 mmol), (4-cyano-3-fluorophenyl)boronic acid (29.77 g, 180.48 mmol), di-tert-butyl-(4-dimethylaminophenyl)phosphinopalladium(II) dichloride (5.32 g, 7.52 mmol), and sodium carbonate (47.82 g, 451.20 mmol) were added to a reaction liquid containing 1,4-dioxane (350 mL) and water (70 mL), and the mixture was stirred at 95°C for 2 hours. Afterward, water (150 mL) was added to the reaction liquid, and the mixture was stirred at room temperature for 30 minutes, then filtered, and the filter cake was loaded into a 500 mL eggplant-shaped flask. Isopropanol (250 mL) was added, and the mixture was stirred at 50°C for 30 minutes, then allowed to cool to room temperature and stirred for an additional hour. After filtration, the filter cake was dried to afford 4-(6-amino-2-methoxypyrimidin-4-yl)-2-fluorobenzonitrile with a yield of 95%.

ESI-MS m/z=245.1 $[M+H]^+$.

Step b): preparation of 4-(6-amino-5-bromo-2-methoxypyrimidin-4-yl)-2-fluorobenzonitrile

4-(6-Amino-2-methoxypyrimidin-4-yl)-2-fluorobenzonitrile (24 g, 98.27 mmol) was added to a reaction liquid containing acetonitrile (240 mL) and DMSO (48 mL), and the mixture was cooled in an ice bath, and NBS (18.36 g, 103.18 mmol) was added, then the reaction liquid was stirred at room temperature for 40 minutes. The reaction liquid was filtered, and the filtrate was concentrated, and EA (300 mL) was added and stirred thoroughly before filtering again. The filter cake was washed with MTBE, and dried to afford 4-(6-amino-5-bromo-2-methoxypyrimidin-4-yl)-2-fluorobenzonitrile with a yield of 92%.

ESI-MS m/z=322.0 $[M+H]^+$.

Step c): preparation of 4-(8-bromo-5-hydroxyimidazo[1,2-c]pyrimidin-7-yl)-2-fluorobenzonitrile

4-(6-Amino-5-bromo-2-methoxypyrimidin-4-yl)-2-fluorobenzonitrile (34 g, 105.22 mmol) and 2-chloroacetaldehyde (103.25 g, 526.1 mmol) were added to a reaction liquid containing IPA (300 mL), and the mixture was stirred at 100°C

for 24 hours. The reaction liquid was transferred to room temperature and stirred for 2 hours, then filtered. The filter cake was washed with MTBE (50 mL), and the filtrate was concentrated, and then isopropanol (70 mL) was added to the concentrate and stirred at 50°C for 30 minutes, then transferred to cool to room temperature and stirred for 2 more hours before filtering. The filter cake was washed with MTBE (70 mL), and the combined cakes from both filtrations were dried to afford 4-(8-bromo-5-hydroxyimidazo[1,2-c]pyrimidin-7-yl)-2-fluorobenzonitrile with a yield of 75%.

ESI-MS m/z=333.0[M+H]$^+$.

Step d): preparation of tert-butyl (1-(8-bromo-7-(4-cyano-3-fluorophenyl)imidazo[1,2-c]pyrimidin-5-yl)piperidin-4-yl) carbamate

4-(8-Bromo-5-hydroxyimidazo[1,2-c]pyrimidin-7-yl)-2-fluorobenzonitrile (8 g, 24.02 mmol), BOP (21.25 g, 48.04 mmol), DIEA (12.42 g, 96.08 mmol), and tert-butyl piperidin-4-yl-carbamate (12.03 g, 60.05 mmol) were added to a reaction liquid containing acetonitrile (80 mL), and the mixture was stirred at 60°C for 16 hours. Silica gel was added to the reaction liquid, and the residue was purified by silica gel chromatography (eluent: DCM/MeOH = 10/1) to afford a crude product, which was further furified using a C18 column to afford tert-butyl (1-(8-bromo-7-(4-cyano-3-fluorophenyl)imidazo [1,2-c]pyrimidin-5-yl)piperidin-4-yl)carbamate with a yield of 31%.

ESI-MS m/z=515.1 [M+H]$^+$.

Step e): preparation of tert-butyl (1-(8-(3-(benzyloxy)-4-methylphenyl)-7-(4-cyano-3-fluorophenyl)imidazo[1,2-c]pyrimidin-5-yl)piperidin-4-yl)carb amate

Tert-butyl (1-(8-bromo-7-(4-cyano-3-fluorophenyl)imidazo[1,2-c]pyrimidin-5-yl)piperidin-4-yl)carbamate (400 mg, 0.78 mmol), Pd(dppf)$Cl_2$ (57 mg, 78 μmol), cesium carbonate (0.51 g, 1.56 mmol), and (3-(benzyloxy)-4-methylphenyl)boronic acid (0.23 g, 0.94 mmol) were added to a reaction liquid containing 1,4-dioxane (2 mL) and water (0.5 mL), and the mixture was stirred under microwave heating at 120°C for 1 hour. The reaction liquid was concentrated by adding silica gel, and the residue was then purified by silica gel chromatography (eluent: petroleum ether/ethyl acetate = 1/1) to afford tert-butyl (1-(8-(3-(benzyloxy)-4-methylphenyl)-7-(4-cyano-3-fluorophenyl)imidazo[1,2-c]pyrimidin-5-yl)piperidin-4-yl)carb amate with a yield of 84%.

ESI-MS m/z=633.3 [M+H]$^+$.

Step f): preparation of 4-(5-(4-aminopiperidin-1-yl)-8-(3-(benzyloxy)-4-methylphenyl)imidazo[1,2-c]pyrimidin-7-yl)-2-fluorobenzonitrile

4.0M Hydrochloric acid solution in EA (25 mL) was added to a reaction flask containing tert-butyl (1-(8-(3-(benzyloxy)-4-methylphenyl)-7-(4-cyano-3-fluorophenyl)imidazo[1,2-c]pyrimidin-5-yl)piperidin-4-yl)carb amate (400 mg, 0.63 mmol), and the mixture was stirred at room temperature for 1 hour. The reaction liquid was concentrated to afford 4-(5-(4-aminopiperidin-1-yl)-8-(3-(benzyloxy)-4-methylphenyl)imidazo[1,2-c]pyrimidin-7-yl)-2-fluorobenzonitrile with a yield of 93%.

ESI-MS m/z=533.2[M+H]$^+$.

Step g): preparation of methyl 8-((1-(8-(3-(benzyloxy)-4-methylphenyl)-7-(4-cyano-3-fluorophenyl)imidazo[1,2-c]pyrimidin-5-yl)piperidin-4-yl)a mino)-8-oxooctanoate

4-(5-(4-Aminopiperidin-1-yl)-8-(3-(benzyloxy)-4-methylphenyl)imidazo[1,2-c]pyrimidin-7-yl)-2-fluorobenzon itrile (420 mg, 0.79 mmol) was added to a reaction flask containing DCM (20 mL), and the mixture was cooled to 0°C, and then TEA (0.32 g, 3.16 mmol) and methyl 8-chloro-8-oxooctanoate (0.24 g, 1.19 mmol) were added sequentially. After stirring for 10 minutes, the mixture was cooled to room temperature and stirred at room temperature for an hour. The reaction liquid was quenched by adding water (20 mL), then extracted with ethyl acetate (20 mL × 2), and the organic phases were combined, washed with saturated brine (15 mL × 2), dried over anhydrous sodium sulfate, filtered, and concentrated to afford methyl 8-((1-(8-(3-(benzyloxy)-4-methylphenyl)-7-(4-cyano-3-fluorophenyl)imidazo[1,2-c]pyrimidin-5-yl)piperidin-4-yl)a mino)-8-oxooctanoate with a yield of 60%.

ESI-MS m/z=703.3 [M+H]$^+$.

Step h): preparation of 8-((1-(8-(3-(benzyloxy)-4-methylphenyl)-7-(4-cyano-3-fluorophenyl)imidazo[1,2-c]pyrimidin-5-yl)piperidin-4-yl)a mino)-8-oxooctanoic acid

Methyl 8-((1-(8-(3-(benzyloxy)-4-methylphenyl)-7-(4-cyano-3-fluorophenyl)imidazo[1,2-c]pyrimidin-5-yl)piperidin-4-yl)a mino)-8-oxooctanoate (500 mg, 0.71 mmol) and lithium hydroxide (29 mg, 0.71 mmol) were added to a reaction

liquid containing THF (10 mL), MeOH (6 mL), and water (4 mL), and the mixture was stirred at room temperature for 1 hour. The pH of the reaction liquid was adjusted to 4 with 2M HCl, and then water (20 mL) was added to quench the reaction. The mixture was extracted with a mixture of DCM/ MeOH (5:1) (20 mL × 2), and the organic phases were combined, washed with saturated brine (20 mL × 2), dried over anhydrous sodium sulfate, filtered, and concentrated to afford 8-((1-(8-(3-(benzyloxy)-4-methylphenyl)-7-(4-cyano-3-fluorophenyl)imidazo[1,2-c]pyrimidin-5-yl)piperidin-4-yl)amino)-8-oxooctanoic acid with a yield of 84%.

ESI-MS m/z=689.3 $[M+H]^+$.

Step i): preparation of $N^1$-(1-(8-(3-(benzyloxy)-4-methylphenyl)-7-(4-cyano-3-fluorophenyl)imidazo[1,2-c]pyrimidin-5-yl)piperidin-4-yl)-$N^8$-((tetrahydro-2H-pyran-2-yl)oxy)octanediamide

8-((1-(8-(3-(Benzyloxy)-4-methylphenyl)-7-(4-cyano-3-fluorophenyl)imidazo[1,2-c]pyrimidin-5-yl)piperidin-4-yl)amino)-8-oxooctanoic acid (520 mg, 0.75 mmol), O-(tetrahydro-2H-pyran-2-yl)hydroxylamine (130 mg, 1.13 mmol), HATU (430 mg, 1.13 mmol), and DIEA (480 mg, 3.75 mmol) were added to a reaction liquid containing DMF (15 mL), and the mixture was stirred at room temperature for 1 hour. The reaction liquid was quenched by adding water (20 mL), then extracted with ethyl acetate (20 mL × 2), and the organic phases were combined, washed with saturated brine (15 mL × 2), dried over anhydrous sodium sulfate, filtered, and the residue was purified by silica gel chromatography (eluent: DCM/MeOH = 8/1) to afford $N^1$-(1-(8-(3-(benzyloxy)-4-methylphenyl)-7-(4-cyano-3-fluorophenyl)imidazo[1,2-c]pyrimidin-5-yl)piperidin-4-yl)-$N^8$-((tetrahydro-2H-pyran-2-yl)oxy)octanediamide with a yield of 48%.

ESI-MS m/z=788.4 $[M+H]^+$.

Step j): preparation of $N^1$-(1-(7-(4-cyano-3-fluorophenyl)-8-(3-hydroxy-4-methylphenyl)imidazo[1,2-c]pyrimidin-5-yl)piperidin-4-yl)-$N^8$-hydroxyoctanediamide hydrochloride

4.0M Hydrochloric acid solution in EA (6 mL) was added to a reaction flask containing $N^1$-(1-(8-(3-(benzyloxy)-4-methylphenyl)-7-(4-cyano-3-fluorophenyl)imidazo[1,2-c]pyrimidin-5-yl)piperidin-4-yl)-$N^8$-((tetrahydro-2H-pyran-2-yl)oxy)octanediamide (120 mg, 0.15 mmol), and the mixture was stirred at room temperature for 1 hour. After concentration of the reaction liquid, it was purified by Prep-HPLC (separation method 1) to afford (E)-3-(4-(6-(4-amino-4-methylpiperidin-1-yl)-5-cyano-4-(4-cyano-3-fluorophenyl)pyridin-3-yl)-2-hydroxyphenyl)-N-hydroxyacrylamide hydrochloride with a yield of 22%.

$^1$H NMR (400 MHz, DMSO-d6) δppm 10.36 (s, 1H), 9.72 (s, 1H), 8.24 (d, J = 2.2 Hz, 1H), 8.14 (s, 1H), 7.98-7.85 (m, 2H), 7.53 (m, 1H), 7.42 - 7.31 (m, 1H), 7.20 (d, *J* = 7.8 Hz, 1H), 6.81 (s, 1H), 6.70 - 6.59 (m, 1H), 4.02 (d, *J* = 13.2 Hz, 2H), 3.93 (d, *J* = 8.6 Hz, 1H), 3.32 (t, *J* = 12.2 Hz, 2H), 2.19 (s, 3H), 2.08 (t, *J* = 7.4 Hz, 2H), 1.99-1.87 (m, 4H), 1.69 (q, *J* = 11.2 Hz, 2H), 1.47 (p, *J* = 7.6 Hz, 4H), 1.32 - 1.16 (m, 4H).

ESI-MS m/z=614.3 $[M+H]^+$.

**Example 57**

The preparation of $N^1$-(4-(5-(4-aminopiperidin-1-yl)-7-(4-cyano-3-fluorophenyl)imidazo[1,2-c]pyrimidin-8-yl)-2-hydroxyphenyl)-$N^8$-h ydroxyoctanediamine diformate was prepared according to the synthetic method of Example 56 (separation method 1), and the structure and characterization data are as follows:

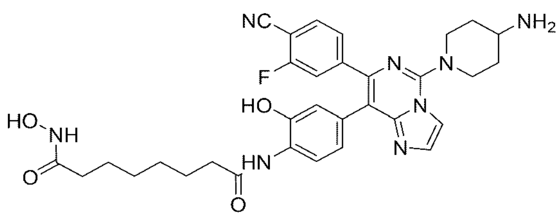

$^1$H NMR (400 MHz, Methanol-$d_4$) δ ppm 8.45 (s, 2H), 7.73 (d, *J* = 1.6 Hz, 1H), 7.67 (d, *J* = 8.2 Hz, 1H), 7.56-7.43 (m, 2H), 7.40 (dd, *J* = 10.8, 1.6 Hz, 1H), 7.24 (dd, *J* = 8.2, 1.6 Hz, 1H), 6.78 (d, *J* = 1.2 Hz, 1H), 6.66 (dd, *J* = 8.2, 1.8 Hz, 1H), 4.46 (d, *J* = *21.8* Hz, 1H), 4.02 (d, *J* = 13.2 Hz, 2H), 3.19 - 3.02 (m, 2H), 2.37 (t, *J* = 7.4 Hz, 2H), 2.15 - 1.93 (m, 4H), 1.91 - 1.75 (m, 2H), 1.59 (m, 4H), 1.32 (dq, *J* = 8.8, 4.8 Hz, 4H).

ESI-MS m/z=615.3 $[M+H]^+$

**Example 58**

Preparation of $N^1$-(4-(6-(4-aminopiperidin-1-yl)-5-cyano-4-(4-cyano-3-fluorophenyl)pyridin-3-yl)-2-hydroxyphenyl)-$N^8$-hydroxyo ctanediamide hydrochloride

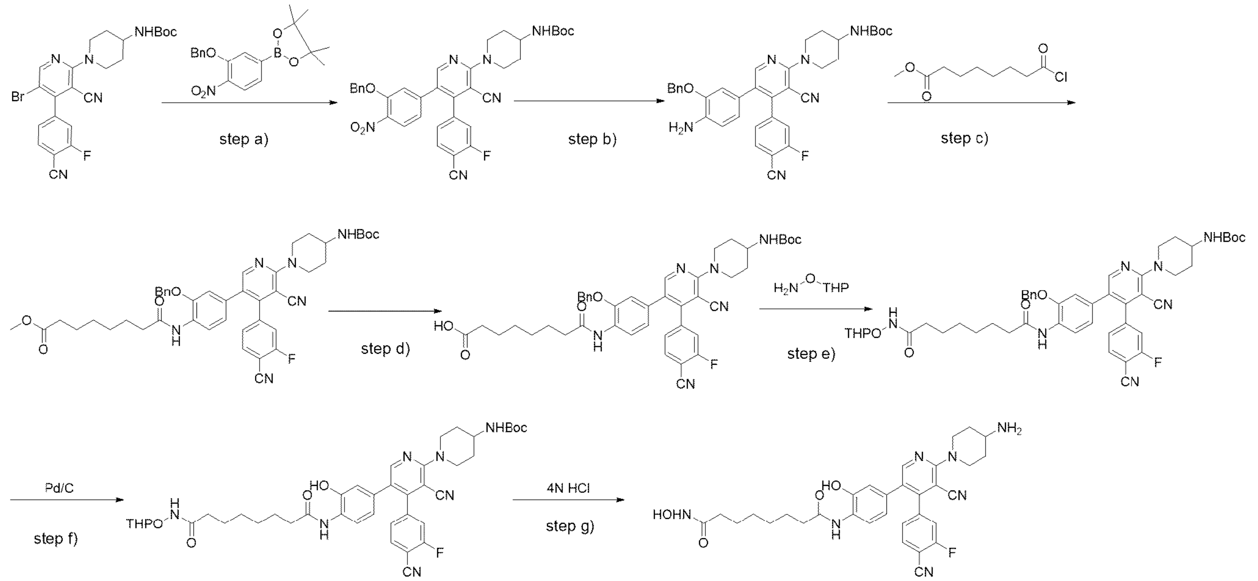


Step a): preparation of tert-butyl (1-(5-(3-(benzyloxy)-4-nitrophenyl)-3-cyano-4-(4-cyano-3-fluorophenyl)pyridin-2-yl)piperidin-4-yl)carbamate

The product of Step c) in Example 37: tert-butyl (1-(5-bromo-3-cyano-4-(4-cyano-3-fluorophenyl)pyridin-2-yl) piperidin-4-yl)carbamate (200 mg, 401 μmol), 2-(3-(Benzyloxy)-4-nitrophenyl)-4,4,5,5-tetramethyl-1,3,2-dioxobenzaldehyde (213 mg, 601 μmol), $Cs_2CO_3$ (261 mg, 802 μmol), $Pd(dppf)Cl_2$ (29 mg, 40 μmol), 1,4-dioxane (4 mL), and $H_2O$ (1 mL) were added to a reaction flask and stirred at 120°C for 1 hour. The reaction liquid was concentrated under reduced pressure, and the residue was purified by silica gel chromatography (eluent: petroleum ether/ethyl acetate = 1/2) to afford tert-butyl (1-(5-(3-(benzyloxy)-4-nitrophenyl)-3-cyano-4-(4-cyano-3-fluorophenyl)pyridin-2-yl)piperidin-4-yl)carbamate with a yield of 78.5%.

ESI-MS(m/z) =529.3 $[M+H]^+$.

Step b): preparation of tert-butyl (1-(5-(4-amino-3-(benzyloxy)phenyl)-3-cyano-4-(4-cyano-3-fluorophenyl)pyridin-2-yl) piperidin-4-yl)carbamate

Tert-butyl (1-(5-(3-(benzyloxy)-4-nitrophenyl)-3-cyano-4-(4-cyano-3-fluorophenyl)pyridin-2-yl)piperidin-4-yl) carbamate (167 mg, 315 μmol), zinc powder (202 mg, 3.1 mmol), ammonium chloride (164 mg, 3.1 mmol), and THF (5 mL) were added to a reaction flask, and the mixture was heated to 65°C and reacted for 12 hours. After the reaction was completed, the reaction liquid was cooled to room temperature, then filtered, and the filtrate was concentrated under reduced pressure. The residue was purified by silica gel chromatography (eluent: petroleum ether/ethyl acetate = 1/2) to afford tert-butyl (1-(5-(4-amino-3-(benzyloxy)phenyl)-3-cyano-4-(4-cyano-3-fluorophenyl)pyridin-2-yl)piperidin-4-yl)carbamate with a yield of 72.3%.

ESI-MS(m/z) =619.3$[M+H]^+$.

Step c): preparation of methyl 8-((2-(benzyloxy)-4-(6-(4-(tert-butoxycarbonyl)amino)piperidin-1-yl)-5-cyano-4-(4-cyano-3-fluorophenyl)pyridin-3 -yl)phenyl)amino)-8-oxooctanoate

Tert-butyl (1-(5-(4-amino-3-(benzyloxy)phenyl)-3-cyano-4-(4-cyano-3-fluorophenyl)pyridin-2-yl)piperidin-4-yl) carbamate (140 mg, 227 μmol), methyl 8-chloro-8-oxooctanoate (93 mg, 454 μmol), triethylamine (46 mg, 454 μmol), and DCM (5 mL) were added to a reaction flask and stirred at room temperature for 2 hours. The mixture was concentrated under reduced pressure, and the residue was purified by silica gel chromatography (eluent: petroleum ether/ethyl acetate

= 1/1) to afford methyl 8-((2-(benzyloxy)-4-(6-(4-(tert-butoxycarbonyl)amino)piperidin-1-yl)-5-cyano-4-(4-cyano-3-fluorophenyl)pyridin-3 -yl)phenyl)amino)-8-oxooctanoate with a yield of 58.0%.

ESI-MS(m/z) =789.4 $[M+H]^+$.

Step d): preparation of 8-((2-(benzyloxy)-4-(6-(4-(tert-butoxycarbonyl)amino)piperidin-1-yl)-5-cyano-4-(4-cyano-3-fluorophenyl)pyridin-3 -yl)phenyl)amino)-8-oxooctanoic acid

Methyl 8-((2-(benzyloxy)-4-(6-(4-(tert-butoxycarbonyl)amino)piperidin-1-yl)-5-cyano-4-(4-cyano-3-fluorophenyl)pyridin-3 -yl)phenyl)amino)-8-oxooctanoate (104 mg, 132 μmol), lithium hydroxide monohydrate (28 mg, 660 μmol), tetrahydrofuran (2 mL), isopropanol (2 mL), and water (1 mL) were added to a reaction flask and stirred at room temperature for 12 hours. Under ice bath cooling, 1N concentrated hydrochloric acid was slowly added dropwise to adjust the pH to 3~4, and then water (10 mL) was added, and the mixture was extracted with ethyl acetate (10 mL × 3). The organic phases were combined, washed with saturated brine (10 mL × 2), and concentrated to dryness under reduced pressure to afford 8-((2-(benzyloxy)-4-(6-(4-(tert-butoxycarbonyl)amino)piperidin-1-yl)-5-cyano-4-(4-cyano-3-fluorophenyl)pyridin-3 -yl)phenyl)amino)-8-oxooctanoic acid, which was used directly for the next reaction step.

ESI-MS(m/z) =775.4$[M+H]^+$.

Step e): preparation of tert-butyl (1-(5-(3-(benzyloxy)-4-(8-oxo-8-((tetrahydro-2H-pyran-2-yl)oxy)amino)octanamino)phenyl)-3-cyano-4-(4-cyano-3-fluorophenyl)pyridin-2-yl)piperidin-4-yl)carbamate 8-((2-(Benzyloxy)-4-(6-(4-(tert-butoxycarbonyl)amino)piperidin-1-yl)-5-cyano-4-(4-cyano-3-fluorophenyl)pyridin-3-yl)phenyl)amino)-8-oxooctanoic acid (102 mg, 132 μmol), O-(tetrahydro-2H-pyran-2-yl)hydroxylamine (31 mg, 264 μmol), DIEA (34 mg, 264 μmol), and DMF (2 mL) were added to a reaction flask. HATU (62 mg, 158 μmol) was added under stirring at room temperature, and the reaction was maintained for 1 hour at room temperature. After the reaction was completed, water (10 mL) was added to quench the reaction, followed by extraction with ethyl acetate (20 mL × 2), and the organic phases were combined, washed sequentially with saturated sodium bicarbonate aqueous solution (20 mL × 1) and saturated brine (10 mL × 2), dried over anhydrous sodium sulfate, filtered, and the filtrate was concentrated under reduced pressure. The residue was purified by silica gel chromatography (eluent: petroleum ether/ethyl acetate = 1/1) to afford tert-butyl (1-(5-(3-(benzyloxy)-4-(8-oxo-8-((tetrahydro-2H-pyran-2-yl)oxy)amino)octanamino)phenyl)-3-cyano-4-(4-cyano-3-fluorophenyl)pyridin-2-yl)piperidin-4-yl)carbamate with a yield of 60.5%.

ESI-MS(m/z) =874.4 $[M+H]^+$.

Step f): preparation of tert-butyl (1-(3-cyano-4-(4-cyano-3-fluorophenyl)-5-(3-hydroxy-4-(8-oxo-8-((tetrahydro-2H-pyran-2-yl)oxy)amino)octanami no)phenyl)pyridin-2-yl)piperidin-4-yl)carbamate

Tert-butyl (1-(5-(3-(benzyloxy)-4-(8-oxo-8-((tetrahydro-2H-pyran-2-yl)oxy)amino)octanamino)phenyl)-3-cyano-4-(4-cyano-3-fluorophenyl)pyridin-2-yl)piperidin-4-yl)carbamate (70 mg, 80 μmol), palladium on carbon (15 mg, 5%), and ethanol (5 mL) were sequentially added to a reaction flask, and the mixture was stirred until dissolved, and the mixture was purged with hydrogen three times, and stirred at room temperature for 2 hours under hydrogen atmosphere. After the reaction was completed, it was filtered, and the filtrate was concentrated under reduced pressure to afford tert-butyl (1-(3-cyano-4-(4-cyano-3-fluorophenyl)-5-(3-hydroxy-4-(8-oxo-8-((tetrahydro-2H-pyran-2-yl)oxy)amino)octanami no)phenyl)pyridin-2-yl)piperidin-4-yl)carbamate with a yield of 85.0%.

ESI-MS(m/z) =784.4$[M+H]^+$.

Step g): preparation of $N^1$-(4-(6-(4-aminopiperidin-1-yl)-5-cyano-4-(4-cyano-3-fluorophenyl)pyridin-3-yl)-2-hydroxyphenyl)-$N^8$-hydroxyo ctanediamide hydrochloride

Tert-butyl (1-(3-cyano-4-(4-cyano-3-fluorophenyl)-5-(3-hydroxy-4-(8-oxo-8-((tetrahydro-2H-pyran-2-yl)oxy) amino)octanami no)phenyl)pyridin-2-yl)piperidin-4-yl)carbamate (53 mg, 68 μmol) was added to a reaction flask, followed by adding hydrogen chloride solution in ethyl acetate (4M, 2.5 mL), and the mixture was stirred at room temperature for 1 hour, resulting in the precipitation of a solid. After concentrating under reduced pressure, the crude product was purified by Prep-HPLC (separation method 1) to afford $N^1$-(4-(6-(4-aminopiperidin-1-yl)-5-cyano-4-(4-cyano-3-fluorophenyl)pyridin-3-yl)-2-hydroxyphenyl)-$N^8$-hydroxyo ctanediamide hydrochloride with a yield of 21.5%.

$^1H$ NMR (400 MHz, Methanol-$d_4$) δ 8.47 (s, 1H), 7.77 (dd, $J$ = 7.8, 6.6 Hz, 1H), 7.60 (d, $J$ = 8.2 Hz, 1H), 7.41 (dd, $J$ = 9.6, 1.4 Hz, 1H), 7.25 (dd, $J$ = 8.0, 1.4 Hz, 1H), 6.65 - 6.43 (m, 2H), 4.44 (d, $J$ = 13.6 Hz, 2H), 3.45 (ddt, $J$ = 11.4, 8.6, 4.4 Hz, 1H), 3.23 (s, 2H), 2.44 (t, $J$ = 7.4 Hz, 2H), 2.26 - 2.05 (m, 4H), 1.94 - 1.55 (m, 6H), 1.41 (dd, $J$ = 7.6, 4.0 Hz, 4H).

ESI-MS(m/z) =500.3 $[M+H]^+$.

**Examples 59-60** were prepared according to the synthetic method of Example **58** (the separation method for the compounds: hydrochloride and formate were prepared according to separation method 1 and 3, respectively), and the structures and characterization data are as follows:

| Example | Chemical name | Structure | 1H NMR | MS(M+H)+ |
|---|---|---|---|---|
| 59 | (E)-3-(3-(N-(1-(3-Cyano-4 -(4-cyano-3-fluorophenyl)-5-(3-hydroxy-4-methoxyp henyl) pyridin-2-yl)piperidin-4-yl)sulfamoyl)phenyl)-N-hydroxyacrylamide hydrochloride | 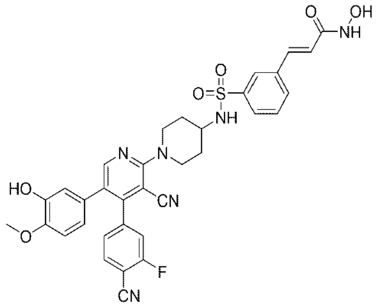 | 1H NMR (400 MHz, Methanol-$d_4$) δ 8.36 (s, 1H), 8.07 (s, 1H), 7.91 (dd, *J* = 7.8, 1.6 Hz, 1H), 7.88 - 7.77 (m, 1H), 7.78 - 7.57 (m, 3H), 7.34 (dd, *J* = 9.8, 1.6 Hz, 1H), 7.20 (dd, *J* = 8.0, 1.6 Hz, 1H), 6.88 - 6.75 (m, 1H), 6.59 (d, *J* = 15.8 Hz, 1H), 6.54 - 6.40 (m, 2H), 4.14 (d, *J* = 13.4 Hz, 2H), 3.80 (s, 3H), 3.40 (td, *J*= 10.2, 5.2 Hz, 1H), 3.25 - 3.07 (m, 2H), 1.91 - 1.80 (m, 2H), 1.66 (d, *J* = 7.0 Hz, 2H). | 669.2 |
| 60 | (E)-N-(1-(3-Cyano-4-(4-cy ano-3-fluorophenyl)-5-(3-h ydroxy-4-methoxyphenyl) pyridin-2-yl)piperidin-4-yl )-4-(3-(hydroxyamino)-3-o xoprop-1-en-1-yl)benzami de formate | 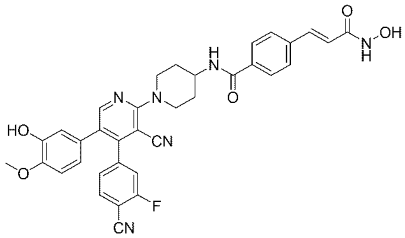 | 1H NMR (400 MHz, DMSO-$d_6$) δ 10.75 (s, 1H), 9.03 (s, 1H), 8.45 (s, 1H), 8.43 (d, *J* = 8.0 Hz, 1H), 7.97 (dd, *J* = 8.0, 6.9 Hz, 1H), 7.90 (d, *J*= 8.2 Hz, 2H), 7.74 - 7.60 (m, 3H), 7.48 (d, *J* = 15.8 Hz, 1H), 7.29 (dd, *J* = 8.0, 1.6 Hz, 1H), 6.82 (d, *J* = 8.0 Hz, 1H), 6.55 (d, *J* = 15.8 Hz, 1H), 6.47 (s, 2H), 4.27 (d, *J* = 12.8 Hz, 2H), 4.21 - 4.05 (m, 1H), 3.72 (s, 3H), 3.22 (t, *J* = 12.0 Hz, 2H), 2.05 - 1.91 (m, 2H), 1.85-1.67 (m, 2H). | 633.2 |

**Example 61**

Preparation of 4-(6-(4-aminopiperidin-1-yl)-5-cyano-4-(4-cyano-3-fluorophenyl)pyridin-3-yl)-N-hydroxybenzamide hydrochloride

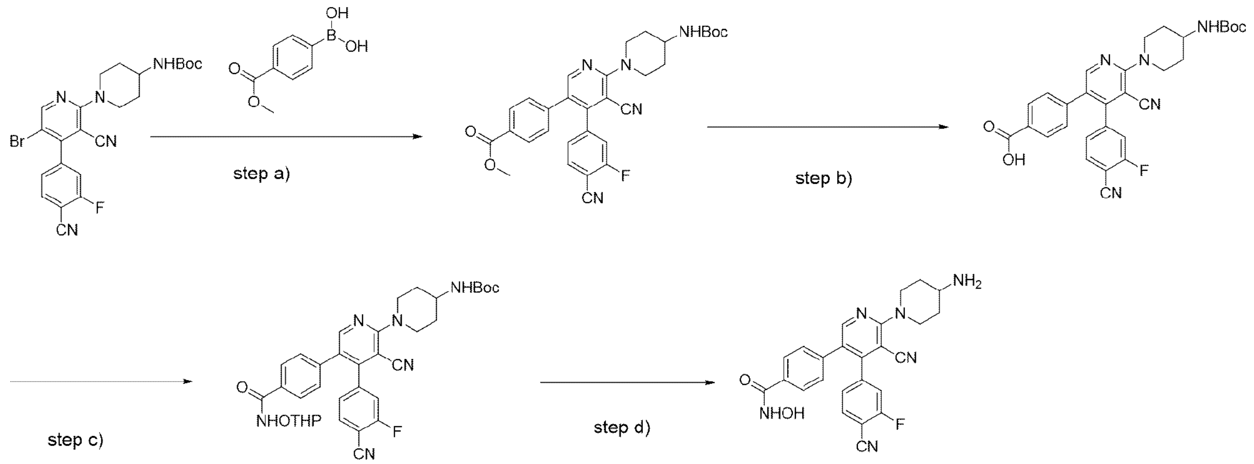


Step a): preparation of ethyl 4-(6-(4-{(tert-butoxy)carbonyl]amino}piperidin-1-yl)-5-cyano-4-(4-cyano-3-fluorophenyl) pyridin-3-yl)benzoate

The product of Step c) in Example 37: tert-butyl (1-(5-bromo-3-cyano-4-(4-cyano-3-fluorophenyl)pyridin-2-yl) piperidin-4-yl)carbamate (100 mg, 0.20 mmol), (4-(methoxycarbonyl)phenyl)boronic acid (53.99 mg, 0.30 mmol), $Cs_2CO_3$ (130.33 mg, 0.40 mmol), and Pd(dppf)$Cl_2$ (14.63 mg, 0.02 mmol) were dissolved in a mixture of water (1 mL) and 1,4-dioxane (4 mL), the reaction liquid was stirred under microwave heating at 120°C for 1 hour under nitrogen protection. After monitoring and confirming the completion of the reaction, the mixture was concentrated to obtain a crude product, and the residue was then purified by silica gel chromatography (eluent: petroleum ether/ethyl acetate = 10/7) to afford ethyl 4-(6-(4 {(tert-butoxy)carbonyl]amino}piperidin-1-yl)-5-cyano-4-(4-cyano-3-fluorophenyl)pyridin-3-yl)benzoate with a yield of 94%.

ESI-MS m/z=556.2 $[M+H]^+$

Step b): preparation of 4-(6-(4-{(tert-butoxy)carbonyl]amino}piperidin-1-yl)-5-cyano-4-(4-cyano-3-fluorophenyl)pyridin-3-yl)benzoic acid

Ethyl 4-(6-(4-{(tert-butoxy)carbonyl]amino}piperidin-1-yl)-5-cyano-4-(4-cyano-3-fluorophenyl)pyridin-3-yl) benzoate (105 mg, 0.19 mmol) and lithium hydroxide monohydrate (80 mg, 1.9 mmol) were dissolved in a mixture of tetrahydrofuran (2.5 mL) and water (1 mL) and reacted for 16 hours. Upon monitoring and confirming the completion of the reaction, water (20 mL) was added, and the pH was adjusted to 3-4 with 1N HCl aqueous solution. The mixture was then extracted with ethyl acetate (20 mL × 2), and the organic phases were combined, washed with saturated brine (15 mL × 2), dried over anhydrous sodium sulfate, filtered, and concentrated to obtain a crude product, which was directly used in the next step.

ESI-MS m/z=542.2 $[M+H]^+$

Step c): preparation of tert-Butyl N-(1-(3-cyano-4-(4-cyano-3-fluorophenyl)-5-(4-[(oxepan-2-yloxy)carbamoyl]phenyl) pyridin-2-yl)piperidin-4-yl)car bamate

4-(6-(4{(Tert-butoxy)carbonyl]amino)piperidin-1-yl)-5-cyano-4-(4-cyano-3-fluorophenyl)pyridin-3-yl)benzoic acid (100 mg, 0.18 mmol) and O-(tetrahydro-2H-pyran-2-yl)hydroxylamine (64 mg, 0.54 mmol) were dissolved in N,N-dimethylformamide (5 mL). DIPEA (120 mg, 0.93 mmol) and HATU (210 mg, 0.55 mmol) were added, and the reaction was conducted for 30 minutes. Upon monitoring and confirming the completion of the reaction, water (20 mL) was added to quench the reaction, followed by extraction with ethyl acetate (20 mL × 2), and the organic phases were combined, washed with saturated brine (15 mL × 2), dried over anhydrous sodium sulfate, filtered, and concentrated to obtain a crude product, which was directly used in the next step.

ESI-MS m/z=641.3 $[M+H]^+$

Step d): preparation of 4-(6-(4-aminopiperidin-1-yl)-5-cyano-4-(4-cyano-3-fluorophenyl)pyridin-3-yl)-N-hydroxybenzamide

Tert-butyl N-(1-(3-cyano-4-(4-cyano-3-fluorophenyl)-5-(4-[(oxepan-2-yloxy)carbamoyl]phenyl)pyridin-2-yl)piperidin-4-yl)car bamate (110 mg, 0.17 mmol) was dissolved in 4N HCl (6 mL) and reacted for 30 minutes. After monitoring

and confirming the completion of the reaction, the mixture was concentrated to obtain a crude product, which was prepared by method 1 into 4-(6-(4-aminopiperidin-1-yl)-5-cyano-4-(4-cyano-3-fluorophenyl)pyridin-3-yl)-N-hydroxybenzamide hydrochloride with a yield of 62%.

$^1$H NMR (400 MHz, Methanol-$d_4$) δ ppm 8.49 (s, 1H), 7.74 (dd, *J* = 7.8, 6.6 Hz, 1H), 7.66 (d, *J* = 7.8 Hz, 2H), 7.47 - 7.34 (m, 1H), 7.24 (m, 3H), 4.49 (d, *J* = 13.2 Hz, 2H), 3.48 (tt, *J* = 11.0, 4.0 Hz, 1H), 3.25 (d, *J* = 11.2 Hz, 1H), 3.00 (s, 1H), 2.31 = 2.11 (m, 2H), 1.83 (qd, *J* = 12.2, 3.8 Hz, 2H).ESI-MS m/z=457.2 [M+H]$^+$

**Examples 62-64** were prepared according to the synthetic method of Example **61** (the compound separation method for the compound: hydrochloride and formate were prepared by separation methods 1 and 3, respectively), and the structure and characterization data are as follows:

| Example | Chemical name | Structure | $^1$H NMR | MS(M+H)$^+$ |
|---|---|---|---|---|
| 62 | (E)-3-(4-(6-(4-Aminopiperidin-1-yl)-5-cyano-4-(4-cyano-3-fluorophenyl) pyri din-3-yl)-2-hydrox yphenyl)-N-hydrox yacrylamide hydrochloride | 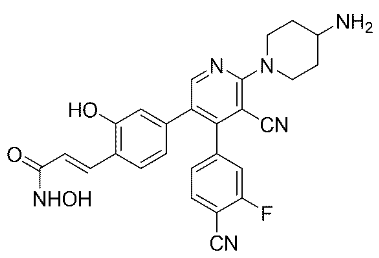 | $^1$H NMR (400 MHz, Methanol-$d_4$) δ 8.47 (s, 1H), 7.86 - 7.69 (m, 2H), 7.44 (dd, *J* = 9.8, 1.6 Hz, 1H), 7.36 (d, *J* = 8.4 Hz, 1H), 7.24 (dd, *J* = 8.0, 1.6 Hz, 1H), 6.68 (m, 1H), 6.62 - 6.54 (m, 2H), 4.46 (dp, *J* = 14.0, 2.2 Hz, 2H), 3.45 (ddt, *J* = 11.4, 8.6, 4.2 Hz, 1H), 3.24 (m, 2H), 2.26 - 2.04 (m, 2H), 1.82 (qd, *J* = 12.4, 4.2 Hz, 2H). | 499.2 |
| 63 | (E)-3-(4-(6-(4-Aminopiperidin-1-yl)-5-cyano-4-(4-cyano-3-fluorophenyl) pyri din-3-yl)phenyl)-N -hydroxyacrylamid e diformate | 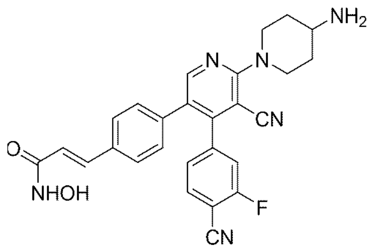 | $^1$H NMR (400 MHz, Methanol-$d_4$) δ 8.56 (s, 2H), 8.47 (s, 1H), 7.70 (t, *J* = 8.0 Hz, 1H), 7.49 - 7.34 (m, 4H),7.27 (d, J = 8, 1H) 7.19 (d, *J* = 8.0 Hz, 2H),, 6.48 (d, *J* = 15.8 Hz, 1H), 4.41 (d, *J* = 13.6 Hz, 2H), 3.30 (s, 1H), 3.22 - 3.05 (m, 2H), 2.09 (d, *J* = 12.3 Hz, 2H), 1.71 (qd, *J* = 12.3, 4.1 Hz, 2H). | 483.2 |
| 64 | (E)-3-(4-(6-(4-Amino-4-methylpiperidin-1-yl)-5-cyano-4-(4-cyano-3-fluorop henyl)pyridin-3-yl) -2-hydroxyphenyl)-N-hydroxyacrylami de formate | 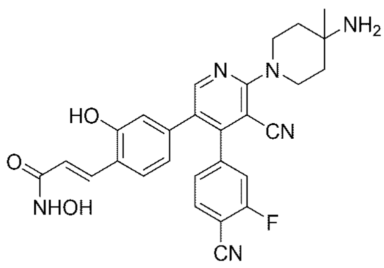 | $^1$H NMR (400 MHz, DMSO-$d_6$) δ ppm: 10.68 (s, 1H), 10.16 (s, 1H), 8.94 (s, 1H), 8.50 (s, 1H), 7.97 (dd, *J* = 8.0, 6.8 Hz, 1H), 7.75 (dd, *J* = 10.0, 1.6 Hz, 1H), 7.54 (d, *J* = 15.8 Hz, 1H), 7.31 (d, *J* = 8.2 Hz, 1H), 7.27 (dd, *J* = 8.0, 1.6 Hz, 1H), 6.62 - 6.54 (m, 2H), 6.50 (d, *J* = 15.8 Hz, 1H), 4.03 (d, *J* = 14.0 Hz, 2H), 3.54 (q, *J* = 10.4, 10.0 Hz, 2H), 1.87 (d, *J* = 9.2 Hz, 2H), 1.80 (d, *J* = 13.8 Hz, 2H), 1.39 (s, 3H). | 513.2 |

**Example 66**

Preparation of (E)-3-(4-(((1-(4-(4-cyano-3-fluorophenyl)-5-(3-fluoro-4-methoxyphenyl)-1-methyl-6-oxo-1,6-dihydropyrimidin-2-y l)piperidin-4-yl)amino)methyl)phenyl)-N-hydroxyacrylamide formate

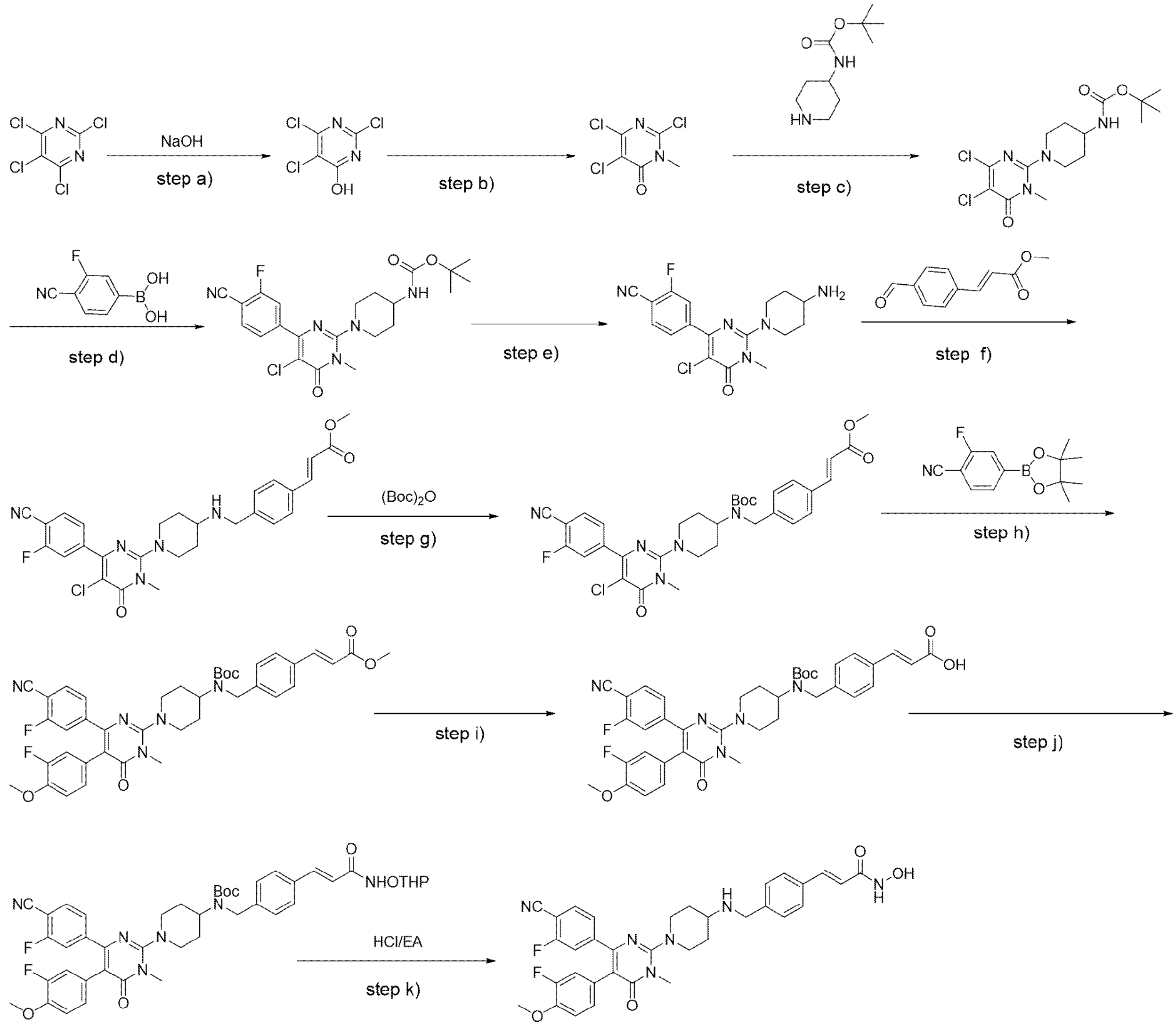


Step a) : synthesis of 2,5,6-trichloropyrimidin-4-ol

Sodium hydroxide (1.19g, 29.84 mmol) was weighed and dissolved in water (15 mL), and then slowly added dropwise to a solution of 2,4,5,6-tetrachloropyrimidine (5 g, 22.95 mmol) in tetrahydrofuran (40 mL), and the mixture was stirred for reaction for 16 hours under nitrogen protection. After the reaction was completed as indicated by LCMS, dilute hydrochloric acid was added to acidify the mixture, followed by extraction with ethyl acetate (20 mL × 3 times), and the organic phases were combined, washed with saturated brine (15 mL), dried with anhydrous sodium sulfate, filtered, and then concentrated to dryness under reduced pressure. The residue was purified by being slurred with diethyl ether, affording 2,5,6-trichloro-4-pyrimidinol with a yield of 74.1%.
ESI-MS m/z: 199.0[M+H]$^+$;

Step b) : synthesis of 2,5,6-trichloro-3-methylpyrimidin-4(3H)-one

2,5,6-Trichloropyrimidin-4-ol (3.3 g, 16.6 mmol) and potassium carbonate (3.43 g, 79.6 mmol) were dissolved in DMF (30 mL), to which iodomethane (4.7 g, 33.1 mmol) was slowly added. After the addition was completed, the reaction was conducted at room temperature for 6 hours. Water (200 mL) was then added, followed by extraction with ethyl acetate (50 mL × 3). The organic layer was washed with saturated brine (100 mL), dried with anhydrous sodium sulfate, filtered, and then concentrated under vacuum. The residue was purified by silica gel chromatography to afford 2,5,6-trichloro-3-methylpyrimidin-4(3H)-one with a yield of 82.5%.
ESI-MS m/z = 213.0 [M+H]$^+$;

Step c): synthesis of tert-butyl (1-(4,5-dichloro-1-methyl-6-oxo-1,6-dihydropyrimidin-2-yl)piperidin-4-yl)carbamate

2,5,6-Trichloro-3-methylpyrimidin-4(3H)-one (2.5 g, 11.71 mmol), tert-butyl piperidin-4-yl-carbamate (2.35 g, 11.71 mmol), and DIPEA (3.03 g, 23.42 mmol) were dissolved in NMP (20 mL), and the mixture was heated to 130°C and reacted for 2 hours. After the reaction was completed as monitored by LC-MS, the reaction liquid was cooled to room temperature, and then water (50 mL) was added. The mixture was extracted with ethyl acetate (30 mL × 3 times), and the organic layers were combined, washed with saturated brine (20 mL), dried with anhydrous sodium sulfate, filtered, and concentrated under vacuum. The residue was purified by silica gel chromatography to afford tert-butyl (1-(4,5-dichloro-1-methyl-6-oxo-1,6-dihydropyrimidin-2-yl)piperidin-4-yl)carbamate with a yield of 67.4%.

ESI-MS m/z = 213.0 $[M+H]^+$;

Step d): synthesis of tert-butyl (1-(5-chloro-4-(4-cyano-3-fluorophenyl)-1-methyl-6-oxo-1,6-dihydropyrimidin-2-yl)piperidin-4-yl)carbamate

Tert-butyl (1-(4,5-dichloro-1-methyl-6-oxo-1,6-dihydropyrimidin-2-yl)piperidin-4-yl)carbamate (1 g, 2.65 mmol), (4-cyano-3-fluorophenyl)boronic acid (570 mg, 3.44 mmol), cesium carbonate (1.73 g, 5.3 mmol), and dppf palladium dichloride (190 mg, 0.27 mmol) were weighed and dissolved in 1,4-dioxane (15 mL), then water (2 mL) was added, and the mixture was subjected to microwave heating at 120°C for 60 minutes under nitrogen protection. After the reaction was completed as indicated by LCMS, water (10 mL) was added to dilute the mixture, followed by extraction with ethyl acetate (20 mL × 3). The organic phases were combined, washed with saturated brine (15 mL × 2), dried with anhydrous sodium sulfate, filtered, and concentrated to dryness under reduced pressure. The residue was purified by silica gel column chromatography to afford tert-butyl (1-(5-chloro-4-(4-cyano-3-fluorophenyl)-1-methyl-6-oxo-1,6-dihydropyrimidin-2-yl) piperidin-4-yl)carbamate with a yield of 70.7%.

ESI-MS m/z: 462.2 $[M+H]^+$;

Step e): synthesis of 4-(2-(4-aminopiperidin-1-yl)-5-chloro-1-methyl-6-oxo-1,6-dihydropyrimidin-4-yl)-2-fluorobenzonitrile

Tert-butyl (1-(5-chloro-4-(4-cyano-3-fluorophenyl)-1-methyl-6-oxo-1,6-dihydropyrimidin-2-yl)piperidin-4-yl)carbamate (920 mg, 1.99 mmol) was added to 4N HCl (EA) (10 mL) and the reaction was conducted at room temperature for 0.5 hours and monitored by LCMS until the starting materials were completely consumed. The reaction system was then poured into a saturated aqueous solution of sodium bicarbonate, and EA was added for extraction. The organic phase was washed with saturated brine, and concentrating was conducted to remove the solvent to afford the crude product 4-(2-(4-aminopiperidin-1-yl)-5-chloro-1-methyl-6-oxo-1,6-dihydropyrimidin-4-yl)-2-fluorobenzonitrile with a yield of 91.2%.

ESI-MS m/z: 362.1 $[M+H]^+$;

Step f): synthesis of (E)-3-(4-((1-(5-chloro-4-(4-cyano-3-fluorophenyl)-1-methyl-6-oxo-1,6-dihydropyrimidin-2-yl)piperidin-4-yl)amino) methyl)phenyl)acrylate

4-(2-(4-Aminopiperidin-1-yl)-5-chloro-1-methyl-6-oxo-1,6-dihydropyrimidin-4-yl)-2-fluorobenzonitrile (679 mg, 1.88 mmol) and methyl (E)-3-(4-formylphenyl)acrylate (536.36 mg, 2.82 mmol) were added to a mixture of DCE:MeOH:$CH_3COOH$=20:1:0.1 (10 mL), and the mixture was stirred at room temperature for 0.5 hours, then cooled in an ice bath, and sodium cyanoborohydride (590.7 mg, 9.40 mmol) was added. The reaction liquid was slowly warmed back to room temperature and reacted for 1 hour, and the reaction was monitored by LCMS until the starting materials were completely consumed. The reaction system was poured into water and extracted with DCM, and the organic phases were combined. Concentrating was conducted to remove the solvent, and the residue was mixed with silica gel and purified by a normal-phase column to afford (E)-3-(4-((1-(5-chloro-4-(4-cyano-3-fluorophenyl)-1-methyl-6-oxo-1,6-dihydropyrimidin-2-yl)piperidin-4-yl)amino) methyl)phenyl)acrylate with a yield of 76.5%.

ESI-MS m/z: 539.2 $[M+H]^+$.

Step g): synthesis of methyl (E)-3-(4-((tert-butoxycarbonyl)(1-(5-chloro-4-(4-cyano-3-fluorophenyl)-1-methyl-6-oxo-1,6-dihydropyrimidin-2-yl) piperidin-4-yl)amino)methyl)phenyl)acrylate

(E)-3-(4-((1-(5-chloro-4-(4-cyano-3-fluorophenyl)-1-methyl-6-oxo-1,6-dihydropyrimidin-2-yl)piperidin-4-yl)a mino)methyl)phenyl)acrylate (1.2 g, 2.24 mmol) was dissolved in a mixture of THF:$H_2O$=3:1 (12 mL). After thorough stirring, $(Boc)_2O$ (1.47 g, 6.72 mmol) and sodium bicarbonate (0.94 g, 11.20 mmol) were added, and the mixture was stirred at room temperature for one hour. After the reaction was completed, 0.5N hydrochloric acid aqueous solution (10 mL) was added, followed by extraction with ethyl acetate (10 mL × 3), and the organic phases were combined, washed with

saturated brine (10 mL × 2), and the organic phase was concentrated to dryness under reduced pressure. The residue was then purified by silica gel chromatography to afford methyl (E)-3-(4-((tert-butoxycarbonyl)(1-(5-chloro-4-(4-cyano-3-fluorophenyl)-1-methyl-6-oxo-1,6-dihydropyrimidin-2-yl) piperidin-4-yl)amino)methyl)phenyl)acrylate with a yield of 89.0%.
ESI-MS(m/z) =636.2 $[M+H]^+$;

Step h): synthesis of methyl (E)-3-(4-((tert-butoxycarbonyl)(1-(4-(4-cyano-3-fluorophenyl)-5-(3-fluoro-4-methoxyphenyl)-1-methyl-6-oxo-1,6-d ihydropyrimidin-2-yl)piperidin-4-yl)amino)methyl)phenyl)acrylate

Methyl (E)-3-(4-((tert-butoxycarbonyl)(1-(5-chloro-4-(4-cyano-3-fluorophenyl)-1-methyl-6-oxo-1,6-dihydropyrimidin-2-yl) piperidin-4-yl)amino)methyl)phenyl)acrylate (200 mg, 0.31 mmol), cesium carbonate (303.0 mg, 0.93 mmol), 2-fluoro-4-(4,4,5,5-tetramethyl-1,3,2-dioxaborolan-2-yl)benzonitrile (66.5 mg, 0.4 mmol), and [1,1'-bis(diphenylphosphino)ferrocene]palladium dichloride (21.8 mg, 0.031 mmol) were weighed and dissolved in a mixture of 1,4-dioxane (4 mL) and water (0.5 mL). The reaction liquid was heated by microwave to 110°C for 35 minutes under nitrogen protection, and water was added to dilute the mixture, which was then extracted with ethyl acetate (15 mL × 3). The organic phases were combined, washed with saturated brine (10 mL × 2), dried with anhydrous sodium sulfate, filtered, and concentrated under reduced pressure. The residue was purified by silica gel chromatography to afford methyl (E)-3-(4-((tert-butoxycarbonyl) (1-(4-(4-cyano-3-fluorophenyl)-5-(3-fluoro-4-methoxyphenyl)-1-methyl-6-oxo-1,6-d ihydropyrimidin-2-yl)piperidin-4-yl) amino)methyl)phenyl)acrylate with a yield of 80.9%.
ESI-MS m/z: 726.3 $[M+H]^+$;

Step i): synthesis of (E)-3-(4-(tert-butoxycarbonyl)(1-(4-(4-cyano-3-fluorophenyl)-5-(3-fluoro-4-methoxyphenyl)-1-methyl-6-oxo-1,6-di hydropyrimidin-2-yl)piperidin-4-yl)amino)methyl)phenyl)acrylic acid

Methyl (E)-3-(4-((tert-butoxycarbonyl)(1-(4-(4-cyano-3-fluorophenyl)-5-(3-fluoro-4-methoxyphenyl)-1-methyl-6-oxo-1,6-d ihydropyrimidin-2-yl)piperidin-4-yl)amino)methyl)phenyl)acrylate (225.1 mg, 0.31 mmol) was dissolved in a mixture of tetrahydrofuran:water = 3: 1 (4 mL), and lithium hydroxide (74.3 mg, 3.1 mmol) was added, and the mixture was stirred for reaction for 30 minutes and extracted with ethyl acetate (10 mL × 3), and the organic phases were combined, washed with saturated brine (10 mL × 2), dried with anhydrous sodium sulfate, filtered, and concentrated under reduced pressure to afford (E)-3-(4-(tert-butoxycarbonyl)(1-(4-(4-cyano-3-fluorophenyl)-5-(3-fluoro-4-methoxyphenyl)-1-methyl-6-oxo-1,6-di hydropyrimidin-2-yl)piperidin-4-yl)amino)methyl)phenyl)acrylic acid with a yield of 93.1%.
ESI-MS m/z: 712.3 $[M+H]^+$;

Step j): synthesis of tert-butyl (E)-(1-(4-(4-cyano-3-fluorophenyl)-5-(3-fluoro-4-methoxyphenyl)-1-methyl-6-oxo-1,6-dihydropyrimidin-2-yl)piper idin-4-yl)(4-(3-oxo-3-((tetrahydro-2H-pyran-2-yloxy)amino)prop-1-en-1-yl)benzyl)carbamate

(E)-3-(4-(Tert-butoxycarbonyl)(1-(4-(4-cyano-3-fluorophenyl)-5-(3-fluoro-4-methoxyphenyl)-1-methyl-6-oxo-1,6-dihydropyrimidin-2-yl)piperidin-4-yl)amino)methyl)phenyl)acrylic acid (200 mg, 0.28 mmol), O-(tetrahydro-2H-pyran-2-yl)hydroxylamine (39.4 mg, 0.34 mmol), HATU (127.8 mg, 0.34 mmol), and DIEA (108.6 mg, 0.84 mmol) were added to a reaction flask containing DMF (5 mL), and the mixture was stirred at room temperature for 1 hour. The reaction liquid was quenched by adding water (10 mL), followed by extraction with ethyl acetate (10 mL × 2). The organic phases were combined, washed with saturated brine (15 mL × 2), dried with anhydrous sodium sulfate, filtered, and the residue was purified by silica gel chromatography to afford tert-butyl (E)-(1-(4-(4-cyano-3-fluorophenyl)-5-(3-fluoro-4-methoxyphenyl)-1-methyl-6-oxo-1,6-dihydropyrimidin-2-yl)piper idin-4-yl)(4-(3-oxo-3-((tetrahydro-2H-pyran-2-yloxy)amino) prop-1-en-1-yl)benzyl)carbamate with a yield of 75.2%.
ESI-MS m/z=810.3 [M+H]+;

Step k): synthesis of (E)-3-(4-((1-(4-(4-cyano-3-fluorophenyl)-5-(3-fluoro-4-methoxyphenyl)-1-methyl-6-oxo-1,6-dihydropyrimidin-2-yl) piperidin-4-yl)amino)methyl)phenyl)-N-hydroxyacrylamide formate

4.0M Hydrochloric acid solution in EA (6 mL) was added to a reaction flask containing tert-butyl (E)-(1-(4-(4-cyano-3-fluorophenyl)-5-(3-fluoro-4-methoxyphenyl)-1-methyl-6-oxo-1,6-dihydropyrimidin-2-yl)piper idin-4-yl)(4-(3-oxo-3-((tetrahydro-2H-pyran-2-yloxy)amino)prop-1-en-1-yl)benzyl)carbamate (180 mg, 0.29 mmol), and the mixture was stirred at room temperature for 1 hour. The reaction liquid was concentrated and then purified by Prep-HPLC (separation method 1) to afford (E)-3-(4-((1-(4-(4-cyano-3-fluorophenyl)-5-(3-fluoro-4-methoxyphenyl)-1-methyl-6-oxo-1,6)-dihydropyrimidin-2-y l)piperidin-4-yl)amino)methyl)phenyl)-N-hydroxyacrylamide formate with a yield of 25%.
ESI-MS m/z=627.3 [M+H]+.
$^1H$ NMR (400 MHz, DMSO-$d_6$) δ 10.71 (s, 1H), 9.01 (s, 1H), 8.21 - 8.16 (m, 1H), 7.80 (dd, *J* = 8.1, 6.9 Hz, 1H), 7.51

(d, $J$ = 7.9 Hz, 2H), 7.42 - 7.38 (m, 3H), 7.20 (dd, $J$ = 8.1, 1.4 Hz, 1H), 7.08 - 6.96 (m, 2H), 6.78 (dt, $J$ = 8.4, 1.4 Hz, 1H), 6.43 (d, $J$ = 15.8 Hz, 1H), 3.80 (m, 5H), 3.62 (d, $J$ = 13.0 Hz, 2H), 3.43 (m, 4H), 2.93 (t, $J$ = 11.9 Hz, 2H), 1.95 (d, $J$ = 12.6 Hz, 2H), 1.48 (q, $J$ = 11.0 Hz, 2H).

**Example 67** is prepared following the synthsis method of Example **66** (compound separation method 3), and the structure and characterization data are as follows:

| Exam ple | Chemical name | Structure | $^1$H NMR | MS(M+H)$^+$ |
|---|---|---|---|---|
| 67 | (E)-3-(4-(((1-(4-(4-Cyan o-3-fluorophenyl)-5-(3-h ydroxy-4-methoxyphenyl )-1-methyl-6-oxo-1,6-dih ydropyrimidin-2-yl)piper idin-4-yl)amino) methyl) phenyl)-N-hydroxyacryla mide formate | 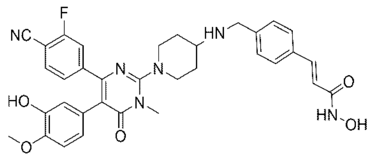 | $^1$H NMR (400 MHz, DMSO-$d_6$) δ 10.71 (s, 1H), 8.89 (s, 1H), 8.19 (s, 1H), 7.78 (t, $J$ = 8.0, 1H), 7.51 (d, $J$ = 7.9 Hz, 2H), 7.23 (dd, $J$ = 8.1, 1.4 Hz, 2H), 6.78 (d, $J$ = 8.4 Hz, 2H), 6.61 (d, $J$ = 2.1 Hz, 1H), 6.45 (s, 1H), 6.43 (s, 1H), 6.43 (s, 1H), 6.41 (d, $J$ = 2.3 Hz, 1H), 3.79 (s, 2H), 3.73 (s, 3H), 3.60 (m, 3H), 2.91 (t, $J$ = 11.8 Hz, 3H), 2.74 - 2.61 (m, 2H), 1.95 (d, $J$ = 10.2 Hz, 2H), 1.48 (d, $J$ = 11.2 Hz, 2H). | 625.3 |

**Example 69**

(E)-3-(4-(((2-(4-Aminopiperidin-1-yl)-6-(4-cyano-3-fluorophenyl)pyridin-4-yl)oxy)methyl)phenyl)-N-hydroxy acrylamide formate was prepared according to the synthetic method of Example 31, and the structure and characterization data are as follows:

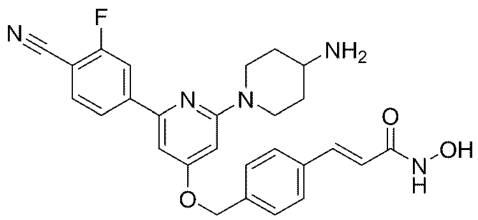

$^1$H NMR (400 MHz, DMSO+D2O-d6) δ ppm :8.41 (s, 1H), 8.20 - 8.06 (m, 2H), 7.99 (t, $J$ = 7.6 Hz, 1H), 7.60 (d, $J$ = 8.0 Hz, 2H), 7.55 - 7.40 (m, 3H), 7.14 (s, 1H), 6.58 - 6.44 (m, 2H), 5.28 (s, 2H), 4.38 (d, $J$ = 13.2 Hz, 2H), 3.08 (m, 1H), 3.02 - 2.89 (m, 2H), 1.89 (d, $J$ = 11.6 Hz, 2H), 1.38 (q, $J$ = 11.6 Hz, 2H).

ESI-MS m/z=488.2 [M+H]$^+$.

**Example 70**

(E)-3-(4-(((1-(7-(4-Cyano-3-fluorophenyl)-8-(3-hydroxy-4-methoxyphenyl)imidazo[1,2-c]pyrimidin-5-yl)piper idin-4-yl)amino)methyl)phenyl)-N-hydroxyacrylamide formate was prepared according to the synthetic method of Example 34, and the structure and characterization data are as follows:

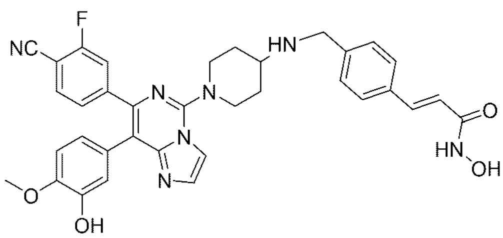

$^1H$ NMR (400 MHz, DMSO-$d_6$) δ 10.72 (s, 1H), 9.04 (s, 1H), 7.85 (s, 1H), 7.80 (t, *J* = 7.6 Hz, 1H), 7.64 (s, 1H), 7.57 - 7.39 (m, 6H), 7.35 - 7.28 (m, 1H), 6.89 (d, *J* = 8.4 Hz, 1H), 6.83 (d, *J* = 2.0 Hz, 1H), 6.65 (dd, *J* = 8.2, 2.0 Hz, 1H), 6.44 (d, *J* = 15.8 Hz, 1H), 3.90 (d, *J* = 12.0 Hz, 2H), 3.84 (s, 2H), 3.79 (s, 3H), 3.08 (t, *J* = 11.2 Hz, 2H), 2.78 (s, 1H), 2.14 - 1.93 (m, 2H), 1.62 (q, *J* = 5.0, 5.6 Hz, 2H).

ESI-MS m/z=634.2 $[M+H]^+$.

**Example 71**

Preparation of (E)-3-(4-(((1-(4-(4-cyano-3-fluorophenyl)-5-(3-hydroxy-4-methoxyphenyl)pyrimidin-2-yl)piperidin-4-yl) amino)met hyl) -N-hydroxyacrylamide formate

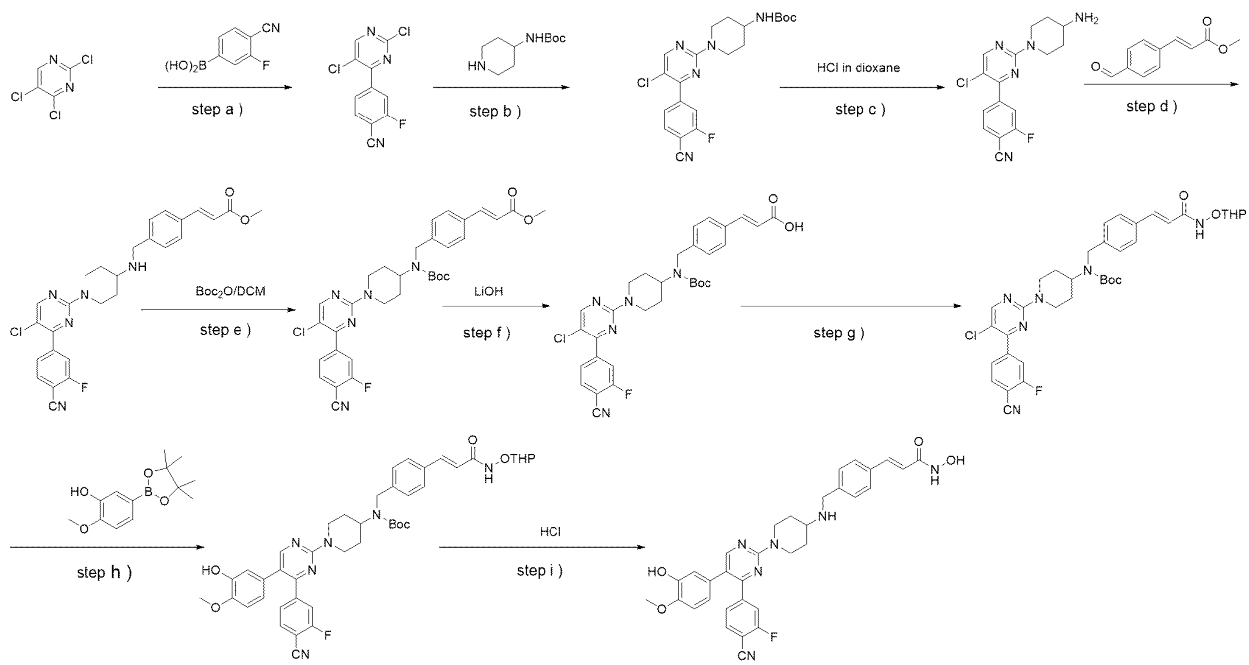


Step a): synthesis of 4-(2,5-dichloropyrimidin-4-yl)-2-fluorobenzonitrile

2,4,5-Trichloropyrimidine (4.0 g, 21.8 mmol), (4-cyano-3-fluorophenyl)boronic acid (3.59 g, 21.8 mmol), cesium carbonate (14.2 g, 43.6 mmol), and [1,1'-bis(diphenylphosphino)ferrocene]palladium dichloride (0.8 g, 0.81 mmol) were weighed and dissolved in 1,4-dioxane (60 mL), and water (15 mL) was added. The reaction was conducted in four parallel vessels and subjected to microwave heating at 110°C for 45 minutes under nitrogen protection. After cooling to room temperature, water (40 mL) was added to dilute the reaction liquid, which was then extracted with ethyl acetate (30 mL × 3). The organic phases were combined, washed with saturated brine (20 mL), dried with anhydrous sodium sulfate, filtered, and concentrated to dryness under reduced pressure. The residue was purified by silica gel column chromatography (eluent: petroleum ether: ethyl acetate = 4:1) to afford 4-(2,5-dichloropyrimidin-4-yl)-2-fluorobenzonitrile with a yield of 53.5%.

ESI-MS m/z: 268.0$[M+H]^+$.

Step b): synthesis of tert-butyl (1-(5-chloro-4-(4-cyano-3-fluorophenyl)pyrimidin-2-yl)piperidin-4-yl)carbamate

4-(2,5-Dichloropyrimidin-4-yl)-2-fluorobenzonitrile (3.5 g, 13.1 mmol) and tert-butyl piperidin-4-yl-carbamate (2.62 g, 13.1 mmol) were dissolved in DMF (50 mL), then DIPEA (5.1 g, 39.6 mmol) was added to the solution, and the mixture was refluxed at 120°C for 2 hours under nitrogen protection. After cooling to room temperature, water (50 mL) was added to dilute the mixture, followed by extraction with ethyl acetate (50 mL × 3), and the organic phases were combined, washed with saturated brine (50 mL × 2), dried with anhydrous sodium sulfate, filtered, and concentrated to dryness under reduced pressure. The residue was purified by silica gel column chromatography (eluent: petroleum ether: ethyl acetate = 2:1) to afford tert-butyl (1-(5-chloro-4-(4-cyano-3-fluorophenyl)pyrimidin-2-yl)piperidin-4-yl)carbamate with a yield of 96.2%.

$^1H$ NMR (400 MHz, DMSO-$d_6$) δ ppm 8.55 (s, 1H), 8.09 (dd, *J* = 8.2, 6.8 Hz, 1H), 7.89 (dd, *J* = 10.2, 1.6 Hz, 1H), 7.78 (dd, *J* = 8.2, 1.6 Hz, 1H), 6.86 (d, *J* = 7.8 Hz, 1H), 4.57 - 4.41 (m, 2H), 3.55 (s, 1H), 3.15 - 2.99 (m, 2H), 1.79 (dd, *J* = 13.4, 3.9 Hz, 2H), 1.38 (s, 9H), 1.35 - 1.26 (m, 2H) .
ESI-MS m/z: 432.2[M+H]$^+$.

Step c): synthesis of 4-(2-(4-aminopiperidin-1-yl)-5-chloropyrimidin-4-yl)-2-fluorobenzonitrile

Tert-butyl (1-(5-chloro-4-(4-cyano-3-fluorophenyl)pyrimidin-2-yl)piperidin-4-yl)carbamate (2.1 g, 4.87 mmol) was weighed and dissolved in 4M hydrochloric acid solution in ethyl acetate (5 mL), and the mixture was stirred at room temperature for 30 minutes under nitrogen protection, After the reaction was completed as indicated by LCMS, the mixture was concentrated under reduced pressure to afford 4-(2-(4-aminopiperidin-1-yl)-5-chloropyrimidin-4-yl)-2-fluorobenzonitrile with a yield of 95%.
ESI-MS m/z: 332.1[M+H] $^+$.

Step d): synthesis of methyl (E)-3-(4-(((1-(5-chloro-4-(4-cyano-3-fluorophenyl)pyrimidin-2-yl)piperidin-4-yl)amino) methyl)phenyl)acrylate

4-(2-(4-Aminopiperidin-1-yl)-5-chloropyrimidin-4-yl)-2-fluorobenzonitrile (1.7 g, 3.94 mmol) and methyl (E)-3-(4-formylphenyl)acrylate (1.1 g, 5.91 mmol) were weighed and dissolved in 1,2-dichloroethane (20 mL), then acetic acid (2.4 g, 39.4 mmol) and methanol (6.3 g, 197 mmol) were added, and the mixture was stirred for reaction for 3 hours under nitrogen protection. Sodium cyanoborohydride (1.2 g, 19.7 mmol) was added, and the stirring for reaction was continued for 16 hours. The reaction liquid was concentrated to dryness under reduced pressure, and the residue was purified by silica gel column chromatography (eluent: dichloromethane:methanol = 10:1) to afford methyl (E)-3-(4-(((1-(5-chloro-4-(4-cyano-3-fluorophenyl)pyrimidin-2-yl)piperidin-4-yl)amino)methyl)phenyl)acrylate with a yield of 11.0%.
ESI-MS m/z: 506.2[M+H] $^+$.

Step e): synthesis of methyl (E)-3-(4-(((tert-butoxycarbonyl)(1-(5-chloro-4-(4-cyano-3-fluorophenyl)pyrimidin-2-yl)piperidin-4-yl)amino)methy l)phenyl)acrylate

Methyl (E)-3-(4-(((1-(5-chloro-4-(4-cyano-3-fluorophenyl)pyrimidin-2-yl)piperidin-4-yl)amino)methyl)phenyl) acrylate (250 mg, 0.49 mmol), di-tert-butyl dicarbonate (214 mg, 0.98 mmol), and triethylamine (150 mg, 1.44 mmol) were weighed and dissolved in dry dichloromethane (10 mL), and the mixture was stirred at room temperature. After the reaction was completed as indicated by LCMS, the mixture was concentrated to dryness under reduced pressure. The residue was purified by silica gel column chromatography (eluent: petroleum ether: ethyl acetate = 1:2) to afford methyl (E)-3-(4-(((tert-butoxycarbonyl)(1-(5-chloro-4-(4-cyano-3-fluorophenyl)pyrimidin-2-yl)piperidin-4-yl)amino)methy l)phenyl)acrylate with a yield of 90.9%.
$^1H$ NMR (400 MHz, DMSO-$d_6$) δ ppm 8.53 (s, 1H), 8.07 (dd, *J* = 8.2, 6.8 Hz, 1H), 7.85 (dd, *J* = 10.2, 1.6 Hz, 1H), 7.76 (dd, *J* = 8.2, 1.6 Hz, 1H), 7.69 - 7.58 (m, 3H), 7.25 (d, *J* = 7.8 Hz, 2H), 6.58 (d, *J* = 160 Hz, 1H), 4.67 (d, *J* = 13.2 Hz, 2H), 4.37 (s, 2H), 4.17 (s, 1H), 3.72 (s, 3H), 2.90 (s, 2H), 1.63 (d, *J* = 8.4 Hz, 4H), 1.41 - 1.28 (m, 9H) .
ESI-MS m/z: 592.2[M+H] $^+$.

Step f): synthesis of (E)-3-(4-(((tert-butoxycarbonyl)(1-(5-chloro-4-(4-cyano-3-fluorophenyl)pyrimidin-2-yl)piperidin-4-yl)amino)methy l)phenyl)acrylic acid

Tert-butyl (E)-(1-(5-chloro-4-(4-cyano-3-fluorophenyl)pyrimidin-2-yl)piperidin-4-yl)(4-(3-methoxyprop-1-en-1-yl)benzyl)carb amate (250 mg, 0.41 mmol) and lithium hydroxide monohydrate (168.2 mg, 4.1 mmol) were weighed and dissolved in tetrahydrofuran (THF) (3 mL), and water (3 mL) was added, and the mixture was stirred for reaction for 16 hours at room temperature under nitrogen protection, acidified by adding dilute hydrochloric acid (0.5 mL), followed by extraction with ethyl acetate (10 mL × 3). The organic phases were combined and washed with saturated brine (10 mL), dried with anhydrous sodium sulfate, filtered, and concentrated to dryness under reduced pressure to afford (E)-3-(4-(((tert-butoxycarbonyl)(1-(5-chloro-4-(4-cyano-3-fluorophenyl)pyrimidin-2-yl)piperidin-4-yl)amino)methy l)phenyl)acrylic acid with a yield of 90.0%.
ESI-MS m/z: 592.2[M+H]$^+$.

Step g): synthesis of tert-butyl (E)-(1-(5-chloro-4-(4-cyano-3-fluorophenyl)pyrimidin-2-yl)piperidin-4-yl)(4-(3-oxo-3-(((tetrahydro-2H-pyran-2-yl) oxy)amino)prop-1-en-1-yl)benzyl)carbamate

(E)-3-(4-(((Tert-butoxycarbonyl)(1-(5-chloro-4-(4-cyano-3-fluorophenyl)pyrimidin-2-yl)piperidin-4-yl)amino)

methyl)phenyl)acrylic acid (260 mg, 0.44 mmol) and O-(tetrahydro-2H-pyran-2-yl)hydroxylamine (206 mg, 1.76 mmol) were weighed and dissolved in DMF (5 mL), and the mixture was stirred for reaction for 20 minutes under nitrogen protection, then 2-(7-azobenzotriazolyl)-N,N,N',N'-tetramethyluronium hexafluorophosphate (251 mg, 0.66 mmol) and DIPEA (284.3 mg, 2.2 mmol) were added, and the mixture was stirred for reaction for 16 hours under nitrogen protection. After the reaction was completed as indicated by LCMS , water (10 mL) was added to dilute the mixture, followed by extraction with ethyl acetate (10 mL × 3), and the organic phases were combined, washed with saturated brine(10 mL × 2), dried with anhydrous sodium sulfate, filtered, and concentrated to dryness under reduced pressure. The residue was purified by silica gel column chromatography (eluent: petroleum ether: ethyl acetate = 1:1) to afford tert-butyl (E)-(1-(5-chloro-4-(4-cyano-3-fluorophenyl)pyrimidin-2-yl)piperidin-4-yl)(4-(3-oxo-3-(((tetrahydro-2H-pyran-2-yl) oxy)amino) prop-1-en-1-yl)benzyl)carbamate with a yield of 77.9%.

ESI-MS m/z: 691.3[M+H] $^+$.

Step h): synthesis of tert-butyl (E)-(1-(4-(4-cyano-3-fluorophenyl)-5-(3-hydroxy-4-methoxyphenyl)pyrimidin-2-yl)piperidin-4-yl)(4-(3-oxo-3-(((tet rahydro-2H-pyran-2-yl)oxy)amino)prop-1-en-1-yl)benzyl)carbamate

Tert-butyl (E)-(1-(5-chloro-4-(4-cyano-3-fluorophenyl)pyrimidin-2-yl)piperidin-4-yl)(4-(3-oxo-3-(((tetrahydro-2H-pyran-2-yl) oxy)amino)prop-1-en-1-yl)benzyl)carbamate (150 mg, 0.22 mmol), 2-methoxy-5-(4,4,5,5-tetramethyl-1,3,2-dioxaborolan-2-yl)phenol (137.6 mg, 0.55 mmol), cesium carbonate (215 mg, 0.66 mmol), palladium acetate (9.8 mg, 0.04 mmol), and bis(di-tert-butyl(4-dimethylaminophenyl)phosphine)dichloropalladium(II) (31.2 mg, 0.04 mmol) were weighed and dissolved in a mixture of 1,4-dioxane (5 mL) and water (1 mL), and the mixture was subjected to microwave heating at 120°C and reacted for 3 hours under nitrogen protection. After cooling to room temperature, the system was concentrated under reduced pressure. Preparative thin-layer chromatography was used for purification (developing agent: dichloromethane:methanol = 12:1) to afford tert-butyl (E)-(1-(4-(4-cyano-3-fluorophenyl)-5-(3-hydroxy-4-methoxyphenyl)pyrimidin-2-yl)piperidin-4-yl)(4-(3-oxo-3-(((tet rahydro-2H-pyran-2-yl)oxy)amino)prop-1-en-1-yl) benzyl)carbamate with a yield of 42.8%.

ESI-MS m/z: 779.3[M+H] $^+$.

Step i): synthesis of (E)-3-(4-(((1-(4-(4-cyano-3-fluorophenyl)-5-(3-hydroxy-4-methoxyphenyl)pyrimidin-2-yl)piperidin-4-yl)amino)met hyl)-N-hydroxyacrylamide formate

Tert-butyl (E)-(1-(4-(4-cyano-3-fluorophenyl)-5-(3-hydroxy-4-methoxyphenyl)pyrimidin-2-yl)piperidin-4-yl) (4-(3-oxo-3-(((tet rahydro-2H-pyran-2-yl)oxy)amino)prop-1-en-1-yl)benzyl)carbamate (64 mg, 0.08 mmol) was weighed and dissolved in 4 M hydrochloric acid solution in ethyl acetate (2 mL), and the mixture was stirred for reaction for 30 minutes under nitrogen protection, then concentrated at low temperature to dryness. The residue was purified by Pre-HPLC (separation method 3) to afford (E)-3-(4-(((1-(4-(4-cyano-3-fluorophenyl)-5-(3-hydroxy-4-methoxyphenyl)pyrimidin-2-yl)piperidin-4-yl)amino)met hyl)-N-hydroxyacrylamide formate, with a yield of 17.0%.

$^1$H NMR (400 MHz, Methanol-$d_4$) δ ppm 8.52 (s, 2H), 8.38 (s, 1H), 7.69 - 7.57 (m, 3H), 7.51 - 7.40 (m, 3H), 7.35 (dd, $J$ = 8.2, 1.6 Hz, 1H), 6.95 - 6.87 (m, 1H), 6.62 - 6.55 (m, 2H), 6.50 (d, $J$ = 15.8 Hz, 1H), 4.94 (d, $J$ = 13.6 Hz, 2H), 4.09 (s, 2H), 3.86 (s, 3H), 3.18 (s, 1H), 3.09 - 2.98 (m, 2H), 2.16 (d, $J$ = 11.8 Hz, 2H), 1.53 (qd, $J$ = 12.0, 4.2 Hz, 2H).

ESI-MS m/z: 595.2[M+H]$^+$.

**Example 72**

Preparation of (S,E)-3-(4-(((1-(4''-cyano-3''-fluoro-3-hydroxy-4-methoxy-[1,1':2',1''-terphenyl]-4'-carbonylpyrrolidin-3-yl) amino) methyl)phenyl)-N-hydroxyacrylamide formate

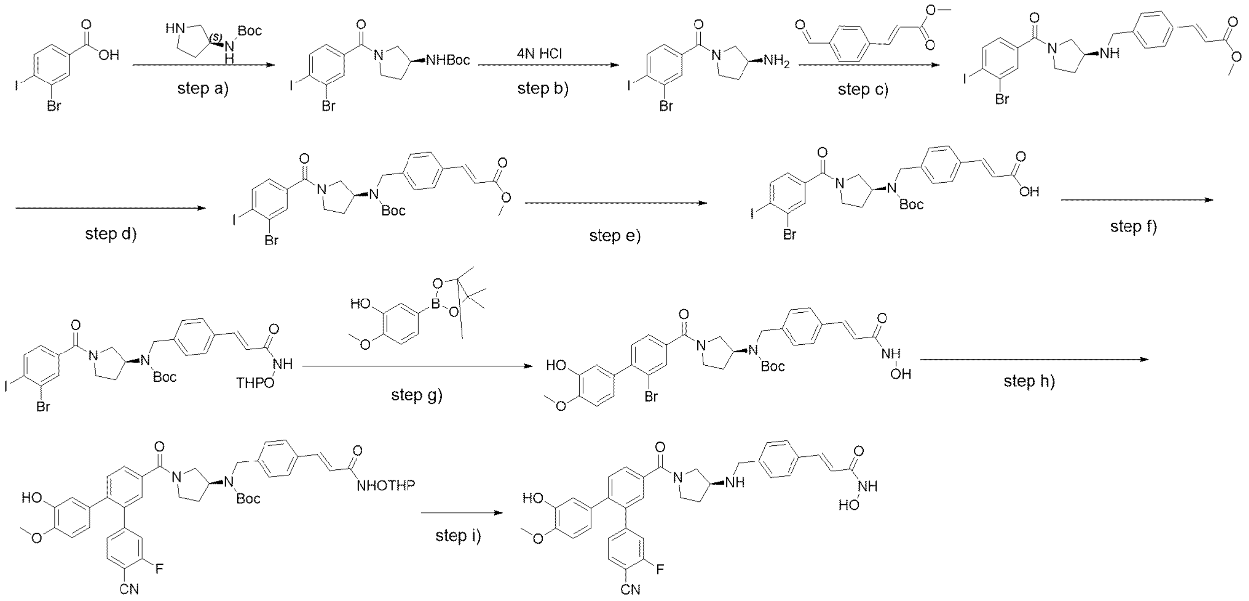


Step a): preparation of tert-butyl (S)-(1-(3-bromo-4-iodobenzoyl)pyrrolidin-3-yl)carbamate

3-Bromo-4-iodobenzoic acid (500 mg, 1.5 mmol), tert-butyl (S)-pyrrolidin-3-ylcarbamate (335 mg, 1.8 mmol), DIEA (387 mg, 3.0 mmol), and DMF (5 mL) were added to a reaction flask, then HATU (684 mg, 1.8 mol) was added under stirring at room temperature, and the reaction was maintained for 1 hour at room temperature. After the reaction was completed, it was quenched with water (10 mL), then extracted with ethyl acetate (20 mL × 2), and the organic phases were combined, washed sequentially with saturated sodium bicarbonate aqueous solution (20 mL × 1) and saturated brine (10 mL × 2), dried with anhydrous sodium sulfate, filtered, and the filtrate was concentrated under reduced pressure. The residue was purified by silica gel chromatography (eluent: petroleum ether/ethyl acetate = 1/1) to afford tert-butyl (S)-(1-(3-bromo-4-iodobenzoyl)pyrrolidin-3-yl)carbamate with a yield of 60.5%.
ESI-MS(m/z) =495.0 $[M+H]^+$.

Step b): preparation of (S)-(3-aminopyrrolidin-1-yl)(3-bromo-4-iodophenyl)methanone

Tert-butyl (S)-(1-(3-bromo-4-iodobenzoyl)pyrrolidin-3-yl)carbamate (448 mg, 908 μmol) was added to a reaction flask, followed by adding hydrogen chloride solution in ethyl acetate (4M, 2.5 mL), and the mixture was stirred at room temperature for 1 hour, resulting in the precipitation of a significant amount of solid, then the mixture was concentrated under reduced pressure to afford (S)-(3-aminopyrrolidin-1-yl)(3-bromo-4-iodophenyl)methanone hydrochloride with a yield of 95.5%.
ESI-MS(m/z) =395.0$[M+H]^+$.

Step c): preparation of methyl (S,E)-3-(4-(1-(3-bromo-4-iodobenzoyl)pyrrolidin-3-yl)amino)methyl)phenyl)acrylate

(S)-(3-Aminopyrrolidin-1-yl)(3-bromo-4-iodophenyl)methanone hydrochloride (343 mg, 867 mmol) and methyl (E)-3-(4-formylphenyl)acrylate (165 mg, 867 mmol) were added to a reaction flask containing DCE (10 mL), and the mixture was stirred at room temperature for 2 hours until the starting materials were completely consumed as indicated by LC-MS, then sodium cyanoborohydride (0.32 g, 5.04 mmol) was added to the mixture in an ice bath, and stirring was continued at room temperature for 2 hours. The reaction liquid was quenched by adding an ice-water mixture of saturated sodium bicarbonate, followed by extraction with ethyl acetate (20 mL × 2). The organic phases were combined, washed with saturated brine (15 mL × 2), dried with anhydrous sodium sulfate, filtered, and the residue was purified by silica gel chromatography (eluent: petroleum ether/ethyl acetate = 1/1) to afford methyl (S,E)-3-(4-(1-(3-bromo-4-iodobenzoyl)pyrrolidin-3-yl)amino)methyl)phenyl)acrylate with a yield of 72%.
ESI-MS m/z=568.3 $[M+H]^+$.

Step d): preparation of methyl (S,E)-3-(4-(((1-(3-bromo-iodophenyl)pyrrolidin-3-yl)(tert-butoxycarbonyl)aminomethyl) phenyl)acrylate

Methyl (S,E)-3-(4-(1-(3-bromo-4-iodobenzoyl)pyrrolidin-3-yl)amino)methyl)phenyl)acrylate (355 mg, 624 μmol)

and triethylamine (1 mL) were dissolved in DCM (5 mL), then di-tert-butyl dicarbonate (150 mg, 936 μmol) was added, and the reaction was conducted at room temperature for 1 hour. After the reaction was completed, the reaction liquid was concentrated to dryness, and the residue was purified by silica gel chromatography (eluent: petroleum ether/ethyl acetate = 1/1) to afford methyl (S,E)-3-(4-((1-(3-bromo-4-iodophenyl)pyrrolidin-3-yl)(tert-butoxycarbonyl)amino)methyl)phenyl) acrylate with a yield of 88%.

ESI-MS m/z=669.1 $[M+H]^+$.

Step e): preparation of (S,E)-3-(4-(1-(3-bromo-4-iodobenzoyl)pyrrolidin-3-yl)(tert-butoxycarbonyl)amino)methyl)phenyl)acrylic acid

Methyl (S,E)-3-(4-((1-(3-bromo-4-iodobenzoyl)pyrrolidin-3-yl)(tert-butoxycarbonyl)amino)methyl)phenyl)acrylate (367 mg, 549 μmol), lithium hydroxide monohydrate (109 mg, 2.6 mmol), THF (2 mL), isopropanol (2 mL), and water (1 mL) were added to a reaction flask and stirred at room temperature for 12 hours. 1N hydrochloric acid was slowly added dropwise with stirring in an ice bath to adjust the pH to 3-4, then water (10 mL) was added, and the mixture was extracted with ethyl acetate (10 mL × 3). The organic phases were combined, washed with saturated brine (10 mL × 2), and then concentrated to dryness under reduced pressure to afford (S,E)-3-(4-(1-(3-bromo-4-iodobenzoyl)pyrrolidin-3-yl) (tert-butoxycarbonyl)amino)methyl)phenyl)acrylic acid, which was directly used in the next reaction step.

ESI-MS(m/z) =655.2$[M+H]^+$.

Step f): preparation of tert-butyl ((S)-1-(3-bromo-4-iodobenzoyl)pyrrolidin-3-yl)(4-(E)-3-oxo-3-(tetrahydro-2H-pyran-2-yl)oxy)amino)prop-1-en-1-y l)benzyl)carbamate

(S,E)-3-(4-(1-(3-Bromo-4-iodobenzoyl)pyrrolidin-3-yl)(tert-butoxycarbonyl)amino)methyl)phenyl)acrylic acid (359 mg, 549 μmol), O-(tetrahydro-2H-pyran-2-yl)hydroxylamine (96 mg, 824 μmol), DIEA (142 mg, 1.1 mmol), and DMF (4 mL) were added to a reaction flask, then HATU (250 mg, 659 μmol) was added under stirring at room temperature, and the reaction was maintained for 1 hour at room temperature. After the reaction was completed, it was quenched with water (10 mL), then extracted with ethyl acetate (20 mL × 2). The organic phases were combined, washed sequentially with saturated sodium bicarbonate aqueous solution (20 mL × 1) and saturated brine (10 mL × 2), dried with anhydrous sodium sulfate, filtered, and the filtrate was concentrated under reduced pressure. The residue was purified by silica gel chromatography (eluent: petroleum ether/ethyl acetate = 1/1) to afford tert-butyl ((S)-1-(3-bromo-4-iodobenzoyl)pyrrolidin-3-yl)(4-(E)-3-oxo-3-(tetrahydro-2H-pyran-2-yl)oxy)amino)prop-1-en-1-y l)benzyl)carbamate with a yield of 60.5%.

ESI-MS(m/z) =754.2 $[M+H]^+$.

Step g): preparation of tert-butyl (S,E)-(1-(2-bromo-3'-hydroxy-4'-methoxy-[1,1'-biphenyl]-4-carbonyl)pyrrolidin-3-yl) (4-(3-(hydroxyamino)-3-oxo prop-1-en-1-yl)benzyl)carbamate

Tert-butyl ((S)-1-(3-bromo-4-iodobenzoyl)pyrrolidin-3-yl)(4-(E)-3-oxo-3-(tetrahydro-2H-pyran-2-yl)oxy)amino) prop-1-en-1-y l)benzyl)carbamate (250 mg, 332 μmol), 2-methoxy-5-(4,4,5,5-tetramethyl-1,3,2-dioxobenzaldehyde-2-yl)phenol (100 mg, 398 μmol), $Cs_2CO_3$ (217 mg, 664 μmol), Pd(dppf)$Cl_2$ (23 mg, 33 μmol), 1,4-dioxane (4 mL), and $H_2O$ (1 mL) were added to a reaction flask, and the mixture was stirred for reaction at 80°C for 1 hour. The mixture was concentrated to dryness under reduced pressure, and the residue was purified by silica gel chromatography (eluent: petroleum ether/ethyl acetate = 2/1) to afford tert-butyl (S,E)-(1-(2-bromo-3'-hydroxy-4'-methoxy-[1,1'-biphenyl]-4-carbonyl)pyrrolidin-3-yl)(4-(3-(hydroxyamino)-3-oxopr op-1-en-1-yl)benzyl)carbamate with a yield of 68.4%.

ESI-MS(m/z) =666.2 $[M+H]^+$.

Step h): preparation of tert-butyl ((S)-1-(4"-cyano-3"-fluoro-3-hydroxy-4-methoxy-[1,1':2',1"-terphenyl]-4'-carbonyl)pyrrolidin-3-yl)(4-((E)-3-oxo-3-((tetrahydro-2H-pyran-2-yl)oxy)amino)prop-1-en-1-yl)benzyl)carbamate

Tert-butyl (S,E)-(1-(2-bromo-3'-hydroxy-4'-methoxy-[1,1'-biphenyl]-4-carbonyl)pyrrolidin-3-yl)(4-(3-(hydroxyamino)-3-oxopr op-1-en-1-yl)benzyl)carbamate (151 mg, 227 μmol), (4-cyano-3-fluorophenyl)boronic acid (45 mg, 272 μmol), $Cs_2CO_3$ (148 mg, 454 μmol), Pd(dppf)$Cl_2$ (16 mg, 23 μmol), 1,4-dioxane (4 mL), and $H_2O$ (1 mL) were added to a reaction flask, and the mixture was stirred for reaction at 90°C for 1 hour. the mixture was concentrated to dryness under reduced pressure, and the residue was purified by silica gel chromatography (eluent: petroleum ether/ethyl acetate = 2/1) to afford tert-butyl ((S)-1-(4"-cyano-3"-fluoro-3-hydroxy-4-methoxy-[1,1':2',1"-terphenyl]-4'-carbonyl)pyrrolidin-3-yl) (4-((E)-3-oxo-3-( (tetrahydro-2H-pyran-2-yl)oxy)amino)prop-1-en-1-yl)benzyl)carbamate with a yield of 78.5%.

ESI-MS(m/z) =791.1 $[M+H]^+$.

Step i): preparation of (S,E)-3-(4-((1-(4"-cyano-3"-fluoro-3-hydroxy-4-methoxy-[1,1]:2',1"-terphenyl]-4'-carbonyl)pyrrolidin-3-yl)amino) methyl)phenyl)-N-hydroxyacrylamide formate

Tert-butyl ((S)-1-(4"-cyano-3"-fluoro-3-hydroxy-4-methoxy-[1,1:2',1"-terphenyl]-4'-carbonyl)pyrrolidin-3-y1) (4-((E)-3-oxo-3-( (tetrahydro-2H-pyran-2-yl)oxy)amino)prop-1-en-1-yl)benzyl)carbamate (141 mg, 178 μmol) was added to a reaction flask, followed by adding hydrogen chloride solution in ethyl acetate (4M,2.5 mL), and the mixture was stirred at room temperature for 1 hour, resulting in the precipitation of a significant amount of solid. After concentrating under reduced pressure, the crude product was purified by Prep-HPLC (separation method 1) to afford (S,E)-3-(4-((1-(4"-cyano-3"-fluoro-3-hydroxy-4-methoxy-[1,1]:2',1"-terphenyl]-4'-carbonyl)pyrrolidin-3-yl)amino) methyl)phenyl)-N-hydroxyacrylamide formate with a yield of 18.5%.

$^1$H NMR (400 MHz, DMSO-$d_6$) δ 7.77 - 7.40 (m, 8H), 7.37 (d, *J* = 7.4 Hz, 1H), 7.16 (dd, *J* = 4.8, 8.8 Hz, 2H), 6.90 - 6.79 (m, 1H), 6.58 (s, 2H), 6.46 (t, *J* = 6.0 Hz, 1H), 3.96 (s, 1H), 3.88-3.80 (m, 6H), 3.61 (s, 2H), 3.40 - 3.31 (m, 1H),, 2.54 - 2.48 (m, 1H), 1.92 (dd, *J*= 7.2, 6.2 Hz, 1H).

ESI-MS(m/z) =607.2 [M+H]$^+$.

**Example 73**

(S,E)-3-(4-(((1-(4"-Cyano-3"-fluoro-4-hydroxy-3-methoxy-[1,1]:2',1"-terphenyl]-4'-carbonyl)pyrrolidin-3-yl)a mino)methyl)phenyl)-N-hydroxyacrylamide formate was prepared according to the synthetic method of Example 72, and the structure and characterization data are as follows:

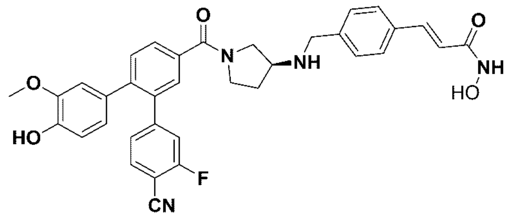

$^1$H NMR (400 MHz, Methanol-$d_4$) δ 8.46 (s, 1H), 7.70 - 7.40 (m, 8H), 7.35 (d, *J* = 7.8 Hz, 1H), 7.14 (dt, *J* = 11.2, 8.8 Hz, 2H), 6.85 (dd, *J* = 8.2, 3.2 Hz, 1H), 6.62 - 6.53 (m, 2H), 6.45 (t, *J* = 5.8 Hz, 1H), 3.89 (s, 1H), 3.84-3.79 (m, 4H), 3.79 - 3.36 (m, 5H),2.34 - 2.11 (m, 1H), 1.93 (dd, *J* = 13.3, 6.7 Hz, 1H).

ESI-MS m/z=607.3 [M+H]$^+$.

**Example 74**

Preparation of (S,E)-3-(4-(((1-((4"-cyano-3"-fluoro-3-hydroxy-4-methoxy-[1,1:2',1"-terphenyl]-4'-yl)methyl)pyrrolidin-3-yl)ami no)methyl)phenyl)-N-hydroxyacrylamide formate

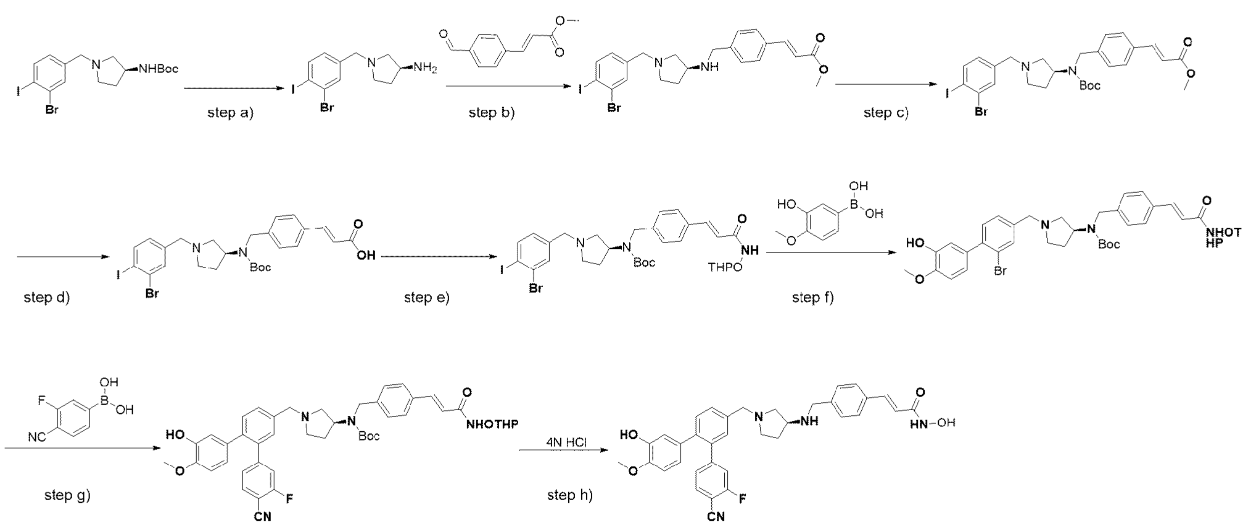

Step a): preparation of (S)-1-(3-bromo-4-iodophenyl)pyrrolidin-3-amine

Tert-butyl (S)-(1-(3-bromo-4-iodobenzyl)pyrrolidin-3-yl)carbamate (500 mg, 1.0 mmol) was added to a reaction flask, followed by adding hydrogen chloride solution in ethyl acetate (4M, 2.5 mL), and the mixture was stirred at room temperature for one hour, resulting in the precipitation of a significant amount of solid. The mixture was concentrated under reduced pressure to afford (S)-1-(3-bromo-4-iodophenyl)pyrrolidin-3-amine hydrochloride with a yield of 95.5%.
ESI-MS(m/z) =380.9[M+H]$^+$.

Step b): preparation of methyl (S,E)-3-(4-(1-(3-bromo-4-iodobenzyl)pyrrolidin-3-yl)amino)methyl)phenyl)acrylate

(S)-1-(3-Bromo-4-iodophenyl)pyrrolidin-3-amine hydrochloride (362 mg, 955 mmol) and methyl (E)-3-(4-formylphenyl)acrylate (181 mg, 955 mmol) were added to a reaction flask containing DCE (10 mL), and the mixture was stirred at room temperature for 2 hours until the starting materials were completely consumed as monitored by LC-MS, then sodium cyanoborohydride (0.32 g, 4.8 mmol) was then added to the mixture in an ice bath, and stirring was continued at room temperature for 2 hours. The reaction liquid was quenched by adding an ice-water mixture of saturated sodium bicarbonate, followed by extraction with ethyl acetate (20 mL × 2). The organic phases were combined, washed with saturated brine (15 mL × 2), dried with anhydrous sodium sulfate, filtered, and the residue was purified by silica gel chromatography (eluent: petroleum ether/ethyl acetate = 1/1) to afford methyl (S,E)-3-(4-(1-(3-bromo-4-iodophenyl) pyrrolidin-3-yl)amino)methyl)phenyl)acrylate with a yield of 68%.
ESI-MS m/z=555.0 [M+H]$^+$.

Step c): preparation of methyl (S,E)-3-(4-(1-(3-bromo-4-iodophenyl)pyrrolidin-3-yl)(tert-butoxycarbonyl)amino)methyl) phenyl)acrylate

Methyl (S,E)-3-(4-(1-(3-bromo-4-iodophenyl)pyrrolidin-3-yl)amino)methyl)phenyl)acrylate (360 mg, 649 μmol) and triethylamine (1 mL) were dissolved in DCM (5 mL), then di-tert-butyl dicarbonate (150 mg, 936 μmol) was added, and the reaction was conducted at room temperature for 1 hour. After the reaction was completed, the reaction liquid was concentrated to dryness, and the residue was purified by silica gel chromatography (eluent: petroleum ether/ethyl acetate = 1/1) to afford methyl (S,E)-3-(4-(1-(3-bromo-4-iodophenyl)pyrrolidin-3-yl)(tert-butoxycarbonyl)amino)methyl)phenyl) acrylate with a yield of 91%.
ESI-MS m/z=655.1 [M+H]$^+$.

Step d): preparation of (S,E)-3-(4-(1-(3-bromo-4-iodophenyl)pyrrolidin-3-yl)(tert-butoxycarbonyl)amino)methyl)phenyl) acrylic acid

Methyl (S,E)-3-(4-(1-(3-bromo-4-iodophenyl)pyrrolidin-3-yl)(tert-butoxycarbonyl)amino)methyl)phenyl)acrylate (387 mg, 591 μmol), lithium hydroxide monohydrate (124 mg, 3.0 mmol), tetrahydrofuran (2 mL), isopropanol (2 mL), and water (1 mL) were added to a reaction flask and the mixture was stirred at room temperature for 12 hours. The pH was adjusted to pH=3~4 by slowly the addition of 1N concentrated hydrochloric acid dropwise under stirring in an ice bath, then water (10 mL) was added, and the mixture was extracted with ethyl acetate (10 mL×3). The organic phases were combined and washed with saturated brine (10 mL×2), concentrated to dryness under reduced pressure to afford (S,E)-3-(4-(1-(3-bromo-4-iodophenyl)pyrrolidin-3-yl)(tert-butoxycarbonyl)amino)methyl)phenyl)acrylic acid, which was directly used in the next reaction step.
ESI-MS(m/z) =641.1[M+H]$^+$.

Step e): preparation of tert-butyl ((S)-1-(3-bromo-4-iodophenyl)pyrrolidin-3-yl)(4-(E)-3-oxo-3-(tetrahydro-2H-pyran-2-yl)oxy)amino)prop-1-en-1-yl) benzyl)carbamate

(S,E)-3-(4-(1-(3-Bromo-4-iodophenyl)pyrrolidin-3-yl)(tert-butoxycarbonyl)amino)methyl)phenyl)acrylic acid (379 mg, 591 μmol), O-(tetrahydro-2H-pyran-2-yl)hydroxylamine (104 mg, 887 μmol), DIEA (142 mg, 1.2 mmol), and DMF (4 mL) were added to a reaction flask, then HATU (270 mg, 709 μmol) was added under stirring at room temperature, and the reaction was maintained for 1 hour at room temperature. After the reaction was completed, it was quenched with the addition of water (10 mL), followed by extraction with ethyl acetate (20 mL × 2). The organic phases were combined, washed sequentially with saturated sodium bicarbonate aqueous solution (20 mL × 1) and saturated brine (10 mL × 2), dried with anhydrous sodium sulfate, filtered, and the filtrate was concentrated under reduced pressure. The residue was purified by silica gel chromatography (eluent: petroleum ether/ethyl acetate = 1/1) to afford tert-butyl ((S)-1-(3-bromo-4-iodophenyl)pyrrolidin-3-yl)(4-(E)-3-oxo-3-(tetrahydro-2H-pyran-2-yl)oxy)amino)prop-1-en-1-yl) benzyl)carbamate with a yield of 63.1%.

ESI-MS(m/z) =740.1 $[M+H]^+$.

Step f): preparation of tert-butyl ((S)-1-((2-bromo-3'-hydroxy-4'-methoxy-[1,1'-biphenyl]-4-yl)methyl)pyrrolidin-3-yl)(4-(E)-3-oxo-3-(tetrahydro-2 H-pyran-2-yl)oxy)amino)prop-1-en-1-yl)benzyl)carbamate

Tert-butyl ((S)-1-(3-bromo-4-iodophenyl)pyrrolidin-3-yl)(4-((E)-3-oxo-3-(tetrahydro-2H-pyran-2-yl)oxy)amino)prop-1-en-1-y l)benzyl)carbamate (275 mg, 372 μmol), 2-methoxy-5-(4,4,5,5-tetramethyl-1,3,2-dioxobenzaldehyde-2-yl)phenol (112 mg, 446 μmol), $Cs_2CO_3$ (243 mg, 744 μmol), Pd(dppf)$Cl_2$ (27 mg, 37 μmol), 1,4-dioxane (4 mL), and water ($H_2O$) (1 mL) were added to a reaction flask, and the mixture was stirred for reaction at 80°C for 1 hour. The mixture was concentrated to dryness under reduced pressure, and the residue was then purified by silica gel chromatography (eluent: petroleum ether/ethyl acetate = 2/1) to afford tert-butyl ((S)-1-((2-bromo-3'-hydroxy-4'-methoxy-[1,1'-biphenyl]-4-yl)methyl)pyrrolidin-3-yl)(4-(E)-3-oxo-3-(tetrahydro-2H -pyran-2-yl)oxy)amino)prop-1-en-1-yl)benzyl)carbamate with a yield of 70.4%.

ESI-MS(m/z) =736.3 $[M+H]^+$.

Step g): preparation of tert-butyl ((S)-1-((4"-cyano-3"-fluoro-3-hydroxy-4-methoxy-[1,1:2',1"-terphenyl]-4'-yl)methyl)pyrrolidin-3-yl)(4-((E)-3-ox o-3-((tetrahydro-2H-pyran-2-yl)oxy)amino)prop-1-en-1-yl)benzyl)carbamate

Tert-butyl ((S)-1-((2-bromo-3'-hydroxy-4'-methoxy-[1,1'-biphenyl]-4-yl)methyl)pyrrolidin-3-yl)(4-(E)-3-oxo-3-(tetrahydro-2H-pyran-2-yl)oxy)amino)prop-1-en-1-yl)benzyl)carbamate (192 mg, 262 μmol), (4-cyano-3-fluorophenyl)boronic acid (52 mg, 314 μmol), $Cs_2CO_3$ (171 mg, 524 μmol), Pd(dppf)$Cl_2$ (17 mg, 26 μmol), 1,4-dioxane (4 mL), and water (1 mL) were added to a reaction flask, and the mixture was stirred for reaction at 90°C for 1 hour. The mixture was concentrated to dryness under reduced pressure, and the residue was then purified by silica gel chromatography (eluent: petroleum ether/ethyl acetate = 2/1) to afford tert-butyl ((S)-1-((4"-cyano-3"-fluoro-3-hydroxy-4-methoxy-[1,1:2',1"-terphenyl]-4'-yl)methyl)pyrrolidin-3-yl)(4-((E)-3-oxo-3 -((tetrahydro-2H-pyran-2-yl)oxy)amino)prop-1-en-1-yl)benzyl)carbamate with a yield of 70.5%.

ESI-MS(m/z) =777.1 $[M+H]^+$.

Step h): preparation of (S,E)-3-(4-(((1-((4"-cyano-3"-fluoro-3-hydroxy-4-methoxy-[1,1:2',1"-terphenyl]-4'-yl)methyl)pyrrolidin-3-yl)ami no)methyl)phenyl)-N-hydroxyacrylamide formate

Tert-butyl ((S)-1-((4"-cyano-3"-fluoro-3-hydroxy-4-methoxy-[1,1:2',1"-terphenyl]-4'-yl)methyl)pyrrolidin-3-yl)(4-((E)-3-oxo-3 -((tetrahydro-2H-pyran-2-yl)oxy)amino)prop-1-en-1-yl)benzyl)carbamate (144 mg, 185 μmol) was added to a reaction flask, followed by adding hydrogen chloride solution in ethyl acetate (4M, 2.5 mL), and the mixture was stirred at room temperature for 1 hour, resulting in the precipitation of a significant amount of solid. After concentrating under reduced pressure, the crude product was purified by Prep-HPLC ( separation method 3) to afford (S,E)-3-(4-((1-((4"-cyano-3"-fluoro-3-hydroxy-4-methoxy-[1,1;2',1"-terphenyl]-4'-yl)methyl)pyrrolidin-3-yl)amino) methyl)phenyl)-N-hydroxyacrylamide formate with a yield of 15.5%.

$^1$H NMR (400 MHz, Methanol-$d_4$) δ 8.48 (s, 1H), 7.56 (d, $J$ = 8.6 Hz, 4H), 7.43- 7.03 (m, 5H), 7.13 (s, 2H), 6.83 (d, $J$ = 7.8 Hz, 1H), 6.61 - 6.42 (m, 3H), 3.98 (d, $J$ = 7.0 Hz, 4H), 3.83 (s, 3H), 3.69 (s, 1H), 3.14 - 2.61 (m, 4H), 2.33 (s, 1H), 1.94 (s, 1H).

ESI-MS(m/z) =593.3 $[M+H]^+$.

**Example 75**

(S,E)-3-(4-(((1-((4"-Cyano-3"-fluoro-4-hydroxy-3-methoxy-[1,1:2,1"-terphenyl]-4'-yl)methyl)pyrrolidin-3-yl)a mino)methyl)phenyl)-N-hydroxyacrylamide formate was prepared according to the synthetic method of Example 74, and the structure and characterization data are as follows:

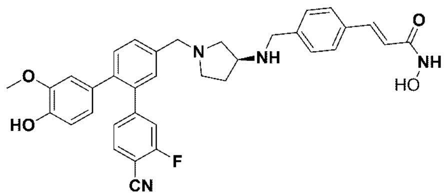

$^1$H NMR (400 MHz, Methanol-$d_4$) δ 8.40 (s, 1H), 7.74 - 7.57 (m, 4H), 7.51 (d, $J$ = 6.8 Hz, 5H), 7.16 (d, $J$ = 3.8 Hz, 2H), 6.73 (d, $J$ = 8.0 Hz, 1H), 6.58 (s, 2H), 6.50 (d, $J$ = 5.2 Hz, 1H), 4.08 (s, 2H), 3.96 (s, 2H), 3.75 (s, 1H), 3.63 (s, 3H), 3.10

(s, 1H), 2.95 (s, 2H), 2.75 (s, 1H),2.38 - 2.30 (m, 4H), 1.97 (dd, *J* = 10.2, 8.0 Hz, 1H).
ESI-MS m/z=593.3 $[M+H]^+$.

**Example 76**

Preparation of (E)-3-(4-(((1-(4-(4-cyano-3-fluorophenyl)-3-fluoro-5-(3-hydroxy-4-methoxyphenyl)pyridin-2-yl)piperidin-4-yl)ami no)methyl)phenyl)-N-hydroxyacrylamide formate

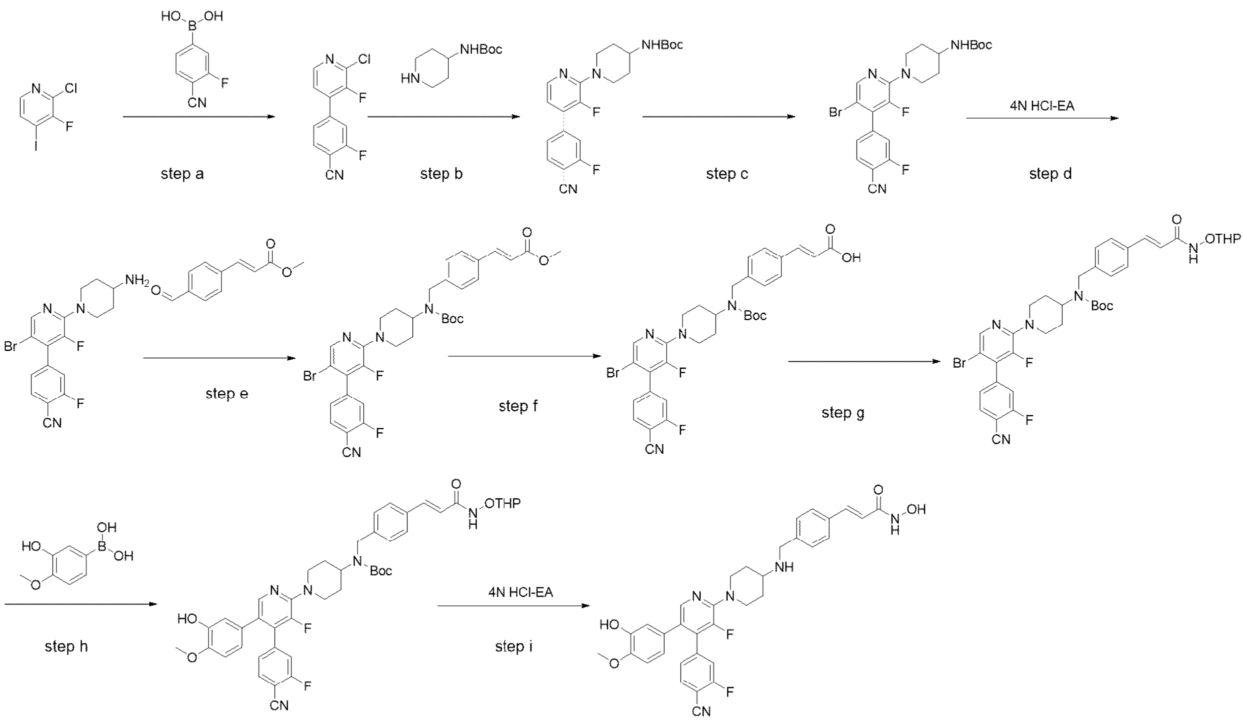


Step a): preparation of 4-(2-chloro-3-fluoropyridin-4-yl)-2-fluorobenzonitrile

2-Chloro-3-fluoro-4-iodopyridine (2 g, 7.78 mmol), (4-cyano-3-fluorophenyl)boronic acid (1.28 g, 7.78 mmol), $CS_2CO_3$ (5.07 g, 15.56 mmol), and Pd(dppf)$Cl_2$ (0.57 g, 0.78 mmol) were dissolved in a mixture of 1,4-dioxane (3 mL) and water (1 mL), and the mixture was subjected to microwave heating at 65°C and reacted for 30 minutes under nitrogen protection. After concentrating, a crude product was obtained and purified by silica gel chromatography (eluent: petroleum ether/ethyl acetate = 10/3) to afford 4-(2-chloro-3-fluoropyridin-4-yl)-2-fluorobenzonitrile with a yield of 78%.
ESI-MS m/z=251.0 $[M+H]^+$

Step b): preparation of tert-butyl N-(1-(4-(4-cyano-3-fluorophenyl)-3-fluoropyridin-2-yl)piperidin-4-yl)carbamate

4-(2-Chloro-3-fluoropyridin-4-yl)-2-fluorobenzonitrile (1.0 g, 3.99 mmol), tert-butyl N-(piperidin-4-yl)carbamate (1.1 g, 5.99 mmol), 2-dicyclohexylphosphino-2',6'-diisopropoxybiphenyl (186.19 mg, 0.40 mmol), tris(dibenzylideneacetone)dipalladium (182.69 mg, 0.20 mmol), and $CS_2CO_3$ (3.9 g, 11.97 mmol) were dissolved in toluene (15 mL), and the mixture was subjected to microwave heating at 110°C and reacted for 4 hours under nitrogen protection. Water (200 mL) was added to quench the reaction, followed by extraction with ethyl acetate (200 mL $\times$ 2), and the organic phases were combined, washed with saturated brine (150 mL $\times$ 2), dried with anhydrous sodium sulfate, filtered, and concentrated to obtain a crude product. The residue was purified by silica gel chromatography (eluent: petroleum ether/ethyl acetate = 5/2) to afford tert-butyl N-(1-(4-(4-cyano-3-fluorophenyl)-3-fluoropyridin-2-yl)piperidin-4-yl)carbamate with a yield of 42%.
ESI-MS m/z=415.2 $[M+H]^+$

Step c): preparation of tert-butyl N-(1-(5-bromo-4-(4-cyano-3-fluorophenyl)-3-fluoropyridin-2-yl)piperidin-4-yl)carbamate

Tert-butyl N-(1-(4-(4-cyano-3-fluorophenyl)-3-fluoropyridin-2-yl)piperidin-4-yl)carbamate (930 mg, 2.24 mmol) and N-bromosuccinimide (398.68 mg, 2.24 mmol) were dissolved in DMF (16 mL) and reacted for 30 minutes. After the reaction was completed, water (20 mL) was added to quench the reaction, followed by extraction with ethyl acetate (20 mL × 2), and the organic phases were combined, washed with saturated brine (15 mL × 2), dried with anhydrous sodium sulfate, filtered, and concentrated to obtain a crude product. The residue was purified by silica gel chromatography (eluent: petroleum ether/ethyl acetate = 10/7) to afford tert-butyl N-(1-(5-bromo-4-(4-cyano-3-fluorophenyl)-3-fluoropyridin-2-yl) piperidin-4-yl)carbamate with a yield of 87%.
ESI-MS m/z=493.1 $[M+H]^+$

Step d): preparation of 4-(2-(4-aminopiperidin-1-yl)-5-bromo-3-fluoropyridin-4-yl)-2-fluorobenzonitrile

Tert-butyl N-(1-(5-bromo-4-(4-cyano-3-fluorophenyl)-3-fluoropyridin-2-yl)piperidin-4-yl)carbamate (970 mg, 1.97 mmol) was dissolved in a 4N hydrochloric acid ethyl acetate (20 mL) and reacted for 30 minutes. After concentrating, a crude product was obtained, which was directly used in the next reaction step.
ESI-MS m/z=393.0 $[M+H]^+$

Step e): preparation of methyl (E)-3-(4-(1-(5-bromo-4-(4-cyano-3-fluorophenyl)-3-fluoropyridin-2-yl)piperidin-4-yl)(tert-butoxycarbonyl)amino) methyl)phenyl)acrylate

4-(2-(4-Aminopiperidin-1-yl)-5-bromo-3-fluoropyridin-4-yl)-2-fluorobenzonitrile (220 mg, 0.51 mmol) and methyl (E)-3-(4-formylphenyl)acrylate (97.00 mg, 0.51 mmol) were dissolved in 1,2-dichloroethane (6 mL), methanol (40 μL), and acetic acid (10 μL) and reacted for 30 minutes, then the reaction liquid was cooled in an ice bath, and sodium cyanoborohydride (96.15 mg, 1.53 mmol) was added and reacted overnight. The reaction liquid was quenched with the addition of water (5 mL), and the mixture was concentrated to obtain a crude product. Then di-tert-butyl dicarbonate (320.83 mg, 1.47 mmol) and $Na_2CO_3$ (155.82 mg, 1.47 mmol) were dissolved in a mixture of tetrahydrofuran (THF) (3 mL) and water (2 mL) and added to the crude product to react for 30 minutes. Water (20 mL) was added to quenched the reaction, and the mixture was extracted with ethyl acetate (20 mL × 2). The organic phases were combined, washed with saturated brine (15 mL × 2), dried with anhydrous sodium sulfate, filtered, and concentrated to obtain a crude product. The residue was purified by silica gel chromatography (eluent: petroleum ether/ethyl acetate = 10/7) to afford methyl (E)-3-(4-(1-(5-bromo-4-(4-cyano-3-fluorophenyl)-3-fluoropyridin-2-yl)piperidin-4-yl)(tert-butoxycarbonyl)amino) methyl) phenyl)acrylate with a yield of 91%.
ESI-MS m/z=666.2 $[M+H]^+$

Step f): preparation of (E)-3-(4-(1-(5-bromo-4-(4-cyano-3-fluorophenyl)-3-fluoropyridin-2-yl)piperidin-4-yl)(tert-butoxycarbonyl)amino) methyl)phenyl)acrylic acid

Methyl (E)-3-(4-(1-(5-bromo-4-(4-cyano-3-fluorophenyl)-3-fluoropyridin-2-yl)piperidin-4-yl)(tert-butoxycarbonyl)amino) methyl)phenyl)acrylate (300 mg, 0.45 mmol) and lithium hydroxide monohydrate (188.82 mg, 4.5 mmol) were dissolved in a mixture of THF (3 mL) and water (1 mL) and reacted for 36 hours. After the reaction, water (10 mL) was added, and the pH was adjusted to 3-4 with 1N HCl solution, the mixture was then extracted with ethyl acetate (20 mL × 2), and the organic phases were combined and washed with saturated brine (15 mL × 2), dried with anhydrous sodium sulfate, filtered, and concentrated to obtain a crude product, which was directly used in the subsequent step.
ESI-MS m/z=653.2 $[M+H]^+$

Step g): preparation of tert-butyl (E)-(1-(5-bromo-4-(4-cyano-3-fluorophenyl)-3-fluoropyridin-2-yl)piperidin-4-yl)(4-(3-oxo-3-((tetrahydro-2H-pyran -2-yl)oxy)amino)prop-1-en-1-yl)benzyl)carbamate

(E)-3-(4-(1-(5-Bromo-4-(4-cyano-3-fluorophenyl)-3-fluoropyridin-2-yl)piperidin-4-yl)(tert-butoxycarbonyl)am ino)methyl)phenyl)acrylic acid (280 mg, 0.43 mmol) and O-(tetrahydro-2H-pyran-2-yl)hydroxylamine (151.12 mg, 1.29 mmol) were dissolved in DMF (30 mL), then HATU (490.51 mg, 1.29 mmol) and DIPEA (277.35 mg, 2.15 mmol) were added, and reacted for 30 minutes. The reaction liquid was quenched by adding water (20 mL), followed by extraction with ethyl acetate (20 mL × 2), and the organic phases were combined, washed with saturated brine (15 mL × 2), dried with anhydrous sodium sulfate, filtered, and concentrated to obtain a crude product. The residue was then purified by silica gel chromatography (eluent: dichloromethane/methanol = 15/1) to afford tert-butyl (E)-(1-(5-bromo-4-(4-cyano-3-fluorophenyl)-3-fluoropyridin-2-yl)piperidin-4-yl)(4-(3-oxo-3-((tetrahydro-2H-pyran -2-yl)oxy)amino)prop-1-en-1-yl)benzyl)carbamate with a yield of 92%.

ESI-MS m/z=752.2 [M+H]+

Step h): preparation of tert-butyl (E)-(1-(4-(4-cyano-3-fluorophenyl)-3-fluoro-5-(3-hydroxy-4-methoxyphenyl)pyridin-2-yl)piperidin-4-yl)(4-(3-oxo-3-(((tetrahydro-2H-pyran-2-yl)oxy)amino)prop-1-en-1-yl)benzyl)carbamate

Tert-butyl (E)-(1-(5-bromo-4-(4-cyano-3-fluorophenyl)-3-fluoropyridin-2-yl)piperidin-4-yl)(4-(3-oxo-3-((tetrahydro-2H-pyran -2-yl)oxy)amino)prop-1-en-1-yl)benzyl)carbamate (300 mg, 0.40 mmol), (3-hydroxy-4-methoxyphenyl) boronic acid (100.77 mg, 0.60 mmol), $Cs_2CO_3$ (390.98 mg, 1.20 mmol), and Pd(dppf)$Cl_2$ (58.54 mg, 0.080 mmol) were dissolved in a mixture of 1,4-dioxane (3 mL) and water (1 mL), and the mixture was subjected to microwave heating at 90°C and reacted for 30 minutes under nitrogen protection. After concentrating, a crude product was obtained, and the residue was then purified by silica gel chromatography (eluent: dichloromethane/methanol = 10/1) to afford tert-butyl (E)-(1-(4-(4-cyano-3-fluorophenyl)-3-fluoro-5-(3-hydroxy-4-methoxyphenyl)pyridin-2-yl)piperidin-4-yl)(4-(3-oxo-3-(((tetrahydro-2H-pyran-2-yl)oxy)amino)prop-1-en-1-yl)benzyl)carbamate with a yield of 80%.

ESI-MS m/z=796.3[M+H]+

Step i): preparation of (E)-3-(4-(4-(4-cyano-3-fluorophenyl)-3-fluoro-5-(3-hydroxy-4-methoxyphenyl)pyridin-2-yl)piperidin-4-yl)amino)m ethyl)phenyl)-N-hydroxyacrylamide

Tert-butyl tert-butyl (E)-(1-(4-(4-cyano-3-fluorophenyl)-3-fluoro-5-(3-hydroxy-4-methoxyphenyl)pyridin-2-yl)piperidin-4-yl)(4-(3-oxo-3-(((tetrahydro-2H-pyran-2-yl)oxy)amino)prop-1-en-1-yl)benzyl)carbamate (0.7 g, 0.88 mmol) was dissolved in a 4N hydrochloric acid solution in ethyl acetate (15 mL) and reacted for 30 minutes. The mixture was concentrated under reduced pressure at low temperature to obtain a residue, and the residue was purified by Prep-HPLC (separation method 3) to afford (E)-3-(4-(((1-(4-(4-cyano-3-fluorophenyl)-3-fluoro-5-(3-hydroxy-4-methoxyphenyl) pyridin-2-yl)piperidin-4-yl)ami no)methyl)phenyl)-N-hydroxyacrylamide formate with a yield of 5%.

$^1$H NMR (400 MHz, Methanol-$d_4$) δppm 8.52 (s, 1H), 8.08 (s, 1H), 7.72 (t, *J* = 7.4 Hz, 1H), 7.65 (t, *J* = 8.6 Hz, 2H), 7.62 - 7.45 (m, 3H), 7.28 (d, *J* = 9.8 Hz, 1H), 7.17 (d, *J* = 8.0 Hz, 1H), 6.84 (d, *J* = 8.8 Hz, 1H), 6.58 - 6.45 (m, 3H), 4.59 (s, 1H), 4.24 (s, 1H), 4.21 (s, 3H), 3.84 (s, 3H), 3.05 (t, *J* = 12.6 Hz, 2H), 2.24 (d, *J* = 12.2 Hz, 2H), 1.77 (dd, *J* = 13.6, 9.8 Hz, 2H).

ESI-MS m/z=612.2 [M+H]+

**Example 77**

(E)-3-(4-(((1-(4-(4-Cyano-3-fluorophenyl)-3-fluoro-5-(4-hydroxy-3-methoxyphenyl)pyridin-2-yl)piperidin-4-yl) amino)methyl)phenyl)-N-hydroxyacrylamide formate was prepared according to the synthetic method of Example 76, and the structure and characterization data are as follows:

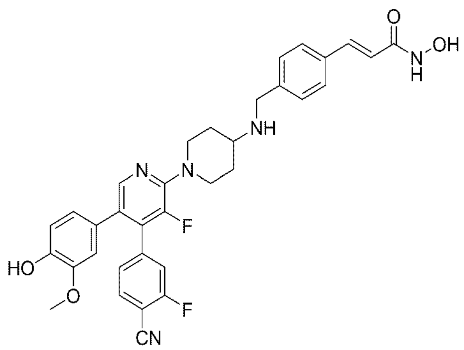

$^1$H NMR (400 MHz, Methanol-$d_4$) δppm 8.52 (s, 2H), 8.12 (s, 1H), 7.73 (dd, *J* = 8.0, 6.8 Hz, 1H), 7.63 (t, *J*= 10.2 Hz, 2H), 7.55 (t, *J* = 8.0 Hz, 3H), 7.30 (d, *J* = 9.8 Hz, 1H), 7.19 (d, *J* = 8.0 Hz, 1H), 6.73 (d, *J* = 8.0 Hz, 1H), 6.64 - 6.47 (m, 3H), 4.23 (s, 1H), 4.19 (s, 3H), 3.65 (s, 3H), 3.26 (s, 1H), 3.04 (t, *J* = 12.6 Hz, 2H), 2.23 (d, *J* = 12.0 Hz, 2H), 1.78 (q, *J* = 11.2, 10.4 Hz, 2H).

ESI-MS m/z=612.2 [M+H]+

**Example 80**

Synthesis of (E)-3-(4-{[(1-(4-(4-cyano-3-fluorophenyl)-5-(3-hydroxy-4-methoxyphenyl)pyridin-2-yl)piperidin-4-yl)amino] methy l}phenyl)-N-hydroxyprop-2-enamide formate

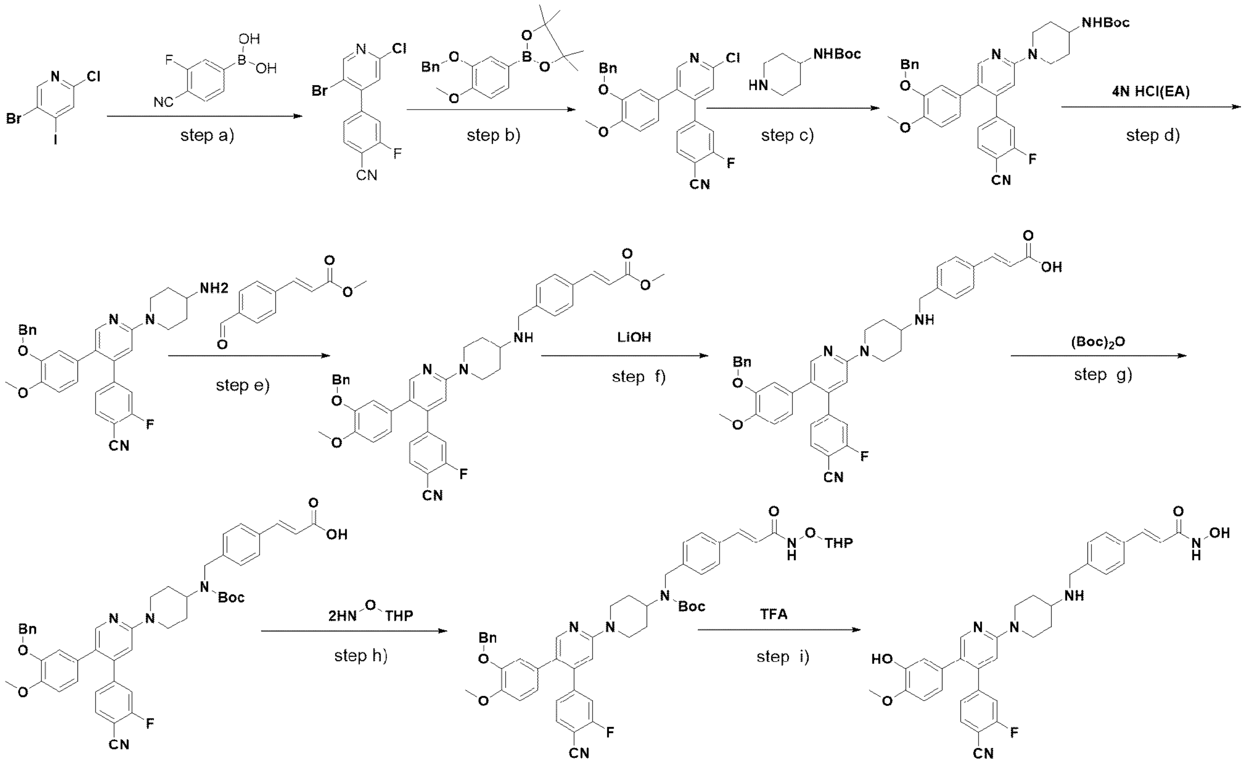


Step a): synthesis of 4-(5-bromo-2-chloropyridin-4-yl)-2-fluorobenzonitrile

5-Bromo-2-chloro-4-iodopyridine (3.0 g, 9.42 mmol), (4-cyano-3-fluorophenyl)boronic acid (1.5 g, 9.42 mmol), [1,1'-bis(diphenylphosphino)ferrocene]palladium dichloride (689.26 mg, 0.94 mmol), and cesium carbonate (6138.45 mg, 18.84 mmol) were added to a mixture of 1,4-dioxane : water =5:1 (20 mL), and the mixture was subjected to microwave heating at 110°C and reacted for 1 hour under nitrogen protection and monitored by LCMS until the starting materials were completely consumed. The solvent was directly removed by concentrating, and the residue was mixed with silica gel and purified by a normal-phase column to afford 4-(5-bromo-2-chloropyridin-4-yl)-2-fluorobenzonitrile with a yield of 82.46%.
ESI-MS(m/z) =311.1 $[M+H]^+$.

Step b): synthesis of 4-(5-(3-(benzyloxy)-4-methoxyphenyl)-2-chloropyridin-4-yl)-2-fluorobenzonitrile

4-(5-Bromo-2-chloropyridin-4-yl)-2-fluorobenzonitrile (1.5 g, 4.81 mmol), 2-(3-(benzyloxy)-4-methoxyphenyl)-4,4,5,5-tetramethyl-1,3,2-dioxaborolane (1.9 g, 5.53 mmol), [1,1'-bis(diphenylphosphino)ferrocene]palladium dichloride (351.95 mg, 0.48 mmol), and cesium carbonate (3.1 g, 9.62 mmol) were added to a mixture of 1,4-dioxane : water = 5:1 (30 mL), and the mixture was reacted at 80°C for 1 hour under nitrogen protection and monitored by LCMS until the starting materials were completely consumed. The solvent was directly removed by concentrating, and the residue was mixed with silica gel and purified by a normal-phase column to afford 4-(5-(3-(benzyloxy)-4-methoxyphenyl)-2-chloropyridin-4-yl)-2-fluorobenzonitrile with a yield of 88.32%.
ESI-MS(m/z) =445.1 $[M+H]^+$.

Step c): synthesis of tert-butyl 4-(5-(3-(benzyloxy)-4-methoxyphenyl)-4-(4-cyano-3-fluorophenyl)pyridin-2-yl)piperazine-1-carboxylate

4-(5-(3-(Benzyloxy)-4-methoxyphenyl)-2-chloropyridin-4-yl)-2-fluorobenzonitrile (1.5 g, 3.3 mmol), tert-butyl

N-(piperidin-4-yl)carbamate (990.5 mg, 5.1 mmol), 2-dicyclohexylphosphino-2',6'-diisopropoxybiphenyl (155 mg, 0.33 mmol), tris(dibenzylideneacetone)dipalladium (150.5 mg, 0.165 mmol), and cesium carbonate (3215.2 mg, 9.9 mmol) were added to toluene (50 mL). The reaction was monitored by LCMS until the starting materials were completely consumed, and concentrating was conducted to remove the solvent. The residue was mixed with silica gel and purified by a normal-phase column to afford tert-butyl 4-(5-(3-(benzyloxy)-4-methoxyphenyl)-4-(4-cyano-3-fluorophenyl)pyridin-2-yl) piperazine-1-carboxylate with a yield of 51.98%.

ESI-MS(m/z) =609.2 $[M+H]^+$.

Step d): synthesis of 4-(2-(4-aminopiperidin-1-yl)-5-(3-(benzyloxy)-4-methoxyphenyl)pyridin-4-yl)-2-fluorobenzonitrile

Tert-butyl 4-(5-(3-(benzyloxy)-4-methoxyphenyl)-4-(4-cyano-3-fluorophenyl)pyridin-2-yl)piperazine-1-carboxylate (780 mg, 1.44 mmol) was added to 4N hydrochloric acid in EA (4 mL), and the reaction was conducted at room temperature for 0.5 hours and monitored by LCMS until the starting materials were completely consumed and the product was dominant. Concentrating was conducted to remove the solvent, and the residue was treated with a saturated solution of sodium bicarbonate to adjust the pH to weakly acidic. The mixture was extracted with EA, and the organic phases were combined. After concentrating by removing the solvent, a solid crude product was obtained and was used directly for the next reaction step.

ESI-MS(m/z) =509.3 $[M+H]^+$.

Step e): synthesis of methyl (E)-3-(4-{[(1-(5-(3-(benzyloxy)-4-methoxyphenyl)-4-(4-cyano-3-fluorophenyl)pyridin-2-yl) piperidin-4-yl)amino]m ethyl}phenyl)prop-2-enoate

4-(2-(4-Aminopiperidin-1-yl)-5-(3-(benzyloxy)-4-methoxyphenyl)pyridin-4-yl)-2-fluorobenzonitrile (0.35 g, 0.68 mmol), methyl (E)-3-(4-formylphenyl)prop-2-enoate (0.16 g, 0.82 mmol), and sodium cyanoborohydride (0.085 g, 1.36 mmol) were added to 1,2-dichloroethane (10 mL), and the mixture was reacted overnight at room temperature and monitored by LCMS until the starting materials were completely consumed. Concentrating was conducted to remove the solvent, and the residue was mixed with silica gel and purified by a normal-phase column to afford methyl (E)-3-(4-{[(1-(5-(3-(benzyloxy)-4-methoxyphenyl)-4-(4-cyano-3-fluorophenyl)pyridin-2-yl)piperidin-4-yl)amino]m ethyl} phenyl)prop-2-enoate with a yield of 75.38%.

ESI-MS(m/z) =683.2 $[M+H]^+$.

Step f): synthesis of (E)-3-(4-(1-(5-(3-(benzyloxy)-4-methoxyphenyl)-4-(4-cyano-3-fluorophenyl)pyridin-2-yl)piperidin-4-amino)methyl) phenyl)acrylic acid

Methyl (E)-3-(4-{[(1-(5-(3-(benzyloxy)-4-methoxyphenyl)-4-(4-cyano-3-fluorophenyl)pyridin-2-yl)piperidin-4-yl) amino]m ethyl}phenyl)prop-2-enoate (254 mg, 0.37 mmol) and lithium hydroxide (44.31 mg, 1.85 mmol) were added to a mixture of tetrahydrofuran : water = 4:1 (5 mL). The mixture was reacted overnight at room temperature and monitored by LCMS until the starting materials were completely consumed, and the system was then directly used for the next reaction step.

ESI-MS(m/z) =669.2 $[M+H]^+$.

Step g): synthesis of (E)-3-(4-{[(1-(5-(3-(benzyloxy)-4-methoxyphenyll)-4-(4-cyano-3-fluorophenyll)pyridin-2-yll)piperidin-4-yl)[(tert-but oxy)carbonyl]amino]methyl}phenyl)prop-2-enoic acid

In the reaction system of Step f, di-tert-butyl dicarbonate (170.5 mg, 0.78 mmol) and sodium bicarbonate were added, and the mixture was stirred overnight at room temperature and monitored by LCMS until the starting materials were completely consumed. The pH was adjusted to weakly acidic with dilute hydrochloric acid, then water was added, and the mixture was extracted with EA. The organic phases were combined, and concentrating was conducted to remove the solvent. The residue was added with silica gel and purified by a normal-phase column to afford (E)-3-(4-{[(1-(5-(3-(benzyloxy)-4-methoxyphenyl)-4-(4-cyano-3-fluorophenyl)pyridin-2-yl)piperidin-4-yl)[(tert-but oxy)carbonyl]amino]methyl} phenyl)prop-2-enoic acid with a yield of 80.64%.

ESI-MS(m/z) =769.3 $[M+H]^+$.

Step h): synthesis of tert-butyl (E)-(1-(5-(3-(benzyloxy)-4-methoxyphenyl)-4-(4-cyano-3-fluorophenyl)pyridin-2-yl)piperidin-4-yl)(4-(3-oxo-3-(((t etrahydro-2H-pyran-2-yl)oxy)amino)prop-1-en-1-yl)benzyl)carbamate

(E)-3-(4-{[(1-(5-(3-(Benzyloxy)-4-methoxyphenyl)-4-(4-cyano-3-fluorophenyl)pyridin-2-yl)piperidin-4-yl)[(te rt-butoxy)carbonyl]amino]methyl}phenyl)prop-2-enoic acid (215 mg, 0.28 mmol), O-(tetrahydro-2H-pyran-2-yl)hydroxylamine (40 mg, 0.33 mmol), 2-(7-azabenzotriazolyl)-N,N,N',N'-tetramethyluronium hexafluorophosphate (128 mg, 0.33 mmol), and ethyldiisopropylamine (109 mg, 0.84 mmol) were added to DMF (8 mL). The mixture was reacted at room temperature for 0.5 hours and monitored by LCMS until the starting materials were completely consumed and the product was dominant. The reaction liquid was poured into water, and ethyl acetate was added for extraction. The organic phase was washed with saturated brine, and concentrating was conducted to remove the solvent. The residue was mixed with silica gel and purified by a normal-phase column to afford tert-butyl (E)-(1-(5-(3-(benzyloxy)-4-methoxyphenyl)-4-(4-cyano-3-fluorophenyl)pyridin-2-yl)piperidin-4-yl)(4-(3-oxo-3-(((t etrahydro-2H-pyran-2-yl)oxy)amino)prop-1-en-1-yl) benzyl)carbamate with a yield of 65.83%.

ESI-MS(m/z) =868.4 [M+H]+.

Step i): synthesis of (E)-3-(4-{[(1-(4-(4-cyano-3-fluorophenyl)-5-(3-hydroxy-4-methoxyphenyl)pyridin-2-yl)piperidin-4-yl)amino]methy l}phenyl)-N-hydroxyprop-2-enamide formate

Tert-butyl (E)-(1-(5-(3-(benzyloxy)-4-methoxyphenyl)-4-(4-cyano-3-fluorophenyl)pyridin-2-yl)piperidin-4-yl) (4-(3-oxo-3-(((t etrahydro-2H-pyran-2-yl)oxy)amino)prop-1-en-1-yl)benzyl)carbamate (120 mg, 0.14 mmol) was added to DCM (8 mL), followed by adding boron tribromide (0.28 g, 1.12 mmol) at -78°C, and the reaction was maintained under a dry ice bath for 0.5 hours and monitored by LCMS until the starting materials were completely consumed. The reaction liquid was quenched with methanol, then concentrating was conducted to remove the solvent, and the residue was purified by Prep-HPLC (separation method 3) to afford (E)-3-(4-{[(1-(4-(4-cyano-3-fluorophenyl)-5-(3-hydroxy-4-methoxyphenyl) pyridin-2-yl)piperidin-4-yl)amino]methy l}phenyl)-N-hydroxyprop-2-enamide formate with a yield of 66.18%.

$^1$H NMR (400 MHz, Methanol-$d_4$) δ8.53 (s, 1H),8.46 (s, 1H), 8.14 (s, 1H), 7.70 - 7.58 (m, 3H), 7.53 (t, *J*= 11.1 Hz, 2H), 7.22 (dd, *J* = 10.2, 1.5 Hz, 1H), 7.15 (dd, *J* = 8.1, 1.5 Hz, 1H), 6.88 - 6.80 (m, 2H), 6.53 (d, *J* = 8.0 Hz, 1H), 6.50 (dt, *J* = 4.4, 2.4 Hz, 2H), 4.52 (d, *J* = 13.6 Hz, 2H), 4.21 (s, 2H), 3.83 (s, 3H), 3.32 (s, 1H), 2.99 (t, *J*= 12.7 Hz, 2H), 2.27 - 2.14 (m, 2H), 1.65 (q, *J* = 12.1 Hz, 2H).

ESI-MS(m/z) =594.2 [M+H]+.

**Example 81**

Preparation of (E)-3-(3-((1-(3-cyano-4-(4-cyano-3-fluorophenyl)-5-(3-hydroxy-4-methoxyphenyl)pyridin-2-yl)piperidin-4-yl)amin o)phenyl)-N-hydroxyacrylamide formate

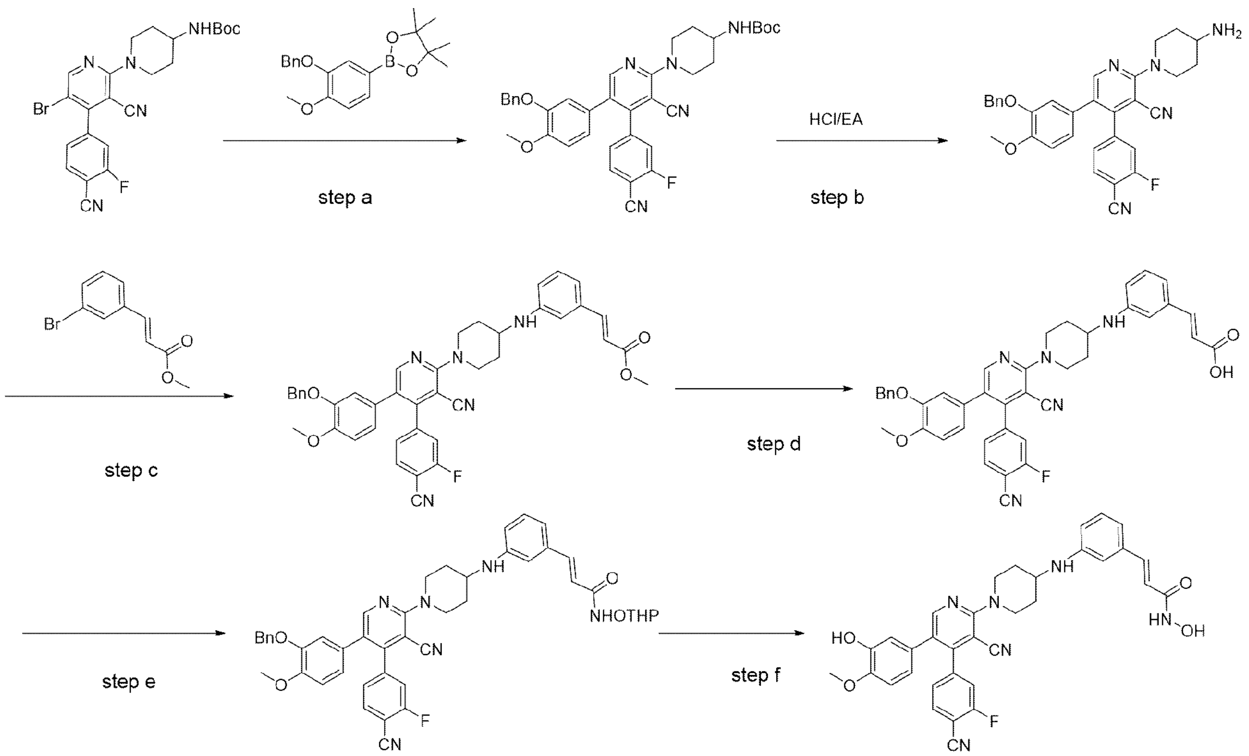

Step a): preparation oftert-butyl (1-(5-(3-(benzyloxy)-4-methoxyphenyl)-3-cyano-4-(4-cyano-3-fluorophenyl)pyridin-2-yl)piperidin-4-yl)carbamate

The product of step c in Example 51tert-butyl (1-(5-bromo-3-cyano-4-(4-cyano-3-fluorophenyl)pyridin-2-yl) piperidin-4-yl)carbamate (558.0 mg, 1.12 mmol) and 2-(3-(benzyloxy)-4-methoxyphenyl)-4,4,5,5-tetramethyl-1,3,2-dioxaborolane (280.1 mg, 1.12 mmol), $Cs_2CO_3$ (1.1 g, 3.36 mmol) and Pd(dppf)$Cl_2$ (82.0 mg, 0.11 mmol) were dissolved in 1,4-dioxane (8 mL), water (1 mL) was added, and the reaction liquid was purged with nitrogen three times and then heated to 100°C. After 2 hours, the reaction was completed as indicated by LC-MS, and the reaction liquid was cooled to room temperature. Water (9 mL) was added, and the mixture was extracted with ethyl acetate (8 mL × 3). The organic layers were combined, washed with saturated brine (8 mL), dried with anhydrous sodium sulfate, filtered, and concentrated under vacuum. The residue was purified by silica gel chromatography to afford tert-butyl (1-(5-(3-(benzyloxy)-4-methoxyphenyl)-3-cyano-4-(4-cyano-3-fluorophenyl)pyridin-2-yl)piperidin-4-yl)carbamate with a yield of 80.6%.
ESI-MS m/z=634.3 [M+H]+.

Step b): preparation of 2-(4-aminopiperidin-1-yl)-5-(3-(benzyloxy)-4-methoxyphenyl)-4-(4-cyano-3-fluorophenyl)nicotinonitrile

Tert-butyl (1-(5-(3-(benzyloxy)-4-methoxyphenyl)-3-cyano-4-(4-cyano-3-fluorophenyl)pyridin-2-yl)piperidin-4-yl)carbamate (200 mg, 0.36 mmol) and 4M hydrochloric acid solution in ethyl acetate (5 mL) were added to a reaction flask, and the mixture was stirred at room temperature for 1 hour. The mixture was concentrated to afford 2-(4-aminopiperidin-1-yl)-5-(3-(benzyloxy)-4-methoxyphenyl)-4-(4-cyano-3-fluorophenyl)nicotinonitrile with a yield of 92%.
ESI-MS m/z = 534.2 $[M+H]^+$.

Step c): preparation of methyl (E)-3-(3-((1-(5-(3-(benzyloxy)-4-methoxyphenyl)-3-cyano-4-(4-cyano-3-fluorophenyl) pyridin-2-yl))piperidin-4-yl)a mino)phenyl)acrylate

2-(4-Aminopiperidin-1-yl)-5-(3-(benzyloxy)-4-methoxyphenyl)-4-(4-cyano-3-fluorophenyl)nicotinonitrile (178 mg, 0.32 mmol) was dissolved in toluene (3 mL), to which methyl (E)-3-(3-bromophenyl)acrylate (77.2 mg, 0.32 mmol), cesium carbonate (208.6 mg, 0.64 mmol), tris(dibenzylideneacetone)dipalladium (58.6 mg, 0.06 mmol), and 2-dicyclohexylphosphino-2',6'-diisopropoxy-1,1'-biphenyl (30.5 mg, 0.06 mmol) were added, and the mixture was refluxed and heated to 100°C for reaction overnight under nitrogen protection. After the reaction was completed as indicated by LCMS, the mixture was concentrated to dryness under reduced pressure. The residue was purified by silica gel chromatography to afford methyl (E)-3-(3-((1-(5-(3-(benzyloxy)-4-methoxyphenyl)-3-cyano-4-(4-cyano-3-fluorophenyl)pyridin-2-yl))piperidin-4-yl)a mino)phenyl)acrylate with a yield of 73.3%.
ESI-MS m/z = 694.3 $[M+H]^+$.

Step d): preparation of (E)-3-(3-((1-(5-(3-(benzyloxy)-4-methoxyphenyl)-3-cyano-4-(4-cyano-3-fluorophenyl)pyridin-2-yl)piperidin-4-yl)a mino)phenyl)acrylic acid

Methyl (E)-3-(3-((1-(5-(3-(benzyloxy)-4-methoxyphenyl)-3-cyano-4-(4-cyano-3-fluorophenyl)pyridin-2-yl))piperidin-4-yl)a mino)phenyl)acrylate (135 mg, 0.20 mmol) was dissolved in a mixture of THF/$H_2$=O = 5:1 (3 mL), then lithium hydroxide (48 mg, 2 mmol) was added, and the mixture was stirred overnight at room temperature. The pH of the system was adjusted to 2-3 with 2 N HCl, and the mixture was then extracted with ethyl acetate. The organic phase was washed once with brine and subsequently concentrated under reduced pressure. The residue was used directly for the next step.
ESI-MS m/z = 680.3 $[M+H]^+$.

Step e): preparation of (E)-3-(3-((1-(5-(3-(benzyloxy)-4-methoxyphenyl)-3-cyano-4-(4-cyano-3-fluorophenyl)pyridin-2-yl)piperidin-4-yl)a mino)phenyl)-N-((tetrahydro-2H-pyran-2-yl)oxy)acrylamide

(E)-3-(3-((1-(5-(3-(benzyloxy)-4-methoxyphenyl)-3-cyano-4-(4-cyano-3-fluorophenyl)pyridin-2-yl)piperidin-4 -yl)amino)phenyl)acrylic acid (80 mg, 0.12 mmol) was dissolved in DMF (2 mL), followed by adding HATU (54.75 mg, 0.14 mmol) and O-(tetrahydro-2H-pyran-2-yl)hydroxylamine (16.9 mg, 0.14 mmol), and the reaction was conducted at room temperature for 1 hour, then quenched with the addition of water. The mixture was extracted with ethyl acetate, and the organic phase was washed twice with water, concentrated under vacuum after drying, and the residue was purified by silica gel column to afford (E)-3-(3-((1-(5-(3-(benzyloxy)-4-methoxyphenyl)-3-cyano-4-(4-cyano-3-fluorophenyl)pyridin-2-yl)piperidin-4-yl)a mino)phenyl)-N-((tetrahydro-2H-pyran-2-yl)oxy)acrylamide with a yield of 85%.
ESI-MS m/z = 779.3 $[M+H]^+$.

Step f): preparation of (E)-3-(3-((1-(3-cyano-4-(4-cyano-3-fluorophenyl)-5-(3-hydroxy-4-methoxyphenyl)pyridin-2-yl)piperidin-4-yl)amin o)phenyl)-N-hydroxyacrylamide formate

(E)-3-(3-((1-(5-(3-(Benzyloxy)-4-methoxyphenyl)-3-cyano-4-(4-cyano-3-fluorophenyl)pyridin-2-yl)piperidin-4-yl)amino)phenyl)-N-((tetrahydro-2H-pyran-2-yl)oxy)acrylamide (80 mg, 0.1 mmol) was dissolved in a solution of HCl/EA (3 mL, 2M), the mixture was reacted for 1 hour at room temperature. Solids were formed in the reaction liquid, and the reaction was completed as indicated by LC-MS detection. The reaction liquid was then filtered to obain solids, which were then prepared and purified by Prep-HPLC (separation method 3) to afford (E)-3-(3-((1-(3-cyano-4-(4-cyano-3-fluorophenyl)-5-(3-hydroxy-4-methoxyphenyl)pyridin-2-yl)piperidin-4-yl)amin o)phenyl)-N-hydroxyacrylamide formate with a yield of 55.2%.

$^1$H NMR (400 MHz, DMSO-$d_6$) δ8.52 (s, 1H), 8.40 (s, 1H), 7.95 - 7.84 (m, 1H), 7.61 (dd, *J*= 10.0, 1.5 Hz, 1H), 7.34 (d, *J* = 15.8 Hz, 1H), 7.26 (dd, *J* = 8.0, 1.6 Hz, 1H), 7.16 (t, *J* = 7.8 Hz, 1H), 6.88 - 6.78 (m, 3H), 6.76 - 6.68 (m, 1H), 6.51 - 6.43 (m, 2H), 6.38 (d, *J* = 15.8 Hz, 1H), 4.21 (d, *J* = 12.8 Hz, 2H), 3.70 (s, 3H), 3.60 (s, 1H), 3.27 (t, *J* = 12.2 Hz, 2H), 2.05 (d, *J* = 11.8 Hz, 2H), 1.53 (q, *J* = 11.4 Hz, 2H).

ESI-MS m/z = 605.2 [M+H]$^+$.

**Example 82**

(E)-3-(4-((1-(3-Cyano-4-(4-cyano-3-fluorophenyl)-5-(3-hydroxy-4-methoxyphenyl)pyridin-2-yl)piperidin-4-yl)amino)phenyl)-N-hydroxyacrylamide formate was prepared according to the synthetic method of Example 81, and the structure and characterization data are as follows:

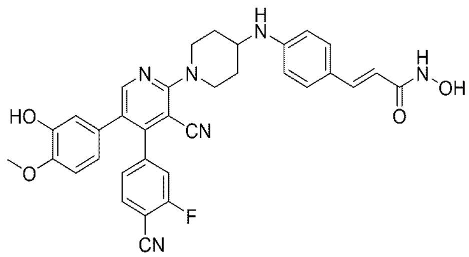

$^1$H NMR (400 MHz, DMSO- $d_6$) δ10.45 (s, 1H),8.98 (s, 1H), 8.43 (s, 1H), 7.95 (t, *J* = 7.4 Hz, 1H), 7.66 (dd, *J* = 10.0, 1.4 Hz, 1H), 7.36 - 7.23 (m, 4H), 6.81 (d, *J* = 8.0 Hz, 1H), 6.65 (d, *J* = 8.2 Hz, 2H), 6.48 (d, *J* = 8.4 Hz, 2H), 6.14 (d, *J* = 12.0 Hz, 1H), 4.22 (d, *J* = 13.2 Hz, 2H), 3.72 (s, 3H), 3.64 (s, 1H), 3.31 (t, *J* = 11.8 Hz, 2H), 2.07 (d, *J* = 11.8 Hz, 2H), 1.63 - 1.46 (m, 2H).

ESI-MS m/z = 605.2 [M+H]$^+$.

**Example 83**

2-((1-(3-Cyano-4-(4-cyano-3-fluorophenyl)-5-(3-hydroxy-4-methoxyphenyl)pyridin-2-yl)piperidin-4-yl)amino)-N-hydroxypyrimidine-5-carboxamide formate was prepared according to the synthetic method of Example 81, and the structure and characterization data are as follows:

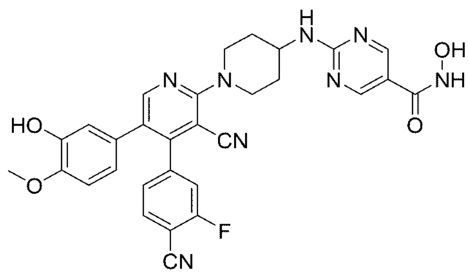

$^1$H NMR (400 MHz, Methanol-$d_4$) δ ppm 8.64 (s, 2H), 8.52 (s, 1H), 8.41 (s, 1H), 7.74 (dd, *J* = 8.0, 6.6 Hz, 1H), 7.37 (dd, *J* = 9.6, 1.6 Hz, 1H), 7.24 (dd, *J* = 8.0, 1.6 Hz, 1H), 6.87 - 6.76 (m, 1H), 6.56 - 6.46 (m, 2H), 4.35 (d,*J* = 13.4 Hz, 2H), 4.17 (td, *J* = 10.6, 5.4 Hz, 1H), 3.81 (s, 3H), 3.30 - 3.24 (m, 2H), 2.21 - 2.11 (m, 2H), 1.84 - 1.71 (m, 2H).

ESI-MS m/z=581.2 [M+H].

**Example 84**

Preparation of (E)-3-(4-[1-(2-cyano-3-(4-cyano-3-fluorophenyl)-4-(3-hydroxy-4-methoxyphenyl)phenyl)piperidin-4-yl) amino]met hyl}phenyl)-N-ylpropanohydroxy-2-enamide

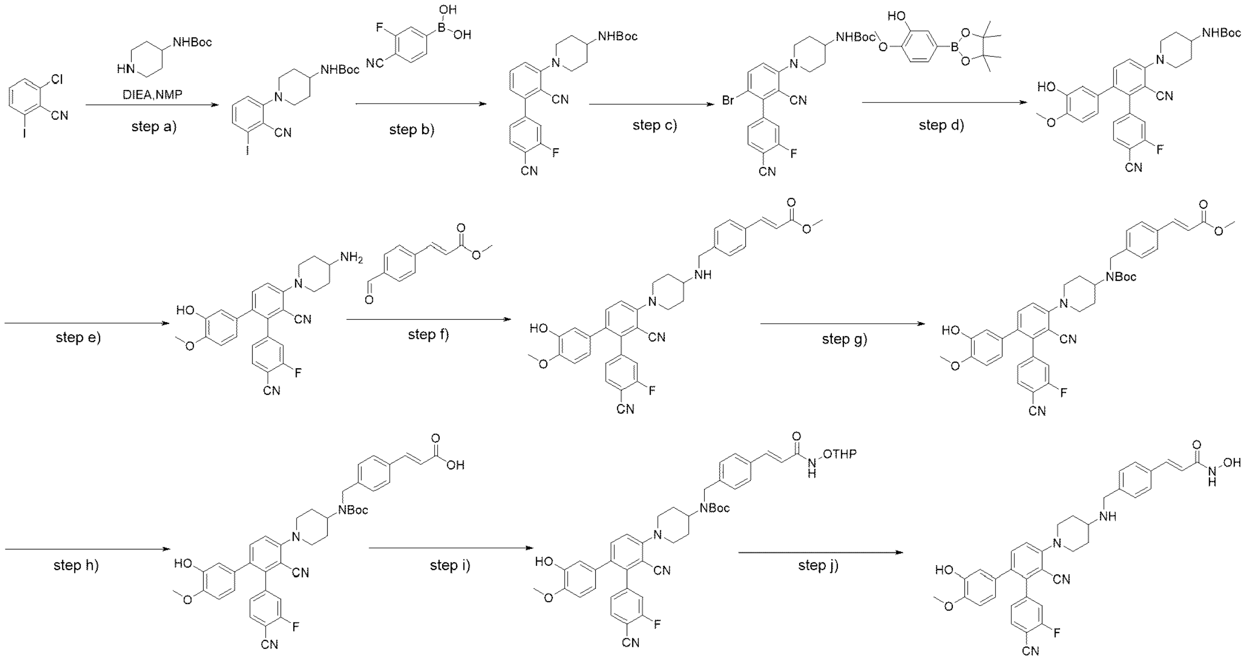


Step a): preparation of tert-butyl (1-(2-cyano-3-iodophenyl)piperidin-4-yl)carbamate

2-Chloro-6-iodobenzonitrile (2 g, 7.63 mmol), tert-butyl piperidin-4-ylcarbamate (2.29 g, 11.45 mmol), and DIEA (3.94 g, 30.52 mmol) were added to a reaction flask containing NMP, (20 mL), and the mixture was stirred at 100°C for 24 hours. The reaction liquid was quenched by adding water (20 mL), extracted with ethyl acetate (20 mL × 2), and the organic phases were combined, washed with saturated brine (15 mL × 2), dried over anhydrous sodium sulfate, filtered, and the residue was purified by silica gel chromatography (eluent: petroleum ether/ethyl acetate = 1/1) to afford tert-butyl (1-(2-cyano-3-iodophenyl)piperidin-4-yl)carbamate with a yield of 37%.
ESI-MS m/z=428.1 [M+H]+.

Step b): preparation of tert-butyl (1-(2,4'-dicyano-3'-fluoro-[1,1'-biphenyl]-3-yl)piperidin-4-yl)carbamate

Tert-butyl (1-(2-cyano-3-iodophenyl)piperidin-4-yl)carbamate (1.3 g, 3.04 mmol), 4-cyano-3-fluorophenylboronic acid (0.60 g, 3.65 mmol), $Pd_2(dppf)Cl_2$ (0.22 g, 0.30 mmol), and cesium carbonate (1.98 g, 6.08 mmol) were added to a microwave tube containing dioxane (2 mL) and water (0.4 mL). The mixture was stirred at 80°C for 45 minutes. The reaction liquid was concentrated by adding silica gel, and the residue was purified by silica gel chromatography (eluent: petroleum ether/ethyl acetate = 1/1) to afford tert-butyl (1-(2,4'-dicyano-3'-fluoro-[1,1'-biphenyl]-3-yl)piperidin-4-yl)carbamate with a yield of 66%.
ESI-MS m/z=421.2 [M+H]+.

Step c): preparation of tert-butyl (1-(6-bromo-2,4'-dicyano-3'-fluoro-[1,1'-biphenyl]-3-yl)piperidin-4-yl)carbamate

Tert-butyl (1-(2,4'-dicyano-3'-fluoro-[1,1'-biphenyl]-3-yl)piperidin-4-yl)carbamate (840 mg, 2.00 mmol) and NBS (0.42 g, 2.36 mmol) were added to a reaction flask containing DMF (10 mL), and the mixture was stirred at room temperature for 2 hours. The reaction liquid was quenched by adding water (20 mL), extracted with ethyl acetate (20 mL × 2), and the organic phases were combined, washed with saturated brine (15 mL × 2), dried over anhydrous sodium sulfate, filtered, and the residue was purified by silica gel chromatography (eluent: petroleum ether/ethyl acetate = 1/1) to afford tert-butyl (1-(6-bromo-2,4'-dicyano-3'-fluoro-[1,1'-biphenyl]-3-yl)piperidin-4-yl)carbamate with a yield of 67%.
ESI-MS m/z=499.1 [M+H]+.

Step d): preparation of tert-butyl (1-(3,4"-dicyano-3-fluoro-3-hydroxy-4-methoxy-[1,1:2,1-terphenyl]-4-yl)piperidin-4-yl) carbamate

Tert-butyl (1-(6-bromo-2,4'-dicyano-3'-fluoro-[1,1'-biphenyl]-3-yl)piperidin-4-yl)carbamate (620 mg, 1.24 mmol), 2-methoxy-5-(4,4,5,5-tetramethyl-1,3,2-dioxaborolan-2-yl)phenol (0.31 g, 1.24 mmol), $Pd_2(dppf)Cl_2$ (0.091 g, 0.12 mmol), and cesium carbonate (0.81 g, 2.48 mmol) were added to a microwave tube containing 1,4-dioxane (8 mL) and water (1.6 mL), and the mixture was subjected to microwave heating at 110°C and stirred for 45 minutes. The reaction liquid was concentrated by adding silica gel, and the residue was purified by silica gel chromatography (eluent: dichloromethane/methanol = 10/1) to afford tert-butyl (1-(3,4"-dicyano-3-fluoro-3-hydroxy-4-methoxy-[1,1:2,1-terphenyl]-4-yl)piperidin-4-yl)carbamate with a yield of 98%.
ESI-MS m/z=443.2 [M+H]+.

Step e): preparation of 6-(4-aminopiperidin-1-yl)-2-(4-cyano-3-fluorophenyl)-3-3-hydroxy-4-methoxyphenyl)benzonitrile

Tert-butyl (1-(3,4"-dicyano-3-fluoro-3-hydroxy-4-methoxy-[1,1:2,1-terphenyl]-4-yl)piperidin-4-yl)carbamate (620 mg, 1.18 mmol) was added to a reaction flask containing a 4M hydrochloric acid solution in ethyl acetate (10 mL), and the mixture was stirred at room temperature for 1 hour. Saturated sodium bicarbonate aqueous solution was added to the reaction liquid to adjust the pH to 7, followed by extraction with a mixture of DCM : MeOH = 10:1 (20 mL × 2), and the organic phases were combined and washed with saturated brine (15 mL × 2), dried with anhydrous sodium sulfate, filtered, and concentrated to afford 6-(4-aminopiperidin-1-yl)-2-(4-cyano-3-fluorophenyl)-3-(3-hydroxy-4-methoxyphenyl)benzonitrile with a yield of 16%.
ESI-MS m/z=443.2 [M+H]+.

Step f): preparation of methyl (E)-3-(4-(((1-(3',4"-dicyano-3"-fluoro-3-hydroxy-4-methoxy-[1,1':2',1'-terphenyl]-4'-yl)piperidin-4-yl)amino)met hyl)phenyl)acrylate

6-(4-Aminopiperidin-1-yl)-2-(4-cyano-3-fluorophenyl)-3-(3-hydroxy-4-methoxyphenyl)benzonitrile (240 mg, 0.54 mmol) and methyl (E)-3-(4-formylphenyl)acrylate (0.30 g, 1.60 mmol) were added to a reaction flask containing DCE (5 mL), and the mixture was stirred at room temperature for 1 hour, then the reaction system was cooled to 0°C, and sodium cyanoborohydride (0.17 g, 2.71 mmol) was added, and the mixture was stirred at room temperature for 16 hours. The reaction liquid was quenched by adding saturated sodium bicarbonate aqueous solution (5 mL) to the reaction liquid, followed by extraction with ethyl acetate (20 mL × 2), and the organic phases were combined and washed with saturated brine (15 mL × 2), dried with anhydrous sodium sulfate, filtered, and concentrated. The residue was purified by silica gel chromatography (eluent: petroleum ether/ethyl acetate = 1/1) to afford methyl (E)-3-(4-(((1-(3',4"-dicyano-3"-fluoro-3-hydroxy-4-methoxy-[1,1':2',1'-terphenyl]-4'-yl)piperidin-4-yl)amino)methyl) phenyl)acrylate with a yield of 63%.
ESI-MS m/z=617.2 [M+H]+.

Step g): preparation of (E)-3-(4-tert-butoxycarbonylmethyl(1-(3,4"-dicyano-3-fluoro-3-hydroxy-4-methoxy-111 '2',1 "-terphenyl]-4" -yl)piperidin-4-yl)amino)methyl)phenyl)acrylates

4-[(1-dicyanomethylene-3'-dicyano-3"-fluoro-3-hydroxy-4-methoxy-1,1':2',1"-terphenyl]-4'-yl)piperidin-4-yl)a minomethyl)phenyl)acrylate (150 mg, 0.24 mmol), di-tert-butyl dicarbonate (0.27 g, 1.22 mmol), and TEA (0.097 g, 0.96 mmol) were added to a reaction flask containing dichloromethane (4 mL), and the mixture was stirred at room temperature for 1 hour. The reaction liquid was concentrated to afford (E)-3-(4-(tert-butoxycarbonylmethyl(1-(3,4"-dicyano-3-fluoro-3-hydroxy-4-methoxy-111,1'2',1'-terphenyl)-4"-yl)pip eridin-4-yl)amino)methyl)phenyl)acrylate with a yield of 93%.
ESI-MS m/z=717.3 [M+H]+.

Step h): preparation of (E)-3-(4-(tert-butoxycarbonyl)(1-(3',4"-dicyano-3"-fluoro-3-hydroxy-4-methoxy-[1,1:2',1"-terphenyl]-4'-yl)piperi din-4-yl)amino)methyl)phenyl)acrylic acid

4-(tert-butoxycarbonylmethyl(1-(3,4"-dicyano-3-fluoro-3-hydroxy-4-methoxy-[111,1':2',1"-terphenyl]-4"-yl)pip eridin-4-yl)amino)methyl)phenyl)acrylate (160 mg, 0.22 mmol) and lithium hydroxide (0.11 g, 4.42 mmol) were added to a reaction flask containing a mixture of THF (3 mL), MeOH(1.8 mL), and water (1.2 mL), and the mixture was stirred at room temperature for 1 hour. To the reaction liquid, 2M HCl solution was added dropwise to adjust the pH to 4, followed by extraction with dichloromethane (20 mL × 2), and the organic phases were combined and washed with saturated brine (15 mL × 2), dried with anhydrous sodium sulfate, filtered, and concentrated to afford (E)-3-(4-(tert-butoxycarbonyl)(1-(3',4"-dicyano-3"-fluoro-3-hydroxy-4-methoxy-[1,1:2',1"-terphenyl]-4'-yl)piperidin -4-yl)amino)methyl)phenyl)acrylic acid with a

yield of 91%.

ESI-MS m/z=703.2 [M+H]+.

Step i): preparation of tert-butyl (E)-(1-(3',4"-dicyano-3"-fluoro-3-hydroxy-4-methoxy-[1,1':2',1"-terphenyl]-4'-yl)piperidin-4-yl)(4-(3-oxo-3-(((te trahydro-2H-pyran-2-yl)oxy)amino)prop-1-en-1-yl)benzyl)carbamate

3-(Tert-butoxycarbonyl)(1-(3,4-dicyano-3-fluoro-3-hydroxy-4-methoxy-[1,1':2',1"-terphenyl]-4-yl)piperidin-4-yl)amino)methyl)phenyl)acrylate (140 mg, 0.20 mmol), HATU (0.11 g, 0.30 mmol), DIEA (0.10 g, 0.80 mmol), and O-(tetrahydro-2H-pyran-2-yl)hydroxylamine (0.047 g, 0.40 mmol) were added to a reaction flask containing DMF (5 mL), and the mixture was stirred at room temperature for 1 hour. Water (20 mL) was added to quench the reaction, followed by extraction with ethyl acetate (20 mL × 2). The organic phases were combined and washed with saturated brine (15 mL × 2), dried with anhydrous sodium sulfate, filtered, and the residue was purified by silica gel chromatography (eluent: DCM/MeOH = 10/1) to afford tert-butyl (E)-(1-(3',4"-dicyano-3"-fluoro-3-hydroxy-4-methoxy-[1,1';2',1"-terphenyl]-4'-yl) piperidin-4-yl)(4-(3-oxo-3-(((tetrah ydro-2H-pyran-2-yl)oxy)amino)prop-1-en-1-yl)benzyl)carbamate with a yield of 93%.

ESI-MS m/z=802.3 [M+H]+.

Step j): preparation of (E)-3-(4-[1-(2-cyano-3-(4-cyano-3-fluorophenyl)-4-(3-hydroxy-4-methoxyphenyl)phenyl)piperidin-4-yl)amino]met hyl}phenyl)-N-ylpropanohydroxy-2-enamide

Tert-butyl (E)-(1-(3',4"-dicyano-3"-fluoro-3-hydroxy-4-methoxy-[1,1':2',1"-terphenyl]-4'-yl)piperidin-4-yl)(4-(3-oxo-3-(((tetrah ydro-2H-pyran-2-yl)oxy)amino)prop-1-en-1-yl)benzyl)carbamate (140 mg, 0.17 mmol) and 4M hydrochloric acid solution in ethyl acetate (12.00 g, 329.13 mmol) were added to a reaction flask, and the mixture was stirred at room temperature for 1 hour. After concentrating, the reaction liquid was purified by Prep-HPLC (separation method Method 3), then lyophilized to afford (E)-3-(4-[1-(2-cyano-3-(4-cyano-3-fluorophenyl)-4-(3-hydroxy-4-methoxyphenyl) phenyl)piperidin-4-yl)amino]met hyl)phenyl)-N-hydroxyacrylamide formate with a yield of 45%.

$^1$H NMR (400 MHz, DMSO-d6) δppm 10.72 (s, 1H), 8.97 (s, 1H),8.51 (s, 1H), 8.19 (s, 1H), 7.89 (t, J = 7.4 Hz, 1H), 7.63 - 7.49 (m, 4H), 7.49 - 7.34 (m, 3H), 7.27 (d, J = 8.6 Hz, 1H), 7.21 (dd, J = 8.0, 1.4 Hz, 1H), 6.77 (d, J = 8.3 Hz, 1H), 6.49 - 6.34 (m, 3H), 3.85 (s, 2H), 3.70 (s, 3H), 3.55 (s, 2H), 2.90 (t, J = 11.3 Hz, 2H), 2.73 - 2.63 (m, 1H), 2.06 - 1.95 (m, 2H), 1.54 (dq, J = 106.2, 9.9 Hz, 2H).

ESI-MS m/z=618.2 [M+H]+.

**Example 85**

Preparation of (E)-3-(4-(4-amino-1-(3-cyano-4-(4-cyano-3-fluorophenyl)-5-(3-hydroxy-4-methoxyphenyl)pyridin-2-yl)) piperidin-4 -yl)phenyl)-N-hydroxyacrylamide formate

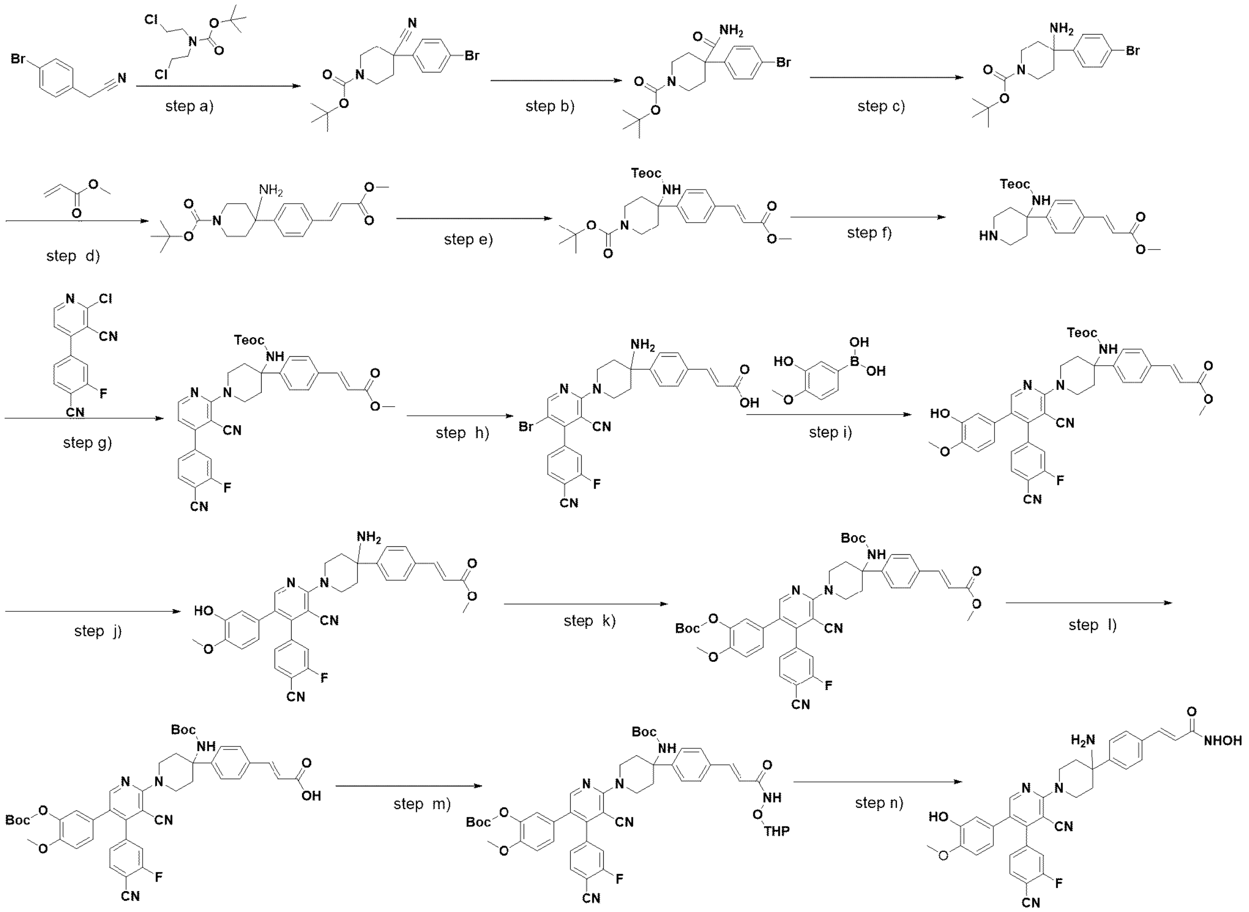


Step a) synthesis of tert-butyl 4-(4-bromophenyl)-4-cyanopiperidine-1-carboxylate

2-(4-Bromophenyl)acetonitrile (5 g, 25.50 mmol) and tert-butyl N,N-bis(2-chloroethyl)carbamate (7.10 g, 29.32 mmol) were added to DMF (75 mL), then sodium hydride (1.84 g, 76.5 mmol) was added portion-wise at 0°C, and after the addition was completed, the temperature was raised to 65°C and the reaction was conducted for 1.5 hours. After cooling to room temperature, the reaction was continued for another hour, and the reaction was monitored by TLC (petroleum ether: ethyl acetate = 3:1). Upon the disappearance of the starting material, the reaction liquid was quenched with water, extracted with EA, and the organic phases were combined, concentrated to remove the solvent, and purified by a normal-phase column to afford tert-butyl 4-(4-bromophenyl)-4-cyanopiperidine-1-carboxylate with a yield of 83.74%.

ESI-MS(m/z) =365.1 $[M+H]^+$.

Step b) synthesis of tert-butyl 4-(4-bromophenyl)-4-carbamoylpiperidine-1-carboxylate

Tert-butyl 4-(4-bromophenyl)-4-cyanopiperidine-1-carboxylate (5 g, 13.7 mmol), potassium hydroxide (1537.4 mg, 27.4 mmol) were added to DMSO (5 mL), then hydrogen peroxide (465.9 mg, 13.7 mmol) was added slowly, and the reaction was conducted at room temperature for 1 h, and the disappearance of the starting material was monitored by TLC (petroleum ether:ethyl acetate=3:1). The reaction liquid was quenched by adding water, extracted by EA, the organic phases were combined, concentrated to remove the solvent, and after the solvent was pumped off by an oil pump, it was used directly for the next reaction step.

ESI-MS(m/z) =383.1 $[M+H]^+$.

Step c) synthesis of tert-butyl 4-amino-4-(4-bromophenyl)piperidine-1-carboxylate

Potassium hydroxide (1641.2 mg, 29.3 mmol) was dissolved in a mixture of ACN:$H_2O$=1:4 (40 mL), and the crude product of Step b, tert-butyl 4-(4-bromophenyl)-4-carbamoylpiperidine-1-carboxylate (2.5 g, 6.5 mmol), was added and stirred for 10 minutes, then 1,3-dibromo-5,5-dimethylhydantoin (1022.2 mg, 3.6 mmol) was added portion-wise, and the mixture was reacted at room temperature for 1 hour and monitored by TLC (ethyl acetate as the developing agent). After the disappearance of the starting material, a more polar spot (Rf: 0.15) (ninhydrin colour-development) appeared. The

system was added with sodium sulfite (0.082 g, 0.65 mmol) and stirred for 15 minutes. After the addition of potassium phosphate (1.7 g, 7.9 mmol), the mixture was extracted with ethyl acetate, and the organic phases were combined, washed with a brine, and concentrated to remove the solvent. The residue was mixed with silica gel and purified by a normal-phase column to afford tert-butyl 4-amino-4-(4-bromophenyl)piperidine-1-carboxylate with a yield of 77.95%.

ESI-MS(m/z) =355.1 $[M+H]^+$.

Step d) synthesis of tert-butyl (E)-4-amino-4-(4-(3-methoxy-3-oxoprop-1-en-1-yl)phenyl)piperidine-1-carboxylate

Tert-butyl 4-amino-4-(4-bromophenyl)piperidine-1-carboxylate (1400 mg, 3.9 mmol), methyl 2-acrylic acid (430.5 mg, 5.0 mmol), tetrakis(triphenylphosphine)palladium (115.6 mg, 0.10 mmol), and triethylamine (202.4 mg, 2.0 mmol) were added to a mixture of tetrahydrofuran : water =5:1 (5 mL). The reaction liquid was heated to 110°C and reacted for 4 hours and monitored by LCMS until the starting materials were completely consumed and the product was dominant. The reaction liquid was quenched with water, extracted with ethyl acetate, and the organic phases were combined, concentrated to remove the solvent, and the residue was mixed with silica gel and purified by a normal-phase column to afford tert-butyl (E)-4-amino-4-(4-(3-methoxy-3-oxoprop-1-en-1-yl)phenyl)piperidine-1-carboxylate with a yield of 82.13%.

ESI-MS(m/z) =361.1 $[M+H]^+$.

Step e) synthesis of tert-butyl (E)-4-(4-(3-methoxy-3-oxoprop-1-en-1-yl)phenyl)-4-(((trimethylsilyl)methoxy)carbonyl) amino)piperidine-1-carbox ylate

Tert-butyl (E)-4-amino-4-(4-(3-methoxy-3-oxoprop-1-en-1-yl)phenyl)piperidine-1-carboxylate (500 mg, 0.80 mmol), 2,5-dioxopyrrolidin-1-yl 2-(trimethylsilyl) ethyl carbonate (1415.9 mg, 5.5 mmol) and sodium bicarbonate (982.9 mg, 11.7 mmol) were added to DMF (40 mL). The mixture was reacted at room temperature overnight, and the reaction was monitored by TLC, which showed the disappearance of the starting material and the appearance of a new spot. The residue was mixed with silica gel, and the mixture was extracted with ethyl acetate. The organic phases were combined, and concentrated to remove the solvent. The residue was mixed with silica gel and purified by a normal-phase column to afford tert-butyl (E)-4-(4-(3-methoxy-3-oxoprop-1-en-1-yl)phenyl)-4-(((trimethylsilyl)methoxy)carbonyl)amino) piperidine-1-carbox ylate with a yield of 73.22%.

ESI-MS(m/z) =491.2 $[M+H]^+$.

Step f) synthesis of methyl (E)-3-(4-(4-(((trimethylsilyl)methoxy)carbonyl)amino)piperidin-4-yl)phenyl)acrylate

Tert-butyl (E)-4-(4-(3-methoxy-3-oxoprop-1-en-1-yl)phenyl)-4-((trimethylsilyl)methoxy)carbonyl)amino)piperidine-1-carboxy late (180 mg, 0.37 mmol) was added to 4N hydrochloric acid solution in ethyl acetate(5 mL), and the mixture was reacted at room temperature for 0.5 hours. Concentrating was conducted to remove the solvent, and sodium bicarbonate aqueous solution was then added to adjust the pH of the system to weakly alkaline. The mixture was extracted with EA, and the organic phases were combined, concentrated to remove the solvent to afford the crude product methyl (E)-3-(4-(4-((trimethylsilyl)methoxy)carbonylamino)piperidin-4-yl)phenyl)acrylate with a yield of 83.04%.

ESI-MS(m/z) =391.2 $[M+H]^+$.

Step g) synthesis of methyl (E)-3-(4-(1-(3-cyano-4-(4-cyano-3-fluorophenyl)pyridin-2-yl)-4-((2-(trimethylsilyl)ethoxy) carbonyl)amino)piperidi n-4-yl)phenyl)acrylate

methyl (E)-3-(4-(4-(((2-(trimethylsilyl)ethoxy)carbonyl)amino)piperidin-4-yl)phenyl)prop-2-enonate (100 mg, 0.24 mmol), 2-chloro-4-(4-cyano-3-fluorophenyl)nicotinonitrile (86.86 mg, 0.34 mmol), and ethyl bis(2-(prop-2-yl)amine (62.04 mg, 0.48 mmol) were added to N-methyl-2-pyrrolidone (4 mL), and the reaction liquid was heated to 110°C and reacted overnight, and purified by a normal-phase column to afford the product methyl (E)-3-(4-(1-(3-cyano-4-(4-cyano-3-fluorophenyl)pyridin-2-yl)-4-((2-(trimethylsilyl)ethoxy)carbonyl)amino)piperidi n-4-yl)phenyl)acrylate.

ESI-MS(m/z) =612.2 $[M+H]^+$.

Step h) synthesis of methyl (E)-3-(4-(4-amino-1-(5-bromo-3-cyano-4-(4-cyano-3-fluorophenyl)pyridin-2-yl)-4-{(2-(trimethylsilyl)ethoxy)carbo nyl]amino}piperidin-4-yl)phenyl)acrylate

Methyl (E)-3-(4-(1-(3-cyano-4-(4-cyano-3-fluorophenyl)pyridin-2-yl)-4-{[(2-(trimethylsilyl)ethoxy)carbonyl]amino}piperi din-4-yl)phenyl)acrylate (100 mg, 0.160 mmol) and N-bromosuccinimide (34 mg, 0.2 mmol) were added to DMF (2 mL), the reaction was conducted at room temperature for 2 hours, and quenched with water, followed by extraction with ethyl acetate. The organic phases were combined, and concentrating was conducted to remove the solvent. The residue

was mixed with silica gel and purified by a normal-phase column to afford the productmethyl (E)-3-(4-(4-amino-1-(5-bromo-3-cyano-4-(4-cyano-3-fluorophenyl)pyridin-2-yl)-4-{(2-(trimethylsilyl)ethoxy)carbo nyl}amino)piperidin-4-yl)phenyl)acrylate with a yield of 49.67%.

ESI-MS(m/z) =690.2 $[M+H]^+$.

Step i) synthesis of methyl (E)-3-(4-(1-(3-cyano-4-(4-cyano-3-fluorophenyl)-5-(3-hydroxy-4-methoxyphenyl)pyridin-2-yl)-4-{(2-(trimethylsily l)ethyoxy)carbonyl)amino)piperidin-4-yl)phenyl)acrylate

methyl (E)-3-(4-(4-amino-1-(5-bromo-3-cyano-4-(4-cyano-3-fluorophenyl)pyridin-2-yl)-4-{(2-(trimethylsilyl) ethoxy)carbo nyl}amino)piperidin-4-yl)phenyl)acrylate (49.25 mg, 0.071 mmol), 2-methoxy-5-(tetramethyl-1,3,2-dioxaborolan-2-yl)phenol (23.08 mg, 0.092 mmol), [1,1'-bis(diphenylphosphino)ferrocene]palladium dichloride (10.39 mg, 0.014 mmol), and cesium carbonate (41.64 mg, 0.13 mmol) were added to a mixture of dioxane : water =5:1 (3 mL), and the mixture was subjected to microwave heating at 105°C and reacted for 1 hour. The reaction was monitored by LCMS until the starting materials were completely consumed and the product was dominant, and concentrating was conducted to remove the solvent. The residue was mixed with silica gel and purified by a normal-phase column to afford the product methyl (E)-3-(4-(1-(3-cyano-4-(4-cyano-3-fluorophenyl)-5-(3-hydroxy-4-methoxyphenyl)pyridin-2-yl)-4-{(2-(trimethylsily l)ethoxy)carbonyl]amino}piperidin-4-yl)phenyl)acrylate with a yield of 72.63%.

ESI-MS(m/z) =734.2 $[M+H]^+$.

Step j) synthesis of methyl (E)-3-(4-(4-amino-1-(3-cyano-4-(4-cyano-3-fluorophenyl)-5-(3-hydroxy-4-methoxyphenyl) pyridin-2-yl)piperidin-4-yl)phenyl)acrylate

Methyl (E)-3-(4-(1-(3-cyano-4-(4-cyano-3-fluorophenyl)-5-(3-hydroxy-4-methoxyphenyl)pyridin-2-yl)-4-{(2-(trimethylsily l)ethoxy)carbonyl)amino)piperidin-4-yl)phenyl)acrylate (30 mg, 0.041 mmol), tetrabutylammonium fluoride (107.20 mg, 0.41 mmol) were added to DCM (5 mL), then water was added, and the mixture was extracted with ethyl acetate. The organic phases were combined, and concentrating was conducted to remove the solvent. The residue was purified by a reverse-phase column chromatography, followed by purification by a silica gel column to afford methyl (E)-3-(4-(4-amino-1-(3-cyano-4-(4-cyano-3-fluorophenyl)-5-(3-hydroxy-4-methoxyphenyl)pyridin-2-yl)piperidin-4-yl) phenyl)acrylate with a yield of 72.63%.

ESI-MS(m/z) =604.2$[M+H]^+$.

Step k) synthesis of methyl (E)-3-(4-(4-{[(tert-butoxy)carbonyl)amino)-1-(5-(3-{[(tert-butoxy)carbonyl]oxy}-4-methoxyphenyl)-3-cyano-4-(4-c yano-3-fluorophenyl)pyridin-2-yl)piperidin-4-yl)phenyl)acrylate

Methyl (E)-3-(4-(4-amino-1-(3-cyano-4-(4-cyano-3-fluorophenyl)-5-(3-hydroxy-4-methoxyphenyl)pyridin-2-yl) piperidin-4-yl)phenyl)acrylate (20 mg, 0.33 mmol), di-tert-butyl dicarbonate (360 mg, 1.7 mmol) and sodium bicarbonate (28.53 mg, 0.33 mmol) were added to a mixture of THF:$H_2O$=4:1 (10 mL). The reaction liquid was reacted at room temperature overnight, and concentrating was conducted to remove the solvent under reduced pressure to afford the crude product methyl (E)-3-(4-(4-{[(tert-butoxy)carbonyl]amino}--1-(5-(3-{[(tert-butoxy)carbonyl]oxy}--4-methoxyphenyl)-3-cyano-4-(4 -cyano-3-fluorophenyl)pyridin-2-yl)piperidin-4-yl)phenyl)acrylate with a yield of 79.06%.

ESI-MS(m/z) =804.3 $[M+H]^+$.

Step 1) synthesis of (E)-3-(4-(4-{[(tert-butoxy)carbonyl]amino}-(5-(3-{[(tert-butoxy)carbonyl)oxy)-4-methoxyphenyl)-3-cyano-4-(4-c yano-3-fluorophenyl)pyridin-2-yl)piperidin-4-yl)phenyl)acrylic acid

Methyl (E)-3-(4-(4-{[(tert-butoxy)carbonyl]amino}-1-(5-(3-{[(tert-butoxy)carbonyl)oxy}-4-methoxyphenyl)-3-cyano-4-(4-cyano-3-fluorophenyl)pyridin-2-yl)piperidin-4-yl)phenyl)acrylate (19 mg, 0.02 mmol), lithium hydroxide (3 mg, 0.1 mmol) were added to a mixture of THF:$H_2O$=4:1 (10 mL), and the reaction liquid was reacted at room temperature overnight. The pH of the mixture was adjusted to weakly acidic with dilute hydrochloric acid, and the mixture was extracted with ethyl acetate. The organic phases were combined, concentrated to remove the solvent to afford the crude product, (E)-3-(4-(4-{[(tert-butoxy)carbonyl]amino}-1-(5-(3-{[(tert-butoxycarbonyl)oxy)-4-methoxyphenyl)-3-cyano-4-(4-c yano-3-fluorophenyl)pyridin-2-yl)piperidin-4-yl)phenyl)acrylic acid with a yield of 80.25%.

ESI-MS(m/z) =790.3 $[M+H]^+$.

Step m) synthesis of tert-butyl (E)-(1-(5-(3-((tert-butoxycarbonyl)oxy)-4-methoxyphenyl)-3-cyano-4-(4-cyano-3-fluorophenyl)pyridin-2-yl))-4-(4-( 3-oxo-3-(((tetrahydro-2H-pyran-2-yl)oxy)amino)prop-1-en-1-yl)phenyl)piperidin-4-yl)carbamate

(E)-3-(4-(4-{[(Tert-butoxy)carbonyl]amino}-1-(5-(3-{[(tert-butoxy)carbonyl]oxy}-4-methoxyphenyl)-3-cyano-4-(4-cyano-3-fluorophenyl)pyridin-2-yl)piperidin-4-yl)phenyl)acrylic acid (15 mg, 0.02 mmol), O-(tetrahydro-2H-pyran-2-yl)hydroxylamine (3 mg, 0.02 mmol), 2-(7-azabenzotriazolyl)-N,N,N',N'-tetramethyluronium hexafluorophosphate (HATU, 10.2 mg, 0.03 mmol), and diisopropylethylamine (90 mg, 0.7 mmol) were added to DMF (8 mL), and the mixture was stirred at room temperature for 0.5 hours and monitored by LCMS until the starting materials were completely consumed and the product was dominant. The reaction was terminated and the system was poured into water, and extracted by adding EA. The organic phase was washed with saturated brine, and concentrated to remove the solvent. The residue was mixed with silica gel and purified by a normal-phase column to afford the product tert-butyl (E)-(1-(5-(3-((tert-butoxycarbonyl)oxy)-4-methoxyphenyl)-3-cyano-4-(4-cyano-3-fluorophenyl)pyridin-2-yl))-4-(4-( 3-oxo-3-(((tetrahydro-2H-pyran-2-yl)oxy)amino)prop-1-en-1-yl)phenyl)piperidin-4-yl)carbamate with a yield of 81.49%.

ESI-MS(m/z) =889.5 $[M+H]^+$.

Step n) synthesis of (E)-3-(4-(4-amino-1-(3-cyano-4-(4-cyano-3-fluorophenyl)-5-(3-hydroxy-4-methoxyphenyl)pyridin-2-yl))piperidin-4 -yl)phenyl)-N-hydroxyacrylamide formate

Tert-butyl (E)-(1-(5-(3-((tert-butoxycarbonyl)oxy)-4-methoxyphenyl)-3-cyano-4-(4-cyano-3-fluorophenyl)pyridin-2-yl))-4-(4-( 3-oxo-3-(((tetrahydro-2H-pyran-2-yl)oxy)amino)prop-1-en-1-yl)phenyl)piperidin-4-yl)carbamate (15 mg, 0.01 mmol) was added to a solution of 4N hydrochloric acid solution in ethyl acetate (4 mL), and the mixture was reacted at room temperature for 0.5 hours, then concentrated to remove the solvent. The residue was sent for preparative purification to afford (E)-3-(4-(4-amino-1-(3-cyano-4-(4-cyano-3-fluorophenyl)-5-(3-hydroxy-4-methoxyphenyl)pyridin-2-yl))piperidin-4 -yl)phenyl)-N-hydroxyacrylamide with a yield of 65.61%.

$^1$H NMR (400 MHz, DMSO-$d_6$) δ 10.73 (s, 1H), 9.00 (s, 1H), 8.52 (s, 1H), 8.43 (s, 1H), 8.18 (s, 1H), 7.96 (t, *J* = 7.4 Hz, 1H), 7.67 (dd, *J* = 10.1, 1.4 Hz, 1H), 7.61 (d, *J* = 8.3 Hz, 2H), 7.58 - 7.51 (m, 2H), 7.45 (d, *J* = 15.8 Hz, 1H), 7.28 (dd, *J* = 8.0, 1.4 Hz, 1H), 6.81 (d, *J* = 8.1 Hz, 1H), 6.52 - 6.39 (m, 3H), 4.08 - 3.95 (m, 2H), 3.75 (d,*J* = 12.0 Hz, 2H), 3.71 (s, 3H), 3.44 (q, *J* = 6.9 Hz, 2H), 2.22 - 2.05 (m, 2H), 1.82 (d, *J* = 13.1 Hz, 2H).

ESI-MS(m/z) =605.2 $[M+H]^+$.

**Example 86** was prepared according to the synthetic method of Example 85 ( separation method 3), and the structure and characterization data are as follows:

preparation of (E)-3-(4-(2-(4-amino-1-(3-cyano-4-(4-cyano-3-fluorophenyl)-5-(3-hydroxy-4-methoxyphenyl)pyridin-2-yl) piperidin -4-yl)ethyl)phenyl)-N-hydroxyacrylamide formate

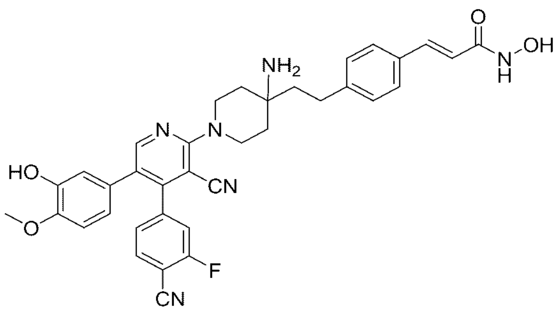

$^1$H NMR (400 MHz, DMSO-$d_6$) δ 10.67 (s, 1H), 9.01 (s, 1H), 8.44 (s, 1H), 8.20 (s, 1H), 7.96 (dd, *J* = 8.0, 6.9 Hz, 1H), 7.67 (dd, *J* = 10.0, 1.4 Hz, 1H), 7.49 (d, *J* = 7.8 Hz, 2H), 7.42 (d, *J* = 15.8 Hz, 1H), 7.34 - 7.24 (m, 3H), 6.85 - 6.76 (m, 1H), 6.53 - 6.37 (m,3H), 3.78 (d, *J* = 5.2 Hz, 4H), 3.72 (s, 3H), 2.77 - 2.65 (m, 2H), 1.93 - 1.71 (m, 6H).

ESI-MS(m/z) =633.3 $[M+H]^+$.

**Example 88**

Preparation of (E)-3-(4-((1-(4-(4-cyano-3-fluorophenyl)-5-(3-hydroxy-4-methoxyphenyl)-3-methoxypyridin-2-yl)piperidin-4-yl)a mino)methyl)phenyl)-N-hydroxyacrylamide

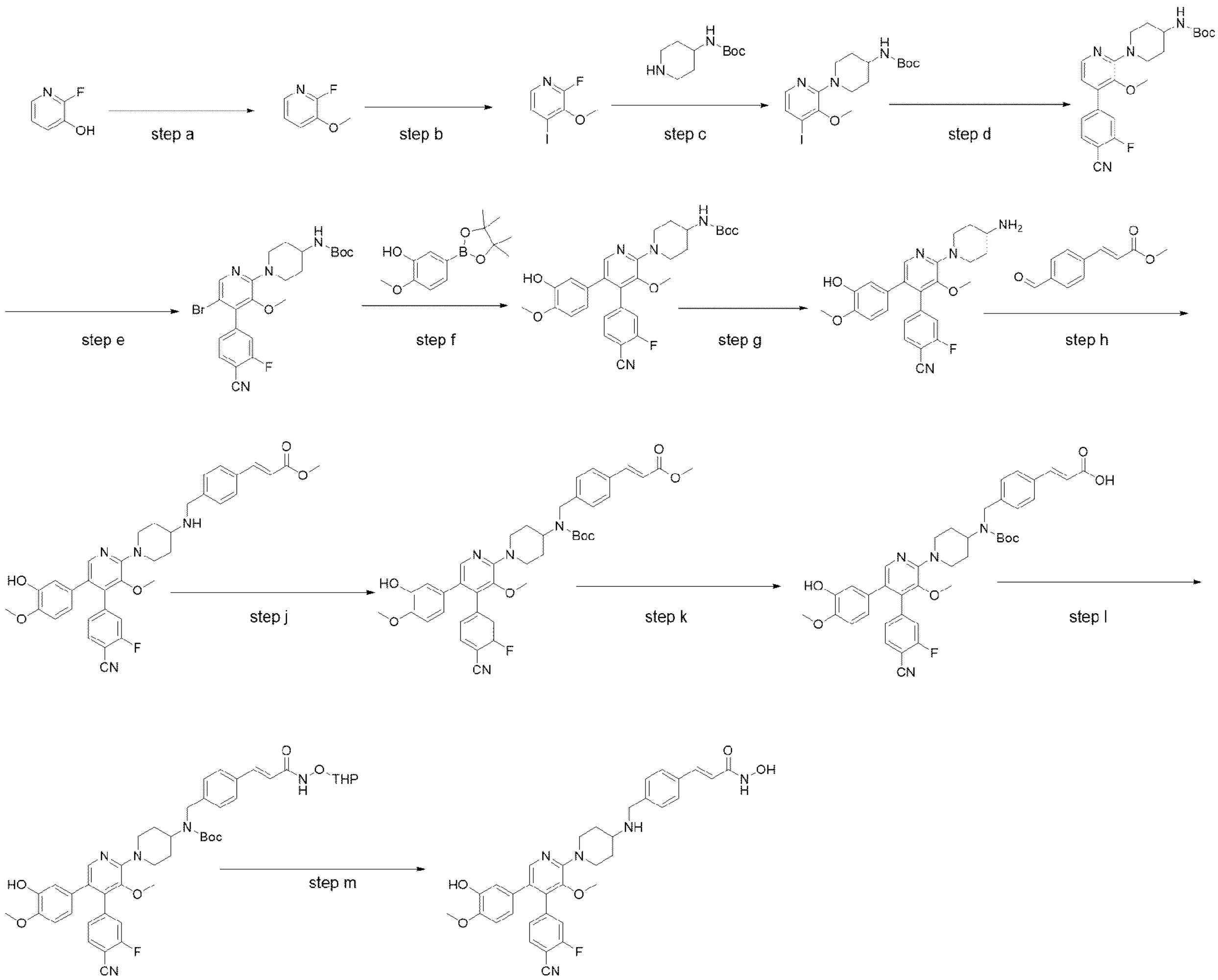


Step a): preparation of 2-fluoro-3-methoxypyridine

2-Fluoropyridin-3-ol (3.0 g, 26.5 mmol) and potassium carbonate (11.0 g, 79.6 mmol) were dissolved in DMF (30 mL), and methyl iodide (7.6 g, 53.1 mmol) was slowly added to the solution. After the addition was completed, the mixture was stirred at room temperature for 6 hours. Water (200 mL) was added, and the mixture was extracted with ethyl acetate (50 mL × 3). The organic layer was washed with saturated brine (100 mL), dried over anhydrous sodium sulfate, filtered, and concentrated under vacuum. The residue was purified by silica gel chromatography (eluent: petroleum ether/ethyl acetate = 5:1) to afford 2-fluoro-3-methoxypyridine with a yield of 75.5%.

ESI-MS m/z = 128.1 $[M+H]^+$.

Step b): preparation of 2-fluoro-4-iodo-3-methoxypyridine

2-Fluoro-3-methoxypyridine (1.2 g, 8.4 mmol) was dissolved in ultradry tetrahydrofuran (50 mL), and the reaction liquid was purged with nitrogen three times, and cooled to -78°C. n-Butyllithium (810 mg, 12.7 mmol) was slowly added to the reaction liquid, which was then stirred at -78°C for 30 minutes. Iodine elemental substance (3.2 g, 12.5 mmol) was dissolved in ultradry THF (20 mL) and slowly added to the reaction liquid. After the addition was completed, the reaction was conducted at room temperature for 1 hour. Saturated ammonium chloride solution (100 mL) was added, and the mixture was extracted with ethyl acetate (30 mL × 3). The organic layers were combined, washed with brine (20 mL), dried over anhydrous sodium sulfate, filtered, and concentrated under vacuum. The residue was purified by silica gel chromatography (eluent: petroleum ether/ethyl acetate = 5/1) to afford 2-fluoro-4-iodo-3-methoxypyridine with a yield of 51.4%.

ESI-MS m/z = 254.1 $[M+H]^+$.

Step c): preparation of tert-butyl (1-(4-iodo-3-methoxypyridin-2-yl)piperidin-4-yl)carbamate

2-Fluoro-4-iodo-3-methoxypyridine (1.1 g, 4.4 mmol) and 4-tert-butoxycarbonylpiperazine (1.3 g, 6.5 mmol) were dissolved in DMF (20 mL), then N-methylmorpholine (2.2 g, 21.8 mmol) was added, and the reaction liquid was heated to 80°C and stirred overnight. Water (100 mL) was added, and the mixture was extracted with ethyl acetate (40 mL × 3). The organic layers were combined, washed with brine (20 mL), dried over anhydrous sodium sulfate, filtered, and concentrated under vacuum. The residue was purified by silica gel chromatography (eluent: petroleum ether/ethyl acetate = 6:1) to afford tert-butyl (1-(4-iodo-3-methoxypyridin-2-yl)piperidin-4-yl)carbamate with a yield of 48.8%.
ESI-MS m/z = 434.0 $[M+H]^+$.

Step d): preparation of tert-butyl (1-(4-(4-cyano-3-fluorophenyl)-3-methoxypyridin-2-yl)piperidin-4-yl)carbamate

Tert-butyl (1-(4-iodo-3-methoxypyridin-2-yl)piperidin-4-yl)carbamate (1.0 g, 2.3 mmol), 4-cyano-3-fluorophenylboronic acid (400 mg, 2.4 mmol), sodium carbonate (730 mg, 6.9 mmol), and [1,1'-bis(diphenylphosphino)ferrocene] palladium dichloride (85 mg, 0.1 mmol) were dissolved in a mixture of 1,4-dioxane (20 mL) and water (5.0 mL), and the reaction liquid was heated to 100°C and stirred for 2 hours. The reaction liquid wasdried by rotary evaporation, and the residue was purified by silica gel chromatography (eluent: petroleum ether/ethyl acetate = 3:1) to afford tert-butyl (1-(4-(4-cyano-3-fluorophenyl)-3-methoxypyridin-2-yl)piperidin-4-yl)carbamate with a yield of 63.8%.
ESI-MS m/z =427.4 $[M+H]^+$.
Synthesis Step e to Step 1 were according to Step c to Step i in Example 57.

Step m): preparation of (E)-3-(4-((1-(4-(4-cyano-3-fluorophenyl)-5-(3-hydroxy-4-methoxyphenyl)-3-methoxypyridin-2-yl)piperidin-4-yl)a mino)methyl)phenyl)-N-hydroxyacrylamide formate

Tert-butyl (E)-(1-(4-(4-cyano-3-fluorophenyl)-5-(3-hydroxy-4-methoxyphenyl)-3-methoxypyridin-2-yl)piperidin-4-yl)(4-(3-ox o-3-((tetrahydro-2H-pyran-2-yl)oxy)amino)prop-1-en-1-yl)benzyl)carbamate (80 mg, 0.19 mmol) was dissolved in a solution of 4 M HCl in ethyl acetate (5 mL) and the mixture was reacted at room temperature for 1 hour. After the reaction was completed, the reaction liquid was dried by rotary evaporation, and the residue was purified by Prep-HPLC (separation method 3) to afford (E)-3-(4-((1-(4-(4-cyano-3-fluorophenyl)-5-(3-hydroxy-4-methoxyphenyl)-3-methoxypyridin-2-yl)piperidin-4-yl)a mino)methyl)phenyl)-N-hydroxyacrylamide formate with a yield of 40.4%.
$^1H$ NMR (400 MHz, DMSO-$d_6$) δ 10.72 (s, 1H), 8.92 (s, 2H), 7.98 (s, 1H), 7.85 (t, *J* = 7.6 Hz, 1H), 7.52 (d, *J* = 8.0 Hz, 2H), 7.48 - 7.37 (m, 4H), 7.18 - 7.08 (m, 1H), 6.77 (d, *J* = 8.8 Hz, 1H), 6.49 - 6.40 (m, 3H), 3.99 (d, *J* = 12.8 Hz, 2H), 3.84 (s, 2H), 3.70 (s, 3H), 3.46 (s, 3H), 2.86 (t, *J* = 11.6 Hz, 2H), 2.68 (s, 1H), 2.06 - 1.96 (m, 2H), 1.48 (q, *J* = 10.8 Hz, 2H).
ESI-MS m/z : 624.3 $[M+H]^+$.

### Example 89

Preparation of (E)-3-(4-(2-(1-(3-cyano-4-(4-cyano-3-fluorophenyl)-5-(3-hydroxy-4-methoxyphenyl)pyridin-2-yl)piperidin-4-yl)eth yl)phenyl)-N-hydroxyacrylamide

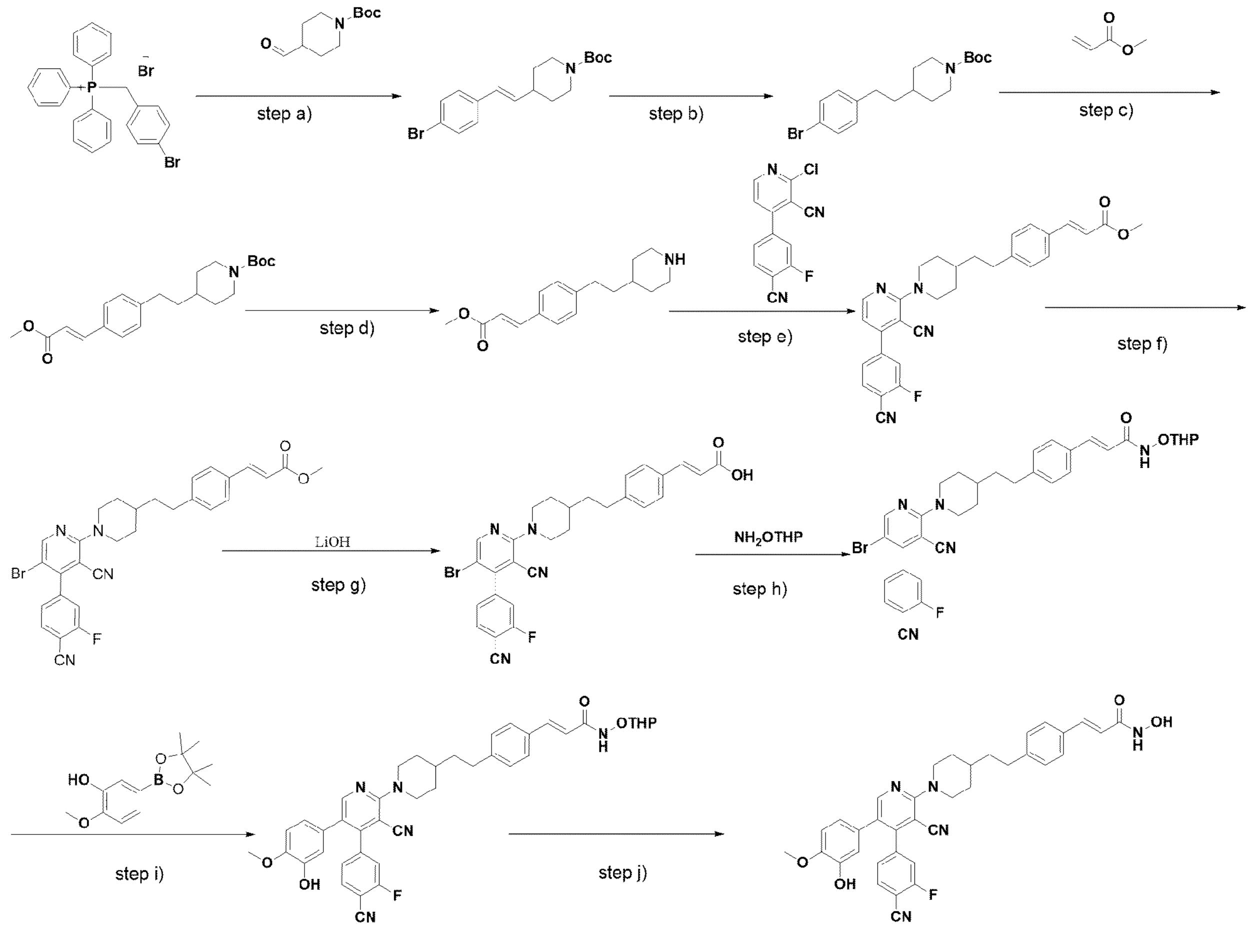


Step a): preparation of tert-butyl (E)-4-(4-bromostyryl)piperidine-1-carboxylate

(4-Bromobenzyl)triphenylphosphonium bromide (2.0 g, 3.8 mmol), NaHMDS (700 mg, 3.8 mmol), and ACN (20 mL) were added to a reaction flask, and the mixture was stirred at room temperature for 8 hours, then tert-butyl 4-formylpiperidine-1-carboxylate (808 mg, 3.8 mmol) was added, and the mixture was stirred overnight. After the reaction was completed, water (20 mL) was added to quench the reaction, and the mixture was extracted with ethyl acetate (20 mL $\times$ 2). The organic phases were combined, washed with saturated brine (20 mL $\times$ 2), dried over anhydrous sodium sulfate, filtered, and the residue was purified by silica gel chromatography (eluent: petroleum ether/ethyl acetate = 3/1) to afford tert-butyl (E)-4-(4-bromostyryl)piperidine-1-carboxylate with a yield of 91%.

ESI-MS m/z=366.1 $[M+H]^+$.

Step b): preparation of tert-butyl 4-(4-bromophenyl)piperidine-1-carboxylate

Tert-butyl (E)-4-(4-bromostyryl)piperidine-1-carboxylate (1.3 g, 3.4 mmol), palladium on carbon (150 mg, 5%) and ethanol (5 mL) were sequentially added to a reaction flask, and the mixture was stirred until dissolved, and the mixture was purged with hydrogen three times, and then stirred under a hydrogen atmosphere at room temperature for 2 hours. After the reaction was completed, the mixture was filtered, and the filtrate was concentrated under reduced pressure to afford tert-butyl 4-(4-bromophenyl)piperidine-1-carboxylate with a yield of 92.0%.

ESI-MS(m/z) =368.1$[M+H]^+$.

Step c): preparation of tert-butyl (E)-4-(4-(3-methoxy-3-oxoprop-1-en-1-yl)phenethyl)piperidine-1-carboxylate

Tert-butyl 4-(4-bromophenyl)piperidine-1-carboxylate (1.2 g, 3.2 mmol), methyl acrylate (535 mg, 6.4 mmol), $Cs_2CO_3$ (2.1 g, 6.4 mmol), Pd(dppf)$Cl_2$ (117 mg, 0.16 mmol), 1,4-dioxane (12 mL), and $H_2O$ (4 mL) were added to a reaction flask, and the mixture was stirred at 100°C for 1 hour. The mixture was concentrated to dryness under reduced pressure, and the residue was then purified by silica gel chromatography (eluent: petroleum ether/ethyl acetate = 1/1) to afford tert-butyl (E)-4-(4-(3-methoxy-3-oxoprop-1-en-1-yl)phenethyl)piperidine-1-carboxylate with a yield of 72.5%.

ESI-MS(m/z) =374.2 $[M+H]^+$.

Step d): preparation of methyl (E)-3-(4-(2-(piperidin-4-yl)ethyl)phenyl)acrylate

Tert-butyl (E)-4-(4-(3-methoxy-3-oxoprop-1-en-1-yl)phenethyl)piperidine-1-carboxylate (868 mg, 2.3 mmol) was added to a reaction flask, followed by adding hydrogen chloride solution in ethyl acetate (4M, 5 mL), and the mixture was stirred at room temperature for 1 hour, resulting in the precipitation of a significant amount of solid. The mixture was concentrated under reduced pressure to afford methyl (E)-3-(4-(2-(piperidin-4-yl)ethyl)phenyl)acrylate with a yield of 98.5%.

ESI-MS(m/z) =274.2 $[M+H]^+$.

Step e): preparation of methyl (E)-3-(4-(2-(1-(3-cyano-4-(4-cyano-3-fluorophenyl)pyridin-2-yl)piperidin-4-yl)ethyl)phenyl)acrylate

2-Chloro-4-(4-cyano-3-fluorophenyl)nicotinonitrile (500 mg, 1.9 mmol), methyl (E)-3-(4-(2-(piperidin-4-yl)ethyl) phenyl)acrylate (521 mg, 1.9 mmol), and triethylamine (2 mL) were added to a sealed reaction vessel containing ACN (20 mL), and the mixture was stirred at 70°C for 8 hours. After the reaction was completed, water (20 mL) was added to quench the reaction, followed by extraction with ethyl acetate (20 mL × 2). The organic phases were combined, washed with saturated brine (20 mL × 2), dried with anhydrous sodium sulfate, filtered, and the residue was purified by silica gel chromatography (eluent: petroleum ether/ethyl acetate = 2/1) to afford methyl (E)-3-(4-(2-(1-(3-cyano-4-(4-cyano-3-fluorophenyl)pyridin-2-yl)piperidin-4-yl)ethyl)phenyl)acrylate with a yield of 71%.

ESI-MS m/z=495.2 $[M+H]^+$.

Step f): preparation of methyl (E)-3-(4-(2-(1-(5-bromo-3-cyano-4-(4-cyano-3-fluorophenyl)pyridin-2-yl)piperidin-4-yl) ethyl)phenyl)acrylate

Methyl (E)-3-(4-(2-(1-(3-cyano-4-(4-cyano-3-fluorophenyl)pyridin-2-yl)piperidin-4-yl)ethyl)phenyl)acrylate (693 mg, 1.4 mmol) and ACN (10 mL) were added to a reaction flask, then NBS (299 mg, 1.7 mmol) was added dropwise under stirring in an ice bath, and the reaction was continued under stirring in the ice bath for 2 hours. After the reaction was completed, saturated sodium bicarbonate aqueous solution (40 mL) was added, and the mixture was extracted with ethyl acetate (40 mL × 3). The organic phases were combined, washed with saturated brine (40 mL × 2), and then concentrated to dryness under reduced pressure. The residue was purified by silica gel chromatography (eluent: petroleum ether/ethyl acetate = 1/1) to afford methyl (E)-3-(4-(2-(1-(5-bromo-3-cyano-4-(4-cyano-3-fluorophenyl)pyridin-2-yl)piperidin-4-yl) ethyl)phenyl)acrylate with a yield of 57.0%.

ESI-MS(m/z) =573.1$[M+H]^+$.

Step g): preparation of (E)-3-(4-(2-(1-(5-bromo-3-cyano-4-(4-cyano-3-fluorophenyl)pyridin-2-yl)piperidin-4-yl)ethyl) phenyl)acrylic acid

Methyl (E)-3-(4-(2-(1-(5-bromo-3-cyano-4-(4-cyano-3-fluorophenyl)pyridin-2-yl)piperidin-4-yl)ethyl)phenyl)acrylate (457 mg, 798 umol) and lithium hydroxide (168 mg, 4.0 mmol) were added to a reaction flask containing THF (5 mL), MeOH (3 mL), and water (2 mL), and the mixture was stirred at room temperature for 2 hours. 2M HCl aqueous solution was added dropwise to adjust the pH to 4, and the mixture was then extracted with a mixture of MeOH/DCM (1:5) (20 mL × 2). The organic phases were combined, washed with saturated brine (15 mL × 2), dried over anhydrous sodium sulfate, and concentrated to afford (E)-3-(4-(2-(1-(5-bromo-3-cyano-4-(4-cyano-3-fluorophenyl)pyridin-2-yl)piperidin-4-yl)ethyl)phenyl)acrylic acid, which was directly used in the next step.

ESI-MS(m/z) =559.1$[M+H]^+$.

Step h): preparation of (E)-3-(4-(2-(1-(5-bromo-3-cyano-4-(4-cyano-3-fluorophenyl)pyridin-2-yl)piperidin-4-yl)ethyl) phenyl)-N-(tetrahydr o-2H-pyran-2-acyl)oxy)acrylamide

(E)-3-(4-(2-(1-(5-Bromo-3-cyano-4-(4-cyano-3-fluorophenyl)pyridin-2-yl)piperidin-4-yl)ethyl)phenyl)acrylic acid (446 mg, 798 umol), O-(tetrahydro-2H-pyran-2-yl)hydroxylamine (187 mg, 1.6 mmol), HATU (364 mg, 957 umol), and DIEA (205 mg, 1.6 mmol) were added to a reaction flask containing DMF (5 mL), and the mixture was stirred at room temperature for 2 hours. Water (10 mL) was added to the reaction liquid to quench the reaction, and the mixture was extracted with ethyl acetate (20 mL × 2). The organic phases were combined, washed with saturated brine (15 mL × 2), dried over anhydrous sodium sulfate, and concentrated under vacuum, and the residue was purified by silica gel chromatography (eluent: DCM/MeOH = 10/1) to afford (E)-3-(4-(2-(1-(5-bromo-3-cyano-4-(4-cyano-3-fluorophenyl)pyridin-2-yl)piperidin-4-yl)ethyl)phenyl)-N-(tetrahydr o-2H-pyran-2-acyl)oxy)acrylamide with a yield of 47%.

ESI-MS m/z=658.2 $[M+H]^+$.

Step i): preparation of (E)-3-(4-(2-(1-(3-cyano-4-(4-cyano-3-fluorophenyl)-5-(3-hydroxy-4-methoxyphenyl)pyridin-2-yl)piperidin-4-yl)eth yl)phenyl)-N-(tetrahydro-2H-pyran-2-acyl)oxy)acrylamide

(E)-3-(4-(2-(1-(5-Bromo-3-cyano-4-(4-cyano-3-fluorophenyl)pyridin-2-yl)piperidin-4-yl)ethyl)phenyl)-N-(tetrahydro-2H-pyran-2-acyl)oxy)acrylamide (247 mg, 375 umol), (3-hydroxy-4-methoxyphenyl)boronic acid (113 mg, 450 umol), $Cs_2CO_3$ (245 mg, 750 umol), $Pd(dppf)Cl_2$ (27 mg, 37.5 umol), 1,4-dioxane (4 mL), and $H_2O$ (1 mL) were added to a reaction flask, and the mixture was stirred at 90°C for 1 hour. The mixture was concentrated to dryness under reduced pressure, and the residue was purified by silica gel chromatography (eluent: petroleum ether/ethyl acetate = 1/3) to afford (E)-3-(4-(2-(1-(3-cyano-4-(4-cyano-3-fluorophenyl)-5-(3-hydroxy-4-methoxyphenyl)pyridin-2-yl)piperidin-4-yl)eth yl)phenyl)-N-(tetrahydro-2H-pyran-2-acyl)oxy)acrylamide with a yield of 42.5%.

ESI-MS(m/z) =702.3 $[M+H]^+$.

Step j): preparation of (E)-3-(4-(2-(1-(3-cyano-4-(4-cyano-3-fluorophenyl)-5-(3-hydroxy-4-methoxyphenyl)pyridin-2-yl)piperidin-4-yl)eth yl)phenyl)-N-hydroxyacrylamide

(E)-3-(4-(2-(1-(3-Cyano-4-(4-cyano-3-fluorophenyl)-5-(3-hydroxy-4-methoxyphenyl)pyridin-2-yl)piperidin-4-yl)ethyl)phenyl)-N-(tetrahydro-2H-pyran-2-acyl)oxy)acrylamide (112 mg, 159 umol) was added to a reaction flask, followed by adding hydrogen chloride solution in ethyl acetate (4M, 2.5 mL), and the mixture was stirred at room temperature for 1 hour, resulting in the precipitation of a significant amount of solid. The mixture was concentrated under reduced pressure, and the obtained crude product was purified by Prep-HPLC (separation method 1) to afford (E)-3-(4-(2-(1-(3-cyano-4-(4-cyano-3-fluorophenyl)-5-(3-hydroxy-4-methoxyphenyl)pyridin-2-yl)piperidin-4-yl)eth yl)phenyl)-N-hydroxyacrylamide with a yield of 11.5%.

$^1H$ NMR (400 MHz, DMSO-$d_6$) δ 10.71 (s, 1H), 8.99 (s, 1H),8.52 (s, 1H), 8.41 (s, 1H), 7.95 (t, *J* = 7.4 Hz, 1H), 7.66 (d, *J* = 10.0 Hz, 1H), 7.53 - 7.38 (m, 3H), 7.27 (d, *J* = 7.8 Hz, 3H), 6.80 (d, *J* = 8.0 Hz, 1H), 6.52 - 6.33 (m, 3H), 4.27 (d, *J* = 12.8 Hz, 2H), 3.71 (s, 3H), 3.03 (t, *J* = 12.0 Hz, 2H), 2.67 (t, *J* = 7.4 Hz, 2H), 1.87 (d, *J* = 5.2 Hz, 2H), 1.56 (q, *J* = 10.0, 8.8 Hz, 3H), 1.30 (d, *J* = 5.2 Hz, 2H).

ESI-MS(m/z) =618.2 $[M+H]^+$.

**Example 90**

Preparation of (E)-3-(4-(1-(3-cyano-4-(4-cyano-3-fluorophenyl)-5-(3-hydroxy-4-(E)-3-(hydroxyamino)-3-oxoprop-1-en-1-yl)phen yl)pyridin-2-yl)piperidin-4-yl)amino)methyl)phenyl)-N-hydroxyacrylamide

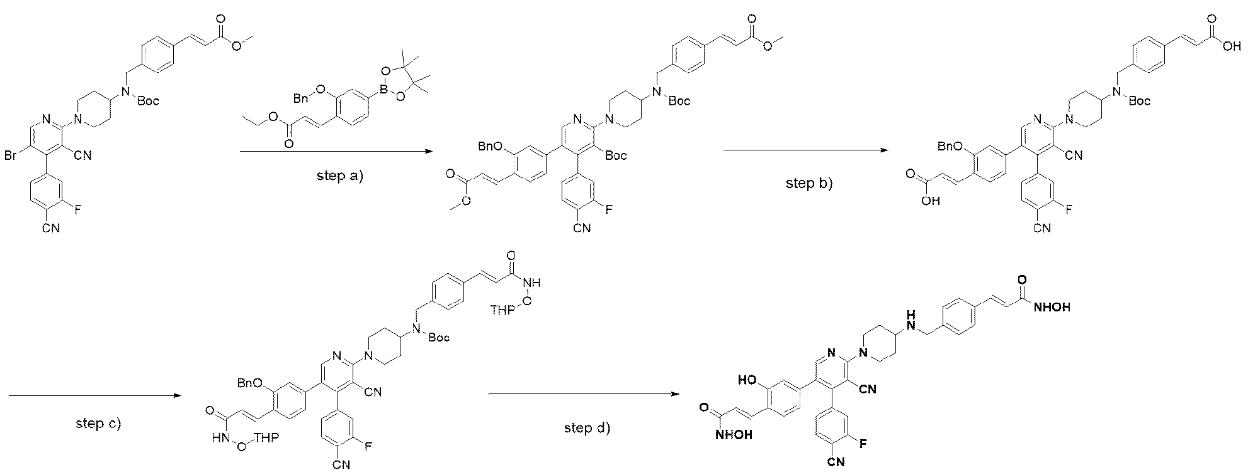


Step a): preparation of tert-butyl 5-(3-(benzyloxy)-4-(E)-3-methoxy-3-oxoprop-1-en-1-yl)phenyl)-2-(4-(tert-butoxycarbonyl)(4-(E)-3-methoxy-3-oxo prop-1-en-1-yl)benzyl)amino)piperidin-1-yl)-4-(4-cyano-3-fluorophenyl)nicotinate

The product of step f in Example 63: Methyl (E)-3-(4-(((1-(5-bromo-3-cyano-4-(4-cyano-3-fluorophenyl)pyridin-2-yl)piperidin-4-yl)(tert-butoxycarbonyl)amino) methyl)phenyl)acrylate (200 mg, 297 μmol), ethyl (E)-3-(2-(benzyloxy)-4-(4,4,5,5-tetramethyl-1,3,2-dioxaborolan-2-yl)phenyl)acrylate (182 mg, 446 μmol), $Cs_2CO_3$ (193 mg, 594 μmol), Pd(dppf)$Cl_2$ (22 mg, 30 μmol), 1,4-dioxane (4 mL), and $H_2O$ (1 mL) were added to a reaction flask, and the mixture was stirred at 120°C for 1 hour. The mixture was concentrated under reduced pressure, and the residue was purified by silica gel chromatography (eluent: petroleum ether/ethyl acetate = 1/1) to afford tert-butyl 5-(3-(benzyloxy)-4-(E)-3-methoxy-3-oxoprop-1-en-1-yl)phenyl)-2-(4-(tert-butoxycarbonyl)(4-(E)-3-methoxy-3-oxo prop-1-en-1-yl)benzyl)amino)piperidin-1-yl)-4-(4-cyano-3-fluorophenyl)nicotinate with a yield of 78.5%.

ESI-MS(m/z) =938.1 $[M+H]^+$.

Step b): preparation of (E)-3-(4-(1-(5-(3-(benzyloxy)-4-(2-carboxyvinyl)phenyl)-3-cyano-4-(4-cyano-3-fluorophenyl) pyridin-2-yl)piperidin -4-yl)(tert-butoxycarbonyl)amino)methyl)phenyl)acrylic acid

Tert-butyl 5-(3-(benzyloxy)-4-(E)-3-methoxy-3-oxoprop-1-en-1-yl)phenyl)-2-(4-(tert-butoxycarbonyl)(4-(E)-3-methoxy-3-oxo prop-1-en-1-yl)benzyl)amino)piperidin-1-yl)-4-(4-cyano-3-fluorophenyl)nicotinate (219 mg, 233 μmol), lithium hydroxide monohydrate (50 mg, 1.2 mmol), tetrahydrofuran (2 mL), isopropanol (2 mL), and water (1 mL) were added to a reaction flask, and the mixture was stirred for reaction at room temperature for 12 hours. Under stirring in an ice bath, 1N concentrated hydrochloric acid was slowly added dropwise to adjust the pH to 3-4, then water (10 mL) was then added, and the mixture was extracted with ethyl acetate (10 mL × 3). The organic phases were combined, washed with saturated brine (10 mL × 2), and concentrated to dryness under reduced pressure to afford (E)-3-(4-(1-(5-(3-(benzyloxy)-4-(2-carboxyvinyl)phenyl)-3-cyano-4-(4-cyano-3-fluorophenyl)pyridin-2-yl)piperidin -4-yl)(tert-butoxycarbonyl) amino)methyl)phenyl)acrylic acid which was used directly for the next reaction step.

ESI-MS(m/z) =834.4$[M+H]^+$.

Step c): preparation of tert-butyl (1-(5-(3-(benzyloxy)-4-(E)-3-oxo-3-(tetrahydro-2H-pyran-2-yl)oxy)amino)prop-1-en-1-yl)phenyl)-3-cyano-4-(4-cya no-3-fluorophenyl)pyridin-2-yl)piperidin-4-yl)(4-((E)-3-oxo-3-((tetrahydro-2H-pyran-2-yl) oxy)amino)prop-1-en-1-yl)benzyl)carbamate

(E)-3-(4-(1-(5-(3-(Benzyloxy)-4-(2-carboxyvinyl)phenyl)-3-cyano-4-(4-cyano-3-fluorophenyl)pyridin-2-yl)pip eridin-4-yl)(tert-butoxycarbonyl)amino)methyl)phenyl)acrylic acid (194 mg, 233 μmol), O-(tetrahydro-2H-pyran-2-yl) hydroxylamine (55 mg, 466 μmol), DIEA (90 mg, 699 μmol), and DMF (2 mL) were added to a reaction flask, then HATU (133 mg, 350 μmol) was added under stirring at room temperature, and the reaction was maintained for 1 hour at room temperature. After the reaction was completed, water (10 mL) was added to quench the reaction, followed by extraction with ethyl acetate (20 mL × 2), and the organic phases were combined and washed sequentially with saturated sodium bicarbonate aqueous solution (20 mL × 1) and saturated brine (10 mL × 2), dried over anhydrous sodium sulfate, and filtered. The filtrate was concentrated under reduced pressure, and the residue was purified by silica gel chromatography (eluent: petroleum ether/ethyl acetate = 1/1) to afford tert-butyl (1-(5-(3-(benzyloxy)-4-(E)-3-oxo-3-(tetrahydro-2H-pyran-2-yl)oxy)amino)prop-1-en-1-yl)phenyl)-3-cyano-4-(4-cya no-3-fluorophenyl)pyridin-2-yl)piperidin-4-yl)(4-((E)-3-oxo-3-((tetrahydro-2H-pyran-2-yl)oxy)amino)prop-1-en-1-yl)benzyl)carbamate with a yield of 60.5%.

ESI-MS(m/z) =1033.2 $[M+H]^+$.

Step d): preparation of (E)-3-(4-(1-(3-cyano-4-(4-cyano-3-fluorophenyl)-5-(3-hydroxy-4-(E)-3-(hydroxyamino)-3-oxoprop-1-en-1-yl)phen yl)pyridin-2-yl)piperidin-4-yl)amino)methyl)phenyl)-N-hydroxyacrylamide

Tert-butyl (1-(5-(3-(benzyloxy)-4-(E)-3-oxo-3-(tetrahydro-2H-pyran-2-yl)oxy)amino)prop-1-en-1-yl)phenyl)-3-cyano-4-(4-cya no-3-fluorophenyl)pyridin-2-yl)piperidin-4-yl)(4-((E)-3-oxo-3-((tetrahydro-2H-pyran-2-yl)oxy)amino) prop-1-en-1-yl)benzyl)carbamate (145 mg, 141 μmol) was added to a reaction flask, followed by adding hydrogen chloride solution in ethyl acetate (4M, 2.5 mL), and the mixture was stirred at room temperature for 1 hour, resulting in the precipitation of a significant amount of solid. After concentrating under reduced pressure, the obtained crude product was purified by Prep-HPLC (separation method 3) to afford (E)-3-(4-(1-(3-cyano-4-(4-cyano-3-fluorophenyl)-5-(3-hydroxy-4-(E)-3-(hydroxyamino)-3-oxoprop-1-en-1-yl)phen yl)pyridin-2-yl)piperidin-4-yl)amino)methyl)phenyl)-N-hydroxyacrylamide formate with a yield of 20.5%.

$^1H$ NMR (400 MHz, Methanol-$d_4$) δ8.52 (s, 1H), 8.46 (s, 1H), 7.81-7.69 (m, 2H), 7.67-7.49 (m, 5H), 7.43 (dd, *J* = 9.6, 1.4 Hz, 1H), 7.33 (d, *J* = 8.4 Hz, 1H), 7.22 (dd, *J* = 8.0, 1.4 Hz, 1H), 6.64-6.45 (m, 4H), 4.48 (d, *J* = 13.0 Hz, 2H), 4.21 (s, 2H), 3.55 (s, 1H), 3.19 (t, *J* = 12.6 Hz, 2H), 2.37-2.21 (m, 2H), 1.80 (td, *J* = 13.0, 9.0 Hz, 2H).

ESI-MS(m/z) =674.2 $[M+H]^+$.

**Examples 91 to 174** were prepared according to the synthetic method of Example **37** (the separation method for the compounds: hydrochloride, trifluoroacetate , and formate were prepared according to separation methods 1, 2, and 3, respectively), and the structure and characterization data are as follows:

| Exampl e | Chemical name | Structure | $^1$H NMR | MS( M+H )$^+$ |
|---|---|---|---|---|
| 91 | 4-(4-(1-(3-Cyan o-4-(4-cyano-3-f luorophenyl)-5-( 4-hydroxy-3-me thoxyphenyl)pyr idin-2-yl)piperid in-4-yl)amino)m ethyl)piperidin-1-yl)-N-hydroxy benzamide formate | 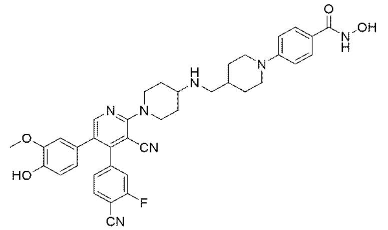 | $^1$H NMR (400 MHz, Methanol-d4)δ ppm 8.52 (s, 1.4H), 8.47 (s, 1H), 7.75 (dd, J = 8.0, 6.6 Hz, 1H), 7.64 (d, J = 8.8 Hz, 2H), 7.37 (dd, J = 9.8, 1.4 Hz, 1H), 7.26 (dd, J = 8.0, 1.4 Hz, 1H), 6.97 (d, J = 8.8 Hz, 2H), 6.77 - 6.63 (m, 1H), 6.62 - 6.47 (m, 2H), 4.42 (d, J = 13.4 Hz, 2H), 3.92 (d, J = 12.6 Hz, 2H), 3.64 (s, 3H), 3.16 (t, J = 12.2 Hz, 3H), 3.00 - 2.74 (m, 4H), 2.34 - 2.13 (m, 2H), 1.90 (d, J = 12.4 Hz, 3H), 1.76 (q, J = 2.0, 5.2 Hz, 2H), 1.41 (q, J = 9.8 Hz, 2H). | 676.3 |
| 92 | (E)-3-(3-(2-(1-( 3-Cyano-4-(4-cy ano-3-fluorophe nyl)-5-(4-hydro xy-3-methoxyph enyl)pyridin-2-y l)piperidin-4-yl) amino)ethyl)phe nyl)-N-hydroxy acrylamide formate | 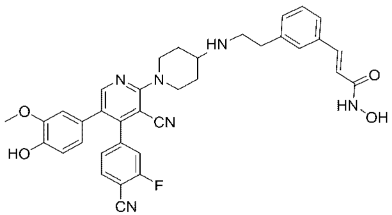 | $^1$H NMR (400 MHz, Methanol-d4)δ ppm: 8.51 (s, 1H), 8.42 (s, 1H), 7.74 (dd, J = 8.0, 6.4 Hz, 1H), 7.60 - 7.42 (m, 3H), 7.42 - 7.27 (m, 3H), 7.22 (dd, J = 8.0, 1.6 Hz, 1H), 6.86 - 6.78 (m, 1H), 6.50 (m, 3H), 4.40 (d, J = 13.6 Hz, 2H), 3.81 (s, 3H), 3.29 - 3.07 (m, 5H), 2.98 (t, J = 8.0 Hz, 2H), 2.19 (d, J = 12.4 Hz, 2H), 1.73 (m, 2H). | 633.2 |
| 93 | 4-(3-(3-Cyano-4 -(4-cyano-3-fluo rophenyl)-5-(4-h ydroxy-3-metho xyphenyl) pyridi n-2-yl)piperidin -4-yl) amino)pro pyl)-N-hydroxy benzamide formate | 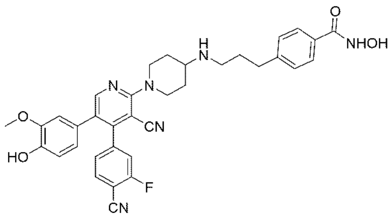 | $^1$H NMR (400 MHz, DMSO-d6) δ ppm: 8.49 (s, 1H), 8.30 (s, 1H), 7.96 (dd, J = 8.0, 6.8 Hz, 1H), 7.74 - 7.58 (m, 3H), 7.35 - 7.24 (m, 3H), 6.66 (d, J = 8.0 Hz, 1H), 6.57 (m, 1H), 6.48 (dd, J = 8.0, 2.0 Hz, 1H), 4.27 - 4.13 (m, 2H), 3.55 (s, 3H), 3.16 (t, J = 12.2 Hz, 2H), 2.80 (s, 1H), 2.66 (m, 4H), 1.97 (d, J = 12.8 Hz, 2H), 1.84 - 1.69 (m, 2H), 1.43 (m, 2H). | 621.3 |
| 94 | (E)-3-(4-(2-(3-C yano-4-(4-cyano -3-fluorophenyl) -5-(4-hydroxy-3 -methoxyphenyl ) pyridin-2-yl)-2-azabicyclo [2.2.1]heptan-5-yl)am ino) methyl)phen yl)-N-hydroxyac rylamide formate | 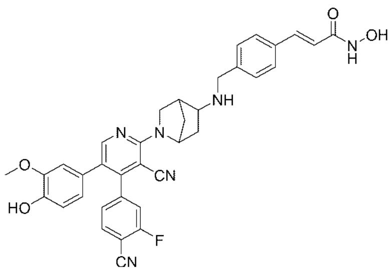 | $^1$H NMR (400 MHz, Methanol-d4) δ 8.52 (s, 1H), 8.32 (d, J = 3.8 Hz, 1H), 7.73 (s, 1H), 7.56 (m, 3H), 7.48 - 7.33 (m, 3H), 7.24 (s, 1H), 6.67 (d, J = 8.2 Hz, 1H), 6.53 - 6.43 (m, 3H), 4.02 - 3.83 (m, 4H), 3.63 (m, 3H),3.58 (s, 1H), 3.08 (s, 1H), 2.84 (d, J = 10.8 Hz, 1H), 2.31 (t, J = 10.6 Hz, 0.5H), 2.18 (t, J = 11.2 Hz, 0.5H), 1.96-1.85 (m, 1H), 1.78-1.70 (m, 1H), 1.63 - 1.51 (m, 1H). | 631.3 |

(continued)

| Exampl e | Chemical name | Structure | $^1$H NMR | MS( M+H )$^+$ |
|---|---|---|---|---|
| 95 | (E)-3-(6-((1-(3-Cyano-4-(4-cya no-3-fluorophen yl)-5-(4-hydrox y-3-methoxyphe nyl) pyridin-2-yl )piperidin-4-yl)a mino)methyl)py ridin-3-yl)-N-hy droxyacrylamid e formate | 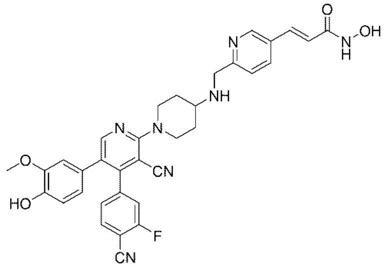 | $^1$H NMR (400 MHz, DMSO-d6) δ 10.80 (s, 1H), 9.09 (s, 1H), 8.69 (d, J = 2.4 Hz, 1H), 8.49 (s, 1H), 8.15 (s, 1H), 8.02 - 7.88 (m, 2H), 7.63 (dd, J = 10.0, 1.2 Hz, 1H), 7.59 - 7.42 (m, 2H), 7.31 (dd, J = 8.0, 1.6 Hz, 1H), 6.65 (d, J = 8.0 Hz, 1H), 6.60 - 6.42 (m, 3H), 4.18 (d, J = 13.2 Hz, 2H), 3.95 (s, 2H), 3.55 (s, 3H), 3.17 (t, J = 12.0 Hz, 2H), 2.80 (s, 1H), 2.01 (d, J = 12.8 Hz, 2H), 1.48 (q, J = 10.8, 10.0 Hz, 2H). | 620.2 |
| 96 | (E)-3-(5-((1-(3-Cyano-4-(4-cya no-3-fluorophen yl)-5-(4-hydrox y-3-methoxyphe nyl) pyridin-2-yl )piperidin-4-yl)a mino)methyl)py rimidin-2-yl)-N-hydroxyacrylam ide for-mate | 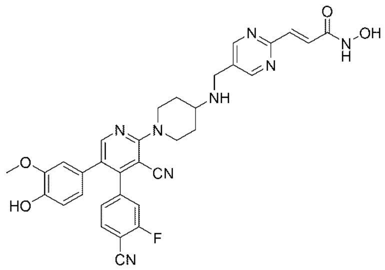 | $^1$H NMR (400 MHz, Methanol-d4) δ 8.85 (s, 1H), 8.44 (s, 1H),8.41 (s, 1H), 7.75 (dd, J = 8.0, 6.8 Hz, 1H), 7.56 (d, J = 15.6 Hz, 1H), 7.37 (dd, J = 9.6, 1.6 Hz, 1H), 7.29 - 7.17 (m, 2H), 6.70 (d, J = 8.4 Hz, 1H), 6.55 (d, J = 6.8 Hz, 2H), 4.38 (d, J = 13.2 Hz, 2H), 4.03 (s, 2H), 3.64 (s, 3H), 3.18 (t, J = 12.4 Hz, 2H), 3.01 (d, J = 11.2 Hz, 1H), 2.27 - 2.10 (m, 2H), 1.68 (t, J = 11.6 Hz, 2H). | 621.2 |
| 97 | (E)-3-(4-((1-(3-Cyano-4-(4-cya no-3-fluorophen yl)-5-(4-fluoro-3 -hydroxyphenyl )pyridin-2-yl)pi peridin-4-yl)ami no)methyl)phen yl)-N-hydro-xyac rylamide formate | 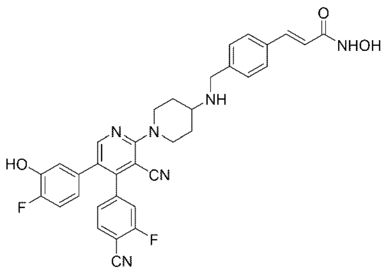 | $^1$H NMR (400 MHz, Methanol-d4) δ 8.49 (s, 1H), 8.43 (s, 1H), 7.75 (dd, J = 8.0, 6.4 Hz, 1H), 7.62 (t, J = 9.2 Hz, 3H), 7.52 (d, J = 7.6 Hz, 2H), 7.39 (dd, J = 9.6, 1.6 Hz, 1H), 7.23 (dd, J = 8.0, 1.6 Hz, 1H), 6.95 (dd, J = 11.2, 8.4 Hz, 1H), 6.63 (dd, J = 8.4, 2.0 Hz, 1H), 6.57 - 6.46 (m, 2H), 4.46 (d, J = 13.2 Hz, 2H), 4.20 (s, 2H), 3.32 (s, 1H), 3.17 (t, J = 12.8 Hz, 2H), 2.27 (d, J = 11.2 Hz, 2H), 1.87 - 1.71 (m, 2H). | 607.2 |
| 98 | (E)-3-(3-((1-(3-Cyano-4-(4-cya no-3-fluorophen yl)-5-(4-hydrox y-3-methoxyphe nyl) pyridin-2-yl )piperidin-4-yl)a mino)methyl)ph enyl)-N-hy-drox yacrylamide formate | 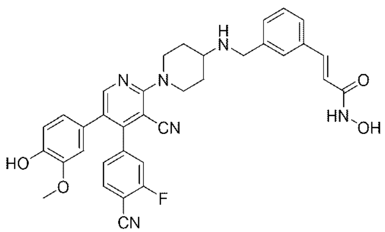 | $^1$H NMR (400 MHz, Methanol-d4) δ ppm 8.52 (s, 1H), 8.45 (s, 1H), 8.31 (s, 1H), 7.92 (t, J = 7.4 Hz, 1H), 7.64 - 7.54 (m, 2H), 7.45 (d, J = 5.6 Hz, 1H), 7.43 - 7.36 (m, 2H), 7.29 (dd, J = 8.0, 1.6 Hz, 1H), 6.65 (d, J = 8.2 Hz, 1H), 6.57 - 6.43 (m, 3H), 4.18 (d, J = 12.8 Hz, 2H), 3.84 (s, 2H), 3.53 (s, 3H), 3.13 (d, J = 24.4 Hz, 2H), 2.80 (s, 1H), 2.02 (d, J = 12.4 Hz, 2H), 1.47 (q, J = 11.0 Hz, 2H). | 619.2 |

(continued)

| Exampl e | Chemical name | Structure | 1H NMR | MS( M+H )+ |
|---|---|---|---|---|
| 99 | (E)-3-(4-(((1-(3-Cyano-4-(4-cya no-3-fluorophen yl)-5-(4-hydrox y-3-methoxyphe nyl) pyridin-2-yl )piperidin-4-yl) ( methyl)amino) methyl)phenyl)-N-hydroxyacryl amide formate | 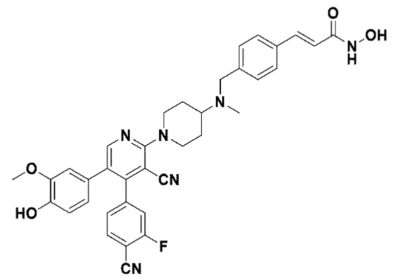 | 1H NMR (400 MHz, DMSO-d6) δ 10.73 (s, 1H), 9.05 (s, 1H), 8.42 (s, 1H), 7.95 (dd, J = 8.0, 6.8 Hz, 1H), 7.66 (dd, J = 10.2, 1.6 Hz, 1H), 7.51 (d, J = 7.8 Hz, 2H), 7.44 (d, J = 15.8 Hz, 1H), 7.36 (d, J = 7.8 Hz, 2H), 7.28 (dd, J = 8.0, 1.6 Hz, 1H), 6.80 (d, J = 8.0 Hz, 1H), 6.52 - 6.39 (m, 3H), 4.34 (d, J = 12.8 Hz, 2H), 3.56 (s, 3H), 3.61 (s, 2H), 3.07 (t, J = 12.4 Hz, 2H), 2.80 - 2.62 (m, 1H), 2.14 (s, 3H), 1.93 (d, J = 12.2 Hz, 2H), 1.77 - 1.54 (m, 2H). | 633.3 |
| 100 | (E)-3-(4-((1-(3-Cyano-4-(4-cya no-3-fluorophen yl)-5-(4-hydrox y-3-methoxyphe nyl) pyridin-2-yl )piperidin-4-yl)a mino)methyl)ph enyl)-N-hy-drox yacrylamide hydrochlor-ide | 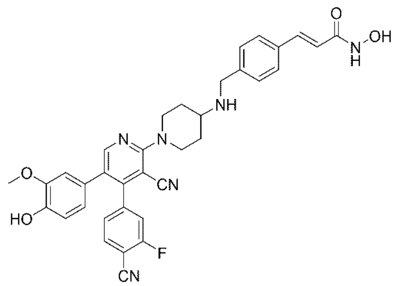 | 1H NMR (400 MHz, DMSO-d6) δ 10.79 (s, 1H), 9.04 (d, J = 20.6 Hz, 2H), 8.90 (s, 2H), 8.47 (s, 1H), 7.97 (t, J = 7.4 Hz, 1H), 7.71 - 7.62 (m, 3H), 7.56 (d, J = 7.8 Hz, 2H), 7.48 (d, J = 15.6 Hz, 1H), 7.28 (d, J = 8.0 Hz, 1H), 6.82 (d, J = 8.0 Hz, 1H), 6.55 - 6.44 (m, 3H), 4.41 - 4.19 (m, 4H), 3.53 (s, 3H), 3.43 (s, 1H), 3.13 (t, J = 12.6 Hz, 2H), 2.25 (d, J = 12.6 Hz, 2H), 1.82 - 1.67 (m, 2H). | 619.2 |
| 101 | (E)-3-(4-((1-(5-( 4-Chloro-3-hydr oxyphenyl)-3-cy ano-4-(4-cyano-3-fluorophenyl) pyridin-2-yl)pip eridin-4-yl)amin o)methyl)phenyl )-N-hydroxyacr ylamide formate | 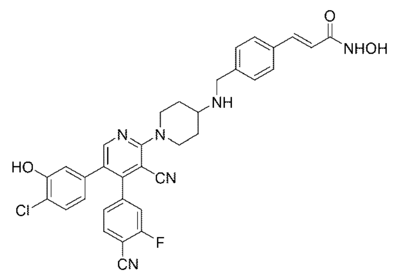 | 1H NMR (400 MHz,Methanol-d4) δ 8.49 (s, 1H), 8.44 (s, 1H), 7.75 (t, J = 7.2 Hz, 1H), 7.62 (t, J = 8.4 Hz, 2H), 7.52 (d, J = 8.0 Hz, 3H), 7.41 (dd, J = 9.6, 1.2 Hz, 1H), 7.26 - 7.14 (m, 2H), 6.62 (d, J = 2.0 Hz, 1H), 6.57 - 6.46 (m, 2H), 4.47 (d, J = 13.2 Hz, 2H), 4.19 (s, 2H), 3.30 (s, 1H), 3.18 (t, J = 12.4 Hz, 2H), 2.27 (d, J = 12.0 Hz, 2H), 1.79 (dd, J = 12.4, 3.9 Hz, 2H). | 623.2 |

(continued)

| Exampl e | Chemical name | Structure | $^1$H NMR | MS( M+H )$^+$ |
|---|---|---|---|---|
| 102 | (E)-3-(4-(((1-(3-Cyano-4-(4-cya no-3-fluorophen yl)-5-(3-hydrox y-4-methylphen yl) pyridin-2-yl) piperidin-4-yl)a mino)methyl)ph enyl)-N-hy-drox yacrylamide formate | 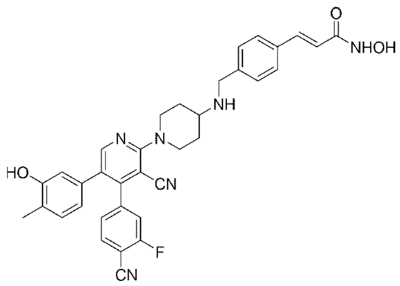 | $^1$H NMR (400 MHz, Methanol-d4) δ 8.52 (s, 1H),8.39 (s, 1H), 7.69 (dd, J = 8.0, 6.6 Hz, 1H), 7.60 (d, J = 8.0 Hz, 2H), 7.56 (s, 1H), 7.48 (d, J = 7.8 Hz, 2H), 7.32 (dd, J = 9.8, 1.6 Hz, 1H), 7.17 (dd, J = 8.0, 1.5 Hz, 1H), 6.92 (d, J = 8.0 Hz, 1H), 6.50 - 6.37 (m, 3H), 4.40 (d, J = 13.6 Hz, 2H), 4.18 (s, 2H), 3.12 (t, J = 13.2 Hz, 3H), 2.24 (d, J = 12.2 Hz, 2H), 2.08 (s, 3H), 1.83 - 1.70 (m, 2H). | 603.2 |
| 103 | (E)-3-(4-(((1-(5-(Benzo[d] [1,3]d ioxol-5-yl)-3-cy ano-4-(4-cyano-3-fluorophenyl) pyridin-2-yl)pip eridin-4-yl)amin o)methyl)phenyl )-N-hydroxyacr ylamide formate | 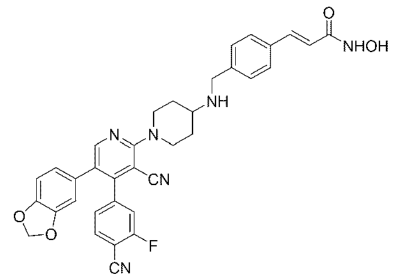 | $^1$H NMR (400 MHz, Methanol-d4) δ 8.51(d, J = 2.7 Hz, 1H), 8.43 (d, J = 2.8 Hz, 1H), 838 (s, 1H), 7.71 (t, J = 7.2 Hz, 1H), 7.59 (d, J = 7.8 Hz, 2H), 7.54 (d, J = 15.8 Hz, 1H), 7.47 (d, J = 7.8 Hz, 2H), 7.36 - 7.29 (m, 1H), 7.21 (d, J = 8.2 Hz, 1H), 6.69 (d, J = 8.6 Hz, 1H), 6.54 - 6.50 (m, 2H), 6.47 (d, J = 15.8 Hz, 1H), 5.88 (s, 2H), 4.40 (d, J = 13.4 Hz, 2H), 4.13 (s, 2H), 3.44 (s, 1H), 3.12 (t, J = 12.8 Hz, 2H), 2.22 (d, J = 12.4 Hz, 2H), 1.73 (q, J = 11.6 Hz, 2H). | 617.2 |
| 104 | (E)-3-(6-((1-(3-Cyano-4-(4-cya no-3-fluorophen yl)-5-(3-hydrox y-4-methoxyphe nyl) pyridin-2-yl )piperidin-4-yl)a mino)methyl)py ridin-3-yl)-N-hy droxyacrylamid e formate | 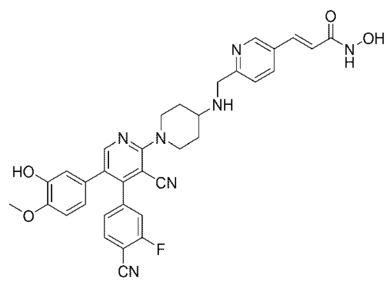 | $^1$H NMR (400 MHz, DMSO-d6) δ10.82 (s, 1H), 9.02 (s, 1H),8.67 (d, J = 2.0 Hz, 1H), 8.41 (s, 1H), 8.26 (s, 1H), 7.94 (t, J = 7.6 Hz, 2H), 7.65 (d, J = 10.0 Hz, 1H), 7.57 - 7.45 (m, 2H), 7.27 (dd, J = 8.0, 1.6 Hz, 1H), 6.80 (d, J = 8.0 Hz, 1H), 6.54 (d, J = 16.0 Hz, 1H), 6.47 (d, J = 7.2 Hz, 2H), 4.16 (dd, J = 10.8, 6.8 Hz, 2H), 3.91 (s, 2H), 3.71 (s, 3H), 3.18 (t, J = 12.0 Hz, 2H), 2.74 (t, J = 4.4 Hz, 1H), 1.98 (dd, J = 13.2, 4.0 Hz, 2H), 1.45 (d, J = 11.2 Hz, 2H). | 620.2 |

(continued)

| Exampl e | Chemical name | Structure | $^1$H NMR | MS( M+H )$^+$ |
|---|---|---|---|---|
| 105 | (E)-3-(5-(((1-(3-Cyano-4-(4-cya no-3-fluorophen yl)-5-(3-hydrox y-4-methoxyphe nyl) pyridin-2-yl )piperidin-4-yl)a mino)methyl)thi ophen-2-yl)-N-h ydroxyacrylami de formate | 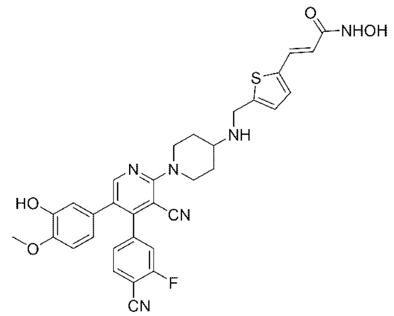 | $^1$H NMR (400 MHz, DMSO-d6) δ 10.62 (s, 1H), 8.98 (s, 2H), 8.41 (s, 1H), 8.21 (s, 1H), 7.95 (t, J = 7.6 Hz, 1H), 7.59 (m, 2H), 6.94 (d, J = 3.6 Hz, 1H), 6.80 (d, J = 8.0 Hz, 1H), 6.47 (d, J = 8.0 Hz, 2H), 6.10 (d, J = 16.4 Hz, 1H), 4.17 (d, J = 13.2 Hz, 2H), 3.95 (s, 2H), 3.71 (s, 3H), 3.19 (d, J = 12.4 Hz, 2H), 2.80 - 2.71 (m, 1H), 1.97 (d, J = 12.4 Hz, 2H), 1.43 (m, 2H). | 625.2 |
| 106 | (E)-3-(5-(((1-(3-Cyano-4-(4-cya no-3-fluorophen yl)-5-(3-hydrox y-4-methoxyphe nyl) pyridin-2-yl )piperidin-4-yl)a mino)methyl)py rimidin-2-yl)-N-hydroxyacrylam ide for-mate | 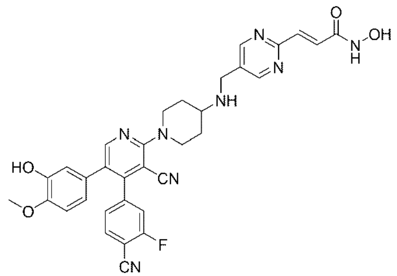 | $^1$H NMR (400 MHz, DMSO-d6) δ 11.03 (s, 1H), 9.22 (s, 1H), 8.99 (s, 1H), 8.82 (s, 2H), 8.41 (s, 1H), 8.14 (s, 1H), 7.95 (t, J = 7.6 Hz, 1H), 7.66 (dd, J = 10.0, 1.6 Hz, 1H), 7.40 - 7.24 (m, 2H), 7.14 (d, J = 15.6 Hz, 1H), 6.80 (d, J = 8.0 Hz, 1H), 6.58 - 6.38 (m, 2H), 4.18 (d, J = 13.2 Hz, 2H), 3.84 (s, 2H), 3.71 (s, 3H), 3.23 - 3.14 (m, 2H), 2.74 (s, 1H), 2.08 - 1.94 (m, 2H), 1.45 (m, 2H). | 621.2 |
| 107 | 4-(3-(3-Cyano-4 -(4-cyano-3-fluo rophenyl)-5-(3-h ydroxy-4-metho xyphenyl) pyridi n-2-yl)piperidin -4-yl) amino)pro pyl)-N-hydroxy benzamide formate | 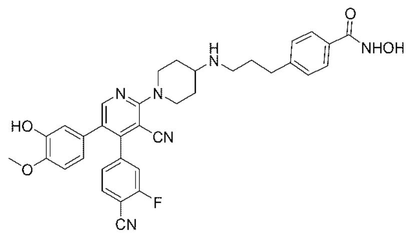 | $^1$H NMR (400 MHz, DMSO-d6) δ 11.11 (s, 1H), 9.00 (s, 1H), 8.42 (s, 1H), 8.24 (s, 1H), 7.95 (t, J = 7.6 Hz, 1H), 7.72 - 7.56 (m, 3H), 7.28 (dd, J = 8.4, 6.4 Hz, 3H), 6.80 (d, J = 8.0 Hz, 1H), 6.47 (d, J = 8.0 Hz, 2H), 4.19 (d, J = 13.2 Hz, 2H), 3.71 (s, 3H), 3.16 (t, J = 12.0 Hz, 2H), 2.82 (s, 1H), 2.67 (q, J = 8.4, 8.0 Hz, 4H), 1.97 (d, J = 12.4 Hz, 2H), 1.76 (p, J = 7.2 Hz, 2H), 1.51 - 1.34 (m, 2H). | 621.3 |

(continued)

| Exampl e | Chemical name | Structure | $^1$H NMR | MS( M+H )$^+$ |
|---|---|---|---|---|
| 108 | (E)-3-(4-(((1-(3-Cyano-4-(4-cya no-3-fluorophen yl)-5-(3-hydrox y-4-methoxyphe nyl) pyridin-2-yl )piperidin-4-yl)a mino)methyl)-2-methoxyphenyl) -N-hydroxyacryl amide formate | 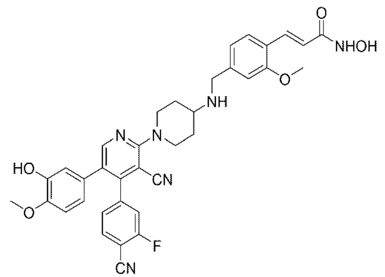 | $^1$H NMR (400 MHz, DMSO-d6) δ 10.69 (s, 1H), 8.90 (s, 1H), 8.41 (s, 1H), 8.19 (s, 1H), 7.95 (t, J = 7.6 Hz, 1H), 7.74 - 7.60 (m, 2H), 7.46 (d, J = 7.6 Hz, 1H), 7.27 (dd, J = 8.0, 1.6 Hz, 1H), 7.12 (s, 1H), 7.00 (d, J = 7.6 Hz, 1H), 6.80 (d, J = 8.0 Hz, 1H), 6.57 - 6.39 (m, 3H), 4.18 (dd, J = 10.4, 7.2 Hz, 2H), 3.87 (s, 3H), 3.84 (s, 2H), 3.71 (s, 3H), 3.25 - 3.11 (m, 2H), 2.77 (m, 1H), 2.10 - 1.95 (m, 2H), 1.48 (m, 2H). | 649.3 |
| 109 | 3-(4-(1-(3-Cyan o-4-(4-cyano-3-f luorophenyl)-5-( 3-hydroxy-4-me thoxyphenyl)pyr idin-2-yl)piperid in-4-yl)amino)m ethyl)phenyl)-N -N-hy-droxyprop iolamide formate | 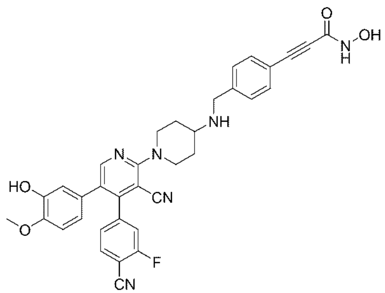 | $^1$H NMR (400 MHz, DMSO-d6) δ ppm : 11.30 (s, 1H), 9.35 (s, 1H), 9.01 (s, 1H), 8.44 (s, 1H), 7.97 (t, J = 7.4 Hz, 1H), 7.67 (dd, J = 10.2, 1.4 Hz, 1H), 7.61 (s, 3H), 7.28 (dd, J = 8.0, 1.4 Hz, 1H), 6.81 (d, J = 8.0 Hz, 1H), 6.46 (s, 2H), 4.27 (d, J = 13.0 Hz, 2H), 4.14 (s, 1H), 3.72 (s, 3H), 3.31 (s, 2H),3.14 (t, J = 12.6 Hz, 2H), 2.17 (s, 2H), 1.67 (s, 2H). | 617.2 |
| 110 | (E)-3-(4-((1-(3-Cyano-4-(4-cya no-3-fluorophen yl)-5-(5-fluoro-3 -methylbenzo[d] isoxazol-6-yl)py ridin-2-yl)piperi din-4-yl)amino) methyl)phenyl)-N-hydroxyacryl amide formate | 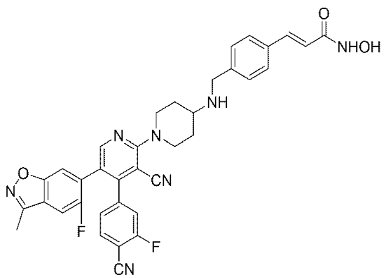 | $^1$H NMR (400 MHz, DMSO-d6) δ ppm : 10.73 (s, 1H), 9.02 (s, 1H), 8.52 (s, 1H), 8.16 (s, 1H), 7.91 (t, J = 7.4 Hz, 1H), 7.75 (dd, J = 16.2, 7.8 Hz, 1H), 7.61 (d, J = 8.8 Hz, 1H), 7.53 (d, J = 7.8 Hz, 2H), 7.49 - 7.40 (m, 3H), 7.31 (d, J = 8.0 Hz, 1H), 6.44 (d, J = 15.8 Hz, 1H), 4.30 (d, J = 13.4 Hz, 2H), 3.86 (s, 2H), 3.22 (s, 2H), 2.87 - 2.78 (m, 1H), 2.48 (s, 3H), 2.07 - 1.99 (m, 2H), 1.49 (q, J = 11.9, 10.7 Hz, 2H). | 646.2 |

(continued)

| Exampl e | Chemical name | Structure | $^1$H NMR | MS( M+H )$^+$ |
|---|---|---|---|---|
| 111 | (E)-3-(4-((1-(3-Cyano-4-(4-cya no-3-fluorophen yl)-5-(4-cyclopr opyl-3-hydroxy phenyl)pyridin-2-yl)piperidin-4 -yl)amino)meth yl)phenyl)-N-hy droxyacrylamid e formate | 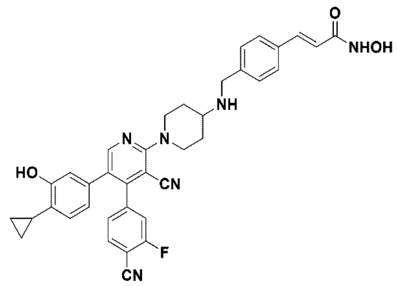 | $^1$H NMR (400 MHz, DMSO-d6) δ 10.72 (s, 1H), 9.32 (s, 1H), 9.01 (s, 1H), 8.40 (s, 1H), 7.94 (t, J = 7.4 Hz, 1H), 7.68 (dd, J = 10.0, 1.4 Hz, 1H), 7.64 - 7.29 (m, 5H), 7.24 (dd, J = 8.0, 1.4 Hz, 1H), 6.63 (d, J = 8.0 Hz, 1H), 6.52 - 6.31 (m, 3H), 4.18 (d, J = 13.0 Hz, 2H), 3.83 (s, 2H), 3.17 (t, J = 5.8 Hz, 2H), 2.76 (s, 1H), 1.99 (ddd, J = 10.6, 8.2, 4.8 Hz, 3H), 1.46 (d, J = 11.0 Hz, 2H), 0.94 - 0.75 (m, 2H), 0.64 - 0.50 (m, 2H). | 629.3 |
| 112 | (E)-3-(5-((1-(3-Cyano-4-(4-cya no-3-fluorophen yl)-5-(3-hydrox y-4-methoxyphe nyl) pyridin-2-yl )piperidin-4-yl)a mino)methyl)thi azol-2-yl)-N-hy droxyacrylamid e formate | 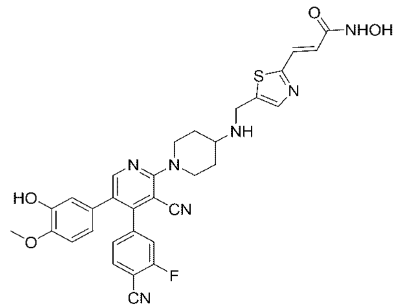 | $^1$H NMR (400 MHz, Methanol-d4) δ 8.40 (s, 1H), 8.39 (s, 1H), 7.84 (s, 1H), 7.73 (t, J = 7.2 Hz, 1H), 7.62 (d, J = 15.6 Hz, 1H), 7.36 (dd, J = 9.6, 1.6 Hz, 1H), 7.23 (dd, J = 8.0, 1.6 Hz, 1H), 6.85 - 6.78 (m, 1H), 6.73 (d, J = 15.6 Hz, 1H), 6.50 (d, J = 6.8 Hz, 2H), 4.36 (d, J = 13.2 Hz, 2H), 4.26 (s, 2H), 3.81 (s, 3H), 3.17 (t, J = 12.0 Hz, 2H), 3.09 - 2.97 (m, 1H), 2.19 - 2.09 (m, 2H), 1.65 (qd, J = 12.0, 4.0 Hz, 2H). | 626.2 |
| 113 | (E)-3-(4-(((1-(3-Cyano-4-(4-cya no-3-fluorophen yl)-5-(4-cyano-3 -hydroxyphenyl )pyridin-2-yl)pi peridin-4-yl)ami no)methyl)phen yl)-N-hydro-xyac rylamide formate | 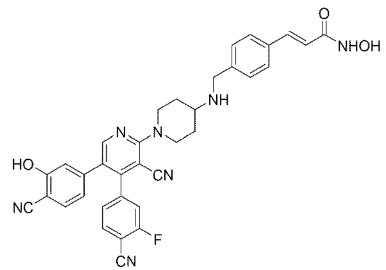 | $^1$H NMR (400 MHz, DMSO-d6) δ 10.72 (s, 1H), 9.01 (s, 1H), 8.50 - 8.43 (m, 1H), 8.14 (s, 1H), 7.96 (t, J = 7.4 Hz, 1H), 7.74 (d, J = 10.0 Hz, 1H), 7.51 (s, 3H), 7.46 (d, J = 26.6 Hz, 2H), 7.26 (dd, J = 8.0, 1.4 Hz, 1H), 6.67 (d, J = 7.8 Hz, 1H), 6.63 (d, J = 1.6 Hz, 1H), 6.44 (d, J = 15.9 Hz, 1H), 4.25 (d, J = 12.9 Hz, 2H), 3.84 (d, J = 10.9 Hz, 2H), 3.20 (d, J = 12.1 Hz, 2H), 2.79 (s, 1H), 2.01 (s, 2H), 1.47 (s, 2H). | 614.2 |
| 114 | (E)-3-(3-(3-Cya no-4-(4-cyano-3 -fluorophenyl)-5 -(3-hydroxy-4-methoxyphenyl) pyri-din-2-yl)pip eridin-4-yl)amin o)methyl)phenyl )-N-hydro-xyacr ylamide formate | 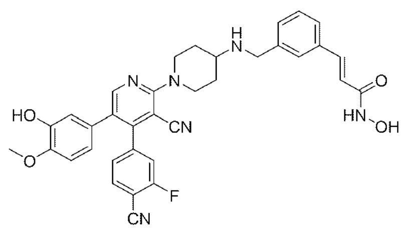 | $^1$H NMR (400 MHz, DMSO-d6) δ ppm 10.80 (s, 1H), 9.01 (s, 2H), 8.43 (s, 1H), 7.96 (t, J = 7.4 Hz, 1H), 7.77 - 7.63 (m, 2H), 7.55 - 7.37 (m, 4H), 7.28 (dd, J = 8.0, 1.6 Hz, 1H), 6.81 (d, J = 8.0 Hz, 1H), 6.55 - 6.42 (m, 3H), 4.23 (d, J = 13.0 Hz, 2H), 3.99 (s, 2H), 3.71 (s, 3H), 3.16 (t, J = 12.2 Hz, 2H), 3.01 (s, 1H), 2.11 (d, J = 12.2 Hz, 2H), 1.58 (d, J = 11.8 Hz, 2H). | 619.2 |

(continued)

| Exampl e | Chemical name | Structure | $^1$H NMR | MS( M+H )$^+$ |
|---|---|---|---|---|
| 115 | 2-(2-(1-(3-Cyan o-4-(4-cyano-3-f luorophenyl)-5-( 3-hydroxy-4-me thoxyphenyl)pyr idin-2-yl)piperid in-4-yl)amino)et hyl)amino)-N-h ydroxypyrimidi ne-5-carboxami de formate | 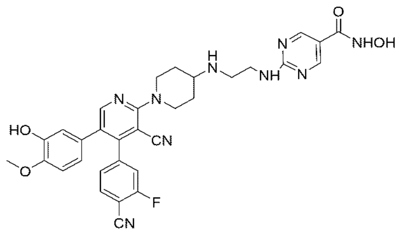 | $^1$H NMR (400 MHz, Methanol-d4) δppm 8.71 (s, 2H), 8.53 (s, 1H), 8.45 (s, 1H), 7.76 (t, J = 7.4 Hz, 1H), 7.38 (dd, J = 9.8, 1.3 Hz, 1H), 7.25 (dd, J = 7.8, 1.4 Hz, 1H), 6.84 (d, J = 8.6 Hz, 1H), 6.53 (d, J = 6.6 Hz, 2H), 4.44 (d, J = 13.4 Hz, 2H), 3.83 (s, 3H), 3.77 (t, J = 5.9 Hz, 2H), 3.38 (s, 1H), 3.31 - 3.25 (m, 2H), 3.18 (t, J = 12.6 Hz, 2H), 2.23 (d, J = 12.0 Hz, 2H), 1.93 - 1.65 (m, 2H). | 624.2 |
| 116 | (E)-3-(4-(1-(3-C yano-4-(4-cyano -3-fluorophenyl) -5-(3-hydroxy-4 -(trifluorometho xy) phenyl)pyrid in-2-yl)piperidin -4-yl)amino)met hyl)phenyl)-N-h ydroxyacrylami de formate | 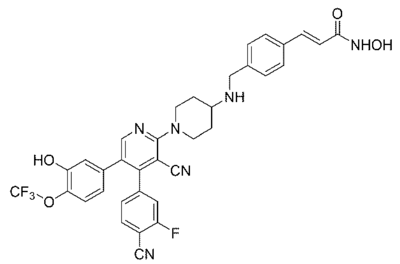 | $^1$H NMR (400 MHz, Methanol-d4) δ ppm 8.53 (s,1H), 8.48 (d, J = 13.8 Hz, 2H), 7.76 (dd, J = 7.8, 6.8 Hz, 1H), 7.62 (t, J = 6.8 Hz, 2H), 7.51 (d, J = 7.8 Hz, 2H), 7.41 (dd, J = 9.8, 1.4 Hz, 1H), 7.24 (dd, J = 8.0, 1.6 Hz, 1H), 7.11 (dd, J = 8.6, 1.6 Hz, 1H), 6.68 (d, J = 2.2 Hz, 1H), 6.59 (dd, J = 8.2, 2.0 Hz, 1H), 6.51 (d, J = 15.8 Hz, 1H), 4.48 (d, J = 13.2 Hz, 2H), 4.17 (s, 2H), 3.27 (s, 1H), 3.18 (t, J = 12.6 Hz, 2H), 2.33 - 2.20 (m, 2H), 1.86 - 1.68 (m, 2H). | 673.2 |
| 117 | (E)-3-(5-((1-(3-Cyano-4-(4-cya no-3-fluorophen yl)-5-(3-hydrox y-4-methoxyphe nyl) pyridin-2-yl )piperidin-4-yl)a mino)methyl)py ridin-2-yl)-N-hy droxyacrylamid e formate | 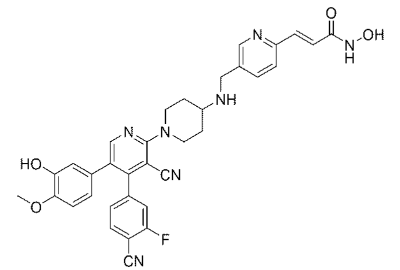 | $^1$H NMR (400 MHz, Methanol-d4) δppm 8.69 (s, 1H), 8.46 (d, J = 11.6 Hz, 2H), 8.01 - 7.92 (m, 1H), 7.76 (dd, J = 7.8, 6.6 Hz, 1H), 7.69 - 7.58 (m, 2H), 7.39 (dd, J = 9.6, 1.4 Hz, 1H), 7.26 (dd, J = 8.0, 1.4 Hz, 1H), 6.94 (d, J = 15.4 Hz, 1H), 6.88 - 6.75 (m, 1H), 6.62 - 6.42 (m, 2H), 4.43 (d, J = 13.2 Hz, 2H), 4.17 (s, 2H), 3.84 (s, 3H), 3.20 (t, J = 12.6 Hz, 3H), 2.34 - 2.11 (m, 2H), 1.85 - 1.67 (m, 2H). | 620.2 |
| 118 | (E)-3-(4-((1-(3-Cyano-4-(4-cya nophenyl)-5-(3-hydroxy-4-meth oxyphenyl)pyrid in-2-yl)piperidin -4-yl)amino) met hyl)phenyl)-N-h ydroxya-crylami de formate | 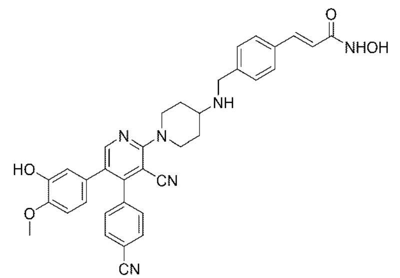 | $^1$H NMR (400 MHz, DMSO-d6) δ ppm : 10.73 (s, 1H), 8.96 (s, 1H), 8.39 (s, 1H), 8.17 (s, 1H), 7.86 (d, J = 8.0 Hz, 2H), 7.53 (td, J = 10.4, 9.0, 7.8 Hz, 3H),7.48 (td, J = 19.8, 19.0, 7.8 Hz, 4H), 6.77 (d, J = 8.2 Hz, 1H), 6.49 - 6.39 (m, 3H), 4.22 - 4.12 (m, 2H), 3.86 (s, 2H), 3.70 (s, 3H), 3.14 (s, 2H), 2.80 (tt, J = 9.7, 4.0 Hz, 1H), 2.01 (dd, J = 13.1, 3.9 Hz, 2H), 1.56 - 1.41 (m, 2H). | 601.2 |

(continued)

| Exampl e | Chemical name | Structure | $^1$H NMR | MS( M+H )$^+$ |
|---|---|---|---|---|
| 119 | (E)-3-(4-((2-(3-Cyano-4-(4-cya no-3-fluorophen yl)-5-(3-hydrox y-4-methoxyphe nyl) pyridin-2-yl )-2-azabicyclo [2 .2.1]heptan-5-yl )amino) methyl) phenyl)-N-hydr oxya-crylamide formate | 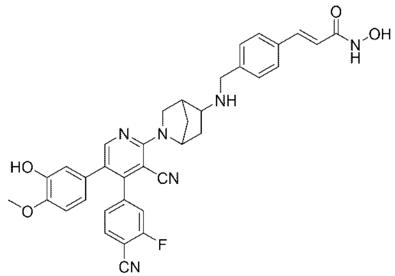 | $^1$H NMR (400 MHz, DMSO-$d_6$) δ 10.72 (s, 1H), 8.98 (m, 2H), 8.30 (m, 1H), 7.94 (s, 1H), 7.67 - 7.27 (m, 6H), 6.77 (d, J = 8.6 Hz, 1H), 6.50 - 6.31 (m, 3H), 4.79 (m, 1H), 4.00 - 3.60 (m, 6H), 3.17 (t, J = 9.8 Hz, 1H), 2.78 (m, 2H), 2.20 - 1.81 (m, 2H), 1.71 (m, 1H), 1.38 (m, 1H). | 631.2 |
| 120 | 2-(4-(2-((1-(3-C yano-4-(4-cyano -3-fluorophenyl) -5-(3-hydroxy-4 -methoxyphenyl ) pyridin-2-yl)pi peridin-4-yl) ami no)ethyl)phenyl )-N-hy-droxyacet amide formate | 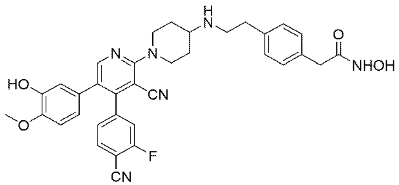 | $^1$H NMR (400 MHz, DMSO-d6) δ ppm : 8.51 (s, 1H), 8.43 (s, 1H), 7.78 - 7.69 (m, 1H), 7.36 (dd, J = 9.8, 1.4 Hz, 1H), 7.33 - 7.19 (m, 5H), 6.85 - 6.78 (m, 1H), 6.56 - 6.46 (m, 2H), 4.41 (d, J = 13.4 Hz, 2H), 3.81 (s, 3H), 3.39 (s, 2H), 3.25 (s, 2H), 3.15 (t, J = 12.6 Hz, 3H), 2.97 (s, 2H), 2.21 (d, J = 11.2 Hz, 2H), 1.75 (d, J = 11.0 Hz, 2H). | 621.2 |
| 121 | 4-(4-((1-(3-Cya no-4-(4-cyano-3 -fluorophenyl)-5 -(3-hydroxy-4-methoxyphenyl) pyri-din-2-yl)pip eridin-4-yl)amin o)methyl)piperi din-1-yl)-N-hyd roxybenzamide formate | 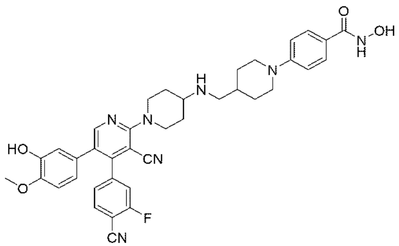 | $^1$H NMR (400 MHz, Methanol-d4) δ 8.42 (s, 1H), 7.83 - 7.68 (m, 1H), 7.64 (d, J = 8.6 Hz, 2H), 7.36 (dd, J = 9.7, 1.4 Hz, 1H), 7.23 (dd, J = 8.0, 1.4 Hz, 1H), 6.97 (d, J = 8.6 Hz, 2H), 6.82 (d, J = 8.6 Hz, 1H), 6.51 (d, J = 6.4 Hz, 2H), 4.41 (d, J = 8.2 Hz, 2H), 3.92 (d, J = 12.8 Hz, 2H), 3.81 (s, 3H), 3.16 (t, J = 12.8 Hz, 3H), 3.01 - 2.76 (m, 4H), 2.19 (d, J = 11.6 Hz, 2H), 1.90 (d, J = 13.0 Hz, 3H), 1.72 (d, J = 8.2 Hz, 2H), 1.41 (t, J = 5.4 Hz, 2H). | 676.3 |
| 123 | (E)-3-(4-((1-(5-Cyano-4-(4-cya no-3-fluorophen yl)-5'-hydroxy-6 '-methoxy-[3,3'-bipyridin]-6-yl) piperidin-4-yl)a mino)methyl)ph enyl)-N-hy-drox yacrylamide trifluoroa-cetate | 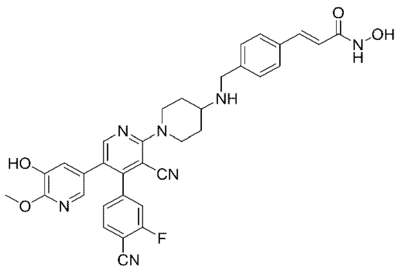 | $^1$H NMR (400 MHz, Methanol-d4) δppm 8.46 (s, 1H), 7.79 (t, J = 7.4 Hz, 1H), 7.67 (d, J = 7.8 Hz, 2H), 7.66 - 7.52 (m, 3H), 7.43 (dd, J = 9.8, 1.4 Hz, 1H), 7.33 (d, J = 2.0 Hz, 1H), 7.25 (dd, J = 7.8, 1.4 Hz, 1H), 6.77 (d, J = 2.2 Hz, 1H), 6.53 (d, J = 15.8 Hz, 1H), 4.50 (d, J = 13.6 Hz, 2H), 4.33 (s, 2H), 3.92 (s, 3H), 3.51 (q, J = 9.4, 5.8 Hz, 1H), 3.19 (t, J = 12.6 Hz, 2H), 2.43 - 2.24 (m, 2H), 1.88 (ddt, J = 22.0, 13.6, 7.2 Hz, 2H). | 620.2 |

(continued)

| Exampl e | Chemical name | Structure | $^1$H NMR | MS( M+H )$^+$ |
|---|---|---|---|---|
| 124 | (E)-3-(4-(((1-(3-Cyano-4-(4-cya no-3-fluorophen yl)-5-(4-(difluor omethoxy)-3-hy droxyphenyl)py ridin-2-yl)) piper idin-4-yl)amino) methyl) phenyl)-N-hydroxyacryl amide formate | 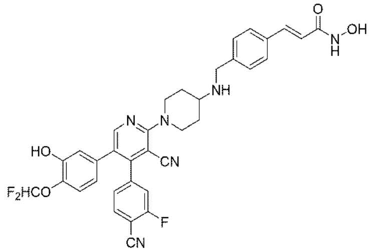 | $^1$H NMR (400 MHz, DMSO-d6) δ 10.79 (s, 1H), 9.06 (s, 1H), 8.48 (s, 1H), 7.98 (t, J = 7.4 Hz, 1H), 7.71 (d, J = 10.0 Hz, 1H), 7.62 (d, J = 7.9 Hz, 2H), 7.52 (d, J = 8.0 Hz, 2H), 7.47 (d, J = 15.8 Hz, 1H), 7.29 (dd, J = 8.0, 1.4 Hz, 1H), 7.05 - 6.95 (m, 2H), 6.62 (d, J = 2.1 Hz, 1H), 6.59 - 6.51 (m, 2H), 6.48 (s, 1H), 4.31 (d, J = 13.1 Hz, 2H), 4.15 (s, 2H), 3.33 (s, 1H), 3.16 (t, J = 12.5 Hz, 2H), 2.26 - 2.12 (m, 2H), 1.77 - 1.56 (m, 2H). | 655.2 |
| 125 | 2-(3-(3-Cyano-4 -(4-cyano-3-fluo rophenyl)-5-(3-h ydroxy-4-metho xyphenyl) pyridi n-2-yl)piperidin -4-yl) amino)pro pyl)-N-hydroxyt hiazole-5-carbo xamide for-mate | 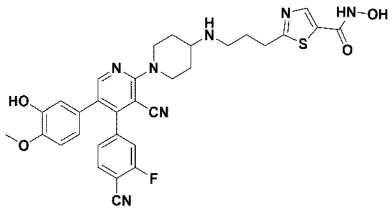 | $^1$H NMR (400 MHz, DMSO-d6) δ 9.02 (s, 1H),8.42 (s, 1H), 8.23 (s, 1H), 8.09 (s, 1H), 7.95 (t, J = 7.4 Hz, 1H), 7.66 (dd, J = 10.0, 1.4 Hz, 1H), 7.27 (dd, J = 8.0, 1.4 Hz, 1H), 6.80 (d, J = 8.0 Hz, 1H), 6.46 (s, 2H), 4.19 (d, J = 13.0 Hz, 2H), 3.71 (s, 3H), 3.16 (t, J = 12.0 Hz, 2H), 3.06 (t, J = 7.4 Hz, 2H), 2.83 (s, 1H), 2.73 (t, J = 6.9 Hz, 2H), 2.06 - 1.82 (m, 4H), 1.44 (q, J = 11.0 Hz, 2H). | 628.2 |
| 126 | 6-(1-(3-Cyano-4 -(4-cyano-3-fluo rophenyl)-5-(3-h ydroxy-4-metho xyphenyl) pyridi n-2-yl)piperidin -4-yl) amino)met hyl)-N-hydroxy benzothiophene-2-carboxa-mide formate | 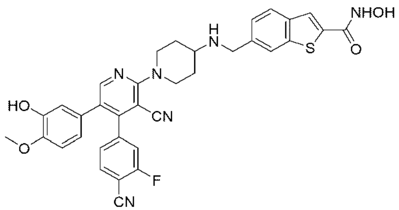 | $^1$H NMR (400 MHz, Methanol-d4) δ 8.45 (s, 1H), 8.39 (s, 1H), 8.12 (s, 1H), 8.01 (d, J = 8.4 Hz, 1H), 7.88 (s, 1H), 7.75 (t, J = 7.2 Hz, 1H), 7.57 (d, J = 8.4 Hz, 1H), 7.37 (d, J = 9.6 Hz, 1H), 7.23 (dd, J = 8.0, 1.2 Hz, 1H), 6.89 - 6.77 (m, 1H), 6.51 (d, J = 6.4 Hz, 2H), 4.47 (d, J = 12.0 Hz, 4H), 4.03 (p, J = 7.6 Hz, 1H), 3.81 (s, 3H), 3.19 (t, J = 12.8 Hz, 2H), 2.36 (d, J = 12.4 Hz, 2H), 1.97 - 1.82 (m, 2H). | 649.2 |
| 127 | (E)-3-(4-(((1-(5-(4-Bromo-3-hyd roxyphenyl)-3-c yano-4-(4-cyano -3-fluorophenyl) pyridin-2-yl)pip eridin-4-yl)amin o)methyl)phenyl )-N-hydroxyacr ylamide formate | 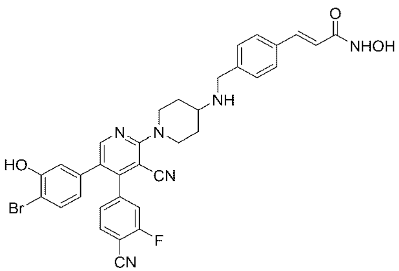 | $^1$H NMR (400 MHz, DMSO-d6) δ ppm : 10.73 (s, 1H), 9.00 (s, 1H), 8.44 (s, 1H), 8.22 - 8.15 (m, 1H), 7.96 (t, J = 7.4 Hz, 1H), 7.53 (d, J = 7.8 Hz, 2H), 7.51 - 7.40 (m, 3H), 7.37 (d, J = 8.2 Hz, 1H), 7.26 (dd, J = 8.0, 1.4 Hz, 1H), 6.59 (d, J = 2.2 Hz, 1H), 6.51 - 6.40 (m, 2H), 4.54 (s, 2H), 4.23 (d, J = 12.8 Hz, 2H), 3.19 (t, J = 11.8 Hz, 2H), 2.83 (s, 1H), 2.03 (d, J = 12.4 Hz, 2H), 1.49 (q, J = 11.8, 11.54Hz, 2H). | 667.2 |

(continued)

| Exampl e | Chemical name | Structure | $^1$H NMR | MS( M+H )$^+$ |
|---|---|---|---|---|
| 128 | 2-(4-((1-(3-Cya no-4-(4-cyano-3 -fluorophenyl)-5 -(3-hydroxy-4-methoxyphenyl) pyri-din-2-yl)piperidin-4-yl)amin o) methyl)-2-met hoxyphenox-y)-N-hydroxyaceta mide for-mate | 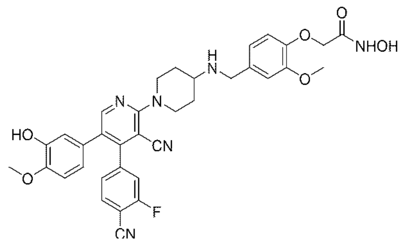 | 8.0 Hz, 2H), 4.39 (s, 2H), 4.26 - 4.10 (m, 2H), 3.79 (d, J = 2.6 Hz, 5H), 3.71 (s, 3H), 3.16 (s, 2H), 2.88 - 2.76 (m, 1H), 2.02 (dd, J = 13.2, 4.0 Hz, 2H), 1.49 (q, J = 11.4, 9.8 Hz, 2H). | 653.2 |
| 129 | (E)-3-(4-(1-(3-C yano-4-(4-cyano -3-fluorophenyl) -5-(3-hydroxy-4 -(trifluoromethy l) phenyl)pyridin -2-yl)piperidin-4-yl)amino)met hyl)phenyl)-N-h ydroxyacrylami de formate | 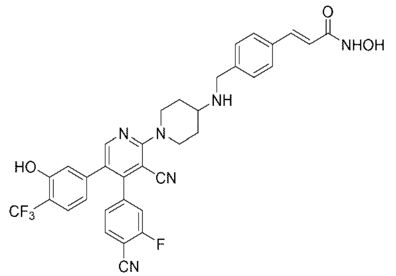 | $^1$H NMR (400 MHz, Methanol-d4) δ ppm 8.52(s, 1H), 8.48 (d, J = 12.4 Hz, 1H), 7.76 (t, J = 7.2 Hz, 1H), 7.67 - 7.34 (m, 7H), 7.21 (dd, J = 8.0, 1.5 Hz, 1H), 6.65 (d, J = 8.6 Hz, 2H), 6.50 (d, J = 15.8 Hz, 1H), 4.50 (d, J = 13.4 Hz, 2H), 4.15 (s, 2H), 3.26 (s, 1H), 3.18 (t, J = 12.6 Hz, 2H), 2.35 - 2.21 (m, 2H), 1.83 - 1.71 (m, 2H). | 657.2 |
| 130 | (E)-3-(4-((1-(3-Cyano-4-(4-cya no-3-fluorophen yl)-5-(2-fluoro-5 -hydroxy-4-met hoxyphenyl)pyri din-2-yl)piperidi n-4-yl)amino)m ethyl)phenyl)-N -hydroxyacryla mide formate | 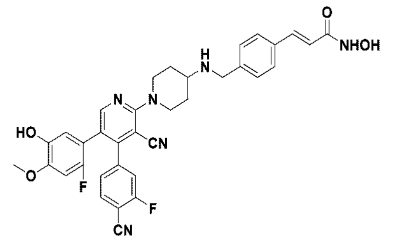 | $^1$H NMR (400 MHz, Methanol-d4) δ 8.48 (s, 1H), 8.38 (s, 1H), 7.73 (t, J = 7.4 Hz, 1H), 7.67 - 7.41 (m, 5H), 7.37 (d, J = 9.7 Hz, 1H), 7.22 (d, J = 8.0 Hz, 1H), 6.61 (dd, J = 16.6, 9.2 Hz, 2H), 6.52 (d, J = 15.8 Hz, 1H), 4.49 (d, J = 13.6 Hz, 2H), 4.20 (s, 2H), 3.80 (s, 3H), 3.18 (t, J = 12.8 Hz, 3H), 2.28 (d, J = 12.2 Hz, 2H), 1.80 (d, J = 12.0 Hz, 2H). | 637.2 |
| 131 | (E)-3-(4-(((1-(3-Cyano-4-(4-cya no-3-fluorophen yl)-5-(3-hydrox y-4-(hydroxyme thyl) phenyl)pyri din-2-yl)piperidi n-4-yl)amino)m ethyl)phenyl)-N -hydroxyacryla mide formate | 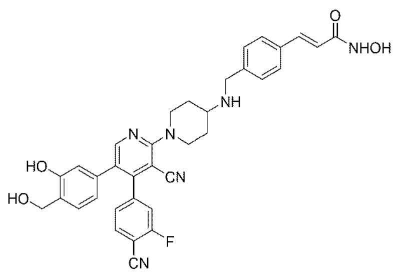 | $^1$H NMR (400 MHz, Methanol-d4)δ ppm : 8.44(s, 1H),8.28(s, 1H), 7.73 (dd, J = 7.9, 6.7 Hz, 1H), 7.65 (d, J = 7.9 Hz, 2H), 7.64 - 7.43 (m, 1H), 7.37 (dd, J = 9.7, 1.4 Hz, 2H), 7.27 (m, 1H), 7.18 (m, 1H) , 7.14 (m, 1H), 6.58 - 6.46 (m, 3H), 4.59 (s, 2H), 4.46 (d, J = 13.4 Hz, 2H), 4.23 (s, 2H), 3.37 (s, 1H), 3.18 (t, J = 12.7 Hz, 2H), 2.34 - 2.25 (m, 2H), 1.89 - 1.75 (m, 2H). | 619.2 |

(continued)

| Exampl e | Chemical name | Structure | $^1$H NMR | MS( M+H )$^+$ |
|---|---|---|---|---|
| 132 | (E)-3-(4-(((1-(3-Cyano-4-(4-cya no-3-fluorophen yl)-5-(3-hydrox y-4-methoxyphe nyl) pyridin-2-yl )piperidin-4-yl)a mino)methyl)-3-methoxyphenyl) -N-hydroxyacryl amide formate | 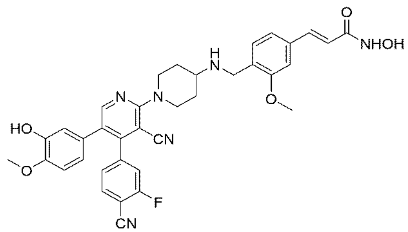 | $^1$H NMR (400 MHz, DMSO-d6) δ 10.71 (s, 1H), 9.00 (s, 1H), 8.41 (s, 1H), 8.19 (s, 1H), 7.95 (t, J = 7.4 Hz, 1H), 7.66 (d, J = 10.0 Hz, 1H), 7.50 - 7.38 (m, 2H), 7.29 - 7.24 (m, 1H), 7.20 - 7.10 (m, 2H), 6.80 (d, J = 8.0 Hz, 1H), 6.53 - 6.40 (m, 3H), 4.18 (dt, J = 13.2, 3.8 Hz, 2H), 3.85 (s, 3H), 3.80 (s, 2H), 3.71 (s, 3H), 3.17 (s, 2H), 2.84 - 2.71 (m, 1H), 2.05 - 1.92 (m, 2H), 1.47 (d, J = 11.0 Hz, 2H). | 649.3 |
| 133 | (E)-3-(5-(((1-(3-Cyano-4-(4-cya no-3-fluorophen yl)-5-(3-hydrox y-4-methoxyphe nyl) pyridin-2-yl )piperidin-4-yl)a mino)methyl)py razin-2-yl)-N-hy droxyacrylamid e trifluor-oacetate | 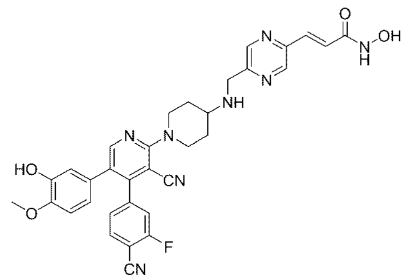 | $^1$H NMR (400 MHz, DMSO-d6) δ ppm : δ 11.04 (s, 1H), 9.24 (s, 1H), 9.03 (s, 1H), 8.94 (s, 1H), 8.82 (s, 1H), 8.46 (s, 1H), 7.97 (t, J = 7.4 Hz, 1H), 7.71 = 7.60 (m, 2H), 7.28 (dd, J = 8.0, 1.4 Hz, 1H), 7.07 (d, J = 15.4 Hz, 1H), 6.82 (d, J = 8.1 Hz, 1H),6.46 (s, 2H), 4.54 (s, 2H), 4.32 (d, J = 13.0 Hz, 2H), 3.72 (s, 3H), 3.50 (s, 1H), 3.13 (t, J = 12.5 Hz, 2H), 2.29 - 2.20 (m, 2H), 1.85 - 1.71 (m, 2H). | 621.2 |
| 134 | (E)-3-(4-(((1-(3-Cyano-4-(4-cya no-2-fluorophen yl)-5-(3-hydrox y-4-methoxyphe nyl) pyridin-2-yl )piperidin-4-yl)a mino)methyl)ph enyl)-N-hy-drox yacrylamide formate | 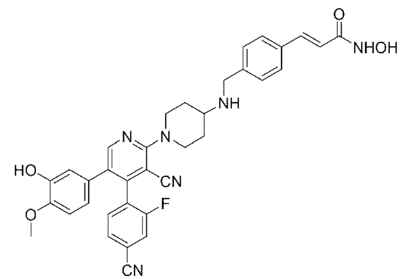 | $^1$H NMR (400 MHz, DMSO-d6) δ 10.80 (s, 1H), 9.04 (s, 2H), 8.48 (s, 1H), 7.97 (d, J = 9.6 Hz, 1H), 7.80 (d, J = 8.0 Hz, 1H), 7.68 - 7.60 (m, 3H), 7.57 (d, J = 8.0 Hz, 2H), 7.47 (d, J = 15.6 Hz, 1H), 6.80 (d, J = 8.4 Hz, 1H), 6.59 - 6.39 (m, 3H), 4.37 - 4.11 (m, 4H), 3.71 (s, 3H), 3.27 (s, 1H),3.14 (m, 2H), 2.22 (d, J = 12.4 Hz, 2H), 1.73 (s, 2H). | 619.2 |
| 135 | (E)-3-(2-(((1-(3-Cyano-4-(4-cya no-3-fluorophen yl)-5-(3-hydrox y-4-methoxyphe nyl) pyridin-2-yl )piperidin-4-yl)a mino)methyl)py rimidin-5-yl)-N-hydroxyacrylam ide for-mate | 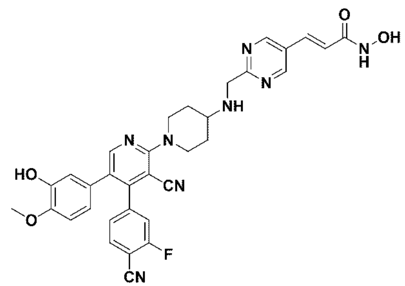 | $^1$H NMR (400 MHz, Methanol-d4) δppm 9.02 (s, 2H), 8.52 (s, 1H), 8.11 (d, J = 9.6 Hz, 1H), 7.74 (t, J = 7.2 Hz, 1H), 7.58 (d, J = 15.8 Hz, 1H), 7.37 (d, J = 9.6 Hz, 1H), 7.23 (d, J = 7.8 Hz, 1H), 6.81 (d, J = 8.4 Hz, 1H), 6.72 (d, J = 15.8 Hz, 1H), 6.51 (d, J = 6.5 Hz, 2H), 4.46 (d, J = 5.8 Hz, 3H), 4.42 (s, 1H), 3.81 (s, 3H), 3.37 (d, J = 11.8 Hz, 1H), 3.18 (t, J = 12.6 Hz, 2H), 2.28 (d, J = 12.0 Hz, 2H), 1.93 - 1.71 (m, 2H). | 621.2 |

(continued)

| Exampl e | Chemical name | Structure | $^1$H NMR | MS( M+H )$^+$ |
|---|---|---|---|---|
| 136 | (E)-3-(4-(((1-(3-Cyano-4-(4-cya no-3-fluorophen yl)-5-(1-methyl-1H-benzo[d]imi dazol-5-yl))pyri din-2-yl)piperidi n-4-yl)amino)m ethyl)phenyl)-N -hydroxyacryla mide formate | 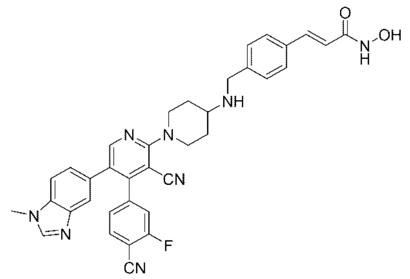 | $^1$H NMR (400 MHz, MeOD) δppm 8.52 (d, J = 3.6 Hz, 1H), 8.12 (s, 1H), 7.67 (t, J = 7.2 Hz, 1H), 7.61 (d, J = 7.8 Hz, 2H), 7.58 - 7.48 (m, 2H), 7.46 (d, J = 8.4 Hz, 3H), 7.43 - 7.35 (m, 2H), 7.24 (d, J = 7.8 Hz, 1H), 7.05 (d, J = 8.4 Hz, 1H), 6.50 (d, J = 15.8 Hz, 1H), 4.46 (d, J = 13.2 Hz, 2H), 4.10 (s, 2H), 3.87 (s, 3H), 3.23 - 3.13 (m, 3H), 2.26 - 2.19 (m, 2H), 1.75 (td, J = 13.1, 9.4 Hz, 2H). | 627.3 |
| 137 | (E)-3-(4-(((1-(3-Cyano-4-(4-cya no-3-fluorophen yl)-5-(3,4-dihyd roxyphenyl) pyri din-2-yl)piperidin-4-yl) amino)m ethyl)phenyl)-N -hy-droxyacryla mide hydrochlor-ide | 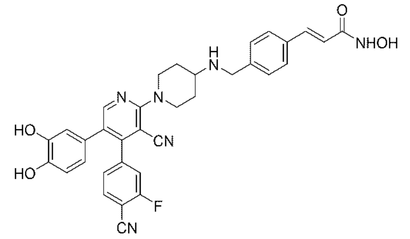 | $^1$H NMR (400 MHz, DMSO-d6) δppm: 10.78 (s, 1H), 9.03 (d, J = 8.4 Hz, 2H), 8.92 (s, 1H), 8.43 (s, 1H), 7.96 (t, J = 7.6 Hz, 1H), 7.71 - 7.51 (m, 5H), 7.47 (d, J = 15.8 Hz, 1H), 7.27 (d, J = 8.4 Hz, 1H), 6.61 (d, J = 8.0 Hz, 1H), 6.50 (d, J = 16.0 Hz, 1H), 6.41 (d, J = 2.4 Hz, 1H), 6.33 (dd, J = 8.0, 2.1 Hz, 1H), 4.27 (d, J = 12.8 Hz, 2H), 4.14 (s, 2H), 3.13 (t, J = 12.4 Hz, 2H), 2.49 (s, 1H), 2.19 (d, J = 11.6 Hz, 2H), 1.70 (s, 2H). | 605.2 |
| 138 | (E)-3-(4-(((7-(3-Cyano-4-(4-cya no-3-fluorophen yl)-5-(3-hydrox y-4-methoxyphe nyl) pyridin-2-yl )-2,7-diazaspiro [ 3.5]nonan-2-yl) methyl)phenyl)-N-hydroxyacryl amide formate | 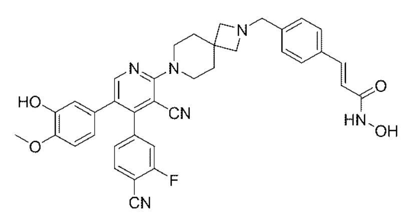 | $^1$H NMR (400 MHz, DMSO-d6) δ ppm : 10.73 (s, 1H), 8.99 (s, 1H), 8.41 (s, 1H), 8.14 (d, J = 3.0 Hz, 1H), 7.95 (t, J = 7.4 Hz, 1H), 7.65 (d, J = 10.0 Hz, 1H), 7.52 (d, J = 7.8 Hz, 2H), 7.44 (d, J = 15.6 Hz, 1H), 7.35 (d, J = 7.8 Hz, 2H), 7.27 (d, J = 8.0 Hz, 1H), 6.80 (d, J = 8.2 Hz, 1H), 6.45 (s, 3H), 3.71 (s, 5H), 3.59 (d, J = 11.0 Hz, 4H), 3.15 (s, 4H), 1.85 (t, J = 5.3 Hz, 4H). | 645.3 |
| 139 | (E)-3-(4-(((1-(3-Cyano-4-(4-cya no-3-fluorophen yl)-5-(3-hydrox yphenyl)pyridin -2-yl) piperidin-4-yl)amino)met hyl) phenyl)-N-h ydroxyacrylami de formate | 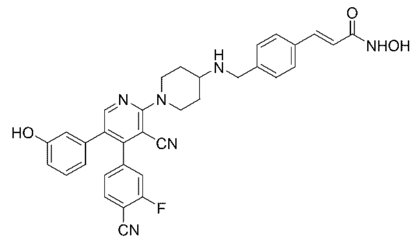 | $^1$H NMR (400 MHz, DMSO-d6) δ ppm : 10.72 (s, 1H), 9.42 (s, 1H), 8.44 (s, 1H), 8.16 (d, J = 1.6 Hz, 1H), 7.95 (t, J = 7.4 Hz, 1H), 7.65 (d, J = 10.0 Hz, 1H), 7.52 (d, J = 7.8 Hz, 2H), 7.44 (t, J = 8.6 Hz, 3H), 7.29 (d, J = 8.0 Hz, 1H), 7.04 (t, J = 7.8 Hz, 1H), 6.63 (dd, J = 8.2, 2.2 Hz, 1H), 6.46 (s, 3H), 4.21 (dt, J = 13.4, 4.0 Hz, 2H), 3.85 (s, 2H), 3.18 (t, J = 11.8 Hz, 2H), 2.79 (s, 1H), 2.01 (dd, J = 13.2, 3.8 Hz, 2H), 1.48 (q, J = 10.4 Hz, 2H). | 589.2 |

(continued)

| Exampl e | Chemical name | Structure | $^1$H NMR | MS( M+H )$^+$ |
|---|---|---|---|---|
| 140 | (E)-3-(4-(((1-(3-Cyano-4-(4-cya no-3-fluorophen yl)-5-(2,3-dihyd roxy-4-methoxy phenyl)pyridin-2-yl)piperidin-4 -yl)amino)meth yl)phenyl)-N-hy droxyacrylamid e formate | 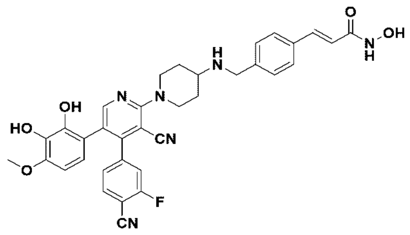 | $^1$H NMR (400 MHz, Methanol-d4) δ ppm: 8.52 (s,1H), 8.38 (s, 1H), 7.80 - 7.40 (m, 6H), 7.32 (d, J = 9.8 Hz, 1H), 7.22 (d, J = 8.0 Hz, 1H), 6.54 (d, J = 15.8 Hz, 1H), 6.43 (s, 2H), 4.44 (d, J = 13.2 Hz, 2H), 4.31 (d, J = 10.6 Hz, 2H), 3.81 (s, 3H), 3.51 (s, 1H), 3.20 (d, J = 12.8 Hz, 2H), 2.34 (d, J = 11.8 Hz, 2H), 1.89 (d, J = 12.4 Hz, 2H). | 635.2 |
| 141 | (E)-3-(4-(((1-(3-Cyano-4-(4-cya no-3-fluorophen yl)-5-(1-methyl-1H-benzo[d][1,2 ,3] triazol-5-yl)p yridin-2-yl)pipe ridin-4-yl)amino )methyl)phenyl) -N-hydroxyacryl amide formate | 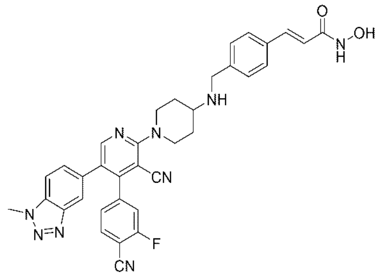 | $^1$H NMR (400 MHz, DMSO-d6) δ 10.73 (s, 1H), 9.02 (s, 1H), 8.58 (s, 1H), 7.89 (s, 2H), 7.70 (dd, J = 11.2, 9.0 Hz, 2H), 7.55 (d, J = 7.8 Hz, 2H), 7.50 - 7.41 (m, 3H), 7.33 (d, J = 8.0 Hz, 1H), 7.14 (dd, J = 8.4, 1.4 Hz, 1H), 6.45 (d, J = 2.8 Hz, 1H), 4.26 (s, 5H), 3.92 (s, 2H), 3.21 (t, J = 12.0 Hz, 2H), 2.90 (s, 1H), 2.07 (d, J = 6.0 Hz, 2H), 1.54 (d, J = 11.4 Hz, 2H). | 628.3 |
| 142 | (E)-3-(4-(((1-(3-Cyano-4-(4-cya no-3-fluorophen yl)-5-(1H-indaz ol-6-yl)pyridin-2-yl)piperidin-4 -yl)amino) meth yl)phenyl)-N-hy droxya-crylamid e formate | 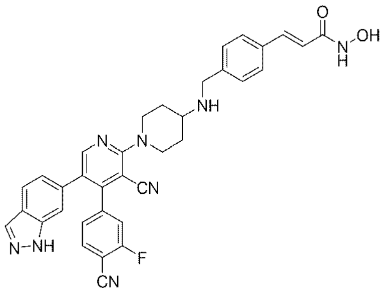 | $^1$H NMR (400 MHz, DMSO-d6) δ 13.06 (s, 1H), 10.72 (s, 1H), 9.01 (s, 1H), 8.56 (s, 1H), 8.01 (s, 1H), 7.89 (t, J = 7.4 Hz, 1H), 7.71 (d, J = 10.0 Hz, 1H), 7.56 (dd, J = 8.6, 8.2 Hz, 3H), 7.44 (t, J = 8.2 Hz, 3H), 7.30 (d, J = 7.2 Hz, 2H), 6.73 (d, J = 8.4 Hz, 1H), 6.44 (d, J = 5.8 Hz, 1H), 4.24 (d, J = 13.2 Hz, 2H), 3.86 (s, 2H), 3.21 (t, J = 11.8 Hz, 2H), 2.81 (s, 1H), 2.03 (d, J = 12.2 Hz, 2H), 1.50 (q, J = 11.4, 10.8 Hz, 2H). | 613.2 |
| 143 | (E)-3-(4-(((1-(3-Cyano-4-(4-cya no-3-fluorophen yl)-5-(3-hydrox y-4-(2-hydroxy-2-methylpropox y)phenyl)pyridi n-2-yl)piperidin -4-yl)amino) met hyl)phenyl)-N-h ydroxya-crylami de formate | 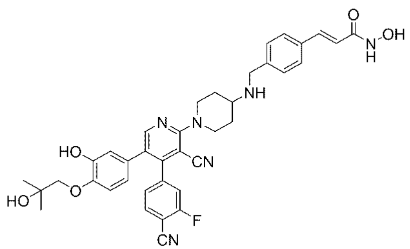 | $^1$H NMR (400 MHz, Methanol-d4) δppm 8.52(s, 1H)8.44 (s, 1H), 7.74 (t, J = 7.4 Hz, 1H), 7.70 - 7.60 (m, 3H), 7.56 (d, J = 9.6 Hz, 2H), 7.36 (d, J = 9.6 Hz, 1H), 7.23 (d, J = 7.8 Hz, 1H), 6.80 (d, J = 8.4 Hz, 1H), 6.55 (d, J = 2.2 Hz, 1H), 6.52 - 6.44 (m, 2H), 4.45 (d, J = 13.2 Hz, 2H), 4.28 (s, 2H), 3.78 (s, 2H), 3.46 (d, J = 15.4 Hz, 1H), 3.17 (t, J = 12.4 Hz, 2H), 2.32 (d, J = 11.6 Hz, 2H), 1.85 (d, J = 11.6 Hz, 2H), 1.30 (s, 6H). | 677.3 |

(continued)

| Exampl e | Chemical name | Structure | $^1$H NMR | MS( M+H )$^+$ |
|---|---|---|---|---|
| 144 | (E)-3-(4-(((1-(4-(4-Cyano-3-fluo rophenyl)-5-(3-h ydroxy-4-metho xyphenyl)-3-me thylpyridin-2-yl )piperidin-4-yl)a mino)methyl)ph enyl)-N-hydrox yacrylamide hydrochloride | 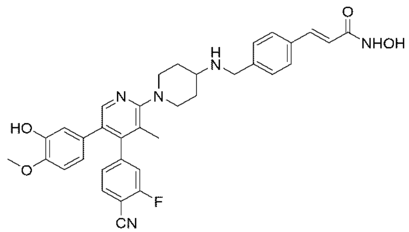 | $^1$H NMR (400 MHz, DMSO-d6) δ10.75 (s, 1H), 8.89 (s, 1H), 8.18 (d, J = 2.4 Hz, 1H), 8.10 (s, 1H), 7.87 (t, J = 7.6 Hz, 1H), 7.53 (d, J = 7.6 Hz, 2H), 7.50 - 7.33 (m, 4H), 7.10 (dd, J = 8.0, 1.6 Hz, 1H), 6.76 (d, J = 8.8 Hz, 1H), 6.43 (m, 3H), 3.87 (s, 2H), 3.70 (s, 3H), 3.46 (s, 2H), 2.77 (m, 3H), 1.97 (s, 5H), 1.52 (s, 2H). | 608.3 |
| 145 | (E)-3-(5-(((1-(3-Cyano-4-(4-cya no-3-fluorophen yl)-5-(4-(difluor omethoxy)-3-hy droxyphenyl)py ridin-2-yl) piperi din-4-yl)amino) methyl) pyrimidi n-2-yl)-N-hydro xya-crylamide formate | 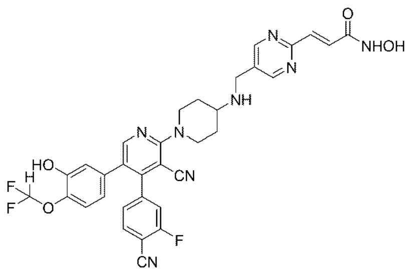 | $^1$H NMR (400 MHz, Methanol-d4) δppm 8.95 (s, 2H), 8.46 (s, 1H), 7.76 (t, J = 7.4 Hz, 1H), 7.55 (d, J = 15.4 Hz, 1H), 7.41 (d, J = 9.6 Hz, 1H), 7.33 - 7.17 (m, 2H), 7.01 (d, J = 8.0 Hz, 2H), 6.64 (s, 1H), 6.60 - 6.50 (m, 1H), 4.60 - 4.32 (m, 4H), 3.72 - 3.47 (m, 1H), 3.22 (t, J = 12.4 Hz, 2H), 2.37 (s, 2H), 1.92 (d, J = 13.8 Hz, 2H). | 657.2 |
| 147 | (E)-3-(4-(4-(4-C yano-3-fluoroph enyl)-5-(3-hydro xy-4-methoxyph enyl)-3-(trifluor omethyl)pyridin -2-yl)piperidin-4-yl)amino)met hyl)phenyl)-N-h ydroxyacrylami de formate | 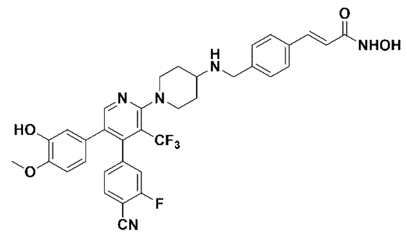 | $^1$H NMR (400 MHz, Methanol-d4) δppm: 8.52 (s, 1H),8.41 (s, 1H), 7.87 (dd, J = 8.0, 6.2 Hz, 1H), 7.75 - 7.37 (m, 7H), 7.09 (d, J = 8.0 Hz, 1H), 6.96 - 6.83 (m, 2H), 6.51 (d, J = 5.8 Hz, 1H), 4.16 (s, 2H), 3.93 (s, 3H), 3.41 (d, J = 6.2 Hz, 2H), 3.23 (s, 1H), 2.92 (t, J = 12.0 Hz, 2H), 2.00 - 1.92 (m, 2H), 1.62 (d, J = 13.6 Hz, 2H). | 662.2 |
| 148 | (E)-3-(5-(((1-(3-Cyano-4-(4-cya no-3-fluorophen yl)-5-(3-hydrox y-4-methylphen yl) pyridin-2-yl) piperidin-4-yl)a mino)methyl)py rimidin-2-yl)-N-hydroxyacrylam ide for-mate | 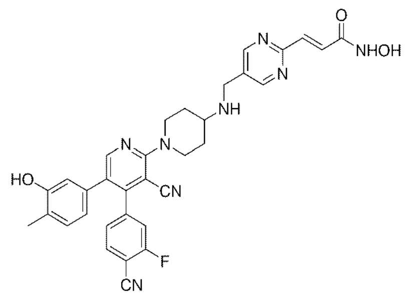 | $^1$H NMR (400 MHz, DMSO-d6)δ ppm : 11.10 (s, 1H), 9.31 (s, 1H), 8.99 (s, 2H), 8.45 (s, 1H), 8.28 (s, 1H), 7.96 (t, J = 7.4 Hz, 1H), 7.70 (d, J = 10.0 Hz, 1H), 7.38 (d, J = 15.4 Hz, 1H), 7.26 (dd, J = 7.8, 1.4 Hz, 1H), 7.19 (d, J = 15.6 Hz, 1H), 6.95 (d, J = 7.8 Hz, 1H), 6.42 (d, J = 1.8 Hz, 2H), 4.30 (s, 2H), 4.22 (s, 1H), 3.31 (s, 2H),3.17 (s, 2H), 2.26 (s, 2H),2.04 (s, 3H), 1.74 (d, J = 13.8 Hz, 2H). | 604.2 |

(continued)

| Exampl e | Chemical name | Structure | $^1$H NMR | MS( M+H )$^+$ |
|---|---|---|---|---|
| 149 | (E)-3-(4-(((1-(3-Cyano-4-(4-cya no-3-fluorophen yl)-5-(3-hydrox y-4-methoxyphe nyl) pyridin-2-yl )azepan-4-yl)am ino)methyl)phen yl)-N-hydro-xyac rylamide formate | 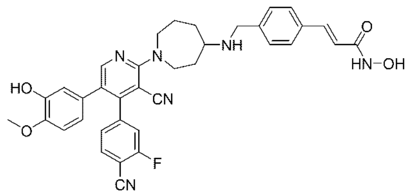 | $^1$H NMR (400 MHz, DMSO-d6)δ ppm : 10.71 (s, 1H), 8.96 (s, 1H), 8.49 (s, 1H), 8.21 - 8.13 (m, 1H), 7.94 (t, J = 7.4 Hz, 1H), 7.61 (d, J = 7.6 Hz, 1H), 7.51 (d, J = 7.8 Hz, 2H), 7.48 - 7.37 (m, 2H), 7.25 (s, 1H), 6.82 - 6.75 (m, 1H), 6.49 - 6.39 (m, 3H), 4.02 - 3.73 (m, 2H), 3.79 (s, 2H), 3.75 (s, 1H),3.71 (s, 3H), 3.69 (s, 1H), 2.76 (s, 1H), 2.19 (s, 1H),2.10 (s, 1H), 1.88 (d, J = 6.2 Hz, 1H), 1.76 (d, J = 10.0 Hz, 2H), 1.51 (t, J = 11.2 Hz, 1H). | 632.2 |
| 150 | (E)-3-(4-(((1-(3-Cyano-5-(3-hyd roxy-4-methoxy phenyl)-4-(4-nit rophenyl)pyridi n-2-yl)piperidin -4-yl)amino) met hyl)phenyl)-N-h ydroxya-crylami de formate | 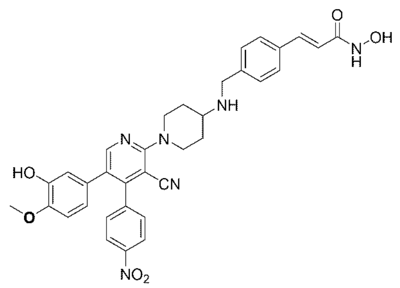 | $^1$H NMR (400 MHz, Methanol-d4) δppm 8.50 (s, 1H), 8.43 (s, 1H), 8.26 - 8.12 (m, 2H), 7.71 = 7.55 (m, 3H), 7.55 - 7.42 (m, 4H), 6.78 (d, J = 8.2 Hz, 1H), 6.62 - 6.34 (m, 3H), 4.42 (d, J = 13.4 Hz, 2H), 4.17 (s, 2H), 3.78 (s, 3H), 3.30 (s, 1H), 3.22 - 3.07 (m, 2H), 2.38 - 2.20 (m, 2H), 1.88 - 1.70 (m, 2H). | 621.2 |
| 151 | (E)-3-(2-(((1-(3-Cyano-4-(4-cya no-3-fluorophen yl)-5-(3-hydrox y-4-methoxyphe nyl) pyridin-2-yl )piperidin-4-yl)a mino)methyl)thi azol-5-yl)-N-hy droxyacrylamid e hydro-chloride | 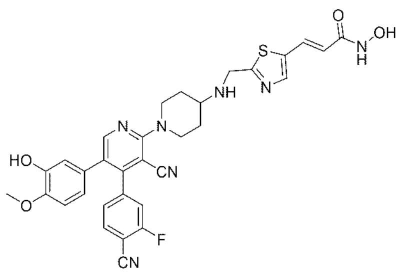 | $^1$H NMR (400 MHz, DMSO-d6) δ 10.68 (s, 1H), 9.01 (s, 2H), 8.42 (s, 1H), 7.99 - 7.89 (m, 2H), 7.70 - 7.56 (m, 2H), 7.27 (dd, J = 8.0, 1.6 Hz, 1H), 6.80 (d, J = 8.4 Hz, 1H), 6.46 (s, 2H), 6.16 (d, J = 15.6 Hz, 1H), 4.18 (d, J = 13.2 Hz, 2H), 4.05 (s, 2H), 3.71 (s, 3H), 3.17 (t, J = 12.4 Hz, 2H), 2.80-2.75 (m, 1H), 1.99 (d, J = 12. 4Hz, 2H), 1.45 (q, J = 10.8, 10.4 Hz, 2H). | 626.2 |
| 152 | 3-(5-((1-(3-Cya no-4-(4-cyano-3 -fluorophenyl)-5 -(3-hydroxy-4-methoxyphenyl) pyri-din-2-yl)pip eridin-4-yl)amin o)methyl)pyrimi din-2-acyl)-N-h ydroxypropana mide hydrochloride | 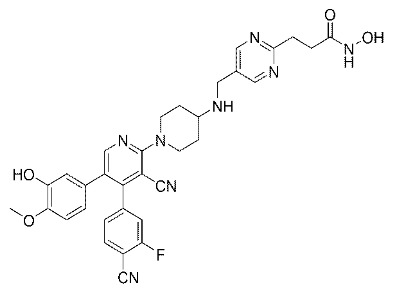 | $^1$H NMR (400 MHz, DMSO-d6) δ 10.42 (s, 1H), 8.98 (s, 1H), 8.68 (s, 2H), 8.41 (s, 1H), 8.14 (s, 1H), 7.95 (dd, J = 8.0, 6.8 Hz, 1H), 7.66 (dd, J = 10.4, 1.5 Hz, 1H), 7.27 (dd, J = 8.0, 1.6 Hz, 1H), 6.80 (d, J = 8.0 Hz, 1H), 6.59 - 6.38 (m, 2H), 4.27 - 4.10 (m, 2H), 3.79 (s, 2H), 3.71 (s, 3H), 3.25 - 3.14 (m, 2H), 3.08 (t, J = 7.6 Hz, 2H), 2.74 (s, 1H), 2.46 (d, J = 7.6 Hz, 2H), 2.00 (d, J = 12.8 Hz, 2H), 1.45 (q, J = 10.4 Hz, 2H). | 623.3 |

(continued)

| Exampl e | Chemical name | Structure | $^1$H NMR | MS( M+H )$^+$ |
|---|---|---|---|---|
| 153 | (E)-3-(4-(((1-(3-Cyano-4-(4-cya no-3-fluorophen yl)-5-(4-(dimeth ylamino)-3-hydr oxyphenyl)pyrid in-2-yl) piperidin -4-yl)amino)met hyl) phenyl)-N-h ydroxyacrylami de formate | 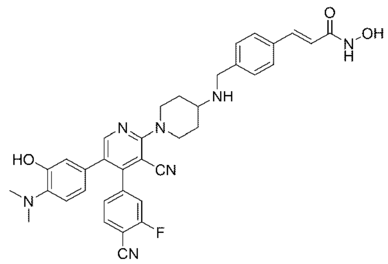 | $^1$H NMR (400 MHz, DMSO) δppm: 10.79 (s, 1H), 8.94 (s, 2H), δ 8.50 (s, 1H), 7.96 (dd, J = 8.0, 6.8 Hz, 1H), 7.75 - 7.62 (m, 3H), 7.57 (d, J = 8.0 Hz, 2H), 7.53 - 7.45 (m, 2H), 7.30 (dd, J = 8.0, 1.4 Hz, 1H), 6.76 (dd, J = 8.4, 2.0 Hz, 1H), 6.71 (d, J = 1.8 Hz, 1H), 6.54 (d, J = 15.8 Hz, 1H), 4.38 (d, J = 12.9 Hz, 2H), 4.26 (s, 2H), 3.23 (m, 1H), 3.16 (t, J = 12.7 Hz, 2H), 3.09 (s, 6H), 2.28 (d, J = 12.0 Hz, 2H), 1.84 - 1.67 (m, 2H). | 632.3 |
| 154 | (E)-3-(4-(((1-(3-Cyano-4-(4-cya no-3-fluorophen yl)-5-(3-hydrox y-4-methoxyphe nyl) pyridin-2-yl )piperidin-4-yl)a mino)methyl)-3-fluorophenyl)-N -hydroxyacryla mide hydrochloride | 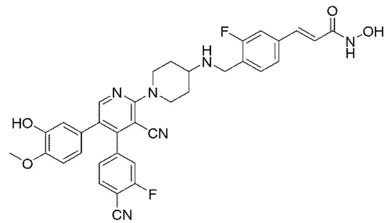 | $^1$H NMR (400 MHz, DMSO-d6) δ 10.76 (s, 1H), 8.99 (s, 1H), 8.41 (s, 1H), 8.15 (s, 1H), 7.95 (dd, J = 8.0, 6.8 Hz, 1H), 7.66 (dd, J = 10.4, 1.6 Hz, 1H), 7.55 (t, J = 7.6 Hz, 1H), 7.48 - 7.34 (m, 3H), 7.27 (dd, J = 8.0, 1.6 Hz, 1H), 6.80 (d, J = 8.4 Hz, 1H), 6.56 - 6.39 (m, 3H), 4.29 - 4.10 (m, 2H), 3.86 (s, 2H), 3.71 (s, 3H), 3.18 (t, J = 11.6 Hz, 1H), 2.13 - 1.93 (m, 2H), 2.78-2.76 (m, 2H), 1.47 (q, J = 10.4 Hz, 2H). | 637.2 |
| 155 | (E)-3-(4-(((1-(3-Cyano-4-(4-cya no-3-fluorophen yl)-5-(3-hydrox y-4-methoxyphe nyl) pyridin-2-yl )piperidin-4-yl)a mino)methyl)cy clohex-1-en-1-yl )-N-hydroxyacr yla-mide formate | 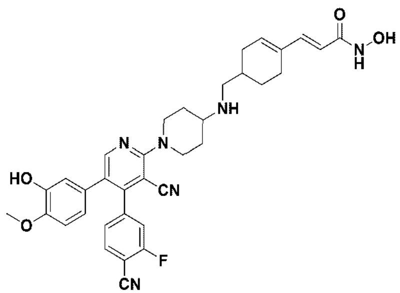 | $^1$H NMR (400 MHz, Methanol-d4) δppm 8.44 (s, 1H), 8.33 (s, 1H), 7.64 (dd, J = 8.0, 6.6 Hz, 1H), 7.26 (dd, J = 9.8, 1.2 Hz, 1H), 7.17 - 7.06 (m, 2H), 6.75 - 6.68 (m, 1H), 6.44 - 6.38 (m, 2H), 6.04 (s, 1H), 5.71 (d, J = 15.6 Hz, 1H), 4.33 (d, J = 13.2 Hz, 2H), 3.71 (s, 3H), 3.20 (d, J = 1.6 Hz, 1H), 3.12 - 3.00 (m, 2H), 2.88 (d, J = 5.6 Hz, 2H), 2.35 (d, J = 16.4 Hz, 1H), 2.25 (d, J = 18.0 Hz, 1H), 2.12 (td, J = 15.8, 15.0, 5.0 Hz, 3H), 1.96 - 1.87 (m, 3H), 1.75 - 1.61 (m, 2H), 1.45 - 1.23 (m, 1H). | 623.3 |
| 156 | (E)-3-(3-Chloro-4-((1-(3-cyano-4 -(4-cyano-3-fluo rophenyl)-5-(3-h ydroxy-4-metho xyphenyl)pyridi n-2-yl)piperidin -4-yl)amino)met hyl)phenyl)-N-h ydroxyacrylami de formate | 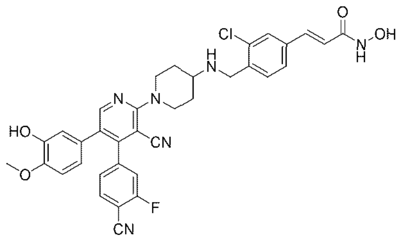 | $^1$H NMR (400 MHz, DMSO-d6) δ ppm : 10.77 (s, 1H), 9.00 (s, 1H), 8.43 (s, 1H), 8.12 (s, 1H), 7.96 (t, J = 7.4 Hz, 1H), 7.66 (dd, J = 5.8, 3.8 Hz, 3H), 7.56 (d, J = 8.0 Hz, 1H), 7.43 (d, J = 15.8 Hz, 1H), 7.28 (dd, J = 8.2, 1.4 Hz, 1H), 6.81 (d, J = 8.2 Hz, 1H), 6.57 - 6.46 (m, 3H), 4.22 (d, J = 13.2 Hz, 2H), 4.00 (s, 2H), 3.71 (s, 3H), 3.24 - 3.12 (m, 2H), 2.95 (s, 1H), 2.08 (d, J = 12.0 Hz, 2H), 1.56 (d, J = 11.4 Hz, 2H). | 653.2 |

(continued)

| Exampl e | Chemical name | Structure | $^1$H NMR | MS( M+H )$^+$ |
|---|---|---|---|---|
| 158 | (E)-3-(4-(((1-(3-Cyano-4-(4-cya no-3-fluorophen yl)-5-(2,5-dihyd roxy-4-methoxy phenyl)pyridin-2-yl)piperidin-4 -yl)amino)meth yl)phenyl)-N-hy droxyacrylamid e trifluoroacetate | 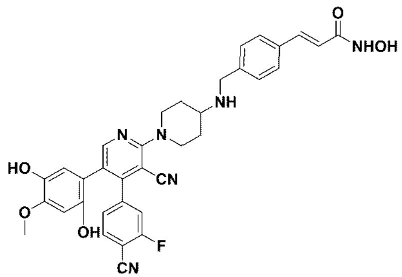 | $^1$H NMR (400 MHz, Methanol-d4) δ 8.35 (s, 1H), 7.73 - 7.62 (m, 3H), 7.59 - 7.46 (m, 3H), 7.33 (dd, J = 9.8, 1.4 Hz, 1H), 7.22 (dd, J = 8.0, 1.4 Hz, 1H), 6.52 (d, J = 5.8 Hz, 1H), 6.43 (s, 1H), 6.31 (s, 1H), 4.42 (d, J = 3.6 Hz, 2H), 4.25 (s, 2H), 3.76 (s, 3H), 3.59 - 3.46 (m, 1H),3.16 (s, 2H), 2.29 (d, J = 8.4 Hz, 2H), 1.90 - 1.76 (m, 2H). | 635.2 |
| 159 | (E)-3-(5-(((1-(5-(4-Chloro-3-hyd roxyphenyl)-3-c yano-4-(4-cyano -3-fluorophenyl) pyridin-2-yl)pip eridin-4-yl)amin o)methyl)pyrimi din-2-yl)-N-hyd roxyacryla-mide hydrochloride | 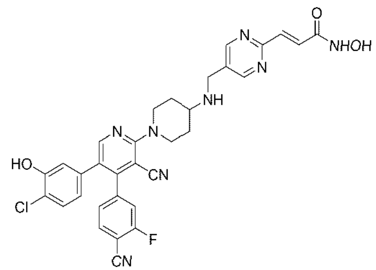 | $^1$H NMR (400 MHz, Methanol-d4) δ ppm 8.90 (s, 2H), 8.45 (s, 1H), 7.76 (dd, J = 8.0, 6.6 Hz, 1H), 7.57 (d, J = 15.4 Hz, 1H), 7.42 (dd, J = 9.6, 1.5 Hz, 1H), 7.29 - 7.16 (m, 3H), 6.62 (d, J = 2.2 Hz, 1H), 6.54 (dd, J = 8.2, 2.0 Hz, 1H), 4.47 (d, J = 13.2 Hz, 2H), 4.21 (s, 2H), 3.22 (t, J = 12.6 Hz, 3H), 2.27 (d, J = 12.2 Hz, 2H), 1.77 (q, J = 11.8 Hz, 2H). | 625.2 |
| 160 | (E)-3-(6-(((1-(3-Cyano-4-(4-cya no-3-fluorophen yl)-5-(3-hydrox y-4-methoxyphe nyl) pyridin-2-yl )piperidin-4-yl)a mino)methyl)py ridazin-3-yl)-N-hydroxyacrylam ide for-mate | 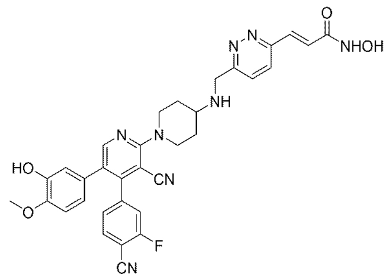 | $^1$H NMR (400 MHz, DMSO-d6) δppm: 9.43 (s, 1H), 9.25 (s, 1H), 9.00 (s, 1H), 8.46 (s, 1H), 8.04 (d, J = 8.6 Hz, 1H), 7.97 (dd, J = 8.0, 6.8 Hz, 1H), 7.86 (d, J = 8.8 Hz, 1H), 7.71 - 7.61 (m, 2H), 7.28 (dd, J = 8.0, 1.4 Hz, 1H), 7.08 (d, J = 5.8 Hz, 1H), 6.82 (d, J = 8.2 Hz, 1H), 6.51 - 6.43 (m, 2H), 4.63 (s, 2H), 4.32 (d, J = 8.2 Hz, 2H), 3.72 (s, 3H), 3.60 - 3.40 (m, 1H), 3.15 (t, J = 12.6 Hz, 2H), 2.25 (s, 2H), 1.78 (s, 2H). | 621.2 |

(continued)

| Exampl e | Chemical name | Structure | $^1$H NMR | MS( M+H )$^+$ |
|---|---|---|---|---|
| 161 | (E)-3-(4-(((1-(3-Cyano-4-(4-cya no-3-fluorophen yl)-5-(3-fluoro-5 -hydroxy-4-met hylphenyl)pyrid in-2-yl)piperidin -4-yl)amino)met hyl)phenyl)-N-h ydroxyacrylami de formate | 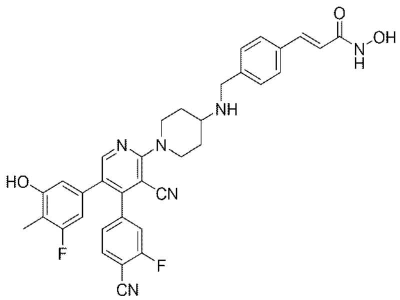 | $^1$H NMR (400 MHz, Methanol-d4) δppm 8.52 (s, 1H),8.35 (s, 1H), 7.67 (dd, J = 8.0, 6.6 Hz, 1H), 7.58 (d, J = 8.0 Hz, 2H), 7.51 - 7.43 (m, 3H), 7.31 (dd, J = 9.8, 1.6 Hz, 1H), 7.13 (dd, J = 8.0, 1.6 Hz, 1H), 6.44 (d, J = 15.8 Hz, 1H), 6.22 (dd, J = 9.8, 1.8 Hz, 1H), 6.16 (d, J = 1.4 Hz, 1H), 4.39 (d, J = 13.4 Hz, 2H), 4.23 (s, 2H), 3.51 = 3.32 (m, 1H), 3.15 - 3.00 (m, 2H), 2.25 (d, J = 12.2 Hz, 2H), 1.93 (d, J = 1.8 Hz, 3H), 1.79 (ddt, J = 21.8, 13.6, 7.4 Hz, 2H). | 621.2 |
| 162 | (E)-3-(2-Cyano-4-((1-(3-cyano-4 -(4-cyano-3-fluo rophenyl)-5-(3-h ydroxy-4-metho xyphenyl)pyridi n-2-yl)piperidin -4-yl)amino)met hyl)phenyl)-N-h ydroxyacrylami de formate | 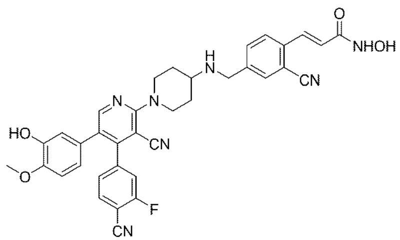 | $^1$H NMR (400 MHz, DMSO-d6) δ 11.01 (s, 1H), 9.24 (s, 1H), 9.01 (s, 1H), 8.93 (s, 1H), 8.45 (d, J = 7.0 Hz, 1H), 8.05 (s, 1H), 7.97 (t, J = 7.4 Hz, 2H), 7.86 (d, J = 7.8 Hz, 1H), 7.72 - 7.61 (m, 2H), 7.28 (dd, J = 8.0, 1.4 Hz, 1H), 6.82 (d, J = 8.0 Hz, 1H), 6.73 (d, J = 9.6 Hz, 1H), 6.52 - 6.44 (m, 2H), 4.29 (d, J = 5.8 Hz, 4H), 3.72 (s, 3H), 3.45 - 3.40 (m, 2H), 3.15 (t, J = 5.6 Hz, 2H), 2.22 (s, 2H), 1.71 (s, 2H). | 644.2 |
| 163 | 2-(4-(((1-(3-Cya no-4-(4-cyano-3 -fluorophenyl)-5 -(3-hydroxy-4-methoxyphenyl) pyri-din-2-yl)pip eridin-4-yl)amin o)methyl)phenyl )-N-hydroxycycl opent-1-ene-1-c arboxa-mide formate | 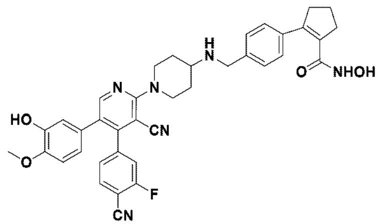 | $^1$H NMR (400 MHz, DMSO-d6) δ 10.51 (s, 1H), 8.94 (s, 1H), 8.42 (s, 1H), 8.18 (s, 1H),7.95 (dd, J = 8.0, 6.8 Hz, 1H), 7.66 (dd, J = 10.2, 1.5 Hz, 1H), 7.44 - 7.30 (m, 4H), 7.27 (dd, J = 8.0, 1.6 Hz, 1H), 6.80 (d, J = 8.0 Hz, 1H), 6.46 (s, 2H), 4.19 (d, J = 13.2 Hz, 2H), 3.85-3.84 (s, 2H), 3.71 (s, 3H), 3.22 - 3.12 (m, 2H), 2.83 (s, 1H), 2.81 - 2.73 (m, 2H), 2.71 - 2.60 (m, 2H), 2.03 (d, J = 12.4 Hz, 2H), 1.92 (p, J = 7.6 Hz, 2H), 1.50 (q, J = 11.6, 11.2 Hz, 2H). | 659.3 |
| 164 | 4-(2-[(1-(3-Cya no-4-(4-cyano-3 -fluorophenyl)-5 -(3-hydroxy-4-methoxyphenyl) pyri-din-2-yl)pip eridin-4-yl)amin o]ethoxy)-N-hy droxybenza-mide formate | 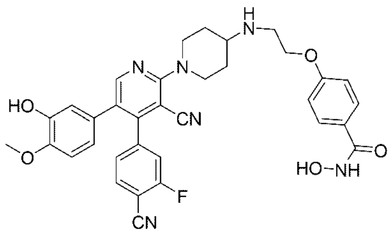 | $^1$H NMR (400 MHz, DMSO-d6) δ ppm : 11.07 (s, 1H), 9.00 (s, 1H), 8.90 (s, 1H), 8.43 (s, 1H), 8.12 (s, 1H), 7.96 (s, 1H), 7.74 (d, J = 8.2 Hz, 2H), 7.67 (d, J = 10.1 Hz, 1H), 7.28 (d, J = 8.1 Hz, 1H), 7.02 (d, J = 8.2 Hz, 2H), 6.81 (d, J = 7.9 Hz, 1H), 6.47 (d, J = 9.7 Hz, 2H), 4.24 - 4.13 (m, 4H), 3.72 (s, 3H), 3.17 (t, J = 11.9 Hz, 4H), 3.04 (s, 1H), 2.07 (d, J = 11.8 Hz, 2H), 1.53 (d, J = 12.0 Hz, 2H). | 623.2 |

(continued)

| Exampl e | Chemical name | Structure | $^1$H NMR | MS( M+H )$^+$ |
|---|---|---|---|---|
| 165 | (E)-3-(4-(((1-(3-Cyano-4-(4-cya no-3-fluorophen yl)-5-(5-hydrox y-2,4-dimethoxy phenyl)pyridin-2-yl)piperidin-4 -yl)amino)meth yl)-N-hydro-xyac rylamide hydrochloride | 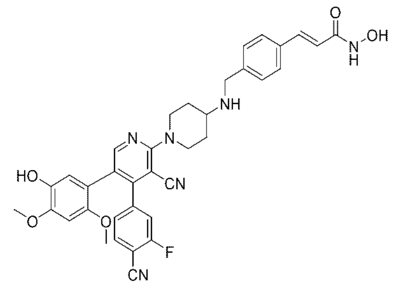 | $^1$H NMR (400 MHz, Methanol-d4) δ ppm 8.31 (s, 1H), 7.76 - 7.52 (m, 6H), 7.25 (dd, J = 39.6, 9.0 Hz, 2H), 6.66 - 6.43 (m, 3H), 4.43 (d, J = 13.4 Hz, 2H), 4.25 (s, 2H), 3.84 (s, 3H), 3.46 (s, 3H), 3.41 (d, J = 11.8 Hz, 1H), 3.17 (t, J = 12.8 Hz, 2H), 2.30 (d, J = 12.2 Hz, 2H), 1.90 - 1.77 (m, 2H). | 649.2 |
| 166 | (E)-3-(4-(((1-(3-Cyano-4-(4-cya no-3-hydroxyph enyl)-5-(3-hydro xy-4-methoxyph enyl)pyridin-2-y l)piperidin-4-yl) amino)methyl) -N-hydroxyacryl amide hy-drochloride | 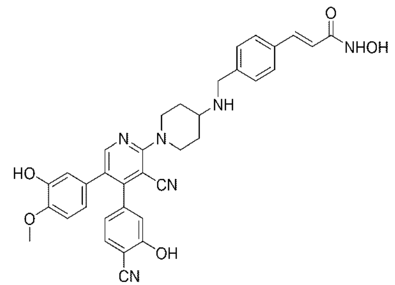 | $^1$H NMR (400 MHz, Methanol-d4) δ ppm 8.40 (s, 1H), 7.65 (d, J = 7.8 Hz, 2H), 7.62 - 7.47 (m, 4H), 6.84 - 6.77 (m, 3H), 6.60 - 6.44 (m, 3H), 4.40 (d, J = 13.2 Hz, 2H), 4.24 (s, 2H), 3.81 (s, 3H), 3.42 - 3.34 (m, 1H), 3.21 - 3.09 (m, 2H), 2.35 - 2.24 (m, 2H), 1.83 (qd, J = 12.2, 11.4, 3.6 Hz, 2H). | 617.2 |
| 167 | 2-(4-((1-(3-Cya no-4-(4-cyano-3 -fluorophenyl)-5 -(3-hydroxy-4-methoxyphenyl) pyri-din-2-yl)pip eridin-4-yl)amin o)methyl)phenyl )-N-hydroxy-cycl opropane-1-carb oxa-mide formate | 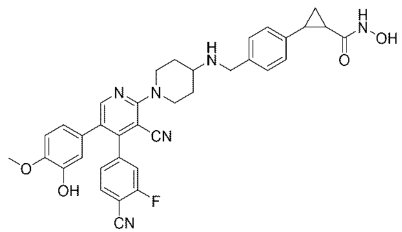 | $^1$H NMR (400 MHz, DMSO-d6) δ 10.61 (s, 1H),9.01 (s, 1H), 8.88 - 8.71 (m, 3H), 8.46 (s, 1H), 8.04 - 7.93 (m, 1H), 7.67 (dd, J = 10.0, 1.4 Hz, 1H), 7.42 (d, J = 8.0 Hz, 2H), 7.28 (dd, J = 8.0, 1.4 Hz, 1H), 7.22 (d, J = 8.0 Hz, 2H), 6.82 (d, J = 8.0 Hz, 1H), 6.46 (s, 2H), 4.30 (d, J = 6.2 Hz, 2H), 4.20 (s, 2H), 3.72 (s, 3H), 3.39 (s, 1H), 3.12 (t, J = 5.6 Hz, 2H), 2.36 - 2.17 (m, 3H), 1.81 - 1.63 (m, 3H), 1.50 - 1.33 (m, 2H). | 633.3 |

(continued)

| Exampl e | Chemical name | Structure | $^1$H NMR | MS( M+H )$^+$ |
|---|---|---|---|---|
| 168 | (E)-3-(4-(((1-(3-Cyano-4-(4-cya no-3-fluorophen yl)-5-(3-fluoro-5 -hydroxy-4-met hoxyphenyl)pyri din-2-yl)piperidi n-4-yl)amino)m ethyl)phenyl)-N -hydroxyacryla mide formate | 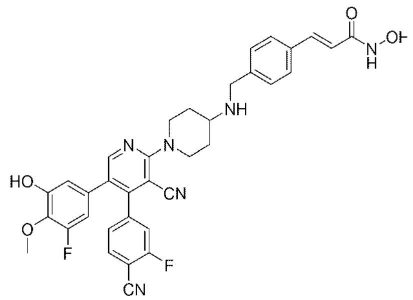 | $^1$H NMR (400 MHz, Methanol-d4) δppm 8.44 (s, 1H), 7.78 (dd, J = 8.0, 6.6 Hz, 1H), 7.67 (d, J = 7.8 Hz, 2H), 7.62 - 7.51 (m, 3H), 7.41 (dd, J = 9.6, 1.6 Hz, 1H), 7.25 (dd, J = 8.0, 1.6 Hz, 1H), 6.53 (d, J = 15.8 Hz, 1H), 6.45 - 6.29 (m, 2H), 4.58 - 4.44 (m, 2H), 4.32 (s, 2H), 3.83 (d, J = 1.0 Hz, 3H), 3.64 - 3.42 (m, 1H), 3.29 - 3.09 (m, 2H), 2.48 - 2.14 (m, 2H), 1.86 (qd, J = 12.4, 4.1 Hz, 2H). | 637.2 |
| 169 | (E)-4-(4-(((1-(3-Cyano-4-(4-cya no-3-fluorophen yl)-5-(3-hydrox y-4-methoxyphe nyl) pyridin-2-yl )piperidin-4-yl)a mino)methyl)ph enyl)-N-hydrox ybut-3-enamide formate | 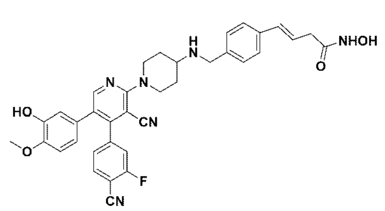 | $^1$H NMR (400 MHz, DMSO) δppm: 10.50 (s, 1H), 8.99 (s, 1H), 8.77 (s, 1H), 8.52 (s, 1H), 7.95 (dd, J = 8.0, 6.8 Hz, 1H), 7.66 (dd, J = 10.0, 1.6 Hz, 1H), 7.43 - 7.33 (m, 4H), 7.27 (dd, J = 8.0, 1.6 Hz, 1H), 6.81 (d, J = 8.2 Hz, 1H), 6.53 - 6.43 (m, 3H), 6.29 (dt, J = 15.4, 7.2 Hz, 1H), 4.21 (d, J = 13.0 Hz, 2H), 3.90 (s, 2H), 3.71 (s, 3H), 3.16 (q, J = 11.2 Hz, 2H), 2.97 - 2.87 (m, 2H), 2.52 (d, J = 11.4 Hz, 1H), 2.06 (d, J = 12.4 Hz, 2H), 1.53 (d, J = 12.0 Hz, 2H). | 632.3 |
| 170 | (E)-3-(4-(((1-(3-Cyano-4-(4-cya no-3-fluorophen yl)-5-(3-hydrox y-4-methoxyphe nyl) pyridin-2-yl )piperidin-4-yl)a mino)methyl)ph enyl)-N-hydrox ybut-2-enamide formate | 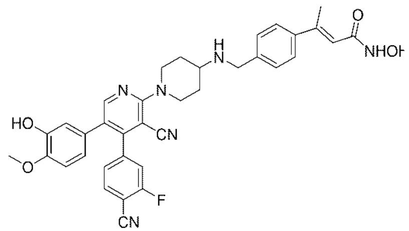 | $^1$H NMR (400 MHz, DMSO-d6) δ 10.61 (s, 1H), 9.01 (s, 1H), 8.93 - 8.82 (m, 2H), 8.47 (s, 1H), 7.97 (dd, J = 8.0, 6.8 Hz, 1H), 7.68 (dd, J = 10.0, 1.6 Hz, 1H), 7.62 - 7.52 (m, 3H), 7.52 - 7.42 (m, 1H), 7.28 (dd, J = 8.0, 1.6 Hz, 1H), 6.82 (d, J = 8.0 Hz, 1H), 6.47 (d, J = 8.0 Hz, 2H), 4.38 - 4.16 (m, 4H), 3.72 (s, 3H), 3.43 (s, 1H), 3.14 (t, J = 12.6 Hz, 2H), 2.54 (s, 3H), 2.25 (d, J = 11.2 Hz, 2H), 1.75 (d, J = 11.6 Hz, 2H). | 633.3 |
| 171 | (Z)-3-(4-(((1-(3-Cyano-4-(4-cya no-3-fluorophen yl)-5-(3-hydrox y-4-methoxyphe nyl) pyridin-2-yl )piperidin-4-yl)a mino)methyl)ph enyl)-2-fluoro-N -hydroxyacryla mide formate | 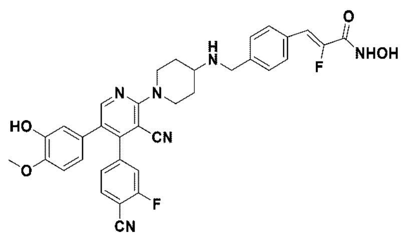 | $^1$H NMR (400 MHz, Methanol-d4) δ ppm 8.44 (d, J = 1.2 Hz, 1H), 7.79 - 7.70 (m, 2H), 7.64 (d, J = 8.0 Hz, 1H), 7.56 (d, J = 8.0 Hz, 1H), 7.47 (d, J = 8.0 Hz, 1H), 7.37 (dd, J = 9.8, 1.6 Hz, 1H), 7.23 (dd, J = 8.1, 1.6 Hz, 1H), 6.97 - 6.72 (m, 2H), 6.56 - 6.46 (m, 2H), 4.44 (d, J = 13.4 Hz, 2H), 4.25 (s, 2H), 3.81 (s, 3H), 3.41 (d, J = 11.4 Hz, 1H), 3.22 - 3.12 (m, 2H), 2.30 (d, J = 12.2 Hz, 2H), 1.93 - 1.76 (m, 2H). | 637.2 |

(continued)

| Exampl e | Chemical name | Structure | $^1$H NMR | MS( M+H )$^+$ |
|---|---|---|---|---|
| 172 | (E)-3-(4-(((1-(3-Cyano-4-(4-cya no-3-fluorophen yl)-5-(3-hydrox y-4-methoxyphe nyl) pyridin-2-yl )piperidin-4-yl)a mino)methyl)ph enyl)-N-hydrox y-2-methylacryl amide formate | 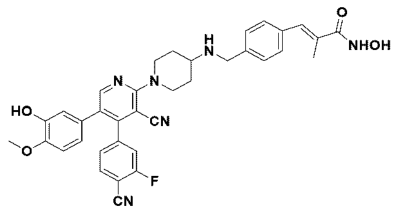 | $^1$H NMR (400 MHz, Methanol-d4) δ 8.35 (d, J = 1.8 Hz, 1H), 7.65 (dd, J = 8.0, 6.6 Hz, 1H), 7.51 - 7.38 (m, 3H), 7.34 (d, J = 8.2 Hz, 0.5H), 7.31 - 7.20 (m, 1.5H), 7.18 - 7.07 (m, 2H), 6.72 (d, J = 8.8 Hz, 1H), 6.44 - 6.38 (m, 2H), 4.36 (d, J = 13.2 Hz, 2H), 4.23 (s, 2H), 3.72 (s, 3H), 3.44 - 3.35 (m, 1H),3.09 (t, J = 12.4 Hz, 2H), 2.25 (d, J = 11.8 Hz, 2H), 1.96 (d, J = 1.6 Hz, 2.5H), 1.78 (td, J = 12.2, 8.2 Hz, 2H), 1.50 (d, J = 7.0 Hz, 0.5H). | 633.3 |
| 173 | 5-(((1-(3-Cyano -4-(4-cyano-3-fl uorophenyl)-5-( 3-hydroxy-4-me thoxyphenyl)pyr idin-2-yl)piperid in-4-yl)amino)m ethyl)-N-hydrox ybenzo [b]thioph ene-2-carboxam ide formate | 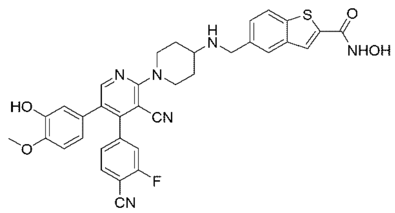 | $^1$H NMR (400 MHz, Methanol-d4) δ ppm 8.52(s, 1H), 8.43 (s, 1H), 8.07 - 7.98 (m, 2H), 7.88 (s, 1H), 7.74 (dd, J = 8.0, 6.6 Hz, 1H), 7.56 (dd, J = 8.6, 1.6 Hz, 1H), 7.36 (dd, J = 9.8, 1.6 Hz, 1H), 7.23 (dd, J = 8.0, 1.6 Hz, 1H), 6.86 - 6.78 (m, 1H), 6.56 - 6.47 (m, 2H), 4.43 (d, J = 13.4 Hz, 2H), 4.28 (s, 2H), 3.81 (s, 3H), 3.26 (s, 1H),3.17 (t, J = 12.0 Hz, 2H), 2.28 (d, J = 12.2 Hz, 2H), 1.87 - 1.73 (m, 2H). | 649.2 |
| 174 | 2-((5-(((1-(3-Cy ano-4-(4-cyano-3-fluorophenyl)-5-(3-hydroxy-4-methoxyphenyl) pyri-din-2-yl)pip eridin-4-yl)amin o)methyl)pyridi n-2-yl)oxy)-N-h ydroxyacetamid e formate | 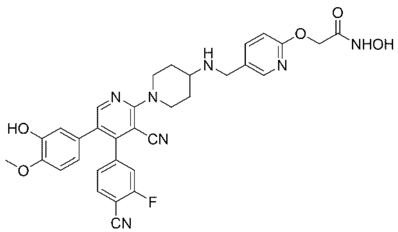 | $^1$H NMR (400 MHz, Methanol-d4) δ ppm 8.47 (s, 1H), 8.42 (s, 1H), 7.74 (dd, J = 8.2, 6.6 Hz, 2H), 7.69 - 7.61 (m, 1H), 7.36 (dd, J = 9.8, 1.6 Hz, 1H), 7.23 (dd, J = 8.0, 1.6 Hz, 1H), 6.88 - 6.77 (m, 1H), 6.60 (t, J = 9.4 Hz, 1H), 6.53 - 6.47 (m, 2H), 4.59 (s, 2H), 4.41 (d, J = 13.2 Hz, 2H), 3.95 (s, 2H), 3.81 (s, 3H), 3.19 (t, J = 12.8 Hz, 3H), 2.22 (d, J = 12.2 Hz, 2H), 1.86 - 1.67 (m, 2H). | 624.2 |

**Example 176**

Preparation of (E)-3-(4-(((1-(4-cyano-6-(4-cyano-3-fluorophenyl)-5-(3-hydroxy-4-methoxyphenyl)pyridin-2-yl)piperidin-4-yl)ami no)methyl)phenyl)-N-hydroxyacrylamide formate

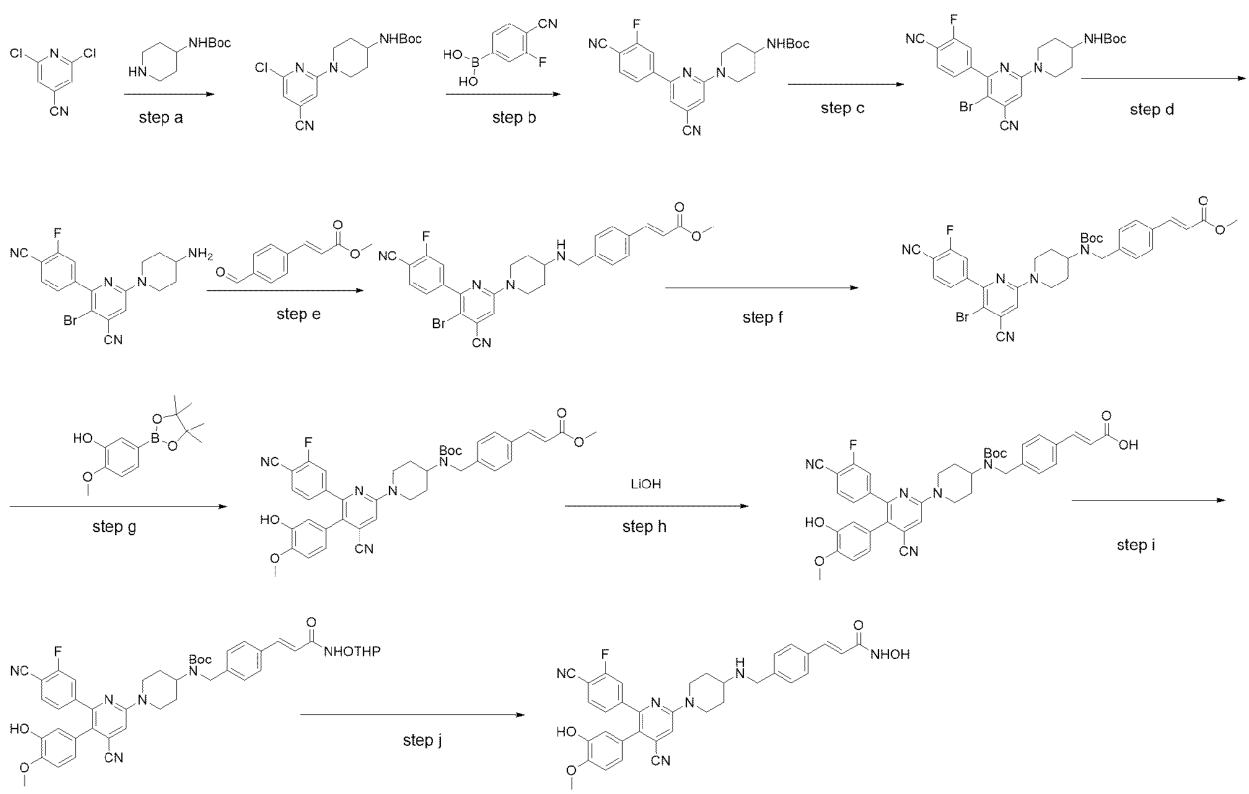


Step a): preparation of tert-butyl (1-(6-chloro-4-cyanopyridin-2-yl)piperidin-4-yl)carbamate

2,6-Dichloroisonicotinonitrile (200 mg, 1.16 mmol) was dissolved in NMP (3 mL), and (tert-butyl piperidin-4-ylcarbamate) (348.5 mg, 1.74 mmol) was added. The reaction was conducted at 60°C overnight, and LCMS indicated the disappearance of the starting material. The system was added into water and extracted with ethyl acetate to obtain a crude product. The solution was concentrated, and the residue was mixed with silica gel, and purified by silica gel column chromatography (EA:PE = 1:3), then purified by a normal-phase column to afford the product tert-butyl (1-(6-chloro-4-cyanopyridin-2-yl)piperidin-4-yl)carbamate with a yield of 74%.
ESI-MS m/z=337.1[M+H]+.

Step b) : preparation of tert-butyl (1-(4-cyano-6-(4-cyano-3-fluorophenyl)pyridin-2-yl)piperidin-4-yl)carbamate

Tert-butyl (1-(6-chloro-4-cyanopyridin-2-yl)piperidin-4-yl)carbamate (240 mg, 0.71 mmol), 4-cyano-3-fluorophenylboronic acid (163.9 mg, 0.99 mmol), cesium carbonate (694 mg, 2.13 mmol), and Pd(dppf)$Cl_2$ (52.0 mg, 0.071 mmol) were added to a mixture of Dioxane:$H_2O$=4:1 (4 mL). After purging with nitrogen, the mixture was subjected to microwave heating at 115°C and reacted for10 minutes, and LCMS indicated the disappearance of the starting materials. The solution was concentrated, and the residue was mixed with silica gel, and purified by silica gel column chromatography (EA:PE = 1:3), then purified by a normal-phase column to afford tert-butyl (1-(4-cyano-6-(4-cyano-3-fluorophenyl) pyridin-2-yl)piperidin-4-yl)carbamate with a yield of 90.6%.
ESI-MS m/z=422.2[M+H]+.

Step c) preparation of (tert-butyl N-(1-(6-(3-(benzyloxy)-4-methoxyphenyl)-5-bromo-4-cyanopyridin-2-yl)piperidin-4-yl) carbamate)

Tert-butyl (1-(4-cyano-6-(4-cyano-3-fluorophenyl)pyridin-2-yl)piperidin-4-yl)carbamate (274 mg, 0.65 mmol)

was dissolved in DMF (4 mL) and the mixture was cooled in an ice bath, then NBS (127.3 mg, 0.72 mmol) was added, and the reaction was conducted in an ice-water bath for 1 hour. LCMS indicated the disappearance of the starting material, then the reaction liquid was poured into saturated sodium thiosulfate solution and extracted with ethyl acetate. The organic phase was washed with sodium thiosulfate solution and brine. After concentrating was conducted to remove the solvent, the residue was mixed with silica gel, and purified by silica gel column chromatography (eluent: EA:PE = 1:3)to afford tert-butyl N-(1-(6-(3-(benzyloxy)-4-methoxyphenyl)-5-bromo-4-cyanopyridin-2-yl)piperidin-4-yl)carbama with a yield of 81%.

ESI-MS m/z=500.1 [M+H]+.

Step d): preparation of 6-(4-aminopiperidin-1-yl)-3-bromo-2-(4-cyano-3-fluorophenyl)isonicotinonitrile

4.0M Hydrochloric acid solution in EA (4 mL) was added to a reaction flask containing tert-butyl N-(1-(6-(3-(benzyloxy)-4-methoxyphenyl)-5-bromo-4-cyanopyridin-2-yl)piperidin-4-yl)carbamate (266 mg, 0.53 mmol), and the mixture was stirred at room temperature for 1 hour. The reaction liquid was concentrated to afford 6-(4-aminopiperidin-1-yl)-3-bromo-2-(4-cyano-3-fluorophenyl)isonicotinonitrile with a yield of 80%.

ESI-MS m/z=400.1 [M+H]+.

Step e): preparation of methyl (E)-3-(4-(((1-(5-bromo-4-cyano-6-(4-cyano-3-fluorophenyl)pyridin-2-yl)piperidin-4-yl) amino)methyl)phenyl)acryla te

6-(4-Aminopiperidin-1-yl)-3-bromo-2-(4-cyano-3-fluorophenyl)isonicotinonitrile (170 mg, 0.42 mmol) and methyl (E)-3-(4-formylphenyl)acrylate (103.9 mg, 0.55 mmol) were added to a mixture of DCE/MeOH/$CH_3COOH$ = 20:1:0.1 (3 mL), and the mixture was stirred at room temperature for 0.5 hours, then cooled in an ice bath, and sodium cyanoborohydride (445.1 mg, 2.1 mmol) was added. The reaction was slowly warmed back to room temperature and stirred for 1 hour. The reaction was monitored by LCMS until the starting materials were completely consumed, then the reaction system was poured into water and extracted with DCM. The organic phase was combined and concentrated to remove the solvent. The residue was mixed with silica gel, and purified by silica gel column chromatography (EA:PE = 1:5), then purified by a normal-phase column to afford methyl (E)-3-(4-(((1-(5-bromo-4-cyano-6-(4-cyano-3-fluorophenyl) pyridin-2-yl)piperidin-4-yl)amino)methyl)phenyl)acryla te with a yield of 69.2%.

ESI-MS m/z=574.1 [M+H]+.

Step f): preparation of methyl (E)-3-(4-(((1-(5-bromo-4-cyano-6-(4-cyano-3-fluorophenyl)pyridin-2-yl)piperidin-4-yl) (tert-butoxycarbonyl)amino) methyl)phenyl)acrylate

Methyl (E)-3-(4-(((1-(5-bromo-4-cyano-6-(4-cyano-3-fluorophenyl)pyridin-2-yl)piperidin-4-yl)amino)methyl) phenyl)acryla te (170 mg, 0.53 mmol) was dissolved in DCM (3 mL), and di-tert-butyl dicarbonate (180 mg, 0.82 mmol) and triethylamine (0.17 mL, 1.23 mmol) were added. The reaction was conducted at room temperature overnight and monitored by LCMS until the starting materials were completely consumed, then dilute hydrochloric acid was added to adjust the pH to 7, and the mixture was extracted with EA. The organic phases were combined and concentrated to remove the solvent. The residue was mixed with silica gel and purified by silica gel column chromatography (eluent: EA:PE = 1:5), then purified by a normal-phase column to afford methyl (E)-3-(4-(((1-(5-bromo-4-cyano-6-(4-cyano-3-fluorophenyl) pyridin-2-yl)piperidin-4-yl)(tert-butoxycarbonyl)amino) methyl)phenyl)acrylate with a yield of 74%.

ESI-MS m/z: 674.2 $[M+H]^+$.

Step g) preparation of methyl (E)-3-(4-(((tert-butoxycarbonyl))(1-(4-cyano-6-(4-cyano-3-fluorophenyl)-5-(3-hydroxy-4-methoxyphenyl)pyridin-2-yl)piperidin-4-yl)amino)methyl)phenyl)acrylate

Methyl (E)-3-(4-(((1-(5-bromo-4-cyano-6-(4-cyano-3-fluorophenyl)pyridin-2-yl)piperidin-4-yl)(tert-butoxycarbonyl)amino) methyl)phenyl)acrylate (140.0 mg, 0.2 mmol), 2-methoxy-5-(tetramethyl-1,3,2-dioxaborolan-2-yl)phenol (75.1 mg, 0.3 mmol), cesium carbonate (195.5 mg, 0.6 mmol), and Pd(dppf)$Cl_2$ (14.6 mg, 0.02 mmol) were added to a mixture of Dioxane:$H_2O$=4:1 (2 mL), and the reaction liquid was subjected to microwave heating at 115°C for 1.5 hours under nitrogen protection. The reaction was monitored by LCMS until the starting materials were completely consumed and the solution was concentrated. The residue was purified by silica gel column (eluent: EA:PE = 1:3) to afford methyl (E)-3-(4-(((tert-butoxycarbonyl))(1-(4-cyano-6-(4-cyano-3-fluorophenyl)-5-(3-hydroxy-4-methoxyphenyl)pyridin-2-yl)piperidin-4-yl)amino)methyl)phenyl)acrylate with a yield of 74.2%.

ESI-MS m/z=718.3 [M+H]+.

Step h): preparation of (E)-3-(4-(((tert-butoxycarbonyl))(1-(4-cyano-6-(4-cyano-3-fluorophenyl)-5-(3-hydroxy-4-methoxyphenyl)pyridin-2-yl)piperidin-4-yl)amino)methyl)phenyl)acrylic acid

Methyl 8-((1-(8-(3-(benzyloxy)-4-methylphenyl)-7-(4-cyano-3-fluorophenyl)imidazo[1,2-c]pyrimidin-5-yl)piperidin-4-yl)a mino)-8-oxooctanoate (100 mg, 0.14 mmol) and lithium hydroxide (33.5 mg, 1.4 mmol) were added to a reaction liquid containing THF (2.5 mL), MeOH (1.5 mL), and water (1 mL), and the mixture was stirred at room temperature for 1 hour. The pH of the reaction liquid was adjusted to 4 with 2M HCl, then water (5 mL)was added to quench the reaction, and the mixture was extracted with ethyl acetate (5 mL × 2). The organic phases were combined, washed with saturated brine (5 mL × 2), dried over anhydrous sodium sulfate, filtered, and concentrated to afford (E)-3-(4-(((tert-butoxycarbonyl))(1-(4-cyano-6-(4-cyano-3-fluorophenyl)-5-(3-hydroxy-4-methoxyphenyl)pyridin-2-yl)piperidin-4-yl)amino)methyl)phenyl)acrylic acid with a yield of 71.1%.

ESI-MS m/z=704.3 [M+H]+.

Step i): preparation of tert-butyl (E)-(1-(4-cyano-6-(4-cyano-3-fluorophenyl)-5-(3-hydroxy-4-methoxyphenyl)pyridin-2-yl)piperidin-4-yl)(4-(3-oxo-3-(((tetrahydro-2H-pyran-2-yl)oxy)amino)prop-1-en-1-yl)benzyl)carbamate

(E)-3-(4-(((Tert-butoxycarbonyl))(1-(4-cyano-6-(4-cyano-3-fluorophenyl)-5-(3-hydroxy-4-methoxyphenyl)pyri din-2-yl)piperidin-4-yl)amino)methyl)phenyl)acrylic acid (70 mg, 0.099 mmol), O-(tetrahydro-2H-pyran-2-yl)hydroxylamine (13.9 mg, 0.12 mmol), HATU (56.5 mg, 0.15 mmol), and DIEA (38.4 mg, 0.3 mmol) were added to a reaction liquid containing DMF (2 mL), and the mixture was stirred at room temperature for 1 hour. Water (5 mL) was added to quench the reaction, followed by extraction with ethyl acetate (5 mL × 2). The organic phases were combined and washed with saturated brine (3 mL × 2), dried with anhydrous sodium sulfate, and filtered. The residue was purified by silica gel chromatography (eluent: PE:EA = 3:1) to afford tert-butyl (E)-(1-(4-cyano-6-(4-cyano-3-fluorophenyl)-5-(3-hydroxy-4-methoxyphenyl)pyridin-2-yl)piperidin-4-yl)(4-(3-oxo-3-(((tetrahydro-2H-pyran-2-yl)oxy)amino)prop-1-en-1-yl)benzyl) carbamate with a yield of 75.5%.

ESI-MS m/z=803.4 [M+H]+.

Step j): preparation of (E)-3-(4-(((1-(4-cyano-6-(4-cyano-3-fluorophenyl)-5-(3-hydroxy-4-methoxyphenyl)pyridin-2-yl) piperidin-4-yl)ami no)methyl)phenyl)-N-hydroxyacrylamide formate

4.0M Hydrochloric acid solution in EA (3 mL) was added to a reaction flask containing tert-butyl (E)-(1-(4-cyano-6-(4-cyano-3-fluorophenyl)-5-(3-hydroxy-4-methoxyphenyl)pyridin-2-yl)piperidin-4-yl)(4-(3-oxo-3-(((tetrahydro-2H-pyran-2-yl)oxy)amino)prop-1-en-1-yl)benzyl)carbamate (60 mg, 0.075 mmol), and the mixture was stirred at room temperature for 1 hour. After concentrating the reaction liquid, the residue was purified by Prep-HPLC (separation method 3) to afford (E)-3-(4-(((1-(4-cyano-6-(4-cyano-3-fluorophenyl)-5-(3-hydroxy-4-methoxyphenyl)pyridin-2-yl)piperidin-4-yl) ami no)methyl)phenyl)-N-hydroxyacrylamide formate with a yield of 23.7%.

$^1$H NMR (400 MHz, DMSO-$d_6$) δppm: 10.78 (s, 1H), 9.13 (s, 1H), 9.05 (s, 1H), 7.81 (t, *J* = 7.4 Hz, 1H), 7.61 (s, 2H), 7.57 (d, *J* = 13.4 Hz, 3H), 7.47 (d, *J* = 15.8 Hz, 1H), 7.40 (dd, *J* = 10.6, 1.4 Hz, 1H), 7.22 (dd, *J* = 8.2, 1.6 Hz, 1H), 6.91 (d, *J* = 8.4 Hz, 1H), 6.65 - 6.55 (m, 2H), 6.49 (d, *J* = 15.8 Hz, 1H), 4.49 (s, 2H), 4.14 (s, 2H), 3.78 (s, 3H), 3.00 (t, *J* = 12.6 Hz, 2H), 2.53 (d, *J* = 10.8 Hz, 1H), 2.14 (s, 2H), 1.56 (s, 2H).

ESI-MS m/z=619.2[M+H]+.

**Example 177**

$N^1$-(1-(3-Cyano-4-(4-cyano-3-fluorophenyl)-5-(3-hydroxy-4-methoxyphenyl)pyridin-2-yl)piperidin-4-yl)-$N^8$-h ydroxyoctanediamide formate was prepared according to the synthetic method of Example 58, and the structure and characterization data are as follows:

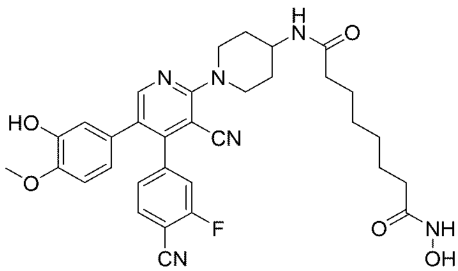

$^1$H NMR (400 MHz, MeOD) δppm: 8.40 (d, *J* = 2.2 Hz, 1H), 7.73 (t, *J* = 7.4 Hz, 1H), 7.36 (d, *J* = 9.8 Hz, 1H), 7.23 (dd, *J* = 8.0, 1.4 Hz, 1H), 6.86 - 6.78 (m, 1H), 6.54 - 6.47 (m, 2H), 4.29 (d, *J* = 13.2 Hz, 2H), 4.02 - 3.88 (m, 1H), 3.81 (s, 3H), 3.23 (t, *J* = 12.0 Hz, 2H), 2.19 (t, *J* = 7.4 Hz, 2H), 2.08 (t, *J* = 7.4 Hz, 2H), 2.04 - 1.94 (m, 2H), 1.65 - 1.59 (m, 6H), 1.39 - 1.30 (m, 4H).

ESI-MS m/z = 615.3 $[M+H]^+$.

**Examples 178-230** were prepared according to the synthetic method of Example 37 (the separation method for the compounds: hydrochloride and formate were prepared according to separation method 1 and 3, respectively), and the structure and characterization data are as follows.

| Example | Chemical name | Structure | $^1$H NMR | MS$(M+H)^+$ |
|---|---|---|---|---|
| 178 | (E)-3-(5-(((1-(3-Cyano -4-(4-cyano-3-fluoroph enyl)-5-(3-hydroxy-4-methoxyphenyl)pyridi n-2-yl)piperidin-4-yl)a mino)methyl)furan-2-y l)-N-hydroxyacrylamid e formate | 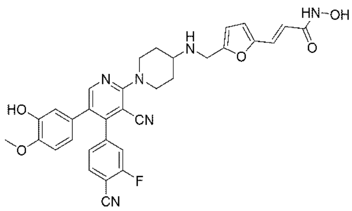 | $^1$H NMR (400 MHz, DMSO-$d_6$) δ 10.85 (s, 1H), 9.03 (s, 1H), 8.50 (s, 1H), 8.47 (s, 1H), 7.98 (dd, *J* = 8.0, 6.8 Hz, 1H), 7.68 (dd, *J* = 10.2, 1.6 Hz, 1H), 7.37 - 7.20 (m, 2H), 6.87 - 6.72 (m, 3H), 6.50 - 6.42 (m, 2H), 6.29 (d, *J* = 5.6 Hz, 1H), 4.38 (s, 2H), 4.31 (d, *J* = 8.2 Hz, 2H), 3.72 (s, 3H), 3.50 (s, 1H), 3.13 (t, *J* = 6.6 Hz, 2H), 2.23 (d, *J* = 5.8 Hz, 2H), 1.79 - 1.63 (m, 2H). | 609.2 |
| 179 | (E)-3-(4-(2-((1-(3-Cya no-4-(4-cyano-3-fluoro phenyl)-5-(3-hydroxy-4-methoxyphenyl)pyri din-2-yl)piperidin-4-yl ) amino)propan-2-yl)ph enyl)-N-hydroxyacryla mide formate | 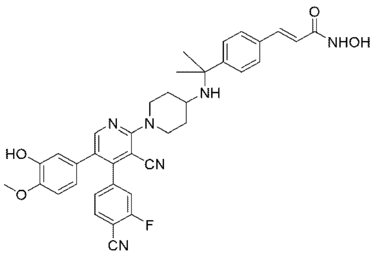 | $^1$H NMR (400 MHz, DMSO-$d_6$) δ 10.78 (s, 1H), 9.03 (m, 2H), 8.39 (s, 1H), 8.14 (s, 1H), 7.94 (dd, *J* = 8.0, 6.8 Hz, 1H), 7.73 - 7.54 (m, 5H), 7.47 (d, *J* = 16.0 Hz, 1H), 7.25 (dd, *J* = 8.0, 1.6 Hz, 1H), 6.87 - 6.73 (m, 1H), 6.55 - 6.40 (m, 2H), 4.09 (d, *J* = 13.2 Hz, 2H), 3.71 (s, 3H), 3.44 (d, *J* = 7.2 Hz, 1H), 3.00 (t, J = 12.4 Hz, 2H), 1.85 - 1.35 (m, 10H). | 647.3 |
| 180 | (E)-3-(4-(((1-(3-Cyano -4-(4-cyano-3-fluoroph enyl)-5-(3-hydroxy-4-methoxyphenyl)pyridi n-2-yl)piperidin-4-yl)a mino)methyl)-1H-inda zol-7-yl)-N-hydroxyac ry-lamide formate | 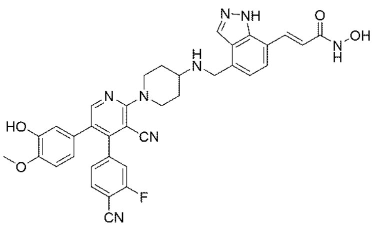 | $^1$H NMR (400 MHz, DMSO-$d_6$) δ 13.50 (s, 1H), 10.78 (s, 1H), 9.07 (s, 1H), 9.00 (s, 1H), 8.42 (s, 1H), 8.38 (s, 1H), 8.18 (s, 1H), 7.96 (m, 2H), 7.67 (dd, *J* = 10.0, 1.6 Hz, 1H), 7.58 (d, *J* = 7.4 Hz, 1H), 7.28 (dd, *J* = 8.0, 1.6 Hz, 1H), 7.22 (d, *J* = 7.4 Hz, 1H), 6.81 (d, *J* = 8.2 Hz, 1H), 6.51 - 6.41 (m, 2H), 4.20 (m, 4H), 3.71 (s, 3H), 3.17 (t, *J* = 11.8 Hz, 2H), 2.90 (s, 1H), 2.07 (d, *J* = 11.8 Hz, 2H), 1.54 (d, *J* = 11.4 Hz, 2H). | 659.3 |

(continued)

| Example | Chemical name | Structure | $^1H$ NMR | MS(M+H)$^+$ |
|---|---|---|---|---|
| 181 | (E) -3-(2-cyano-4-((1-(3-cyano-4-(4-cyano-3-flu orophenyl)-5-(3-hydroxy-4-methoxyphenyl)pyridin-2-yl)piperidin-4 -yl)amino)methyl)phenoxy)-N-hydroxyacetamide formate | 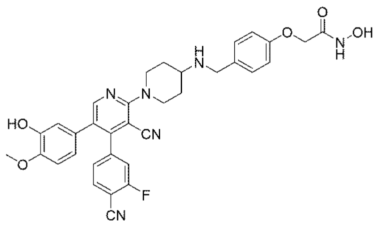 | $^1H$ NMR (400 MHz, DMSO-$d_6$) δ 10.85 (s, 1H), 9.01 (s, 1H), 8.97 (d, *J* = 2.6 Hz, 1H), 8.46 (s, 1H), 7.97 (dd, *J* = 8.0, 6.8 Hz, 1H), 7.67 (dd, *J* = 10.0, 1.6 Hz, 1H), 7.43 (d, *J* = 8.2 Hz, 2H), 7.28 (dd, *J* = 8.0, 1.4 Hz, 1H), 7.02 (d, *J* = 8.2 Hz, 2H), 6.82 (d, *J* = 8.0 Hz, 1H), 6.47 (d, *J* = 8.0 Hz, 2H), 4.49 (s, 2H), 4.29 (d, *J* = 13.0 Hz, 2H), 4.13 (s, 2H), 3.72 (s, 3H), 3.28 (s, 1H), 3.13 (t, *J* = 10.4 Hz, 2H), 2.21 (d, *J* = 12.0 Hz, 2H), 1.71 (s, 2H). | 623.2 |
| 182 | (E)-3-(5-(((1-(3-Cyano -4-(4-cyano-3-fluorophenyl)-5-(1H-indazol-6-yl) pyridin-2-yl)piperidi n-4-yl)amino)methyl)p yridin-2-yl)-N-hydroxy acrylamide formate | 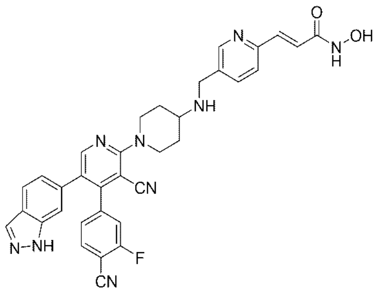 | $^1H$ NMR (400 MHz, Methanol-$d_4$) δ ppm 8.70 (d, *J* = 2.2 Hz, 1H), 8.56 (s, 1H), 8.29 (s, 1H), 8.05 - 7.94 (m, 2H), 7.74 - 7.58 (m, 4H), 7.44 (dd, *J* = 9.6, 1.6 Hz, 1H), 7.37 - 7.31 (m, 1H), 7.23 (dd, *J* = 8.0, 1.6 Hz, 1H), 6.96 (d, *J* = 15.4 Hz, 1H), 6.81 (dd, *J* = 8.4, 1.4 Hz, 1H), 4.51 (d, *J* = 13.4 Hz, 2H), 4.29 (s, 2H), 3.40 (s, 1H), 3.22 (t, *J* = 12.6 Hz, 2H), 2.32 (d, *J* = 12.4 Hz, 2H), 1.84 (t, *J* = 11.6 Hz, 2H). | 614.2 |
| 183 | 2-(4-(((1-(3-Cyano-4-( 4-cyano-3-fluorophenyl)-5-(3-hydroxy-4-methoxyphenyl)pyridin-2-yl) piperidin-4-yl)amin o) methyl)phenoxy)-N-hydroxypropanamide formate | 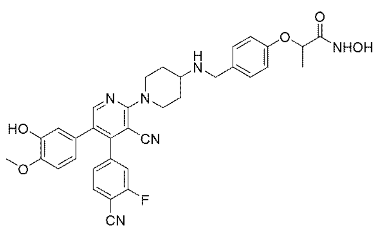 | $^1H$ NMR (400 MHz, DMSO-$d_6$) δ 10.86 (d, *J* = 1.8 Hz, 1H), 9.03 (s, 1H), 8.95 (d, *J* = 1.7 Hz, 1H), 8.46 (s, 1H), 7.97 (t, *J* = 7.4 Hz, 1H), 7.68 (dd, *J* = 10.0, 1.4 Hz, 1H), 7.43 (d, *J* = 8.2 Hz, 2H), 7.28 (dd, *J* = 8.0, 1.4 Hz, 1H), 6.99 (d, J = 8.2 Hz, 2H), 6.82 (d, *J* = 8.0 Hz, 1H), 6.47 (d, *J* = 8.4 Hz, 2H), 4.71 (q, *J* = 6.4 Hz, 1H), 4.30 (d, *J* = 13.2 Hz, 2H), 4.16 (s, 2H), 3.72 (s, 3H), 3.35 (s, 1H), 3.12 (t, *J* = 12.6 Hz, 2H), 2.23 (d, *J* = 11.8 Hz, 2H), 1.72 (d, *J* = 12.4 Hz, 2H), 1.44 (d, *J* = 6.4 Hz, 3H). | 637.3 |

(continued)

| Example | Chemical name | Structure | $^1$H NMR | MS(M+H)$^+$ |
|---|---|---|---|---|
| 184 | (E)-3-(5-((1-(3-Cyano-4-(4-cyano-3-fluorophenyl)-5-(1H-indazol-6-yl)pyridin-2-yl)piperidin-4-yl)amino)methyl)pyrimidin-2-yl)-N-hydroxyacrylamide formate | 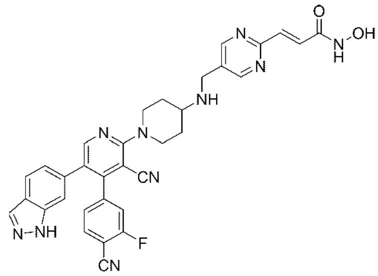 | $^1$H NMR (400 MHz, DMSO-$d_6$) δ 13.05 (s, 1H), 11.04 (s, 1H), 9.39 - 9.10 (m, 1H), 8.84 (s, 2H), 8.56 (s, 1H), 8.31 (s, 1H), 8.01 (s, 1H), 7.89 (t, $J$ = 7.4 Hz, 1H), 7.72 (dd, $J$ = 10.1, 1.4 Hz, 1H), 7.60 (d, $J$ = 8.3 Hz, 1H), 7.43 - 7.26 (m, 3H), 7.14 (d, $J$ = 15.5 Hz, 1H), 6.73 (dd, $J$ = 8.3, 1.4 Hz, 1H), 4.25 (d, $J$ = 13.1 Hz, 2H), 3.90 (s, 2H), 3.22 (t, $J$ = 11.8 Hz, 2H), 2.83 (s, 1H), 2.05 (d, $J$ = 12.4 Hz, 2H), 1.50 (d, $J$ = 12.8 Hz, 2H). | 615.2 |
| 185 | 3-(5-((1-(3-Cyano-4-(4-cyano-3-fluorophenyl)-5-(3-hydroxy-4-(2-hydroxy-2-methylpropoxy)phenyl)pyridin-2-yl)piperidin-4-yl)amino)methyl)thiazol-2-yl)-N-hydroxypropanamide formate | 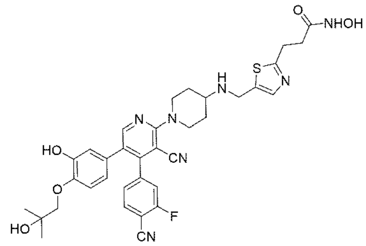 | $^1$H NMR (400 MHz, DMSO-$d_6$) δ 10.44 (s, 1H), 9.36 (s, 1H), 8.74 (m, 1H), 8.46 (s, 1H), 7.97 (dd, $J$ = 8.0, 6.8 Hz, 1H), 7.74 - 7.57 (m, 2H), 7.29 (dd, $J$ = 8.0, 1.6 Hz, 1H), 6.78 (d, $J$ = 8.4 Hz, 1H),6.56-6.38 (m, 2H), 4.66 (m, 2H), 4.31 (d, $J$ = 13.2 Hz, 2H), 3.65 (s, 2H), 3.45 (s, 1H), 3.19 - 3.05 (m, 4H), 2.34 (t, $J$ = 7.2 Hz, 2H), 2.22 (d, $J$ = 12.0 Hz, 2H), 1.83 - 1.68 (m, 2H), 1.18 (s, 6H). | 686.3 |
| 186 | (E)-3-(5-(((1-(3-Cyano-4-(4-cyano-3-fluorophenyl)-5-(3-hydroxy-4-(trifluoromethyl)phenyl)pyridin-2-yl)piperidin-4-yl)amino)methyl)pyrimidin-2-yl)-N-hydroxyacrylamide formate | 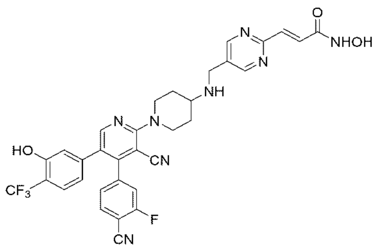 | $^1$H NMR (400 MHz, DMSO-$d_6$) δ 11.08 (s, 1H), 10.59 (s, 1H), 9.24 (s, 1H), 8.97 (s, 2H), 8.51 (s, 1H), 7.98 (dd, $J$ = 8.0, 6.8 Hz, 1H), 7.76 (dd, $J$ = 10.0, 1.5 Hz, 1H), 7.44 - 7.34 (m, 2H), 7.28 (dd, $J$ = 8.0, 1.5 Hz, 1H), 7.18 (d, $J$ = 15.5 Hz, 1H), 6.67 (d, J = 9.5 Hz, 2H), 4.35 (d, $J$ = 13.0 Hz, 2H), 4.20 (s, 2H), 3.21 (s, 2H), 2.61 (t, $J$ = 12.5 Hz, 1H), 1.70 (s, 2H), 1.24 (s, 2H). | 659.2 |

(continued)

| Example | Chemical name | Structure | $^1H$ NMR | MS(M+H)$^+$ |
|---|---|---|---|---|
| 187 | (E)-3-(5-(((1-(3-Cyano -4-(4-cyano-3-fluoroph enyl)-5-(3-hydroxy-4-( 2-hydroxy-2-methylpr opoxy)phenyl)pyridin-2-yl)piperidin-4-yl)ami no)methyl)pyrimidin-2 -yl)-N-hydroxyacrylam ide formate | 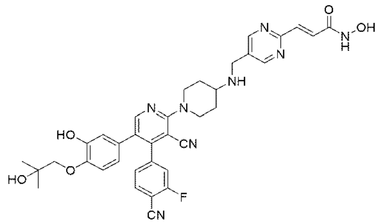 | $^1H$ NMR (400 MHz, DMSO-d6) δ ppm : 11.09 (s, 1H), 9.26 (s, 1H), 8.98 (s, 2H), 8.70 (s, 1H), 8.46 (d, J = 6.4 Hz, 1H), 7.97 (dd, J = 8.0, 6.8 Hz, 1H), 7.67 (dd, J = 10.0, 1.5 Hz, 1H), 7.39 (d, J = 15.5 Hz, 1H), 7.30 (dd, J = 8.0, 1.5 Hz, 1H), 7.20 (d, J = 15.4 Hz, 1H), 6.78 (d, J = 8.4 Hz, 1H), 6.51 (d, J = 2.2 Hz, 1H), 6.44 (dd, J = 8.2, 2.2 Hz, 1H), 4.70 (s, 1H), 4.32 (d, J = 12.4 Hz, 4H), 3.65 (s, 2H), 3.17 (t, J = 12.4 Hz, 2H), 2.61 (t, J = 12.5 Hz, 1H), 2.26 (d, J = 12.0 Hz, 2H), 1.75 (d, J = 12.7 Hz, 2H), 1.18 (s, 6H). | 679.3 |
| 188 | 3-(4-(((1-(3-Cyano-4-( 4-cyano-3-fluoropheny l)-5-(3-hydroxy-4-(2-h ydroxy-2-methylpropo xy)phenyl)pyridin-2-yl ) piperidin-4-yl)amino) methyl)phenyl)-N-hydr oxypropanamide formate | 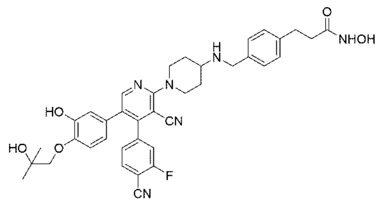 | $^1H$ NMR (400 MHz, DMSO-$d_6$) δ 10.45 (s, 1H), 8.68 (s, 1H), 8.42 (s, 1H), 8.16 (s, 1H), 7.95 (dd, *J* = 8.0, 6.8 Hz, 1H), 7.65 (dd, *J* = 10.0, 1.4 Hz, 1H), 7.34 - 7.25 (m, 3H), 7.17 (d, *J* = 7.8 Hz, 2H), 6.77 (d, *J* = 8.4 Hz, 1H), 6.50 (d, *J* = 2.2 Hz, 1H), 6.43 (dd, *J* = 8.2, 2.2 Hz, 1H), 4.20 (d, *J* = 13.0 Hz, 2H), 3.84 (s, 2H), 3.65 (s, 2H), 3.16 (t, *J* = 11.8 Hz, 2H), 2.87 (s, 1H), 2.80 (t, *J* = 7.6 Hz, 2H), 2.61 (t, *J* = 12.5 Hz, 1H), 2.25 (t, *J* = 7.6 Hz, 2H), 2.04 (d, *J* = 12.0 Hz, 2H), 1.51 (q, *J* = 11.0, 10.6 Hz, 2H), 1.18 (s, 6H). | 679.3 |
| 189 | (E)-3-(2-Cyano-4-((1-( 3-cyano-4-(4-cyano-3-fluorophenyl)-5-(3-hyd roxy-4-(2-hydroxy-2-methylpropoxy)phenyl ) pyridin-2-yl)piperidin -4-yl)amino)methyl)ph en-yl)-N-hydroxyacrylamide formate | 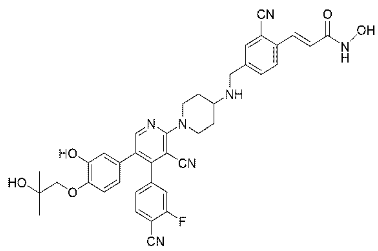 | $^1H$ NMR (400 MHz, Metha-nol-$d_4$) δ 8.51 (s, 1H), 8.20 (s, 1H), 7.85 (d, *J* = 4.2 Hz, 2H), 7.76 (t, *J* = 13.0 Hz, 2H), 7.65 (dd, *J* = 8.0, 6.6 Hz, 1H), 7.27 (dd, *J* = 9.6, 1.4 Hz, 1H), 7.14 (dd, *J* = 8.0, 1.6 Hz, 1H), 6.77 - 6.59 (m, 2H), 6.46 (d, *J* = 2.2 Hz, 1H), 6.39 (dd, *J* = 8.2, 2.2 Hz, 1H), 4.37 (d, *J* = 13.3 Hz, 2H), 4.28 (s, 2H), 3.68 (s, 2H), 3.42 (s, 1H), 3.10 (t, *J* = 12.6 Hz, 2H), 2.24 (d, *J* = 11.8 Hz, 2H), 1.77 (d, *J* = 12.4 Hz, 2H), 1.20 (s, 6H). | 702.3 |

(continued)

| Example | Chemical name | Structure | $^1$H NMR | MS(M+H)$^+$ |
|---|---|---|---|---|
| 190 | 3-(4-((1-(3-Cyano-4-(4 -cyano-3-fluorophenyl) -5-(3-hydroxy-4-(triflu oromethyl)phenyl)pyri din-2-yl)piperidin-4-yl ) amino)methyl)phenyl) -N-hydroxypropanami de formate | 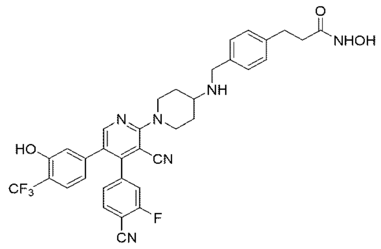 | $^1$H NMR (400 MHz, DMSO-$d_6$) δ 10.57 (s, 1H), 10.35 (s, 1H), 8.68 (s, 1H), 8.52 (s, 1H), 8.48 (d, *J* = 2.0 Hz, 1H), 8.14 (s, 1H), 7.97 (t, *J* = 7.5 Hz, 1H), 7.75 (d, *J* = 9.9 Hz, 1H), 7.40 (d, *J* = 8.3 Hz, 1H), 7.31 (d, *J* = 7.6 Hz, 1H), 7.27 (d, *J* = 8.2 Hz, 1H), 7.17 (s, 1H), 6.67 (s, 2H), 4.27 (d, *J* = 13.1 Hz, 2H), 3.87 (s, 2H), 3.19 (d, *J* = 12.4 Hz, 2H), 2.80 (t, *J* = 7.6 Hz, 2H), 2.33 (s, 1H), 2.25 (t, *J* = 7.7 Hz, 2H), 2.06 (d, *J* = 12.5 Hz, 2H), 1.53 (d, *J* = 11.9 Hz, 2H). | 659.2 |
| 191 | 3-(5-(((1-(3-Cyano-4-( 4-cyano-3-fluoropheny l)-5-(3-hydroxy-4-met hoxyphenyl)pyridin-2-yl) piperidin-4-yl)amin o) methyl)pyridin-2-yl) -N-hydroxypropanami de formate | 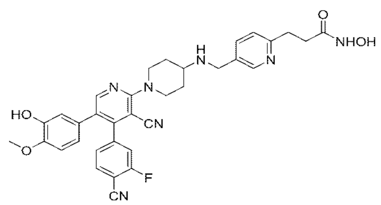 | $^1$H NMR (400 MHz, DMSO-$d_6$) δ ppm : 10.39 (s, 1H), 8.99 (s, 1H), 8.67 (s, 1H), 8.52 (s, 1H), 8.$^{44}$ (s, 1H), 7.96 (t, J = 7.4 Hz, 1H), 7.76 (s, 1H), 7.66 (d, J = 10.1 Hz, 1H), 7.28 (d, J = 8.0 Hz, 2H), 6.81 (d, J = 8.1 Hz, 1H), 6.47 (d, J = 9.0 Hz, 2H), 4.24 (d, J = 13.1 Hz, 2H), 4.00 (s, 2H), 3.76 - 3.63 (m, 3H), 3.16 (t, J = 12.3 Hz, 3H), 2.96 (t, J = 7.7 Hz, 2H), 2.39 (t, J = 7.7 Hz, 2H), 2.11 (s, 2H), 1.59 (s, 2H). | 622.3 |
| 192 | 2-(4-(((1-(3-cyano-4-(4 -cyano-3-fluorophenyl) -5-(3-hydroxy-4-(2-hy droxy-2-methylpropox y) phenyl)pyridin-2-yl) piperidin-4-yl)amino) methyl)phenoxy)-N-hy droxy acetamide formate | 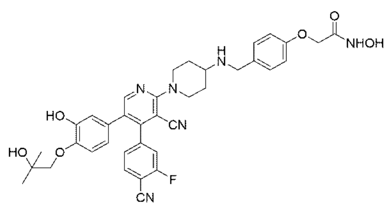 | $^1$H NMR (400 MHz, DMSO-$d_6$) δ ppm : 10.83 (s, 1H), 9.04 - 8.89 (m, 1H), 8.70 (s, 1H), 8.45 (s, 1H), 7.97 (dd, J = 8.0, 6.8 Hz, 1H), 7.67 (dd, J = 10.1, 1.5 Hz, 1H), 7.41 (d, J = 8.3 Hz, 2H), 7.29 (dd, J = 8.0, 1.5 Hz, 1H), 7.00 (d, J = 8.6 Hz, $^{2H}$), 6.78 (d, J = 8.4 Hz, 1H), 6.50 (d, J = 2.2 Hz, 1H), 6.43 (dd, J = 8.3, 2.2 Hz, 1H), 4.71 (s, 1H), 4.48 (s, 2H), 4.28 (d, J = 13.3 Hz, 2H), 4.07 (s, 2H), 3.65 (s, 2H), 3.13 (t, J = 12.9 Hz, 2H), 3.13 (t, J = 12.9 Hz, 2H), 2.65 (d, J = 12.0 Hz, 1H), 1.66 (d, J = 12.1 Hz, 2H), 1.18 (s, 6H). | 681.3 |
| 193 | 3-(2-Cyano-4-((1-(3-cy ano-4-(4-cyano-3-fluor ophenyl)-5-(3-hydroxy -4-methoxyphenyl)pyri din-2-yl)piperidin-4-yl ) amino)methyl)phenyl) | 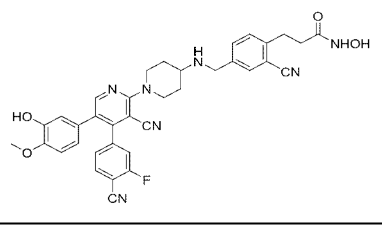 | $^1$H NMR (400 MHz, DMSO-$d_6$) δ 10.40 (s, 1H), 8.99 (s, 1H), 8.41 (s, 1H), 8.16 (s, 1H), 7.95 (dd, *J* = 8.0, 6.8 Hz, 1H), 7.76 (d, *J* = 1.8 Hz, 1H), 7.64 (ddd, *J* = 13.6, 9.0, 1.6 Hz, 2H), 7.39 (d, *J* = 8.0 Hz, 1H), | 646.2 |

(continued)

| Example | Chemical name | Structure | $^1$H NMR | MS(M+H)$^+$ |
|---|---|---|---|---|
| | -N-hydroxypropanami de formate | | 7.27 (dd, *J* = 8.0, 1.6 Hz, 1H), 6.80 (d, *J* = 8.0 Hz, 1H), 6.51 - 6.42 (m, 2H), 4.16 (d, *J* = 13.0 Hz, 2H), 3.80 (s, 2H), 3.71 (s, 3H), 3.17 (t, *J* = 12.0 Hz, 2H), 3.00 (t, *J* = 7.6 Hz, 2H), 2.70 (s, 1H), 2.34 (t, *J* = 7.6 Hz, 2H), 1.98 (d, *J* = 12.6 Hz, 2H), 1.44 (d, *J* = 11.6 Hz, 2H). | |
| 194 | 3-(6-((1-(3-Cyano-4-(4 -cyano-3-fluorophenyl) -5-(3-hydroxy-4-(meth ylamino)phenyl)pyridi n-2-yl)piperidin-4-yl)a mino)methyl)pyridin-3 -yl)-N-hydroxypropana mide hydrochloride | 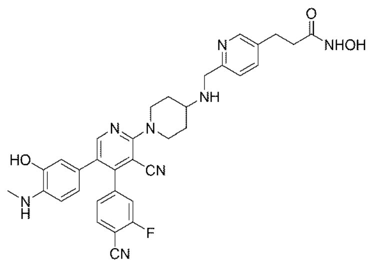 | $^1$H NMR (400 MHz, DMSO-$d_6$) δ 10.79 (s, 1H), 10.44 (s, 1H), 9.52 (s, 2H), 8.54 (s, 1H), 8.48 (s, 1H), 7.97 (dd, *J* = 8.0, 6.8 Hz, 1H), 7.79 (d, *J* = 8.0 Hz, 1H), 7.71 (d, *J* = 10.0 Hz, 1H), 7.57 (d, *J* = 9.2 Hz, 1H), 7.32 - 7.21 (m, 2H), 6.65 (d, *J* = 8.0 Hz, 2H), 4.36 (d, *J* = 8.8 Hz, 4H), 3.44 (s, 1H), 3.14 (t, *J* = 12.8 Hz, 2H), 2.94 - 2.85 (m, 2H), 2.79 (d, *J* = 1.6 Hz, 3H), 2.38 - 2.23 (m, 4H), 1.83 (d, *J* = 12.4 Hz, 2H). | 621.3 |
| 195 | 3-(5-(1-(3-Cyano-4-(4-cyano-3-fluorophenyl)-5-(3-hydroxy-4-tri-fluor omethylphenyl)pyri-din -2-yl)piperidin-4-ylami no)methyl)pyrimidin-2 -yl)-N-hydroxypropana mide formate | 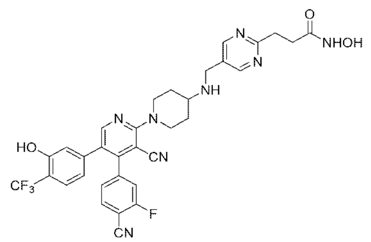 | $^1$H NMR (400 MHz, Metha-nol-$d_4$) δ 8.78 (s, 2H), 8.54 (s, 1H), 8.45 (s, 1H), 7.75 (dd, *J* = 8.0, 6.6 Hz, 1H), 7.43 (dd, *J* = 9.7, 1.5 Hz, 1H), 7.37 (d, *J* = 8.0 Hz, 1H), 7.20 (dd, *J* = 8.0, 1.5 Hz, 1H), 6.64 (d, J = 9.9 Hz, 2H), 4.47 (d, *J* = 13.5 Hz, 2H), 4.12 (s, 2H), 3.26 (d, *J* = 7.4 Hz, 2H), 3.24 (s, 1H), 3.19 (d, *J* = 12.8 Hz, 2H), 2.64 (t, *J* = 7.4 Hz, 2H), 2.23 (d, *J* = 12.3 Hz, 2H), 1.72 (q, *J* = 11.5 Hz, 2H). | 661.2 |
| 196 | (E)-3-(5-(((1-(3-Cyano -4-(4-cyano-3-fluoroph enyl)-5-(3-hydro-xy-4-( methylamino)phe-nyl)p yridin-2-yl)piperidin-4 -yl)amino)methyl) pyri midin-2-yl)-N-hydro-xy acrylamide hydro-chloride | 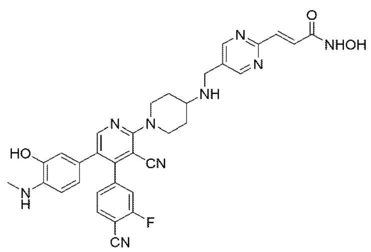 | $^1$H NMR (400 MHz, DMSO-$d_6$) δ 10.80 (s, 1H), 10.44 (s, 1H), 9.87 (s, 2H), 9.12 - 9.07 (m, 2H), 8.49 (s, 1H), 8.00 - 7.93 (m, 1H), 7.72 (dd, *J* = 10.0, 1.6 Hz, 1H), 7.42 - 7.17 (m, 4H), 6.67 (d, *J* = 7.2 Hz, 2H), 4.39 - 4.30 (m, 4H), 4.18 - 4.16 (m, 1H), 3.45 (d, *J* = 7.2 Hz, 1H), 3.18 (t, *J* = 12.4 Hz, 2H), 2.80 (s, 3H), 2.31 (m, 2H), 1.85 (m, 2H). | 620.3 |

(continued)

| Example | Chemical name | Structure | $^1$H NMR | MS(M+H)$^+$ |
|---|---|---|---|---|
| 197 | (E)-3-(4-(((1-(3-Cyano -4-(4-cyano-3-fluoroph enyl)-5-(1-(2-hydroxy-2-methylpropyl)-1H-be nzo [d][1,2,3]triazol-5-yl)pyridin-2-yl)piperidi n-4-yl) amino)methyl)p henyl)-N-hydroxyacryl amide formate | 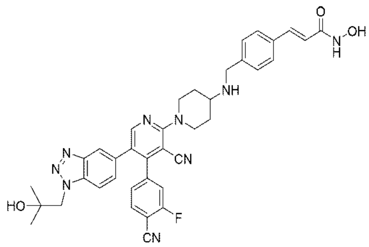 | $^1$H NMR (400 MHz, DMSO-$d_6$) δ 10.72 (s, 1H), 8.57 (s, 1H), 8.19 (s, 1H), 7.91 (t, *J* = 7.4 Hz, 1H), 7.82 (d, *J* = 1.4 Hz, 1H), 7.77 - 7.66 (m, 2H), 7.52 (d, *J* = 7.8 Hz, 2H), 7.50 - 7.40 (m, 3H), 7.34 (dd, *J* = 7.9, 1.5 Hz, 1H), 7.13 (dd, *J* = 8.6, 1.5 Hz, 1H), 6.44 (d, *J* = 5.8 Hz, 1H), 4.77 (s, 1H), 4.55 (s, 2H), 4.24 (d, *J* = 13.0 Hz, 2H), 3.83 (s, 2H), 3.22 (t, *J* = 12.0 Hz, 2H), 2.77 (s, 1H), 2.01 (t, *J* = 80 Hz, 2H), 1.48 (d, *J* = 11.4 Hz, 2H), 1.12 (d, *J* = 3.4 Hz, 6H). | 686.3 |
| 198 | (Z)-3-(4-(((1-(3-Cyano -4-(4-cyano-3-fluoroph enyl)-5-(3-hydroxy-4-( 2-hydroxy-2-methylpr opoxy)phenyl)pyridin-2-yl)piperidin-4-yl)ami no) methyl)phenyl)-2-fl uoro-N-hydroxyacryla mide formate | 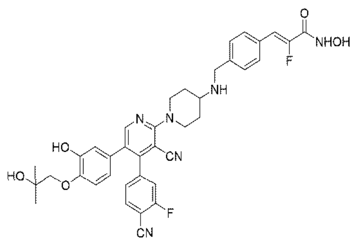 | $^1$H NMR (400 MHz, DMSO-$d_6$) δ ppm : 9.26 (s, 1H), 8.69 (s, 1H), 8.43 (d, J = 1.5 Hz, 1H), 8.14 (s, 1H), 7.96 (t, J = 7.4 Hz, 1H), 7.71 - 7.59 (m, 2H), 7.51 (dd, J = 18.6, 8.0 Hz, 2H), 7.40 (d, J = 7.9 Hz, 1H), 7.29 (dd, J = 8.0, 1.5 Hz, 1H), 6.77 (d, J = 8.6 Hz, 2H), 6.50 (d, J = 2.1 Hz, 1H), 6.43 (dd, J = 8.3, 2.2 Hz, 1H), 4.71 (s, 1H), 4.21 (d, J = 12.7 Hz, 2H), 3.94 (s, 2H), 3.65 (s, 2H), 3.16 (t, J = 12.0 Hz, 2H), 2.61 *(t, J* = 12.5 Hz, 1H), 2.07 (s, 2H), 1.53 (d, J = 11.8 Hz, 2H), 1.18 (s, 6H). | 695.3 |
| 199 | (E)-3-(5-((1-(3-Cyano-4-(4-cyano-3-fluoroph enyl)-5-(3-hydroxy-4-( 2-hydroxy-2-methylpr opoxy)phenyl)pyridin-2-yl)piperidin-4-yl)ami no) methyl)pyridin-2-yl )-N-hydroxyacrylamid e for-mate | 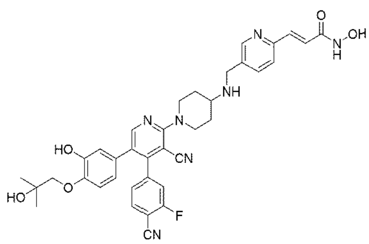 | $^1$H NMR (400 MHz, DMSO-$d_6$) δ 10.88 (s, 1H), 9.09 (s, 1H), 8.68 (s, 1H), 8.60 (d, *J* = 2.1 Hz, 1H), 8.42 (s, 1H), 8.14 (s, 1H), 7.96 (dd, *J* = 8.0, 6.8 Hz, 1H), 7.83 (dd, *J* = 7.9, 2.2 Hz, 1H), 7.66 (dd, *J* = 10.1, 1.5 Hz, 1H), 7.55 (d, *J* = 7.9 Hz, 1H), 7.29 (dd, *J* = 8.0, 1.5 Hz, 1H), 6.91 (d, *J* = 15.4 Hz, 1H), 6.77 (d, *J* = 8.3 Hz, 1H), 6.50 (d, *J* = 2.2 Hz, 1H), 6.43 (dd, *J* = 8.2, 2.2 Hz, 1H), 4.71 (s, 1H), 4.19 (d, *J* = 13.1 Hz, 2H), 3.89 (s, 2H), 3.64 (s, 2H), 3.17 (t, *J* = 12.1 Hz, 2H), 2.54 (s, 1H), 2.02 (d, *J* = 12.3 Hz, 2H), 1.48 (q, *J* = 11.2 Hz, 2H), 1.18 (s, 6H). | 678.3 |

(continued)

| Example | Chemical name | Structure | $^1H$ NMR | MS(M+H)$^+$ |
|---|---|---|---|---|
| 200 | (E)-3-( 4-(((1-(3-Cyano -4-(4-cyano-3-fluorophenyl)-5-(3-hydroxy-4-methoxyphenyl)pyridin-2-yl)piperidin-4-yl)amino)methyl)-2-fluorophenyl)-N-hydroxyacrylamide formate | 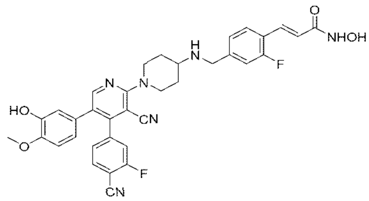 | $^1H$ NMR (400 MHz, DMSO-$d_6$) δ 9.10 (s, 1H), 9.00 (s, 1H), 8.51 (s, 1H), 8.43 (s, 1H), 7.96 (t, *J* = 7.4 Hz, 1H), 7.70 - 7.63 (m, 2H), 7.50 (d, *J* = 15.9 Hz, 1H), 7.35 (dd, *J* = 22.7, 9.7 Hz, 2H), 7.28 (dd, *J* = 8.0, 1.5 Hz, 1H), 6.81 (d, *J* = 8.1 Hz, 1H), 6.57 (d, *J* = 15.9 Hz, 1H), 6.47 (d, *J* = 8.5 Hz, 2H), 4.22 (d, *J* = 12.9 Hz, 2H), 4.01 (s, 2H), 3.71 (s, 3H), 3.38 (m, 1H),3.15 (t, *J* = 12.3 Hz, 2H), 2.08 (s, 2H), 1.57 (s, 2H). | 637.2 |
| 201 | 3-(4-((1-(3-Cyano-4-(4 -cyano-3-fluorophenyl) -5-(3-hydroxy-4-methoxyphenyl)pyridin-2-yl) piperidin-4-yl)amino) methyl)-2-fluorophenyl)-N-hydroxypropanamide formate | 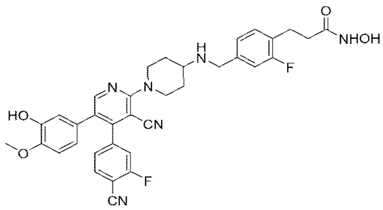 | $^1H$ NMR (400 MHz, Methanol-$d_4$) δ 8.49 (s, 1H), 8.34 (s, 1H), 7.65 (dd, *J* = 8.0, 6.6 Hz, 1H), 7.32 - 7.23 (m, 2H), 7.21 - 7.10 (m, 3H), 6.78 - 6.68 (m, 1H), 6.43 - 6.39 (m, 2H), 4.35 (d, *J* = 13.5 Hz, 2H), 4.15 (s, 2H), 3.72 (s, 3H), 3.40 - 3.28 (m, 2H), 3.13 - 3.01 (m, 2H), 2.89 (t, *J* = 7.5 Hz, 1H), 2.31 (t, *J* = 7.5 Hz, 2H), 2.20 (d, *J* = 12.5 Hz, 2H), 1.80 - 1.68 (m, 2H). | 639.3 |
| 202 | (E)-3-5-(((1-(4-(4-Cyano-3-fluorophenyl)-5-( 3-hydroxy-4-methoxyphenyl)-3-methylpyridin -2-yl)piperidin-4-yl)amino) methyl)pyrimidin-2-yl)-N-hydroxyacrylamide formate | 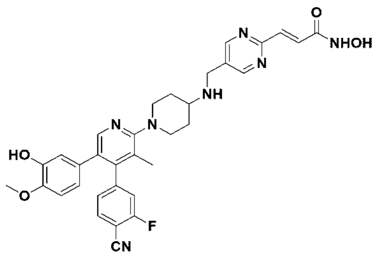 | $^1H$ NMR (400 MHz, DMSO-$d_6$) δ 9.86 (s, 2H), 9.09 (s, 2H), 8.51 (s, 1H), 8.13 (s, 1H), 7.90 (dd, *J* = 8.0, 6.8 Hz, 1H), 7.44 (dd, *J* = 10.0, 1.6 Hz, 1H), 7.38 (d, *J* = 15.6 Hz, 1H), 7.21 (d, *J* = 15.6 Hz, 1H), 7.12 (dd, *J* = 7.6, 1.6 Hz, 1H), 6.78 (d, *J* = 8.4 Hz, 1H), 6.56 - 6.30 (m, 2H), 4.30 (t, *J* = 6.0 Hz, 2H), 3.71 (s, 3H), 3.64 (d, *J* = 12.4 Hz, 2H), 3.37 (s, 1H), 3.02 - 2.85 (m, 2H), 2.28 (d, *J* = 11.6 Hz, 2H), 2.01 (s, 3H), 1.92 (d, *J* = 11.6 Hz, 2H). | 610.3 |

(continued)

| Example | Chemical name | Structure | $^1$H NMR | MS(M+H)$^+$ |
|---|---|---|---|---|
| 203 | (E)-3-(4-(((1-(3-Cyano -4-(4-cyano-3-fluorophenyl)-5-(3-hydroxy-4-morpholinophenyl)pyridin-2-yl)piperidin-4-yl ) amino)methyl)-N-hydroxyacrylamide formate | 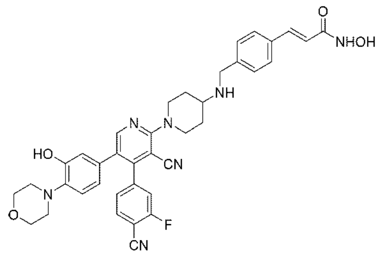 | $^1$H NMR (400 MHz, Methanol-$d_4$) δppm 8.45 (d, *J* = 1.6 Hz, 1H), 8.44 (s, 1H), 7.74 (dd, *J* = 8.0, 6.6 Hz, 1H), 7.68-7.49 (m, 5H), 7.36 (dd, *J* = 9.8, 1.6 Hz, 1H), 7.24 (dd, *J* = 8.0, 1.6 Hz, 1H), 6.90 (d, *J* = 8.0 Hz, 1H), 6.59-6.48 (m, 3H), 4.44 (d, *J* = 13.6 Hz, 2H), 4.21 (s, 2H), 3.87-3.72 (m, 4H), 3.34 (s, 1H), 3.17 (t, *J* = 12.0 Hz, 2H), 2.99-2.85 (m, 4H), 236-223 (m, 2H), 1.81 (qd, *J* = 12.0, 4.2 Hz, 2H). | 674.3 |
| 204 | 3-(5-((((1-(3-Cyano-4-(4-cyano-3-fluorophenyl)-5-(3-hydroxy-4-(2-hydroxy-2-methylprop oxy) phenyl)pyridin-2-yl)piperidin-4-yl)amin o)methyl) pyridin-2-yl) -N-hydroxy-propanami de formate | 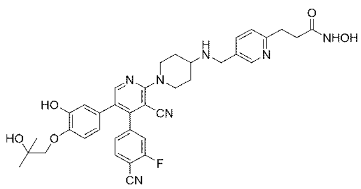 | $^1$H NMR (400 MHz, DMSO-$d_6$) δ ppm : 10.07 (s, 2H), 9.05 (d, J = 6.4 Hz, 1H), 8.69 (d, J = 8.6 Hz, 1H), 8.51 (s, 1H), 8.46 (s, 1H), 8.05 - 7.87 (m, 2H), 7.68 (dd, J = 10.0, 1.5 Hz, 1H), 7.31 (dd, J = 7.9, 1.5 Hz, 1H), 6.77 (d, J = 8.4 Hz, 1H), 6.52 (d, J = 2.2 Hz, 1H), 6.44 (dd, J = 8.2, 2.2 Hz, 1H), 4.56 (s, 1H), 4.40 (d, J = 6.1 Hz, 2H), 4.35 (d, J = 13.3 Hz, 2H), 3.65 (s, 2H), 3.44 (s, 2H), 3.24 (t, J = 7.2 Hz, 2H), 3.15 (t, J = 12.5 Hz, 2H), 2.58 (t, J = 7.2 Hz, 1H), 2.36 - 2.23 (m, 2H), 1.94 - 1.80 (m, 2H), 1.18 (s, 6H). | 680.3 |
| 205 | (E)-3-(6-(((1-(3-Cyano -4-(4-cyano-3-fluorophenyl)-5-(3-hydroxy-4-( 2-hydroxy-2-methylpr opoxy)phenyl)pyridin-2-yl)piperidin-4-yl)ami no) methyl)pyridin-3-yl )-N-hydroxyacrylamide formate | 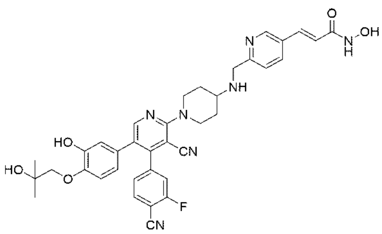 | $^1$H NMR (400 MHz, DMSO-$d_6$) δ 10.83 (s, 1H), 9.09 (s, 1H), 8.77 (s, 1H), 8.44 (s, 1H), 8.13 (s, 1H), 8.04 (d, *J* = 8.2 Hz, 1H), 7.96 (dd, *J* = 8.0, 6.8 Hz, 1H), 7.66 (dd, *J* = 10.1, 1.5 Hz, 1H), 7.60 - 7.46 (m, 2H), 7.29 (dd, *J* = 8.0, 1.4 Hz, 1H), 6.77 (d, *J* = 8.4 Hz, 1H), 6.59 (d, *J* = 5.8 Hz, 1H), 6.50 (d, *J* = 2.2 Hz, 1H), 6.43 (dd, *J* = 8.3, 2.2 Hz, 1H), 4.70 (s, 1H), 4.33 - 4.14 (m, 4H), 3.65 (s, 2H), 3.15 - 3.10 (m, 3H), 2.13 (d, *J* = 12.2 Hz, 2H), 1.64 (d, *J* = 12.0 Hz, 2H), 1.18 (s, 6H). | 678.3 |

(continued)

| Example | Chemical name | Structure | $^1$H NMR | MS(M+H)$^+$ |
|---|---|---|---|---|
| 206 | (E)-3-(5-(((1-(3-Cyano -4-(4-cyano-3-fluorophenyl)-5-(3-hydroxy-4-methoxyphenyl)pyridin-2-yl)-4-methylpiperidin-4-yl)amino)methyl ) pyrimidin-2-yl)-N-hydroxyacrylamide formate | 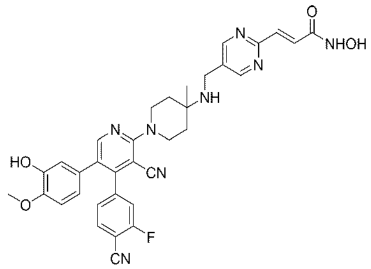 | $^1$H NMR (400 MHz, DMSO-$d_6$) δ ppm 10.83 (s, 1H), 9.0 (s, 1H),8.85 (s, 2H), 8.51 (s, 1H), 8.40 (s, 1H), 7.73 (dd, *J* = 8.0, 6.6 Hz, 1H), 7.56 (d, *J* = 15.4 Hz, 1H), 7.35 (dd, *J* = 9.8, 1.6 Hz, 1H), 7.27 -7.14 (m, 2H), 6.88-6.75 (m, 1H), 6.56-6.47 (m, 2H), 3.95 (s, 4H), 3.81 (s, 3H). 3.73-3.60 (m, 2H), 1.97-1.78 (m, 4H), 1.39 (s, 3H). | 635.2 |
| 207 | (E)-3-(4-(((1-(4-(4-Cyano-3-fluorophenyl)-5-( 3-hydroxy-4-(2-hydrox y-2-methylpropoxy)phenyl)-3-methylpyridin-2-yl)piperidin-4-yl)ami no) methyl)phenyl)-N-hydroxyacrylamide hydrochloride | 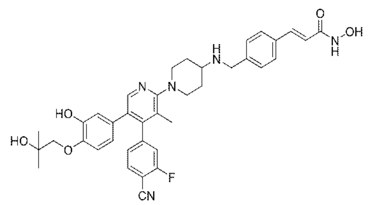 | $^1$H NMR (400 MHz, DMSO-$d_6$) δ10.83 (s, 1H), 9.63 (s, 2H), 8.13 (s, 1H), 7.91 (dd, *J* = 8.0, 6.8 Hz, 1H), 7.68 (d, *J* = 80 Hz, 2H), 7.63 (d, *J* = 8.0 Hz, 2H), 7.54 - 7.39 (m, 2H), 7.14 (dd, *J* = 8.0, 1.6 Hz, 1H), 6.75 (d, *J* = 8.4 Hz, 1H), 6.56 (d, *J* = 15.6 Hz, 1H), 6.49 (d, *J* = 2.4 Hz, 1H), 6.41 (dd, *J* = 8.0, 2.4 Hz, 1H), 4.70 (s, 1H), 4.23 (t, *J* = 6.0 Hz, 2H), 3.64 (s, 4H), 3.28 (s, 1H), 3.02 - 2.86 (m, 2H), 2.26 (t, *J* = 7.2 Hz, 2H), 2.01 (s, 3H), 1.93 (d, *J* = 10.0 Hz, 2H), 1.18 (s, 6H). | 666.3 |
| 208 | (E)-3-(4-(((1-(3',6-Dicyano-5'-(3-hydroxy-4-methoxyphenyl)-[3,4'-bipyridin]-2'-yl)piperid in-4-yl)amino)methyl) phenyl)-N-hydroxyacr ylamide | 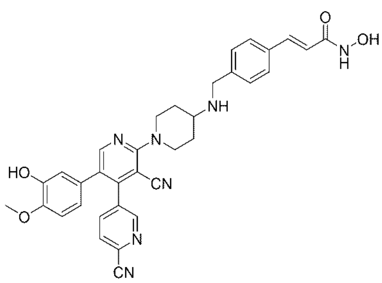 | $^1$H NMR (400 MHz, DMSO-$d_6$) δ 10.72 (s, 1H), 9.01 (s, 1H), 8.65 (dd, *J* = 2.2, 0.9 Hz, 1H), 8.44 (s, 1H), 8.18 - 8.08 (m, 1H), 8.05 (dd, *J* = 8.0, 2.2 Hz, 1H), 7.53 (d, *J* = 7.9 Hz, 2H), 7.44 (t, *J* = 8.2 Hz, 3H), 6.80 (d, *J* = 8.9 Hz, 1H), 6.54 - 6.29 (m, 3H), 4.20 (d, *J* = 13.2 Hz, 2H), 3.86 (s, 2H), 3.71 (s, 3H), 3.18 (t, *J* = 11.9 Hz,3H), 2.81 (s, 1H), 2.02 (d, *J* = 12.4 Hz, 2H), 1.49 (q, *J* = 11.3 Hz, 2H). | 602.2 |

(continued)

| Example | Chemical name | Structure | $^1$H NMR | MS(M+H)$^+$ |
|---|---|---|---|---|
| 209 | 3-(4-(((1-(4-(4-Cyano-3-fluorophenyl)-5-(3-hydroxy-4-methoxyphenyl)-3-methylpyridin-2-yl)piperidin-4-yl)amin o)methyl)phenyl)-N-hydroxypropanamide hydrochloride | 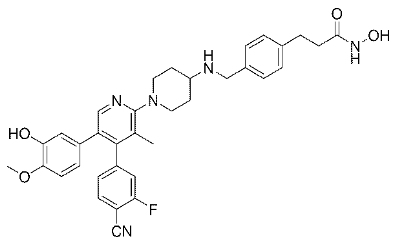 | $^1$H NMR (400 MHz, DMSO-$d_6$) δ 10.45 (s, 1H), 9.61 (s, 2H), 8.51 (s, 1H), 8.12 (s, 1H), 7.91 (t, *J* = 7.6 Hz, 1H), 7.55 (d, *J* = 7.6 Hz, 2H), 7.46 (dd, *J* = 10.0, 1.6 Hz, 1H), 7.27 (d, *J* = 7.6 Hz, 2H), 7.12 (dd, *J* = 8.0, 1.6 Hz, 1H), 6.79 (d, *J* = 8.4 Hz, 1H), 6.51 - 6.40 (m, 2H), 4.16 (t, *J* = 5.6 Hz, 2H), 3.71 (s, 3H), 3.66 (d, *J* = 12.8 Hz, 2H), 3.35-3.32 (m, 1H), 3.06 - 2.90 (m, 2H), 2.84 (t, *J* = 7.6 Hz, 2H), 228 (q, *J* = 7.2, 6.8 Hz, 4H), 2.01 (s, 3H), 1.98 - 1.87 (m, 2H). | 610.3 |
| 210 | (E)-3-(4-(1-((1-(3-Cyano-4-(4-cyano-3-fluorophenyl)-5-(3-hydroxy-4-methoxyphenyl)pyridin-2-yl)piperidin-4-yl )amino)ethyl)phenyl)-N-hydroxyacrylamide formate | 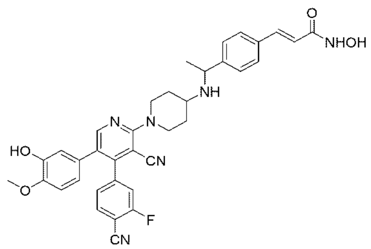 | $^1$H NMR (400 MHz, Methanol-$d_4$) δ 8.57 (s, 1H), 8.48 (s, 1H), 7.91 (dd, *J* = 8.0, 6.6 Hz, 1H), 7.82 (d, *J* = 7.9 Hz, 1H), 7.77 (d, *J* = 15.8 Hz, 2H), 7.68 (d, *J* = 8.0 Hz, 2H), 7.52 (dd, *J* = 9.8, 1.5 Hz, 1H), 7.39 (dd, *J* = 8.0, 1.5 Hz, 1H), 7.02 - 6.95 (m, 1H), 6.72 - 6.67 (m, 2H), 6.66 (t, *J* = 2.1 Hz, 1H), 4.57 (s, 1H), 4.51 (d, *J* = 12.5 Hz, 2H), 3.81 (s, 3H), 3.24 (d, *J* = 12.8 Hz, 1H), 3.19 - 3.09 (m, 2H), 2.31 (dd, *J* = 74.1, 12.5 Hz, 2H), 1.93 - 1.85 (m, 2H), 1.74 (d, *J* = 6.7 Hz, 3H). | 633.3 |
| 211 | (E)-3-(4-(((1-(3-Cyano-4-(4-cyano-3-fluorophenyl)-5-(3-hydroxy-4-methoxyphenyl)pyridin-2-yl)piperidin-4-yl)amino)methyl)-3-methylphenyl)-N-hydroxyacrylamide formate | 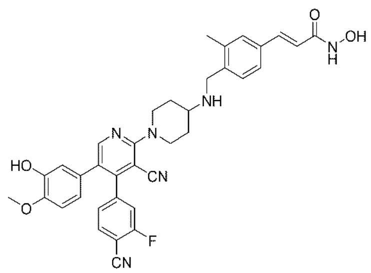 | $^1$H NMR (400 MHz, Methanol-$d_4$) δ 8.41 (s, 1H), 8.32 (s, 1H), 7.64 (dd, *J* = 8.0, 6.6 Hz, 1H), 7.44 (d, *J* = 15.8 Hz, 1H), 7.34 (d, *J* = 3.8 Hz, 3H), 7.26 (dd, *J* = 9.8, 1.5 Hz, 1H), 7.13 (dd, *J* = 8.0, 1.5 Hz, 1H), 6.76 - 6.67 (m, 1H), 6.46 - 6.33 (m, 3H), 4.33 (d, *J* = 13.3 Hz, 2H), 4.03 (s, 2H), 3.71 (s, 3H), 3.08 (q, *J* = 12.3, 11.9 Hz, 2H), 2.55 (s, 1H), 2.35 (s, 3H), 2.23 - 2.10 (m, 2H), 1.76 - 1.59 (m, 2H). | 633.3 |

(continued)

| Example | Chemical name | Structure | $^1H$ NMR | MS(M+H)$^+$ |
|---|---|---|---|---|
| 212 | (E)-3-(4-(((1-(3-Cyano -4-(4-cyano-3-fluoroph enyl)-5-(3-hydroxy-4-( 2-hydroxy-2-methylpr opoxy)phenyl)pyridin-2-yl)piperidin-4-yl)ami no)methyl)phenyl)-N-h ydroxybut-2-enamide hy-drochloride | 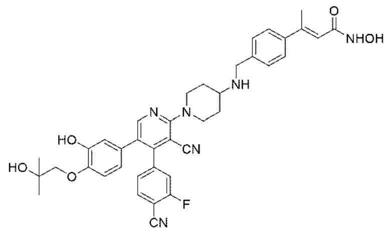 | $^1H$ NMR (400 MHz, DMSO-$d_6$) δ 10.63 (s, 1H), 9.18 (s, 1H), 8.71 (s, 1H), 8.46 (s, 1H), 7.97 (dd, *J* = 8.0, 6.8 Hz, 1H), 7.68 (dd, *J* = 10.0, 1.4 Hz, 1H), 7.63 - 7.51 (m, 4H), 7.30 (dd, *J* = 8.0, 1.4 Hz, 1H), 6.78 (d, *J* = 8.4 Hz, 1H), 6.51 (d, *J* = 2.2 Hz, 1H), 6.44 (dd, *J* = 8.2, 2.2 Hz, 1H), 6.07 (d, *J* = 1.6 Hz, 1H), 4.32 (d, *J* = 13.0 Hz, 2H), 4.24 (s, 2H), 3.65 (s, 2H), 3.26 (s, 1H), 3.13 (t, *J* = 12.6 Hz, 2H), 2.52 (s, 3H), 2.27 (d, *J* = 11.4 Hz, 2H), 1.88 - 1.71 (m, 2H), 1.18 (s, 6H). | 691.3 |
| 213 | (E)-3-(5-(((1-(3-Cyano -4-(4-cyano-3-fluoroph enyl)-5-(3-hydroxy-4-methoxyphenyl)pyridi n-2-yl)-4-methylpiperi din-4-yl)amino)methyl ) pyridin-2-yl)-N-hydro xyacrylamide formate | 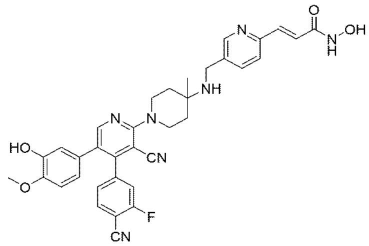 | $^1H$ NMR (400 MHz, Methanol-$d_4$) δ 8.64 (s, 1H), 8.41 (s, 1H), 8.20 (s, 1H), 7.97 - 7.88 (m, 1H), 7.73 (dd, *J* = 8.0, 6.6 Hz, 1H), 7.60 (d, *J* = 6.6 Hz, 1H), 7.57 (s, 1H), 7.36 (dd, *J* = 9.8, 1.5 Hz, 1H), 7.23 (dd, *J* = 8.0, 1.5 Hz, 1H), 6.88 (d, *J* = 5.4 Hz, 1H), 6.81 (d, *J* = 8.8 Hz, 1H), 6.55 - 6.45 (m, 2H), 4.11 - 3.95 (m, 4H), 3.81 (s, 3H), 3.66 - 3.53 (m, 2H), 1.94 (t, *J* = 6.6 Hz, 2H), 1.88 (s, 2H), 1.43 (s, 3H). | 634.2 |
| 214 | (E)-3-(4-(((1-(3-Cyano -4-(4-cyano-3-fluoroph enyl)-5-(3-hydroxy-4-( trifluoromethoxy)phen yl) pyridin-2-yl)-4-met hylpiperidin-4-yl)amin o) methyl)phenyl)-N-hy droxyacrylamide formate | 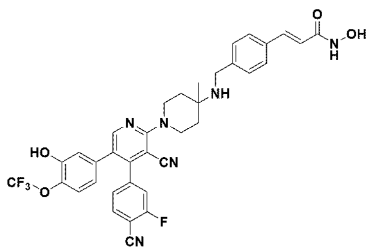 | $^1H$ NMR (400 MHz, DMSO-$d_6$) δ 10.80 (s, 1H), 10.25 (s, 1H), 9.07 (d, *J* = 2.4 Hz, 1H), 8.96 (s, 2H), 8.53 (s, 1H), 7.99 (dd, *J* = 8.0, 6.8 Hz, 1H), 7.76-7.55 (m, 3H), 7.45 (dd, *J* = 8.0, 1.6 Hz, 1H), 7.30 (dd, *J* = 8.0, 1.6 Hz, 1H), 7.18 (dd, *J* = 8.4, 1.4 Hz, 1H), 6.67 (d, *J* = 2.4 Hz, 1H), 6.64 - 6.43 (m, 2H), 4.37 - 4.14 (m, 4H), 3.35 (s, 2H), 2.04 (d, *J* = 14.4 Hz, 4H), 1.59 (s, 3H). | 687.2 |
| 215 | (E)-3-(4-(((1-(3-Cyano -4-(4-cyano-3-fluoroph enyl)-5-(3-hydroxy-4-( 2-hydroxy-2-methylpr opoxy)phenyl)pyridin-2-yl)-4-methylpiperidi n-4-yl)amino)methyl)p henyl)-N-hydroxyacryl amide formate | 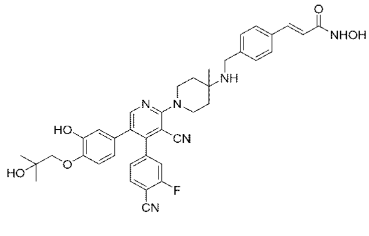 | $^1H$ NMR (400 MHz, Methanol-$d_4$) δ ppm 8.55 (s, 1H), 8.44 (s, 1H), 7.75 (dd, *J* = 8.0, 6.6 Hz, 1H), 7.66-7.49 (m, 5H), 7.37 (dd, *J* = 9.6, 1.6 Hz, 1H), 7.24 (dd, *J* = 8.0, 1.6 Hz, 1H), 6.80 (d, *J* = 8.2 Hz, 1H), 6.57-6.45 (m, 3H), 4.23 (d, *J* = 13.8 Hz, 2H), 4.13 (s, 2H), 3.78 (s, 2H), 3.46 (t, *J* = 12.4 Hz, 2H), 2.11-1.95 (m, 4H), 1.56 (s, 3H), 1.30 (s, 6H). | 691.3 |

(continued)

| Example | Chemical name | Structure | $^1$H NMR | MS(M+H)$^+$ |
|---|---|---|---|---|
| 216 | 2-((4-((1-(3-cyano-4-(4-cyano-3-fluorophenyl)-5-(3-hydroxy-4-methoxyphenyl)pyridin-2-yl) piperidin-4-yl)amino) methyl)phenyl)thio)-N-hydroxyacetamide formate | 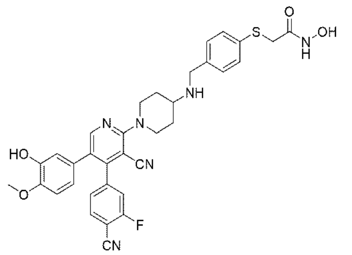 | $^1$H NMR (400 MHz, Methanol-$d_4$) δ 8.51 (s, 1H), 8.33 (s, 1H), 7.64 (dd, $J$ = 8.0, 6.6 Hz, 1H), 7.39 (d, $J$ = 8.0 Hz, 2H), 7.33 (d, $J$ = 8.0 Hz, 2H), 7.27 (dd, $J$ = 9.8, 1.5 Hz, 1H), 7.13 (dd, $J$ = 8.0, 1.5 Hz, 1H), 6.77 - 6.65 (m, 1H), 6.46 - 6.35 (m, 2H), 4.32 (d, $J$ = 13.3 Hz, 2H), 4.03 (s, 2H), 3.71 (s, 3H), 3.46 (s, 2H), 3.15 (d, $J$ = 10.9 Hz, 1H), 3.12 - 3.00 (m, 2H), 2.15 (d, $J$ = 12.3 Hz, 2H), 1.78 - 1.57 (m, 2H). | 639.2 |
| 217 | (E)-3-(5-(((1-(4-(4-cyano-3-fluorophenyl)-3-(cyanomethyl)-5-(3-hydroxy-4-methoxyphenyl ) pyridin-2-yl)piperidin -4-yl)amino)methyl)py ridin-2-yl)-N-hydroxya crylamide formate | 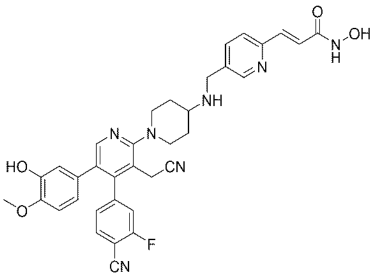 | $^1$H NMR (400 MHz, DMSO-$d_6$) δ ppm : 10.88 (s, 1H), 8.95 (s, 1H), 8.61 (d, J = 2.1 Hz, 1H), 8.31 (s, 1H), 8.15 (s, 1H), 7.94 (dd, J = 8.0, 6.9 Hz, 1H), 7.84 (dd, J = 8.0, 2.2 Hz, 1H), 7.59 - 7.43 (m, 3H), 7.18 (dd, J = 8.0, 1.5 Hz, 1H), 6.92 (d, J = 15.4 Hz, 1H), 6.83 - 6.74 (m, 1H), 6.53 - 6.43 (m, 2H), 3.90 (s, 2H), 3.76 (d, J = 1.6 Hz, 2H), 3.71 (s, 3H), 3.36 (d, J = 12.5 Hz, 2H), 2.89 (q, J = 11.4 Hz, 2H), 2.71 (d, J = 11.4 Hz, 1H), 2.01 (t, J = 7.4 Hz, 2H), 1.60 (s, 2H). | 634.3 |
| 218 | (E)-3-(4-(((1-(4-(4-Cyano-3-fluorophenyl)-3-(cyanomethyl)-5-(3-hydroxy-4-(2-hydroxy-2-methylpropoxy)phenyl ) pyridin-2-yl)piperidin -4-yl)amino)methyl)ph enyl)-N-hydroxyacryla mide formate | 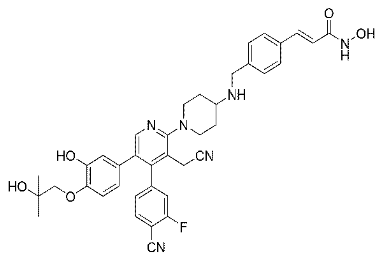 | $^1$H NMR (400 MHz, DMSO-$d_6$) δ ppm : 10.75 (s, 1H), 9.04 (s, 1H), 8.64 (s, 1H), 8.32 (s, 1H), 8.15 (s, 1H), 7.95 (dd, J = 8.0, 6.9 Hz, 1H), 7.57 (d, J = 7.9 Hz, 2H), 7.54 - 7.41 (m, 4H), 7.19 (dd, J = 8.0, 1.4 Hz, 1H), 6.75 (d, J = 8.4 Hz, 1H), 6.52 - 6.47 (m, 1H), 6.47 - 6.41 (m, 2H), 4.72 (s, 1H), 3.99 (s, 2H), 3.79 - 3.72 (m, 2H), 3.64 (s, 2H), 3.40 (d, J = 12.5 Hz, 2H), 2.91 (q, J = 11.2 Hz, 3H), 2.08 (d, J = 12.1 Hz, 2H), 1.68 (s, 2H), 1.18 (s, 6H). | 691.3 |

(continued)

| Example | Chemical name | Structure | $^1$H NMR | MS(M+H)$^+$ |
|---|---|---|---|---|
| 219 | 3-(4-((1-(3-Cyano-4-(4-cyano-3-fluorophenyl)-5-(3-hydroxy-4-methoxyphenyl)pyridin-2-yl) piperidin-4-yl)amino) methyl)phenyl)-2-fluor o-N-hydroxypropanam ide formate | 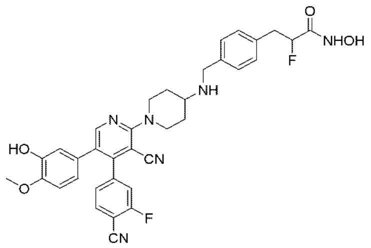 | $^1$H NMR (400 MHz, DMSO-$d_6$) δ ppm : 10.93 (s, 1H), 9.05 (s, 1H), 9.01 (s, 1H), 8.46 (s, 1H), 7.97 (t, J = 7.4 Hz, 1H), 7.67 (dd, J = 10.1, 1.4 Hz, 1H), 7.46 (d, J = 7.6 Hz, 2H), 7.36 - 7.25 (m, 3H), 6.81 (d, J = 8.1 Hz, 1H), 6.47 (d, J = 8.0 Hz, 2H), 5.07 (ddd, J = 48.3, 8.0, 4.4 Hz, 1H), 4.29 (d, J = 13.0 Hz, 2H), 4.15 (s, 2H), 3.72 (s, 3H), 3.20 - 3.03 (m, 4H), 2.79 (s, 1H), 2.21 (s, 2H), 1.71 (s, 2H). | 639.3 |
| 220 | (E)-3-(5-(((1-(3-Cyano-4-(4-cyano-3-fluorophenyl)-5-(3-hydroxy-4-methoxyphenyl)pyridin-2-yl)piperidin-4-yl)amino)methyl)pyridin-2-yl)-N-hydroxy-2-methylacrylamide formate | 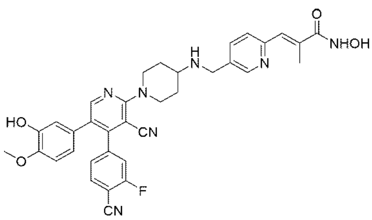 | $^1$H NMR (400 MHz, Methanol-$d_4$) δ ppm 8.71 (d, *J* = 2.2 Hz, 1H), 8.41 (s, 1H)7.94 (d, *J* = 8.2 Hz, 1H), 7.62 - 7.49 (m, 2H), 7.38 - 7.29 (m, 2H), 7.25 (dd, *J* = 8.0, 1.6 Hz, 1H), 7.16 (d, *J* = 1.6 Hz, 1H), 6.93 (d, *J* = 8.8 Hz, 1H), 6.63 (h, *J* = 2.2 Hz, 2H), 4.60 (d, *J* = 13.6 Hz, 2H), 4.20 (s, 2H), 3.88 (s, 3H), 3.28 (s, 1H), 3.07 (t, *J* = 12.7 Hz, 2H), 2.27 (d, *J* = 1.5 Hz, 3H), 2.23 (d, *J* = 12.1 Hz, 2H), 1.61 (q, *J* = 11.9 Hz, 2H). | 634.3 |
| 221 | (E)-3-5-(((1-(4-(4-Cyano-3-fluorophenyl)-3-( cyanomethyl)-5-(3-hydroxy-4-(2-hydroxy-2-methylpropoxy)phenyl ) pyridin-2-yl)piperidin -4-yl)amino)methyl)py ridin-2-yl)-N-hydroxya crylamide formate | 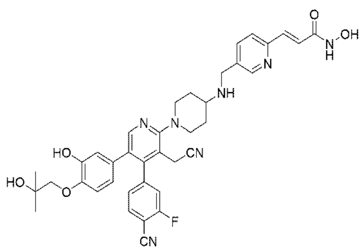 | $^1$H NMR (400 MHz, DMSO-$d_6$) δ ppm : δ 10.91 (s, 1H), 9.11 (s, 1H), 8.71 (s, 1H), 8.63 (s, 1H), 8.33 (s, 1H), 7.95 (t, J = 7.4 Hz, 2H), 7.64 (s, 1H), 7.50 (d, J = 10.3 Hz, 2H), 7.19 (dd, J = 8.0, 1.4 Hz, 1H), 6.96 (d, J = 15.4 Hz, 1H), 6.75 (d, J = 8.4 Hz, 1H), 6.51 (d, J = 2.2 Hz, 1H), 6.44 (dd, J = 8.2, 2.2 Hz, 1H), 4.69 (s, 1H), 4.22 (s, 2H), 3.75 (s, 2H), 3.65 (s, 2H), 3.44 (d, J = 12.4 Hz, 2H), 2.95 (d, J = 12.6 Hz, 2H), 2.71 (d, J = 11.4 Hz, 1H), 2.18 (s, 2H), 1.82 (s, 2H), 1.18 (s, 6H). | 692.3 |

(continued)

| Example | Chemical name | Structure | $^1$H NMR | MS(M+H)$^+$ |
|---|---|---|---|---|
| 222 | 3-(3-chloro-4-((1-(3-cyano-4-(4-cyano-3-fluorophenyl)-5-(3-hydroxy-4-methoxyphenyl)pyridin-2-yl)piperidin-4-yl )amino)methyl)phenyl)-N-hydroxypropanamide formate | 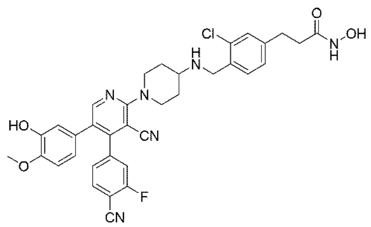 | $^1$H NMR (400 MHz, DMSO-) δ 10.35 (s, 1H), 8.98 (s, 1H), 8.69 (s, 1H), 8.43 (d, *J* = 3.4 Hz, 1H), 7.66 (d, *J* = 10.4 Hz, 1H), 7.44 (d, *J* = 15.9 Hz, 2H), 7.34 (s, 1H), 7.28 (dd, *J*= 8.0, 1.5 Hz, 1H), 7.23 (d, *J* = 8.0 Hz, 1H), 6.81 (d, *J* = 8.1 Hz, 1H), 6.57 - 6.47 (m, 1H), 6.46 (d, *J* = 1.6 Hz, 2H), 4.25 (d, *J* = 13.2 Hz, 2H), 4.06 (s, 2H), 3.71 (s, 3H), 3.17 (t, *J* = 12.8 Hz, 2H), 2.83 (t, *J* = 7.4 Hz, 2H), 2.54 (s, 1H), 2.28 (t, *J* = 7.5 Hz, 2H), 2.13 (s, 2H), 1.61 (s, 2H). | 655.2 |
| 223 | (E)-3-(4-(2-((1-(3-Cyano-4-(4-cyano-3-fluorophenyl)-5-(3-hydroxy-4-(2-hydroxy-2-methylpropoxy)phenyl)pyridin-2-yl)piperidin-4-yl)amino)propan-2-yl)phenyl)-N-hydroxyacrylamide hydrochloride | 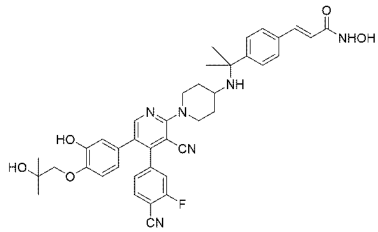 | $^1$H NMR (400 MHz, DMSO-$d_6$) δ 10.81 (s, 1H), 9.32 (s, 2H), 8.72 (s, 1H), 8.41 (s, 1H), 7.95 (t, *J* = 7.6 Hz, 1H), 7.77 (d, *J* = 8.0 Hz, 2H), 7.72 - 7.61 (m, 3H), 7.49 (d, *J* = 15.6 Hz, 1H), 7.26 (dd, *J* = 8.0, 1.5 Hz, 1H), 6.76 (d, *J* = 8.4 Hz, 1H), 6.62 - 6.46 (m, 2H), 6.41 (dd, *J* = 8.0, 2.4 Hz, 1H), 4.12 (d, *J* = 13.2 Hz, 2H), 3.64 (s, 2H), 3.29 (s, 1H), 3.05 - 2.91 (m, 2H), 1.81 (s, 6H), 1.72 (d, *J* = 7.6 Hz, 4H), 1.17 (s, 6H). | 705.3 |
| 224 | (E)-3-(6-(((1-(3-Cyano-4-(4-cyano-3-fluorophenyl)-5-(1-(2-hydroxy-2-methylpropyl)-1H-benzo[d][1,2,3]triazol-5-yl)pyridin-2-yl)piperidin-4-yl)amino)methyl)pyridin-3-yl)-N-hydroxy acrylamide formate | 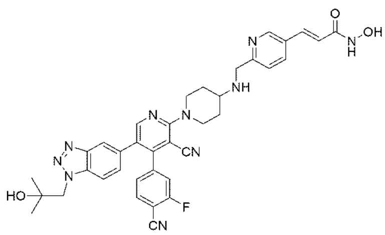 | $^1$H NMR (400 MHz, DMSO-$d_6$) δ 10.78 (s, 1H), 8.68 (d, *J* = 2.2 Hz, 1H), 8.57 (s, 1H), 8.18 (s, 1H), 7.96 (dd, *J* = 8.0, 2.2 Hz, 1H), 7.90 (t, *J* = 7.4 Hz, 1H), 7.82 (s, 1H), 7.75 - 7.66 (m, 2H), 7.59 - 7.41 (m, 2H), 7.34 (dd, *J* = 8.0, 1.4 Hz, 1H), 7.14 (dd, *J* = 8.6, 1.4 Hz, 1H), 6.54 (d, *J* = 15.9 Hz, 1H), 4.75 (s, 1H), 4.55 (s, 2H), 4.24 (d, *J* = 13.0 Hz, 2H), 3.93 (s, 2H), 3.22 (d, *J* = 11.6 Hz, 2H), 2.79 (s, 1H), 2.08 - 1.94 (m, 2H), 1.48 (q, *J* = 11.4 Hz, 2H), 1.12 (s, 6H). | 687.3 |

(continued)

| Example | Chemical name | Structure | $^1$H NMR | MS(M+H)$^+$ |
|---|---|---|---|---|
| 225 | (E)-3-(5-(((1-(3-Cyano -4-(4-cyano-3-fluoroph enyl)-5-(1-(2-hydroxy-2-methylpropyl)-1H-be nzo [d][1,2,3]triazol-5-yl)pyridin-2-yl)piperidi n-4-yl) amino)methyl)p yri-din-2-)-N-hydroxya cryla-mide formate | 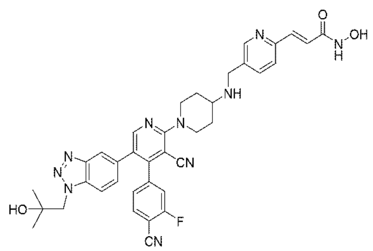 | $^1$H NMR (400 MHz, DMSO-$d_6$) δ 10.88 (s, 1H), 8.63 (s, 1H), 8.58 (s, 1H), 8.14 (s, 1H), 7.95 - 7.80 (m, 3H), 7.72 (dd, *J* = 9.4, 6.8 Hz, 2H), 7.57 (d, *J* = 7.8 Hz, 1H), 7.48 (d, *J* = 5.4 Hz, 1H), 7.34 (d, *J* = 8.0 Hz, 1H), 7.13 (d, *J* = 8.6 Hz, 1H), 6.92 *(d, J* = 5.4 Hz, 1H), 4.76 (s, 1H), 4.55 (s, 2H), 4.27 (d, *J* = 13.0 Hz, 2H), 3.95 (s, 2H), 3.22 (t, *J* = 12.2 Hz, 2H), 2.90 (s, 1H), 2.07 (d, *J* = 12.4 Hz, 2H), 1.54 (d, *J* = 11.6 Hz, 2H), 1.12 (s, 6H). | 687.3 |
| 226 | (E)-3-(4-(((1-(3-Cyano -4-(4-cyano-3-fluoroph enyl)-5-(6,7-difluoro-1 -(2-hydroxy-2-methylp ropyl)-1H-benzo[d][1, 2,3]triazol-5-yl)pyridin -2-yl)piperidin-4-yl)am ino) methyl)-N-hydrox yacry-lamide formate | 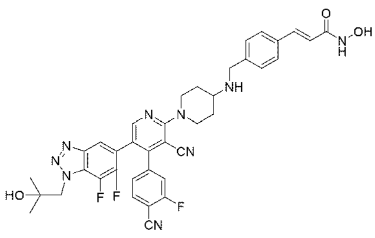 | $^1$H NMR (400 MHz, Metha-nol-$d_4$) δ ppm 8.51 (s, 1H), 8.49 (d, *J* = 3.4 Hz, 1H), 7.78 (d, *J* = 5.2 Hz, 1H), 7.74 - 7.66 (m, 1H), 7.67 - 7.41 (m, 6H), 7.28 (d, *J* = 8.1 Hz, 1H), 6.51 (d, *J* = 15.8 Hz, 1H), 4.71 (d, *J* = 21.2 Hz, 2H), 4.57 (d, *J* = 13.7 Hz, 2H), 4.15 (s, 2H), 3.23 (t, *J* = 12.4 Hz, 3H), 2.26 (d, *J* = 12.4 Hz, 2H), 1.84 - 1.68 (m, 2H), 1.25 (d, *J* = 4.9 Hz, 6H). | 722.3 |
| 227 | (E)-3-(4-Chloro-5-(((1-(3-cyano-4-(4-cyano-3 -fluorophenyl)-5-(3-hy droxy-4-methoxyphen yl) pyridin-2-yl)piperidi n-4-yl)amino)methyl)p yridin-2-yl)-N-hydroxy acry-lamide formate | 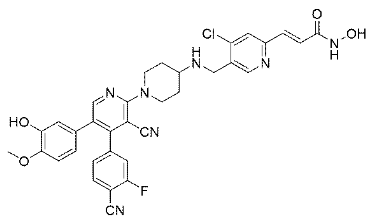 | $^1$H NMR (400 MHz, DMSO-$d_6$) δ 10.92 (s, 1H), 9.14 (s, 1H), 8.98 (s, 1H), 8.42 (s, 1H), 8.13 (s, 1H), 7.95 (dd, *J* = 8.0, 6.8 Hz, 1H), 7.75 (s, 1H), 7.66 (dd, *J* = 10.0, 1.4 Hz, 1H), 7.48 (d, *J* = 5.4 Hz, 1H), 7.28 (dd, *J* = 8.0, 1.6 Hz, 1H), 6.97 (d, *J* = 5.0 Hz, 1H), 6.80 (d, *J* = 8.0 Hz, 1H), 6.50 - 6.43 (m, 2H), 4.18 (d, *J* = 13.0 Hz, 2H), 3.92 (s, 2H), 3.71 (s, 3H), 3.20 (t, *J* = 12.0 Hz, 2H), 2.78 (s, 1H), 2.02 (d, *J* = 12.4 Hz, 2H), 1.47 (d, *J* = 11.4 Hz, 2H). | 654.2 |

(continued)

| Example | Chemical name | Structure | $^1$H NMR | MS(M+H)$^+$ |
|---|---|---|---|---|
| 228 | (E)-3-(4-((((1-(3-Cyano-4-(4-cyano-3-fluorophenyl)-5-(3-hydroxy-4-methoxyphenyl)pyridin-2-yl)pyrrolidin-3-yl)methyl)amino)methyl)phenyl)-N-hydroxyacrylamide hydrochloride | 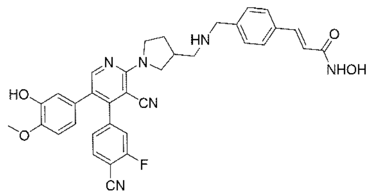 | $^1$H NMR (400 MHz, DMSO-$d_6$) δ 10.83 (s, 1H), 9.60 (d, $J$ = 5.6 Hz, 1H), 9.47 (s, 1H), 8.35 (s, 1H), 8.25 (s, 1H), 7.96 (t, $J$ = 7.6 Hz, 1H), 7.72 - 7.55 (m, 4H), 7.47 (d, $J$ = 15.6 Hz, 1H), 7.25 (d, $J$ = 7.6 Hz, 1H), 6.79 (d, $J$ = 8.0 Hz, 1H), 6.55 (d, $J$ = 15.6 Hz, 1H), 6.49 - 6.35 (m, 2H), 4.19 (t, $J$ = 5.6 Hz, 2H), 3.95 (dd, $J$ = 11.2, 6.8 Hz, 1H), 3.85 (dd, $J$ = 11.6, 7.6 Hz, 1H), 3.76 (dt, $J$ = 11.2, 7.6 Hz, 1H), 3.71 (s, 3H), 3.55 (dd, $J$ = 11.2, 7.2 Hz, 1H), 3.07 (q, $J$ = 6.4 Hz, 2H), 2.79 (d, $J$ = 7.2 Hz, 1H), 2.23 (dq, $J$ = 12.0, 6.4 Hz, 1H), 1.87 (dq, $J$ = 12.2, 7.6 Hz, 1H). | 619.2 |
| 229 | (E)-3-(4-(1-(3-cyano-4-(4-cyano-3-fluorophenyl)-5-(3-hydroxy-4-(( 1-hydroxycyclopropyl)methoxy)phenyl)pyridin-2-yl)-4-methylpiperidin-4-yl)amino)methyl )phenyl)-N-hydroxyacrylamide formate | 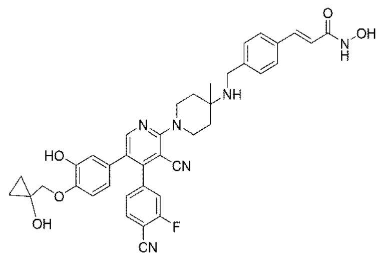 | $^1$H NMR (400 MHz, Methanol-$d_4$) δ 8.51 (s, 1H), 8.44 (s, 1H), 7.74 (t, $J$ = 7.3 Hz, 1H), 7.68 - 7.48 (m, 5H), 7.36 (d, $J$ = 9.6 Hz, 1H), 7.28 - 7.19 (m, 1H), 6.78 (d, $J$ = 8.3 Hz, 1H), 6.59 - 6.43 (m, 3H), 4.27 (d, $J$ = 13.7 Hz, 2H), 4.17 (s, 2H), 3.96 (s, 2H), 3.43 (t, $J$ = 11.3 Hz, 2H), 2.14 - 1.95 (m, 4H), 1.59 (s, 3H), 0.84 - 0.78 (m, 2H), 0.71 - 0.65 (m, 2H). | 689.3 |
| 230 | (E)-3-(6-(((1-(3-Cyano-4-(4-cyano-3-fluorophenyl)-5-(3-hydroxy-4-methoxyphenyl)pyridin-2-yl)piperidin-4-yl)amino)methyl)-5-fluoropyridin-3-yl)-N-hydroxyacrylamide formate | 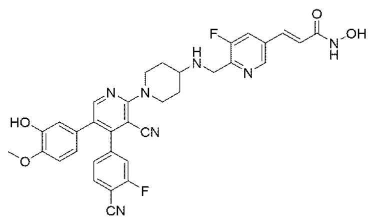 | $^1$H NMR (400 MHz, DMSO-$d_6$) δ 10.86 (s, 1H), 8.99 (s, 1H), 8.64 (s, 1H), 8.42 (s, 1H), 8.14 (s, 1H), 8.02 - 7.89 (m, 2H), 7.66 (d, $J$ = 10.0 Hz, 1H), 7.52 (d, $J$ = 5.0 Hz, 1H), 7.28 (d, $J$ = 7.8 Hz, 1H), 6.81 (d, $J$ = 8.0 Hz, 1H), 6.63 (d, $J$ = 10.0 Hz, 1H), 6.47 (d, $J$ = 8.2 Hz, 2H), 4.20 (d, $J$ = 13.2 Hz, 2H), 4.10 (s, 2H), 3.71 (s, 3H), 3.17 (t, $J$ = 12.0 Hz, 2H), 2.98 (s, 1H), 2.06 (d, $J$ = 12.2 Hz, 2H), 1.55 *(d, J*= 11.0 Hz, 2H). | 638.2 |

**Example 231**

(E)-3-(4-(2-(4-(6-(4-Aminopiperidin-1-yl)-5-cyano-4-(4-cyano-3-fluorophenyl)pyridin-3-yl)-2-hydroxyphenox y)ethyl)phenyl)-N-hydroxyacrylamide hydrochloride was prepared according to the synthetic method of Example **24**, and the structure and characterization data are as follows:

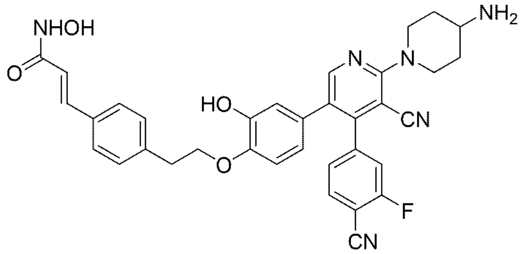

$^1$H NMR (400 MHz, DMSO-$d_6$) δ 10.74 (s, 1H), 9.01 (s, 1H), 8.44 (s, 1H), 8.16 (s, 3H), 7.96 (dd, *J* = 8.0, 6.8 Hz, 1H), 7.67 (dd, *J* = 10.0, 1.4 Hz, 1H), 7.52 - 7.39 (m, 3H), 7.36 (d, *J* = 8.0 Hz, 3H), 7.26 (dd, *J* = 8.0, 1.4 Hz, 1H), 6.82 (d, *J* = 8.2 Hz, 1H), 6.51 - 6.38 (m, 2H), 4.26 (d, *J* = 13.4 Hz, 2H), 4.12 (t, *J* = 6.8 Hz, 2H), 3.41 - 3.28 (m, 1H), 3.16 (t, *J* = 12.2 Hz, 2H), 3.02 (t, *J* = 6.8 Hz, 2H), 2.06 (d, *J* = 11.2 Hz, 2H), 1.75 - 1.62 (m, 2H).

ESI-MS(m/z) =619.2 [M+H]$^+$.

### Example 232

Preparation of 2-(4-(1H-pyrazol-1-yl)phenyl)-6-((((1R,2S)-2-(4-fluorophenyl)cyclopropyl)amino) methyl)-N-(4-((E)-3-(hydroxyam ino)-3-oxoprop-1-en-1-yl)benzyl)pyrimidine-4-carboxamide formate

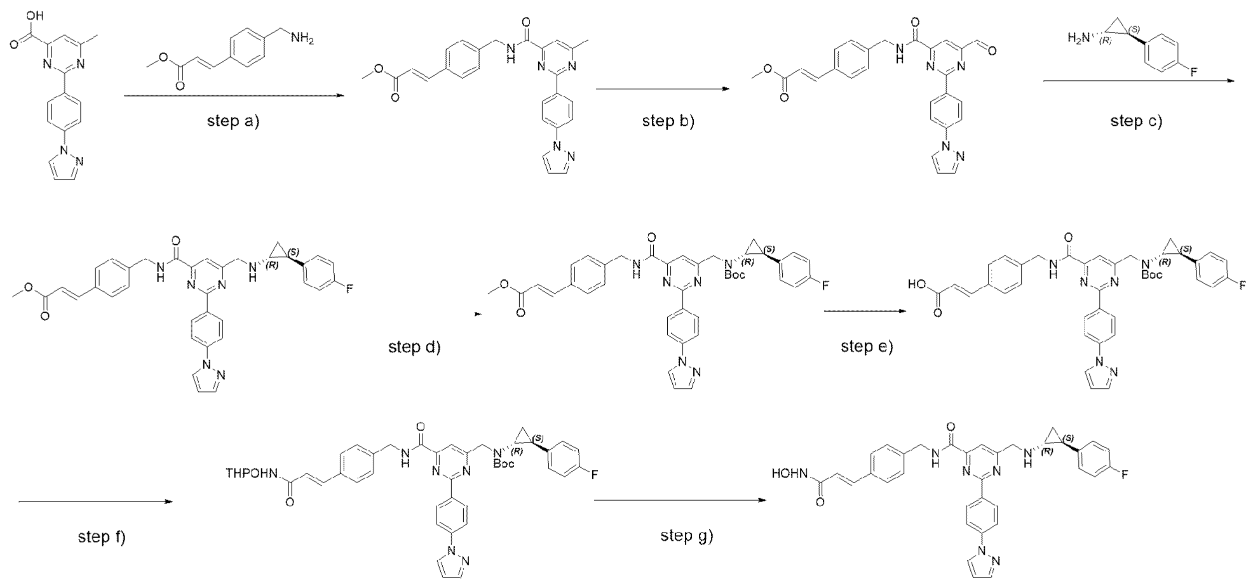


Step a): preparation of methyl (E)-3-(4-((2-(4-(1H-pyrazol-1-yl)phenyl)-6-methylpyrimidine-4-carboxamido)methyl) phenyl)acrylate

2-(4-(1H-Pyrazol-1-yl)phenyl)-6-methylpyrimidine-4-carboxylic acid (500 mg, 1.8 mmol), methyl (E)-3-(4-(aminomethyl)phenyl)acrylate (344 mg, 1.8 mmol), HATU (821 mg, 2.2 mmol), and DIPEA (464 mg, 3.6 mmol) were added to a reaction flask containing DMF (5 mL), and the mixture was stirred at room temperature for 2 hours. The reaction liquid was quenched by adding water (10 mL), and then extracted with ethyl acetate (20 mL × 2). The organic phases were combined, washed with saturated brine (15 mL × 2), dried over anhydrous sodium sulfate, and concentrated under vacuum. The residue was purified by silica gel chromatography (eluent: DCM/MeOH = 10/1) to afford methyl (E)-3-(4-((2-(4-(1H-pyrazol-1-yl)phenyl)-6-methylpyrimidine-4-carboxamido)methyl)phenyl)acrylate with a yield of 74%

ESI-MS m/z=454.2 [M+H]$^+$.

Step b): preparation of methyl (E)-3-(4-((2-(4-(1H-pyrazol-1-yl)phenyl)-6-formylpyrimidine-4-carboxamido)methyl)phenyl)acrylate

Methyl (E)-3-(4-((2-(4-(1H-Pyrazol-1-yl)phenyl)-6-methylpyrimidine-4-carboxamido)methyl)phenyl)acrylate

(605 mg, 1.3 mmol), selenium dioxide (432 mg, 3.9 mmol), and 1,4-dioxane (12 mL) were added to a reaction flask, and the mixture was stirred at 100°C for 16 hours. After concentrating to dryness under reduced pressure, the residue was purified by silica gel chromatography (eluent: petroleum ether/ethyl acetate = 1/2) to afford methyl (E)-3-(4-((2-(4-(1H-pyrazol-1-yl)phenyl)-6-formylpyrimidine-4-carboxamido)methyl)phenyl)acrylate with a yield of 52.5%

ESI-MS m/z=468.2 $[M+H]^+$.

Step c): preparation of methyl (E)-3-(4-((2-(4-(1H-pyrazol-1-yl)phenyl)-6-((((1R,2S)-2-(4-fluorophenyl)cyclopropyl)amino)methyl)pyrimidine-4-c arboxamido)methyl)phenyl)acrylate

Methyl (E)-3-(4-((2-(4-(1H-pyrazol-1-yl)phenyl)-6-formylpyrimidine-4-carboxamido)methyl)phenyl)acrylate (319 mg, 683 μmol) and (1R,2S)-2-(4-fluorophenyl)cyclopropane-1-amine (127 mg, 683 μmol) were added to a reaction flask containing DCE (10 mL) and acetic acid (100 μL), and the mixture was stirred at room temperature for 2 hours. The reaction was monitored by LC-MS until the starting materials were completely consumed, then sodium cyanoborohydride (0.32 g, 2.3 mmol) was added at 0°C, and the mixture was stirred at room temperature for 2 hours. The reaction liquid was quenched by adding ice-water mixture of saturated solution of sodium bicarbonate, and then extracted with ethyl acetate (20 mL × 2). The organic phases were combined, washed with saturated brine (15 mL × 2), dried over anhydrous sodium sulfate, and filtered. The residue was purified by silica gel chromatography (eluent: petroleum ether/ethyl acetate = 1/1) to afford methyl (E)-3-(4-((2-(4-(1H-pyrazol-1-yl)phenyl)-6-((((1R,2S)-2-(4-fluorophenyl)cyclopropyl)amino)methyl)pyrimidine-4-c arboxamido)methyl)phenyl)acrylate with a yield of 58%.

ESI-MS m/z=603.2 $[M+H]^+$.

Step d): preparation of methyl (E)-3-(4-((2-(4-(1H-pyrazol-1-yl)phenyl)-6-((tert-butoxycarbonyl)((1R,2S)-2-(4-fluorophenyl)cyclopropyl)amino)m ethyl)pyrimidine-4-carboxamido)methyl)phenyl)acrylate

Methyl (E)-3-(4-((2-(4-(1H-pyrazol-1-yl)phenyl)-6-((((1R,2S)-2-(4-fluorophenyl)cyclopropyl)amino)methyl)pyrimidine-4-c arboxamido)methyl)phenyl)acrylate (239 mg, 396 μmol) and triethylamine (1 mL) were dissolved in DCM (10 mL), then di-tert-butyl dicarbonate (131 mg, 594 μmol) was added, and the reaction was conducted at room temperature for 12 hours. After the reaction was completed, the reaction liquid was concentrated to dryness, and the residue was purified by silica gel chromatography (eluent: petroleum ether/ethyl acetate = 1/1) to afford methyl (E)-3-(4-((2-(4-(1H-pyrazol-1-yl)phenyl)-6-((tert-butoxycarbonyl)((1R,2S)-2-(4-fluorophenyl)cyclopropyl)amino)m ethyl)pyrimidine-4-carboxamido)methyl)phenyl)acrylate with a yield of 90%.

ESI-MS m/z=703.2 $[M+H]^+$.

Step e): preparation of (E)-3-(4-((2-(4-(1H-pyrazol-1-yl)phenyl)-6-((tert-butoxycarbonyl)((1R,2S)-2-(4-fluorophenyl)cyclopropyl)amino)m ethyl)pyrimidine-4-carboxamido)methyl)phenyl)acrylic acid

Methyl (E)-3-(4-((2-(4-(1H-pyrazol-1-yl)phenyl)-6-((tert-butoxycarbonyl)((1R,2S)-2-(4-fluorophenyl)cyclopropyl)amino)m ethyl)pyrimidine-4-carboxamido)methyl)phenyl)acrylate (251 mg, 356 μmol) and lithium hydroxide monohydrate (75 mg, 1.8 mmol) were added to a reaction flask containing THF (4 mL) and water (1 mL), and the mixture was stirred at room temperature for 2 hours. The pH of the reaction liquid was adjusted to 4 by dropwise addition of 2M hydrochloric acid aqueous solution, followed by extraction with MeOH/DCM (1:5) (20 mL × 2). The organic phases were combined, washed with saturated brine (15 mL × 2), dried with anhydrous sodium sulfate, and concentrated to afford (E)-3-(4-((2-(4-(1H-pyrazol-1-yl)phenyl)-6-((tert-butoxycarbonyl)((1R,2S)-2-(4-fluorophenyl)cyclopropyl)amino)m ethyl) pyrimidine-4-carboxamido)methyl)phenyl)acrylic acid, which was used directly for the next step.

ESI-MS m/z=689.2 $[M+H]^+$.

Step f): preparation of tert-butyl ((2-(4-(1H-pyrazol-1-yl)phenyl)-6-((4-((E)-3-oxo-3-((tetrahydro-2H-pyran-2-yl)oxy)amino)prop-1-en-1-yl)benzyl)c arbamoyl)pyrimidin-4-yl)methyl)((1R,2S)-2-(4-fluorophenyl)cyclopropyl)carbamate

(E)-3-(4-((2-(4-(1H-pyrazol-1-yl)phenyl)-6-((tert-butoxycarbonyl)((1R,2S)-2-(4-fluorophenyl)cyclopropyl)ami no)methyl)pyrimidine-4-carboxamido)methyl)phenyl)acrylic acid (245 mg, 356 μmol), O-(tetrahydro-2H-pyran-2-yl)hydroxylamine (62 mg, 534 μmol), HATU (162 mg, 427 μmol), and DIPEA (67 mg, 534 μmol) were added to a reaction flask containing DMF (5 mL), and the mixture was stirred at room temperature for 2 hours. The reaction liquid was quenched by adding water (10 mL), followed by extraction with ethyl acetate (20 mL × 2), and the organic phases were combined, washed with saturated brine (15 mL × 2), dried with anhydrous sodium sulfate, and concentrated under vacuum. The residue was purified by silica gel chromatography (eluent: DCM/MeOH = 10/1) to afford tert-butyl ((2-(4-(1H-pyrazol-1-yl) phenyl)-6-((4-((E)-3-oxo-3-((tetrahydro-2H-pyran-2-yl)oxy)amino)prop-1-en-1-yl)benzyl)c arbamoyl)pyrimidin-4-yl) methyl)((1R,2S)-2-(4-fluorophenyl)cyclopropyl)carbamate with a yield of 48%.

ESI-MS m/z=788.3 [M+H]$^+$.

Step g): preparation of 2-(4-(1H-pyrazol-1-yl)phenyl)-6-((((1R,2S)-2-(4-fluorophenyl)cyclopropyl)amino) methyl)-N-(4-((E)-3-(hydroxyam ino)-3-oxoprop-1-en-1-yl)benzyl)pyrimidine-4-carboxamide

Tert-butyl ((2-(4-(1H-pyrazol-1-yl)phenyl)-6-((4-((E)-3-oxo-3-((tetrahydro-2H-pyran-2-yl)oxy)amino)prop-1-en-1-yl)benzyl)c arbamoyl)pyrimidin-4-yl)methyl)((1R,2S)-2-(4-fluorophenyl)cyclopropyl)carbamate (135 mg, 171 μmol) was added to a reaction flask, followed by adding hydrogen chloride solution in ethyl acetate (4M, 2.5 mL), and the mixture was stirred at room temperature for 1 hour, resulting in the precipitation of a significant amount of solid. After concentrating under reduced pressure, the obtained crude product was purified by Prep-HPLC (separation method 3) to afford 2-(4-(1H-pyrazol-1-yl)phenyl)-6-((((1R,2S)-2-(4-fluorophenyl)cyclopropyl)amino)methyl)-N-(4-((E)-3-(hydroxyam ino)-3-oxoprop-1-en-1-yl)benzyl)pyrimidine-4-carboxamide formate with a yield of 22.5%.

$^1$H NMR (400 MHz, DMSO-$d_6$) δ 10.73 (s, 1H), 9.83 (t, *J* = 6.4 Hz, 1H), 9.02 (s, 1H), 8.79 - 8.69 (m, 2H), 8.66 (d, *J* = 2.6 Hz, 1H), 8.10 - 7.95 (m, 3H), 7.82 (d, *J* = 1.6 Hz, 1H), 7.54 (d, *J* = 7.8 Hz, 2H), 7.49 - 7.35 (m, 3H), 7.19 -7.02 (m, 4H), 6.62 (t, *J* = 2.0 Hz, 1H), 6.43 (d, *J* = 15.8 Hz, 1H), 4.61 (d, *J* = 6.4 Hz, 2H), 4.08 (s, 2H), 2.32 (dd, *J* = 7.0, 3.6 Hz, 1H), 1.92 (ddd, *J* = 9.2, 5.8, 2.4 Hz, 1H), 1.07 (dt, *J* = 9.2, 4.6 Hz, 1H), 0.97 (q, *J* = 5.8 Hz, 1H).

ESI-MS(m/z) =604.2 [M+H]$^+$.

**Example 233**

Preparation of (E)-3-(4-(((1-(4-(4-cyano-3-fluorophenyl)-3-(cyanomethyl)-5-(3-hydroxy-4-methoxyphenyl)pyridin-2-yl) piperidin-4-yl)amino)methyl)phenyl)-N-hydroxyacrylamide formate:

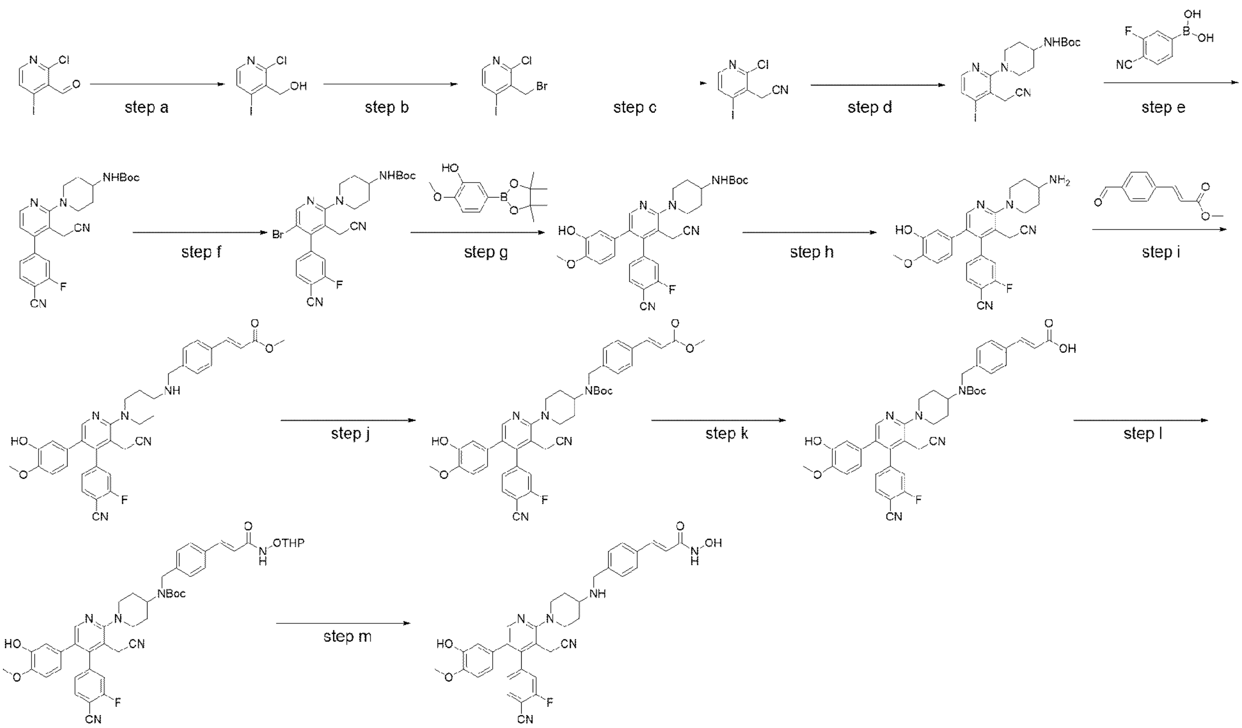


Step a): preparation of (2-chloro-4-iodopyridin-3-yl)methanol

2-Chloro-4-iodopyridine-3-formaldehyde (2 g, 7.48 mmol) was added to a reaction flask containing methanol (15 mL). After cooling the reaction system to 0°C, sodium borohydride (0.57 g, 15.03 mmol) was added, and the mixture was stirred at room temperature for 1 hour. The reaction liquid was quenched by adding saturated aqueous solution of sodium bicarbonate (20 mL), followed by extraction with ethyl acetate (20 mL × 2), and the organic phases were combined, washed with saturated brine (15 mL × 2), dried with anhydrous sodium sulfate, filtered, and concentrated to afford (2-chloro-4-iodopyridin-3-yl)methanol with a yield of 89%

ESI-MS m/z=269.9 [M+H]$^+$.

Step b): preparation of 3-(bromomethyl)-2-chloro-4-iodopyridine

(2-Chloro-4-iodopyridin-3-yl)methanol (400 mg, 1.48 mmol), triphenylphosphine (1.16 g, 4.43 mmol), and carbon tetrabromide (1.47 g, 4.43 mmol) were added to a reaction flask containing DCM (20 mL), and the mixture was stirred at room temperature for 3 hours. The reaction liquid was concentrated by adding silica gel, and the residue was purified by silica gel chromatography (eluent: petroleum ether/ethyl acetate = 2/1) to afford 3-(bromomethyl)-2-chloro-4-iodopyridine with a yield of 81%

ESI-MS m/z=331.8 $[M+H]^+$.

Step c): preparation of 2-(2-chloro-4-iodopyridin-3-yl)acetonitrile

3-(Bromomethyl)-2-chloro-4-iodopyridine (1.13 g, 3.40 mmol) and lithium hydroxide (0.17 g, 4.08 mmol) were added to a reaction flask containing acetonitrile (20 mL), and the reaction system was cooled to 0°C. Then trimethylsilyl cyanide (0.40 g, 4.08 mmol) was added, and the mixture was stirred at room temperature for 4 hours. The reaction liquid was quenched by adding water (20 mL), followed by extraction with ethyl acetate (20 mL × 2), and the organic phases were combined, washed with saturated brine (15 mL × 2), dried with anhydrous sodium sulfate, and filtered. The residue was purified by silica gel chromatography (eluent: petroleum ether/ethyl acetate = 1/1) to afford 2-(2-chloro-4-iodopyridin-3-yl) acetonitrile with a yield of 97%

ESI-MS m/z=278.9 $[M+H]^+$.

Step d): preparation of tert-butyl N-(1-(3-(cyanomethyl)-4-iodopyridin-2-yl)piperidin-4-yl)carbamate

2-(2-Chloro-4-iodopyridin-3-yl)acetonitrile (950 mg, 3.41 mmol), 4-Boc-aminopiperidine (1.37 g, 6.82 mmol), and DIEA (1.32 g, 10.23 mmol) were added to a reaction flask containing NMP (8 mL), and the mixture was stirred at 70°C for 4 hours. The reaction liquid was quenched by adding water (20 mL), followed by extraction with ethyl acetate (20 mL × 2), and the organic phases were combined, washed with saturated brine (15 mL × 2), dried with anhydrous sodium sulfate, and filtered. The residue was purified by silica gel chromatography (eluent: petroleum ether/ethyl acetate = 1/1) to afford tert-butyl N-(1-(3-(cyanomethyl)-4-iodopyridin-2-yl)piperidin-4-yl)carbamate with a yield of 19%.

ESI-MS m/z=442.2 $[M+H]^+$.

Step e): preparation of tert-butyl N-(1-(4-(4-cyano-3-fluorophenyl)-3-(cyanomethyl)pyridin-2-yl)piperidin-4-yl)carbamate

Tert-butyl N-(1-(3-(cyanomethyl)-4-iodopyridin-2-yl)piperidin-4-yl)carbamate (270 mg, 0.61 mmol), 4-cyano-3-fluorophenylboronic acid (0.12 g, 0.73 mmol), $Pd(dppf)Cl_2$ (0.045 g, 0.061 mmol), and cesium carbonate (0.40 g, 1.22 mmol) were added to a microwave tube containing 1,4-dioxane (4 mL) and water (1 mL), and the mixture was stirred at 80°C for 30 minutes. The reaction liquid was concentrated by adding silica gel, and the residue was purified by silica gel chromatography (eluent: petroleum ether/ethyl acetate = 1/1) to afford tert-butyl N-(1-(4-(4-cyano-3-fluorophenyl)-3-(cyanomethyl)pyridin-2-yl)piperidin-4-yl)carbamate with a yield of 90%.

ESI-MS m/z=436.2 $[M+H]^+$.

Step f): preparation of tert-butyl N-(1-(5-bromo-4-(4-cyano-3-fluorophenyl)-3-(cyanomethyl)pyridin-2-yl)piperidin-4-yl) carbamate

Tert-butyl N-(1-(4-(4-cyano-3-fluorophenyl)-3-(cyanomethyl)pyridin-2-yl)piperidin-4-yl)carbamate (210 mg, 0.48 mmol) and 1-bromopyrrolidine-2,5-dione (0.13 g, 0.73 mmol) were added to a reaction flask containing acetonitrile (5 mL), and the mixture was stirred at 0°C for 1 hour. The reaction liquid was quenched by adding water (10 mL), followed by extraction with ethyl acetate (20 mL × 2), and the organic phases were combined, washed with saturated brine (15 mL × 2), dried with anhydrous sodium sulfate, and filtered. The residue was purified by silica gel chromatography (eluent: petroleum ether/ethyl acetate = 1/1) to afford tert-butyl N-(1-(5-bromo-4-(4-cyano-3-fluorophenyl)-3-(cyanomethyl)pyridin-2-yl)piperidin-4-yl)carbamate with a yield of 97%

ESI-MS m/z=514.1 $[M+H]^+$.

Step g): preparation of tert-butyl N-(1-(4-(4-cyano-3-fluorophenyl)-3-(cyanomethyl)-5-(3-hydroxy-4-methoxyphenyl) pyridin-2-yl)piperidin-4-yl)carb amate

Tert-butyl N-(1-(5-bromo-4-(4-cyano-3-fluorophenyl)-3-(cyanomethyl)pyridin-2-yl)piperidin-4-yl)carbamate (230 mg, 0.45 mmol), 2-methoxy-5-(tetramethyl-1,3,2-dioxaborolan-2-yl)phenol (0.14 g, 0.54 mmol), $Pd(dppf)Cl_2$

(0.033 g, 0.045 mmol), and cesium carbonate (0.29 g, 0.90 mmol) were added to a microwave tube containing 1,4-dioxane (2 mL) and water (0.4 mL), and the mixture was stirred at 100°C for 30 minutes. The reaction liquid was concentrated by adding silica gel, and the residue was purified by silica gel chromatography (eluent: petroleum ether/ethyl acetate = 1/1) to afford tert-butyl N-(1-(4-(4-cyano-3-fluorophenyl)-3-(cyanomethyl)-5-(3-hydroxy-4-methoxyphenyl)pyridin-2-yl)piperidin-4-yl)carb amate with a yield of 64%.
ESI-MS m/z=558.2 $[M+H]^+$.

Step h): preparation of 6-(4-aminopiperidin-1-yl)-2-(4-cyano-3-fluorophenyl)-3-(3-hydroxy-4-methoxyphenyl)benzonitrile

Tert-butyl N-(1-(4-(4-cyano-3-fluorophenyl)-3-(cyanomethyl)-5-(3-hydroxy-4-methoxyphenyl)pyridin-2-yl)piperidin-4-yl)carb amate (160 mg, 0.29 mmol) and 4M hydrochloric acid solution in ethyl acetate (10 mL) were added to a reaction flask, and the mixture was stirred at room temperature for 1 hour. The pH of the reaction liquid was adjusted to 8 with saturated aqueous solution of $NaHCO_3$, followed by extraction with ethyl acetate (20 mL × 2). The organic phases were combined, washed with saturated brine (15 mL × 2), dried with anhydrous sodium sulfate, and filtered, concentrated to afford 4-(2-(4-aminopiperidin-1-yl)-3-(cyanomethyl)-5-(3-hydroxy-4-methoxyphenyl)pyridin-4-yl)-2-fluorobenzonitrile with a yield of 98%.
ESI-MS m/z=458.2 $[M+H]^+$.

Step i): preparation of methyl (E)-3-(4-{[(1-(4-(4-cyano-3-fluorophenyl)-3-(cyanomethyl)-5-(3-hydroxy-4-methoxyphenyl)pyridin-2-yl)piperidin-4-yl)amino]methyl}phenyl) prop-2-enoate

4-(2-(4-Aminopiperidin-1-yl)-3-(cyanomethyl)-5-(3-hydroxy-4-methoxyphenyl)pyridin-4-yl)-2-fluorobenzonitr ile (135 mg, 0.30 mmol) and methyl (E)-3-(4-formylphenyl)acrylate (0.11 g, 0.60 mmol) were added to a reaction flask containing DCE (5 mL). The mixture was stirred at room temperature for 1 hour, then the reaction system was cooled to 0°C and sodium cyanoborohydride (0.075 g, 1.2 mmol) was added, and the mixture was stirred at room temperature for 18 hours. The reaction liquid was quenched by adding water (20 mL), extracted with ethyl acetate (20 mL × 2), and the organic phases were combined, washed with saturated brine (15 mL × 2), dried with anhydrous sodium sulfate, and filtered. The residue was purified by silica gel chromatography (eluent: DCM/MeOH = 10/1) to afford methyl (E)-3-(4-{[(1-(4-(4-cyano-3-fluorophenyl)-3-(cyanomethyl)-5-(3-hydroxy-4-methoxyphenyl)pyridin-2-yl)piperidin-4-yl)amino]methyl}phenyl)prop-2-enoate with a yield of 40%.
ESI-MS m/z=632.3 $[M+H]^+$.

Step j): preparation of methyl (E)-3-(4-({[(tert-butoay)carbonyl](1-(4-(4-cyano-3-fluorophenyl)-3-(cyanomethyl)-5-(3-hydroay-4-methoayphenyl) pyridin-2-yl)piperidin-4-yl)amino}methyl)phenyl)prop-2-enoate

Methyl (E)-3-(4-{[(1-(4-(4-cyano-3-fluorophenyl)-3-(cyanomethyl)-5-(3-hydroxy-4-methoxyphenyl)pyridin-2-yl) piperidin-4-yl)amino]methyl}phenyl)prop-2-enoate (75 mg, 0.12 mmol), di-tert-butyl dicarbonate (0.13 g, 0.60 mmol), and triethylamine (0.036 g, 0.36 mmol) were added to a reaction flask containing DCM (5 mL). The mixture was stirred at room temperature for 1 hour, and the reaction liquid was concentrated to afford methyl (E)-3-(4-({[tert-butoxycarbonyl](1-(4-(4-cyano-3-fluorophenyl)-3-(cyanomethyl)-5-(3-hydroxy-4-methoxyphenyl)p yridin-2-yl)piperidin-4-yl)amino}methyl)phenyl)prop-2-enoate with a yield of 97%.
ESI-MS m/z=732.3 $[M+H]^+$.

Step k): preparation of (E)-3-(4-({[(tert-butoxy)carbonyl](1-(4-(4-cyano-3-fluorophenyl)-3-(cyanomethyl)-5-(3-hydroxy-4-methoxyphenyl) pyridin-2-yl)piperidin-4-yl)amino}methyl)phenyl)prop-2-enoic acid

Methyl (E)-3-(4-({[(tert-butoxy)carbonyl](1-(4-(4-cyano-3-fluorophenyl)-3-(cyanomethyl)-5-(3-hydroxy-4-methoxyphenyl) pyridin-2-yl)piperidin-4-yl)amino)methyl)phenyl)prop-2-enoate (85 mg, 0.12 mmol) and lithium hydroxide (0.057 g, 2.38 mmol) were added to a reaction flask containing THF (5 mL), methanol (3 mL), and water (2 mL), and the mixture was stirred at room temperature for 1 hour. The pH of the reaction liquid was adjusted to 4 by dropwise addition of 2M hydrochloric acid aqueous solution, followed by extraction with a mixture of DCM: MeOH=5: 1 (20 mL × 2), and the organic phases were combined, washed with saturated brine (15 mL × 2), dried with anhydrous sodium sulfate, filtered and concentrated to afford (E)-3-(4-({[(tert-butoay)carbonyl](1-(4-(4-cyano-3-fluorophenyl)-3-(cyanomethyl)-5-(3-hydroay-4-methoayphenyl) pyridin-2-yl)piperidin-4-yl)amino}methyl)phenyl) prop-2-enoic acid with a yield of 81%.
ESI-MS m/z=718.3 $[M+H]^+$.

Step 1): preparation of tert-Butyl N-(1-(4-(4-(4-cyano-3-fluorophenyl)-3-(cyanomethyl)-5-(3-hydroxy-4-methoxyphenyl)pyridin-2-yl)piperidin-4-yl)-N-[(4-[(1E)-2-[(oxacyclopropan-2-yloxy)carbamoyl]eth-1-en-1-yl]phenyl)methyl]carbamate

(E)-3-(4-(1[(Tert-butoxy)carbonyl](1-(4-(4-cyano-3-fluorophenyl)-3-(cyanomethyl)-5-(3-hydroxy-4-methoxyphenyl)pyridin-2-yl)piperidin-4-yl)amino}methyl)phenyl)prop-2-enoic acid (70 mg, 0.098 mmol), O-(tetrahydro-2H-pyran-2-yl)hydroxylamine (0.023 g, 0.20 mmol), HATU (0.056 g, 0.15 mmol), and DIEA (0.051 g, 0.39 mmol) were added to a reaction flask containing DMF (4 mL), and the mixture was stirred at room temperature for 1 hour. The reaction liquid was quenched by adding water (10 mL), followed by extraction with ethyl acetate (10 mL × 2), and the organic phases were combined, washed with saturated brine (15 mL × 2), dried with anhydrous sodium sulfate, and filtered. The residue was purified by silica gel chromatography (eluent: petroleum ether/ethyl acetate = 1/1) to tert-butyl N-(1-(4-(4-(4-cyano-3-fluorophenyl)-3-(cyanomethyl)-5-(3-hydroxy-4-methoxyphenyl)pyridin-2-yl)piperidin-4-yl)-N-[(4-[(1E)-2-[(oxacyclopropan-2-yloxy)carbamoyl]eth-1-en-1-yl]phenyl)methyl]carbamate with a yield of 62%.
ESI-MS m/z=817.4 $[M+H]^+$.

Step m): preparation of (E)-3-(4-(((1-(4-(4-cyano-3-fluorophenyl)-3-(cyanomethyl)-5-(3-hydroxy-4-methoxyphenyl)pyridin-2-yl)piperidin-4-yl)amino)methyl)phenyl)-N-hydroxyacrylamide formate

Tert-butyl N-(1-(4-(4-(4-cyano-3-fluorophenyl)-3-(cyanomethyl)-5-(3-hydroxy-4-methoxyphenyl)pyridin-2-yl)piperidin-4-yl)-N-[(4-[(1E)-2-[(oxacyclopropan-2-yloxy)carbamoyl]eth-1-en-1-yl]phenyl)methyl]carbamate (50 mg, 0.061 mmol) was added to a reaction flask containing a 4M hydrochloric acid solution in ethyl acetate (5 mL), and the mixture was stirred at room temperature for 30 minutes. The reaction liquid was concentrated, and the residue was purified by Prep-HPLC (separation method 3) to afford (E)-3-(4-(((1-(4-(4-cyano-3-fluorophenyl)-3-(cyanomethyl)-5-(3-hydroxy-4-methoxyphenyl)pyridin-2-yl)piperidin-4-yl)amino)methyl)phenyl)-N-hydroxyacrylamide formate with a yield of 50%.
$^1$H NMR (400 MHz, DMSO-$d_6$)δ ppm : 10.78 (s, 1H), 8.97 (s, 1H), 8.51 (s, 1H), 8.32 (d, J = 2.1 Hz, 1H), 8.15 (d, J = 0.7 Hz, 1H), 7.95 (dd, J = 8.0, 6.9 Hz, 1H), 7.59 (d, J = 8.0 Hz, 2H), 7.56 - 7.47 (m, 3H), 7.44 (s, 1H), 7.22 - 7.14 (m, 1H), 6.81 - 6.74 (m, 1H), 6.53 - 6.43 (m, 3H), 4.04 (s, 2H), 3.86 (s, 1H), 3.76 (d, J = 1.8 Hz, 2H), 3.71 (s, 3H), 3.42 (t, J = 13.1 Hz, 2H), 2.94 (dd, J = 22.3, 11.0 Hz, 2H), 2.12 (d, J = 12.1 Hz, 2H), 1.74 (s, 2H).
ESI-MS m/z=633.3 $[M+H]^+$.

**Example 234**

(E)-3-(5-(((1-(4-Cyano-6-(4-cyano-3-fluorophenyl)-5-(3-hydroxy-4-methoxyphenyl)pyridin-2-yl)piperidin-4-yl)amino)methyl)pyrimidin-2-yl)-N-hydroxyacrylamide formate was prepared according to the synthetic method of Example 176, and the structure and characterization data are as follows:

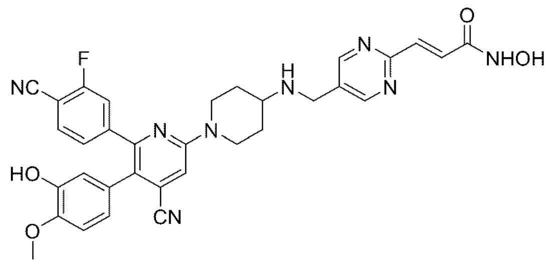

$^1$H NMR (400 MHz, Methanol-$d_4$) δ ppm 8.95 (s, 2H), 8.76 (s, 1H), 7.63 - 7.52 (m, 2H), 7.42 - 7.21 (m, 4H), 6.93 (d, *J* = 8.8 Hz, 1H), 6.63 (dq, *J* = 4.4, 2.2 Hz, 2H), 4.69 (d, *J* = 13.8 Hz, 2H), 4.41 (s, 2H), 3.88 (s, 3H), 3.62 (t, *J* = 11.4 Hz, 1H), 3.08 (d, *J* = 12.8 Hz, 2H), 2.32 (d, *J* = 11.6 Hz, 2H), 1.82 - 1.64 (m, 2H).
ESI-MS(m/z)= 621.2 $[M+H]^+$.
**Examples 235-262** were prepared according to the synthetic method of Example **37** (the separation method for the compounds: hydrochloride and formate were prepared by separation method 1 and 3, respectively), and the structure and characterization data are as follows:

| Example | Chemical name | Structure | $^1$H NMR | MS(M+H)$^+$ |
|---|---|---|---|---|
| 235 | (E)-3-(4-(1-((1-(3 -Cyano-4-(4-cyan o-3-fluorophenyl) -5-(3-hydroxy-4-( 2-hydroxy-2-meth ylpropoxy)phenyl )pyridin-2-yl)pipe ridin-4-yl)amino) ethyl)phenyl)-N-hydroxyacrylamide formate | 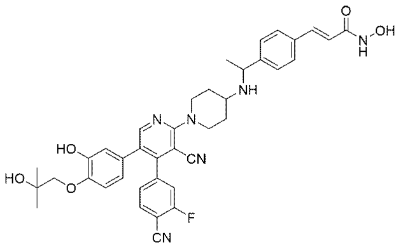 | $^1$H NMR (400 MHz, Methanol-d4) δ ppm 8.48 (s, 1H), 8.41 (s, 1H), 7.74 (t, J = 7.2 Hz, 1H), 7.66 (d, J = 7.8 Hz, 2H), 7.60 (d, J = 15.8 Hz, 1H), 7.52 (d, J = 8.0 Hz, 2H), 7.39 - 7.30 (m, 1H), 7.22 (dd, J = 8.0, 1.6 Hz, 1H), 6.79 (d, J = 8.2 Hz, 1H), 6.58 - 6.43 (m, 3H), 4.46 (d, J = 7.0 Hz, 1H), 4.42 - 4.25 (m, 2H), 3.78 (s, 2H), 3.04 (d, J = 13.2 Hz, 3H), 2.24 (d, J = 12.6 Hz, 1H), 2.07 (d, J = 12.4 Hz, 1H), 1.81-1.67 (m, 2H), 1.59 (d, J = 6.6 Hz, 3H), 1.30 (s, 6H). | 691.3 |
| 236 | (E)-3-(6-(((1-(3-C yano-4-(4-cyano-3-fluorophenyl)-5 -(5-fluoro-3-meth ylbenzo[d]isoxazo 1-6-yl)pyridin-2-yl )piperidin-4-yl)a mino)methyl) pyridin-3-yl)-N-hydro xyacrylamide formate | 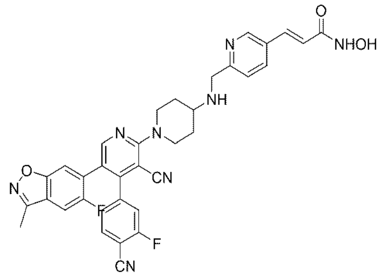 | $^1$H NMR (400 MHz, DMSO-d6) δ ppm : 10.82 (s, 1H), 9.10 (s, 1H), 8.72 (d, J = 2.2 Hz, 1H), 8.53 (s, 1H), 8.00 (dd, J = 8.1, 2.2 Hz, 1H), 7.91 (t, J = 7.4 Hz, 1H), 7.76 (dd, J = 23.4, 7.6 Hz, 1H), 7.62 (d, J = 8.8 Hz, 1H), 7.54 (d, J = 7.6 Hz, 1H), 7.59 - 7.46 (m, 2H), 7.32 (d, J = 8.1 Hz, 1H), 6.56 (d, J = 15.9 Hz, 1H), 4.33 (d, J = 13.1 Hz, 2H), 4.03 (s, 2H), 3.25 (t, J = 12.0 Hz, 2H), 2.93 (s, 1H), 2.49 (t, J = 12.6 Hz, 3H), 2.06 (d, J = 12.0 Hz, 2H), 1.54 (d, J = 11.6 Hz, 2H). | 647.2 |
| 237 | (E)-3-(4-((1-(5'-(4 -Chloro-3-hydrox yphenyl)-3',6-dicyano-[3,4'-bipyridi n]-2'-yl)piperidin-4-yl)amino)methy 1) phenyl)-N-hydro xyacrylamide formate | 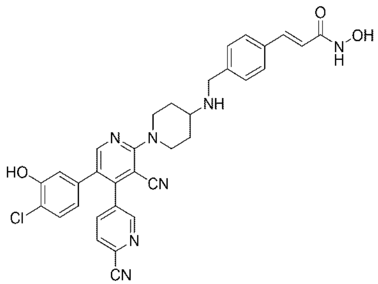 | $^1$H NMR (400 MHz, Methanol-$d_4$) δ 8.52 (d, *J* = 2.0 Hz, 1H), 8.42 (s, 1H), 8.37 (s, 1H), 7.88 - 7.76 (m, 2H), 7.57 - 7.45 (m, 3H), 7.39 (d, *J* = 7.9 Hz, 2H), 7.10 (d, *J* = 8.2 Hz, 1H), 6.52 (d, *J* = 2.2 Hz, 1H), 6.46 - 6.36 (m, 2H), 4.39 (d, *J* = 13.4 Hz, 2H), 3.97 (s, 2H), 3.15 - 3.00 (m, 3H), 2.11 (d, *J* = 12.4 Hz, 2H), 1.61 (q, *J* = 11.6 Hz, 2H). | 606.2 |

(continued)

| Example | Chemical name | Structure | $^1$H NMR | MS(M+H)$^+$ |
|---|---|---|---|---|
| 238 | (E)-3-(6-((1-(5'-(4 -Chloro-3-hydrox yphenyl)-3',6-dicy ano-[3,4'-bipyridi n]-2'-yl)piperidin-4-yl)amino)methy 1) pyridin-3-yl)-N-hydroxyacry-lamid e formate | 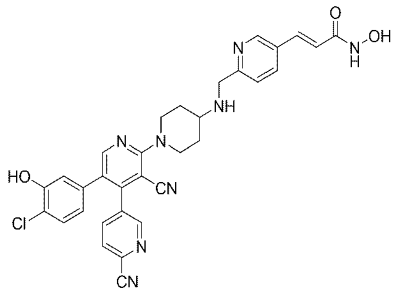 | $^1$H NMR (400 MHz, Methanol-$d_4$) δ 8.71 (s, 1H), 8.52 (s, 1H), 8.40 (s, 1H), 8.05 - 7.95 (m, 1H), 7.88 - 7.75 (m, 2H), 7.53 (d, $J$ = 8.8 Hz, 2H), 7.43 (d, $J$ = 8.2 Hz, 1H), 7.10 (d, $J$ = 8.1 Hz, 1H), 6.60 - 6.48 (m, 2H), 6.42 (d, $J$ = 8.0 Hz, 1H), 4.44 (d, $J$ = 17.0 Hz, 4H), 3.18 - 2.98 (m, 3H), 2.25 (d, $J$ = 12.2 Hz, 2H), 1.82 (d, $J$ = 12.6 Hz, 2H). | 607.2 |
| 239 | (E)-3-(6-(((1-(5-( 4-Chloro-3-hydro xyphenyl)-3-cyan o-4-(4-cyano-3-fl uorophenyl) pyridi n-2-yl)piperidin-4 -yl) amino)methyl) pyridin-3-yl)-N-h ydroxyacrylamide for-mate | 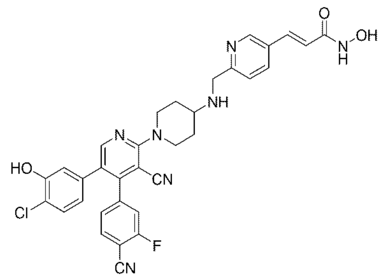 | $^1$H NMR (400 MHz, Methanol-$d_4$) δ 8.75 (d, $J$ = 2.1 Hz, 1H), 8.46 (d, $J$ = 21.4 Hz, 2H), 8.06 (dd, $J$ = 8.1, 2.2 Hz, 1H), 7.76 (t, $J$ = 7.3 Hz, 1H), 7.61 (d, $J$ = 15.9 Hz, 1H), 7.53 (d, $J$ = 8.2 Hz, 1H), 7.42 (d, $J$ = 9.6 Hz, 1H), 7.25 - 7.16 (m, 2H), 6.68 - 6.56 (m, 2H), 6.53 (dd, $J$ = 8.2, 2.0 Hz, 1H), 4.46 (d, $J$ = 13.3 Hz, 2H), 4.29 (s, 2H), 3.24 (s, 1H), 3.18 (t, $J$ = 12.9 Hz, 2H), 2.27 - 2.20 (m, 2H), 1.79 (dd, $J$ = 13.4, 9.6 Hz, 2H). | 624.2 |
| 240 | (E)-3-(6-(((1-(3',6 -Dicyano-5'-(2-flu oro-5-hydroxy-4-methoxyphenyl)-[ 3,4'-bipyridin]-2'-yl)piperidin-4-yl) amino)methyl)pyr idin-3-yl)-N-hydr oxyacrylamide formate | 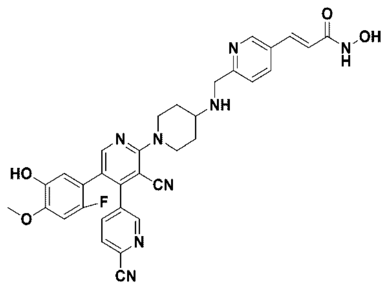 | $^1$H NMR (400 MHz, DMSO-$d_6$) δ 10.82 (s, 1H), 9.07 (s, 1H), 8.85 - 8.60 (m, 2H), 8.42 (s, 1H), 8.14 (d, $J$ = 1.6 Hz, 1H), 8.11 (d, $J$ = 8.0 Hz, 1H), 8.06 - 7.95 (m, 2H), 7.65 - 7.44 (m, 2H), 6.67 (m, 2H), 6.57 (d, $J$ = 16.0 Hz, 1H), 4.29 (d, $J$ = 13.2 Hz, 2H), 4.09 (s, 2H), 3.71 (s, 3H), 3.19 (d, $J$ = 12.0 Hz, 2H), 3.00 (s, 1H), 2.08 (d, $J$ = 12.4 Hz, 2H), 1.57 (d, $J$ = 11.6 Hz, 2H). | 621.2 |

(continued)

| Example | Chemical name | Structure | $^1$H NMR | MS(M+H)$^+$ |
|---|---|---|---|---|
| 241 | (E)-3-(4-(((1-(5'-( 4-Chloro-3-hydro xyphenyl)-3',6-dicyano-[3,4'-bipyrid in]-2'-yl)piperidin -4-yl)amino)methyl)-3-fluoropheny 1)-N-hydroxyacryl amide formate | 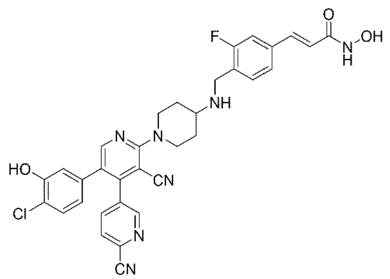 | $^1$H NMR (400 MHz, DMSO-$d_6$) $\delta$ 10.80 (s, 1H), 10.27 (s, 1H), 9.12 (s, 1H), 8.72 (d, $J$ = 2.0 Hz, 1H), 8.52 (s, 1H), 8.15 (d, $J$ = 8.0 Hz, 1H), 8.05 (dd, $J$ = 8.0, 2.2 Hz, 1H), 7.68 (t, $J$ = 7.6 Hz, 1H), 7.61 - 7.42 (m, 3H), 7.24 (d, $J$ = 8.2 Hz, 1H), 6.66 - 6.46 (m, 3H), 4.40 (s, 2H), 4.36 (t, $J$ = 9.2 Hz, 2H), 4.27 (s, 2H), 3.50 (s, 1H), 3.18 (t, $J$ = 12.0 Hz, 2H), 2.26 (s, 2H). | 624.2 |
| 242 | (E)-3-(4-(((1-(3-C yano-4-(4-cyano-3-fluorophenyl)-5 -(3-hydroxy-4-me thoxyphenyl) pyri din-2-yl)-3-methy lpiperidin-4-yl)am ino)methyl)-N-hy droxyacrylamide formate | 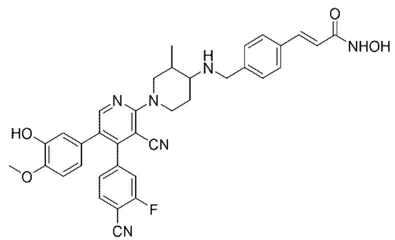 | $^1$H NMR (400 MHz, Methanol-$d_4$) $\delta$ ppm 8.51 (s, 1H), 8.40 (s, 1H), 7.74 (t, $J$ = 7.2 Hz, 1H), 7.67-7.53 (m, 3H), 7.47 (d, $J$ = 7.8 Hz, 2H), 7.36 (d, $J$ = 9.6 Hz, 1H), 7.23 (d, $J$ = 8.0 Hz, 1H), 6.81 (d, $J$ = 8.6 Hz, 1H), 6.56-6.42 (m, 3H), 4.41 (d, $J$ = 13.2 Hz, 1H), 4.28 (d, $J$ = 12.8 Hz, 1H), 4.08 (d, $J$ = 13.4 Hz, 1H), 3.93 (d, $J$ = 13.4Hz, 1H), 3.81 (s, 3H), 3.18-3.09 (m, 1H), 2.84 (dd, $J$ = 13.4, 10.6 Hz, 1H), 2.63 (s, 1H), 2.2 2 (d, $J$ = 13.2 Hz, 1H), 1.91 (s, 1H), 1.68 (d, $J$ = 11.8 Hz, 1H), 1.09 (d, $J$ = 6.6 Hz, 3H). | 633.3 |
| 243 | (E)-3-(4-(((1-(3',6 -Dicyano-5'-(3-hy droxy-4-methoxy phenyl)-5-methox y-[3,4'-bipyridin]-2'-yl)piperidin-4-y 1)amino)methyl)p henyl)-N-hydroxy acrylamide hydrochloride | 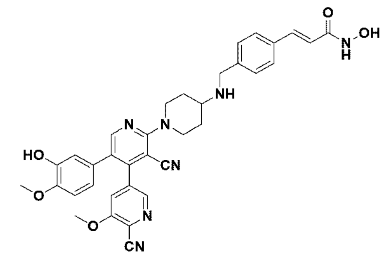 | $^1$H NMR (400 MHz, DMSO-$d_6$) $\delta$10.82 (s, 1H), 9.42 (s, 2H), 8.50 (s, 1H), 8.42 (s, 1H), 8.12 (d, $J$ = 1.6 Hz, 1H), 7.96 (d, $J$ = 1.6 Hz, 1H), 7.64 (s, 3H), 7.48 (d, $J$ = 15.6 Hz, 1H), 6.84 (d, $J$ = 8.0 Hz, 1H), 6.59 - 6.43 (m, 3H), 4.35 (d, $J$ = 13.2 Hz, 2H), 4.24 (t, $J$ = 6.0 Hz, 2H), 3.89 (s, 3H), 3.73 (s, 3H), 3.37 (d, $J$ = 13.6 Hz, 1H), 3.14 (t, $J$ = 12.8 Hz, 2H), 2.29 (d, $J$ = 120 Hz, 2H), 1.84 (t, $J$ = 12.0 Hz, 2H). | 632.3 |
| 244 | (E)-3-(4-(((1-(3',6 -Dicyano-5'-(3-hy droxy-4-morpholinophenyl)-[3,4'-bi pyridin]-2'-yl)pipe ridin-4-yl)amino) methyl)phenyl)-N -hydroxyacrylami de formate | 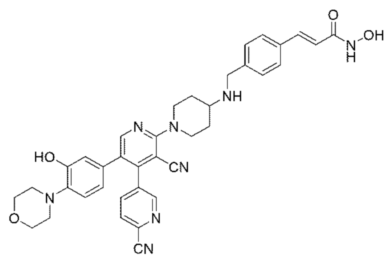 | $^1$H NMR (400 MHz, DMSO-$d_6$) $\delta$ 10.80 (s, 1H), 9.22 (s, 1H), 9.08 (s, 1H), 8.68 (d, $J$ = 2.2 Hz, 1H), 8.49 (s, 1H), 8.18 - 8.10 (m, 1H), 8.06 (dd, $J$ = 8.0, 2.2 Hz, 1H), 7.63 (d, $J$ = 7.6 Hz, 2H), 7.59 - 7.41 (m, 3H), 6.75 (d, $J$ = 8.2 Hz, 1H), 6.56 - 6.45 (m, 2H), 6.41 (d, $J$ = 2.0 Hz, 1H), 4.31 (d, $J$ = 12.8 Hz, 2H), 4.19 (s, 2H), 3.69 (t, $J$ = 4.4 Hz, 4H), 3.30 (s, 1H), 3.15 (t, $J$ = 12.4 Hz, 2H), 2.91 (d, $J$ = 4.4 Hz, 4H), 2.22 (s, 2H), 1.71 (s, 2H). | 657.3 |

(continued)

| Example | Chemical name | Structure | $^1$H NMR | MS(M+H)$^+$ |
|---|---|---|---|---|
| 245 | (E)-3-(4-(((1-(3'-Cyano-5'-(3-hydr oxy-4-methoxyph enyl)-6-(trifluoro methyl)-[3,4'-bipy ridin]-2'-yl)piperi din-4-yl)amino)m ethyl)phenyl)-N-h ydroxyacrylamide formate | 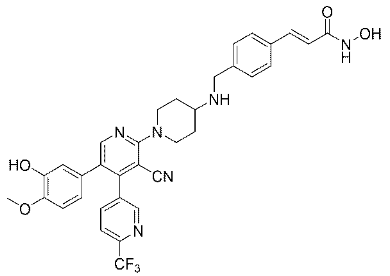 | $^1$H NMR (400 MHz, DMSO-$d_6$) δ ppm : 10.75 (s, 1H), 9.01 (s, 1H), 8.67 (d, J = 2.0 Hz, 1H), 8.45 (s, 1H), 8.16 (s, 1H), 8.07 (dd, J = 8.1, 2.1 Hz, 1H), 7.98 (d, J = 8.1 Hz, 1H), 7.55 (d, J = 7.9 Hz, 2H), 7.51 - 7.41 (m, 3H), 6.81 (d, J = 8.1 Hz, 1H), 6.51 - 6.42 (m, 3H), 4.24 (d, J = 13.1 Hz, 2H), 3.94 (s, 2H), 3.71 (s, 3H), 3.17 (t, J = 12.0 Hz, 2H), 2.93 (s, 1H), 2.08 (d, J = 12.2 Hz, 2H), 1.56 (q, J = 11.5, 10.9 Hz, 2H). | 645.2 |
| 246 | (E)-3-(6-(((1-(3-C yano-4-(4-cyano-3-fluorophenyl)-5 -(3-hydroxy-4-mo rpholinophenyl)p yridin-2-yl)piperi din-4-yl)amino)m ethyl)pyridin-3-yl )-N-hydroxyacryl amide formate | 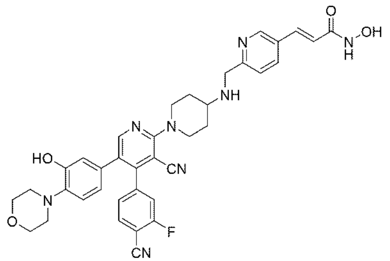 | $^1$H NMR (400 MHz, Methanol-$d_4$) δ 8.75 (d, *J* = 2.2 Hz, 1H), 8.50 (s, 1H), 8.42 (s, 1H), 8.05 (dd, *J* = 8.2, 2.2 Hz, 1H), 7.78 - 7.70 (m, 1H), 7.61 (d, *J* = 15.9 Hz, 1H), 7.53 (d, *J* = 8.1 Hz, 1H), 7.36 (dd, *J* = 9.7, 1.4 Hz, 1H), 7.23 (dd, *J* = 8.0, 1.5 Hz, 1H), 6.89 (d, *J* = 8.1 Hz, 1H), 6.67 - 6.47 (m, 3H), 4.42 (d, *J*= 13.4 Hz, 2H), 4.29 (s, 2H), 3.81 (t, *J* = 4.5 Hz, 4H), 3.19 (q, *J* = 14.4, 12.8 Hz, 3H), 2.94 (t, *J* = 4.5 Hz, 4H), 2.23 (d, *J* = 12.3 Hz, 2H), 1.87 - 1.70 (m, 2H). | 675.3 |
| 247 | (E)-3-(4-(((1-(3',6 -Dicyano-5'-(3-hy droxy-4-methoxy phenyl)-5-methyl-[3,4'-bipyridin]-2' -yl)piperidin-4-yl) amino)methyl)phe nyl)-N-hydro-xyac rylamide formate | 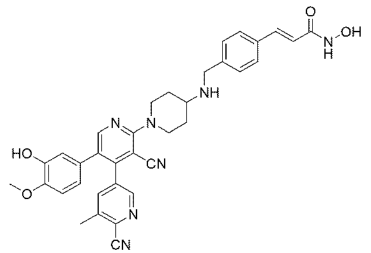 | $^1$H NMR (400 MHz, DMSO-$d_6$) δ ppm : 10.74 (s, 1H), 8.82 (d, J = 124.3 Hz, 1H), 8.44 (s, 1H), 8.40 (d, J = 1.9 Hz, 1H), 8.18 (d, J = 1.3 Hz, 1H), 8.02 (d, J = 2.0 Hz, 1H), 7.54 (d, J = 7.9 Hz, 2H), 7.49 - 7.40 (m, 3H), 6.81 (d, J = 8.1 Hz, 1H), 6.50 - 6.41 (m, 3H), 4.23 (dd, J = 10.4, 6.4 Hz, 2H), 3.91 (s, 2H), 3.71 (s, 3H), 3.15 (d, J = 11.2 Hz, 2H), 2.94 - 2.84 (m, 1H), 2.53 - 2.46 (m, 3H), 2.05 (dd, J = 12.7, 3.9 Hz, 2H), 1.53 (q, J = 10.3 Hz, 2H). | 616.3 |

(continued)

| Example | Chemical name | Structure | $^1$H NMR | MS(M+H)$^+$ |
|---|---|---|---|---|
| 248 | (E)-3-(4-(((1-(3'-Cyano-5'-(3-hydr oxy-4-methoxyph enyl)-6-nitro-[3,4' -bipyridin]-2'-yl)p iperidin-4-yl)amin o)methyl)phenyl)-N-hydro-xyacryla mide formate | 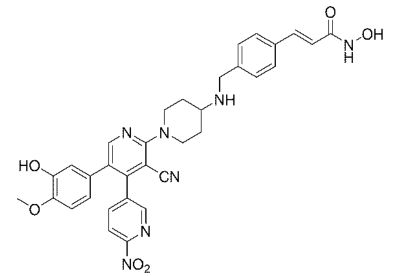 | $^1$H NMR (400 MHz, Methanol-$d_4$) δ ppm 8.50 (d, $J$ = 5.8 Hz, 2H),8.44 (s, 1H), 8.32 (d, $J$ = 8.2 Hz, 1H), 8.14 (dd, $J$ = 8.4, 2.2 Hz, 1H), 7.73-7.45 (m, 5H), 6.94-6.70 (m, 1H), 6.59- 6.27 (m, 3H), 4.49 (d, $J$ = 13.4 Hz, 2H), 4.25 (s, 2H), 3.80 (s, 3H), 3.42 (d, $J$ = 12.0 Hz, 1H), 3.20 (t, $J$ = 12.8 Hz, 2H), 2.31 (d, $J$ = 12.2 Hz, 2H), 1.96-1.70 (m,2H). | 622.2 |
| 249 | (E)-3-(4-(((1-(3',6 -Dicyano-5-fluoro -5'-(3-hydroxy-4-methoxyphenyl)-[ 3,4'-bipyridin]-2'-yl)piperidin-4-yl) amino)methyl)phe nyl)-N-hydro-xyac rylamide formate | 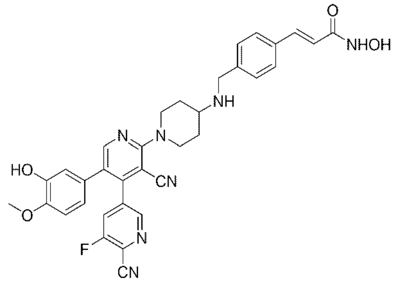 | $^1$H NMR (400 MHz, DMSO-$d_6$) δ 10.73 (s, 1H), 9.07 (s, 1H), 8.48 (s, 2H), 8.35 (dd, $J$ = 9.6, 1.6 Hz, 1H), 8.15 (s, 1H), 7.54 (d, $J$ = 7.8 Hz, 2H), 7.48 - 7.40 (m, 3H), 6.83 (d, $J$ = 8.4 Hz, 1H), 6.52 - 6.40 (m, 3H), 4.23 (d, $J$ = 13.2 Hz, 2H), 3.89 (s, 2H), 3.72 (s, 3H), 3.19 (t, $J$ = 12.0 Hz, 2H), 2.86 (s, 1H), 2.04 (d, $J$ = 12.4 Hz, 2H), 1.51 (q, $J$ = 11.2 Hz, 2H). | 620.2 |
| 250 | E)-3-(4-(((1-(3-C yano-4-(2-cyanop yrimidin-5-yl)-5-( 3-hydroxy-4-meth oxyphenyl) pyridi n-2-yl)piperidin-4 -yl) amino)methyl) phenyl)-N-hy-drox yacrylamide formate | 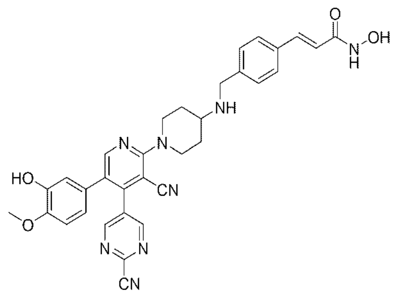 | $^1$H NMR (400 MHz, Methanol-$d_4$) δ 8.85 (s, 2H), 8.49 (s, 1H), 8.48 (s, 1H), 7.70 - 7.58 (m, 2H), 7.54 (t, $J$ = 8.7 Hz, 3H), 6.86 (d, $J$ = 8.1 Hz, 1H), 6.53 (dd, $J$ = 4.0, 1.8 Hz, 2H), 6.50 (d, $J$ = 2.1 Hz, 1H), 4.50 (d, $J$ = 13.4 Hz, 2H), 4.21 (s, 2H), 3.83 (s, 3H), 3.37 (d, $J$ = 11.7 Hz, 1H), 3.20 (t, $J$ = 12.7 Hz, 2H), 2.29 (d, $J$ = 12.2 Hz, 2H), 1.81 (tt, $J$ = 12.6, 5.8 Hz, 2H). | 603.2 |

(continued)

| Example | Chemical name | Structure | $^1$H NMR | MS(M+H)$^+$ |
|---|---|---|---|---|
| 251 | (E)-3-(4-(((1-(3'-Cyano-6-(difluoro methyl)-5'-(3-hydroxy-4-methoxyp henyl)-[3,4'-bipyri din]-2'-yl)piperidi n-4-yl)amino)met hyl)phenyl)-N-hy droxyacrylamide formate | 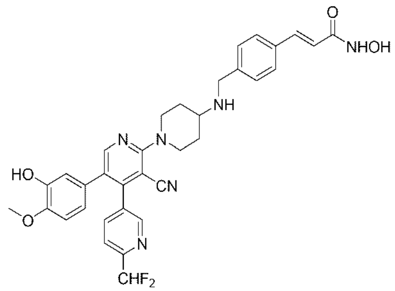 | $^1$H NMR (400 MHz, DMSO-$d_6$) δ 10.74 (s, 1H), 8.99 (s, 1H), 8.51 (s, 1H), 8.55 (d, *J* = 2.1 Hz, 1H), 8.42 (s, 1H), 7.94 (dd, *J* = 8.2, 2.2 Hz, 1H), 7.72 (d, *J* = 8.1 Hz, 1H), 7.55 (d, *J* = 7.9 Hz, 2H), 7.49 - 7.40 (m, 3H), 6.86 - 6.76 (m, 1H), 6.45 (ddd, *J* = 7.8, 6.0, 3.4 Hz, 3H), 4.22 (d, *J* = 13.0 Hz, 2H), 3.95 (s, 2H), 3.69 (s, 3H), 3.15 (t, *J* = 12.2 Hz, 2H), 2.94 (s, 1H), 2.15 - 1.99 (m, 2H), 1.55 (d, *J* = 11.7 Hz, 2H). | 627.3 |
| 252 | (E)-3-(4-(((1-(3',6 -Dicyano-5'-(3-hy droxy-4-methoxy phenyl)-[3,4'-bipy ridin]-2'-yl)piperi din-4-yl)amino)m ethyl)-3-fluorophe nyl)-N-hydroxyac rylamide formate | 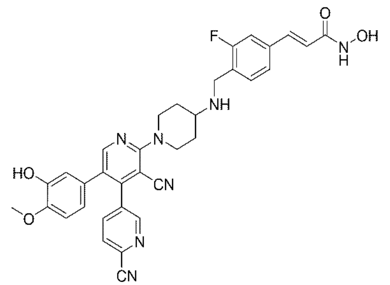 | $^1$H NMR (400 MHz, DMSO-$d_6$) δ ppm 10.81 (s, 1H), 9.03 (s, 2H), 8.65 (s, 1H), 8.46 (s, 1H), 8.13 (d, *J* = 7.8 Hz, 1H), 8.06 (dd, *J* = 8.0, 1.2 Hz, 1H), 7.60 (t, *J* = 13.6 Hz, 1H), 7.52 - 7.37 (m, 3H), 6.79 (dd, *J* = 19.2, 14.2 Hz, 1H), 6.53 (d, *J* = 15.8 Hz, 1H), 6.45 (d, *J* = 6.0 Hz, 2H), 4.26 (d, *J* = 12.8 Hz, 2H), 4.04 (s, 2H), 3.71 (s, 3H), 3.15 (dd, *J* = 29.8, 18.0 Hz, 3H), 2.13 (d, *J* = 10.2 Hz, 2H), 1.61 (d, *J* = 10.2 Hz, 2H). | 620.2 |
| 253 | Methyl (E)-3'-cyano-5'-(3 -hydroxy-4-metho xyphenyl)-2'-(4-(( 4-(3-(hydroxyami no)-3-oxoprop-1-en-1-yl) benzyl)a mino)piperidin-1-yl)-[3,4'-bipyridine]-6-carboxylate formate | 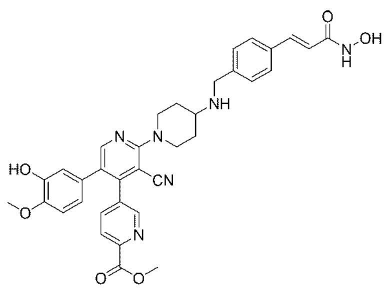 | $^1$H NMR (400 MHz, DMSO-$d_6$) δ 10.73 (s, 1H), 8.98 (s, 1H), 8.57 (d, *J* = 2.0 Hz, 1H), 8.43 (s, 1H), 8.25 (s, 1H), 8.06 (d, *J* = 8.0 Hz, 1H), 7.95 (dd, *J* = 8.0, 2.2 Hz, 1H), 7.53 (d, *J* = 8.0 Hz, 2H), 7.50 - 7.38 (m, 3H), 6.79 (d, *J* = 8.1 Hz, 1H), 6.50 - 6.40 (m, 3H), 4.21 (d, *J* = 13.0 Hz, 2H), 3.88 (s, 5H), 3.70 (s, 3H), 3.17 (t, *J* = 12.0 Hz, 2H), 2.84 (s, 1H), 2.03 (d, *J* = 12.4 Hz, 2H), 1.53 (dd, *J* = 7.0, 9.2 Hz, 2H). | 635.3 |

(continued)

| Example | Chemical name | Structure | $^1$H NMR | MS(M+H)$^+$ |
|---|---|---|---|---|
| 254 | (E)-3-(4-(((1-(6-C yano-5'-(3-hydrox y-4-methoxyphen yl)-3'-methyl-[3,4' -bipyridin]-2'-yl)p iperidin-4-yl)amin o)methyl)phenyl)-N-hydroxyacryla mide formate | 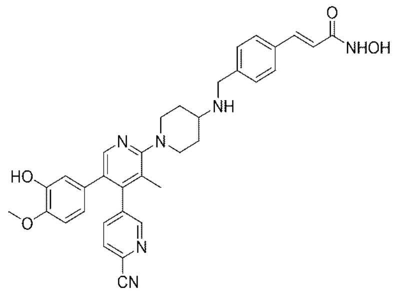 | $^1$H NMR (400 MHz, DMSO-$d_6$) δ 10.95 (s, 1H), 9.07 (s, 1H), 8.94 (s, 1H), 8.48 (d, $J$ = 2.0 Hz, 1H), 8.16 (s, 1H), 8.05 (d, $J$ = 8.0 Hz, 1H), 7.85 (dd, $J$ = 8.0, 2.0 Hz, 1H), 7.62 (q, $J$ = 8.0 Hz, 4H), 7.48 (d, $J$ = 15.9 Hz, 1H), 6.76 (d, $J$ = 8.2 Hz, 1H), 6.52 (d, $J$ = 15.8 Hz, 1H), 6.46 - 6.35 (m, 2H), 4.22 (s, 2H), 3.70 (d, $J$ = 5.2 Hz, 3H), 3.64 - 3.52 (m, 2H), 3.20 (s, 1H), 2.85 (t, $J$ = 12.2 Hz, 2H), 2.21 (d, $J$ = 11.6 Hz, 2H), 2.00 (s, 3H), 1.81 (d, $J$ = 11.6 Hz, 2H). | 591.3 |
| 255 | (E)-3-(4-(((1-(3',6 -Dicyano-5'-(2-flu oro-5-hydroxy-4-methoxyphenyl)-[ 3,4'-bipyridin]-2'-yl)piperidin-4-yl) amino)methyl)phe nyl)-N-hydroxyac rylamide formate | 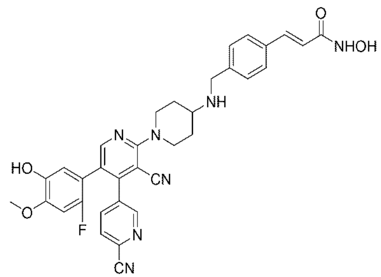 | $^1$H NMR (400 MHz, Methanol-$d_4$) δ 8.59 (s, 1H), 8.44 (s, 1H), 8.25 (s, 1H), 7.92 (dd, $J$ = 8.0, 2.2 Hz, 1H), 7.89 - 7.83 (m, 1H), 7.68 (d, $J$ = 8.0 Hz, 2H), 7.64 - 7.52 (m, 3H), 6.69 - 6.48 (m, 3H), 4.58 - 4.50 (m, 2H), 4.33 (s, 2H), 3.80 (s, 3H), 3.54 (s, 1H), 3.21 (t, $J$ = 12.8 Hz, 2H), 2.35 (d, $J$ = 12.0 Hz, 2H), 1.88 (tt, $J$ = 12.8, 7.2 Hz, 2H). | 620.2 |
| 256 | (E)-3-(4-(((1-(3-C yano-4-(5-cyanop yrazin-2-yl)-5-(3-hydroxy-4-metho xyphenyl)pyridin-2-yl)piperidin-4-y 1)amino)methyl)p henyl)-N-hydroxy acrylamide formate | 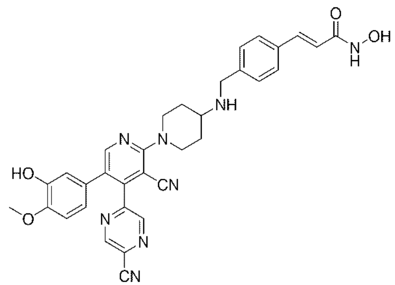 | $^1$H NMR (400 MHz, DMSO-$d_6$) δ 10.72 (s, 1H), 9.41 (d, $J$ = 1.6 Hz, 1H), 9.09 (s, 1H), 8.71 (d, $J$ = 1.6 Hz, 1H), 8.55 (s, 1H), 8.15 (s, 1H), 7.53 (d, $J$ = 7.6 Hz, 2H), 7.44 (t, $J$ = 8.4 Hz, 3H), 6.82 (d, $J$ = 8.4 Hz, 1H), 6.48 - 6.35 (m, 3H), 4.21 (d, $J$ = 13.2 Hz, 2H), 3.86 (s, 2H), 3.72 (s, 3H), 3.21 (t, $J$= 12.1 Hz, 2H), 2.81 (s, 1H), 2.02 (d, $J$= 11.6 Hz, 2H), 1.49 (q, $J$ = 10.8, 10.2 Hz, 2H). | 603.2 |

(continued)

| Example | Chemical name | Structure | $^1$H NMR | MS(M+H)$^+$ |
|---|---|---|---|---|
| 257 | (E)-3-(4-(((1-(3',6 -Dicyano-5'-(3-hy droxy-4-methoxy phenyl)-[3,4'-bipy ridin]-2'-yl) piperi din-4-yl)amino)m ethyl)-2-methoxy phenyl)-N-hydrox yacrylamide formate | 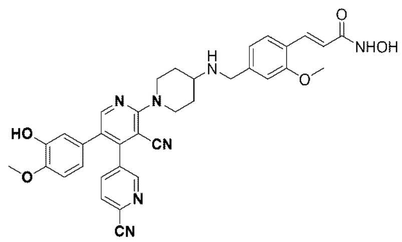 | $^1$H NMR (400 MHz, Methanol-$d_4$) δppm 8.62 (s, 1H),8.58 (dd, $J$ = 2.2, 0.9 Hz, 1H), 8.48 (s, 1H), 7.98-7.78 (m, 3H), 7.62 (d, $J$ = 7.8 Hz, 1H), 7.28-7.21 (m, 1H), 7.13 (d, $J$ = 7.8 Hz, 1H), 6.83 (d, $J$ = 8.0 Hz, 1H), 6.62 (d, $J$ = 16.0 Hz, 1H), 6.54-6.45 (m, 2H), 4.50 (d, $J$= 13.4 Hz, 2H), 4.33 (s, 2H), 3.98 (s, 3H), 3.81 (s, 3H), 3.54 (t, $J$ = 12.0 Hz, 1H), 3.21 (t, $J$ = 12.8 Hz, 2H), 2.36 (d, $J$ = 11.4 Hz, 2H), 1.90 (d, $J$ = 11.4 Hz, 2H). | 632.3 |
| 258 | (E)-3-(4-(((1-(3',6 -Dicyano-5'-(3-hy droxy-4-methoxy phenyl)-[3,4'-bipy ridin]-2'-yl) piperi din-4-yl)amino)m ethyl)-2-fluorophe nyl)-N-hydroxyac rylamide formate | 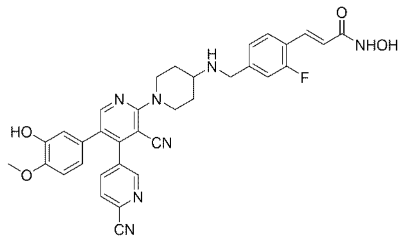 | $^1$H NMR (400 MHz, DMSO-$d_6$) δ 10.89 (s, 1H), 9.12 (s, 1H), 9.04 (s, 1H), 8.65 (d, $J$ = 2.2 Hz, 1H), 8.47 (s, 1H), 8.13 (d, $J$ = 8.0 Hz, 1H), 8.05 (dd, $J$ = 8.2, 2.2 Hz, 1H), 7.72 (t, $J$ = 8.1 Hz, 1H), 7.52 (t, $J$ = 13.6 Hz, 2H), 7.43 (d, $J$ = 7.8 Hz, 1H), 6.80 (d, $J$ = 8.9 Hz, 1H), 6.61 (d, $J$ = 16.0 Hz, 1H), 6.47 - 6.40 (m, 2H), 4.31 (d, $J$ = 13.2 Hz, 2H), 4.23 (s, 2H), 3.71 (s, 3H), 3.54 (t, $J$ = 11.0 Hz, 1H), 3.14 (t, $J$ = 12.6 Hz, 2H), 2.22 (s, 2H), 1.75 (s, 2H). | 620.2 |
| 259 | (E)-3'-Cyano-5'-(3 -hydroxy-4-metho xyphenyl)-2'-(4-(( 4-(3-(hydroxyami no)-3-oxoprop-1-en-1-yl) benzyl)a mino)piperidin-1-yl)-[3,4'-bipyridin e]-6-carboxamide formate | 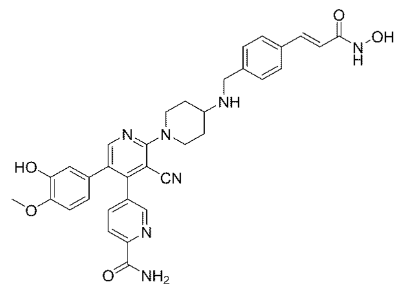 | $^1$H NMR (400 MHz, DMSO-$d_6$) δ 10.85 (s, 1H), 9.51 (s, 2H), 9.10 (s, 1H), 8.49 (d, $J$ = 2.2 Hz, 1H), 8.46 (s, 1H), 8.18 (d, $J$ = 2.4 Hz, 1H), 8.04 (d, $J$ = 8.0 Hz, 1H), 7.95 (dd, $J$ = 8.0, 2.2 Hz, 1H), 7.72 (s, 1H), 7.69 - 7.57 (m, 4H), 7.48 (d, $J$ = 15.8 Hz, 1H), 6.80 (d, $J$ = 8.2 Hz, 1H), 6.59 - 6.48 (m, 2H), 6.45 (dd, $J$ = 8.2, 2.2 Hz, 1H), 4.34 (d, $J$ = 12.8 Hz, 2H), 4.23 (d, $J$ = 6.2 Hz, 2H), 3.70 (s, 3H), 3.37 (s, 1H), 3.13 (t, $J$ = 12.6 Hz, 2H), 2.29 (d, J = 11.8 Hz, 2H), 1.95 - 1.76 (m, 2H). | 620.3 |
| 260 | (Z)-3-(4-(((1-(3',6 -Dicyano-5'-(3-hy droxy-4-methoxy phenyl)-[3,4'-bipy ridin]-2'-yl) piperi din-4-yl)amino)m ethyl) phenyl)-2-fl uoro-N-hydroxya crylamide formate | 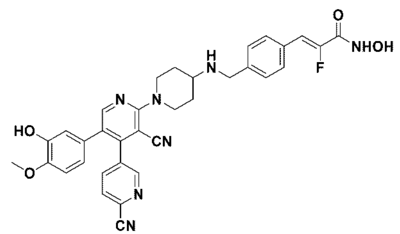 | $^1$H NMR (400 MHz, Methanol-$d_4$) δppm 8.57 (d, $J$ = 2.0 Hz, 1H), 8.50 (s, 1H),8.46 (d, $J$ = 2.4 Hz, 1H), 7.98-7.84 (m, 2H), 7.71 (d, $J$ = 8.2 Hz, 1H), 7.62 (d, $J$ = 8.0 Hz, 1H), 7.53 (d, $J$ = 8.0 Hz, 1H), 7.45 (d, $J$ = 8.0 Hz, 1H), 6.82 (dd, $J$ = 8.2 Hz, 2H), 6.55-6.45 (m, 2H), 4.50- 4.42 (m, 2H), 4.18 (d, $J$= 7.4 Hz, 2H),3.28 (s, 1H), 3.81 (s, 3H), 3.24-3.14 (m, 2H), 2.27 (s, 2H), 1.86-1.73 (m, 2H). | 620.2 |

(continued)

| Example | Chemical name | Structure | $^1$H NMR | MS(M+H)$^+$ |
|---|---|---|---|---|
| 261 | (E)-3-(4-(((1-(6-Cyano-3'-(cyanomethyl)-5'-(3-hydroxy-4-methoxyphenyl)-[3,4'-bipyridin]-2'-yl)piperidin-4-yl)amino)methyl)phenyl)-N-hydroxyacrylamide formate | 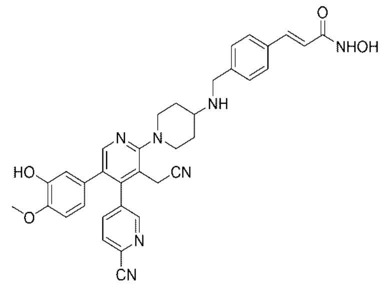 | $^1$H NMR (400 MHz, DMSO-$d_6$) δ 10.72 (s, 1H), 8.97 (s, 1H), 8.52 (d, $J$ = 2.0 Hz, 1H), 8.33 (s, 1H), 8.24 (s, 1H), 8.11 (d, $J$ = 8.0 Hz, 1H), 7.95 (dd, $J$= 8.0, 2.0 Hz, 1H), 7.51 (d, $J$= 7.6 Hz, 2H), 7.43 (t, $J$ = 10.4 Hz, 3H), 6.77 (d, $J$ = 8.4 Hz, 1H), 6.43 (d, $J$ = 10.0 Hz, 3H), 3.80 (d, $J$ = 5.6 Hz, 4H), 3.70 (s, 3H), 3.36 (s, 2H), 2.88 (t, $J$ = 11.6 Hz, 2H), 2.64 (t, $J$ = 12.4 Hz, 1H), 1.99 (d, $J$ = 12.4 Hz, 2H), 1.56 (d, $J$ = 11.6 Hz, 2H). | 616.3 |
| 262 | (E)-3-(4-(((1-(6-Cyano-5'-(2-fluoro-5-hydroxy-4-methoxyphenyl)-3'-methyl-[3,4'-bipyridin]-2'-yl)piperidin-4-yl)amino)methyl)phenyl)-N-hydroxyacrylamide formate | 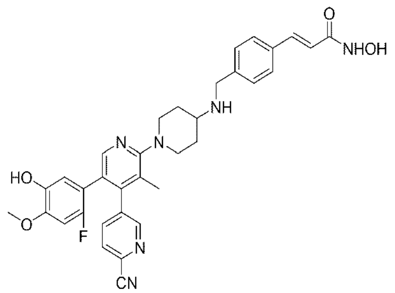 | $^1$H NMR (400 MHz, DMSO-$d_6$) δ 10.72 (s, 1H), 8.98 (s, 1H), 8.48 (d, J = 2.0 Hz, 1H), 8.28 (s, 1H), 8.10 (s, 1H), 8.02 (d, J = 8.0 Hz, 1H), 7.82 (dd, J = 8.0, 2.2 Hz, 1H), 7.52 (d, J = 7.8 Hz, 2H), 7.48 - 7.36 (m, 3H), 6.65 (d, J = 11.4 Hz, 1H), 6.57 (d, J = 7.6 Hz, 1H), 6.44 (d, J = 15.8 Hz, 1H), 3.83 (s, 2H), 3.70 (s, 3H), 3.51 - 3.44 (m, 2H), 2.82 (t, J = 11.8 Hz, 2H), 2.64 (d, J = 9.6 Hz, 1H), 2.00-1.80 (m, 5H), 1.49 (q, J = 11.0 Hz, 2H). | 609.3 |

**Examples 263-266** were prepared according to the synthetic method of Example **88**, and the structure and characterization data are as follows.

| Example | Chemical name | Structure | $^1$H NMR | MS(M+H)$^+$ |
|---|---|---|---|---|
| 263 | (E)-3-(4-(((1-(6-Cyano-5'-(3-hydroxy-4-methoxyphenyl)-3'-methoxy-[3,4'-bipyridin]-2'-yl)piperidin-4-yl)amino)methyl)phenyl)-N-hydroxyacrylamide formate | 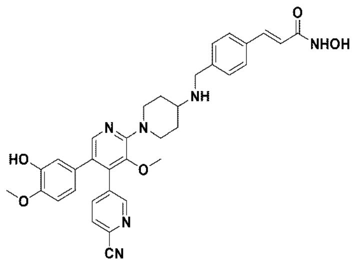 | $^1$H NMR (400 MHz, DMSO-$d_6$) δ 10.74 (s, 1H), 8.95 (s, 1H), 8.56 - 8.41 (m, 1H), 8.16 (s, 1H), 8.08 - 7.94 (m, 2H), 7.83 (dd, *J* = 8.0, 2.0 Hz, 1H), 7.55 (d, *J* = 8.0 Hz, 2H), 7.50 - 7.39 (m, 3H), 6.78 (d, *J* = 8.0 Hz, 1H), 6.52 - 6.36 (m, 3H), 4.05 (d, *J* = 12.8 Hz, 2H), 3.93 (s, 2H), 3.71 (s, 3H), 3.45 (s, 3H), 2.88 (t, *J* = 12.4 Hz, 3H), 2.04 (d, *J* = 12.0 Hz, 2H), 1.55 (q, *J* = 11.2 Hz, 2H). | 607.3 |
| 264 | (E)-3-(4-(((1-(6-Cyano-5'-(3-hydroxy-4-methoxyphenyl)-3'-methoxy-[3,4'-bipyridin]-2'-yl)piperidin-4-yl)amino)methyl)-3-fluorophenyl)-N-hydroxyacrylamide formate | 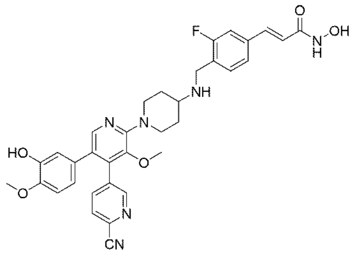 | $^1$H NMR (400 MHz, DMSO-$d_6$) δ 10.80 (s, 1H), 9.09 (s, 1H), 8.95 (s, 1H), 8.50 (d, *J* = 2.0 Hz, 1H), 8.13 (s, 1H), 8.02 (d, *J* = 8.4 Hz, 2H), 7.84 (dd, *J* = 8.0, 2.0 Hz, 1H), 7.60 (s, 1H), 7.45 (d, *J* = 12.0 Hz, 2H), 6.78 (d, *J* = 8.0 Hz, 1H), 6.52 (d, *J* = 15.6 Hz, 1H), 6.46 - 6.35 (m, 2H), 4.09 (d, *J* = 12.4 Hz, 4H), 3.71 (s, 3H), 3.45 (s, 3H), 3.36 (d, *J* = 11.2 Hz, 1H), 2.90 (t, *J* = 12.4 Hz, 2H), 2.10 (s, 2H), 1.63 (s, 2H). | 625.3 |
| 265 | (E)-3-(4-(((1-(6-Cyano-5'-(2-fluoro-5-hydroxy-4-methoxyphenyl)-3'-methoxy-[3,4'-bipyridin]-2'-yl)piperidin-4-yl)amino)methyl)phenyl)-N-hydroxyacrylamide formate | 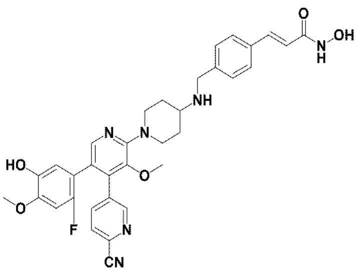 | $^1$H NMR (400 MHz, DMSO-$d_6$) δ 10.79 (s, 1H), 9.03 (s, 1H), 8.51 (d, *J* = 2.0 Hz, 1H), 8.25 (s, 1H), 8.02 (t, *J* = 4.0 Hz, 2H), 7.80 (dd, *J* = 8.0, 2.2 Hz, 1H), 7.62 (d, *J* = 8.0 Hz, 2H), 7.55 (d, *J* = 7.8 Hz, 2H), 7.47 (d, *J* = 5.8 Hz, 1H), 6.68 (d, *J* = 11.4 Hz, 1H), 6.61 (d, *J* = 7.6 Hz, 1H), 6.50 (d, *J* = 5.8 Hz, 1H), 4.19-4.01 (m, 4H), 3.72 (s, 3H), 3.46 (s, 3H), 3.27 (s, 1H), 2.92 (t, *J* = 12.4 Hz, 2H), 2.17 (d, *J* = 11.6 Hz, 2H), 1.8 *(d, J*= 10.2 Hz, 2H). | 625.2 |
| 266 | (E)-3-(5-(((1-(6-Cyano-5'-(3-hydroxy-4-methoxyphenyl)-3'-methoxy-[3,4'-bipyridin]-2'-yl)piperidin-4-yl)amino)methyl)pyridin-2-yl)-N-hydroxyacrylamide formate | 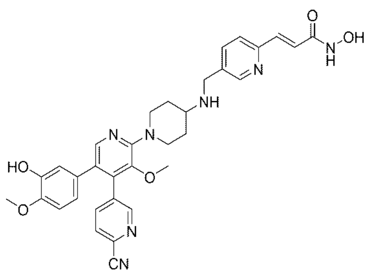 | $^1$H NMR (400 MHz, DMSO-$d_6$) δ 10.93 (s, 1H), 9.14 (s, 1H), 8.96 (s, 1H), 8.69 (s, 1H), 8.50 (d, *J* = 2.0 Hz, 1H), 8.03 (d, *J* = 9.2 Hz, 2H), 7.96 (s, 1H), 7.84 (dd, *J* = 8.0, 2.0 Hz, 1H), 7.63 (d, *J* = 7.6 Hz, 1H), 7.50 (d, *J* = 15.2 Hz, 1H), 6.95 (d, *J* = 15.2 Hz, 1H), 6.78 (d, *J* = 8.4 Hz, 1H), 6.48 - 6.37 (m, 2H), 4.13 (d, *J* = 12.5 Hz, 4H), 3.71 (s, 3H), 3.46 (s, 3H), 3.32 (s, 1H), 2.91 (t, *J* = 12.4 Hz, 2H), 2.14 (s, 2H), 1.70 (s, 2H). | 608.3 |

**Example 267**

(E)-3-(4-(((1-(3-Cyano-2-(6-cyanopyridin-3-yl)-3'-hydroay-4'-methoay-[1,1'-biphenyl]-4-yl)piperidin-4-yl)amino)methyl)phenyl)-N-hydroxyacrylamide formate was prepared according to the synthetic method of Example **84**, and the structure and characterization data are as follows:

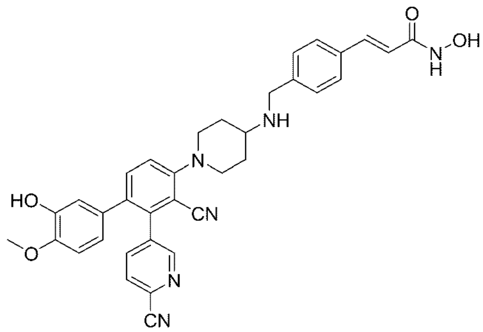

$^1$H NMR (400 MHz, DMSO-$d_6$) δ 10.78 (s, 1H), 9.03 (m, 2H), 8.58 (d, *J* = 2.0 Hz, 1H), 8.14 (s, 1H), 8.09 (d, *J* = 8.0 Hz, 1H), 7.99 (dd, *J* = 8.0, 2.1 Hz, 1H), 7.65 - 7.43 (m, 6H), 7.33 (d, *J* = 8.4 Hz, 1H), 6.77 (d, *J* = 8.4 Hz, 1H), 6.54 - 6.36 (m, 3H), 4.11 (s, 2H), 3.71 (s, 3H), 3.64 (d, *J* = 11.6 Hz, 2H), 3.07 (s, 1H), 2.94 (t, *J* = 11.6 Hz, 2H), 2.18 (d, *J* = 12.0 Hz, 2H), 1.73 (s, 2H).

ESI-MS(m/z) =601.3 [M+H]$^+$.

**Examples 268-271** were prepared according to the synthetic method of Example **76** (the separation method for the compounds: hydrochloride and formate were prepared by separation method 1 and 3, respectively), and the structure and characterization data are as follows:

| Example | Chemical name | Structure | $^1$H NMR | MS( M+H) + |
|---|---|---|---|---|
| 268 | (E)-3-(4-(((1-(6-Cyano-3'-fluoro-5'-(3-hydroxy-4-methoxyphe nyl)-[3,4'-bipyridin]-2'-yl)piperidin-4-yl)a mino) methyl)phenyl) -N-hydroxyacrylami de formate | 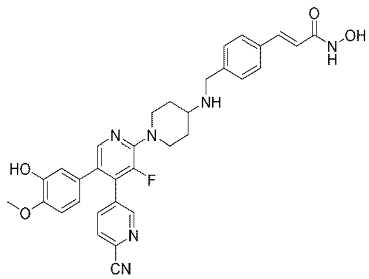 | $^1$H NMR (400 MHz, DMSO-$d_6$) δ ppm : 10.78 (s, 1H), 9.02 (s, 2H), 8.56 (s, 1H), 8.14 (d, J = 1.8 Hz, 1H), 8.13 - 8.02 (m, 2H), 7.95 (d, J = 8.2 Hz, 1H), 7.59 (d, J = 7.7 Hz, 2H), 7.55 - 7.38 (m, 3H), 6.86 - 6.76 (m, 1H), 6.54 - 6.38 (m, 3H), 4.05 (d, J = 9.0 Hz, 4H), 3.72 (d, J = 1.9 Hz, 3H), 3.08 (s, 1H), 2.99 (t, J = 12.5 Hz, 2H), 2.10 (d, J = 12.3 Hz, 2H), 1.62 (d, J = 12.4 Hz, 2H). | 595.2 |
| 269 | (E)-3-(4-(((1-(6-Cyano-3'-fluoro-5'-(3-hydroxy-4-methoxyphe nyl)-[3,4'-bipyridin]-2'-yl)piperidin-4-yl)a mino) methyl)-3-fluo rophenyl)-N-hydrox yacrylamide hydrochloride | 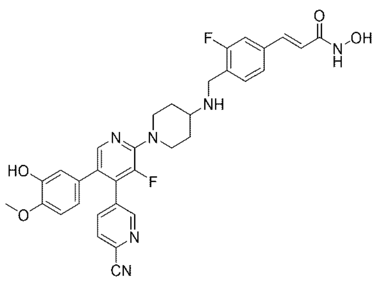 | $^1$H NMR (400 MHz, Methanol-$d_4$) δ ppm, 8.66 (s, 1H), 8.56 (s, 1H), 8.48 (s, 1H), 821-803 (m, 2H), 7.99-7.88 (m, 1H), 7.64 (t, *J* = 7.8 Hz, 1H), 7.47 (q, *J* = 9.4, 8.4 Hz, 2H), 6.81 (d, *J* = 8.4 Hz, 1H), 6.54 (d, *J* = 15.8 Hz, 1H), 6.44 (d, *J* = 6.6 Hz, 2H), 4.07 (d, *J* = 16.2 Hz, 4H), 3.72 (s, 3H), 3.20 (s, 1H), 3.00 (t, *J* = 12.4 Hz, 2H), 2.12 (d, *J* = 12.2 Hz, 2H), 1.65 (s, 2H). | 613.2 |

(continued)

| Example | Chemical name | Structure | $^1$H NMR | MS( M+H)+ |
|---|---|---|---|---|
| 270 | (E)-3-(4-(((1-(6-Cyano-3'-fluoro-5'-(2-fluoro-5-hydroxy-4-met hoxyphenyl)-[3,4'-bi pyridin]-2'-yl)piperid in-4-yl)amino)methy 1)phenyl)-N-hydroxy acrylamide formate | 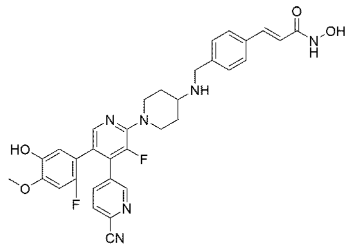 | $^1$H NMR (400 MHz, DMSO-$d_6$) δ ppm : 10.77 (s, 1H), 9.09 (s, 2H), 8.58 (s, 1H), 8.14 (s, 1H), 8.06 (t, J = 4.1 Hz, 2H), 7.92 (dd, J = 8.0, 2.1 Hz, 1H), 7.59 (d, J = 7.9 Hz, 2H), 7.55 - 7.39 (m, 2H), 6.67 (dd, J = 24.9, 9.5 Hz, 2H), 6.48 (d, J = 15.8 Hz, 1H), 4.21 - 3.96 (m, 4H), 3.72 (s, 3H), 3.04 (s, 1H), 3.00 (d, J = 12.3 Hz, 2H), 2.09 (d, J = 12.2 Hz, 2H), 1.61 (d, J = 12.2 Hz, 2H). | 613.2 |
| 271 | (Z)-3-(4-(((1-(6-Cyano-3'-fluoro-5'-(3-hydroxy-4-methoxyphe nyl)-[3,4'-bi-pyridin]-2'-yl)piperi-din-4-yl)a mino) methyl)-2-fluo ro-N-hydroxyacryla mide formate | 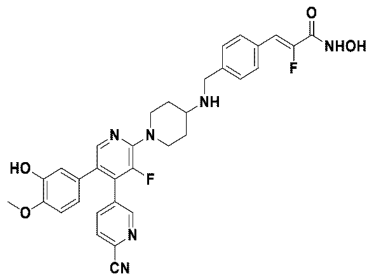 | $^1$H NMR (400 MHz, Methanol-$d_4$) δ ppm 8.53 (s, 1H), 8.49 (d, *J* = 2.2 Hz, 1H), 8.10 (d, *J* = 1.8 Hz, 1H), 7.84 (t, *J* = 1.4 Hz, 2H), 7.67 (dd, *J* = 34.6, 8.0 Hz, 2H), 7.50 (dd, *J* = 32.0, 7.8 Hz, 2H), 6.96-6.73 (m, 2H), 6.56-6.44 (m, 2H), 4.30-4.17 (m, 4H), 3.82 (s, 3H), 3.32 (s, 1H),3.05 *(t, J*= 12.4 Hz, 2H), 2.23 (d, *J* = 12.2 Hz, 2H), 1.77 (d, *J* = 10.6 Hz, 2H). | 613.2 |

**Example 272**

((E)-3-(4-(((1-(6-Cyano-5'-(3-hydroxy-4-methoxyphenyl)-[3,4'-bipyridin]-2'-yl)piperidin-4-yl)amino)methyl)p henyl)-N-hydroxyacrylamide formate was prepared according to the synthetic method of Example **80**, and the structure and characterization data are as follows:

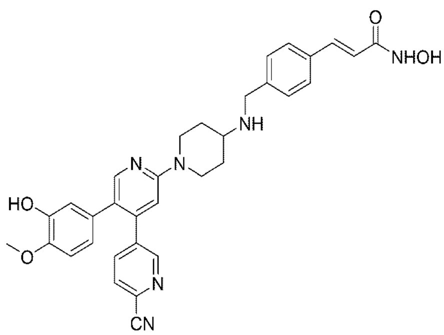

$^1$H NMR (400 MHz, Methanol-$d_4$) δ 8.52 (d, *J* = 1.5 Hz, 1H), 8.49 (s, 1H), 8.17 (d, *J* = 1.8 Hz, 1H), 7.81 (d, *J* = 1.5 Hz, 2H), 7.68 (d, *J* = 7.7 Hz, 2H), 7.64 - 7.52 (m, 3H), 7.01 (d, *J* = 4.1 Hz, 1H), 6.88 - 6.82 (m, 1H), 6.58 - 6.46 (m, 3H), 4.58 (d, *J* = 13.5 Hz, 2H), 4.32 (s, 2H), 3.83 (s, 3H), 3.51 (d, *J* = 17.8 Hz, 1H), 3.07 (t, *J* = 12.7 Hz, 2H), 2.29 (d, *J* = 12.0 Hz, 2H), 1.74 (d, *J* = 12.4 Hz, 2H).

ESI-MS(m/z) =577.3 $[M+H]^+$.

**Bioactivity test**

**1. Method for detecting LSD1 enzyme activity**

The effect of the compounds on LSD1 enzyme activity was examined by HTRF technique to assess the level of inhibition on LSD1 enzyme activity. Firstly, the master solution of 90 μM test compound (dissolved in DMSO) was diluted sequentially with DMSO in a 5-fold concentration gradient to obtain a total of 9 concentrations of the compound working solution 1 (90×). Then the 9 concentrations of working solution 1 were sequentially diluted by 30-fold concentration gradient, i.e., 2μL of working solution 1 was aspirated and added to 58μL Buffer, and mixed on a vortex mixer with sufficient shaking to obtain a total of 9 concentrations of the screening test compound working solution 2 (3×). In the 384-well shallow white plate, 2 μL of the compound working solution 2 (3×) was added to each well, followed by adding 2 uL of the 6× LSD1 (Activemotif, 31426) and 6× FAD (Sigma, F8384) pre-mixed (1:1) solution, mixed evenly, and incubated at room temperature for 15 minutes; 2 μL of the 3× H3K4me1 (Anaspec, AS-64355-025) substrate solution was added to each well, mixed evenly, and incubated at room temperature for 60 minutes; 2 μL of the termination solution (containing 5.4 mM 2-PCPA) was added to each well, mixed evenly, and incubated at room temperature for 15 minutes; 4 μL of the Eu-anti H3K4 (PerkinElmer, TRF0404-D) and allophycocyanin (Prozyme, PJ27S) pre-mixed antibody (1:1) solution was sequentially added to each well, mixed evenly, and incubated at room temperature for 60 minutes. The 384-well plate was placed on a multifunctional microplate reader to have the values read, with the excitation wavelength set to 337 nm, and readings were recorded at 620 nm and 665 nm. The data results were presented as the ratio of the 665 nm signal value to the 620 nm signal value in each well, which was calculated as: Ratio = $10^4$ × 665 nm signal value / 620 nm signal value. The inhibition rate was calculated through the following formula:

$$\% \text{ Inhibition rate} = [(\text{Ratio}_{\text{negative}} - \text{Ratio}_{\text{compound}})/(\text{Ratio}_{\text{negative}} - \text{Ratio}_{\text{Blank}})] \times 100$$

Note: Negative is the group without inhibitor; Blank is the group without enzyme.

$IC_{50}$ was calculated by fitting the inhibition rate by GraphPad Prism software, choosing log(inhibitor) vs. response-Variable Slope (four parameters).

**2. Method for detecting HDAC enzyme activity**

The effect of the compound on the enzymatic activity of pan-HDAC and 2 isoforms (HDAC1 and HDAC8) was examined by the HDAC-Glo I/II Assay and Screening System assay to assess the level of inhibition of HDAC protease activity and selectivity in each isoform. The proteins and detection reagents used were from the HDAC-Glo™ I/II Assay and Screening System (Promega). 1 mM compound was diluted with DMSO in a 5-fold gradient to create a series of 8 concentrations, and a certain volume was diluted 25 times with HDAC Buffer to obtain 4× working solution of the compound. In a 384-well plate, 5 μL of the 4× HDAC enzyme solution and 5 μL of the test compound (4×) were sequentially added to each well, mixed evenly, and incubated at room temperature for 30 minutes; 10 μL of 2× Developer reagent was sequentially added to each well, mixed evenly, and incubated at room temperature for 15-45 minutes; the 384-well plate was placed on a multifunctional microplate reader to read the values, and the Luminescence channel was selected and the Luminescence values (RLU) recorded. The inhibition rate was calculated through the following formula:

$$\% \text{ Inhibition rate} = \left[ \frac{RLU_{\text{Negative control}} - RLU_{\text{Test compoud}}}{RLU_{\text{Negative control}} - RLU_{Blank}} \right] *100$$

Note: Negative is the group without inhibitor; Blank is the group without enzyme.

$IC_{50}$ was calculated by fitting the inhibition rate by GraphPad Prism software, choosing log(inhibitor) vs. response-Variable Slope (four parameters).

**(Table I) Data for the Enzymatic Activity of the Compounds**

| Example | LSD1 $IC_{50}$(nM) | HDAC $IC_{50}$ (nM) | | |
|---|---|---|---|---|
| | | HDAC 1 | HDAC 8 | Pan-HDAC |
| 1 | 1.0 | 68.1 | 214.8 | 17.5 |
| 2 | 1.58 | 92.3 | 289 | 66.9 |
| 3 | 13.7 | N.T | N.T | 29 |

(continued)

| Example | LSD1 $IC_{50}$(nM) | HDAC $IC_{50}$ (nM) | | |
|---|---|---|---|---|
| | | HDAC 1 | HDAC 8 | Pan-HDAC |
| 4 | 11.9 | N.T | N.T | 12 |
| 5 | 35.2 | N.T | N.T | 15 |
| 6 | 254 | N.T | N.T | 134 |
| 7 | 36 | N.T | N.T | 41.3 |
| 8 | 18.6 | N.T | N.T | 21.7 |
| 9 | 1.82 | N.T | N.T | 25.3 |
| 10 | 75.4 | N.T | N.T | 39 |
| 11 | 74.5 | N.T | N.T | 116.5 |
| 12 | 0.92 | N.T | N.T | 7.31 |
| 13 | 1.95 | N.T | N.T | 3.32 |
| 15 | 1.45 | N.T | N.T | 27.5 |
| 16 | 33.2 | N.T | N.T | 4.23 |
| 17 | 23.8 | N.T | N.T | 21.6 |
| 19 | 0.86 | N.T | 221.1 | 173.9 |
| 20 | 16.9 | 311.3 | 445.5 | 96.5 |
| 21 | 0.05 | 400 | 275.1 | 119 |
| 22 | 0.31 | N.T | N.T | 144.4 |
| 23 | 0.23 | 198.1 | 320.2 | 74.4 |
| 24 | 0.41 | 8.28 | 117.7 | 5.83 |
| 25 | 0.23 | N.T | 128.9 | 6.66 |
| 26 | 0.12 | N.T | N.T | 35 |
| 27 | 1.38 | N.T | 185.7 | 134.2 |
| 28 | 0.64 | 607.8 | 2331 | 269.7 |
| 29 | 0.96 | 123.2 | 696.2 | 66.9 |
| 30 | 4.25 | 39.9 | 65.8 | 18.1 |
| 31 | 311 | 1.78 | 263.4 | 0.25 |
| 32 | 0.35 | N.T | 279.4 | 14 |
| 33 | 3.71 | 56.4 | 182.4 | 34.2 |
| 34 | 1.13 | 1063 | 353 | 551.8 |
| 35 | 4.63 | N.T | N.T | 266.1 |
| 36 | 1.18 | 118.2 | 97.7 | 75.1 |
| 37 | 1.95 | 4.7 | 93.6 | 21.2 |
| 39 | 0.15 | 335 | 207.3 | 166.6 |
| 40 | 8.25 | 108.3 | 391.6 | 54.4 |
| 41 | 0.13 | 1124 | 1368 | 1014 |
| 42 | 0.33 | 7.33 | 327.5 | 9.25 |
| 43 | 0.62 | 5.54 | 306.2 | 4.3 |
| 44 | 2.58 | 45.8 | 187.6 | 27.9 |

(continued)

| Example | LSD1 $IC_{50}$(nM) | HDAC $IC_{50}$ (nM) | | |
|---|---|---|---|---|
| | | HDAC 1 | HDAC 8 | Pan-HDAC |
| 45 | 0.36 | 70.6 | 156.4 | 32.1 |
| 50 | 4.33 | 117.5 | 169.5 | 70.7 |
| 52 | 47.8 | 15.9 | 140.2 | 4.22 |
| 54 | 0.62 | 58.2 | 125.4 | 34.4 |
| 56 | 91.5 | 641.8 | 892.6 | 467 |
| 57 | 0.26 | N.T | N.T | 72 |
| 58 | 0.1 | 47.4 | 135.9 | 31.9 |
| 62 | 0.49 | 17.3 | 3188 | 5.53 |
| 63 | 8.7 | 9.28 | 561.2 | 6.22 |
| 64 | 1.4 | 9.08 | 440.9 | 5.95 |
| 67 | 1.89 | N.T | N.T | 39 |
| 69 | >1000 | N.T | 198.4 | 30.1 |
| 70 | 1.18 | N.T | 26.5 | 76.6 |
| 71 | 4.61 | N.T | 124.6 | 51.8 |
| 72 | 8.42 | N.T | 159.5 | 110.5 |
| 73 | 21.2 | N.T | 151.8 | 185.7 |
| 74 | 2.26 | N.T | 207.1 | 23.1 |
| 75 | 129.2 | N.T | 211.8 | 76.6 |
| 76 | 10.7 | N.T | 157.4 | 1.59 |
| 80 | 0.78 | N.T | 164.6 | 14.9 |
| 81 | 140.1 | N.T | 695.3 | 1221 |
| 83 | 8.51 | N.T | 374.1 | 438 |
| 84 | 0.62 | N.T | 73.6 | 5.45 |
| 85 | 208.9 | N.T | 541.9 | 110.2 |
| 86 | 9.46 | N.T | 578 | 13.8 |
| 88 | 28.7 | 0.21 | 178.2 | 5.09 |
| 90 | 8.01 | 10.6 | 63.5 | 19.5 |
| 92 | 1.41 | N.T | 622.7 | 340.2 |
| 94 | 172.8 | N.T | 256.5 | 110.1 |
| 95 | 63.6 | N.T | 87.2 | 49 |
| 96 | 39.9 | N.T | 16.3 | 16.4 |
| 97 | 1.06 | 8.3 | 92.4 | 13.9 |
| 98 | 160 | N.T | 480.3 | 394.5 |
| 99 | 9.5 | N.T | 120.4 | 50.9 |
| 100 | 49.5 | N.T | 36 | 35.5 |
| 101 | 0.52 | 13.6 | 206.7 | 27.5 |
| 102 | 1.31 | 16.3 | 178.5 | 45.2 |
| 103 | 14.2 | N.T | 158.8 | 62.3 |

(continued)

| Example | LSD1 $IC_{50}$(nM) | HDAC $IC_{50}$ (nM) | | |
|---|---|---|---|---|
| | | HDAC 1 | HDAC 8 | Pan-HDAC |
| 104 | 0.71 | 5.3 | 70.1 | 10.7 |
| 105 | 1.24 | 120 | 185 | 114.1 |
| 106 | 2.62 | 3.81 | 13.1 | 4.85 |
| 107 | 1.29 | N.T | 50.6 | 378.2 |
| 108 | 2.3 | 25.8 | 93.1 | 26.6 |
| 109 | 4.19 | N.T | 391.4 | 657.1 |
| 110 | 1.05 | 156.4 | 137.3 | 57.3 |
| 111 | 1.56 | 83.1 | 357.6 | 65.3 |
| 112 | 2.66 | 26.3 | 81.4 | 26.9 |
| 113 | 2 | 17.3 | 72.7 | 25 |
| 114- | 0.97 | 177.7 | 571.3 | 175.3 |
| 115 | 0.16 | N.T | 37.1 | 20.6 |
| 116 | 1.82 | 5.59 | 212.3 | 12.7 |
| 117 | 0.36 | 0.59 | 13 | 0.8 |
| 118 | 0.58 | 0.44 | 30.3 | 0.75 |
| 119 | 1.98 | N.T | 245.8 | 33.6 |
| 120 | 0.33 | N.T | 1949 | 1314 |
| 121 | 0.35 | N.T | 17.2 | 404.1 |
| 123 | 0.74 | N.T | 39 | 7.34 |
| 124 | 1.63 | 3.95 | 178.7 | 7.69 |
| 125 | 0.74 | N.T | 23.4 | 226 |
| 126 | 1.11 | N.T | 132.3 | 197.3 |
| 127 | 1.2 | 5.82 | 422.8 | 12.4 |
| 128 | 0.39 | N.T | 222.7 | 5 |
| 129 | 1.47 | N.T | 321.9 | 18.2 |
| 130 | 1.89 | 0.77 | 219.9 | 6.13 |
| 131 | 1.31 | 0.22 | 46.6 | 1.57 |
| 132 | 10.8 | N.T | 171.3 | 3.86 |
| 133 | 22 | 1.45 | 139.5 | 11.78 |
| 134 | 13.6 | 0.32 | 46 | 3.55 |
| 135 | 3.16 | 17.26 | 1009 | 39.7 |
| 136 | 17.2 | N.T | 77.7 | 10.4 |
| 137 | 27.8 | 0.52 | 106.8 | 6.04 |
| 138 | 21.4 | N.T | 401.4 | 61 |
| 139 | 43.4 | 0.53 | 147 | 6.82 |
| 140 | 44.2 | N.T | 465.5 | 5.64 |
| 141 | 1.72 | N.T | 53 | 5.62 |
| 142 | 1.8 | 0.6 | 129 | 1.27 |

(continued)

| Example | LSD1 $IC_{50}$(nM) | HDAC $IC_{50}$ (nM) | | |
|---|---|---|---|---|
| | | HDAC 1 | HDAC 8 | Pan-HDAC |
| 143 | 0.1 | 1.25 | 111.1 | 1.7 |
| 144 | 1.07 | 0.21 | 91.6 | 0.86 |
| 145 | 3.35 | N.T | 74.9 | 5.23 |
| 147 | 153.4 | N.T | 1174 | 33.2 |
| 148 | 1.05 | 0.43 | 58.4 | 2.47 |
| 149 | 0.54 | 0.5 | 58.9 | 1.05 |
| 150 | 0.59 | 0.21 | 89.3 | 1.3 |
| 151 | 2.7 | N.T | 618.1 | 27.4 |
| 152 | 1.15 | N.T | 131.4 | 2.57 |
| 153 | 0.74 | N.T | 400.4 | 5.49 |
| 154 | 0.44 | N.T | 309.2 | 4.3 |
| 155 | 0.58 | N.T | 23.7 | 20 |
| 156 | 2.53 | N.T | 730.8 | 8.88 |
| 158 | 0.73 | N.T | 87.8 | 0.61 |
| 159 | 1.82 | N.T | 83.5 | 2.71 |
| 160 | 1.12 | N.T | 183.5 | 1.48 |
| 161 | 0.58 | N.T | 303.8 | 6.83 |
| 162 | 0.4 | N.T | 209.2 | 3.34 |
| 164 | 0.44 | N.T | 332.5 | 38.1 |
| 165 | 1.35 | N.T | 144.8 | 7.31 |
| 166 | 15.1 | N.T | 64 | 2.27 |
| 167 | 0.37 | N.T | 823.5 | 21.9 |
| 168 | 1.34 | N.T | 249.8 | 7.35 |
| 169 | 0.42 | N.T | 1400 | 51.7 |
| 170 | 0.43 | N.T | 62.7 | 2 |
| 171 | 0.55 | N.T | 53.1 | 1.68 |
| 172 | 0.74 | N.T | 327.4 | 162.4 |
| 173 | 0.82 | N.T | 251.9 | 114 |
| 176 | 0.69 | N.T | 507.8 | 38.2 |
| 177 | 963 | N.T | 580.5 | 40.2 |
| 178 | 0.65 | N.T | 1713 | 588.3 |
| 179 | 100.3 | 1.74 | 374.5 | 3.1 |
| 180 | 0.11 | N.T | 1235 | 23.4 |
| 181 | 0.13 | 0.08 | 75.1 | 2.11 |
| 182 | 0.09 | 1.3 | 30.1 | 3.02 |
| 183 | 0.09 | N.T | 3723 | 969.9 |
| 184 | 0.09 | 1.69 | 45.2 | 6.58 |
| 185 | 3.9 | N.T | 2163 | 60.1 |

(continued)

| **Example** | LSD1 $IC_{50}$(nM) | HDAC $IC_{50}$ (nM) | | |
|---|---|---|---|---|
| | | HDAC 1 | HDAC 8 | Pan-HDAC |
| 186 | 10.2 | N.T | 50.9 | 11.9 |
| 187 | 4.11 | 1.98 | 15.5 | 3.71 |
| 188 | 0.3 | 3.06 | 198.4 | 5.07 |
| 189 | 4.11 | 13.4 | 129.9 | 7.8 |
| 190 | 2.85 | N.T | 336 | 5.96 |
| 191 | 2.24 | 0.54 | 37.5 | 2.17 |
| 192 | 0.45 | 1.49 | 64.9 | 2.75 |
| 193 | 1.71 | N.T | 296.9 | 8.36 |
| 194 | 1.09 | N.T | 542.7 | 7.27 |
| 195 | 4.12 | N.T | 172.1 | 11.8 |
| 196 | 6.68 | N.T | 26.5 | 5.69 |
| 197 | 0.49 | N.T | 30.1 | 7.48 |
| 198 | 0.40 | 4.85 | 67.2 | 6.35 |
| 199 | 0.54 | 1.92 | 33.9 | 2.77 |
| 200 | 0.45 | 2.95 | 126.7 | 4.18 |
| 201 | 0.22 | 4.81 | 438.5 | 7.71 |
| 202 | 74.2 | 0.88 | 9.03 | 1.85 |
| 203 | 1.03 | 1.58 | 25.3 | 1.39 |
| 204 | 1.58 | N.T | 26.2 | 2.37 |
| 205 | 1.91 | 3.08 | 100.5 | 4.37 |
| 206 | 2.03 | 32.9 | 32.5 | 11.5 |
| 207 | 6.65 | 2.51 | 170.4 | 12.4 |
| 208 | 0.73 | 1.74 | 17.2 | 1.13 |
| 209 | 0.6 | N.T | 3.09 | 163 |
| 210 | 1.13 | 4.44 | 456 | 2.5 |
| 211 | 0.31 | N.T | 83.5 | 2.04 |
| 212 | 0.04 | N.T | 41.5 | 6.55 |
| 213 | 2.12 | N.T | N.T | 19.7 |
| 214 | 4.98 | N.T | N.T | 142.9 |
| 215 | 0.39 | N.T | N.T | 68 |
| 216 | 0.15 | N.T | N.T | 3.57 |
| 217 | 13.6 | 0.73 | N.T | 0.25 |
| 218 | 10.2 | 1.77 | N.T | 1.01 |
| 219 | 0.21 | N.T | N.T | 9.28 |
| 220 | 0.42 | N.T | N.T | 937 |
| 221 | 17.1 | N.T | N.T | 2.2 |
| 222 | 0.03 | N.T | N.T | 11.5 |
| 223 | 1.01 | N.T | N.T | 4.37 |

(continued)

| Example | LSD1 $IC_{50}$(nM) | HDAC $IC_{50}$ (nM) | | |
|---|---|---|---|---|
| | | HDAC 1 | HDAC 8 | Pan-HDAC |
| 224 | 0.19 | N.T | N.T | 10.6 |
| 225 | 0.35 | N.T | N.T | 3.43 |
| 226 | 0.02 | N.T | N.T | 24.8 |
| 227 | 0.64 | 9.71 | N.T | 11.3 |
| 228 | 0.90 | N.T | N.T | 24.3 |
| 229 | 0.88 | N.T | N.T | 30.8 |
| 230 | 1.45 | N.T | N.T | 10.3 |
| 231 | 0.07 | N.T | 140 | 82.4 |
| 232 | 45.01 | N.T | N.T | 114 |
| 233 | 1.76 | 0.43 | 42.1 | 2.23 |
| 234 | 1.08 | N.T | 51.1 | 13.3 |
| 235 | 2.38 | N.T | N.T | 8.01 |
| 236 | 0.59 | N.T | N.T | 37 |
| 237 | 1.75 | 1.29 | N.T | 3.37 |
| 238 | 3.48 | 1.62 | N.T | 4.05 |
| 239 | 2.36 | N.T | N.T | 10.2 |
| 240 | 0.82 | 1.85 | N.T | 4.73 |
| 241 | 2.95 | 1.86 | N.T | 7.81 |
| 242 | 2.44 | N.T | N.T | 16 |
| 243 | 13.6 | 0.6 | N.T | 1.74 |
| 244 | 0.98 | 0.51 | N.T | 1.54 |
| 245 | 24.7 | 0.64 | N.T | 3.2 |
| 246 | 0.93 | N.T | N.T | 5.38 |
| 247 | 3.49 | 0.84 | N.T | 0.87 |
| 248 | 0.81 | 0.61 | N.T | 0.62 |
| 249 | 1.1 | N.T | N.T | 0.86 |
| 250 | 17.0 | N.T | N.T | 1.08 |
| 251 | 245 | N.T | N.T | 0.59 |
| 252 | 3.24 | N.T | N.T | 1.03 |
| 253 | 342 | N.T | N.T | 0.3 |
| 254 | 35.4 | N.T | N.T | 0.41 |
| 255 | 1.54 | N.T | N.T | 0.68 |
| 256 | 28.8 | N.T | N.T | 0.26 |
| 257 | 7.93 | N.T | N.T | 0.59 |
| 258 | 1.41 | N.T | N.T | 0.30 |
| 259 | 81.2 | N.T | N.T | 0.25 |
| 260 | 2.37 | N.T | N.T | 0.77 |
| 261 | 3.18 | N.T | N.T | 1.42 |

(continued)

| Example | LSD1 $IC_{50}$(nM) | HDAC $IC_{50}$ (nM) | | |
|---|---|---|---|---|
| | | HDAC 1 | HDAC 8 | Pan-HDAC |
| 262 | 7.04 | N.T | N.T | 0.61 |
| 263 | 16.8 | N.T | N.T | 0.34 |
| 264 | 25.4 | N.T | N.T | 0.89 |
| 265 | 10.2 | N.T | N.T | 0.45 |
| 266 | 24.2 | N.T | N.T | 0.16 |
| 267 | 7.36 | N.T | N.T | 1.93 |
| 268 | 147 | N.T | N.T | 0.27 |
| 269 | 193 | N.T | N.T | 0.93 |
| 270 | 24.8 | N.T | N.T | 0.32 |
| 271 | 35.1 | N.T | N.T | 0.83 |
| 272 | 25.2 | N.T | N.T | 0.30 |
| **CC-90011** | 1.1 | > 10000 | > 10000 | > 10000 |
| **SAHA (Vorinostat)** | >1000 | 51.8 | N.T | 38.6 |
| Note: N.T means untested. | | | | |

The data presented in Table I shows that the compounds of the present disclosure has a potent inhibitory effect on both LSD1 and HDAC enzymes.

The structure of SAHA is:

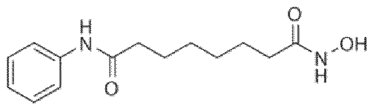

,

and it is brought from Selleck company;

The structure of CC-90011 is :

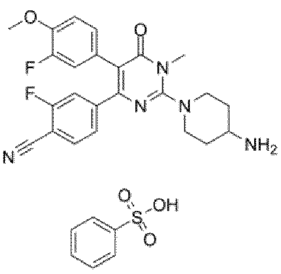

,and it is synthesized according to the literature methods (Kanouni, T., et al, J. Med. Chem., 2020, 63, 14522-14529).

### 3. Detecting method for screening the *in vitro* H1417 cell proliferation

The inhibitory effect of the compounds on cell proliferation was assessed by detecting the number of viable cells by CellTiter-Glo® reagent. NCI-H1417 cells in logarithmic growth phase were collected and inoculated into transparent-bottom 96-well plates with 100 μL per well and a density of 7 × $10^3$ cells/well, and incubated at 37°C, 5% $CO_2$ overnight; the compounds were sequentially diluted 5-fold with DMSO to obtain 8 concentrations of gradient dilutions, and then diluted with BEGM (10% FBS) cell culture medium to obtain the compound working solution (2 ×), which was added into the cell supernatant at 100 μL per well, and the incubation was continued for 7 days at 37°C, 5% $CO_2$. The plate was removed and allowed to equilibrate at room temperature (25°C) along with the CellTiter-Glo® mixing reagent for approximately 10-30

minutes. After centrifugation, 100 μL of the culture medium was carefully aspirated from each well, followed by adding 85 μL of CellTiter-Glo® reagent. The cells were thoroughly mixed with the CellTiter-Glo® mixing reagent using a microplate shaker for 2 min and then incubated at room temperature for 10 min. The 96-well plate was subsequently placed on a multifunctional microplate reader to record the Luminescence values (RLU).

The inhibition rate is calculated by the following

$$\%\ \text{Inhibition rate} = \left[\frac{RLU_{\text{Negative control}} - RLU_{\text{Test compoud}}}{RLU_{\text{Negative control}} - RLU_{Blank}}\right] * 100$$

Note: Negative is the group without inhibitors; Blank is the group without cells.

$IC_{50}$ was calculated by fitting the inhibition rate by GraphPad Prism software.

### 4. Detecting method for screening the *in vitro* NCI-H69 cell proliferation

The inhibitory effect of the compounds on cell proliferation was assessed by detecting the number of viable cells by CellTiter-Glo® reagent. NCI-H69 cells in logarithmic growth phase were collected and inoculated into transparent bottom 96-well plates at 100 μL per well and a density $1\times10^3$ cells/well, and incubated at 37°C, 5% $CO_2$ overnight; the compounds were sequentially 5-fold diluted with DMSO to obtain 8 concentrations of gradient dilutions, and then diluted with RPMI-640 (20% FBS) cell culture medium to obtain the compounds working solution respectively (2×), which was added into the cell supernatant at 100μL per well, and continued to incubate at 37°C, 5% $CO_2$ for 7 days. The plate was removed and allowed to equilibrate at room temperature (25°C) along with the CellTiter-Glo® mixing reagent for approximately 10-30 minutes; the plate was centrifuged at 1200 rpm for 5 minutes, after which 120 μL of the culture medium was carefully aspirated from each well, and then 60 μL of CellTiter-Glo® reagent was added. The cells were thoroughly mixed with the CellTiter-Glo® mixing reagent using a formula: microplate shaker for 2 min and then incubated at room temperature for 10 min. The 96-well plate was subsequently placed on a multifunctional microplate reader to record the Luminescence values (RLU).

The inhibition rate is calculated by the following formula:

$$\%\ \text{Inhibition rate} = \left[\frac{RLU_{\text{Negative control}} - RLU_{\text{Test compoud}}}{RLU_{\text{Negative control}} - RLU_{Blank}}\right] * 100$$

Note: Negative is the group without inhibitors; Blank is the group without cells.

$IC_{50}$ was calculated by fitting the inhibition rate by GraphPad Prism software.

### 5. Detecting method for screening the *in vitro* DU145 cell proliferation

The inhibitory effect of the compounds on cell proliferation was assessed by detecting the number of viable cells by CellTiter-Glo® reagent. DU145 cells in logarithmic growth phase were collected and inoculated into transparent bottom 96-well plates with 100μL per well and a density of $1.2\times10^3$ cells/well, and incubated at 37°C, 5% $CO_2$ overnight; the compounds were diluted 5-fold with DMSO in sequence to obtain 8 concentrations of gradient dilutions, and then diluted with RPMI-640 (10% FBS) cell culture medium to obtain the compound working solution respectively (2×), which was added into the cell supernatant at 100 μL per well, and continued to incubate for 6 days at 37°C, 5% $CO_2$. The plate was removed and allowed to equilibrate at room temperature (25°C) along with the CellTiter-Glo® mixing reagent for approximately 10-30 minutes; 120 μL of the culture medium was carefully aspirated from each well, followed by adding 60 μL of CellTiter-Glo® reagent. The cells were thoroughly mixed with the CellTiter-Glo® mixing reagent using a microplate shaker for 2 min and then incubated at room temperature for 10 min. The 96-well plate was subsequently placed on a multifunctional microplate reader to record the Luminescence values (RLU).

The inhibition rate is calculated by the following formula:

$$\%\ \text{Inhibition rate} = \left[\frac{RLU_{\text{Negative control}} - RLU_{\text{Test compoud}}}{RLU_{\text{Negative control}} - RLU_{Blank}}\right] * 100$$

Note: Negative is the group without inhibitors; Blank is the group without cells.

$IC_{50}$ was calculated by fitting the inhibition rate by GraphPad Prism software.

**6. Detecting method for screening the *in vitro* NCI-H526 cell proliferation**

The inhibitory effect of the compounds on cell proliferation was assessed by detecting the number of viable cells by CellTiter-Glo® reagent. NCI-H526 cells in logarithmic growth phase were collected and inoculated into transparent bottom 96-well plates at 100 μL per well and a density $2\times10^3$ cells/well, and incubated overnight at 37°C, 5% $CO_2$, the compounds were sequentially 5-fold diluted with DMSO to obtain 8 concentrations of gradient dilutions, and then diluted with RPMI-640 (10% FBS) cell culture medium to obtain the compound working solution respectively (2×), which was added into the cell supernatant at 100 μL per well, and continued to incubate for 5 days at 37°C, 5% $CO_2$. The plate was removed and allowed to equilibrate at room temperature (25°C) along with the CellTiter-Glo® mixing reagent for approximately 10-30 minutes; 80 μL of the culture medium was carefully aspirated from each well, and then 80 μL of CellTiter-Glo® reagent was added. The cells were thoroughly mixed with the CellTiter-Glo® mixng reagent using a microplate shaker for 2 min and then incubated at room temperature for 10 min. The 96-well plate was subsequently placed on a multifunctional microplate reader to record the Luminescence values (RLU).

The inhibition rate is calculated by the following formula:

$$\% \text{ Inhibition rate} = \left| \frac{RLU_{\text{Negative control}} - RLU_{\text{Test compoud}}}{RLU_{\text{Negative control}} - RLU_{Blank}} \right| * 100$$

Note: Negative is the group without inhibitors; Blank is the group without cells.

$IC_{50}$ was calculated by fitting the inhibition rate by GraphPad Prism software.

**(Table II) Data for the Cell Activity of the Compounds**

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| 1 | 216 | N.T | N.T | N.T |
| 2 | 51.9 | 7893 | N.T | N.T |
| 3 | 225.9 | N.T | N.T | N.T |
| 4 | 209.2 | N.T | N.T | N.T |
| 5 | 127.9 | N.T | N.T | N.T |
| 7 | 762.4 | 2544 | 2957 | N.T |
| 8 | 500.7 | 2532 | 2851 | N.T |
| 9 | 914.8 | 3095 | N.T | N.T |
| 11 | >1000 | 2355 | 2000 | N.T |
| 13 | 331.6 | 774.6 | 2768 | N.T |
| 14 | >1000 | 2525 | 3206 | N.T |
| 15 | 221.3 | 2991 | N.T | N.T |
| 16 | >1000 | 3644 | 3702 | N.T |
| 17 | >1000 | 3273 | N.T | N.T |
| 19 | 2816 | N.T | N.T | N.T |
| 20 | 845 | N.T | N.T | N.T |
| 21 | 65 | N.T | N.T | N.T |
| 22 | 106.1 | 4734 | N.T | N.T |
| 23 | 1.55 | 760.4 | N.T | N.T |
| 24 | 42.3 | N.T | N.T | N.T |
| 25 | 50.1 | N.T | N.T | N.T |

(continued)

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| 26 | 152 | N.T | N.T | N.T |
| 27 | 48.9 | N.T | N.T | N.T |
| 28 | 25.5 | N.T | N.T | N.T |
| 29 | 92.2 | 3205 | N.T | N.T |
| 30 | 205.8 | 1502 | 2827 | N.T |
| 31 | 51 | 410.4 | 93.6 | 37.4 |
| 32 | 54.7 | N.T | N.T | N.T |
| 33 | 140.2 | 185.6 | 1981 | 470.1 |
| 34 | 140 | N.T | N.T | N.T |
| 35 | 187.7 | N.T | N.T | N.T |
| 36 | 6.58 | 108.6 | 1013 | 664.9 |
| 37 | 6.56 | 8.52 | 26 | 10.3 |
| 39 | 5.2 | 2393 | N.T | N.T |
| 40 | 65 | 193.9 | 1642 | N.T |
| 41 | 1.29 | 1678 | 5055 | N.T |
| 42 | 0.38 | 39.9 | 107.8 | 22.8 |
| 43 | 4.96 | 38.5 | 313.7 | 35.3 |
| 44 | 16.1 | 150.1 | 566.8 | 139.3 |
| 45 | 6.48 | 150.7 | 1047 | 287.5 |
| 49 | 80.1 | 232.2 | N.T | 332.7 |
| 50 | 5.58 | 162.7 | 368.6 | 216 |
| 52 | 81.6 | 71.03 | 216.1 | 111.3 |
| 54 | 0.8 | 248.2 | 1049 | N.T |
| 56 | 5.4 | 1282 | N.T | N.T |
| 57 | 14.5 | N.T | N.T | N.T |
| 58 | 46.3 | 3000 | N.T | N.T |
| 62 | 9.86 | 3225 | N.T | 1207 |
| 63 | 72.6 | 421.7 | 1805 | N.T |
| 64 | 28.6 | 2912 | 9361 | N.T |
| 67 | 21.2 | 444.4 | N.T | N.T |
| 70 | 1.44 | 213.6 | 262 | N.T |
| 71 | 42.3 | 99.0 | 431.3 | N.T |
| 72 | 165.5 | 969.6 | 2752 | N.T |
| 73 | 125.2 | 678.0 | 2233 | N.T |
| 74 | 40.1 | 204.8 | 552.7 | N.T |
| 75 | 473.0 | 402.8 | 677.4 | N.T |
| 76 | 11.7 | 3.96 | 11 | 8.27 |
| 77 | 660.9 | 120.9 | 319.5 | N.T |

(continued)

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| 80 | 14.0 | 27.8 | 56.5 | 29.3 |
| 81 | 73.9 | 516.2 | 776 | N.T |
| 82 | 11.5 | 267.6 | 352.3 | N.T |
| 83 | 76.8 | 1140 | 2820 | N.T |
| 84 | 4.0 | 32.9 | 115.5 | 68.4 |
| 85 | 256.3 | 407.1 | 1547 | N.T |
| 86 | 15.4 | 144.8 | N.T | N.T |
| 88 | 9.28 | 5.25 | 14.2 | 10.7 |
| 89 | >1000 | 120.5 | 390.1 | N.T |
| 90 | 42.0 | 6.49 | 45.3 | 34.6 |
| 91 | 623.4 | 905.6 | 2627 | N.T |
| 92 | 63.0 | 496.9 | 2373 | N.T |
| 93 | 343.1 | 1282 | 2841 | N.T |
| 94 | 160.5 | 190.1 | 495.2 | N.T |
| 95 | 334.6 | 304.3 | 628 | N.T |
| 96 | 74.2 | 101.0 | 148.6 | 107.6 |
| 97 | 8.93 | 8.76 | 20.96 | 14.2 |
| 98 | 706.6 | 527.9 | 2227 | N.T |
| 99 | 11.8 | 97.3 | 122.4 | N.T |
| 100 | 117.9 | 103.7 | 312.7 | N.T |
| 101 | 4.31 | 6.02 | 20.6 | 8.26 |
| 102 | 3.10 | 0.36 | 7.43 | 4.27 |
| 103 | 43.3 | 83.6 | 254.2 | N.T |
| 104 | 6.58 | 2.29 | 24.8 | 16.9 |
| 105 | 4.48 | 102.1 | 317.1 | 124.3 |
| 106 | 3.29 | 3.48 | 13.6 | 7.09 |
| 107 | 10.4 | 1041 | 2326 | N.T |
| 108 | 4.4 | 16 | 28.6 | 13.8 |
| 109 | 2.76 | 411.3 | 3816 | N.T |
| 110 | 2.7 | 82.7 | 186.6 | 127.2 |
| 111 | 1.98 | 3.41 | 14.2 | 16.2 |
| 112 | 4.14 | 32.7 | 86.8 | 63.5 |
| 113 | 29.8 | 76.3 | 130 | 80 |
| 114 | 9.40 | 78.2 | 390.7 | 154.6 |
| 115 | 2.25 | 3340 | 6337 | N.T |
| 116 | 4.89 | 33.8 | 82 | 33.8 |
| 117 | 1.70 | 4.21 | 10.4 | 4.54 |
| 118 | 2.80 | 3.24 | 11.8 | 8.09 |

(continued)

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| 119 | 1.68 | 121.7 | 487.4 | 194 |
| 120 | 11.1 | 938.1 | 3767 | N.T |
| 121 | 15.6 | 127.3 | 2224 | N.T |
| 123 | 4.21 | 76.3 | 111.7 | 71.3 |
| 124 | 3.34 | 18.3 | 30.5 | 20.6 |
| 125 | 12.0 | 1949 | 3035 | N.T |
| 126 | 1.76 | 283.5 | 715 | 280 |
| 127 | 2.65 | 8.85 | 15.5 | 15.4 |
| 128 | 2.01 | 130.3 | 238.8 | N.T |
| 129 | 4.38 | 25.8 | 86.9 | N.T |
| 130 | 1.09 | 25.2 | 25 | 22.8 |
| 131 | 0.62 | 2.59 | 7.09 | 3.54 |
| 132 | 13.2 | 102.9 | 234.9 | N.T |
| 133 | 5.42 | 25.5 | 128.3 | 45.8 |
| 134 | 5.10 | 8.37 | 20.3 | 13.8 |
| 135 | 6.00 | 199 | 1585 | 200.3 |
| 136 | 29.1 | 93.7 | 364.4 | N.T |
| 137 | 5.43 | 7.01 | 63.5 | 22.9 |
| 138 | 30.7 | 91.6 | 371.6 | N.T |
| 139 | 10.1 | 13.2 | 12.6 | 17.1 |
| 140 | 219.9 | 405.1 | 5186 | N.T |
| 141 | 31.0 | 95.8 | 129.4 | N.T |
| 142 | 7.59 | 4.73 | 4.24 | 4.61 |
| 143 | 1.03 | 16.8 | 23.7 | 18.1 |
| 144 | 11.2 | 11.2 | 9.28 | 8.4 |
| 145 | 18.6 | 39.3 | 37.8 | N.T |
| 147 | 255.2 | 440.2 | 657.9 | N.T |
| 148 | 2.64 | 13.1 | 21.8 | 6.22 |
| 149 | 2.08 | 23.6 | 33.9 | 14.5 |
| 150 | 5.53 | 16.9 | 9.26 | 9.84 |
| 151 | 10.4 | 189 | 307.7 | N.T |
| 152 | 4.91 | 208 | 223 | N.T |
| 153 | 6.21 | 65.5 | N.T | N.T |
| 154 | 1.41 | 16.5 | N.T | 21.7 |
| 155 | 11.5 | 314.3 | N.T | N.T |
| 156 | 6.64 | 58.1 | N.T | 80.4 |
| 158 | 93.6 | 250.5 | N.T | N.T |
| 159 | 3.50 | 17.9 | N.T | 16.1 |

(continued)

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| 160 | 5.59 | 80.6 | N.T | N.T |
| 161 | 3.13 | 65 | N.T | N.T |
| 162 | 2.98 | 21.7 | N.T | 18.5 |
| 163 | 4.97 | 1398 | N.T | N.T |
| 164 | 2.66 | 432.3 | N.T | N.T |
| 165 | 11.4 | 114 | N.T | N.T |
| 166 | 61.6 | 207.9 | N.T | N.T |
| 167 | 0.39 | 381.3 | N.T | N.T |
| 168 | 5.23 | 65.6 | N.T | N.T |
| 169 | 2.33 | 310 | N.T | N.T |
| 170 | 1.28 | 27.6 | N.T | 27.5 |
| 171 | 0.54 | 14.5 | N.T | 12.3 |
| 172 | 1.71 | 1284 | N.T | N.T |
| 173 | 2.23 | 613.4 | N.T | N.T |
| 174 | 8.67 | 4169 | N.T | N.T |
| 175 | 161.7 | 126.5 | N.T | N.T |
| 176 | 1.88 | 245.3 | N.T | 497 |
| 177 | 0.74 | 638.9 | 2032 | N.T |
| 178 | 4.21 | 905.9 | N.T | N.T |
| 179 | 3.66 | 2.75 | N.T | 4.44 |
| 180 | 3.63 | 5042 | N.T | N.T |
| 181 | 2.93 | 13.1 | N.T | 28.4 |
| 182 | 9.29 | 4.36 | N.T | 7.04 |
| 183 | 1.49 | 741.6 | N.T | N.T |
| 184 | 10.2 | 8.97 | N.T | 15.9 |
| 185 | 6.16 | 274.6 | N.T | N.T |
| 186 | 23.5 | 85 | N.T | N.T |
| 187 | 4.89 | 14.8 | N.T | 20.9 |
| 188 | 1.74 | 29.7 | N.T | 80.4 |
| 189 | 6.31 | 39.2 | N.T | 27.8 |
| 190 | 7.33 | 93.4 | N.T | N.T |
| 191 | 3.38 | 24.6 | N.T | 25.9 |
| 192 | 1.98 | 28 | N.T | 82.1 |
| 193 | 12.6 | 221 | N.T | N.T |
| 194 | 119.2 | 1029 | N.T | N.T |
| 195 | 52.4 | 1667 | N.T | N.T |
| 196 | 39.3 | 83.3 | N.T | N.T |
| 197 | 3.59 | 71.8 | N.T | 193 |

(continued)

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| 198 | 0.63 | 23.7 | N.T | 21.2 |
| 199 | 2.78 | 9.24 | N.T | 4.05 |
| 200 | 2.27 | 5.16 | N.T | 4.1 |
| 201 | 3.33 | 56.4 | N.T | 20.5 |
| 202 | 6.15 | 13.6 | N.T | 4.35 |
| 203 | 1.99 | 9.38 | N.T | 12.3 |
| 204 | 5.14 | 89.2 | N.T | N.T |
| 205 | 6.86 | 25.2 | N.T | 39.2 |
| 206 | 6.21 | 94.7 | N.T | 106 |
| 207 | 9.50 | 33.6 | N.T | 26.6 |
| 208 | 2.20 | 4.62 | N.T | 3.82 |
| 209 | 14.5 | 40.3 | N.T | N.T |
| 210 | 3.53 | 15.1 | N.T | 5.86 |
| 211 | 10.7 | 189 | N.T | N.T |
| 212 | 1.16 | 53.8 | N.T | N.T |
| 213 | 7.32 | 76.1 | N.T | N.T |
| 214 | 7.91 | 496 | N.T | N.T |
| 215 | 2.65 | 295 | N.T | N.T |
| 216 | 6.06 | 51.6 | N.T | N.T |
| 217 | 5.06 | 11.1 | N.T | 20.9 |
| 218 | 13.4 | 15.3 | N.T | 35.6 |
| 219 | 7.13 | 149 | N.T | N.T |
| 220 | 6.86 | 2287 | N.T | N.T |
| 221 | 16.8 | 19.3 | N.T | 29.0 |
| 222 | 6.42 | 35.8 | N.T | N.T |
| 223 | 3.23 | 4.56 | N.T | 16.8 |
| 224 | 24.5 | 305 | N.T | N.T |
| 225 | 8.51 | 78 | N.T | N.T |
| 226 | 2.31 | 99.2 | N.T | N.T |
| 227 | 7.36 | 24.1 | N.T | 109.2 |
| 228 | 91.7 | 84.8 | N.T | N.T |
| 229 | 9.36 | 529 | N.T | N.T |
| 230 | 4.67 | 77 | N.T | N.T |
| 231 | 1.31 | 1138 | N.T | N.T |
| 232 | 32.1 | 160 | N.T | N.T |
| 233 | 10.2 | 3.81 | N.T | 8.35 |
| 234 | 15.0 | 77.6 | N.T | N.T |
| 235 | 2.35 | 19.2 | N.T | N.T |

(continued)

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| 236 | 6.02 | 410 | N.T | N.T |
| 237 | 4.06 | 3.27 | N.T | 4.4 |
| 238 | 25.7 | 28.7 | N.T | 56.1 |
| 239 | 11.6 | 25.2 | N.T | N.T |
| 240 | 9.62 | 24.8 | N.T | 60.9 |
| 241 | 5.78 | 5.7 | N.T | 11.2 |
| 242 | 18.4 | 17.8 | N.T | N.T |
| 243 | 10.5 | 3.24 | N.T | 10.6 |
| 244 | 2.83 | 1.66 | N.T | 16.2 |
| 245 | 14.1 | 4.16 | N.T | 11.8 |
| 246 | 10.8 | 14.3 | N.T | N.T |
| 247 | 4.93 | 3.17 | N.T | 7.56 |
| 248 | 5.33 | 3.94 | N.T | 9.06 |
| 249 | 1.26 | 2.74 | N.T | 11.7 |
| 250 | 2.42 | 1.93 | N.T | N.T |
| 251 | 7.41 | 3.11 | N.T | 7.18 |
| 252 | 1.86 | 4.32 | N.T | N.T |
| 253 | 13.2 | 5.25 | N.T | N.T |
| 254 | 5.77 | 4.69 | N.T | N.T |
| 255 | 3.39 | 7.82 | N.T | 11.0 |
| 256 | 8.48 | 8.63 | N.T | N.T |
| 257 | 7.61 | 11.2 | N.T | N.T |
| 258 | 1.05 | 1.81 | N.T | 2.09 |
| 259 | 7.20 | 7.48 | N.T | N.T |
| 260 | 3.02 | 8.18 | N.T | 11.5 |
| 261 | 19.0 | 8.47 | N.T | N.T |
| 262 | 11.8 | 11.1 | N.T | N.T |
| 263 | 1.64 | 1.14 | N.T | 2.22 |
| 264 | 2.2 | 1.84 | N.T | 5.12 |
| 265 | 5.09 | 7.20 | N.T | 5.85 |
| 266 | 2.09 | 2.02 | N.T | 2.35 |
| 267 | 7.49 | 13.6 | N.T | N.T |
| 268 | 2.03 | 1.58 | N.T | 2.22 |
| 269 | 2.66 | 1.87 | N.T | N.T |
| 270 | 4.88 | 3.24 | N.T | N.T |
| 271 | 2.19 | 1.80 | N.T | N.T |
| 272 | 16.8 | 13.3 | N.T | N.T |
| CC-90011 | 3.1 | 8376 | >10000 | >10000 |

(continued)

| Example | H1417 | H69 | Du145 | H526 |
|---|---|---|---|---|
| | $IC_{50}$(nM) | $IC_{50}$(nM) | $IC_{50}$(nM) | $IC_{50}$(nM) |
| SAHA(Vori nostat) | 709.7 | 473 | 1349 | 397.4 |

As can be seen from the data in Table I, the Example compounds of the present disclosure have significant inhibitory effects on both HDAC and LSD1. The present disclosure provides structurally novel compounds represented by formula (I), which have dual-target inhibitory effects on HDAC and LSD1, and the IC50s for inhibiting the activity of both HDAC and LSD1 are below 2 $\mu$M, or less than 1 $\mu$M, or less than 100 nM, or even both less than 1 nM.

The data in Table II shows that the compounds of the present disclosure have significant inhibitory activity against small cell lung cancer cell H1417, small cell lung cancer cell H69, small cell lung cancer cell H526, or prostate cancer cell Du145, and have the potential to be used as medicaments for the treatment of these cancers.

## Claims

1. A compound represented by formula (I) or a tautomer, stereoisomer, solvate, metabolite, isotopically-labeled compound, pharmaceutically acceptable salt or co-crystal thereof,

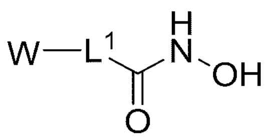

**(I)**

$L^1$ is selected from a bond, -$C_{1-10}$ alkyl-, -$C_{2-6}$ alkenyl-, -$C_{1-10}$ alkyl-$C_{2-6}$ alkenyl-, -$C_{2-6}$ alkynyl-, -$C_{6-10}$ heteroaryl-, -$C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, -$C_{1-10}$ alkyl-($C_{6-10}$ aryl)-, -$C_{1-10}$ alkyl-($C_{6-10}$ aryl)-$C_{1-10}$ alkyl-, -$NR^a$-, -$C_{1-10}$ alkyl-($C_{6-10}$ aryl)-$C_{2-6}$ alkynyl-, -$C_{1-10}$ alkyl-($C_{6-10}$ heterocycloalkyl)-($C_{6-10}$ aryl)-, -$C_{1-10}$ alkyl-NH-6-10-membered heteroaryl-, -$C_{1-10}$ alkyl-6-10-membered heteroaryl-, -$C_{1-10}$ alkyl-$C_{6-10}$ cycloalkenyl-$C_{2-6}$ alkenyl-, -$C_{1-10}$ alkyl-$C_{6-10}$ aryl-$C_{3-6}$ cycloalkenyl-, -$C_{1-10}$ alkyl-$C_{6-10}$ aryl-$C_{3-6}$ cycloalkyl-, -$C_{1-10}$ alkyl-O-$C_{6-10}$ aryl-, -$C_{1-10}$ alkyl-6-10-membered heteroaryl-$C_{1-10}$ alkyl-, -$C_{1-10}$ alkyl-6-10-membered aryl-O-$C_{1-10}$ alkyl-, -$C_{1-10}$ alkyl-6-10-membered heteroaryl-O-$C_{1-10}$ alkyl-, -$C_{1-10}$ alkyl-6-10-membered aryl-S-$C_{1-10}$ alkyl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, -$NR^aR^b$, COOH, -C(=O)$NR^aR^b$, the heteroaryl, heterocycloalkyl contain 1 to 4 heteroatom selected from N, O, or S; alternatively, one or more alkyl groups of the alkyl may optionally be replaced by one or more groups selected from -C(=O)-, -$S(=O)_2$- or -$NR^a$-;

W is selected from:

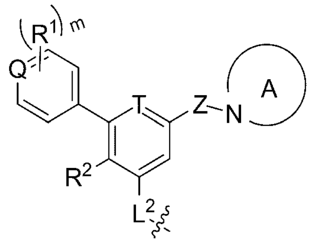 , 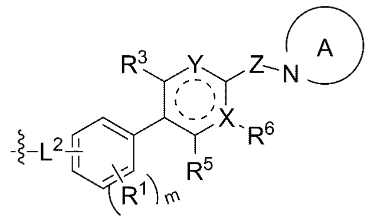 , 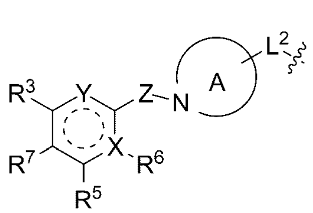 ,

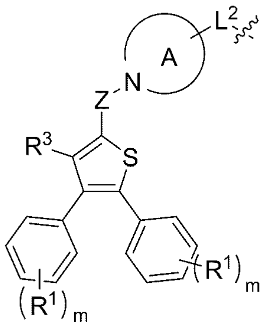 or 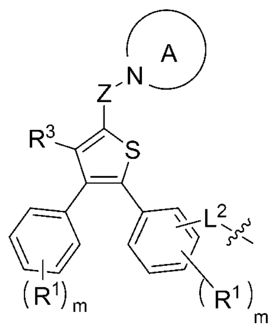 ;

$L^2$ is selected from a bond, -O-, -C(=O)-, $-NR^a-$, $-CH_2-NR^a-$, $-NR^a-C(O)-$, $-NR^a-S(=O)_2-$, -S- or $-S(=O)_2-$;
ring A is selected from nitrogen-containing $C_{3-10}$ heteroaryl, $C_{3-10}$ heterocycloalkyl or $C_{3-10}$ heterocycloalkenyl, wherein, the heteroaryl, heterocycloalkyl or heterocycloalkenyl is optionally substituted with one or more $R^4$, the heteroaryl, heterocycloalkyl contain 1 to 4 heteroatoms optionally selected from N, O or S;
$R^4$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, $-C(=O)NR^aR^b$; optionally, when $R^4$ is selected from $C_{1-6}$ alkyl, any two $R^4$ and the atom to which they connect can collectively form a 5 to 10-membered heteroalicyclic;
$R^1$, $R^6$ are each independently selected at each occurrence from hydrogen, halogen, CN, hydroxyl, $C_{1-6}$ alkyl, $C_{1-6}$ alkyl-CN, $C_{1-6}$ alkoxy, hydroxyl-substituted $C_{1-6}$ alkyl, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl, COOH, $-NR^aR^b$, $-C(=O)NR^aR^b$, $-S(=O)_2R^a$ or $-C_{2-6}$ alkenyl-$C(=O)NR^aR^b$;
$R^2$, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkoxy, halogen-substituted $C_{1-6}$ alkyl, hydroxyl-substituted $C_{1-6}$ alkyl, hydroxyl-substituted $C_{1-6}$ alkoxy, hydroxyl-substituted $C_{1-6}$ alkoxy-$C_{3-6}$ cycloalkyl, COOH, $-NR^aR^b$, $-S(=O)_2R^a$, $-C(=O)NR^aR^b$, $-C_{2-6}$ alkenyl-$C(=O)NR^aR^b$, $C_{3-6}$ cycloalkyl, 3- to 6-membered heterocycloalkyl, heterocycloalkenyl, the heteroaryl, heterocycloalkyl contain 1 to 4 heteroatoms optionally selected from N, O or S;
m is selected from 0, 1, 2, 3, 4 or 5;
Q, T are each independently selected from N or C;
X, Y are each independently selected from C and N;
Z is selected from a bond, $-CH_2-$, -C(=O) or $-S(=O)_2-$;
$R^5$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, =O, COOH, $-NR^aR^b$, $-C(=O)NR^aR^b$, $C_{3-6}$ cycloalkyl, 3- to 6-membered heterocycloalkyl, $C_{6-10}$ aryl or $C_{6-10}$ heteroaryl, wherein, the alkyl, alkoxy, alkenyl, alkynyl, cycloalkyl, heterocycloalkyl, aryl or heteroaryl is optionally substituted with one or more substituents selected from hydrogen, halogen, CN, $NO_2$, $CF_3$, $CHF_2$, hydroxyl, $C_{1-6}$ alkyl, $C_{1-6}$ alkoxy, $-C(=O)-C_{1-6}$ alkoxy, $C_{2-6}$ alkenyl, $C_{2-6}$ alkynyl, COOH, $-NR^aR^b$, $-C(=O)NR^aR^b$, the heteroaryl, heterocycloalkyl contain 1 to 4 heteroatoms optionally selected from N, O or S;
alternatively, $R^5$, $R^6$ and the atom to which both of them directly connect collectively form cycloalkyl, heteroalicyclic, aryl or heteroaryl, wherein the cycloalkyl, heteroalicyclic, aryl or heteroaryl is optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl, $-NR^aR^b$, $-C(=O)NR^aR^b$;
$R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkyl, $C_{3-6}$ cycloalkyl, 3- to 6-membered heterocycloalkyl, $C_{6-10}$ aryl or $C_{6-10}$ heteroaryl, the heteroaryl, heterocycloalkyl contain 1 to 4 heteroatoms optionally selected from N, O or S;

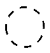 indicates a double bond may be present or not present at any position within the ring.

**2.** The compound or tautomer, stereoisomer, solvate, metabolite, isotopically-labeled compound, pharmaceutically acceptable salt or co-crystal thereof according to claim 1, wherein, the left end group of $L^1$ is connected to W, the right end group of $L^1$ is connected to

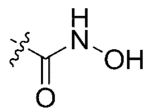

;

preferably, the compound is represented by formula (II), (III), (IV), (V) or (VI):

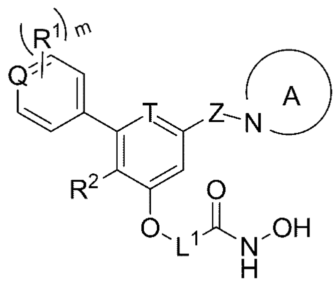

**(II)**

,

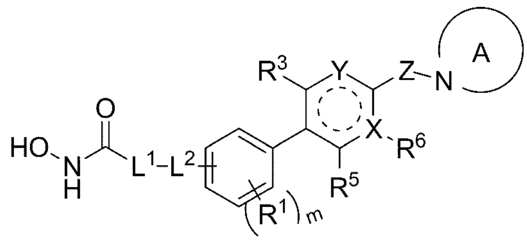

**(III)**

,

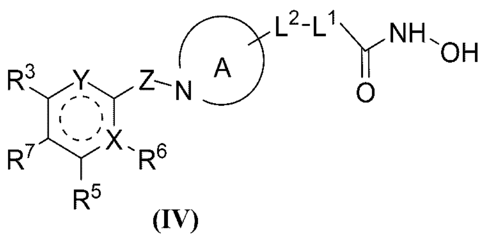

**(IV)**

,

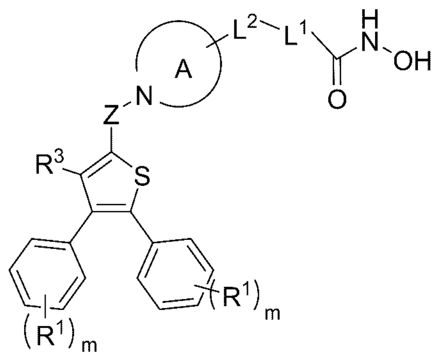

**(V)**

or

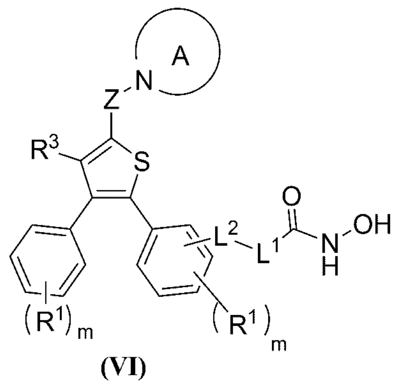

**(VI)**

the definition of each substituent is defined as in claim 1.

**3.** The compound or tautomer, stereoisomer, solvate, metabolite, isotopically-labeled compound, pharmaceutically

acceptable salt or co-crystal thereof according to claim 1 or 2, wherein, the compound is represented by formula (II-1):

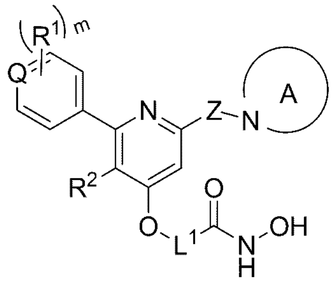

**(II-1)**

wherein,

$L^1$ is selected from $-C_{1-10}$ alkyl-, $-C_{2-6}$ alkenyl-, $-C_{1-10}$ alkyl-$C_{2-6}$ alkenyl-, $-C_{2-6}$ alkynyl-, $-C_{1-10}$ alkyl-($C_{6-10}$ aryl)-$C_{2-6}$ alkenyl-, the alkyl, alkenyl are optionally substituted with one or more substituents selected from hydrogen, halogen, CN, $CF_3$, hydroxyl, $C_{1-6}$ alkyl, $C_{1-6}$ alkoxy;
preferably, $L^1$ is selected from $-C_{1-10}$ alkyl-, $-C_{1-10}$ alkyl-$C_{2-6}$ alkenyl-, $-C_{1-10}$ alkyl-($C_{6-10}$ aryl)-$C_{2-6}$ alkenyl-;
preferably, $L^1$ is selected from $-C_{1-10}$ alkyl-, $-C_{1-10}$ alkyl-($C_{6-10}$ aryl)-$C_{2-6}$ alkenyl-;
preferably, $L^1$ is selected from $-C_{1-6}$ alkyl-, $-C_{1-6}$ alkyl-phenylene-$C_{2-6}$ alkenyl-.

**4.** The compound or tautomer, stereoisomer, solvate, metabolite, isotopically-labeled compound, pharmaceutically acceptable salt or co-crystal thereof according to any one of claims 1 to 3, wherein,

ring A is selected from nitrogen-containing $C_{3-10}$ heteroaryl or $C_{3-10}$ heterocycloalkyl, wherein, the heteroaryl, heterocycloalkyl are optionally substituted with one or more $R^4$;
preferably, ring A is selected from nitrogen-containing $C_{3-10}$ heterocycloalkyl, wherein, the heterocycloalkyl is optionally substituted with one or more $R^4$;
preferably, ring A is selected from:

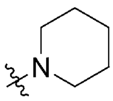 , 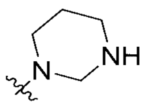 , 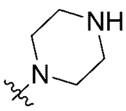 , 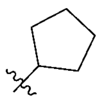 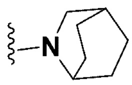 ,

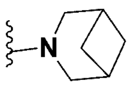 , 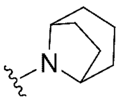 , 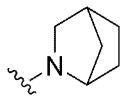 ,

wherein the

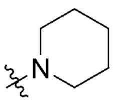 , 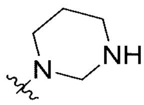 , 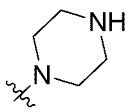 , 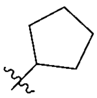 ,

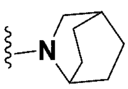 , 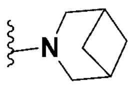 , 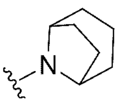

are optionally substituted with $R^4$,
preferably, ring A is selected from:

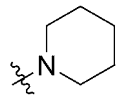, 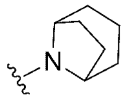 and 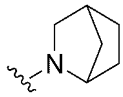,

the

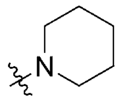,

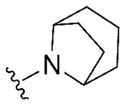 and 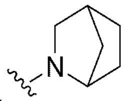

are optionally substituted with $R^4$.

**5.** The compound or tautomer, stereoisomer, solvate, metabolite, isotopically-labeled compound, pharmaceutically acceptable salt or co-crystal thereof according to any one of claims 1 to 4, wherein,

$R^4$ is each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, $-C(=O)NR^aR^b$;
preferably, $R^4$ is each independently selected at each occurrence from hydrogen, halogen, CN, hydroxyl, $C_{1-6}$ alkyl, $C_{1-6}$ alkoxy, $-NR^aR^b$;
preferably, $R^4$ is selected from hydrogen, $-NR^aR^b$, $C_{1-56}$ alkyl;
preferably, $R^4$ is selected from hydrogen, $-NR^aR^b$.

**6.** The compound or tautomer, stereoisomer, solvate, metabolite, isotopically-labeled compound, pharmaceutically acceptable salt or co-crystal thereof according to any one of claims 1 to 5, wherein,

$R^1$ is selected from hydrogen, halogen, CN, hydroxyl, $C_{1-6}$ alkyl, $C_{1-6}$ alkoxy, hydroxyl-substituted $C_{1-6}$ alkyl, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl;
preferably, $R^1$ is selected from hydrogen, halogen, CN, $C_{1-6}$ alkyl;
preferably, $R^1$ is selected from hydrogen, halogen, CN;
$R^2$ is selected from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl, COOH, $-NR^aR^b$, $-S(=O)_2R^a$, $-O-C_{1-6}$ alkyl-OH, the heteroaryl, heterocycloalkyl contain 1 to 4 heteroatoms optionally selected from N, O or S;
preferably, $R^2$ is selected from hydrogen, $C_{6-10}$ aryl or $C_{6-10}$ heteroaryl, wherein, the aryl or heteroaryl is optionally substituted with one or more substituents selected from hydrogen, halogen, hydroxyl, $C_{1-6}$ alkyl, $C_{1-6}$ alkoxy, $-NR^aR^b$, $-S(=O)_2R^a$, $-O-C_{1-6}$ alkyl-OH, the heteroaryl, heterocycloalkyl contain 1 to 4 heteroatoms optionally selected from N, O or S;
preferably, $R^2$ is selected from hydrogen,

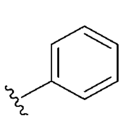, 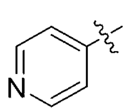, 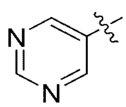, 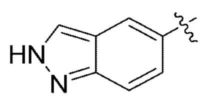,

wherein, the

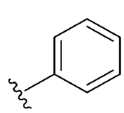, 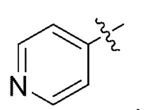, 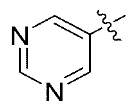, 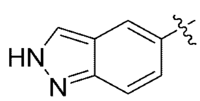

are optionally substituted with one or more substituents selected from hydrogen, halogen, hydroxyl, $C_{1-6}$ alkyl,

$C_{1-6}$ alkoxy, $-NR^aR^b$, $-S(=O)_2-R^a$, $-O-C_{1-6}$ alkyl-OH;
preferably, $R^2$ is selected from

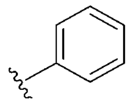

, wherein, the

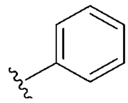

is optionally substituted with one or more substituents selected from hydrogen, hydroxyl, $C_{1-6}$ alkoxy.

**7.** The compound or tautomer, stereoisomer, solvate, metabolite, isotopically-labeled compound, pharmaceutically acceptable salt or co-crystal thereof according to any one of claims 1 to 6, wherein,

m is selected from 0, 1, 2 or 3; preferably, m is selected from 1 and 2; more preferably, m is 2;
Q is each independently selected from N or C; preferably, Q is selected from C.

**8.** The compound or tautomer, stereoisomer, solvate, metabolite, isotopically-labeled compound, pharmaceutically acceptable salt or co-crystal thereof according to any one of claims 1 to 7, wherein,

$R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkyl;
preferably, $R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl,
preferably, $R^a$, $R^b$ are each independently selected at each occurrence from hydrogen and methyl;
preferably, $R^a$, $R^b$ are each independently selected from hydrogen.

**9.** The compound or tautomer, stereoisomer, solvate, metabolite, isotopically-labeled compound, pharmaceutically acceptable salt or co-crystal thereof according to claim 1 or 2, wherein, the compound is represented by formula (III-1):

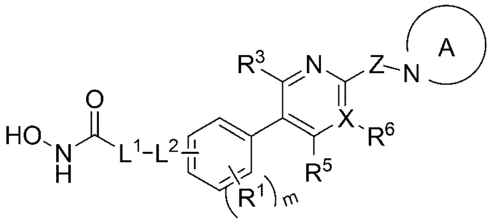

**(III-1)**

wherein,

X is selected from C and N;
$L^1$ is selected from a bond, $-C_{1-10}$ alkyl-, $-C_{2-6}$ alkenyl-, $-C_{1-10}$ alkyl-$C_{2-6}$ alkenyl-, $-C_{2-6}$ alkynyl-, $-C_{1-10}$ alkyl-($C_{6-10}$ aryl or heteroaryl)-$C_{2-6}$ alkenyl-, $-C_{1-10}$ alkyl-($C_{6-10}$ aryl)-, $-NR^a-$, the alkyl, alkoxy, alkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $-NR^aR^b$, COOH;
alternatively, one or more carbon atoms in the alkyl can optionally be replaced by one or more groups selected from -NH-;
preferably, $L^1$ is selected from a bond, $-C_{1-10}$ alkyl-, $-C_{2-6}$ alkenyl-, $-C_{1-10}$ alkyl-$C_{2-6}$ alkenyl-, $-C_{1-10}$ alkyl-($C_{6-10}$ aryl or heteroaryl)-$C_{2-6}$ alkenyl-, $-C_{1-10}$ alkyl-($C_{6-10}$ aryl)-, the alkyl, alkoxy, alkenyl, aryl or heteroaryl is optionally substituted with one or more substituents selected from hydrogen, halogen, CN, $CF_3$, hydroxyl, $C_{1-6}$ alkoxy, $C_{2-6}$ alkenyl, $-NR^aR^b$, $-NR^aR^b$;

alternatively, one or more carbon atoms in the alkyl can optionally be replaced by one or more groups selected from -NH-;

preferably, $L^1$ is selected from -$C_{1-10}$ alkyl-, -$C_{1-10}$ alkyl-($C_{6-10}$ aryl or heteroaryl)-$C_{2-6}$ alkenyl-, -$C_{1-10}$ alkyl-($C_{6-10}$ aryl)-, the alkyl, aryl or heteroaryl is optionally substituted with one or more substituents selected from hydrogen, halogen, CN, $CF_3$, hydroxyl, $C_{1-6}$ alkyl, $C_{1-6}$ alkoxy.

**10.** The compound or tautomer, stereoisomer, solvate, metabolite, isotopically-labeled compound, pharmaceutically acceptable salt or co-crystal thereof according to any one of claims 1 to 9, wherein,

$L^2$ is selected from a bond, -O-, -C(=O)-, -$NR^a$-, -$NR^a$-C(O)- or -$S(=O)_2$-;

preferably, $L^2$ is selected from a bond, -O-, -$NR^a$-;

preferably, $L^2$ is selected from -O-, -$NR^a$-.

**11.** The compound or tautomer, stereoisomer, solvate, metabolite, isotopically-labeled compound, pharmaceutically acceptable salt or co-crystal thereof according to any one of claims 1 to 10, wherein,

ring A is selected from nitrogen-containing $C_{3-10}$ heteroaryl or $C_{3-10}$ heterocycloalkyl, wherein, the heteroaryl, heterocycloalkyl are optionally substituted with one or more $R^4$;

preferably, ring A is selected from nitrogen-containing $C_{3-10}$ heterocycloalkyl, the $C_{3-10}$ heterocycloalkyl is optionally substituted with one or more $R^4$;

preferably, ring A is selected from:

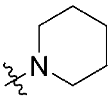 , 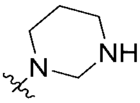 , 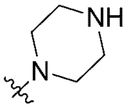 , 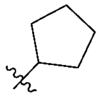 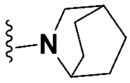 ,

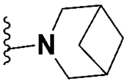 , 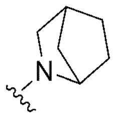 ,

wherein, the

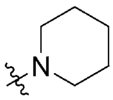 , 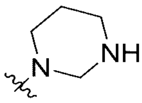 , 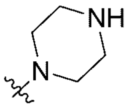 , 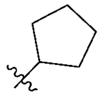 , 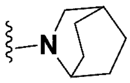 ,

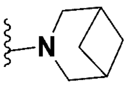 , 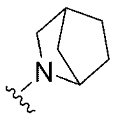

are optionally substituted with $R^4$;

preferably, ring A is selected from:

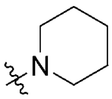 and 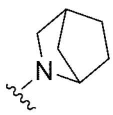

, the

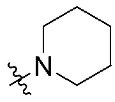 and 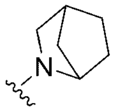

are optionally substituted with $R^4$.

**12.** The compound or tautomer, stereoisomer, solvate, metabolite, isotopically-labeled compound, pharmaceutically acceptable salt or co-crystal thereof according to any one of claims 1 to 11, wherein,

$R^4$ is each independently selected at each occurrence from hydrogen, halogen, CN, hydroxyl, $C_{1-6}$ alkyl, $C_{1-6}$ alkoxy, $C_{2-6}$ alkenyl, $-NR^aR^b$;
preferably, $R^4$ is selected from hydrogen, $C_{1-6}$ alkyl, $-NR^aR^b$;
preferably, $R^4$ is selected from hydrogen, $-NR^aR^b$.

**13.** The compound or tautomer, stereoisomer, solvate, metabolite, isotopically-labeled compound, pharmaceutically acceptable salt or co-crystal thereof according to any one of claims 1 to 12, wherein,

$R^1$, $R^6$ are each independently selected at each occurrence from hydrogen, halogen, CN, hydroxyl, $C_{1-6}$ alkyl, $C_{1-6}$ alkoxy, halogen-substituted $C_{1-6}$ alkyl, halogen-substituted $C_{1-6}$ alkoxy, $C_{3-6}$ cycloalkyl, $C_{2-6}$ alkenyl, $C_{2-6}$ alkynyl, COOH, $-NR^aR^b$, $-C(=O)NR^aR^b$;
preferably, $R^1$, $R^6$ are each independently selected at each occurrence from hydrogen, CN, hydroxyl, $C_{1-6}$ alkoxy;
preferably, $R^1$, $R^6$ are each independently selected at each occurrence from hydrogen, CN, hydroxyl;
$R^3$ is selected from hydrogen, $C_{1-6}$ alkyl, $C_{1-6}$ alkoxy, $C_{6-10}$ aryl or $C_{6-10}$ heteroaryl, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O or S;
preferably, $R^3$ is selected from hydrogen, $C_{1-6}$ alkyl, $C_{6-10}$ aryl or $C_{6-10}$ heteroaryl, wherein, the aryl or heteroaryl is optionally substituted with one or more substituents selected from hydrogen, halogen, CN, the heteroaryl, heterocycloalkyl contain 1 to 4 heteroatoms optionally selected from N, O or S;
preferably, $R^3$ is selected from hydrogen, methyl,

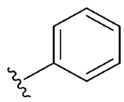, 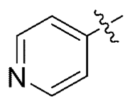, 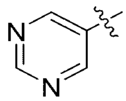

, the

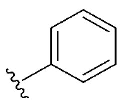,

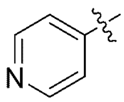, 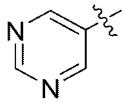

are optionally substituted with one or more substituents selected from hydrogen, halogen, CN;
preferably, $R^3$ is selected from hydrogen,

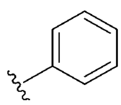,

the

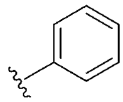

is optionally substituted with one or more substituents selected from hydrogen, halogen, CN.

**14.** The compound or tautomer, stereoisomer, solvate, metabolite, isotopically-labeled compound, pharmaceutically acceptable salt or co-crystal thereof according to any one of claims 1 to 13, wherein,

$R^5$ is each independently selected at each occurrence from hydrogen, halogen, CN, hydroxyl, $C_{1-6}$ alkyl, $C_{1-6}$ alkoxy, $C_{2-6}$ alkenyl, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O or S;

alternatively, $R^5$, $R^6$ and the atom to which both of them directly connect collectively form cycloalkyl, heteroalicyclic, aryl or heteroaryl, wherein, the cycloalkyl, heteroalicyclic, aryl, heteroaryl are optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl;

preferably, $R^5$ is selected from hydroxyl, $C_{1-6}$ alkoxy,

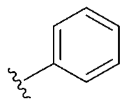 , 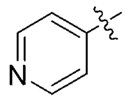 ,

wherein, the alkoxy,

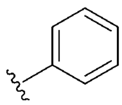 , 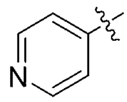

are optionally substituted with one or more substituents selected from hydrogen, halogen, CN, hydroxyl, $C_{1-6}$ alkyl, $C_{1-6}$ alkoxy,

alternatively, $R^5$, $R^6$ and the atom to which both of them directly connect collectively form

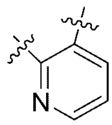 ,

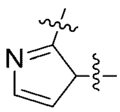 , 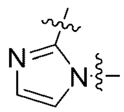 ,

wherein, the

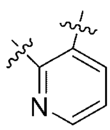 , 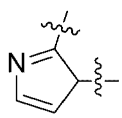 , 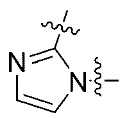

are optionally substituted with one or more groups selected from hydrogen, halogen, CN, hydroxyl, $C_{1-6}$ alkyl, $C_{1-6}$ alkoxy;

preferably, $R^5$ is selected from

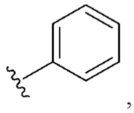

wherein, the

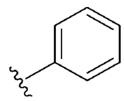

is optionally substituted with one or more substituents selected from hydrogen, halogen, CN; alternatively, $R^5$, $R^6$ and the atom to which both of them directly connect collectively form

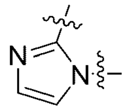

**15.** The compound or tautomer, stereoisomer, solvate, metabolite, isotopically-labeled compound, pharmaceutically acceptable salt or co-crystal thereof according to any one of claims 1 to 14, wherein,

m is selected from 0, 1, 2 or 3; preferably, m is selected from 1 and 2;
Z is selected from a bond, $-CH_2-$ or -C(=O); preferably, Z is selected from a bond.

**16.** The compound or tautomer, stereoisomer, solvate, metabolite, isotopically-labeled compound, pharmaceutically acceptable salt or co-crystal thereof according to any one of claims 1 to 15, wherein,

$R^a$, $R^b$ are each independently selected at each occurrence from hydrogen, halogen, CN, hydroxyl, $C_{1-6}$ alkyl, $C_{1-6}$ alkoxy, $C_{2-6}$ alkenyl, $C_{2-6}$ alkynyl, COOH, wherein, the alkyl, alkoxy, alkenyl, alkynyl are optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, COOH;
preferably, $R^a$, $R^b$ are each independently selected at each occurrence from hydrogen, $C_{1-6}$ alkyl;
preferably, $R^a$, $R^b$ are each independently selected at each occurrence from hydrogen and methyl.

**17.** The compound or tautomer, stereoisomer, solvate, metabolite, isotopically-labeled compound, pharmaceutically acceptable salt or co-crystal thereof according to claim 1 or 2, wherein, the compound is represented by formula (IV-1a):

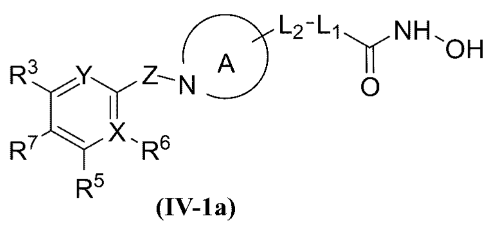

**(IV-1a)**

wherein,

$L^1$ is selected from a bond, $-C_{1-10}$ alkyl-, $-C_{2-6}$ alkenyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-NR^a-$, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, $-C_{1-10}$ alkyl-NH-6-10-membered heteroaryl-, $-C_{1-10}$ alkyl-6-10-membered heteroaryl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$ cycloalkenyl-, $-C_{1-10}$ alkyl-$C_{6-10}$ aryl-$C_{3-6}$ cycloalkyl-, $-C_{1-10}$ alkyl-O-$C_{6-10}$ aryl-, $-C_{1-10}$ alkyl-6-10-membered heteroaryl-$C_{1-10}$ alkyl-, $-C_{1-10}$ alkyl-6-10-membered aryl-O-$C_{1-10}$ alkyl-, $-C_{1-10}$ alkyl-6-10-membered heteroaryl-O-$C_{1-10}$ alkyl-, $-C_{1-10}$ alkyl-6-10-membered aryl-S-$C_{1-10}$ alkyl-, the alkyl, alkenyl, cycloalkyl, heterocycloalkyl, heterocycloalkenyl, aryl or heteroaryl is optionally substituted with one or more substituents selected

from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O or S;

alternatively, one or more alkyl groups of the alkyl may optionally be replaced by one or more groups selected from -C(=O)-, $-S(=O)_2-$ or $-NR^a-$;

preferably, $L^1$ is selected from $-C_{1-10}$ alkyl-, $-C_{2-6}$ alkenyl-, $-C_{6-10}$ heteroaryl-, $-C_{1-10}$ alkyl-($C_{6-10}$ aryl or heteroaryl or heterocycloalkyl or heterocycloalkenyl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-C_{1-10}$ alkyl-($C_{6-10}$ aryl)-$C_{2-6}$ alkynyl-, $-C_{1-10}$ alkyl-($C_{6-10}$ heterocycloalkyl)-($C_{6-10}$ aryl)-, $-C_{1-10}$ alkyl-NH-6-10-membered heteroaryl-, $-C_{1-10}$ alkyl-6-10-membered heteroaryl-, $-C_{1-10}$ alkyl-$C_{6-10}$ cycloalkenyl-$C_{2-6}$ alkenyl-, $-C_{1-10}$ alkyl-$C_{6-10}$ aryl-$C_{3-6}$ cycloalkenyl-, $-C_{1-10}$ alkyl-$C_{6-10}$ aryl-$C_{3-6}$ cycloalkyl-, $-C_{1-10}$ alkyl-O-$C_{6-10}$ aryl-, $-C_{1-10}$ alkyl-6-10-membered heteroaryl-$C_{1-10}$ alkyl-, $-C_{1-10}$ alkyl-6-10-membered aryl-O-$C_{1-10}$ alkyl-, $-C_{1-10}$ alkyl-6-10-membered heteroaryl-O-$C_{1-10}$ alkyl-, $-C_{1-10}$ alkyl-6-10-membered aryl-S-$C_{1-10}$ alkyl-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O or S;

preferably, $L^1$ is selected from $-C_{1-10}$ alkyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl)-$C_{2-6}$ alkenyl-, -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-C_{1-10}$ alkyl-($C_{6-10}$ heterocycloalkyl)-($C_{6-10}$ aryl)-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O or S;

preferably, $L^1$ is selected from $-C_{1-10}$ alkyl-, $-C_{1-10}$ alkyl-$C_{2-6}$ alkenyl-, $-C_{6-10}$ heteroaryl-, $-C_{1-10}$ alkyl-($C_{6-10}$ aryl or heteroaryl)-$C_{2-6}$ alkenyl- , -($C_{6-10}$ aryl or heteroaryl)-$C_{2-6}$ alkenyl-, $-C_{1-10}$ alkyl-($C_{6-10}$ aryl)-, $-C_{1-10}$ alkyl-($C_{6-10}$ aryl)-$C_{1-10}$ alkyl-, $-C_{1-10}$ alkyl-($C_{6-10}$ heterocycloalkyl)-($C_{6-10}$ aryl)-, the alkyl, alkenyl, cycloalkyl, heterocycloalkyl, heterocycloalkenyl, aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, the heteroaryl, heterocycloalkyl contain 1 to 4 heteroatoms optionally selected from N, O or S;

preferably, $L^1$ is selected from $-C_{1-10}$ alkyl-($C_{6-10}$ aryl or heteroaryl)-$C_{2-6}$ alkenyl-, the alkyl, aryl or heteroaryl is optionally substituted with one or more substituents selected from hydrogen, halogen, CN, $CF_3$, hydroxyl, $C_{1-6}$ alkyl, $C_{1-6}$ alkoxy;

preferably, $L^1$ is selected from $-CH_2-$, $-CH_2-(C{=}C)-$, $-(CH_2)_4-$, $-(CH_2)_6-$, -(C=O)-phenyl-(C=C)-, $-CH_2$-phenyl-, $-(CH_2)_3$-phenyl-, $-CH_2$-phenyl-$(CH_2)_2$-, $-(CH_2)_2$-phenyl-$CH_2$-, $-CH_2$-phenyl-(C=C)-, $-(CH_2)_2$-phenyl-(C=C)-, $-CH_2$-phenyl-(C≡C)-, $-CH_2$-phenyl-(C=C)-$CH_2$-, -phenyl-(C=C)-, pyrimidinyl,

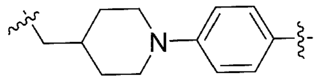,

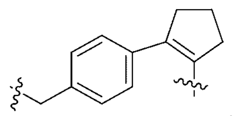, 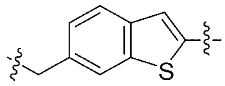, 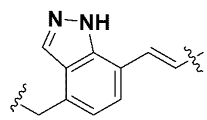, 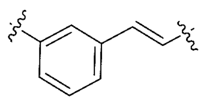, 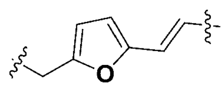,

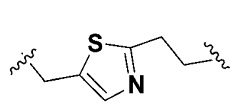, 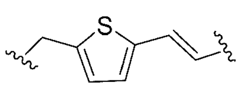, 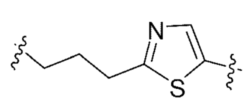, 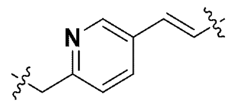,

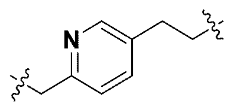, 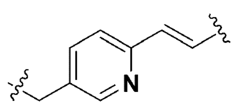, 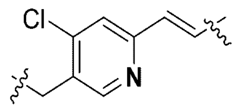, 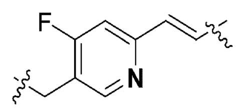,

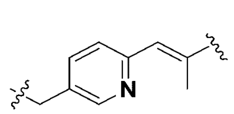, 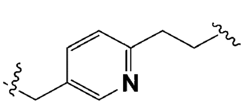, 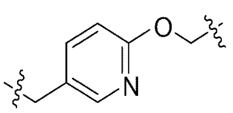, 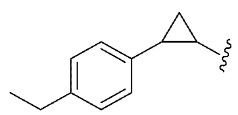,

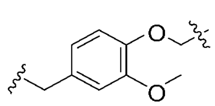, 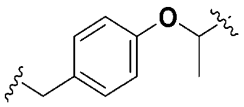, 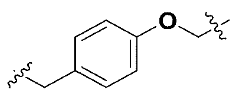, 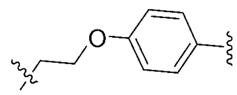,

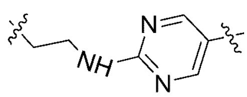, 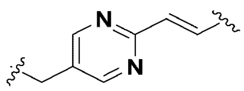, 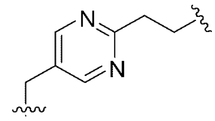, 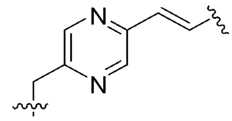,

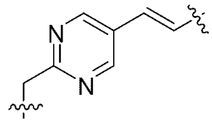, 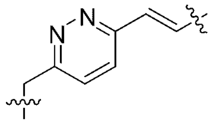, 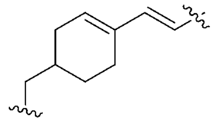, 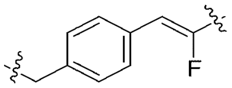,

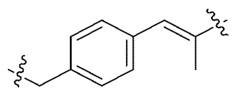, 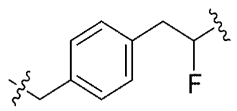, 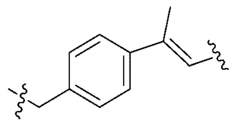, 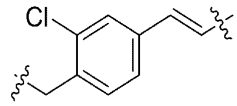,

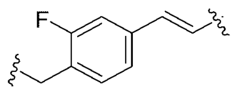, 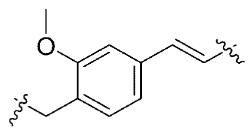, 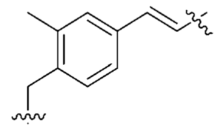, 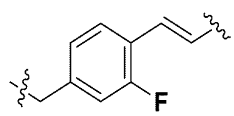,

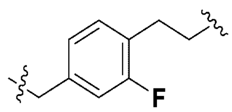, 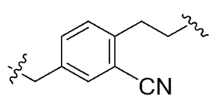, 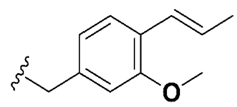, 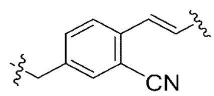,

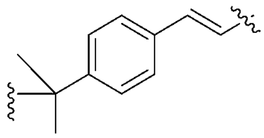, 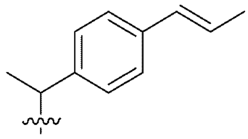, 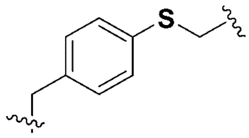;

preferably, $L^1$ is selected from

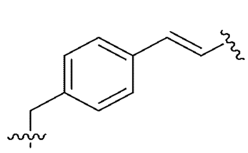, 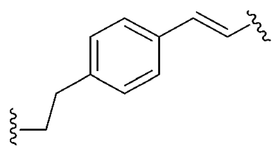, 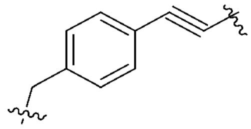,

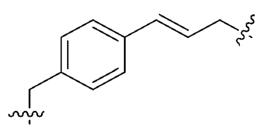, 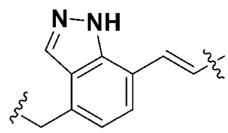, 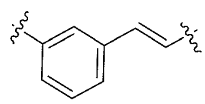, 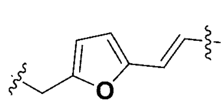,

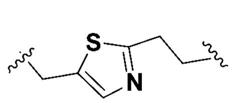, 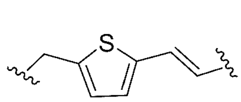, 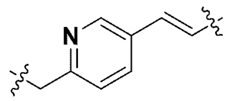, 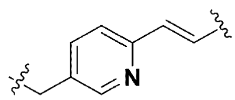,

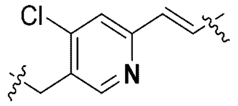, 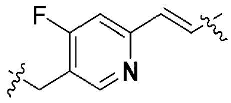, 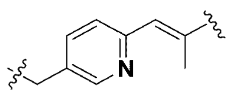, 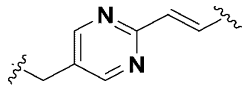,

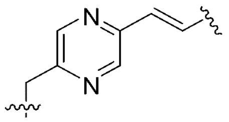, 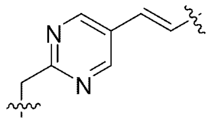, 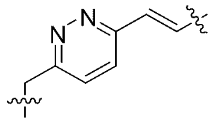, 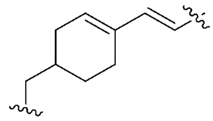,

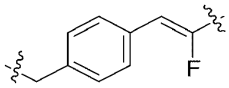, 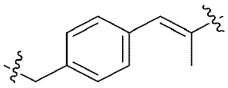, 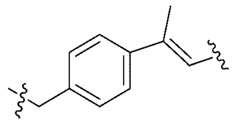, 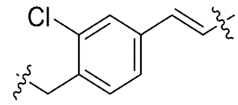,

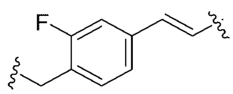, 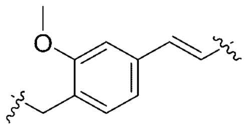, 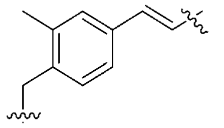, 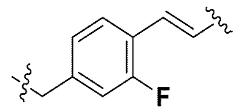,

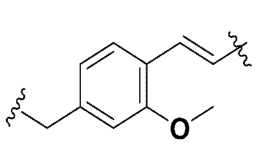, 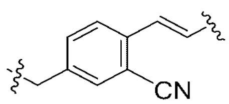, 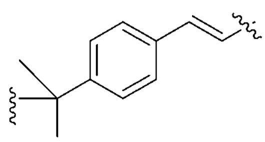, 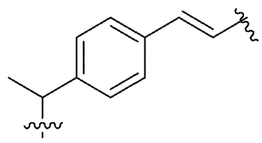

**18.** The compound or tautomer, stereoisomer, solvate, metabolite, isotopically-labeled compound, pharmaceutically acceptable salt or co-crystal thereof according to any one of claims 1 to 17, wherein,

$L^2$ is selected from a bond, -O-, -C(=O)-, -S-, -NR$^a$-, -CH$_2$-NR$^a$-, -NR$^a$-C(=O) and -NR$^a$-S(=O)$_2$-;
preferably, $L^2$ is selected from a bond, -NR$^a$-, -CH$_2$-NR$^a$-, -NR$^a$-C(=O) and -NR$^a$-S(=O)$_2$-;
preferably, $L^2$ is selected from -NR$^a$-, -NR$^a$-C(=O) and -NR$^a$-S(=O)$_2$-;
preferably, $L^2$ is selected from a bond, -C(=O)- or -NR$^a$-;
preferably, $L^2$ is selected from -NR$^a$-.

**19.** The compound or tautomer, stereoisomer, solvate, metabolite, isotopically-labeled compound, pharmaceutically acceptable salt or co-crystal thereof according to any one of claims 1 to 18, wherein,

ring A is selected from nitrogen-containing $C_{3-10}$ heteroaryl or $C_{3-10}$ heterocycloalkyl, wherein, the heteroaryl, heterocycloalkyl are optionally substituted with one or more $R^4$; the heteroaryl, heterocycloalkyl contain 1 to 4 heteroatoms optionally selected from N, O or S;
preferably, ring A is selected from $C_{3-10}$ heterocycloalkyl, wherein, the heterocycloalkyl is optionally substituted with one or more $R^4$;
preferably, ring A is selected from:

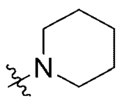, 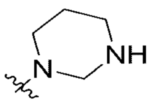, 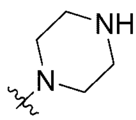, 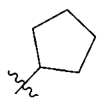 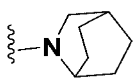,

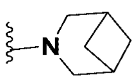, 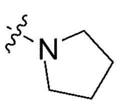, 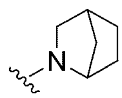, 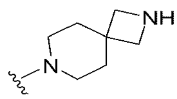, 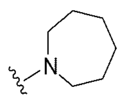

, wherein, the

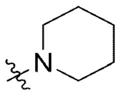, 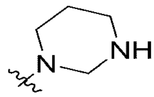,

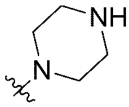, 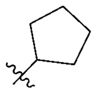, 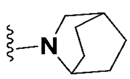, 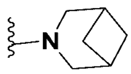, 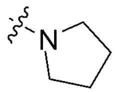, 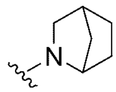, 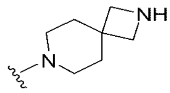, 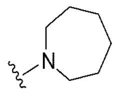

are optionally substituted with $R^4$;
preferably, ring A is selected from :

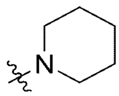, 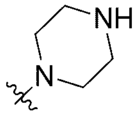, 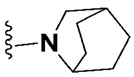, 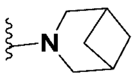, 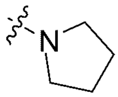,

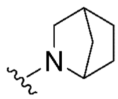, 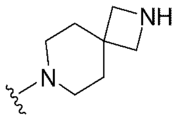, 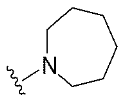,

wherein, the

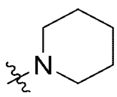, 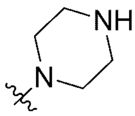, 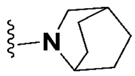, 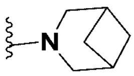,

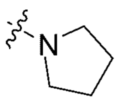, 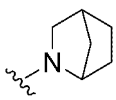, 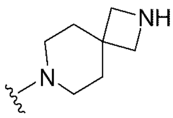, 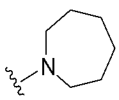

are optionally substituted with $R^4$;
preferably, ring A is selected from:

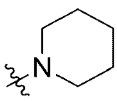, 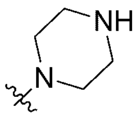, 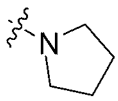, 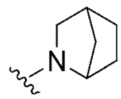, 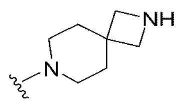,

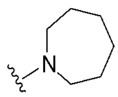,

wherein, the

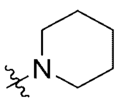, 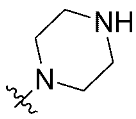, 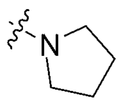, 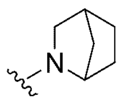, 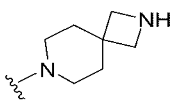, 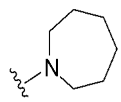

are optionally substituted with $R^4$;
preferably, ring A is selected from:

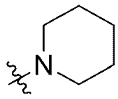, 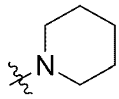

is optionally substituted with $R^4$.

**20.** The compound or tautomer, stereoisomer, solvate, metabolite, isotopically-labeled compound, pharmaceutically acceptable salt or co-crystal thereof according to any one of claims 1 to 19, wherein,

$R^4$ is each independently selected at each occurrence from hydrogen, halogen, CN, hydroxyl, $C_{1-6}$ alkyl, $C_{1-6}$ alkoxy, $C_{2-6}$alkenyl, $-NR^aR^b$;
preferably, $R^4$ is selected from hydrogen, $C_{1-6}$ alkyl, $-NR^aR^b$;
preferably, $R^4$ is selected from hydrogen, $C_{1-6}$ alkyl.

**21.** The compound or tautomer, stereoisomer, solvate, metabolite, isotopically-labeled compound, pharmaceutically acceptable salt or co-crystal thereof according to any one of claims 1 to 20, wherein,

$R^6$ is each independently selected at each occurrence from hydrogen, halogen, CN, hydroxyl, $C_{1-6}$ alkyl, $C_{1-6}$ alkyl-CN, halogen-substituted $C_{1-6}$ alkyl, $C_{1-6}$ alkoxy, $C_{3-6}$ cycloalkyl, $C_{2-6}$ alkenyl, $C_{2-6}$ alkynyl, COOH, $-NR^aR^b$, $-C(=O)NR^aR^b$;
preferably, $R^6$ is each independently selected at each occurrence from hydrogen, halogen, $CH_2$-CN, CN, $C_{1-6}$ alkyl, $C_{1-6}$ alkoxy, halogen-substituted $C_{1-6}$ alkyl;
preferably, $R^6$ is each independently selected at each occurrence from hydrogen, halogen, $CH_2$-CN, CN, $C_{1-6}$ alkyl;
preferably, $R^6$ is each independently selected at each occurrence from hydrogen, CN;
$R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and R’are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkoxy, halogen-substituted $C_{1-6}$ alkyl, hydroxyl-substituted $C_{1-6}$ alkyl, hydroxyl-substituted $C_{1-6}$ alkoxy, COOH, $-NR^aR^b$, $-S(=O)_2R^a$, $-C(=O)NR^aR^b$, 3- to 6-membered heterocycloalkyl, hydroxyl-substituted $C_{1-6}$ alkoxy-$C_{3-6}$ cycloalkyl, heterocycloalkenyl, the heteroaryl, heterocycloalkyl contain 1 to 4 heteroatoms optionally selected from N, O or S;
preferably, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, hydroxyl, $C_{1-6}$ alkyl, $C_{1-6}$ alkoxy, heterocycloalkenyl, the heteroaryl, heterocycloalkyl contain 1 to 4 heteroatoms optionally selected from N, O or S;
preferably, $R^3$, $R^7$ are each independently selected from hydrogen, $C_{1-6}$ alkyl, $C_{1-6}$ alkoxy, $C_{6-10}$ aryl or $C_{6-10}$ heteroaryl, and $R^3$ and $R^7$ are not both hydrogen, wherein, the aryl or heteroaryl is optionally substituted with one or more substituents selected from hydrogen, halogen, CN, $CF_3$, hydroxyl, $C_{1-6}$ alkyl, $C_{1-6}$ alkoxy, $C_{2-6}$ alkenyl, $C_{2-6}$ alkynyl, halogen-substituted $C_{1-6}$ alkoxy, halogen-substituted $C_{1-6}$ alkyl, hydroxyl-substituted $C_{1-6}$ alkoxy, hydroxyl-substituted $C_{1-6}$ alkoxy-$C_{3-6}$ cycloalkyl, COOH, $-NR^aR^b$, $- S(=O)_2R^a$, $-C(=O)NR^aR^b$, 3- to 6-membered heterocycloalkyl, heterocycloalkenyl, the heteroaryl, heterocycloalkyl contain 1 to 4 heteroatoms optionally selected from N, O or S;
preferably, $R^3$, $R^7$ are selected from hydrogen,

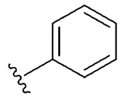, 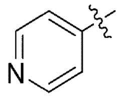, 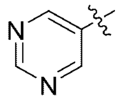, 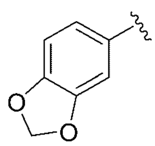,

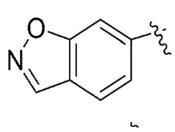, 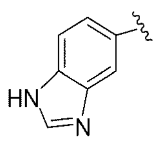, 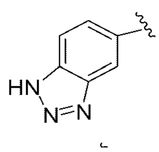, 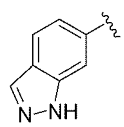,

the

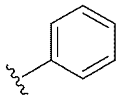, 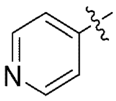, 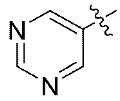,

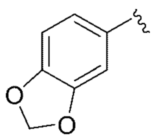, 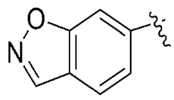, 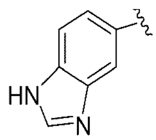, 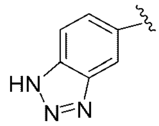, 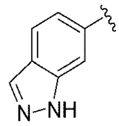

are optionally substituted with one or more substituents selected from hydrogen, halogen, CN, heterocycloalkenyl, hydroxyl, $CF_3$, $C_{1-6}$ alkyl, $C_{1-6}$ alkoxy, $C_{3-6}$ cycloalkyl, halogen-substituted $C_{1-6}$ alkoxy, 3- to 6-membered heterocycloalkyl, hydroxyl-substituted $C_{1-6}$ alkoxy-$C_{3-6}$ cycloalkyl, hydroxyl-substituted $C_{1-6}$ alkyl, hydroxyl-substituted $C_{1-6}$ alkoxy, $-NH_2$, $-N(C_{1-6}\text{ alkyl})_2$, $-NH(C_{1-6}\text{ alkyl})$;
preferably, $R^3$, $R^7$ are selected from hydrogen,

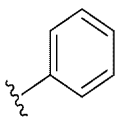, 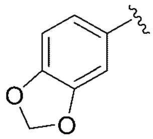, 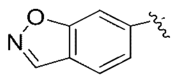, 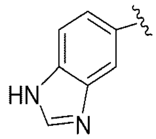,

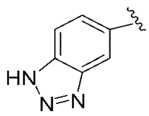, 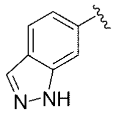, the 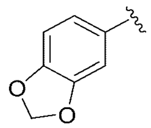, 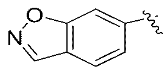, 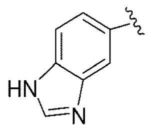, 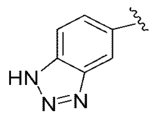, 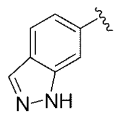

are optionally substituted with one or more substituents selected from hydrogen, halogen, CN, hydroxyl, $CF_3$, $C_{1-6}$ alkyl, $C_{1-6}$ alkoxy, $C_{3-6}$ cycloalkyl, halogen-substituted $C_{1-6}$ alkoxy, 3- to 6-membered heterocycloalkyl, hydroxyl-substituted $C_{1-6}$ alkoxy-$C_{3-6}$ cycloalkyl, hydroxyl-substituted $C_{1-6}$ alkyl, hydroxyl-substituted $C_{1-6}$ alkoxy, $-NH_2$, $-N(C_{1-6}\text{ alkyl})_2$, $-NH(C_{1-6}\text{ alkyl})$;
preferably, $R^3$, $R^7$ are selected from hydrogen,

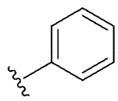,

the

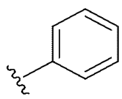

is optionally substituted with one or more substituents selected from hydrogen, halogen, heterocycloalkenyl, hydroxyl.

**22.** The compound or tautomer, stereoisomer, solvate, metabolite, isotopically-labeled compound, pharmaceutically acceptable salt or co-crystal thereof according to any one of claims 1 to 21, wherein,

$R^5$ is each independently selected at each occurrence from hydrogen, halogen, CN, hydroxyl, $C_{1-6}$ alkyl, $C_{1-6}$ alkoxy, =O, $C_{2-6}$ alkenyl, $C_{6-10}$ aryl or $C_{6-10}$ heteroaryl, wherein, the alkyl, alkoxy, alkenyl, aryl or heteroaryl is optionally substituted with one or more substituents selected from hydrogen, halogen, CN, $NO_2$, $CF_3$, $CHF_2$, hydroxyl, $C_{1-6}$ alkyl, $C_{1-6}$ alkoxy, $-C(=O)-C_{1-6}$ alkoxy, $C_{2-6}$ alkenyl, $-C(=O)-NH_2$, the heteroaryl, heterocycloalkyl contain 1 to 4 heteroatoms optionally selected from N, O or S;

preferably, $R^5$ is each independently selected at each occurrence from hydrogen, CN, $C_{1-6}$ alkoxy, =O, $C_{6-10}$ aryl or $C_{6-10}$ heteroaryl, wherein, the alkoxy, aryl or heteroaryl is optionally substituted with one or more substituents selected from hydrogen, halogen, CN, $NO_2$, $CF_3$, $CHF_2$, hydroxyl, $C_{1-6}$ alkyl, -C(=O)-$C_{1-6}$ alkoxy, -C(=O)-$NH_2$, the heteroaryl, heterocycloalkyl contain 1 to 4 heteroatoms optionally selected from N, O or S;
alternatively, $R^5$, $R^6$ and the atom to which both of them directly connect collectively form cycloalkyl, heteroalicyclic, aryl or heteroaryl, wherein, the cycloalkyl, heteroalicyclic, aryl, heteroaryl are optionally substituted with one or more substituents selected from hydrogen, halogen, CN, hydroxyl, $C_{1-6}$ alkyl, $C_{1-6}$ alkoxy, $C_{2-6}$ alkenyl, $C_{2-6}$ alkynyl;
preferably, $R^5$ is each independently selected from CN, $C_{1-6}$ alkoxy, =O,

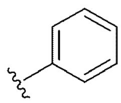, 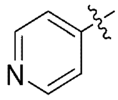, 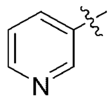,

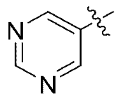, 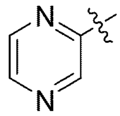

, wherein, the alkoxy,

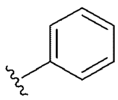, 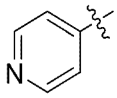, 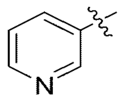, 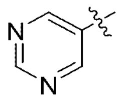, 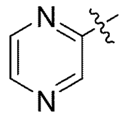

are optionally substituted with one or more substituents selected from hydrogen, halogen, CN, $NO_2$, $CF_3$, $CHF_2$, hydroxyl, $C_{1-6}$alkyl, -C(=O)-$NH_2$, -C(O)$OCH_3$;
alternatively, $R^5$, $R^6$ and the atom to which both of them directly connect collectively form

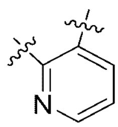,

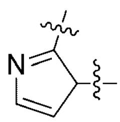, 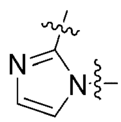

, wherein, the

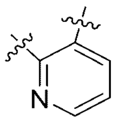, 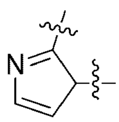, 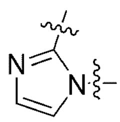

are optionally substituted with one or more groups selected from hydrogen, halogen, CN, hydroxyl, $C_{1-6}$ alkyl, $C_{1-6}$ alkoxy;
preferably, $R^5$ is selected from $C_{1-6}$ alkoxy,

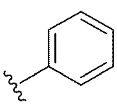,

wherein, the

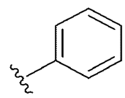

is optionally substituted with one or more substituents selected from hydrogen, halogen, CN, $NO_2$; alternatively, $R^5$, $R^6$ and the atom to which both of them directly connect collectively form

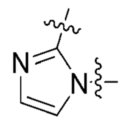

**23.** The compound or tautomer, stereoisomer, solvate, metabolite, isotopically-labeled compound, pharmaceutically acceptable salt or co-crystal thereof according to any one of claims 1 to 22, wherein,

X, Y are each independently selected from C, N;
Z is selected from a bond, $-CH_2-$, -C(=O) or $-S(=O)_2-$;
preferably, Z is selected from a bond, $-CH_2-$ or -C(=O);
preferably, Z is selected from a bond.

**24.** The compound or tautomer, stereoisomer, solvate, metabolite, isotopically-labeled compound, pharmaceutically acceptable salt or co-crystal thereof according to any one of claims 1 to 23, wherein,

$R^a$, $R^b$ are each independently selected at each occurrence from hydrogen, hydroxyl, $C_{1-6}$ alkyl, $C_{1-6}$ alkoxy, $C_{2-6}$ alkenyl, $C_{2-6}$ alkynyl, $C_{3-6}$ cycloalkyl, halogen-substituted $C_{1-6}$ alkyl, the heteroaryl, heterocycloalkyl contain 1 to 4 heteroatoms optionally selected from N, O or S;
preferably, $R^a$, $R^b$ are each independently selected at each occurrence from hydrogen, $C_{1-6}$ alkyl;
preferably, $R^a$, $R^b$ are each independently selected at each occurrence from hydrogen.

**25.** The following compound or tautomer, stereoisomer, solvate, metabolite, isotopically-labeled compound, pharmaceutically acceptable salt or co-crystal thereof:

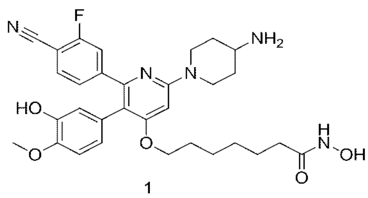
1

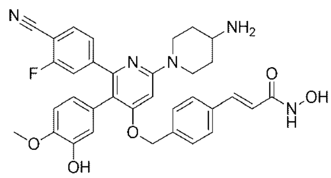
2

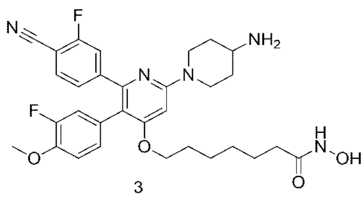
3

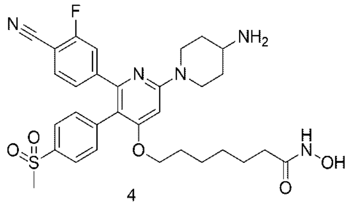
4

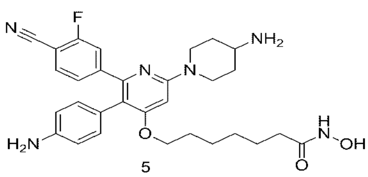
5

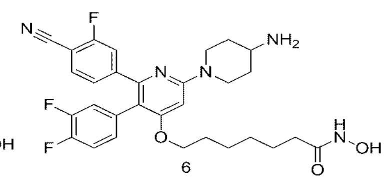
6

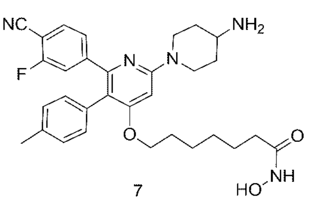
7

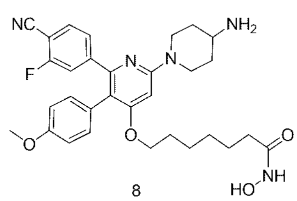
8

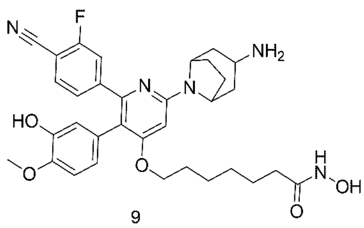
9

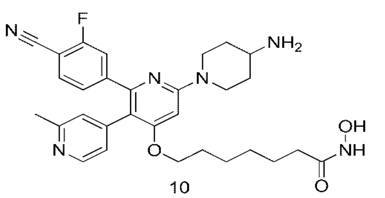
10

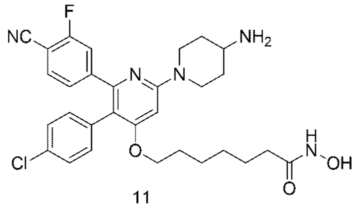
11

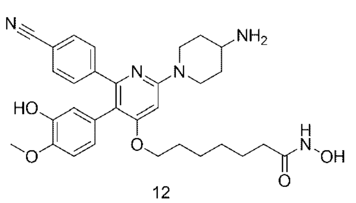
12

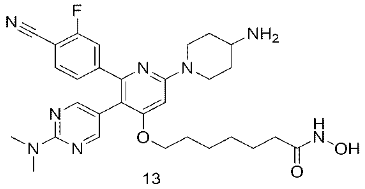
13

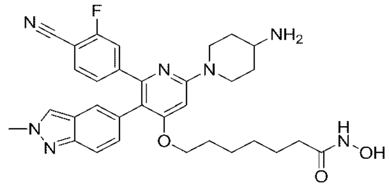
14

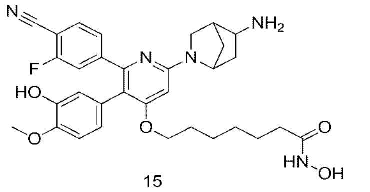
15

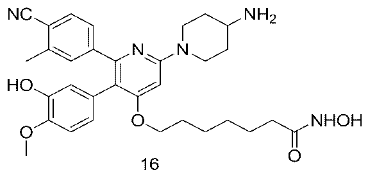
16

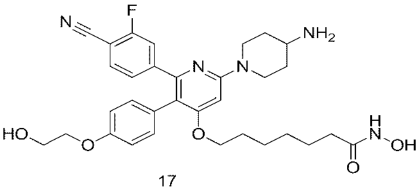
17

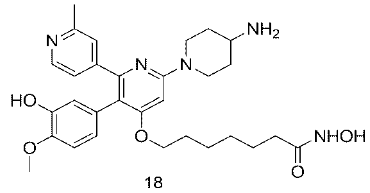
18

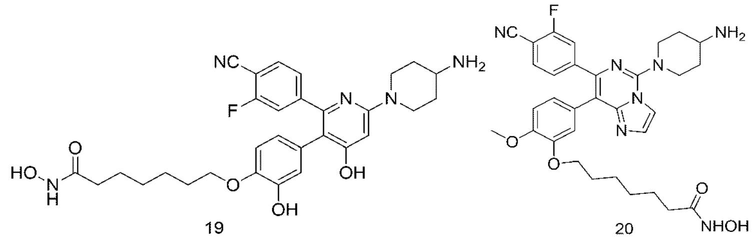
19

20

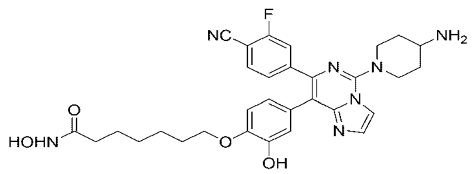
21

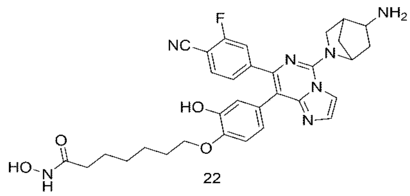
22

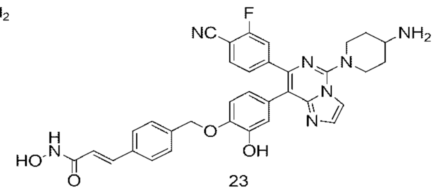
23

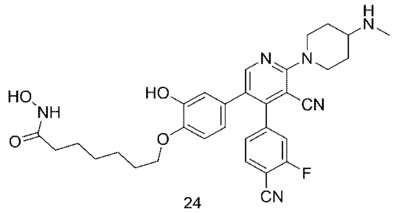
24

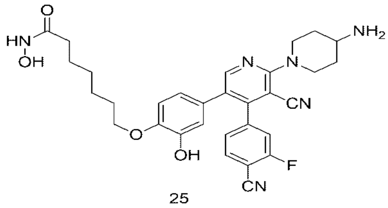

25

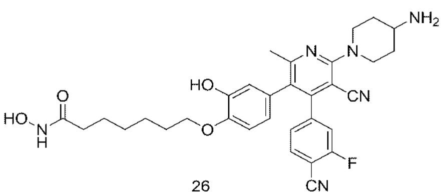

26

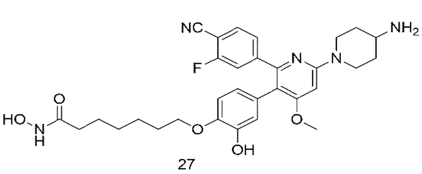

27

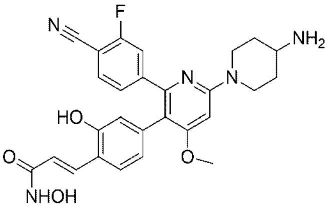

28

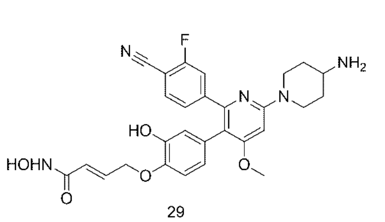

29

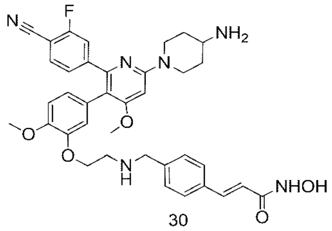

30

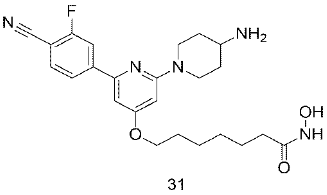

31

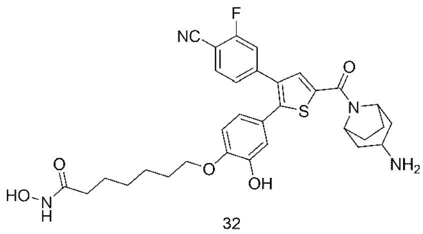

32

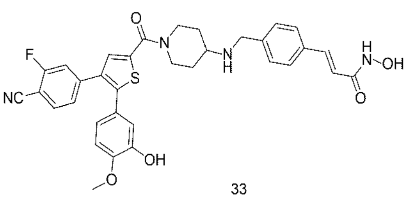

33

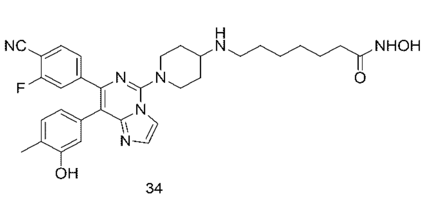

34

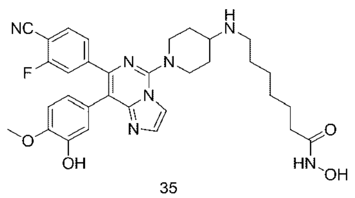

35

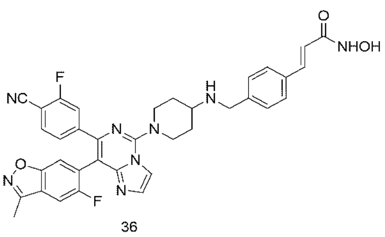

36

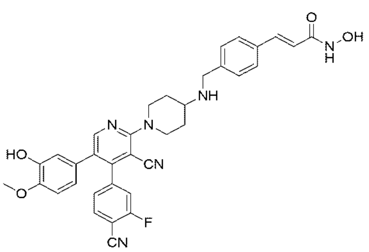

37

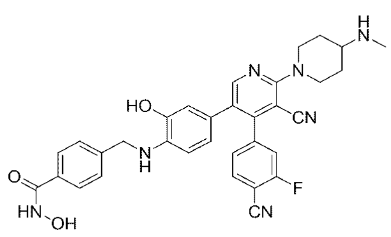

39

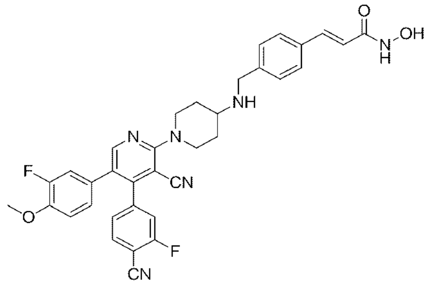
40

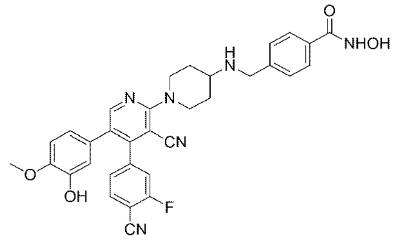
41

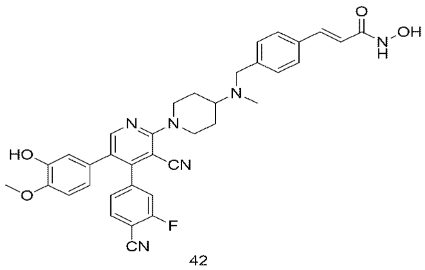
42

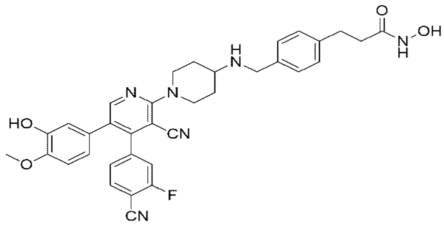
43

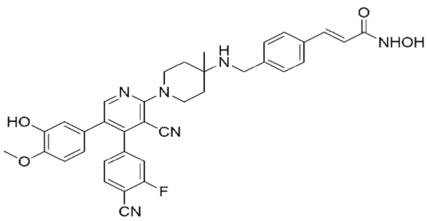
44

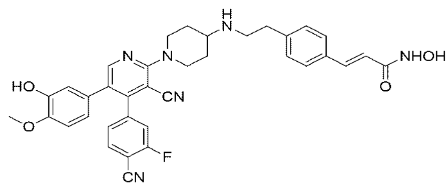
45

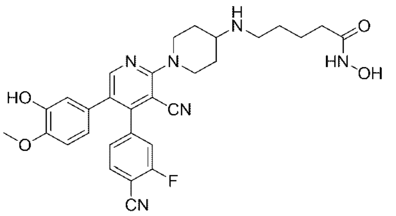
46

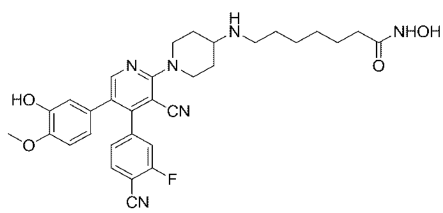
47

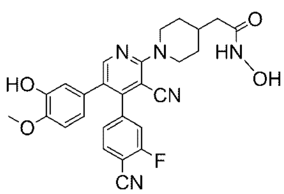
48

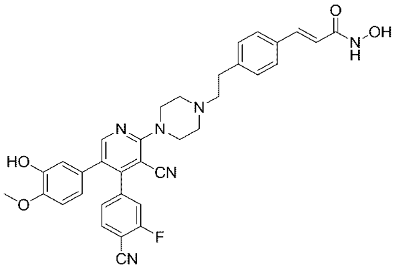
49

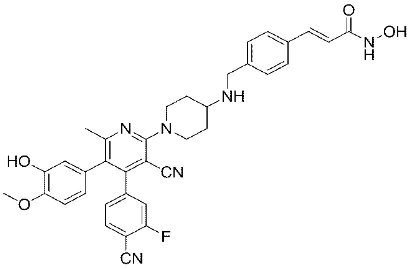
50

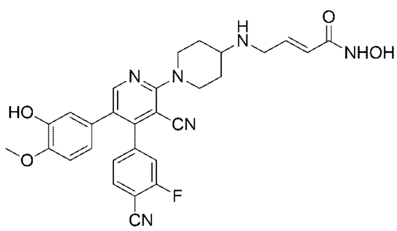
51

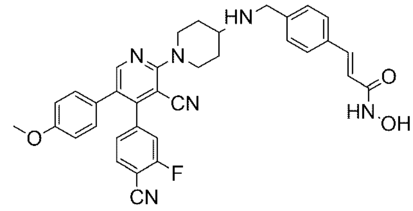

52

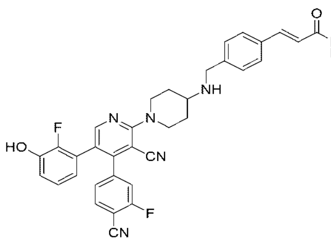

53

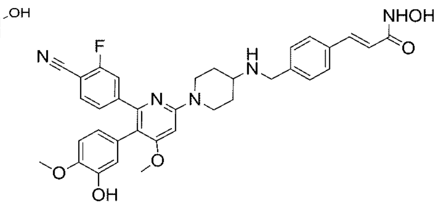

54

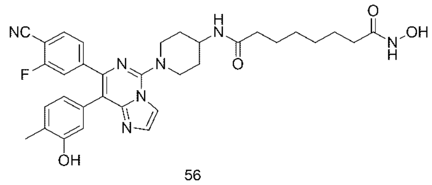

56

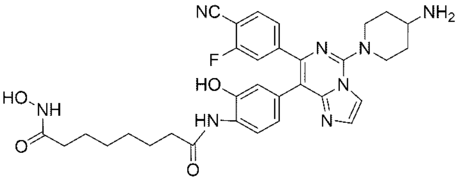

57

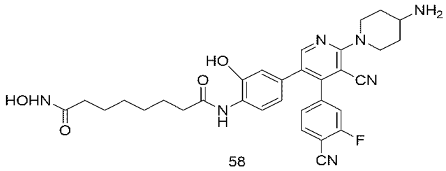

58

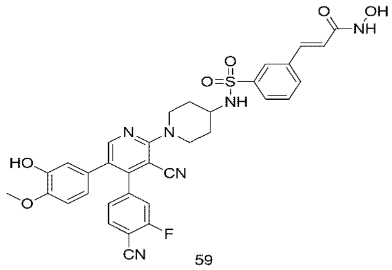

59

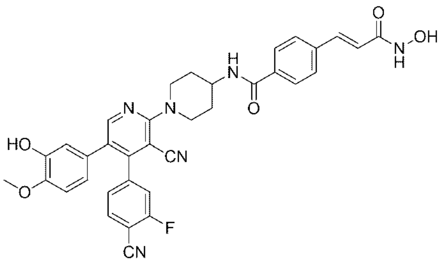

60

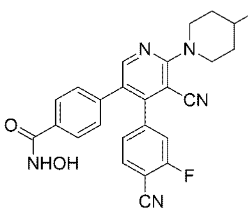

61

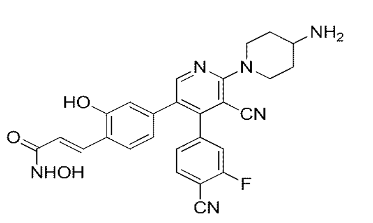

62

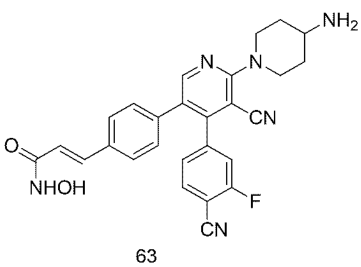

63

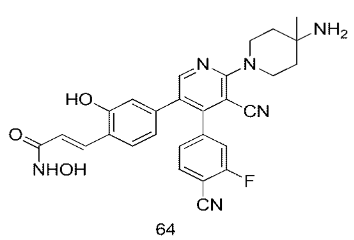

64

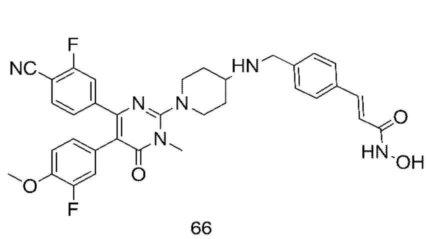

66

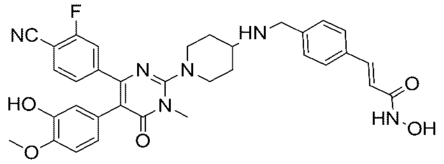

67

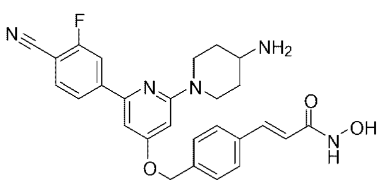

69

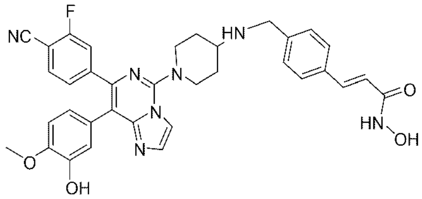

70

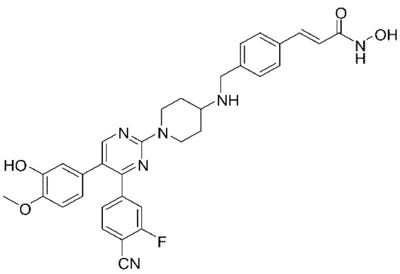

71

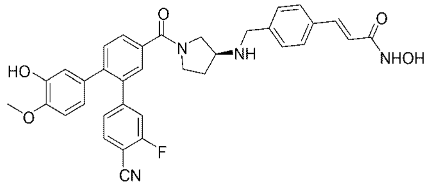

72

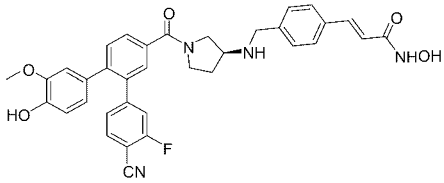

73

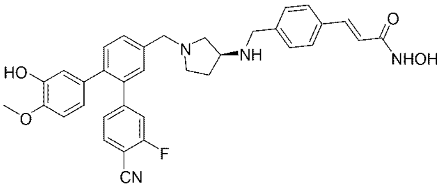

74

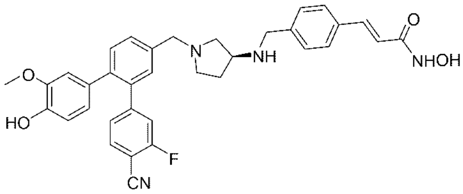

75

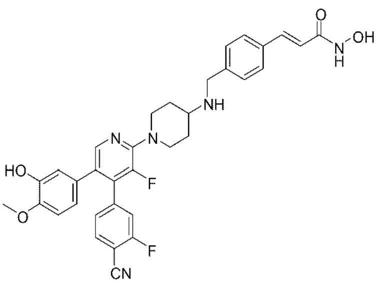

76

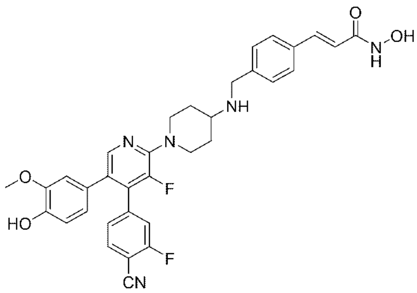

77

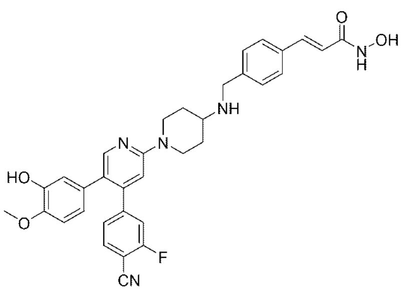

80

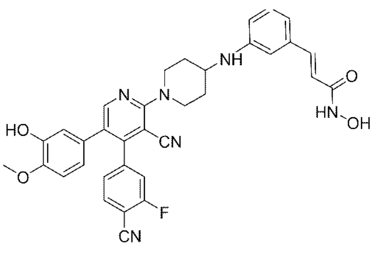

81

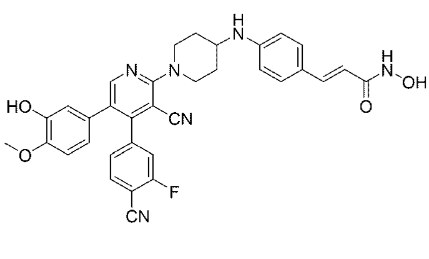

82

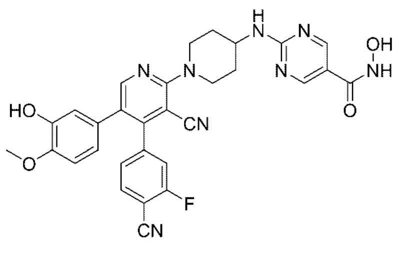

83

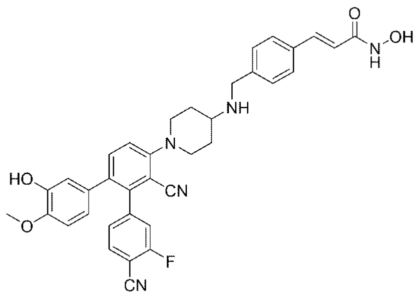

84

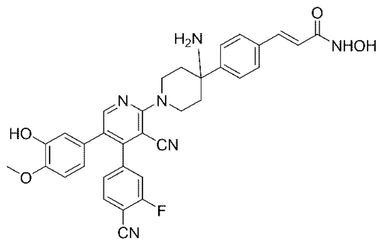

85

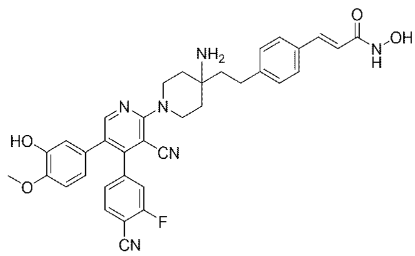

86

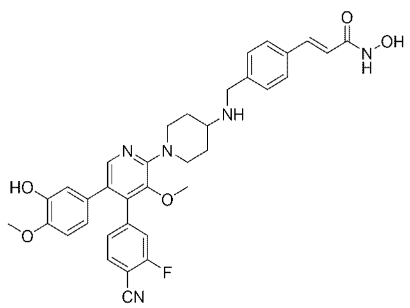

88

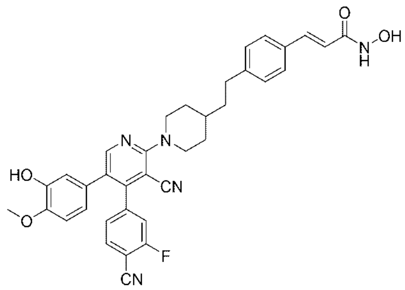

89

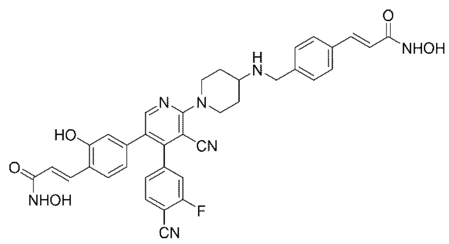

90

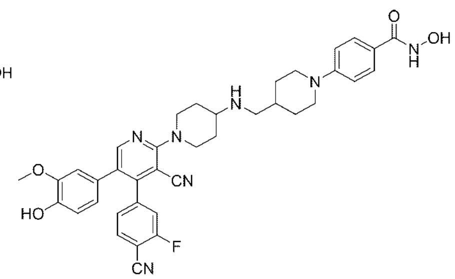

91

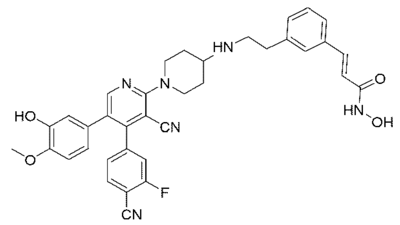

92

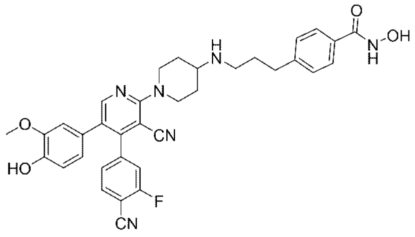

93

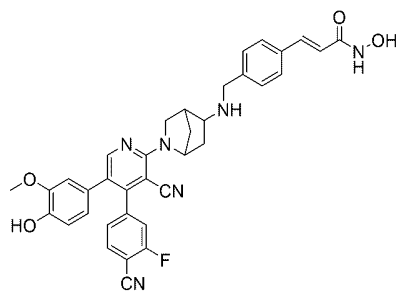

94

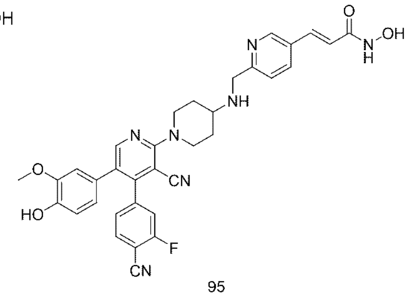

95

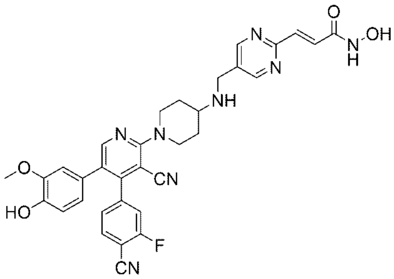

96

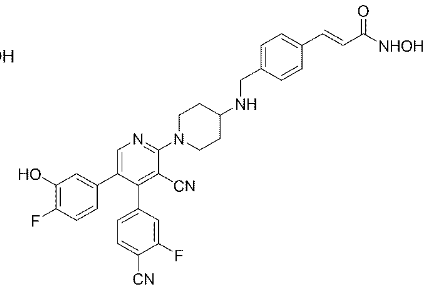

97

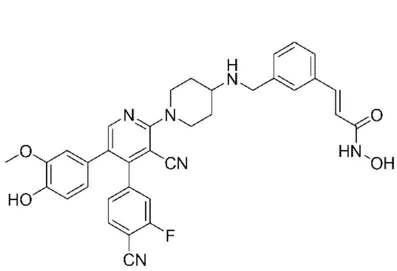

98

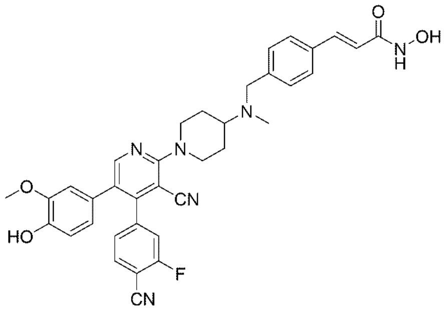

99

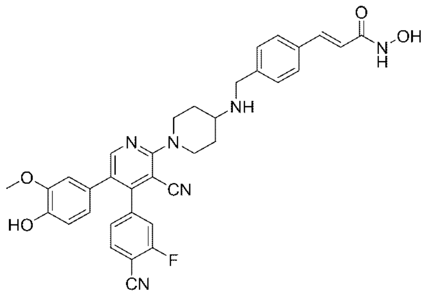

100

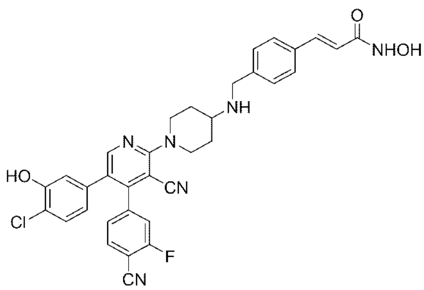

101

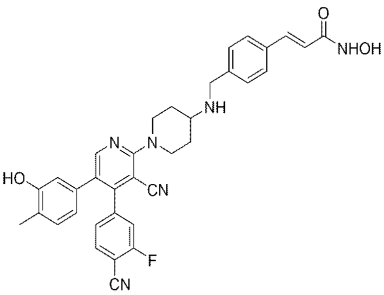

102

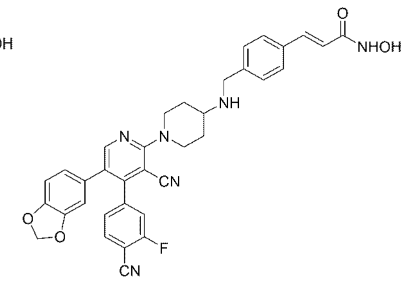

103

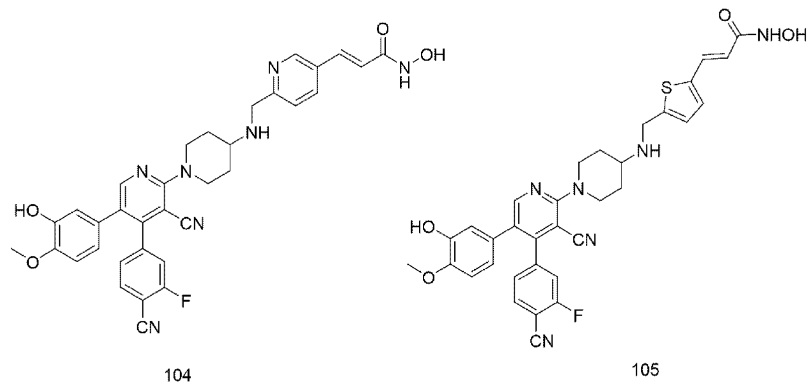

104

105

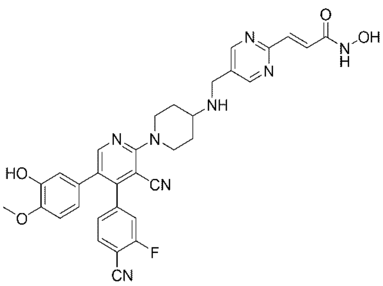

106

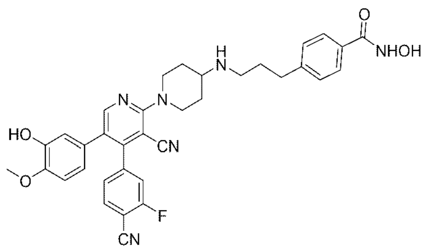

107

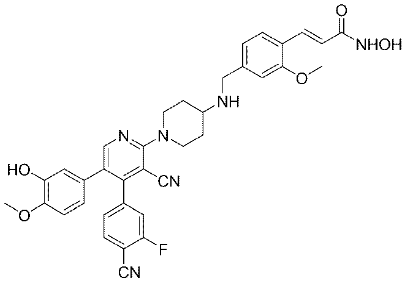

108

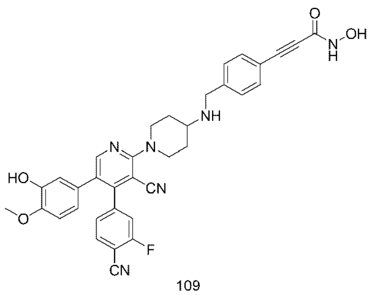

109

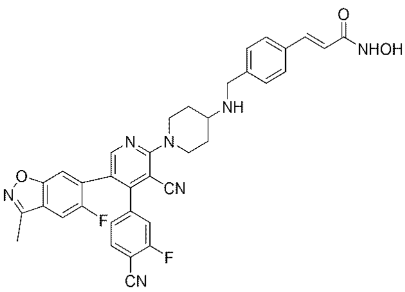

110

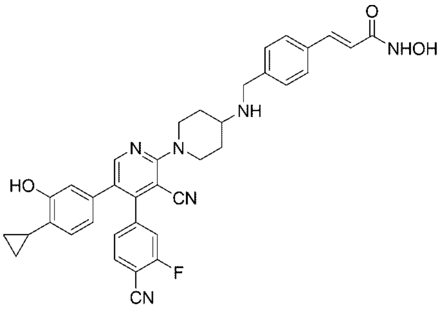

111

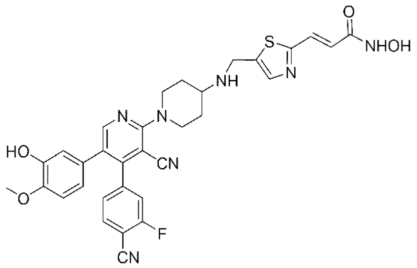

112

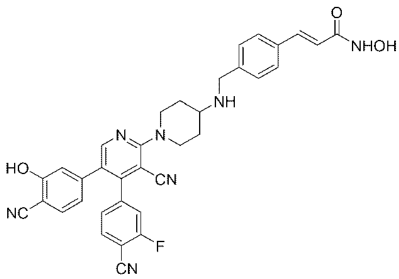

113

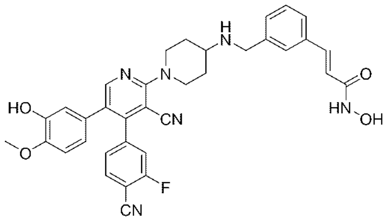

114

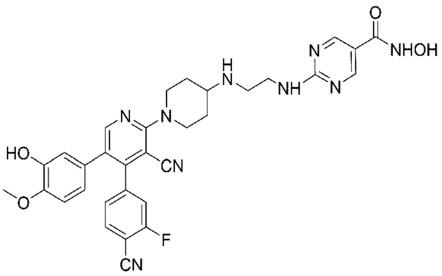

115

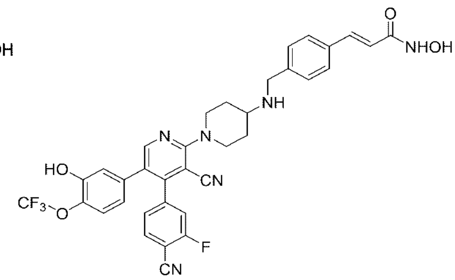

116

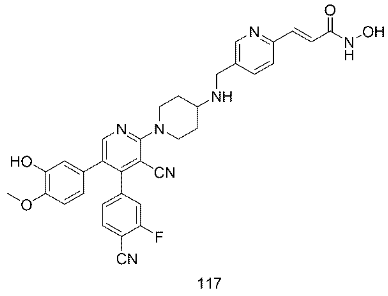

117

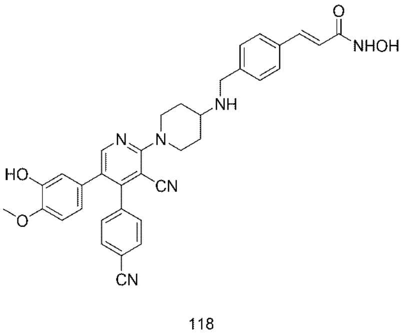

118

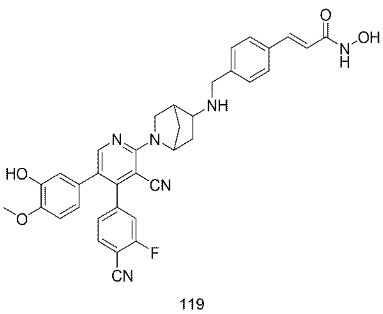

119

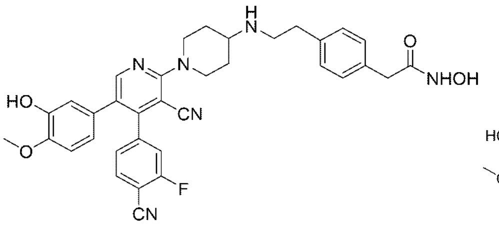

120

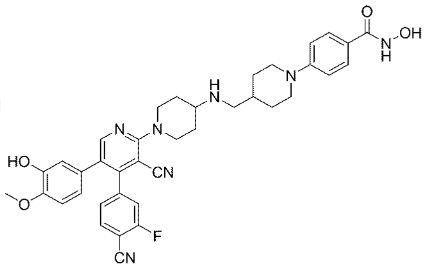

121

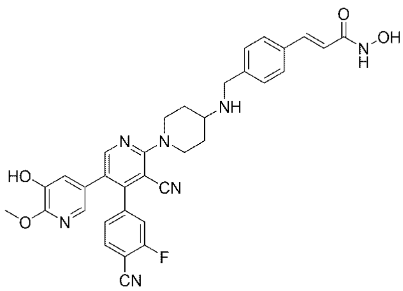

123

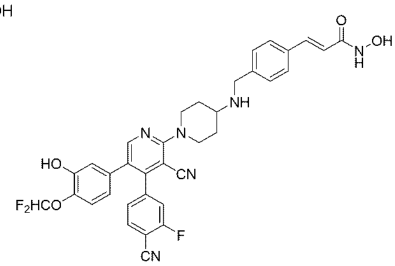

124

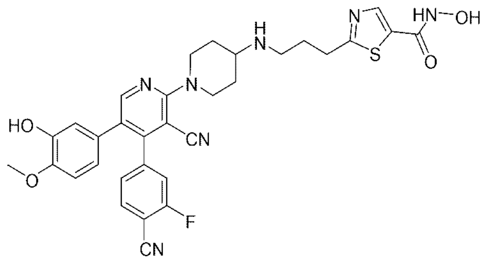

125

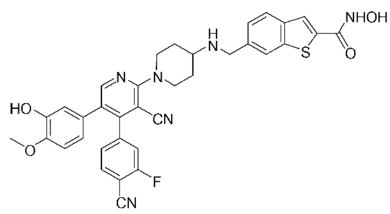

126

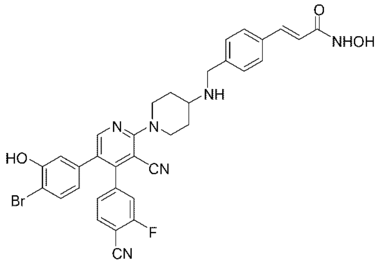

127

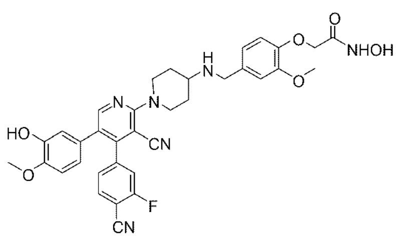

128

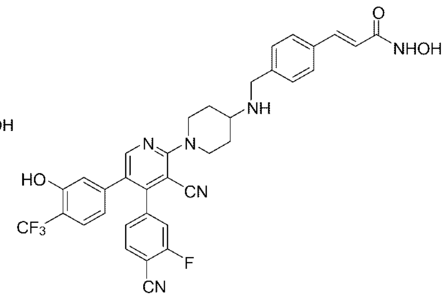

129

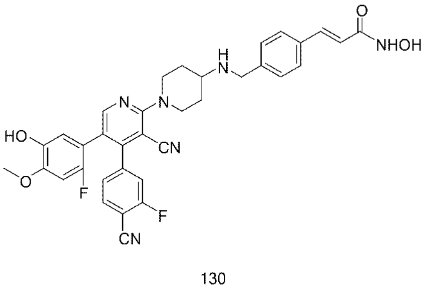

130

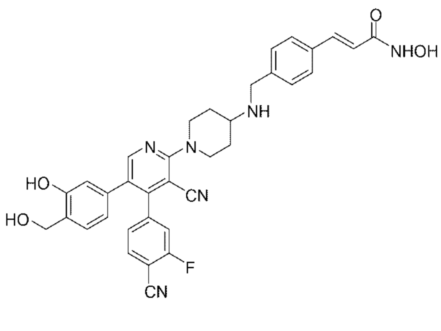

131

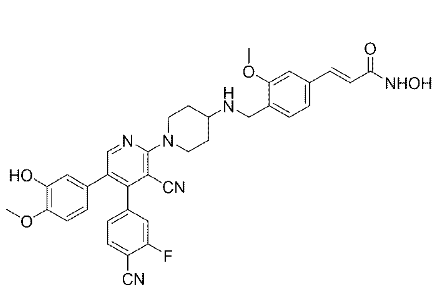
132

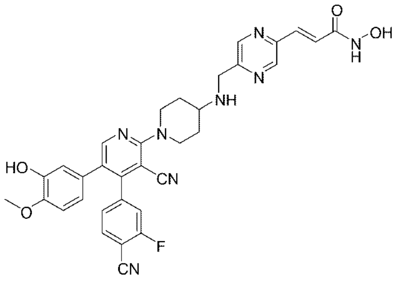
133

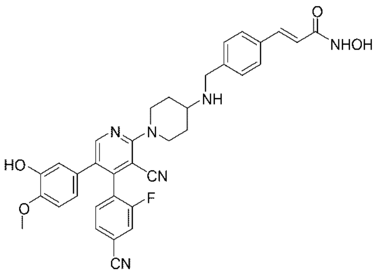
134

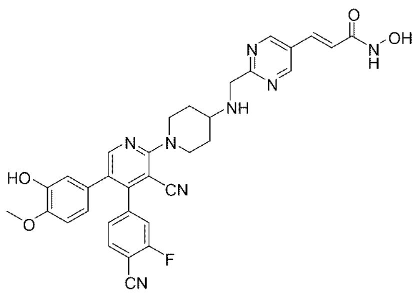
135

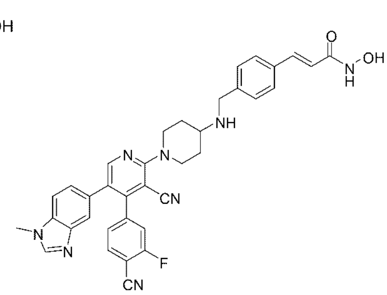
136

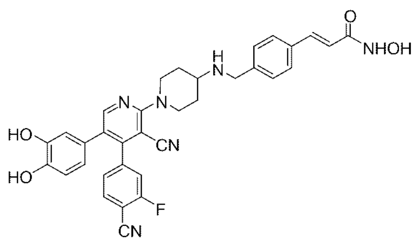
137

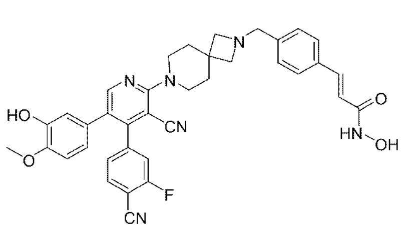
138

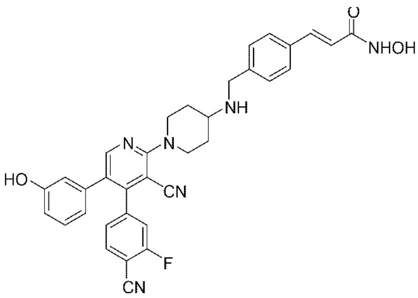
139

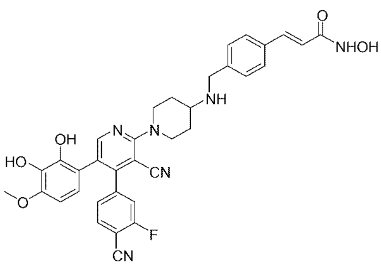
140

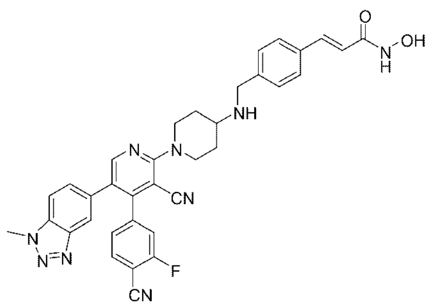
141

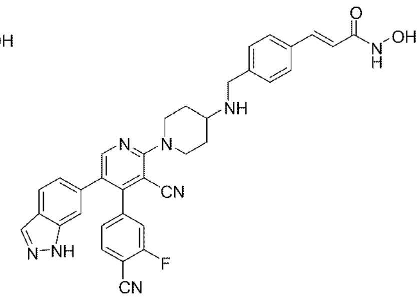
142

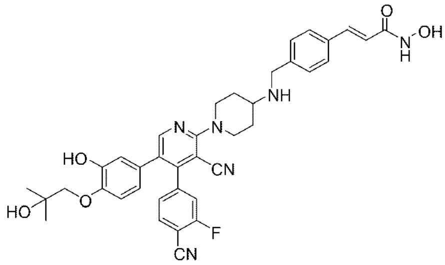
143

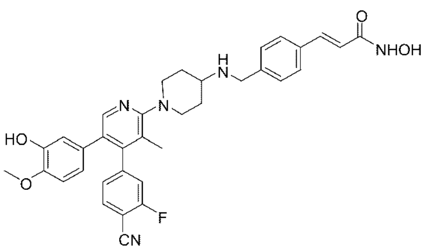
144

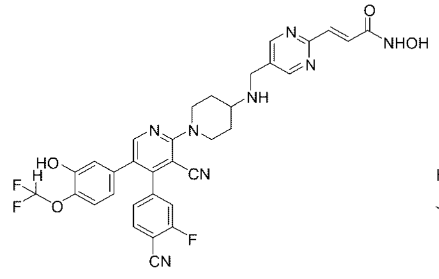

145

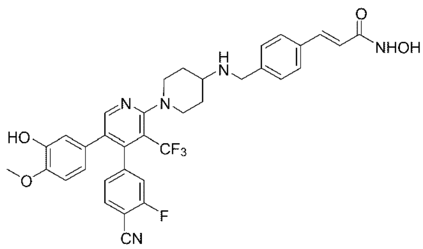

147

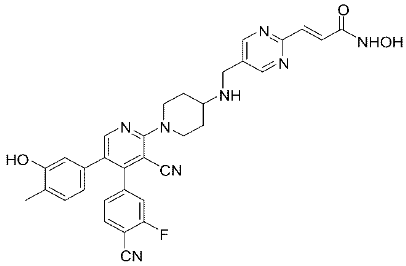

148

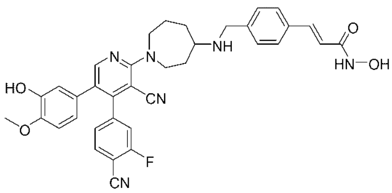

149

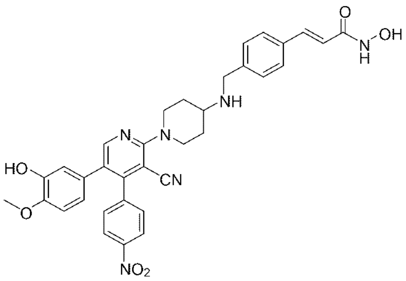

150

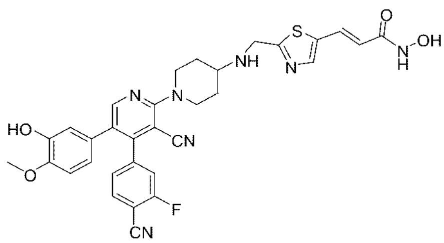

151

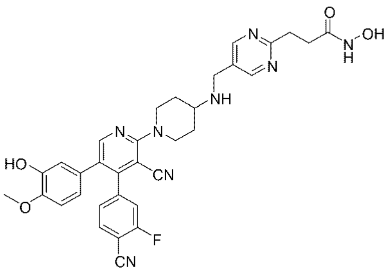

152

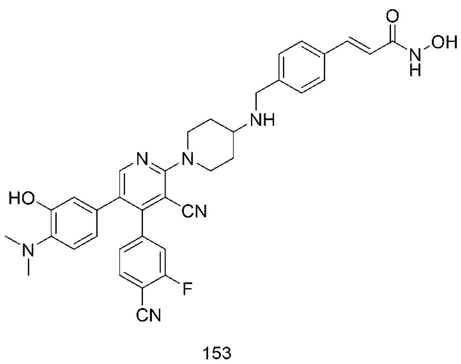

153

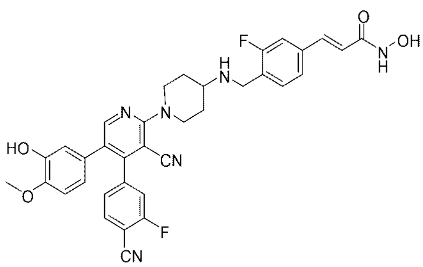

154

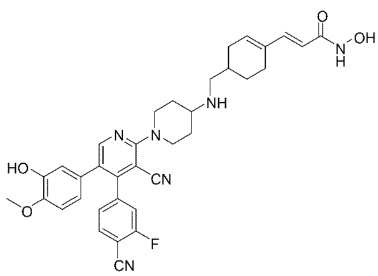

155

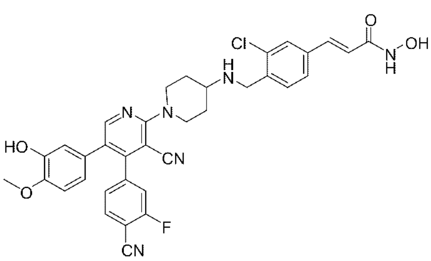

156

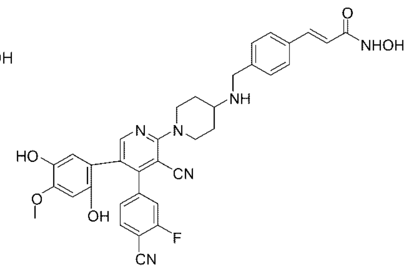

158

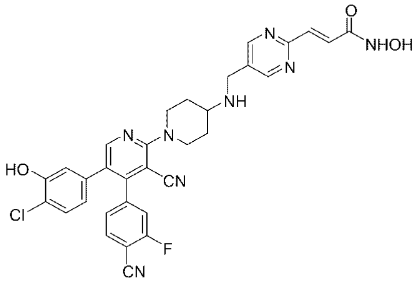

159

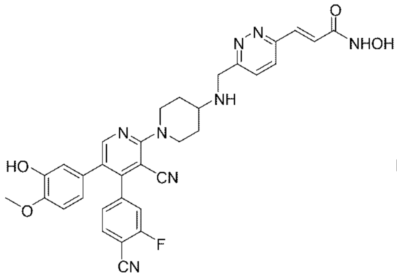

160

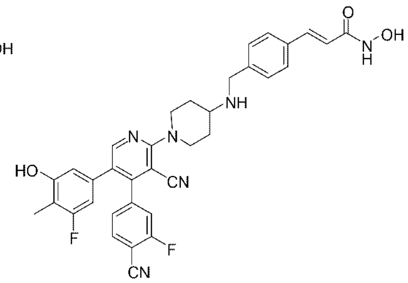

161

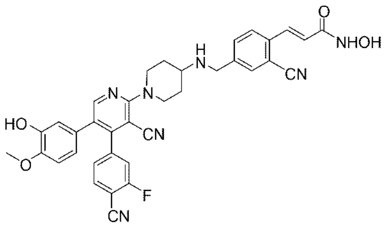

162

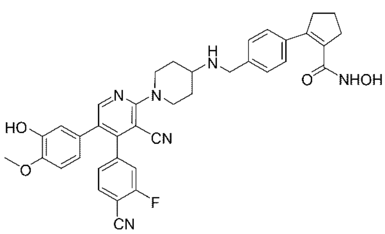

163

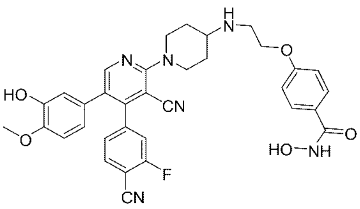

164

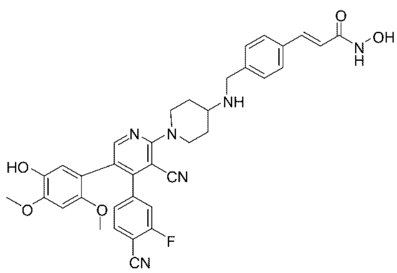

165

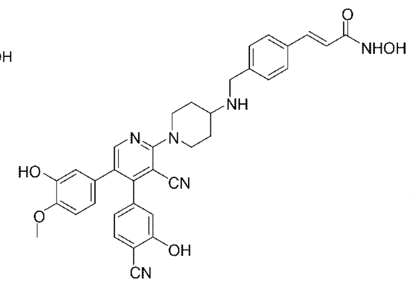

166

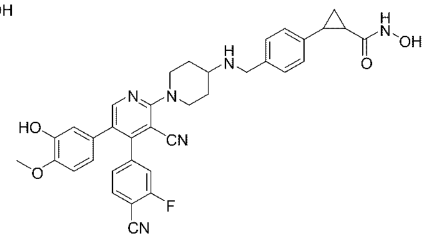

167

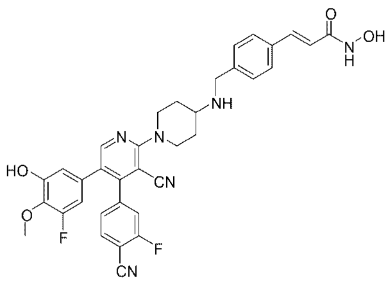

168

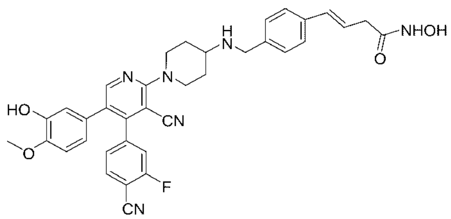

169

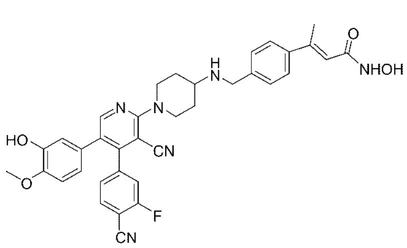

170

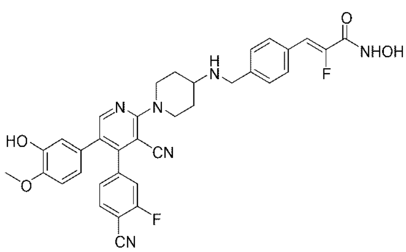

171

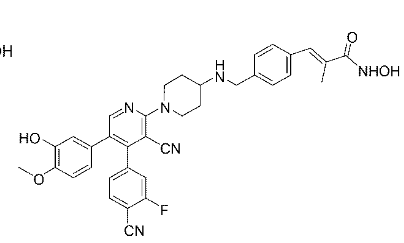

172

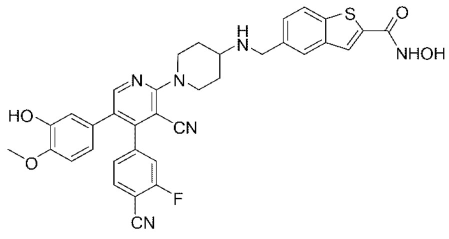

173

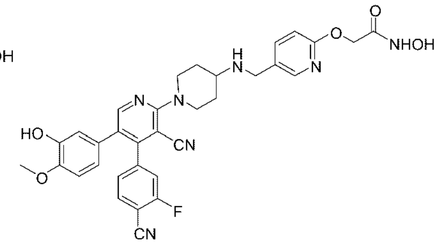

174

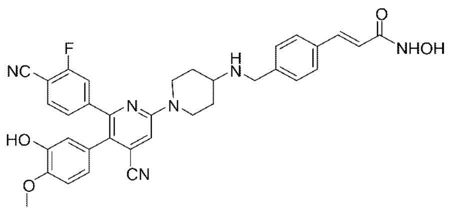

176

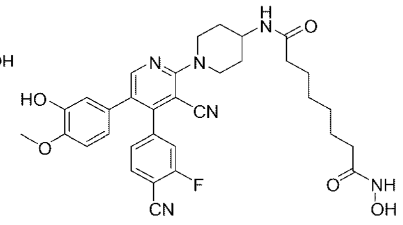

177

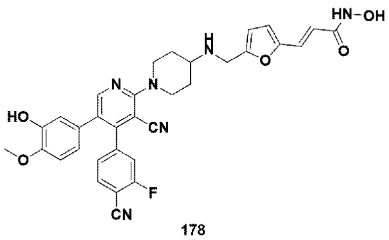

178

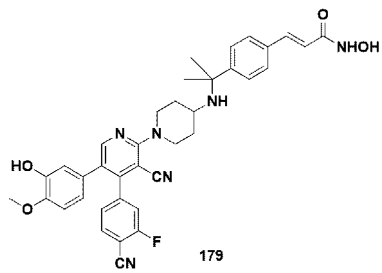

179

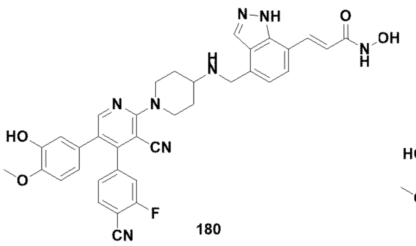

180

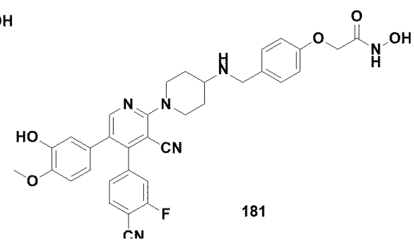

181

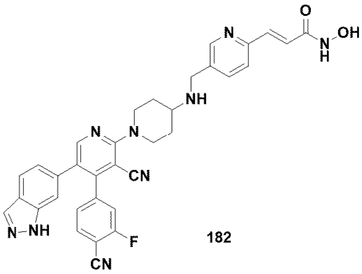

182

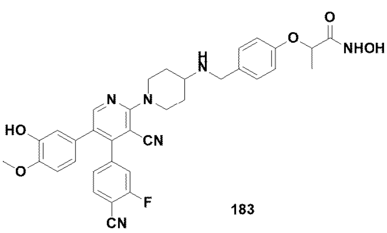

183

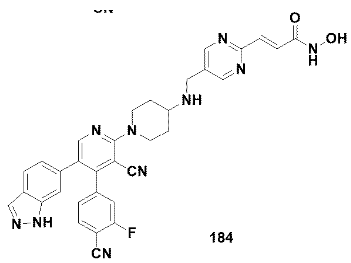

184

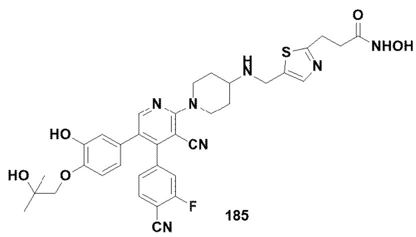

185

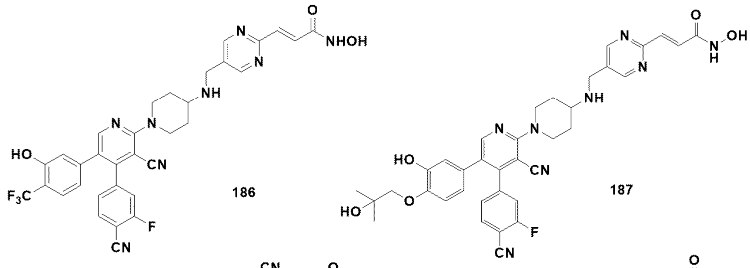

186

187

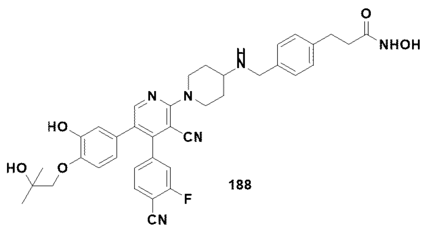

188

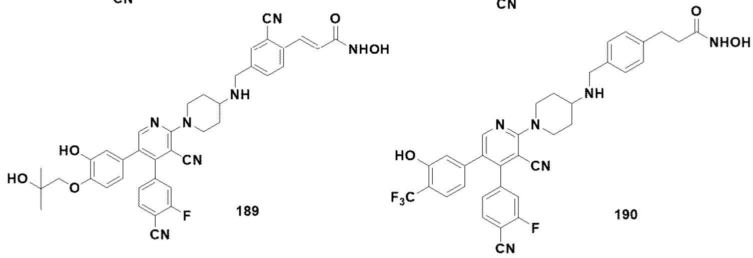

189

190

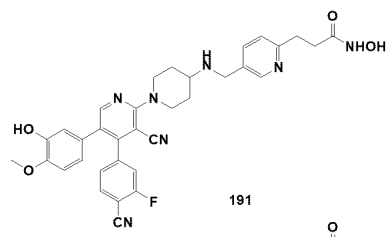

191

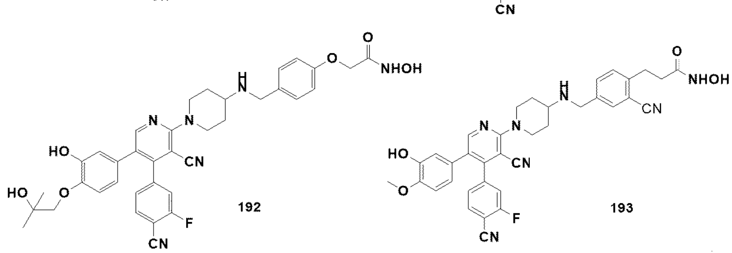

192

193

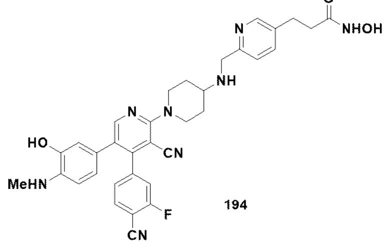

194

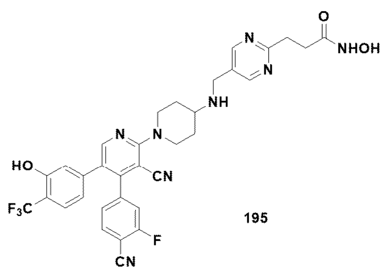

195

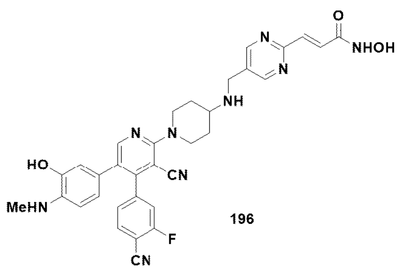

196

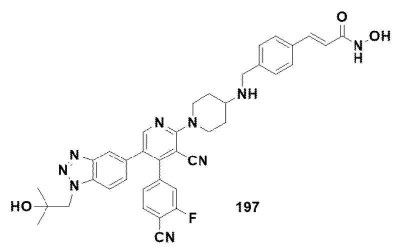

197

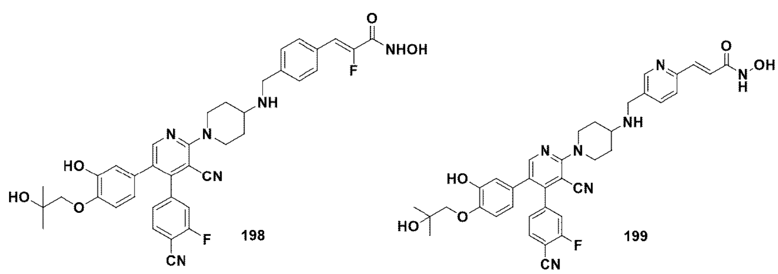

198

199

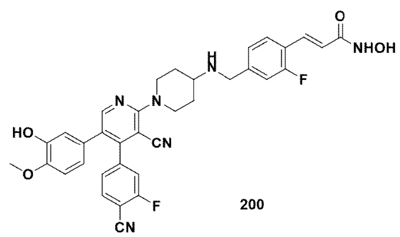

200

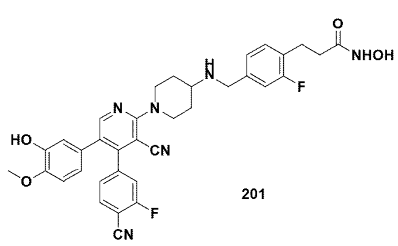

201

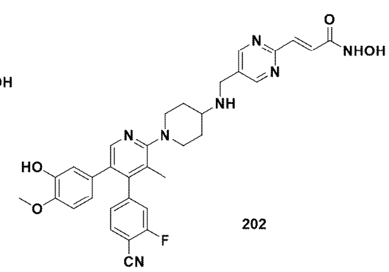

202

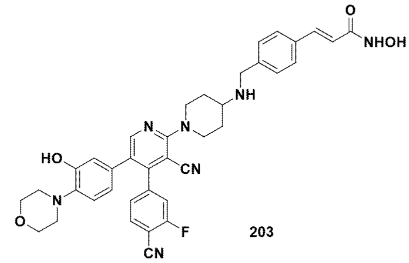

203

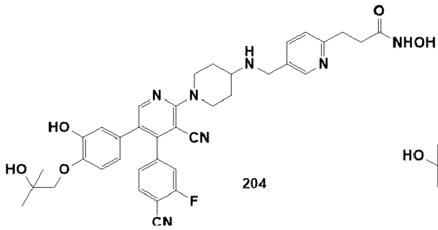

204

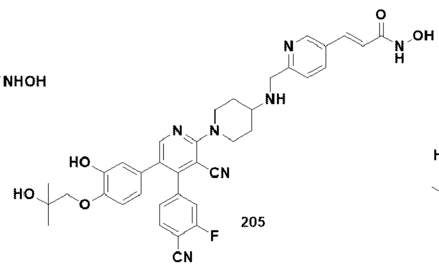

205

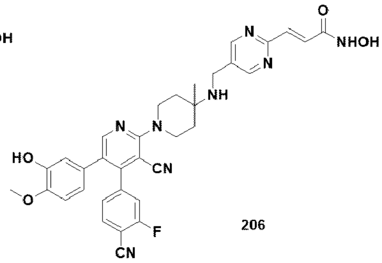

206

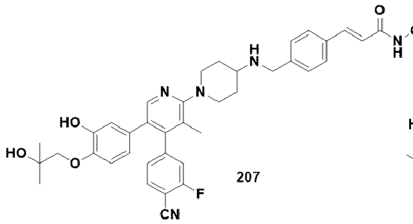

207

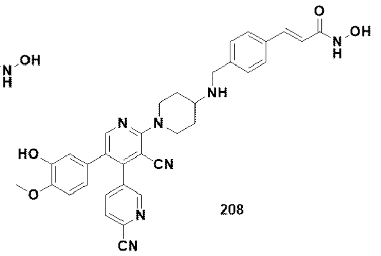

208

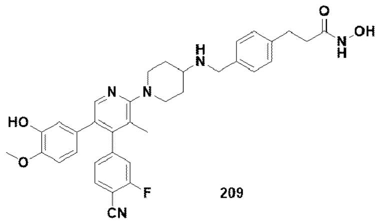

209

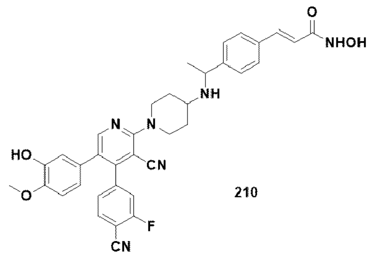

210

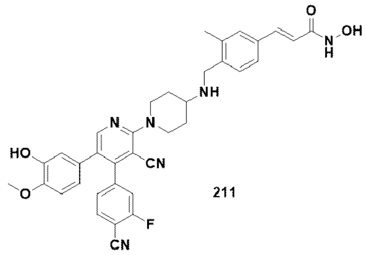

211

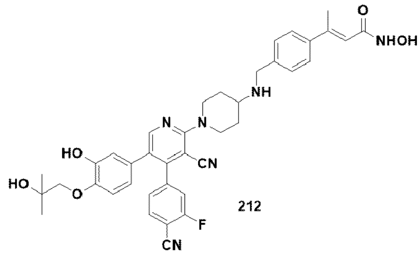

212

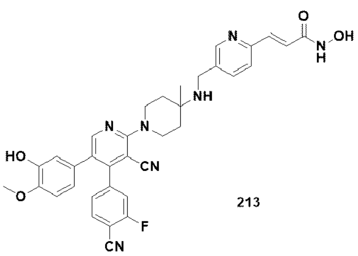

213

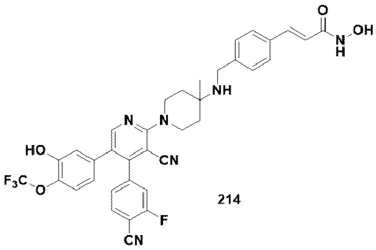

214

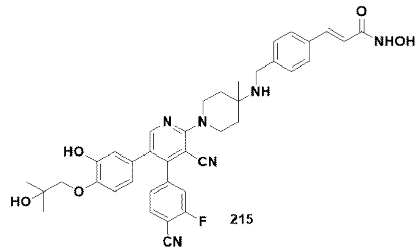

215

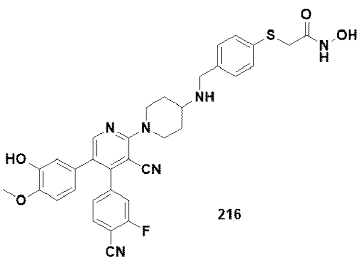

216

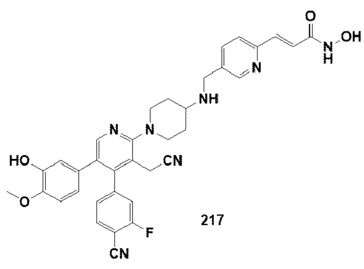

217

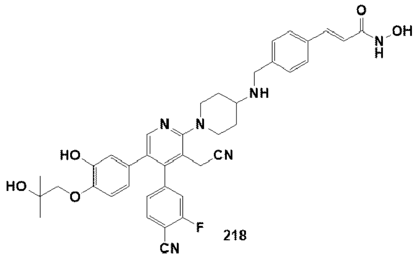

218

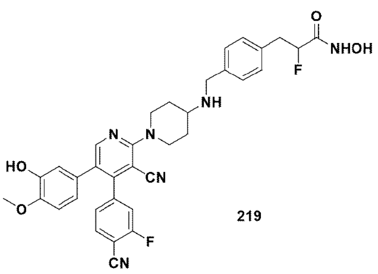

219

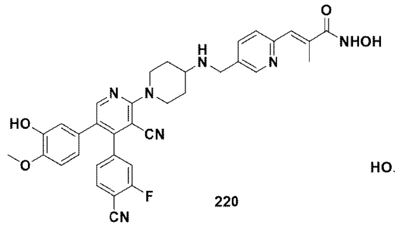

220

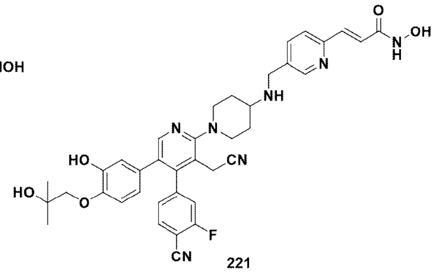

221

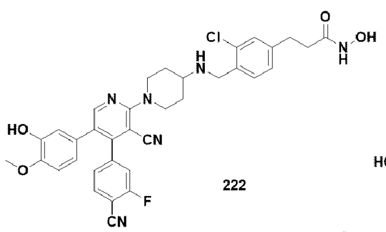
222

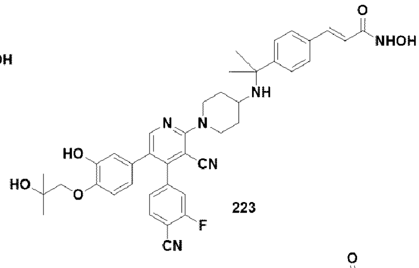
223

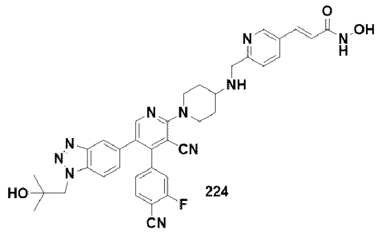
224

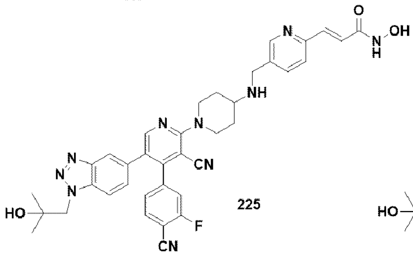
225

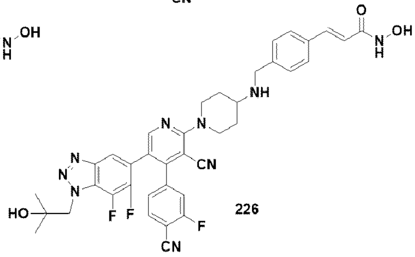
226

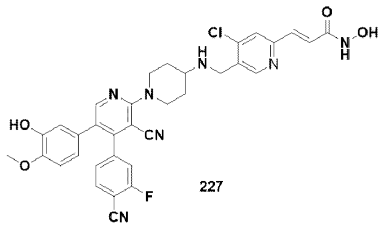
227

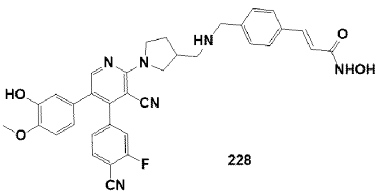
228

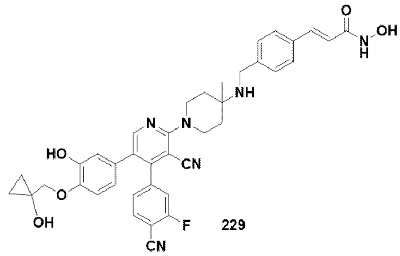
229

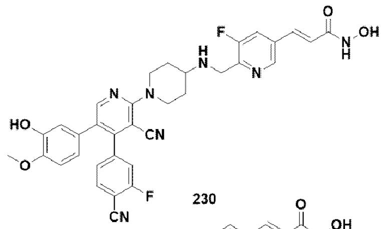
230

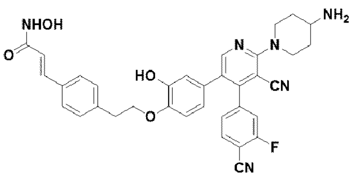
231

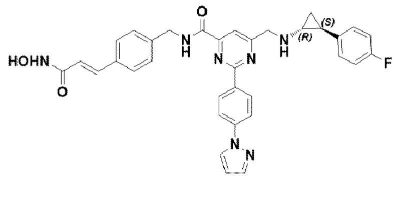
232

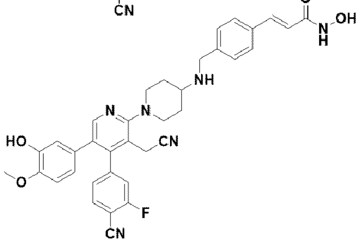
233

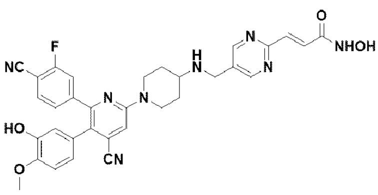
234

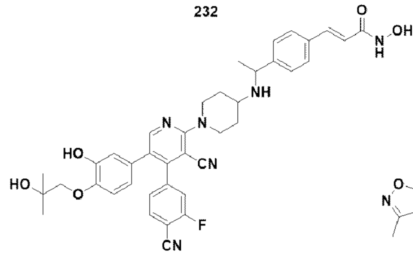
235

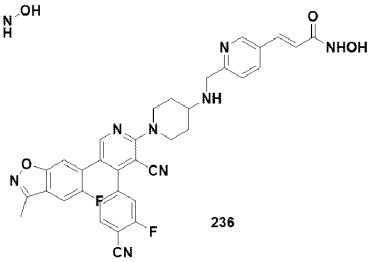
236

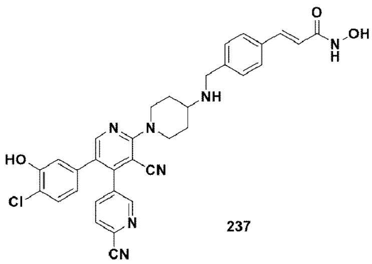
237

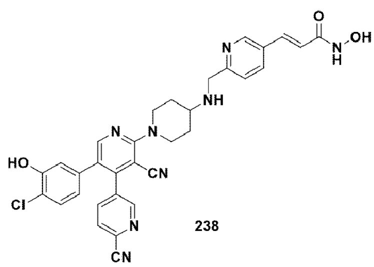
238

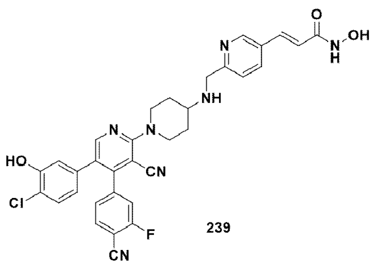
239

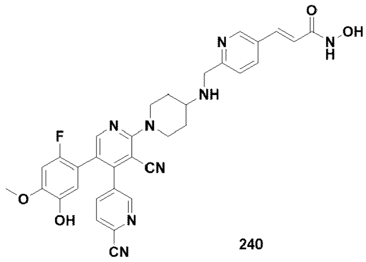
240

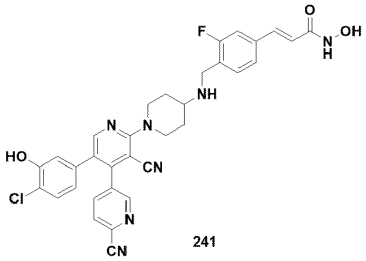
241

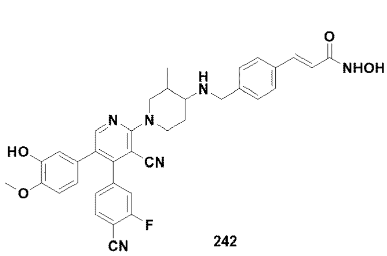
242

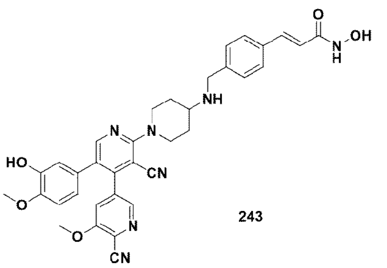
243

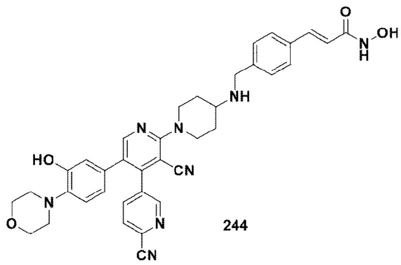
244

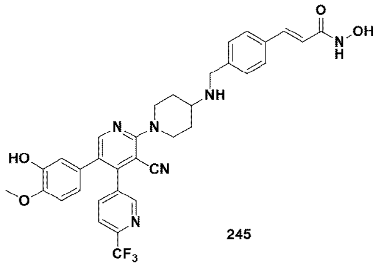
245

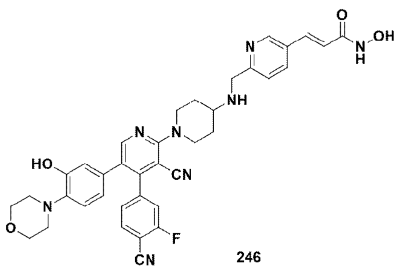
246

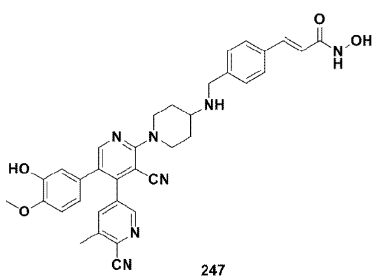
247

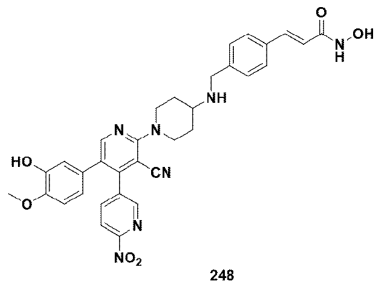
248

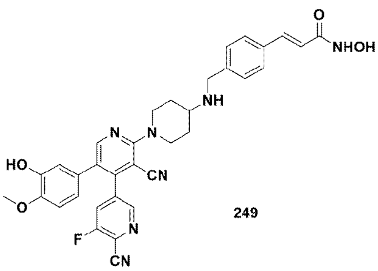
249

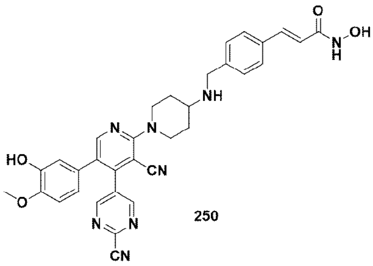
250

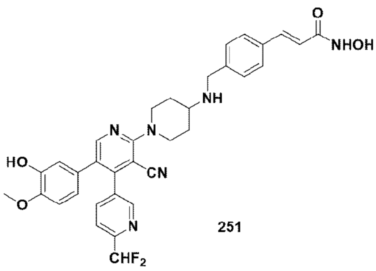
251

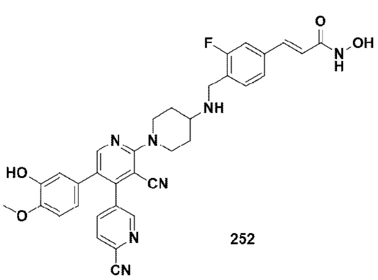
252

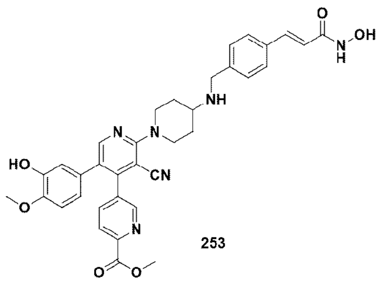
253

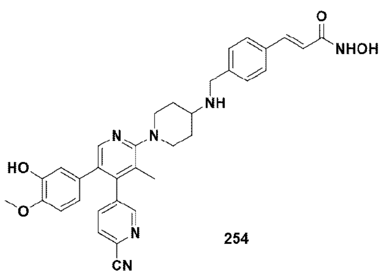
254

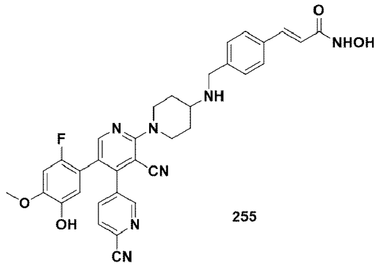
255

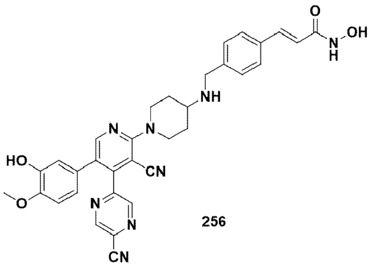
256

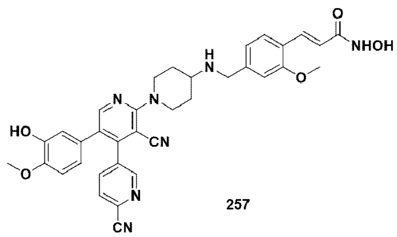
257

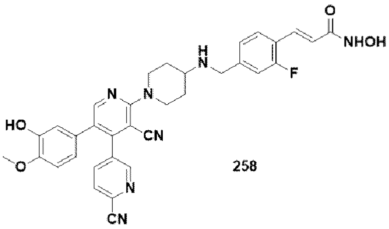
258

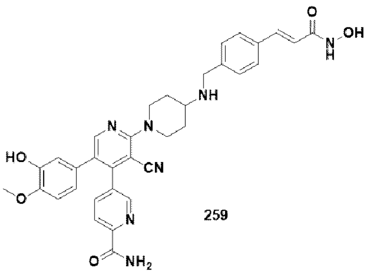
259

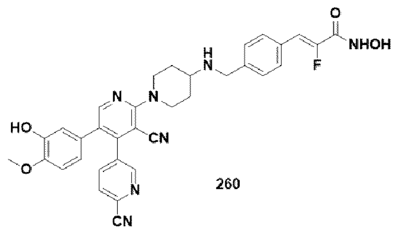
260

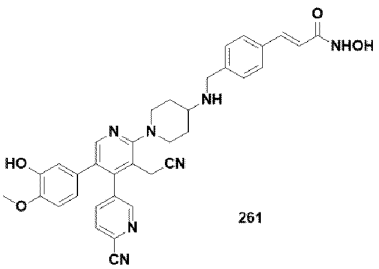
261

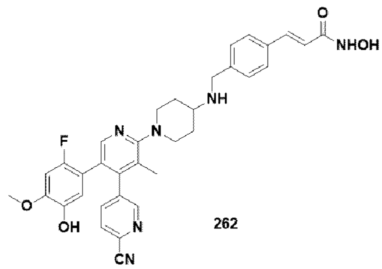
262

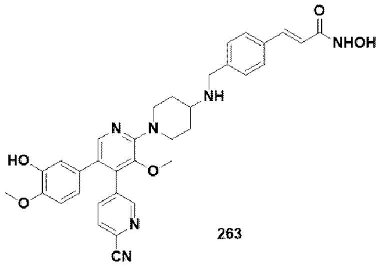
263

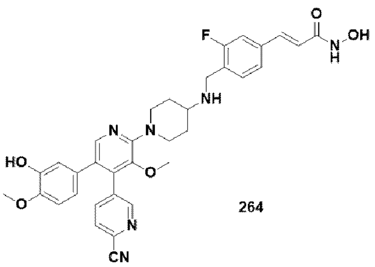
264

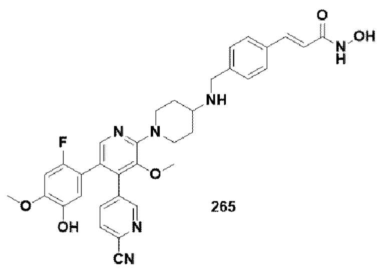
265

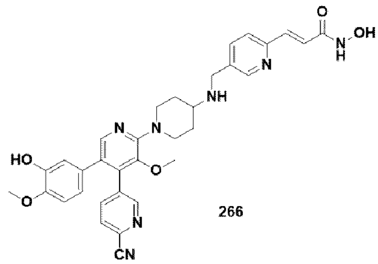
266

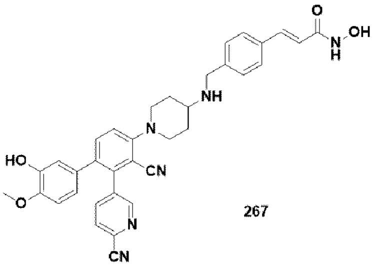
267

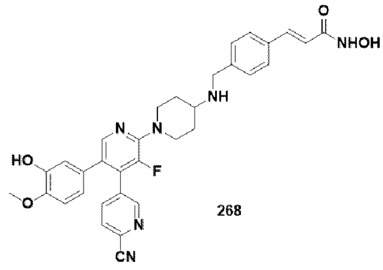
268

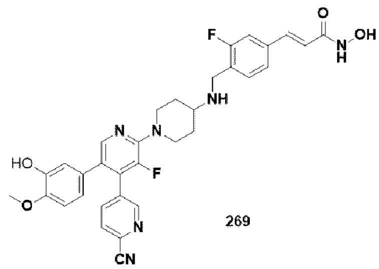
269

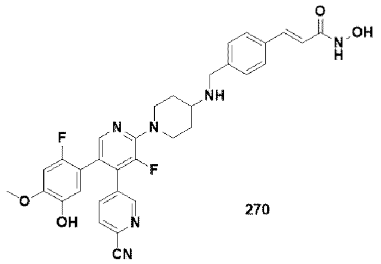

270

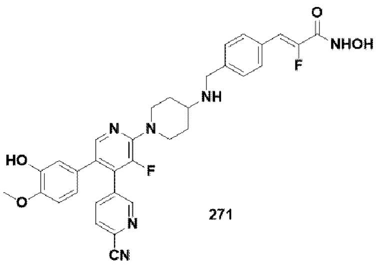

271

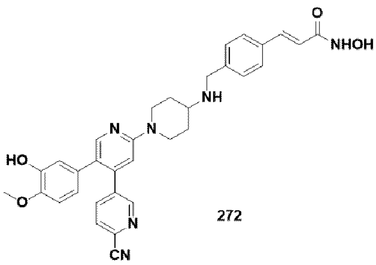

272

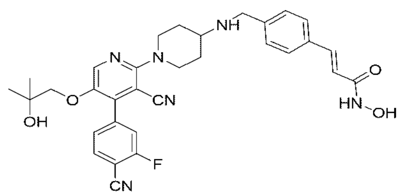

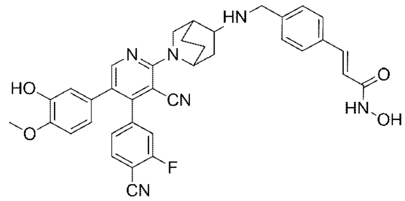

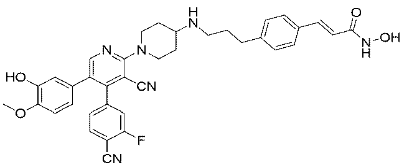

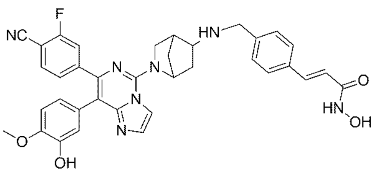

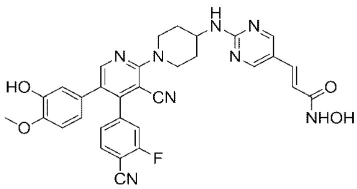

**26.** A pharmaceutical composition **characterized in that** the active ingredient of the pharmaceutical composition is selected from one or a combination of two or more of the compound or tautomer, stereoisomer, solvate, metabolite, isotopically-labeled compound, pharmaceutically acceptable salt or co-crystal thereof of any one of claims 1 to 25.

**27.** Use of the compound or tautomer, stereoisomer, solvate, metabolite, isotopically-labeled compound, pharmaceutically acceptable salt or co-crystal thereof of any one of claims 1 to 25 for preventing and treating the related disease mediated respectively or synergistically by LSD1 and/or HDAC;
further, the HDAC enzyme comprises, but is not limited to isoforms of HDAC1, HDAC2, HDAC3, HDAC4, HDAC5, HDAC6, HDAC7, HDAC8 etc., preferably HDAC1 or HDAC8 isoform, further preferably HDAC1 isoform.

**28.** Use of the compound or tautomer, stereoisomer, solvate, metabolite, isotopically-labeled compound, pharmaceutically acceptable salt or co-crystal thereof of any one of claims 1 to 25 in the manufacture of a medicament for treating the disease mediated by LSD1 and/or HDAC;

further, in the manufacture of a medicament for treating the disease mediated by one or more of LSD1, HDAC6, HDAC8;
preferably, the disease is cancer or autoimmune disease;
preferably, the cancer is selected from: non-small cell lung cancer, small cell lung cancer, pancreatic cancer, ovarian cancer, bladder cancer, prostate cancer, chronic myeloid leukemia, colorectal cancer, brain cancer, liver cancer, kidney cancer, gastric cancer, breast cancer, triple negative breast cancer, skin cancer, melanoma, head and neck cancer, bone cancer, cervical cancer, pelvic cancer, vaginal cancer, oral cancer, lymphoma, blood cancer, esophageal cancer, urethral cancer, nasal cavity cancer.

**29.** Use of the compound or tautomer, stereoisomer, solvate, metabolite, isotopically-labeled compound, pharmaceutically acceptable salt or co-crystal thereof of any one of claims 1 to 25, for preventing and treating the related disease mediated respectively or synergistically by LSD1 protein and/or HDAC1 protein, LSD1 protein and/or HDAC8 protein.

**INTERNATIONAL SEARCH REPORT**

International application No.
**PCT/CN2023/095304**

**A. CLASSIFICATION OF SUBJECT MATTER**

C07D401/04(2006.01)i; C07D401/14(2006.01)i; C07D471/08(2006.01)i; C07D487/04(2006.01)i; C07D409/06(2006.01)i; C07D207/14(2006.01)i; C07D211/58(2006.01)i; C07D405/14(2006.01)i; C07D413/04(2006.01)i; C07D417/14(2006.01)i; C07D413/14(2006.01)i; C07D409/14(2006.01)i; C07D471/10(2006.01)i; C07D403/10(2006.01)i; A61K31/4545(2006.01)i; A61K31/4427(2006.01)i; A61K31/506(2006.01)i; A61K31/439(2006.01)i; A61P35/00(2006.01)i; A61P37/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, VEN, ENTXT, CNKI, 万方数据, WANFANG DATA, STNext(CAplus, Registry): LSD1, Lysine Specific Demethylase 1, 组蛋白去甲基化酶, KDM1A, 组蛋白去乙酰化酶, HDAC, histone deacetylase, 癌, 肿瘤, cancer, tumor, structure search

**C. DOCUMENTS CONSIDERED TO BE RELEVANT**

| Category* | Citation of document, with indication, where appropriate, of the relevant passages | Relevant to claim No. |
|---|---|---|
| X | WO 2019134087 A1 (PEKING UNIVERSITY SHENZHEN GRADUATE SCHOOL) 11 July 2019 (2019-07-11)<br>entire document, in particular claims 1-15 | 1-2, 9-16, 25-26, 28 |
| X | CN 103328446 A (F. HOFFMANN-LA ROCHE AG) 25 September 2013 (2013-09-25)<br>entire document, in particular claims 1-12, 15-18 | 1-2, 17-26, 28 |
| X | CN 106928198 A (HITGEN LTD.) 07 July 2017 (2017-07-07)<br>entire document, in particular claims 1-32, 37-43 | 1-2, 17-26, 28 |
| A | CN 101445469 A (METHYLGENE INC.) 03 June 2009 (2009-06-03)<br>entire document | 1-26, 28 |
| A | CN 109810108 A (EAST CHINA UNIVERSITY OF SCIENCE AND TECHNOLOGY et al.) 28 May 2019 (2019-05-28)<br>entire document | 1-26, 28 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 October 2023** | **07 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/095304**

**C. DOCUMENTS CONSIDERED TO BE RELEVANT**

| Category* | Citation of document, with indication, where appropriate, of the relevant passages | Relevant to claim No. |
|---|---|---|
| A | CN 1639125 A (JANSSEN PHARMACEUTICA N.V.) 13 July 2005 (2005-07-13) entire document | 1-26, 28 |
| A | US 2005234033 A1 (ANANDAN, SAMPATH K. et al.) 20 October 2005 (2005-10-20) entire document | 1-26, 28 |
| A | WO 03076430 A1 (JANSSEN PHARMACEUTICA N.V. et al.) 18 September 2003 (2003-09-18) entire document | 1-26, 28 |
| A | WO 2010100475 A1 (ASTRAZENECA AB et al.) 10 September 2010 (2010-09-10) entire document | 1-26, 28 |
| A | WO 2020193431 A1 (DEUTSCHES KREBSFORSCH) 01 October 2020 (2020-10-01) entire document | 1-26, 28 |

Form PCT/ISA/210 (second sheet) (July 2022)

**INTERNATIONAL SEARCH REPORT**

International application No.
**PCT/CN2023/095304**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **27, 29**
because they relate to subject matter not required to be searched by this Authority, namely:

   The subject matter of claims 27 and 29 relates to a method for treatment of the human or animal body, which belongs to the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/095304**

| Patent document cited in search report | | | Publication date (day/month/year) | Patent family member(s) | | | Publication date (day/month/year) |
|---|---|---|---|---|---|---|---|
| WO | 2019134087 | A1 | 11 July 2019 | US | 2020331882 | A1 | 22 October 2020 |
| | | | | EP | 3736266 | A1 | 11 November 2020 |
| | | | | JP | 2021510152 | A | 15 April 2021 |
| CN | 103328446 | A | 25 September 2013 | CA | 2823780 | A1 | 26 July 2012 |
| | | | | US | 2012190700 | A1 | 26 July 2012 |
| | | | | WO | 2012098132 | A1 | 26 July 2012 |
| | | | | US | 8404738 | B2 | 26 March 2013 |
| | | | | KR | 20130120517 | A | 04 November 2013 |
| | | | | EP | 2665706 | A1 | 27 November 2013 |
| | | | | JP | 2014502983 | A | 06 February 2014 |
| | | | | HK | 1185874 | A0 | 28 February 2014 |
| | | | | EP | 2665706 | B1 | 22 October 2014 |
| | | | | ES | 2525569 | T3 | 26 December 2014 |
| | | | | RU | 2013136412 | A | 27 February 2015 |
| | | | | JP | 5778297 | B2 | 16 September 2015 |
| | | | | KR | 101569261 | B1 | 13 November 2015 |
| | | | | CN | 103328446 | B | 20 January 2016 |
| CN | 106928198 | A | 07 July 2017 | CA | 3010326 | A1 | 06 July 2017 |
| | | | | CA | 3010326 | C | 14 July 2020 |
| | | | | DK | 3398598 | T3 | 11 July 2022 |
| | | | | PL | 3398598 | T3 | 22 August 2022 |
| | | | | KR | 20180086258 | A | 30 July 2018 |
| | | | | KR | 102264012 | B1 | 10 June 2021 |
| | | | | PT | 3398598 | T | 12 July 2022 |
| | | | | US | 2019031612 | A1 | 31 January 2019 |
| | | | | US | 11149008 | B2 | 19 October 2021 |
| | | | | AU | 2016382372 | A1 | 19 July 2018 |
| | | | | AU | 2016382372 | B2 | 19 September 2019 |
| | | | | ES | 2920888 | T3 | 11 August 2022 |
| | | | | EP | 3398598 | A1 | 07 November 2018 |
| | | | | EP | 3398598 | A4 | 06 April 2022 |
| | | | | JP | 2019501919 | A | 24 January 2019 |
| | | | | JP | 6804540 | B2 | 23 December 2020 |
| | | | | WO | 2017114448 | A1 | 06 July 2017 |
| | | | | CN | 106928198 | B | 28 July 2020 |
| CN | 101445469 | A | 03 June 2009 | WO | 2005030705 | A1 | 07 April 2005 |
| | | | | AU | 2004276337 | A1 | 27 April 2006 |
| | | | | EP | 1663953 | A1 | 07 June 2006 |
| | | | | KR | 20060065730 | A | 14 June 2006 |
| | | | | CN | 1882529 | A | 20 December 2006 |
| | | | | JP | 2008094847 | A | 24 April 2008 |
| | | | | US | 2008132459 | A1 | 05 June 2008 |
| | | | | AU | 2004276337 | B2 | 12 November 2009 |
| | | | | JP | 4809228 | B2 | 09 November 2011 |
| | | | | US | 7868205 | B2 | 11 January 2011 |
| | | | | CN | 101445469 | B | 13 February 2013 |
| CN | 109810108 | A | 28 May 2019 | CN | 109810108 | B | 06 August 2021 |
| CN | 1639125 | A | 13 July 2005 | ES | 2371632 | T3 | 05 January 2012 |
| | | | | DE | 60326549 | D1 | 23 April 2009 |

Form PCT/ISA/210 (patent family annex) (July 2022)

**INTERNATIONAL SEARCH REPORT**
**Information on patent family members**

International application No.
**PCT/CN2023/095304**

| Patent document cited in search report | Publication date (day/month/year) | Patent family member(s) | | | Publication date (day/month/year) |
|---|---|---|---|---|---|
| | | AU | 2003212337 | A1 | 22 September 2003 |
| | | AU | 2003212337 | B2 | 11 June 2009 |
| | | IL | 164007 | A | 31 March 2011 |
| | | JP | 2005525381 | A | 25 August 2005 |
| | | JP | 4472353 | B2 | 02 June 2010 |
| | | HRP | 20040799 | A2 | 30 April 2005 |
| | | AU | 2003212335 | A1 | 22 September 2003 |
| | | AU | 2003212335 | B2 | 27 November 2008 |
| | | AU | 2003212335 | B8 | 23 April 2009 |
| | | IL | 164003 | A0 | 18 December 2005 |
| | | EP | 1485353 | A1 | 15 December 2004 |
| | | EP | 1485353 | B1 | 24 August 2011 |
| | | US | 2009170836 | A1 | 02 July 2009 |
| | | US | 8268833 | B2 | 18 September 2012 |
| | | AR | 039566 | A1 | 23 February 2005 |
| | | NZ | 534834 | A | 29 July 2005 |
| | | HRP | 20040803 | A2 | 28 February 2005 |
| | | AU | 2003218736 | A1 | 22 September 2003 |
| | | AU | 2003218736 | B2 | 08 January 2009 |
| | | ZA | 200407232 | B | 06 October 2005 |
| | | WO | 03075929 | A1 | 18 September 2003 |
| | | US | 2010010004 | A1 | 14 January 2010 |
| | | US | 8455498 | B2 | 04 June 2013 |
| | | AT | 521592 | T | 15 September 2011 |
| | | WO | 03076400 | A1 | 18 September 2003 |
| | | ZA | 200407234 | B | 06 October 2005 |
| | | DE | 60326436 | D1 | 16 April 2009 |
| | | CA | 2476583 | A1 | 18 September 2003 |
| | | CA | 2476583 | C | 05 July 2011 |
| | | NO | 20044113 | L | 28 September 2004 |
| | | WO | 03076430 | A1 | 18 September 2003 |
| | | ZA | 200407236 | B | 06 October 2005 |
| | | CA | 2475764 | A1 | 18 September 2003 |
| | | CA | 2475764 | C | 31 May 2011 |
| | | OA | 12791 | A | 10 July 2006 |
| | | MXPA | 04008797 | A | 26 November 2004 |
| | | US | 2013303506 | A1 | 14 November 2013 |
| | | US | 8916554 | B2 | 23 December 2014 |
| | | IL | 164005 | A0 | 18 December 2005 |
| | | ES | 2322950 | T3 | 02 July 2009 |
| | | UA | 78032 | C2 | 15 February 2007 |
| | | HK | 1078473 | A1 | 17 March 2006 |
| | | WO | 03076421 | A1 | 18 September 2003 |
| | | KR | 20040090985 | A | 27 October 2004 |
| | | US | 2005096468 | A1 | 05 May 2005 |
| | | US | 7615553 | B2 | 10 November 2009 |
| | | ES | 2322252 | T3 | 18 June 2009 |
| | | NZ | 534832 | A | 30 September 2005 |
| | | UA | 78031 | C2 | 15 February 2007 |
| | | US | 2009227558 | A1 | 10 September 2009 |

Form PCT/ISA/210 (patent family annex) (July 2022)

**INTERNATIONAL SEARCH REPORT**
**Information on patent family members**

International application No.
**PCT/CN2023/095304**

| Patent document cited in search report | Publication date (day/month/year) | Patent family member(s) | | | Publication date (day/month/year) |
|---|---|---|---|---|---|
| | | EP | 1485364 | A1 | 15 December 2004 |
| | | EP | 1485364 | B1 | 11 March 2009 |
| | | KR | 20040090979 | A | 27 October 2004 |
| | | US | 2012252790 | A1 | 04 October 2012 |
| | | US | 8524711 | B2 | 03 September 2013 |
| | | IL | 164006 | A | 30 November 2010 |
| | | EP | 1485370 | A1 | 15 December 2004 |
| | | EP | 1485370 | B1 | 04 March 2009 |
| | | UA | 77263 | C2 | 15 November 2006 |
| | | US | 2013245034 | A1 | 19 September 2013 |
| | | US | 8697717 | B2 | 15 April 2014 |
| | | ES | 2347544 | T3 | 02 November 2010 |
| | | BR | 0307624 | A | 11 January 2005 |
| | | IL | 164007 | A0 | 18 December 2005 |
| | | PL | 370992 | A1 | 13 June 2005 |
| | | PL | 213783 | B1 | 31 May 2013 |
| | | MXPA | 04008806 | A | 26 November 2004 |
| | | US | 2014171439 | A1 | 19 June 2014 |
| | | US | 9150560 | B2 | 06 October 2015 |
| | | JP | 2005525379 | A | 25 August 2005 |
| | | JP | 4725944 | B2 | 13 July 2011 |
| | | US | 2005222414 | A1 | 06 October 2005 |
| | | US | 7501417 | B2 | 10 March 2009 |
| | | US | 2005107384 | A1 | 19 May 2005 |
| | | US | 7816363 | B2 | 19 October 2010 |
| | | EP | 1485099 | A1 | 15 December 2004 |
| | | EP | 1485099 | B1 | 07 July 2010 |
| | | AU | 2003218737 | A1 | 22 September 2003 |
| | | AU | 2003218737 | B2 | 10 April 2008 |
| | | JP | 2005526067 | A | 02 September 2005 |
| | | JP | 4644428 | B2 | 02 March 2011 |
| | | US | 2010048588 | A1 | 25 February 2010 |
| | | US | 8114999 | B2 | 14 February 2012 |
| | | AT | 473005 | T | 15 July 2010 |
| | | ZA | 200407233 | B | 06 October 2005 |
| | | IL | 164004 | A0 | 18 December 2005 |
| | | JP | 2005523907 | A | 11 August 2005 |
| | | JP | 4836405 | B2 | 14 December 2011 |
| | | DE | 60333260 | D1 | 19 August 2010 |
| | | ZA | 200407235 | B | 04 October 2005 |
| | | US | 2009170881 | A1 | 02 July 2009 |
| | | US | 8343988 | B2 | 01 January 2013 |
| | | ZA | 200407237 | B | 28 September 2005 |
| | | US | 2012108603 | A1 | 03 May 2012 |
| | | NO | 20044194 | L | 01 October 2004 |
| | | AT | 424395 | T | 15 March 2009 |
| | | EA | 200401199 | A1 | 24 February 2005 |
| | | EA | 008245 | B1 | 27 April 2007 |
| | | UA | 78033 | C2 | 15 February 2007 |
| | | OA | 12790 | A | 10 July 2006 |

Form PCT/ISA/210 (patent family annex) (July 2022)

**INTERNATIONAL SEARCH REPORT**
**Information on patent family members**

International application No.
**PCT/CN2023/095304**

| Patent document cited in search report | | | Publication date (day/month/year) | Patent family member(s) | | | Publication date (day/month/year) |
|---|---|---|---|---|---|---|---|
| | | | | EA | 200401201 | A1 | 24 February 2005 |
| | | | | EA | 007272 | B1 | 25 August 2006 |
| | | | | AT | 425152 | T | 15 March 2009 |
| | | | | US | 2005119250 | A1 | 02 June 2005 |
| | | | | US | 7541369 | B2 | 02 June 2009 |
| | | | | CA | 2476065 | A1 | 18 September 2003 |
| | | | | CA | 2476065 | C | 24 July 2012 |
| | | | | CA | 2476296 | A1 | 18 September 2003 |
| | | | | CA | 2476296 | C | 22 February 2011 |
| | | | | TW | 200400822 | A | 16 January 2004 |
| | | | | BR | 0308081 | A | 21 December 2004 |
| | | | | IL | 164006 | A0 | 18 December 2005 |
| | | | | PL | 370991 | A1 | 13 June 2005 |
| | | | | PL | 212089 | B1 | 31 August 2012 |
| | | | | DK | 1485353 | T3 | 28 November 2011 |
| | | | | US | 2015065478 | A1 | 05 March 2015 |
| | | | | US | 9533979 | B2 | 03 January 2017 |
| | | | | US | 2015353549 | A1 | 10 December 2015 |
| | | | | US | 9556161 | B2 | 31 January 2017 |
| | | | | CN | 100519527 | C | 29 July 2009 |
| US | 2005234033 | A1 | 20 October 2005 | US | 2008139535 | A1 | 12 June 2008 |
| | | | | US | 7345043 | B2 | 18 March 2008 |
| WO | 03076430 | A1 | 18 September 2003 | AU | 2003212337 | A1 | 22 September 2003 |
| | | | | EP | 1485370 | A1 | 25 December 2004 |
| | | | | US | 2005119250 | A1 | 02 June 2005 |
| | | | | EP | 1485370 | B1 | 04 March 2009 |
| | | | | US | 7541369 | B2 | 02 June 2009 |
| | | | | AU | 2003212337 | B2 | 11 June 2009 |
| | | | | US | 2009227558 | A1 | 10 September 2009 |
| | | | | JP | 4472353 | B2 | 02 June 2010 |
| | | | | CA | 2476296 | C | 22 February 2011 |
| | | | | US | 2012252790 | A1 | 04 October 2012 |
| | | | | US | 8524711 | B2 | 03 September 2013 |
| WO | 2010100475 | A1 | 10 September 2010 | | None | | |
| WO | 2020193431 | A1 | 01 October 2020 | EP | 3941900 | A1 | 26 January 2022 |
| | | | | US | 2022151962 | A1 | 19 May 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111592487
- CN 113444038
- CN 113527195


### Non-patent literature cited in the description


- **SHI, Y.** ; **LAN, F.** ; **MATSON, C** ; **MULLIGAN, P.** ; **WHETSTINE, J.R.** ; **COLE, P.A.** ; **CASERO, R.A.** ; **SHI, Y.** Histone demethylation mediated by the nuclear amine oxidase homolog LSD1.. *Cell*, 2004, vol. 119, 941-953
- **DOLL, S.** ; **KRIEGMAIR, M. C.** ; **SANTOS, A.** ; **WIERER, M.** ; **COSCIA, F.** ; **NEIL, H. M. et al.** Rapid proteomic analysis for solid tumors reveals LSD1 as a drug target in an end-stage cancer patient.. *Molecular Oncology*, 2018, vol. 12 (8), 1296-1307
- **CHIAPPINELLI, K.B.** ; **STRISSEL, P.L.** ; **DESRICHARD, A.** ; **LI, H.** ; **HENKE, C.** ; **AKMAN, B.** ; **HEIN, A** ; **ROTE, N.S** ; **COPE, L.M** ; **SNYDER, A**. Inhibiting DNA methylation causes an interferon response in cancer via dsRNA including endogenous retroviruses.. *Cell*, 2015, vol. 162, 974-986
- **TOPPER, M.J.** ; **VAZ, M.** ; **CHIAPPINELLI, K.B** ; **DESTEFANO SHIELDS, C.E** ; **NIKNAFS, N.** ; **YEN, R.C** ; **WENZEL, A** ; **HICKS, J.** ; **BALLEW, M.** ; **STONE, M. et al.** Epigenetic therapy ties MYC depletion to reversing immune evasion and treating lung cancer.. *Cell*, 2017, vol. 171, 1284-130
- **GHONEIM, H.E** ; **FAN, Y** ; **MOUSTAKI, A** ; **ABDELSAMED, H.A.** ; **DASH, P.** ; **DOGRA, P.** ; **CARTER, R.** ; **AWAD, W.** ; **NEALE, G** ; **THOMAS, P.G. et al.** De novo epigenetic programs inhibit PD-1 blockade-mediated t cell rejuvenation.. *Cell*, 2017, vol. 170, 142-157
- **WANG, Y.** ; **ZHANG, H. et al.** *Cell*, 2009, vol. 138 (4), 660-72
- **ZHAO, L.-J.** ; **FAN, Q.-Q. et al.** *Pharmacol. Res.*, 2020, vol. 159, 104991
- **SEHRAWAT, A.** ; **GAO,L et al.** *Proc. Nat. Acad. Sci. USA*, 2018, vol. 115 (18), E4179-E4188
- **HATZI , K.** ; **GENG, H. et al.** *Nature Immunology*, 2019, vol. 20 (1), 86-96
- **LYNCH, J.** ; **HARRIS, W. et al.** *Expert Opinion on Therapeutic Targets*, 2012, vol. 16 (12), 1239-1249
- **ANCELIN, K.** ; **SYX , L et al.** *eLife*, 2016, vol. 5, e08851, 1-e08851, 24
- **SUN, J** ; **ERMANN, J. et al.** *Bone Res*, 2018, vol. 6 (1), 1-12
- **TAN, A.H.Y.** ; **TU, W.J. et al.** *Front. Immunol.*, 2019, vol. 10, 1351
- **HATZI , K.** ; **GENG, H et al.** *Nature Immunology*, 2019, vol. 20 (1), 86-96
- **DUAN, Y.C et al.** *Eur J Med Chem*, 2021, vol. 220, 113453
- **GIULIA S. et al.** *Current Opinion in Chemical Biology*, 2019, vol. 50, 89-100
- **DE LERA, A.R.** ; **GANESAN, A**. *Clin Epigenetics*, 2016, vol. 8, 105
- **FU, R.G.** ; **SUN, Y** ; **SHENG, W.B.** ; **LIAO, D.F.** *Eur. J. Med. Chem.*, 2017, vol. 136, 195-211
- **KALIN, J.H. et al.** *Nat. Commun.*, 2018, vol. 9, 53
- **STAHL** ; **WERMUTH'S**. Handbook of Pharmaceutical Salts: properties, selection, and use. Wiley-VCH, 2002
- **KANOUNI, T et al.** *J. Med. Chem.*, 2020, vol. 63, 14522-14529